(19) Europäisches Patentamt
European Patent Office
Office européen des brevets

(11) EP 3 581 651 A1

(12) EUROPEAN PATENT APPLICATION
published in accordance with Art. 153(4) EPC

(43) Date of publication:
18.12.2019 Bulletin 2019/51

(21) Application number: 18750649.8

(22) Date of filing: 06.02.2018

(51) Int Cl.:
*C12N 15/09* (2006.01) *A61K 39/395* (2006.01)
*A61P 35/00* (2006.01) *A61P 35/02* (2006.01)
*C07K 16/28* (2006.01) *C07K 16/46* (2006.01)
*C12N 1/15* (2006.01) *C12N 1/19* (2006.01)
*C12N 1/21* (2006.01) *C12N 5/10* (2006.01)
*C12P 21/08* (2006.01)

(86) International application number:
PCT/JP2018/003888

(87) International publication number:
WO 2018/147245 (16.08.2018 Gazette 2018/33)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA MD TN

(30) Priority: 07.02.2017 JP 2017020220

(71) Applicant: Daiichi Sankyo Company, Limited
Tokyo 103-8426 (JP)

(72) Inventors:
- CHAEN, Takashi
Tokyo 103-8426 (JP)
- OHTSUKA, Toshiaki
Tokyo 103-8426 (JP)
- IIDA, Kenji
Tokyo 103-8426 (JP)
- NAKAMURA, Kensuke
Tokyo 103-8426 (JP)
- ABURATANI, Takahide
Tokyo 103-8426 (JP)
- ICHIKAWA, Junya
Tokyo 103-8426 (JP)
- KUDO, Shota
Tokyo 103-8426 (JP)
- PISCITELLI, Chayne Lee
Vancouver
BC V6K 1L1 (CA)
- SANCHES, Mario
Vancouver
BC V6K 1Z9 (CA)

(74) Representative: Arends, William Gerrit
Marks & Clerk LLP
15 Fetter Lane
London EC4A 1BW (GB)

# (54) ANTI-GPRC5D ANTIBODY AND MOLECULE CONTAINING SAME

(57) [Problem to be Solved]

The present invention provides a novel antibody that binds to human GPRC5D, or a molecule having antigen binding activity, comprising the antibody.

[Solution]

The present invention provides a novel antibody that binds to human GPRC5D, a molecule having antigen binding activity, comprising the antibody, an anti-tumor pharmaceutical composition comprising the antibody or the molecule as an active ingredient, etc.

EP 3 581 651 A1

## Description

[Technical Field]

The present invention relates to a novel anti-GPRC5D antibody and a molecule comprising the antibody.

[Background Art]

G-protein coupled receptor family C group 5 member D (GPRC5D) is one of the G-protein coupled receptors found by a homology search of the EST database using the amino acid sequences of a series of human GPCRs (Non Patent Literature 1). GPCR family C group 5 receptors (GPRC5 receptors) have 4 subtypes (GPRC5A, GPRC5B, GPRC5C, and GPRC5D) and are also known as retinoic acid-inducible orphan G protein-coupled receptors (RAIG) because their expression is induced by retinoic acid stimulation (Non Patent Literature 2). However, the biological functions or biological ligand of GPRC5D, the subtype of G protein to be coupled therewith, etc. have not yet been revealed.

As for the association of the GPRC5D gene with cancers, GPRC5D is known to be highly expressed in multiple myeloma. Specifically, it is known that, for example: the overexpression of GPRC5D correlates with the poor prognosis of multiple myeloma patients (Non Patent Literature 3); and the proportion of cells expressing GPRC5D is decreased by the medication of multiple myeloma patients (Non Patent Literature 4). Such association of the overexpression of GPRC5D with cancers suggests the possibility that GPRC5D serves as an excellent therapeutic target for cancers. Furthermore, there is also a report on an anti-GPRC5D antibody and a bispecific antibody that comprises the antibody and an anti-CD3 antibody, which exhibits binding properties to 3T3 cells expressing GPRC5D (Patent Literature 1) exogenously. However, medical pharmaceutical products targeting GPRC5D have not yet been developed.

[Prior Art Literature]

[Patent Literature]

[Patent Literature 1] International Publication No. WO2016/090329

[Non-Patent Literature]

[Non-Patent Literature 1] H Brauner-Osborne, et al., Biochim Biophys Acta., published in April 2001, Vol. 1518 (No. 3), p. 237-248 [Non-Patent Literature 2] S Inoue, et al., Journal of Investigative Dermatology, published in March 2004, Vol. 122 (No. 3), p. 565-573 [Non-Patent Literature 3] J Atamaniuk, et al., European Journal of Clinical Investigation, published in May 2012, Vol. 42 (No. 9), p. 953-960 [Non-Patent Literature 4] Y Cohen, et al., Hematology), published in November 2013, Vol. 18 (No. 6), p. 348-351

[Summary of Invention]

[Technical Problem]

An object of the present invention is to provide an anti-GPRC5D antibody having an anticancer effect, an antigen-binding fragment of the antibody, and a molecule comprising the antibody or the antigen-binding fragment of the antibody.

Another object of the present invention is to provide a pharmaceutical composition comprising the antibody, the antigen-binding fragment of the antibody, or the molecule, etc.

An alternative object of the present invention is to provide a polynucleotide comprising a nucleotide sequence encoding the amino acid sequence of the antibody, the antigen-binding fragment of the antibody, or the molecule, a vector having an insert of the polynucleotide, a cell transfected with the polynucleotide or vector, and a method for producing the antibody or the antigen-binding fragment of the antibody, or the molecule, comprising a step of culturing the cell. A further alternative object of the present invention is to provide a method for treating a cancer using the antibody, the antigen-binding fragment of the antibody, or the molecule.

[Means for Solving the Problem]

The present inventors have conducted diligent studies to attain the foregoing objects and have completed the present invention by developing a novel anti-GPRC5D antibody and finding that the antibody has an anticancer effect.

The present invention provides:

(1) An antibody or an antigen-binding fragment of the antibody, wherein the antibody comprises a heavy chain variable region and a light chain variable region according to any one of the following (I) to (III):

(II)

a heavy chain variable region comprising
heavy chain CDR1 consisting of an amino acid sequence represented by SEQ ID NO: 48,
heavy chain CDR2 consisting of an amino acid sequence represented by SEQ ID NO: 49, and
heavy chain CDR3 consisting of an amino acid sequence represented by SEQ ID NO: 50,
and
a light chain variable region comprising
light chain CDR1 consisting of an amino acid sequence represented by SEQ ID NO: 57,
light chain CDR2 consisting of an amino acid sequence represented by SEQ ID NO: 58, and
light chain CDR3 consisting of an amino acid sequence represented by SEQ ID NO: 59,

(I)

a heavy chain variable region comprising
heavy chain CDR1 consisting of an amino acid sequence represented by SEQ ID NO: 45,
heavy chain CDR2 consisting of an amino acid sequence represented by SEQ ID NO: 46, and
heavy chain CDR3 consisting of an amino acid sequence represented by SEQ ID NO: 47,
and
a light chain variable region comprising
light chain CDR1 consisting of an amino acid sequence represented by SEQ ID NO: 54,
light chain CDR2 consisting of an amino acid sequence represented by SEQ ID NO: 55, and
light chain CDR3 consisting of an amino acid sequence represented by SEQ ID NO: 56,

and
(III)

a heavy chain variable region comprising
heavy chain CDR1 consisting of an amino acid sequence represented by SEQ ID NO: 51,
heavy chain CDR2 consisting of an amino acid sequence represented by SEQ ID NO: 52, and
heavy chain CDR3 consisting of an amino acid sequence represented by SEQ ID NO: 53,
and
a light chain variable region comprising
light chain CDR1 consisting of an amino acid sequence represented by SEQ ID NO: 60,
light chain CDR2 consisting of an amino acid sequence represented by SEQ ID NO: 61, and
light chain CDR3 consisting of an amino acid sequence represented by SEQ ID NO: 62
and binds to human GPRC5D;

(2) The antibody or the antigen-binding fragment of the antibody according to (1), wherein the heavy chain variable region and the light chain variable region are the heavy chain variable region and the light chain variable region according to (II);
(3) The antibody or the antigen-binding fragment of the antibody according to (1), wherein the heavy chain variable region and the light chain variable region are the heavy chain variable region and the light chain variable region according to (I);
(4) The antibody or the antigen-binding fragment of the antibody according to (1), wherein the heavy chain variable region and the light chain variable region are the heavy chain variable region and the light chain variable region according to (III);
(5) The antibody or the antigen-binding fragment of the antibody according to any one of (1) to (4), wherein the antibody or the antigen-binding fragment is a chimeric antibody or an antigen-binding fragment of the antibody;
(6) The antibody or the antigen-binding fragment of the antibody according to any one of (1) to (4), wherein the antibody or the antigen-binding fragment is a humanized antibody or an antigen-binding fragment of the antibody;
(7) The antibody or the antigen-binding fragment of the antibody according to any one of (1) to (4), wherein the antibody or the antigen-binding fragment is a human antibody or an antigen-binding fragment of the antibody;
(8) The antibody or the antigen-binding fragment of the antibody according to any one of (1), (2), and (6), wherein the antibody comprises

a light chain variable region comprising any one amino acid sequence represented by
amino acid residues 21 to 127 of an amino acid sequence represented by SEQ ID NO: 64,
amino acid residues 21 to 127 of an amino acid sequence represented by SEQ ID NO: 66,
amino acid residues 21 to 127 of an amino acid sequence represented by SEQ ID NO: 68,
amino acid residues 21 to 127 of an amino acid sequence represented by SEQ ID NO: 70, and
amino acid residues 21 to 127 of an amino acid sequence represented by SEQ ID NO: 72, and
a heavy chain variable region comprising any one amino acid sequence represented by
amino acid residues 20 to 142 of an amino acid sequence represented by SEQ ID NO: 74,
amino acid residues 20 to 142 of an amino acid sequence represented by SEQ ID NO: 76,
amino acid residues 20 to 142 of an amino acid sequence represented by SEQ ID NO: 78, and
amino acid residues 20 to 142 of an amino acid sequence represented by SEQ ID NO: 80;
(9) The antibody or the antigen-binding fragment of the antibody according to any one of (1), (2), (6), and (8), wherein the antibody comprises any one combination of a heavy chain variable region and a light chain variable region of

- a heavy chain variable region comprising an amino acid sequence represented by amino acid residues 20 to 142 of the amino acid sequence represented by SEQ ID NO: 74, and a light chain variable region comprising an amino acid sequence represented by amino acid residues 21 to 127 of the amino acid sequence represented by SEQ ID NO: 64,
- a heavy chain variable region comprising an amino acid sequence represented by amino acid residues 20 to 142 of the amino acid sequence represented by SEQ ID NO: 74, and a light chain variable region comprising an amino acid sequence represented by amino acid residues 21 to 127 of the amino acid sequence represented by SEQ ID NO: 66,
- a heavy chain variable region comprising an amino acid sequence represented by amino acid residues 20 to 142 of the amino acid sequence represented by SEQ ID NO: 76, and a light chain variable region comprising an amino acid sequence represented by amino acid residues 21 to 127 of the amino acid sequence represented by SEQ ID NO: 66,
- a heavy chain variable region comprising an amino acid sequence represented by amino acid residues 20 to 142 of the amino acid sequence represented by SEQ ID NO: 76, and a light chain variable region comprising an amino acid sequence represented by amino acid residues 21 to 127 of the amino acid sequence represented by SEQ ID NO: 68,
- a heavy chain variable region comprising an amino acid sequence represented by amino acid residues 20 to 142 of the amino acid sequence represented by SEQ ID NO: 76, and a light chain variable region comprising an amino acid sequence represented by amino acid residues 21 to 127 of the amino acid sequence represented by SEQ ID NO: 70,
- a heavy chain variable region comprising an amino acid sequence represented by amino acid residues 20 to 142 of the amino acid sequence represented by SEQ ID NO: 76, and a light chain variable region comprising an amino acid sequence represented by amino acid residues 21 to 127 of the amino acid sequence represented by SEQ ID NO: 72,
- a heavy chain variable region comprising an amino acid sequence represented by amino acid residues 20 to 142 of the amino acid sequence represented by SEQ ID NO: 78, and a light chain variable region comprising an amino acid sequence represented by amino acid residues 21 to 127 of the amino acid sequence represented by SEQ ID NO: 68,
- a heavy chain variable region comprising an amino acid sequence represented by amino acid residues 20 to 142 of the amino acid sequence represented by SEQ ID NO: 78, and a light chain variable region comprising an amino acid sequence represented by amino acid residues 21 to 127 of the amino acid sequence represented by SEQ ID NO: 70,
- a heavy chain variable region comprising an amino acid sequence represented by amino acid residues 20 to 142 of the amino acid sequence represented by SEQ ID NO: 78, and a light chain variable region comprising an amino acid sequence represented by amino acid residues 21 to 127 of the amino acid sequence represented by SEQ ID NO: 72,
- a heavy chain variable region comprising an amino acid sequence represented by amino acid residues 20 to 142 of the amino acid sequence represented by SEQ ID NO: 80, and a light chain variable region comprising an amino acid sequence represented by amino acid residues 21 to 127 of the amino acid sequence represented by SEQ ID NO: 64,
- a heavy chain variable region comprising an amino acid sequence represented by amino acid residues 20 to 142 of the amino acid sequence represented by SEQ ID NO: 80, and a light chain variable region comprising an amino acid sequence represented by amino acid residues 21 to 127 of the amino acid sequence represented by SEQ ID NO: 68,

- a heavy chain variable region comprising an amino acid sequence represented by amino acid residues 20 to 142 of the amino acid sequence represented by SEQ ID NO: 80, and a light chain variable region comprising an amino acid sequence represented by amino acid residues 21 to 127 of the amino acid sequence represented by SEQ ID NO: 70, or
- a heavy chain variable region comprising an amino acid sequence represented by amino acid residues 20 to 142 of the amino acid sequence represented by SEQ ID NO: 80, and a light chain variable region comprising an amino acid sequence represented by amino acid residues 21 to 127 of the amino acid sequence represented by SEQ ID NO: 72;

(10) The antibody or the antigen-binding fragment of the antibody according to any one of (1), (2), and (6), wherein the antibody comprises a light chain variable region comprising an amino acid sequence represented by
amino acid residues 21 to 126 of an amino acid sequence represented by SEQ ID NO: 82, or
amino acid residues 21 to 126 of an amino acid sequence represented by SEQ ID NO: 84, and
a heavy chain variable region comprising any one amino acid sequence represented by
amino acid residues 20 to 142 of an amino acid sequence represented by SEQ ID NO: 86,
amino acid residues 20 to 142 of an amino acid sequence represented by SEQ ID NO: 88,
amino acid residues 20 to 142 of an amino acid sequence represented by SEQ ID NO: 90, and
amino acid residues 20 to 142 of an amino acid sequence represented by SEQ ID NO: 92;
(11) The antibody or the antigen-binding fragment of the antibody according to any one of (1), (4), (6), and (10), wherein the antibody comprises any one combination of a heavy chain variable region and a light chain variable region of

- a heavy chain variable region comprising an amino acid sequence represented by amino acid residues 20 to 142 of the amino acid sequence represented by SEQ ID NO: 86, and a light chain variable region comprising an amino acid sequence represented by amino acid residues 21 to 126 of the amino acid sequence represented by SEQ ID NO: 84,
- a heavy chain variable region comprising an amino acid sequence represented by amino acid residues 20 to 142 of the amino acid sequence represented by SEQ ID NO: 88, and a light chain variable region comprising an amino acid sequence represented by amino acid residues 21 to 126 of the amino acid sequence represented by SEQ ID NO: 84,
- a heavy chain variable region comprising an amino acid sequence represented by amino acid residues 20 to 142 of the amino acid sequence represented by SEQ ID NO: 90, and a light chain variable region comprising an amino acid sequence represented by amino acid residues 21 to 126 of the amino acid sequence represented by SEQ ID NO: 82,
- a heavy chain variable region comprising an amino acid sequence represented by amino acid residues 20 to 142 of the amino acid sequence represented by SEQ ID NO: 90, and a light chain variable region comprising an amino acid sequence represented by amino acid residues 21 to 126 of the amino acid sequence represented by SEQ ID NO: 84, or
- a heavy chain variable region comprising an amino acid sequence represented by amino acid residues 20 to 142 of the amino acid sequence represented by SEQ ID NO: 92, and a light chain variable region comprising an amino acid sequence represented by amino acid residues 21 to 126 of the amino acid sequence represented by SEQ ID NO: 82;

(12) The antibody according to any one of (1) to (11), wherein the antibody comprises Fc;
(13) An antibody or an antigen-binding fragment of the antibody, wherein the antibody binds to human GPRC5D and comprises a heavy chain variable region and a light chain variable region according to any one of the following ① to ④:

①

a heavy chain variable region comprising
heavy chain CDR1 consisting of an amino acid sequence represented by SEQ ID NO: 111,
heavy chain CDR2 consisting of an amino acid sequence represented by SEQ ID NO: 112, and
heavy chain CDR3 consisting of an amino acid sequence represented by SEQ ID NO: 113,
and
a light chain variable region comprising
light chain CDR1 consisting of an amino acid sequence represented by SEQ ID NO: 114,
light chain CDR2 consisting of an amino acid sequence represented by SEQ ID NO: 115, and

light chain CDR3 consisting of an amino acid sequence represented by SEQ ID NO: 116,

②

a heavy chain variable region comprising
heavy chain CDR1 consisting of an amino acid sequence represented by SEQ ID NO: 117,
heavy chain CDR2 consisting of an amino acid sequence represented by SEQ ID NO: 118, and
heavy chain CDR3 consisting of an amino acid sequence represented by SEQ ID NO: 119,
and
a light chain variable region comprising
light chain CDR1 consisting of an amino acid sequence represented by SEQ ID NO: 120,
light chain CDR2 consisting of an amino acid sequence represented by SEQ ID NO: 121, and
light chain CDR3 consisting of an amino acid sequence represented by SEQ ID NO: 122,

③

a heavy chain variable region comprising
heavy chain CDR1 consisting of an amino acid sequence represented by SEQ ID NO: 123,
heavy chain CDR2 consisting of an amino acid sequence represented by SEQ ID NO: 124, and
heavy chain CDR3 consisting of an amino acid sequence represented by SEQ ID NO: 125,
and
a light chain variable region comprising
light chain CDR1 consisting of an amino acid sequence represented by SEQ ID NO: 126,
light chain CDR2 consisting of an amino acid sequence represented by SEQ ID NO: 127, and
light chain CDR3 consisting of an amino acid sequence represented by SEQ ID NO: 128, and

④

a heavy chain variable region comprising
heavy chain CDR1 consisting of an amino acid sequence represented by SEQ ID NO: 129,
heavy chain CDR2 consisting of an amino acid sequence represented by SEQ ID NO: 130, and
heavy chain CDR3 consisting of an amino acid sequence represented by SEQ ID NO: 131,
and
a light chain variable region comprising
light chain CDR1 consisting of an amino acid sequence represented by SEQ ID NO: 132,
light chain CDR2 consisting of an amino acid sequence represented by SEQ ID NO: 133, and
light chain CDR3 consisting of an amino acid sequence represented by SEQ ID NO: 134;

(14) The antibody or the antigen-binding fragment of the antibody according to (13), wherein the heavy chain variable region and the light chain variable region are the heavy chain variable region and the light chain variable region according to ①;
(15) The antibody or the antigen-binding fragment of the antibody according to (13), wherein the heavy chain variable region and the light chain variable region are the heavy chain variable region and the light chain variable region according to ②;
(16) The antibody or the antigen-binding fragment of the antibody according to (13), wherein the heavy chain variable region and the light chain variable region are the heavy chain variable region and the light chain variable region according to ③;
(17) The antibody or the antigen-binding fragment of the antibody according to (13), wherein the heavy chain variable region and the light chain variable region are the heavy chain variable region and the light chain variable region according to ④;
(18) The antibody or the antigen-binding fragment of the antibody according to any one of (13) to (17), wherein the antibody comprises
a heavy chain variable region comprising any one of
the amino acid sequence represented by SEQ ID NO: 97,
the amino acid sequence represented by SEQ ID NO: 101,
the amino acid sequence represented by SEQ ID NO: 105, and
the amino acid sequence represented by SEQ ID NO: 109,
and

a light chain variable region comprising any one of
the amino acid sequence represented by SEQ ID NO: 99,
the amino acid sequence represented by SEQ ID NO: 103,
the amino acid sequence represented by SEQ ID NO: 107, and
the amino acid sequence represented by SEQ ID NO: 135;
(19) The antibody or the antigen-binding fragment of the antibody according to any one of (13) to (18), wherein the antibody comprises any one combination of a heavy chain variable region and a light chain variable region of

- a heavy chain variable region comprising the amino acid sequence represented by SEQ ID NO: 97, and a light chain variable region comprising the amino acid sequence represented by SEQ ID NO: 99,
- a heavy chain variable region comprising the amino acid sequence represented by SEQ ID NO: 101, and a light chain variable region comprising the amino acid sequence represented by SEQ ID NO: 103,
- a heavy chain variable region comprising the amino acid sequence represented by SEQ ID NO: 105, and a light chain variable region comprising the amino acid sequence represented by SEQ ID NO: 107, and
- a heavy chain variable region comprising the amino acid sequence represented by SEQ ID NO: 109, and a light chain variable region comprising the amino acid sequence represented by SEQ ID NO: 135;

(20) An antibody or an antigen-binding fragment of the antibody, wherein the antibody comprises any one combination of a heavy chain and a light chain of
a heavy chain comprising an amino acid sequence represented by SEQ ID NO: 144, and a light chain comprising an amino acid sequence represented by SEQ ID NO: 145,
a heavy chain comprising an amino acid sequence represented by SEQ ID NO: 146, and a light chain comprising an amino acid sequence represented by SEQ ID NO: 147,
a heavy chain comprising an amino acid sequence represented by SEQ ID NO: 148, and a light chain comprising an amino acid sequence represented by SEQ ID NO: 149, or
a heavy chain comprising an amino acid sequence represented by SEQ ID NO: 150, and a light chain comprising an amino acid sequence represented by SEQ ID NO: 151;
(21) An antibody or an antigen-binding fragment of the antibody, wherein the antibody or the antigen-binding fragment comprises an amino acid sequence encoded by a nucleotide sequence contained in a polynucleotide hybridizing under stringent conditions to a complementary strand of a polynucleotide comprising a nucleotide sequence encoding an amino acid sequence contained in the antibody or the antigen-binding fragment of the antibody according to any one of (8) to (12) and (18) to (20), and binds to human GPRC5D;
(22) An antibody or an antigen-binding fragment of the antibody, wherein the antibody or the antigen-binding fragment comprises an amino acid sequence 90% or more identical to an amino acid sequence contained in the antibody or the antigen-binding fragment of the antibody according to any one of (8) to (12) and (18) to (20), and binds to human GPRC5D;
(23) An antibody or an antigen-binding fragment of the antibody, wherein the antibody or the antigen-binding fragment comprises an amino acid sequence derived by the substitution, deletion, or addition of 1 to several amino acid(s) from an amino acid sequence contained in the antibody or the antigen-binding fragment of the antibody according to any one of (8) to (12) and 18 to 20, and binds to human GPRC5D;
(24) An antibody or an antigen-binding fragment of the antibody, wherein the antibody or the antigen-binding fragment binds to a site on human GPRC5D bound by the antibody or the antigen-binding fragment of the antibody according to any one of (1) to (20);
(25) An antibody or an antigen-binding fragment of the antibody, wherein the antibody or the antigen-binding fragment competes with the antibody or the antigen-binding fragment of the antibody according to any one of (1) to (20) for that binds to human GPRC5D.
(26) The antibody or the antigen-binding fragment according to any one of (1) to (25), wherein the antibody or the antigen-binding fragment binds to cynomolgus monkey GPRC5D;
(27) The antibody or the antigen-binding fragment of the antibody according to any one of (1) to (26), wherein the antigen-binding fragment is Fab, F(ab)', Fv, scFv, or sdAb;
(28) An antibody or an antigen-binding fragment of the antibody, wherein the antibody binds to human GPRC5D, comprising the heavy chain variable region and the light chain variable region according to (2), (8) or (9), and

i) a heavy chain constant region comprising an amino acid sequence represented by amino acid residues 147 to 475 of an amino acid sequence represented by SEQ ID NO: 199, and a light chain constant region comprising an amino acid sequence represented by amino acid residues 131 to 237 of the amino acid sequence represented by SEQ ID NO: 203,
ii) a heavy chain constant region comprising an amino acid sequence represented by amino acid residues 147 to 475 of an amino acid sequence represented by SEQ ID NO: 201, and a light chain constant region comprising an amino acid sequence represented by amino acid residues 131 to 237 of an amino acid sequence represented by SEQ ID NO: 205,
iii) a heavy chain constant region comprising an amino acid sequence represented by amino acid residues 147 to 475 of an amino acid sequence represented by SEQ ID NO: 215, and a light chain constant region comprising an amino acid sequence represented by amino acid residues 131 to 237 of an amino acid sequence represented by SEQ ID NO: 217,
or
iv) a heavy chain constant region comprising an amino acid sequence represented by amino acid residues 147 to 476 of an amino acid sequence represented by SEQ ID NO: 237, and a light chain constant region comprising an amino acid sequence represented by amino acid residues 131 to 237 of an amino acid sequence represented by SEQ ID NO: 217;

(29) An antibody or an antigen-binding fragment of the antibody, wherein the antibody binds to human GPRC5D, comprising a heavy chain variable region comprising an amino acid sequence represented by amino acid residues 20 to 142 of the amino acid sequence represented by SEQ ID NO: 76 and a light chain variable region comprising an amino acid sequence represented by amino acid residues 21 to 127 of the amino acid sequence represented by SEQ ID NO: 72, and

i) a heavy chain constant region comprising an amino acid sequence represented by amino acid residues 147 to 475 of the amino acid sequence represented by SEQ ID NO: 199, and a light chain constant region comprising an amino acid sequence represented by amino acid residues 131 to 237 of the amino acid sequence represented by SEQ ID NO: 203,
ii) a heavy chain constant region comprising an amino acid sequence represented by amino acid residues 147 to 475 of the amino acid sequence represented by SEQ ID NO: 201, and a light chain constant region comprising an amino acid sequence represented by amino acid residues 131 to 237 of the amino acid sequence represented by SEQ ID NO: 205,
iii) a heavy chain constant region comprising an amino acid sequence represented by amino acid residues 147 to 475 of the amino acid sequence represented by SEQ ID NO: 215, and a light chain constant region comprising an amino acid sequence represented by amino acid residues 131 to 237 of the amino acid sequence represented by SEQ ID NO: 217,
or
iv) a heavy chain constant region comprising an amino acid sequence represented by amino acid residues 147 to 475 of the amino acid sequence represented by SEQ ID NO: 237, and a light chain constant region comprising an amino acid sequence represented by amino acid residues 131 to 237 of the amino acid sequence represented by SEQ ID NO: 217;

(30) An antibody or an antigen-binding fragment of the antibody, wherein the antibody binds to human GPRC5D, comprising the heavy chain variable region and the light chain variable region according to (2), (8) or (9), and an Fc mutation;
(31) An antibody or an antigen-binding fragment of the antibody, wherein the antibody binds to human GPRC5D, comprising a heavy chain variable region comprising an amino acid sequence represented by amino acid residues 20 to 142 of the amino acid sequence represented by SEQ ID NO: 76 and a light chain variable region comprising an amino acid sequence represented by amino acid residues 21 to 127 of the amino acid sequence represented by SEQ ID NO: 72, and an Fc mutation;
(32) A polynucleotide encoding the antibody or the antigen-binding fragment of the antibody according to any one of (1) to (31);
(33) A vector comprising any one of the polynucleotides according to (32);
(34) A cell comprising any one of the polynucleotides according to (32) or the vector according to (33), or for producing the antibody or the antigen-binding fragment of the antibody according to any one of (1) to (31);
(35) An artificial immunocyte comprising any one of the polynucleotides according to (32) or the vector according to (33), or for expressing the antibody or the antigen-binding fragment of the antibody according to any one of (1) to (31) on the cell surface;
(36) A method for producing an antibody or an antigen-binding fragment of the antibody which binds to human GPRC5D, comprising steps of: culturing the cell according to (34); and recovering an antibody or an antigen-binding fragment of the antibody which binds to human GPRC5D from the cultures;
(37) An antibody or an antigen-binding fragment of the antibody which binds to human GPRC5D, the antibody or the antigen-binding fragment being obtained by the method according to (36);

(38) A pharmaceutical composition for treatment and/or prevention comprising the antibody or the antigen-binding fragment of the antibody according to any one of (1) to (31) and (37), the polynucleotide according to (32), the vector according to (33), or the artificial immunocyte according to (35) as an active ingredient;
(39) The pharmaceutical composition according to (38), wherein the pharmaceutical composition is for the treatment and/or prevention of a cancer;
(40) The pharmaceutical composition according to (39), wherein the cancer is breast cancer, endometrial cancer, ovary cancer, lung cancer, stomach cancer, prostate cancer, kidney cancer, liver cancer, pancreatic cancer, colorectal cancer, esophageal cancer, urinary bladder cancer, uterine cervix cancer, blood cancer, lymphoma, or malignant melanoma expressing a GPRC5D protein;
(41) The pharmaceutical composition according to (40), wherein the cancer is multiple myeloma expressing a GPRC5D protein;
(42) A molecule having antigen binding activity, comprising the antibody or the antigen-binding fragment of the antibody according to any one of (1) to (31) and (37);
(43) The molecule according to (42), wherein the molecule is multispecific;
(44) The molecule according to (42) or (43), wherein the molecule comprises the antibody that binds to human GPRC5D or the antigen-binding fragment of the antibody according to any one of (1) to (31) and (37), and
an antibody or an antigen-binding fragment of the antibody comprising
a heavy chain variable region comprising
heavy chain CDR1 comprising an amino acid sequence represented by SEQ ID NO: 183,
heavy chain CDR2 comprising an amino acid sequence represented by SEQ ID NO: 238, and
heavy chain CDR3 comprising an amino acid sequence represented by SEQ ID NO: 185, and
a light chain variable region comprising
light chain CDR1 comprising an amino acid sequence represented by SEQ ID NO: 186,
light chain CDR2 comprising an amino acid sequence represented by SEQ ID NO: 239, and
light chain CDR3 comprising an amino acid sequence represented by SEQ ID NO: 188, and binds to human CD3 and cynomolgus monkey CD3;
(45) The molecule according to (44), wherein, in the heavy chain CDR2, a first $X_{aa}$ is selected from a group consisting of (A, E, G, H, I, L, T, V, R, and S), and
a second $X_{aa}$ is S; or
the first $X_{aa}$ is N, and
the second $X_{aa}$ is selected from a group consisting of (E, R, F, Y, L, V, I, K, and T), and
in the light chain CDR2, $X_{aa}$ is selected from a group consisting of (Q, A, G, S, N, and D), and
the molecule binds to human CD3 and cynomolgus monkey CD3;
(46) The molecule according to (44) or (45), wherein, in the heavy chain CDR2, the first $X_{aa}$ is selected from a group consisting of (R and S), and the second $X_{aa}$ is S, and
in the light chain CDR2, $X_{aa}$ is selected from a group consisting of (Q, A, G, S, N, and D), and
the molecule binds to human CD3 and cynomolgus monkey CD3;
(47) The molecule according to any one of (42) to (45), comprising a heavy chain variable region comprising an amino acid sequence represented by SEQ ID NO: 240, and
a light chain variable region comprising an amino acid sequence represented by any one of SEQ ID NOs: 241, 242, and 243; wherein
in the amino acid sequence represented by SEQ ID NO: 240, the first $X_{aa}$ is selected from a group consisting of (A, E, G, H, I, L, T, V, R, and S), and the second $X_{aa}$ is S; or
the first $X_{aa}$ is N, and
the second $X_{aa}$ is selected from a group consisting of (E, R, F, Y, L, V, I, K, and T), and
in the amino acid sequence represented by any one of SEQ ID NOs: 241, 242, and 243,
$X_{aa}$ is selected from a group consisting of (Q, A, G, S, N, and D);
(48) The molecule according to (47), wherein
in SEQ ID NO: 240, the first $X_{aa}$ is selected from the group consisting of (R and S), and
the second $X_{aa}$ is S, and
in the amino acid sequence represented by any one of SEQ ID NOs: 241, 242, and 243,
$X_{aa}$ is selected from a group consisting of (Q, A, G, S, N, and D);
(49) The molecule according to any one of (42) to (44), wherein the molecule comprises the antibody that binds to human GPRC5D or the antigen-binding fragment of the antibody according to any one of (1) to (31) and (37), and
an antibody or an antigen-binding fragment of the antibody that comprises
a heavy chain variable region comprising
the amino acid sequence of heavy chain CDR1 represented by SEQ ID NO: 183,
the amino acid sequence of heavy chain CDR2 represented by SEQ ID NO: 184, and

the amino acid sequence of heavy chain CDR3 represented by SEQ ID NO: 185, and
a light chain variable region comprising
the amino acid sequence of light chain CDR1 represented by SEQ ID NO: 186,
the amino acid sequence of light chain CDR2 represented by SEQ ID NO: 187, and
the amino acid sequence of light chain CDR3 represented by SEQ ID NO: 188, and binds to human CD3 and cynomolgus monkey CD3;
(50) The molecule according to (49), wherein the antibody that binds to human CD3 and cynomolgus monkey CD3 or the antigen-binding fragment of the antibody is an antibody or an antigen-binding fragment of the antibody comprising a heavy chain variable region comprising the amino acid sequence represented by SEQ ID NO: 155 and a light chain variable region comprising an amino acid sequence represented by any one of SEQ ID NOs: 156, 158, and 160;
(51) The molecule according to any one of (44) to (50), wherein the antigen-binding fragment of the antibody that binds to human CD3 and cynomolgus monkey CD3 is Fab, F(ab)', Fv, scFv, or sdAb;
(52) The molecule according to any one of (44) to (51), wherein the antibody that binds to human CD3 and cynomolgus monkey CD3 is a humanized antibody or a human antibody comprising a human immunoglobulin constant region, or Fc or an Fc mutation;
(53) The molecule according to any one of (44) to (52), wherein the antibody that binds to human CD3 and cynomolgus monkey CD3 or the antigen-binding fragment of the antibody is an antibody or an antigen-binding fragment of the antibody comprising an amino acid sequence represented by any one of SEQ ID NOs: 180, 181, and 182;
(54) The molecule according to any one of (40) to (44), wherein the antibody that binds to human CD3 and cynomolgus monkey CD3 or the antigen-binding fragment of the antibody is bound with the antibody or the antigen-binding fragment of the antibody according to any one of (1) to (31) and (37) via a linker or without a linker;
(55) The molecule according to any one of (42) to (44), wherein the molecule comprises the antibody that binds to human GPRC5D or the antigen-binding fragment of the antibody according to (2), (8) or (9), and an antibody that binds to human CD3 and cynomolgus monkey CD3 or an antigen-binding fragment of the antibody comprising

- a heavy chain variable region comprising an amino acid sequence represented by amino acid residues 25 to 142 of the amino acid sequence represented by SEQ ID NO: 207 and a light chain variable region comprising an amino acid sequence represented by amino acid residues 24 to 132 of the amino acid sequence represented by SEQ ID NO: 209,
- a heavy chain variable region comprising an amino acid sequence represented by amino acid residues 25 to 142 of the amino acid sequence represented by SEQ ID NO: 211 and a light chain variable region comprising an amino acid sequence represented by amino acid residues 24 to 130 of the amino acid sequence represented by SEQ ID NO: 213,
- a heavy chain variable region comprising an amino acid sequence represented by amino acid residues 2 to 119 of SEQ ID NO: 244 and a light chain variable region comprising an amino acid sequence represented by amino acid residues 135 to 243 of SEQ ID NO: 244,
- a heavy chain variable region comprising an amino acid sequence represented by amino acid residues 2 to 119 of SEQ ID NO: 245 and a light chain variable region comprising an amino acid sequence represented by amino acid residues 135 to 241 of SEQ ID NO: 245,
- a heavy chain variable region comprising an amino acid sequence represented by amino acid residues 2 to 119 of SEQ ID NO: 246 and a light chain variable region comprising an amino acid sequence represented by amino acid residues 135 to 243 of SEQ ID NO: 246,
- a heavy chain variable region comprising an amino acid sequence represented by amino acid residues 2 to 119 of SEQ ID NO: 247 and a light chain variable region comprising an amino acid sequence represented by amino acid residues 135 to 243 of SEQ ID NO: 247,
- a heavy chain variable region comprising an amino acid sequence represented by amino acid residues 2 to 119 of SEQ ID NO: 248 and a light chain variable region comprising an amino acid sequence represented by amino acid residues 135 to 243 of SEQ ID NO: 248,
- a heavy chain variable region comprising an amino acid sequence of amino acid residues 2 to 119 of SEQ ID NO: 249 and a light chain variable region comprising an amino acid sequence represented by amino acid residues 135 to 243 of SEQ ID NO: 249,
- a heavy chain variable region comprising an amino acid sequence represented by amino acid residues 2 to 119 of SEQ ID NO: 250 and a light chain variable region comprising an amino acid sequence represented by amino acid residues 135 to 243 of SEQ ID NO: 250,
- a heavy chain variable region comprising an amino acid sequence represented by amino acid residues 2 to 119 of SEQ ID NO: 251 and a light chain variable region comprising an amino acid sequence represented by amino acid residues 135 to 243 of SEQ ID NO: 251,

- a heavy chain variable region comprising an amino acid sequence represented by amino acid residues 2 to 119 of SEQ ID NO: 252 and a light chain variable region comprising an amino acid sequence represented by amino acid residues 135 to 243 of SEQ ID NO: 252,
- a heavy chain variable region comprising an amino acid sequence represented by amino acid residues 2 to 119 of SEQ ID NO: 253 and a light chain variable region comprising an amino acid sequence represented by amino acid residues 135 to 242 of SEQ ID NO: 253,
- a heavy chain variable region comprising an amino acid sequence represented by amino acid residues 2 to 119 of SEQ ID NO: 254 and a light chain variable region comprising an amino acid sequence represented by amino acid residues 135 to 243 of SEQ ID NO: 254, or
- a heavy chain variable region comprising an amino acid sequence represented by amino acid residues 2 to 119 of SEQ ID NO: 255 and a light chain variable region comprising an amino acid sequence represented by amino acid residues 135 to 243 of SEQ ID NO: 255; and includes an antibody that binds to human CD3 and cynomolgus monkey CD3 or an antigen-binding fragment of the antibody;

(56) The molecule according to (55), wherein the antibody that binds to human GPRC5D or the antigen-binding fragment of the antibody comprises a heavy chain variable region comprising an amino acid sequence represented by amino acid residues 20 to 142 of the amino acid sequence represented by SEQ ID NO: 76 and a light chain variable region comprising an amino acid sequence represented by amino acid residues 21 to 127 of the amino acid sequence represented by SEQ ID NO: 72, and

i) a heavy chain constant region comprising an amino acid sequence represented by amino acid residues 147 to 475 of the amino acid sequence represented by SEQ ID NO: 199, and a light chain constant region comprising an amino acid sequence represented by amino acid residues 131 to 237 of the amino acid sequence represented by SEQ ID NO: 203,
ii) a heavy chain constant region comprising an amino acid sequence represented by amino acid residues 147 to 475 of the amino acid sequence represented by SEQ ID NO: 201, and a light chain constant region comprising an amino acid sequence represented by amino acid residues 131 to 237 of the amino acid sequence represented by SEQ ID NO: 205,
iii) a heavy chain constant region comprising an amino acid sequence represented by amino acid residues 147 to 475 of the amino acid sequence represented by SEQ ID NO: 215, and a light chain constant region comprising an amino acid sequence represented by amino acid residues 131 to 237 of the amino acid sequence represented by SEQ ID NO: 217,
or
iv) a heavy chain constant region comprising an amino acid sequence represented by amino acid residues 147 to 476 of the amino acid sequence represented by SEQ ID NO: 237, and a light chain constant region comprising an amino acid sequence represented by amino acid residues 131 to 237 of the amino acid sequence represented by SEQ ID NO: 217,
and
the antibody that binds to human CD3 and cynomolgus monkey CD3 or the antigen-binding fragment of the antibody further comprises an Fc mutation. The applicable form contains a hybrid-type bispecific molecule;

(57) The molecule according to (55), wherein the antibody that binds to human GPRC5D or the antigen-binding fragment of the antibody comprises a heavy chain variable region comprising an amino acid sequence represented by amino acid residues 20 to 142 of the amino acid sequence represented by SEQ ID NO: 76 and a light chain variable region comprising an amino acid sequence represented by amino acid residues 21 to 127 of the amino acid sequence represented by SEQ ID NO: 72, and

iii) a heavy chain constant region comprising an amino acid sequence represented by amino acid residues 147 to 475 of the amino acid sequence represented by SEQ ID NO: 215, and a light chain constant region comprising an amino acid sequence represented by amino acid residues 131 to 237 of the amino acid sequence represented by SEQ ID NO: 217,
or
iv) a heavy chain constant region comprising an amino acid sequence represented by amino acid residues 147 to 475 of the amino acid sequence represented by SEQ ID NO: 237, and a light chain constant region comprising an amino acid sequence represented by amino acid residues 131 to 237 of the amino acid sequence represented by SEQ ID NO: 217,
and
the antibody that binds to human CD3 and cynomolgus monkey CD3 or the antigen-binding fragment of the antibody further comprises an Fc mutation. The applicable form contains a hybrid-type bispecific molecule;

(58) The molecule according to (55), comprising the antibody that binds to human GPRC5D or the antigen-binding fragment of the antibody, comprising a heavy chain comprising an amino acid sequence represented by amino acid residues 24 to 475 of the amino acid sequence represented by SEQ ID NO: 215 and a light chain comprising an amino acid sequence represented by amino acid residues 24 to 237 of the amino acid sequence represented by SEQ ID NO: 217; and an antibody that binds to human CD3 and cynomolgus monkey CD3 or the antigen-binding fragment of the antibody, comprising an amino acid sequence represented by amino acid residues 24 to 266 of the amino acid sequence represented by SEQ ID NO: 219 and an Fc mutation;
the antibody that binds to human GPRC5D or the antigen-binding fragment of the antibody, comprising a heavy chain comprising an amino acid sequence represented by amino acid residues 24 to 475 of the amino acid sequence represented by SEQ ID NO: 215 and a light chain comprising an amino acid sequence represented by amino acid residues 24 to 237 of the amino acid sequence represented by SEQ ID NO: 217; and the antibody that binds to human CD3 and cynomolgus monkey CD3 or the antigen-binding fragment of the antibody, comprising an amino acid sequence represented by amino acid residues 24 to 264 of the amino acid sequence represented by SEQ ID NO: 221 and an Fc mutation;
the antibody that binds to human GPRC5D or the antigen-binding fragment of the antibody, comprising a heavy chain comprising an amino acid sequence represented by amino acid residues 24 to 475 of the amino acid sequence represented by SEQ ID NO: 215 and a light chain comprising an amino acid sequence represented by amino acid residues 24 to 237 of the amino acid sequence represented by SEQ ID NO: 217; and the antibody that binds to human CD3 and cynomolgus monkey CD3 or the antigen-binding fragment of the antibody, comprising an amino acid sequence represented by amino acid residues 24 to 264 of the amino acid sequence represented by SEQ ID NO: 225 and an Fc mutation;
the antibody that binds to human GPRC5D or the antigen-binding fragment of the antibody, comprising a heavy chain comprising an amino acid sequence represented by amino acid residues 24 to 475 of the amino acid sequence represented by SEQ ID NO: 215 and a light chain comprising an amino acid sequence represented by amino acid residues 24 to 237 of the amino acid sequence represented by SEQ ID NO: 217; and the antibody that binds to human CD3 and cynomolgus monkey CD3 or the antigen-binding fragment of the antibody, comprising an amino acid sequence represented by amino acid residues 24 to 266 of the amino acid sequence represented by SEQ ID NO: 227 and an Fc mutation;
the antibody that binds to human GPRC5D or the antigen-binding fragment of the antibody, comprising a heavy chain comprising an amino acid sequence represented by amino acid residues 24 to 475 of the amino acid sequence represented by SEQ ID NO: 215 and a light chain comprising an amino acid sequence represented by amino acid residues 24 to 237 of the amino acid sequence represented by SEQ ID NO: 217; and the antibody that binds to human CD3 and cynomolgus monkey CD3 or the antigen-binding fragment of the antibody, comprising an amino acid sequence represented by amino acid residues 24 to 266 of the amino acid sequence represented by SEQ ID NO: 229 and an Fc mutation;
the antibody that binds to human GPRC5D or the antigen-binding fragment of the antibody, comprising a heavy chain comprising an amino acid sequence represented by amino acid residues 24 to 476 of the amino acid sequence represented by SEQ ID NO: 237 and a light chain comprising an amino acid sequence represented by amino acid residues 24 to 237 of the amino acid sequence represented by SEQ ID NO: 217; and the antibody that binds to human CD3 and cynomolgus monkey CD3 or the antigen-binding fragment of the antibody, comprising an amino acid sequence represented by amino acid residues 24 to 264 of the amino acid sequence represented by SEQ ID NO: 231 and an Fc mutation;
the antibody that binds to human GPRC5D or the antigen-binding fragment of the antibody, comprising a heavy chain comprising an amino acid sequence represented by amino acid residues 24 to 476 of the amino acid sequence represented by SEQ ID NO: 237 and a light chain comprising an amino acid sequence represented by amino acid residues 24 to 237 of the amino acid sequence represented by SEQ ID NO: 217; and the antibody that binds to human CD3 and cynomolgus monkey CD3 or the antigen-binding fragment of the antibody, comprising an amino acid sequence represented by amino acid residues 24 to 266 of the amino acid sequence represented by SEQ ID NO: 233 and an Fc mutation; or
the antibody that binds to human GPRC5D or the antigen-binding fragment of the antibody comprising a heavy chain comprising an amino acid sequence represented by amino acid residues 24 to 476 of the amino acid sequence represented by SEQ ID NO: 237 and a light chain comprising an amino acid sequence represented by amino acid residues 24 to 237 of the amino acid sequence represented by SEQ ID NO: 217; and the antibody that binds to human CD3 and cynomolgus monkey CD3 or the antigen-binding fragment of the antibody, comprising an amino acid sequence represented by amino acid residues 24 to 266 of the amino acid sequence represented by SEQ ID NO: 235 and an Fc mutation;

(59) The molecule according to (55), comprising the antibody that binds to human GPRC5D or an antigen-binding fragment of the antibody, comprising a heavy chain comprising an amino acid sequence represented by amino acid residues 24 to 475 of the amino acid sequence represented by SEQ ID NO: 215 and a light chain comprising an amino acid sequence represented by amino acid residues 24 to 237 of the amino acid sequence represented by SEQ ID NO: 217; and the antibody that binds to human CD3 and cynomolgus monkey CD3 or the antigen-binding fragment of the antibody, comprising an amino acid sequence represented by amino acid residues 24 to 264 of the amino acid sequence represented by SEQ ID NO: 225 and an Fc mutation;
the antibody that binds to human GPRC5D or the antigen-binding fragment of the antibody, comprising a heavy chain comprising an amino acid sequence represented by amino acid residues 24 to 475 of the amino acid sequence represented by SEQ ID NO: 215 and a light chain comprising an amino acid sequence represented by amino acid residues 24 to 237 of the amino acid sequence represented by SEQ ID NO: 217; and the antibody that binds to human CD3 and cynomolgus monkey CD3 or the antigen-binding fragment of the antibody, comprising an amino acid sequence represented by amino acid residues 24 to 266 of the amino acid sequence represented by SEQ ID NO: 227 and an Fc mutation;
the antibody that binds to human GPRC5D or the antigen-binding fragment of the antibody, comprising a heavy chain comprising an amino acid sequence represented by amino acid residues 24 to 475 of the amino acid sequence represented by SEQ ID NO: 215 and a light chain comprising an amino acid sequence represented by amino acid residues 24 to 237 of the amino acid sequence represented by SEQ ID NO: 217; and the antibody that binds to human CD3 and cynomolgus monkey CD3 or the antigen-binding fragment of the antibody, comprising an amino acid sequence represented by amino acid residues 24 to 266 of the amino acid sequence represented by SEQ ID NO: 229 and an Fc mutation;
the antibody that binds to human GPRC5D or the antigen-binding fragment of the antibody, comprising a heavy chain comprising an amino acid sequence represented by amino acid residues 24 to 476 of the amino acid sequence represented by SEQ ID NO: 237 and a light chain comprising an amino acid sequence represented by amino acid residues 24 to 237 of the amino acid sequence represented by SEQ ID NO: 217; and the antibody that binds to human CD3 and cynomolgus monkey CD3 or the antigen-binding fragment of the antibody, comprising an amino acid sequence represented by amino acid residues 24 to 264 of the amino acid sequence represented by SEQ ID NO: 231 and an Fc mutation;
the antibody that binds to human GPRC5D or the antigen-binding fragment of the antibody, comprising a heavy chain comprising an amino acid sequence represented by amino acid residues 24 to 476 of the amino acid sequence represented by SEQ ID NO: 237 and a light chain comprising an amino acid sequence represented by amino acid residues 24 to 237 of the amino acid sequence represented by SEQ ID NO: 217; and the antibody that binds to human CD3 and cynomolgus monkey CD3 or the antigen-binding fragment of the antibody, comprising an amino acid sequence represented by amino acid residues 24 to 266 of the amino acid sequence represented by SEQ ID NO: 233 and an Fc mutation; or
the antibody that binds to human GPRC5D or the antigen-binding fragment of the antibody, comprising a heavy chain comprising an amino acid sequence represented by amino acid residues 24 to 476 of the amino acid sequence represented by SEQ ID NO: 237 and a light chain comprising an amino acid sequence represented by amino acid residues 24 to 237 of the amino acid sequence represented by SEQ ID NO: 217; and the antibody that binds to human CD3 and cynomolgus monkey CD3 or the antigen-binding fragment of the antibody, comprising an amino acid sequence represented by amino acid residues 24 to 266 of the amino acid sequence represented by SEQ ID NO: 235 and an Fc mutation. The applicable form contains a hybrid-type bispecific molecule;
(60) The molecule according to (55), wherein the antibody that binds to human GPRC5D or the antigen-binding fragment of the antibody comprises a heavy chain variable region comprising an amino acid sequence represented by amino acid residues 20 to 142 of the amino acid sequence represented by SEQ ID NO: 76 and a light chain variable region comprising an amino acid sequence represented by amino acid residues 21 to 127 of the amino acid sequence represented by SEQ ID NO: 72, and

i) a heavy chain constant region comprising an amino acid sequence represented by amino acid residues 147 to 475 of the amino acid sequence represented by SEQ ID NO: 199, and a light chain constant region comprising an amino acid sequence represented by amino acid residues 131 to 237 of the amino acid sequence represented by SEQ ID NO: 203,
ii) a heavy chain constant region comprising an amino acid sequence represented by amino acid residues 147 to 475 of the amino acid sequence represented by SEQ ID NO: 201, and a light chain constant region comprising an amino acid sequence represented by amino acid residues 131 to 237 of the amino acid sequence represented by SEQ ID NO: 205,
iii) a heavy chain constant region comprising an amino acid sequence represented by amino acid residues 147 to 475 of the amino acid sequence represented by SEQ ID NO: 215, and a light chain constant region comprising an amino acid sequence represented by amino acid residues 131 to 237 of the amino acid sequence represented by SEQ ID NO: 217,

or

iv) a heavy chain constant region comprising an amino acid sequence represented by amino acid residues 147 to 476 of the amino acid sequence represented by SEQ ID NO: 237, and a light chain constant region comprising an amino acid sequence represented by amino acid residues 131 to 237 of the amino acid sequence represented by SEQ ID NO: 217, and the antibody that binds to human CD3 and cynomolgus monkey CD3 or the antigen-binding fragment of the antibody further comprises

v) a heavy chain constant region comprising an amino acid sequence represented by amino acid residues 143 to 471 of the amino acid sequence represented by SEQ ID NO: 207, and a light chain constant region comprising an amino acid sequence represented by amino acid residues 133 to 238 of the amino acid sequence represented by SEQ ID NO: 209, or

vi) a heavy chain constant region comprising an amino acid sequence represented by amino acid residues 143 to 471 of the amino acid sequence represented by SEQ ID NO: 211, and a light chain constant region comprising an amino acid sequence represented by amino acid residues 131 to 236 of the amino acid sequence represented by SEQ ID NO: 213. The applicable form contains an FSA-type bispecific molecule;

(61) The molecule according to claim 55, wherein the antibody that binds to human GPRC5D or the antigen-binding fragment of the antibody comprises a heavy chain variable region comprising an amino acid sequence represented by amino acid residues 20 to 142 of the amino acid sequence represented by SEQ ID NO: 76 and a light chain variable region comprising an amino acid sequence represented by amino acid residues 21 to 127 of the amino acid sequence represented by SEQ ID NO: 72, and

iii) a heavy chain constant region comprising an amino acid sequence represented by amino acid residues 147 to 475 of the amino acid sequence represented by SEQ ID NO: 215, and a light chain constant region comprising an amino acid sequence represented by amino acid residues 131 to 237 of the amino acid sequence represented by SEQ ID NO: 217,

or

iv) a heavy chain constant region comprising an amino acid sequence represented by amino acid residues 147 to 476 of the amino acid sequence represented by SEQ ID NO: 237, and a light chain constant region comprising an amino acid sequence represented by amino acid residues 131 to 237 of the amino acid sequence represented by SEQ ID NO: 217, and the antibody that binds to human CD3 and cynomolgus monkey CD3 or the antigen-binding fragment of the antibody further comprises

v) a heavy chain constant region comprising an amino acid sequence represented by amino acid residues 143 to 471 of the amino acid sequence represented by SEQ ID NO: 207, and a light chain constant region comprising an amino acid sequence represented by amino acid residues 133 to 238 of the amino acid sequence represented by SEQ ID NO: 209, or

vi) a heavy chain constant region comprising an amino acid sequence represented by amino acid residues 143 to 471 of the amino acid sequence represented by SEQ ID NO: 211, and a light chain constant region comprising an amino acid sequence represented by amino acid residues 131 to 236 of the amino acid sequence represented by SEQ ID NO: 213. The applicable form contains an FSA-type bispecific molecule;

(62) The molecule according to (55), comprising

the antibody that binds to human GPRC5D or the antigen-binding fragment of the antibody, comprising a heavy chain comprising an amino acid sequence represented by amino acid residues 24 to 475 of the amino acid sequence represented by SEQ ID NO: 199 and a light chain comprising an amino acid sequence represented by amino acid residues 24 to 237 of the amino acid sequence represented by SEQ ID NO: 203; and the antibody that binds to human CD3 and cynomolgus monkey CD3 or the antigen-binding fragment of the antibody, comprising a heavy chain comprising an amino acid sequence represented by amino acid residues 25 to 471 of the amino acid sequence represented by SEQ ID NO: 207 and a light chain comprising an amino acid sequence represented by amino acid residues 24 to 238 of the amino acid sequence represented by SEQ ID NO: 209, or

the antibody that binds to human GPRC5D or the antigen-binding fragment of the antibody, comprising a heavy chain comprising an amino acid sequence represented by amino acid residues 24 to 475 of the amino acid sequence represented by SEQ ID NO: 201 and a light chain comprising an amino acid sequence represented by amino acid residues 24 to 237 of the amino acid sequence represented by SEQ ID NO: 205; and the antibody that binds to human CD3 and cynomolgus monkey CD3 or the antigen-binding fragment of the antibody, comprising a heavy chain comprising an amino acid sequence represented by amino acid residues 25 to 471 of the amino acid sequence represented by SEQ ID NO: 211 and a light chain comprising an amino acid sequence represented by amino acid residues 24 to 236 of the amino acid sequence represented by SEQ ID NO: 213. The applicable form contains an FSA-type bispecific molecule;

(63) The molecule according to (55), wherein the antibody that binds to human GPRC5D or the antigen-binding fragment of the antibody comprises a heavy chain variable region comprising an amino acid sequence represented by amino acid residues 20 to 142 of the amino acid sequence represented by SEQ ID NO: 76 and a light chain variable region comprising an amino acid sequence represented by amino acid residues 21 to 127 of the amino acid sequence represented by SEQ ID NO: 72, and further comprises an Fc mutation, and the antibody that binds to human CD3 and cynomolgus monkey CD3 or the antigen-binding fragment of the antibody, further comprises an Fc mutation. The applicable form contains a dual-type bispecific molecule;

(64) The molecule according to (55), comprising

the antibody that binds to human GPRC5D or the antigen-binding fragment of the antibody, comprising an amino acid sequence represented by amino acid residues 24 to 271 of the amino acid sequence represented by SEQ ID NO: 223 and an Fc mutation; and the antibody that binds to human CD3 and cynomolgus monkey CD3 or the antigen-binding fragment of the antibody, comprising an amino acid sequence represented by amino acid residues 24 to 266 of the amino acid sequence represented by SEQ ID NO: 219, and an Fc mutation, or

the antibody that binds to human GPRC5D or the antigen-binding fragment of the antibody, comprising an amino acid sequence represented by amino acid residues 24 to 271 of the amino acid sequence represented by SEQ ID NO: 223, and an Fc mutation; and the antibody that binds to human CD3 and cynomolgus monkey CD3 or the antigen-binding fragment of the antibody, comprising an amino acid sequence represented by amino acid residues 24 to 264 of the amino acid sequence represented by SEQ ID NO: 221, and an Fc mutation. The applicable form contains a dual-type bispecific molecule;

(65) The molecule according to (53), wherein the antibody that binds to human CD3 and cynomolgus monkey CD3 or the antigen-binding fragment of the antibody is bound with the antibody or the antigen-binding fragment of the antibody according to (19) via a linker or without a linker;

(66) The molecule according to (54) or (65) that binds to human CD3 and cynomolgus monkey CD3, and also to human GPRC5D, wherein the molecule has an amino acid sequence represented by any one of SEQ ID NOs: 171 to 179;

(67) A molecule comprising an amino acid sequence encoded by a nucleotide sequence contained in a polynucleotide hybridizing under stringent conditions to a complementary strand of a polynucleotide comprising a nucleotide sequence encoding an amino acid sequence contained in the antibody that binds to human CD3 and cynomolgus monkey CD3 or the antigen-binding fragment of the antibody contained in the molecule according to any one of (50), (53), (58), (59), (62), (64), (65) and (66), and that binds to human CD3 and cynomolgus monkey CD3, and also to human GPRC5D;

(68) A molecule comprising an amino acid sequence 90% or more identical to the amino acid sequence contained in the antibody that binds to human CD3 and cynomolgus monkey CD3 or the antigen-binding fragment of the antibody according to any one of (50), (53), (58), (59), (62), (64), (65) and (66), and that binds to human CD3 and cynomolgus monkey CD3, and also to human GPRC5D;

(69) A molecule comprising an amino acid sequence derived by the substitution, deletion, or addition of 1 to several amino acid(s) from an amino acid sequence contained in the antibody that binds to human CD3 and cynomolgus monkey CD3 or the antigen-binding fragment of the antibody contained in the molecule according to any one of (50), (53), (58), (59), (62), (64), (65) and (66), and that binds to human CD3 and cynomolgus monkey CD3, and also to human GPRC5D;

(70) The molecule according to any one of (42) to (69), wherein the molecule binds to cynomolgus monkey GPRC5D;

(71) The molecule according to any one of (43) to (70), wherein the molecule is bispecific;

(72) The molecule according to any one of (42) to (71), wherein the molecule is a polypeptide;

(73) A polynucleotide comprising a nucleotide sequence encoding the amino acid sequence of the molecule according to (72);

(74) A vector comprising the polynucleotide according to (73);

(75) A cell producing the polynucleotide according to (73) or the vector according to (74), or the molecule according to (71);

(76) A method for producing a molecule that binds to human CD3 and cynomolgus monkey CD3, and also to human GPRC5D, comprising steps of: culturing the cell according to (75); and recovering a molecule that binds to human CD3 and cynomolgus monkey CD3 and/or to human GPRC5D from the cultures;

(77) A molecule that binds to human CD3 and cynomolgus monkey CD3, and also to human GPRC5D, the molecule being obtained by the method according to (76);

(78) The molecule according to (77), wherein the molecule binds to cynomolgus monkey GPRC5D;

(79) A pharmaceutical composition for treatment and/or prevention comprising the molecule according to any one of (42) to (72), (77), and (78), the polynucleotide according to (73), or the vector according to (74) as an active ingredient;
(80) The pharmaceutical composition according to (79), wherein the pharmaceutical composition is for the treatment and/or prevention of a cancer;
(81) The pharmaceutical composition according to (80), wherein the cancer is breast cancer, endometrial cancer, ovary cancer, lung cancer, stomach cancer, prostate cancer, kidney cancer, liver cancer, pancreatic cancer, colorectal cancer, esophageal cancer, urinary bladder cancer, uterine cervix cancer, blood cancer, lymphoma, or malignant melanoma expressing a GPRC5D protein;
(82) The pharmaceutical composition according to (79) or (80), wherein the cancer is multiple myeloma expressing a GPRC5D protein;
(83) A method for treating and/or preventing a cancer, comprising administering the molecule according to any one of (42) to (72), (77), and (78) or the pharmaceutical composition according to any one of (79) to (82);
(84) The pharmaceutical composition according to any one of (79) to (82), wherein the pharmaceutical composition induces cytotoxicity to cells expressing GPRC5D by the redirection of T cells to the cells;
(85) The method according to (83), wherein the method induces cytotoxicity to cells expressing GPRC5D by the redirection of T cells to the cells;
(86) A method for inducing cytotoxicity to cells expressing GPRC5D by the redirection of T cells to the cells, comprising a step of administering the molecule according to any one of (42) to (72), (77), and (78) or the pharmaceutical composition according to any one of (79) to (82); and
(87) A method for redirecting T cells to cells expressing GPRC5D, comprising a step of administering the molecule according to any one of (42) to (72), (77), and (78) or the pharmaceutical composition according to any one of (79) to (82).

[Effects of Invention]

According to the present invention, a novel anti-GPRC5D antibody that binds to human GPRC5D or an antigen-binding fragment of the antibody and a novel molecule comprising the antibody or the antigen-binding fragment of the antibody and having antigen binding activity are obtained. The molecule can comprise an anti-CD3 antibody.
Use of the antibody or the antigen-binding fragment of the antibody, and the molecule provided by the present invention allows for treatment or prevention of various cancers, preferably, multiple myeloma, expressing a GPRC5D protein.

[Brief Description of Drawings]

[Figure 1] Figure 1 is a diagram showing results of testing the binding properties of rat anti-GPRC5D antibodies (2A4, 2B1, and 7B4) against human GPRC5D by flow cytometry. The vertical axis represents a relative value of the mean fluorescence intensity assayed by flow cytometry.
[Figure 2] Figure 2 is a diagram showing the amino-terminal amino acid sequence of human GPRC5D (SEQ ID NO: 1 of the Sequence Listing).
[Figure 3] Figure 3 is a diagram showing the amino-terminal amino acid sequence of human GPRC5D (SEQ ID NO: 2 of the Sequence Listing).
[Figure 4] Figure 4 is a diagram showing results of testing the binding properties of the rat anti-GPRC5D antibodies (2A4, 2B1, and 7B4) against human GPRC5D using a flow cytometer (FACS). The vertical axis represents a relative value of the mean fluorescence intensity assayed by flow cytometry. The intramolecular disulfide bond of a peptide was present (A) or absent (B).
[Figure 5] Figure 5 is a diagram showing that the rat anti-GPRC5D antibodies (2A4, 2B1, and 7B4) have ADCC activity.
[Figure 6] Figure 6 is a diagram showing the nucleotide sequence of a primer for the PCR amplification of the variable region cDNA of the heavy chain gene of 2A4 (SEQ ID NO: 3 of the Sequence Listing).
[Figure 7] Figure 7 is a diagram showing the nucleotide sequence of a primer for the PCR amplification of the variable region cDNA of the light chain gene of 2A4 (SEQ ID NO: 10 of the Sequence Listing).
[Figure 8] Figure 8 is a diagram showing the nucleotide sequence of a cDNA encoding the heavy chain variable region of 2A4 (SEQ ID NO: 4 of the Sequence Listing).
[Figure 9] Figure 9 is a diagram showing the amino acid sequence of the heavy chain variable region of 2A4 (SEQ ID NO: 5 of the Sequence Listing).
[Figure 10] Figure 10 is a diagram showing the nucleotide sequence of a cDNA encoding the heavy chain variable region of 2B 1 (SEQ ID NO: 6 of the Sequence Listing).
[Figure 11] Figure 11 is a diagram showing the amino acid sequence of the heavy chain variable region of 2B1 (SEQ ID NO: 7 of the Sequence Listing).

[Figure 12] Figure 12 is a diagram showing the nucleotide sequence of a cDNA encoding the heavy chain variable region of 7B4 (SEQ ID NO: 8 of the Sequence Listing).

[Figure 13] Figure 13 is a diagram showing the amino acid sequence of the heavy chain variable region of 7B4 (SEQ ID NO: 9 of the Sequence Listing).

[Figure 14] Figure 14 is a diagram showing the nucleotide sequence of a cDNA encoding the light chain variable region of 2A4 (SEQ ID NO: 11 of the Sequence Listing).

[Figure 15] Figure 15 is a diagram showing the amino acid sequence of the light chain variable region of 2A4 (SEQ ID NO: 12 of the Sequence Listing).

[Figure 16] Figure 16 is a diagram showing the nucleotide sequence of a cDNA encoding the light chain variable region of 2B1 (SEQ ID NO: 13 of the Sequence Listing).

[Figure 17] Figure 17 is a diagram showing the amino acid sequence of the light chain variable region of 2B1 (SEQ ID NO: 14 of the Sequence Listing).

[Figure 18] Figure 18 is a diagram showing the nucleotide sequence of a cDNA encoding the light chain variable region of 7B4 (SEQ ID NO: 15 of the Sequence Listing).

[Figure 19] Figure 19 is a diagram showing the amino acid sequence of the light chain variable region of 7B4 (SEQ ID NO: 16 of the Sequence Listing).

[Figure 20] Figure 20 is a diagram showing the nucleotide sequence of a DNA fragment comprising a DNA sequence encoding the amino acids of a human κ chain secretory signal sequence and a human κ chain constant region (SEQ ID NO: 17 of the Sequence Listing).

[Figure 21] Figure 21 is a diagram showing the nucleotide sequence of a primer F for a light chain expression vector (SEQ ID NO: 18 of the Sequence Listing).

[Figure 22] Figure 22 is a diagram showing the nucleotide sequence of a primer R for the light chain expression vector (SEQ ID NO: 19 of the Sequence Listing).

[Figure 23] Figure 23 is a diagram showing the nucleotide sequence of a DNA fragment comprising a DNA sequence encoding the amino acids of a human heavy chain signal sequence and a human IgG1 constant region (SEQ ID NO: 20 of the Sequence Listing).

[Figure 24] Figure 24 is a diagram showing the nucleotide sequence of the light chain of human chimeric 2A4 (c2A4) (SEQ ID NO: 21 of the Sequence Listing).

[Figure 25] Figure 25 is a diagram showing the amino acid sequence of the light chain of human chimeric 2A4 (c2A4) (signal sequence (1 to 20), variable region (21 to 127), and constant region (128 to 234)) (SEQ ID NO: 22 of the Sequence Listing).

[Figure 26] Figure 26 is a diagram showing the nucleotide sequence of a primer set F for the light chain of human chimeric 2A4 (c2A4) (SEQ ID NO: 23 of the Sequence Listing).

[Figure 27] Figure 27 is a diagram showing the nucleotide sequence of a primer set R for the light chain of human chimeric 2A4 (c2A4) (SEQ ID NO: 24 of the Sequence Listing).

[Figure 28] Figure 28 is a diagram showing the nucleotide sequence of the heavy chain of human chimeric 2A4 (c2A4) (SEQ ID NO: 25 of the Sequence Listing).

[Figure 29] Figure 29 is a diagram showing the amino acid sequence of the heavy chain of human chimeric 2A4 (c2A4) (signal sequence (1 to 19), variable region (20 to 141), and constant region (142 to 471)) (SEQ ID NO: 26 of the Sequence Listing).

[Figure 30] Figure 30 is a diagram showing the nucleotide sequence of a primer set F for the heavy chain of human chimeric 2A4 (c2A4) (SEQ ID NO: 27 of the Sequence Listing).

[Figure 31] Figure 31 is a diagram showing the nucleotide sequence of a primer set R for the heavy chain of human chimeric 2A4 (c2A4) (SEQ ID NO: 28 of the Sequence Listing).

[Figure 32] Figure 32 is a diagram showing the nucleotide sequence of the light chain of human chimeric 2B1 (c2B1) (SEQ ID NO: 29 of the Sequence Listing).

[Figure 33] Figure 33 is a diagram showing the amino acid sequence of the light chain of human chimeric 2B1 (c2B1) (signal sequence (1 to 20), variable region (21 to 127), and constant region (128 to 234)) (SEQ ID NO: 30 of the Sequence Listing).

[Figure 34] Figure 34 is a diagram showing the nucleotide sequence of a primer set F for the light chain of human chimeric 2B1 (c2B1) (SEQ ID NO: 31 of the Sequence Listing).

[Figure 35] Figure 35 is a diagram showing the nucleotide sequence of a primer set R for the light chain of human chimeric 2B1 (c2B1) (SEQ ID NO: 32 of the Sequence Listing).

[Figure 36] Figure 36 is a diagram showing the nucleotide sequence of the heavy chain of human chimeric 2B1 (c2B1) (SEQ ID NO: 33 of the Sequence Listing).

[Figure 37] Figure 37 is a diagram showing the amino acid sequence of the heavy chain of human chimeric 2B1 (c2B1) (signal sequence (1 to 19), variable region (20 to 142), and constant region (143 to 472)) (SEQ ID NO: 34 of the Sequence Listing).

[Figure 38] Figure 38 is a diagram showing the nucleotide sequence of a primer set F for the heavy chain of human chimeric 2B1 (c2B1) (SEQ ID NO: 35 of the Sequence Listing).

[Figure 39] Figure 39 is a diagram showing the nucleotide sequence of a primer set R for the heavy chain of human chimeric 2B1 (c2B1) (SEQ ID NO: 36 of the Sequence Listing).

[Figure 40] Figure 40 is a diagram showing the nucleotide sequence of the light chain of human chimeric 7B4 (c7B4) (SEQ ID NO: 37 of the Sequence Listing).

[Figure 41] Figure 41 is a diagram showing the amino acid sequence of the light chain of human chimeric 7B4 (c7B4) (signal sequence (1 to 20), variable region (21 to 126), and constant region (127 to 233)) (SEQ ID NO: 38 of the Sequence Listing).

[Figure 42] Figure 42 is a diagram showing the nucleotide sequence of a primer set F for the light chain of human chimeric 7B4 (c7B4) (SEQ ID NO: 39 of the Sequence Listing).

[Figure 43] Figure 43 is a diagram showing the nucleotide sequence of a primer set R for the light chain of human chimeric 7B4 (c7B4) (SEQ ID NO: 40 of the Sequence Listing).

[Figure 44] Figure 44 is a diagram showing the nucleotide sequence of the heavy chain of human chimeric 7B4 (c7B4) (SEQ ID NO: 41 of the Sequence Listing).

[Figure 45] Figure 45 is a diagram showing the amino acid sequence of the heavy chain of human chimeric 7B4 (c7B4) (signal sequence (1 to 19), variable region (20 to 142), and constant region (143 to 472)) (SEQ ID NO: 42 of the Sequence Listing).

[Figure 46] Figure 46 is a diagram showing the nucleotide sequence of a primer set F for the heavy chain of human chimeric 7B4 (c7B4) (SEQ ID NO: 43 of the Sequence Listing).

[Figure 47] Figure 47 is a diagram showing the nucleotide sequence of a primer set R for the heavy chain of human chimeric 7B4 (c7B4) (SEQ ID NO: 44 of the Sequence Listing).

[Figure 48] Figure 48 is a diagram showing results of testing the binding properties of the human chimeric antibodies (c2A4, c2B1, and c7B4) against human GPRC5D using a flow cytometer (FACS). The vertical axis represents a relative value of the mean fluorescence intensity assayed by flow cytometry.

[Figure 49] Figure 49 is a diagram showing results of testing the binding properties of the human chimeric antibodies (c2A4, c2B1, and c7B4) against cynomolgus monkey GPRC5D using a flow cytometer (FACS). The vertical axis represents a relative value of the mean fluorescence intensity assayed by flow cytometry.

[Figure 50] Figure 50 is a diagram showing that the human chimeric antibodies (c2A4, c2B1, and c7B4) have ADCC activity against human GPRC5D.

[Figure 51] Figure 51 is a diagram showing the *in vivo* tumor growth inhibitory activity of human chimeric 2A4 (c2A4) in GPRC5D-expressing human multiple myeloma cell line KHM-1B-grafted BALB/c-nu/nu mice.

[Figure 52] Figure 52 is a diagram showing the *in vivo* tumor growth inhibitory activity of human chimeric 2B1 (c2B1) in GPRC5D-expressing human multiple myeloma cell line KHM-1B-grafted BALB/c-nu/nu mice.

[Figure 53] Figure 53 is a diagram showing the *in vivo* tumor growth inhibitory activity of human chimeric 7B4 (c7B4) in GPRC5D-expressing human multiple myeloma cell line KHM-1B-grafted BALB/c-nu/nu mice.

[Figure 54] Figure 54 is a diagram showing the amino acid sequence of the heavy chain CDR1 of the rat anti-GPRC5D antibody 2A4 (SEQ ID NO: 45 of the Sequence Listing).

[Figure 55] Figure 55 is a diagram showing the amino acid sequence of the heavy chain CDR2 of the rat anti-GPRC5D antibody 2A4 (SEQ ID NO: 46 of the Sequence Listing).

[Figure 56] Figure 56 is a diagram showing the amino acid sequence of the heavy chain CDR3 of the rat anti-GPRC5D antibody 2A4 (SEQ ID NO: 47 of the Sequence Listing).

[Figure 57] Figure 57 is a diagram showing the amino acid sequence of the heavy chain CDR1 of the rat anti-GPRC5D antibody 2B1 (SEQ ID NO: 48 of the Sequence Listing).

[Figure 58] Figure 58 is a diagram showing the amino acid sequence of the heavy chain CDR2 of the rat anti-GPRC5D antibody 2B1 (SEQ ID NO: 49 of the Sequence Listing).

[Figure 59] Figure 59 is a diagram showing the amino acid sequence of the heavy chain CDR3 of the rat anti-GPRC5D antibody 2B1 (SEQ ID NO: 50 of the Sequence Listing).

[Figure 60] Figure 60 is a diagram showing the amino acid sequence of the heavy chain CDR1 of the rat anti-GPRC5D antibody 7B4 (SEQ ID NO: 51 of the Sequence Listing).

[Figure 61] Figure 61 is a diagram showing the amino acid sequence of the heavy chain CDR2 of the rat anti-GPRC5D antibody 7B4 (SEQ ID NO: 52 of the Sequence Listing).

[Figure 62] Figure 62 is a diagram showing the amino acid sequence of the heavy chain CDR3 of the rat anti-GPRC5D antibody 7B4 (SEQ ID NO: 53 of the Sequence Listing).

[Figure 63] Figure 63 is a diagram showing the amino acid sequence of the light chain CDR1 of the rat anti-GPRC5D antibody 2A4 (SEQ ID NO: 54 of the Sequence Listing).

[Figure 64] Figure 64 is a diagram showing the amino acid sequence of the light chain CDR2 of the rat anti-GPRC5D antibody 2A4 (SEQ ID NO: 55 of the Sequence Listing).

[Figure 65] Figure 65 is a diagram showing the amino acid sequence of the light chain CDR3 of the rat anti-GPRC5D antibody 2A4 (SEQ ID NO: 56 of the Sequence Listing).

[Figure 66] Figure 66 is a diagram showing the amino acid sequence of the light chain CDR1 of the rat anti-GPRC5D antibody 2B1 (SEQ ID NO: 57 of the Sequence Listing).

[Figure 67] Figure 67 is a diagram showing the amino acid sequence of the light chain CDR2 of the rat anti-GPRC5D antibody 2B1 (SEQ ID NO: 58 of the Sequence Listing).

[Figure 68] Figure 68 is a diagram showing the amino acid sequence of the light chain CDR3 of the rat anti-GPRC5D antibody 2B1 (SEQ ID NO: 59 of the Sequence Listing).

[Figure 69] Figure 69 is a diagram showing the amino acid sequence of the light chain CDR1 of the rat anti-GPRC5D antibody 7B4 (SEQ ID NO: 60 of the Sequence Listing).

[Figure 70] Figure 70 is a diagram showing the amino acid sequence of the light chain CDR2 of the rat anti-GPRC5D antibody 7B4 (SEQ ID NO: 61 of the Sequence Listing).

[Figure 71] Figure 71 is a diagram showing the amino acid sequence of the light chain CDR3 of the rat anti-GPRC5D antibody 7B4 (SEQ ID NO: 62 of the Sequence Listing).

[Figure 72] Figure 72 is a diagram showing the nucleotide sequence of a humanized 2B1 light chain (h2B1_L1) (SEQ ID NO: 63 of the Sequence Listing).

[Figure 73] Figure 73 is a diagram showing the amino acid sequence of the humanized 2B1 light chain (h2B1_L1) (signal sequence (1 to 20), variable region (21 to 127), and constant region (128 to 234)) (SEQ ID NO: 64 of the Sequence Listing).

[Figure 74] Figure 74 is a diagram showing the nucleotide sequence of a humanized 2B1 light chain (h2B1_L2) (SEQ ID NO: 65 of the Sequence Listing).

[Figure 75] Figure 75 is a diagram showing the amino acid sequence of the humanized 2B1 light chain (h2B1_L2) (signal sequence (1 to 20), variable region (21 to 127), and constant region (128 to 234)) (SEQ ID NO: 66 of the Sequence Listing).

[Figure 76] Figure 76 is a diagram showing the nucleotide sequence of a humanized 2B1 light chain (h2B1_L3) (SEQ ID NO: 67 of the Sequence Listing).

[Figure 77] Figure 77 is a diagram showing the amino acid sequence of the humanized 2B1 light chain (h2B1_L3) (signal sequence (1 to 20), variable region (21 to 127), and constant region (128 to 234)) (SEQ ID NO: 68 of the Sequence Listing).

[Figure 78] Figure 78 is a diagram showing the nucleotide sequence of a humanized 2B1 light chain (h2B1_L4) (SEQ ID NO: 69 of the Sequence Listing).

[Figure 79] Figure 79 is a diagram showing the amino acid sequence of the humanized 2B1 light chain (h2B1_L4) (signal sequence (1 to 20), variable region (21 to 127), and constant region (128 to 234)) (SEQ ID NO: 70 of the Sequence Listing).

[Figure 80] Figure 80 is a diagram showing the nucleotide sequence of a humanized 2B1 light chain (h2B1_L5) (SEQ ID NO: 71 of the Sequence Listing).

[Figure 81] Figure 81 is a diagram showing the amino acid sequence of the humanized 2B1 light chain (h2B1_L5) (signal sequence (1 to 20), variable region (21 to 127), and constant region (128 to 234)) (SEQ ID NO: 72 of the Sequence Listing).

[Figure 82] Figure 82 is a diagram showing the nucleotide sequence of a humanized 2B1 heavy chain (h2B1_H1) (SEQ ID NO: 73 of the Sequence Listing).

[Figure 83] Figure 83 is a diagram showing the amino acid sequence of the humanized 2B1 heavy chain (h2B1_H1) (signal sequence (1 to 19), variable region (20 to 142), and constant region (143 to 472)) (SEQ ID NO: 74 of the Sequence Listing).

[Figure 84] Figure 84 is a diagram showing the nucleotide sequence of a humanized 2B1 heavy chain (h2B1_H2) (SEQ ID NO: 75 of the Sequence Listing).

[Figure 85] Figure 85 is a diagram showing the amino acid sequence of the humanized 2B1 heavy chain (h2B1_H2) (signal sequence (1 to 19), variable region (20 to 142), and constant region (143 to 472)) (SEQ ID NO: 76 of the Sequence Listing).

[Figure 86] Figure 86 is a diagram showing the nucleotide sequence of a humanized 2B1 heavy chain (h2B1_H3) (SEQ ID NO: 77 of the Sequence Listing).

[Figure 87] Figure 87 is a diagram showing the amino acid sequence of the humanized 2B1 heavy chain (h2B1_H3) (signal sequence (1 to 19), variable region (20 to 142), and constant region (143 to 472)) (SEQ ID NO: 78 of the Sequence Listing).

[Figure 88] Figure 88 is a diagram showing the nucleotide sequence of a humanized 2B1 heavy chain (h2B1_H4) (SEQ ID NO: 79 of the Sequence Listing).

[Figure 89] Figure 89 is a diagram showing the amino acid sequence of the humanized 2B1 heavy chain (h2B1_H4) (signal sequence (1 to 19), variable region (20 to 142), and constant region (143 to 472)) (SEQ ID NO: 80 of the Sequence Listing).

[Figure 90] Figure 90 is a diagram showing the nucleotide sequence of a humanized 7B4 light chain (h7B4_L1) (SEQ ID NO: 81 of the Sequence Listing).

[Figure 91] Figure 91 is a diagram showing the amino acid sequence of the humanized 7B4 light chain (h7B4_L1) (signal sequence (1 to 20), variable region (21 to 126), and constant region (127 to 233)) (SEQ ID NO: 82 of the Sequence Listing).

[Figure 92] Figure 92 is a diagram showing the nucleotide sequence of a humanized 7B4 light chain (h7B4_L2) (SEQ ID NO: 83 of the Sequence Listing).

[Figure 93] Figure 93 is a diagram showing the amino acid sequence of the humanized 7B4 light chain (h7B4_L2) (signal sequence (1 to 20), variable region (21 to 126), and constant region (127 to 233)) (SEQ ID NO: 84 of the Sequence Listing).

[Figure 94] Figure 94 is a diagram showing the nucleotide sequence of a humanized 7B4 heavy chain (h7B4_H1) (SEQ ID NO: 85 of the Sequence Listing).

[Figure 95] Figure 95 is a diagram showing the amino acid sequence of the humanized 7B4 heavy chain (h7B4_H1) (signal sequence (1 to 19), variable region (20 to 142), and constant region (143 to 472)) (SEQ ID NO: 86 of the Sequence Listing).

[Figure 96] Figure 96 is a diagram showing the nucleotide sequence of a humanized 7B4 heavy chain (h7B4_H2) (SEQ ID NO: 87 of the Sequence Listing).

[Figure 97] Figure 97 is a diagram showing the amino acid sequence of the humanized 7B4 heavy chain (h7B4_H2) (signal sequence (1 to 19), variable region (20 to 142), and constant region (143 to 472)) (SEQ ID NO: 88 of the Sequence Listing).

[Figure 98] Figure 98 is a diagram showing the nucleotide sequence of a humanized 7B4 heavy chain (h7B4_H3) (SEQ ID NO: 89 of the Sequence Listing).

[Figure 99] Figure 99 is a diagram showing the amino acid sequence of the humanized 7B4 heavy chain (h7B4_H3) (signal sequence (1 to 19), variable region (20 to 142), and constant region (143 to 472)) (SEQ ID NO: 90 of the Sequence Listing).

[Figure 100] Figure 100 is a diagram showing the nucleotide sequence of a humanized 7B4 heavy chain (h7B4_H5) (SEQ ID NO: 91 of the Sequence Listing).

[Figure 101] Figure 101 is a diagram showing the amino acid sequence of the humanized 7B4 heavy chain (h7B4_H5) (signal sequence (1 to 19), variable region (20 to 142), and constant region (143 to 472)) (SEQ ID NO: 92 of the Sequence Listing).

[Figure 102] Figure 102 is a diagram showing results of testing the binding properties of humanized 2B1 against human GPRC5D using a flow cytometer (FACS). The vertical axis represents a relative value of the mean fluorescence intensity assayed by flow cytometry.

[Figure 103] Figure 103 is a diagram showing results of testing the binding properties of humanized 7B4 against human GPRC5D using a flow cytometer (FACS). The vertical axis represents a relative value of the mean fluorescence intensity assayed by flow cytometry.

[Figure 104] Figure 104 is a diagram showing that humanized 2B1 and humanized 7B4 have ADCC activity.

[Figure 105] Figure 105 is a diagram showing the amino acid sequence of the amino-terminal peptide of cynomolgus monkey GPRC5D (SEQ ID NO: 93).

[Figure 106] Figure 106 is a diagram showing the nucleotide sequence of a primer A used in the sequence analysis of scFv (SEQ ID NO: 94).

[Figure 107] Figure 107 is a diagram showing the nucleotide sequence of a primer B used in the sequence analysis of scFv (SEQ ID NO: 95).

[Figure 108] Figure 108 is a diagram showing the nucleotide sequence of the heavy chain variable region of a human antibody C2037 (SEQ ID NO: 96 of the Sequence Listing).

[Figure 109] Figure 109 is a diagram showing the amino acid sequence of the heavy chain variable region of the human antibody C2037 (SEQ ID NO: 97 of the Sequence Listing).

[Figure 110] Figure 110 is a diagram showing the nucleotide sequence of the light chain variable region of the human antibody C2037 (SEQ ID NO: 98 of the Sequence Listing).

[Figure 111] Figure 111 is a diagram showing the amino acid sequence of the light chain variable region of the human antibody C2037 (SEQ ID NO: 99 of the Sequence Listing).

[Figure 112] Figure 112 is a diagram showing the nucleotide sequence of the heavy chain variable region of a human antibody C3048 (SEQ ID NO: 100 of the Sequence Listing).

[Figure 113] Figure 113 is a diagram showing the amino acid sequence of the heavy chain variable region of the human antibody C3048 (SEQ ID NO: 101 of the Sequence Listing).

[Figure 114] Figure 114 is a diagram showing the nucleotide sequence of the light chain variable region of the human antibody C3048 (SEQ ID NO: 102 of the Sequence Listing).
[Figure 115] Figure 115 is a diagram showing the amino acid sequence of the light chain variable region of the human antibody C3048 (SEQ ID NO: 103 of the Sequence Listing).
[Figure 116] Figure 116 is a diagram showing the nucleotide sequence of the heavy chain variable region of a human antibody C3015 (SEQ ID NO: 104 of the Sequence Listing).
[Figure 117] Figure 117 is a diagram showing the amino acid sequence of the heavy chain variable region of the human antibody C3015 (SEQ ID NO: 105 of the Sequence Listing).
[Figure 118] Figure 118 is a diagram showing the nucleotide sequence of the light chain variable region of the human antibody C3015 (SEQ ID NO: 106 of the Sequence Listing).
[Figure 119] Figure 119 is a diagram showing the amino acid sequence of the light chain variable region of the human antibody C3015 (SEQ ID NO: 107 of the Sequence Listing).
[Figure 120] Figure 120 is a diagram showing the nucleotide sequence of the heavy chain variable region of a human antibody C3022 (SEQ ID NO: 108 of the Sequence Listing).
[Figure 121] Figure 121 is a diagram showing the amino acid sequence of the heavy chain variable region of the human antibody C3022 (SEQ ID NO: 109 of the Sequence Listing).
[Figure 122] Figure 122 is a diagram showing the nucleotide sequence of the light chain variable region of the human antibody C3022 (SEQ ID NO: 110 of the Sequence Listing).
[Figure 123] Figure 123 is a diagram showing the amino acid sequence of the light chain variable region of the human antibody C3022 (SEQ ID NO: 135 of the Sequence Listing).
[Figure 124] Figure 124 is a diagram showing the amino acid sequence of the heavy chain CDR1 of the human antibody C2037 (SEQ ID NO: 111 of the Sequence Listing).
[Figure 125] Figure 125 is a diagram showing the amino acid sequence of the heavy chain CDR2 of the human antibody C2037 (SEQ ID NO: 112 of the Sequence Listing).
[Figure 126] Figure 126 is a diagram showing the amino acid sequence of the heavy chain CDR3 of the human antibody C2037 (SEQ ID NO: 113 of the Sequence Listing).
[Figure 127] Figure 127 is a diagram showing the amino acid sequence of the light chain CDR1 of the human antibody C2037 (SEQ ID NO: 114 of the Sequence Listing).
[Figure 128] Figure 128 is a diagram showing the amino acid sequence of the light chain CDR2 of the human antibody C2037 (SEQ ID NO: 115 of the Sequence Listing).
[Figure 129] Figure 129 is a diagram showing the amino acid sequence of the light chain CDR3 of the human antibody C2037 (SEQ ID NO: 116 of the Sequence Listing).
[Figure 130] Figure 130 is a diagram showing the amino acid sequence of the heavy chain CDR1 of the human antibody C3048 (SEQ ID NO: 117 of the Sequence Listing).
[Figure 131] Figure 131 is a diagram showing the amino acid sequence of the heavy chain CDR2 of the human antibody C3048 (SEQ ID NO: 118 of the Sequence Listing).
[Figure 132] Figure 132 is a diagram showing the amino acid sequence of the heavy chain CDR3 of the human antibody C3048 (SEQ ID NO: 119 of the Sequence Listing).
[Figure 133] Figure 133 is a diagram showing the amino acid sequence of the light chain CDR1 of the human antibody C3048 (SEQ ID NO: 120 of the Sequence Listing).
[Figure 134] Figure 134 is a diagram showing the amino acid sequence of the light chain CDR2 of the human antibody C3048 (SEQ ID NO: 121 of the Sequence Listing).
[Figure 135] Figure 135 is a diagram showing the amino acid sequence of the light chain CDR3 of the human antibody C3048 (SEQ ID NO: 122 of the Sequence Listing).
[Figure 136] Figure 136 is a diagram showing the amino acid sequence of the heavy chain CDR1 of the human antibody C3015 (SEQ ID NO: 123 of the Sequence Listing).
[Figure 137] Figure 137 is a diagram showing the amino acid sequence of the heavy chain CDR2 of the human antibody C3015 (SEQ ID NO: 124 of the Sequence Listing).
[Figure 138] Figure 138 is a diagram showing the amino acid sequence of the heavy chain CDR3 of the human antibody C3015 (SEQ ID NO: 125 of the Sequence Listing).
[Figure 139] Figure 139 is a diagram showing the amino acid sequence of the light chain CDR1 of the human antibody C3015 (SEQ ID NO: 126 of the Sequence Listing).
[Figure 140] Figure 140 is a diagram showing the amino acid sequence of the light chain CDR2 of the human antibody C3015 (SEQ ID NO: 127 of the Sequence Listing).
[Figure 141] Figure 141 is a diagram showing the amino acid sequence of the light chain CDR3 of the human antibody C3015 (SEQ ID NO: 128 of the Sequence Listing).
[Figure 142] Figure 142 is a diagram showing the amino acid sequence of the heavy chain CDR1 of the human antibody C3022 (SEQ ID NO: 129 of the Sequence Listing).
[Figure 143] Figure 143 is a diagram showing the amino acid sequence of the heavy chain CDR2 of the human antibody C3022 (SEQ ID NO: 130 of the Sequence Listing).

[Figure 144] Figure 144 is a diagram showing the amino acid sequence of the heavy chain CDR3 of the human antibody C3022 (SEQ ID NO: 131 of the Sequence Listing).

[Figure 145] Figure 145 is a diagram showing the amino acid sequence of the light chain CDR1 of the human antibody C3022 (SEQ ID NO: 132 of the Sequence Listing).

[Figure 146] Figure 146 is a diagram showing the amino acid sequence of the light chain CDR2 of the human antibody C3022 (SEQ ID NO: 133 of the Sequence Listing).

[Figure 147] Figure 147 is a diagram showing the amino acid sequence of the light chain CDR3 of the human antibody C3022 (SEQ ID NO: 134 of the Sequence Listing).

[Figure 148] Figure 148 is a diagram showing the nucleotide sequence of the heavy chain of an IgG form of the human antibody C2037 (SEQ ID NO: 136 of the Sequence Listing).

[Figure 149] Figure 149 is a diagram showing the nucleotide sequence of the light chain of an IgG form of the human antibody C2037 (SEQ ID NO: 137 of the Sequence Listing).

[Figure 150] Figure 150 is a diagram showing the nucleotide sequence of the heavy chain of an IgG form of the human antibody C3048 (SEQ ID NO: 138 of the Sequence Listing).

[Figure 151] Figure 151 is a diagram showing the nucleotide sequence of the light chain of an IgG form of the human antibody C3048 (SEQ ID NO: 139 of the Sequence Listing).

[Figure 152] Figure 152 is a diagram showing the nucleotide sequence of the heavy chain of an IgG form of the human antibody C3015 (SEQ ID NO: 140 of the Sequence Listing).

[Figure 153] Figure 153 is a diagram showing the nucleotide sequence of the light chain of an IgG form of the human antibody C3015 (SEQ ID NO: 141 of the Sequence Listing).

[Figure 154] Figure 154 is a diagram showing the nucleotide sequence of the heavy chain of an IgG form of the human antibody C3022 (SEQ ID NO: 142 of the Sequence Listing).

[Figure 155] Figure 155 is a diagram showing the nucleotide sequence of the light chain of an IgG form of the human antibody C3022 (SEQ ID NO: 143 of the Sequence Listing).

[Figure 156] Figure 156 is a diagram showing the amino acid sequence of the heavy chain of the IgG form of the human antibody C2037 (signal sequence (1 to 19), variable region (20 to 134), and constant region (135 to 464)) (SEQ ID NO: 144 of the Sequence Listing).

[Figure 157] Figure 157 is a diagram showing the amino acid sequence of the light chain of the IgG form of the human antibody C2037 (signal sequence (1 to 20), variable region (21 to 130), and constant region (131 to 236)) (SEQ ID NO: 145 of the Sequence Listing).

[Figure 158] Figure 158 is a diagram showing the amino acid sequence of the heavy chain of the IgG form of the human antibody C3048 (signal sequence (1 to 19), variable region (20 to 142), and constant region (143 to 472)) (SEQ ID NO: 146 of the Sequence Listing).

[Figure 159] Figure 159 is a diagram showing the amino acid sequence of the light chain of the IgG form of the human antibody C3048 (signal sequence (1 to 20), variable region (21 to 130), and constant region (131 to 236)) (SEQ ID NO: 147 of the Sequence Listing).

[Figure 160] Figure 160 is a diagram showing the amino acid sequence of the heavy chain of the IgG form of the human antibody C3015 (signal sequence (1 to 19), variable region (20 to 140), and constant region (141 to 470)) (SEQ ID NO: 148 of the Sequence Listing).

[Figure 161] Figure 161 is a diagram showing the amino acid sequence of the light chain of the IgG form of the human antibody C3015 (signal sequence (1 to 20), variable region (21 to 126), and constant region (127 to 232)) (SEQ ID NO: 149 of the Sequence Listing).

[Figure 162] Figure 162 is a diagram showing the amino acid sequence of the heavy chain of the IgG form of the human antibody C3022 (signal sequence (1 to 19), variable region (20 to 134), and constant region (135 to 464)) (SEQ ID NO: 150 of the Sequence Listing).

[Figure 163] Figure 163 is a diagram showing the amino acid sequence of the light chain of the IgG form of the human antibody C3022 (signal sequence (1 to 20), variable region (21 to 130), and constant region (131 to 236)) (SEQ ID NO: 151 of the Sequence Listing).

[Figure 164] Figure 164 is a diagram showing results of testing the binding properties of human antibody scFv against the amino terminus of biotinylated human (A) or cynomolgus monkey (B) GPRC5D by ELISA. The vertical axis represents the emission intensity assayed by ELISA.

[Figure 165] Figure 165 is a diagram showing results of testing the binding properties of an IgG form of a human antibody against the amino terminus of biotinylated human or cynomolgus monkey GPRC5D by ELISA. The vertical axis represents the emission intensity assayed by ELISA.

[Figure 166] Figure 166 is a diagram showing results of testing the binding properties of human antibody scFv against a human GPRC5D-expressing cancer cell line using a flow cytometer (FACS). The vertical axis represents a relative value of the mean fluorescence intensity assayed by flow cytometry.

[Figure 167] Figure 167 is a diagram showing results of testing the binding properties of an IgG form of a human antibody against a human GPRC5D-expressing cancer cell line using a flow cytometer (FACS). The vertical axis represents a relative value of the mean fluorescence intensity assayed by flow cytometry.
[Figure 168] Figure 168 is a diagram showing a nucleotide sequence encoding the heavy chain variable region of a rat anti-CD3 antibody C3-147 (SEQ ID NO: 152).
[Figure 169] Figure 169 is a diagram showing a nucleotide sequence encoding the light chain variable region of the rat anti-CD3 antibody C3-147 (SEQ ID NO: 153).
[Figure 170] Figure 170 is a diagram showing a nucleotide sequence encoding C3E-7000 (58 to 867) (signal sequence (1 to 57), scFv (58 to 783), and FLAG-His tag (793 to 867)) (SEQ ID NO: 154).
[Figure 171] Figure 171 is a diagram showing the amino acid sequence of the heavy chain variable region of C3E-7034 (SEQ ID NO: 155).
[Figure 172] Figure 172 is a diagram showing the amino acid sequence of the light chain variable region of C3E-7034 (SEQ ID NO: 156).
[Figure 173] Figure 173 is a diagram showing a nucleotide sequence encoding C3E-7034 (58 to 864) (signal sequence (1 to 57), scFv (61 to 786), and FLAG-His tag (790 to 864)) (SEQ ID NO: 157).
[Figure 174] Figure 174 is a diagram showing the amino acid sequence of the light chain variable region of C3E-7035 (SEQ ID NO: 158).
[Figure 175] Figure 175 is a diagram showing a nucleotide sequence encoding C3E-7035 (58 to 864) (signal sequence (1 to 57), scFv (61 to 786), and FLAG-His tag (790 to 864)) (SEQ ID NO: 159).
[Figure 176] Figure 176 is a diagram showing the amino acid sequence of the light chain variable region of C3E-7036 (SEQ ID NO: 160).
[Figure 177] Figure 177 is a diagram showing a nucleotide sequence encoding C3E-7036 (58 to 858) (signal sequence (1 to 57), scFv (61 to 780), and FLAG-His tag (784 to 858)) (SEQ ID NO: 161).
[Figure 178] Figure 178 is a diagram showing a nucleotide sequence encoding ORF of an expression vector pC2037-C3E-7034 (SEQ ID NO: 162).
[Figure 179] Figure 179 is a diagram showing a nucleotide sequence encoding ORF of an expression vector pC3048-C3E-7034 (SEQ ID NO: 163).
[Figure 180] Figure 180 is a diagram showing a nucleotide sequence encoding ORF of an expression vector pC3022-C3E-7034 (SEQ ID NO: 164).
[Figure 181] Figure 181 is a diagram showing a nucleotide sequence encoding ORF of an expression vector pC2037-C3E-7035 (SEQ ID NO: 165).
[Figure 182] Figure 182 is a diagram showing a nucleotide sequence encoding ORF of an expression vector pC3048-C3E-7035 (SEQ ID NO: 166).
[Figure 183] Figure 183 is a diagram showing a nucleotide sequence encoding ORF of an expression vector pC3022-C3E-7035 (SEQ ID NO: 167).
[Figure 184] Figure 184 is a diagram showing a nucleotide sequence encoding ORF of an expression vector pC2037-C3E-7036 (SEQ ID NO: 168).
[Figure 185] Figure 185 is a diagram showing a nucleotide sequence encoding ORF of an expression vector pC3048-C3E-7036 (SEQ ID NO: 169).
[Figure 186] Figure 186 is a diagram showing a nucleotide sequence encoding ORF of an expression vector pC3022-C3E-7036 (SEQ ID NO: 170).
[Figure 187] Figure 187 is a diagram showing the amino acid sequence of C2037-C3E-7034 (signal sequence (1 to 19), C2037 (21 to 260), and C3E-7034 (266 to 507)) (SEQ ID NO: 171).
[Figure 188] Figure 188 is a diagram showing the amino acid sequence of C3048-C3E-7034 (signal sequence (1 to 19), C3048 (21 to 268), and C3E-7034 (274 to 515)) (SEQ ID NO: 172).
[Figure 189] Figure 189 is a diagram showing the amino acid sequence of C3022-C3E-7034 (signal sequence (1 to 19), C3022 (21 to 260), and C3E-7034 (266 to 507)) (SEQ ID NO: 173).
[Figure 190] Figure 190 is a diagram showing the amino acid sequence of C2037-C3E-7035 (signal sequence (1 to 19), C2037 (21 to 260), and C3E-7035 (266 to 507)) (SEQ ID NO: 174).
[Figure 191] Figure 191 is a diagram showing the amino acid sequence of C3048-C3E-7035 (signal sequence (1 to 19), C3048 (21 to 268), and C3E-7035 (274 to 515)) (SEQ ID NO: 175).
[Figure 192] Figure 192 is a diagram showing the amino acid sequence of C3022-C3E-7035 (signal sequence (1 to 19), C3022 (21 to 260), and C3E-7035 (266 to 507)) (SEQ ID NO: 176).
[Figure 193] Figure 193 is a diagram showing the amino acid sequence of C2037-C3E-7036 (signal sequence (1 to 19), C2037 (21 to 260), and C3E-7036 (266 to 505)) (SEQ ID NO: 177).
[Figure 194] Figure 194 is a diagram showing the amino acid sequence of C3048-C3E-7036 (signal sequence (1 to 19), C3048 (21 to 268), and C3E-7036 (274 to 513)) (SEQ ID NO: 178).
[Figure 195] Figure 195 is a diagram showing the amino acid sequence of C3022-C3E-7036 (signal sequence (1 to 19), C3022 (21 to 260), and C3E-7036 (266 to 505)) (SEQ ID NO: 179).

[Figure 196] Figure 196 is a diagram showing results of testing the binding properties of an anti-GPRC5D-anti-CD3 bispecific molecule against cells expressing endogenous human GPRC5D (human lymphoma cell line A4/FuK cells) using a flow cytometer (FACS). The vertical axis represents a relative value of the mean fluorescence intensity assayed by flow cytometry.

[Figure 197] Figure 197 is a diagram showing results of testing the binding properties of the anti-GPRC5D-anti-CD3 bispecific molecule against cells expressing cynomolgus monkey GPRC5D using a flow cytometer (FACS). The vertical axis represents a relative value of the mean fluorescence intensity assayed by flow cytometry.

[Figure 198] Figure 198 is a diagram showing results of testing the binding properties of the anti-GPRC5D-anti-CD3 bispecific molecule against human CD3 (PBMC) using a flow cytometer (FACS). The vertical axis represents a relative value of the mean fluorescence intensity assayed by flow cytometry.

[Figure 199] Figure 199 is a diagram showing results of testing the binding properties of the anti-GPRC5D-anti-CD3 bispecific molecule against cynomolgus monkey CD3 (PBMC) using a flow cytometer (FACS). The vertical axis represents a relative value of the mean fluorescence intensity assayed by flow cytometry.

[Figure 200] Figure 200 is a diagram showing that the anti-GPRC5D-anti-CD3 bispecific molecule has cytotoxic activity against cells expressing endogenous human GPRC5D (human lymphoma cell line A4/FuK cells).

[Figure 201] Figure 201 is a diagram showing results of testing the binding properties of humanized 2B1 against cynomolgus monkey GPRC5D using a flow cytometer (FACS). The vertical axis represents a relative value of the mean fluorescence intensity assayed by flow cytometry.

[Figure 202] Figure 202 is a diagram showing results of testing the binding properties of humanized 7B4 against cynomolgus monkey GPRC5D using a flow cytometer (FACS). The vertical axis represents a relative value of the mean fluorescence intensity assayed by flow cytometry.

[Figure 203] Figure 203 is a diagram showing the amino acid sequence of C3E-7034 (1 to 269). VH (2 to 119), VL (135 to 243), and FLAG-His tag (244 to 269) (SEQ ID NO: 180).

[Figure 204] Figure 204 is a diagram showing the amino acid sequence of C3E-7035 (1 to 269). VH (2 to 119), VL (135 to 243), and FLAG-His tag (244 to 269)) (SEQ ID NO: 181).

[Figure 205] Figure 205 is a diagram showing the amino acid sequence of C3E-7036 (1 to 267). VH (2 to 119), VL (135 to 241), and FLAG-His tag (242 to 267)) (SEQ ID NO: 182).

[Figure 206] Figure 206 is a diagram showing the amino acid sequence of the heavy chain CDR1 of C3E-7000 (SEQ ID NO: 183).

[Figure 207] Figure 207 is a diagram showing the amino acid sequence of the heavy chain CDR2 of C3E-7000 (SEQ ID NO: 184).

[Figure 208] Figure 208 is a diagram showing the amino acid sequence of the heavy chain CDR3 of C3E-7000 (SEQ ID NO: 185).

[Figure 209] Figure 209 is a diagram showing the amino acid sequence of the light chain CDR1 of C3E-7000 (SEQ ID NO: 186).

[Figure 210] Figure 210 is a diagram showing the amino acid sequence of the light chain CDR2 of C3E-7000 (SEQ ID NO: 187).

[Figure 211] Figure 211 is a diagram showing the amino acid sequence of the light chain CDR3 of C3E-7000 (SEQ ID NO: 188).

[Figure 212] Figure 212 is a diagram showing the amino acid sequence of human CD3ε (SEQ ID NO: 189).

[Figure 213] Figure 213 is a diagram showing the nucleotide sequence of the heavy chain variable region of E1018 (SEQ ID NO: 190).

[Figure 214] Figure 214 is a diagram showing the amino acid sequence of the heavy chain variable region of E1018 (SEQ ID NO: 191).

[Figure 215] Figure 215 is a diagram showing the nucleotide sequence of the light chain variable region of E1018 (SEQ ID NO: 192).

[Figure 216] Figure 216 is a diagram showing the amino acid sequence of the light chain variable region of E1018 (SEQ ID NO: 193).

[Figure 217] Figure 217 is a diagram showing the nucleotide sequence of the heavy chain variable region of D1012 (SEQ ID NO: 194).

[Figure 218] Figure 218 is a diagram showing the amino acid sequence of the heavy chain variable region of D1012 (SEQ ID NO: 195).

[Figure 219] Figure 219 is a diagram showing the nucleotide sequence of the light chain variable region of D1012 (SEQ ID NO: 196).

[Figure 220] Figure 220 is a diagram showing the amino acid sequence of the light chain variable region of D1012 (SEQ ID NO: 197).

[Figure 221] Figure 221 is a diagram showing the binding dissociation constant determined by assaying the binding properties of the anti-GPRC5D antibodies (C3022, E1018, C3048, and D1012) against human GPRC5D by SPR.

[Figure 222] Figure 222 is a diagram showing the nucleotide sequence of h2B1_Fab_HC_1 (SEQ ID NO: 198).

[Figure 223] Figure 223 is a diagram showing the amino acid sequence of h2B1_Fab_HC_1 (signal sequence (1 to 23), variable region (24 to 146), and constant region (147 to 475)) (SEQ ID NO: 199).

[Figure 224] Figure 224 is a diagram showing the nucleotide sequence of h2B1_Fab_HC_2 (SEQ ID NO: 200).

[Figure 225] Figure 225 is a diagram showing the amino acid sequence of h2B1_Fab_HC_2 (signal sequence (1 to 23), variable region (24 to 146), and constant region (147 to 475)) (SEQ ID NO: 201).

[Figure 226] Figure 226 is a diagram showing the nucleotide sequence of h2B1_Fab_LC_1 (SEQ ID NO: 202).

[Figure 227] Figure 227 is a diagram showing the amino acid sequence of h2B1_Fab_LC_1 (signal sequence (1 to 23), variable region (24 to 130), and constant region (131 to 237)) (SEQ ID NO: 203).

[Figure 228] Figure 228 is a diagram showing the nucleotide sequence of h2B1_Fab_LC_2 (SEQ ID NO: 204).

[Figure 229] Figure 229 is a diagram showing the amino acid sequence of h2B1_Fab_LC_2 (signal sequence (1 to 23), variable region (24 to 130), and constant region (131 to 237)) (SEQ ID NO: 205).

[Figure 230] Figure 230 is a diagram showing the nucleotide sequence of C3E-7034_Fab_HC (SEQ ID NO: 206).

[Figure 231] Figure 231 is a diagram showing the amino acid sequence of C3E-7034_Fab_HC (signal sequence (1 to 23), variable region (25 to 142), and constant region (143 to 471)) (SEQ ID NO: 207).

[Figure 232] Figure 232 is a diagram showing the nucleotide sequence of C3E-7034_Fab_LC (SEQ ID NO: 208).

[Figure 233] Figure 233 is a diagram showing the amino acid sequence of C3E-7034_Fab_LC (signal sequence (1 to 23), variable region (24 to 132), and constant region (133 to 238)) (SEQ ID NO: 209).

[Figure 234] Figure 234 is a diagram showing the nucleotide sequence of C3E-7036_Fab_HC (SEQ ID NO: 210).

[Figure 235] Figure 235 is a diagram showing the amino acid sequence of C3E-7036_Fab_HC (signal sequence (1 to 23), variable region (25 to 142), and constant region (143 to 471)) (SEQ ID NO: 211).

[Figure 236] Figure 236 is a diagram showing the nucleotide sequence of C3E-7036_Fab_LC (SEQ ID NO: 212).

[Figure 237] Figure 237 is a diagram showing the amino acid sequence of C3E-7036_Fab_LC (signal sequence (1 to 23), variable region (24 to 130), and constant region (131 to 236)) (SEQ ID NO: 213).

[Figure 238] Figure 238 is a diagram showing the nucleotide sequence of h2B1_Fab_HC_3 (SEQ ID NO: 214).

[Figure 239] Figure 239 is a diagram showing the amino acid sequence of h2B1_Fab_HC_3 (signal sequence (1 to 23), variable region (24 to 146), and constant region (147 to 475)) (SEQ ID NO: 215).

[Figure 240] Figure 240 is a diagram showing the nucleotide sequence of h2B1_Fab_LC_3 (SEQ ID NO: 216).

[Figure 241] Figure 241 is a diagram showing the amino acid sequence of h2B1_Fab_LC_3 (signal sequence (1 to 23), variable region (24 to 130), and constant region (131 to 237)) (SEQ ID NO: 217).

[Figure 242] Figure 242 is a diagram showing the nucleotide sequence of C3E-7034_scFv_Fc (SEQ ID NO: 218).

[Figure 243] Figure 243 is a diagram showing the amino acid sequence of C3E-7034_scFv_Fc (signal sequence (1 to 23), and scFv (24 to 266)) (SEQ ID NO: 219).

[Figure 244] Figure 234 is a diagram showing the nucleotide sequence of C3E-7036_scFv_Fc (SEQ ID NO: 220).

[Figure 245] Figure 245 is a diagram showing the amino acid sequence of C3E-7036_scFv_Fc (signal sequence (1 to 23), and scFv (24 to 264)) (SEQ ID NO: 221).

[Figure 246] Figure 246 is a diagram showing the nucleotide sequence of humanized 2B1_scFv_Fc (h2B1_scFv_Fc) (SEQ ID NO: 222).

[Figure 247] Figure 247 is a diagram showing the amino acid sequence of humanized_2B1_scFv_Fc (h2B1_scFv_Fc) (signal sequence (1 to 23), and scFv (24 to 271)) (SEQ ID NO: 223).

[Figure 248] Figure 248 is a diagram showing results of testing the binding properties of a Fc-containing anti-GPRC5D-anti-CD3 bispecific molecule against cells expressing endogenous human GPRC5D by flow cytometry. The vertical axis represents the mean fluorescence intensity assayed by flow cytometry. A, B, and C are diagrams showing results of testing the binding properties of FSA-type, hybrid-type and dual-type bispecific molecules respectively.

[Figure 249] Figure 249 is a diagram showing results of testing the binding properties of a Fc-containing anti-GPRC5D-anti-CD3 bispecific molecule against cells expressing cynomolgus monkey GPRC5D by flow cytometry. The vertical axis represents the mean fluorescence intensity assayed by flow cytometry. A, B, and C are diagrams showing results of testing the binding properties of FSA-type, hybrid-type and dual-type bispecific molecules respectively.

[Figure 250] Figure 250 is a diagram showing results of testing the binding properties of the Fc-containing anti-GPRC5D-anti-CD3 bispecific molecule against cells expressing human CD3 by flow cytometry. The vertical axis represents the mean fluorescence intensity assayed by flow cytometry. A, B, and C are diagrams showing results of testing the binding properties of FSA-type, hybrid-type and dual-type bispecific molecules respectively.

[Figure 251] Figure 251 is a diagram showing results of testing the binding properties of the Fc-containing anti-GPRC5D-anti-CD3 bispecific molecule against cells expressing cynomolgus monkey CD3 by flow cytometry. The vertical axis represents the mean fluorescence intensity assayed by flow cytometry. A, B, and C are diagrams showing results of testing the binding properties of FSA-type, hybrid-type and dual-type bispecific molecules respectively.

[Figure 252-1] Figure 252 is a diagram showing the cytotoxic activity of the Fc-containing anti-GPRC5D-anti-CD3 bispecific molecule. A and B are diagrams showing the cytocidal activity of FSA-type and hybrid-type bispecific molecules respectively.

[Figure 252-2] Figure 252 is a diagram showing the cytotoxic activity of the Fc-containing anti-GPRC5D-anti-CD3 bispecific molecule. C is a diagram showing results of the cytocidal activity of dual-type bispecific molecules.

[Figure 253] Figure 253 is a diagram showing the anti-tumor activity of the Fc-containing anti-GPRC5D-anti-CD3 bispecific molecule in a co-grafting model of human PBMC and cancer cells.

[Figure 254] Figure 254 is a diagram showing the anti-tumor activity of Fc-containing hybrid-type anti-GPRC5D-anti-CD3 bispecific molecule in a co-grafting model of human PBMC and cancer cells.

[Figure 255] Figure 255 is a diagram showing the nucleotide sequence of C3E-8015 (SEQ ID NO: 224).

[Figure 256] Figure 256 is a diagram showing the amino acid sequence of C3E-8015 (signal sequence (1 to 23), and scFv (24 to 264)) (SEQ ID NO: 225).

[Figure 257] Figure 257 is a diagram showing the nucleotide sequence of C3E-8017 (SEQ ID NO: 226).

[Figure 258] Figure 258 is a diagram showing the amino acid sequence of C3E-8017 (signal sequence (1 to 23), and scFv (24 to 266)) (SEQ ID NO: 227).

[Figure 259] Figure 259 is a diagram showing the nucleotide sequence of C3E-8018 (SEQ ID NO: 228).

[Figure 260] Figure 260 is a diagram showing the amino acid sequence of C3E-8018 (signal sequence (1 to 23), and scFv (24 to 266)) (SEQ ID NO: 229).

[Figure 261] Figure 261 is a diagram showing the nucleotide sequence of C3E-8025 (SEQ ID NO: 230).

[Figure 262] Figure 262 is a diagram showing the amino acid sequence of C3E-8025 (signal sequence (1 to 23), and scFv (24 to 264)) (SEQ ID NO: 231).

[Figure 263] Figure 263 is a diagram showing the nucleotide sequence of C3E-8027 (SEQ ID NO: 232).

[Figure 264] Figure 264 is a diagram showing the amino acid sequence of C3E-8027 (signal sequence (1 to 23), and scFv (24 to 266)) (SEQ ID NO: 233).

[Figure 265] Figure 265 is a diagram showing the nucleotide sequence of C3E-8028 (SEQ ID NO: 234).

[Figure 266] Figure 266 is a diagram showing the amino acid sequence of C3E-8028 (signal sequence (1 to 23), and scFv (24 to 266)) (SEQ ID NO: 235).

[Figure 267] Figure 267 is a diagram showing the nucleotide sequence of h2B1_Fab_HC_4 (SEQ ID NO: 236).

[Figure 268] Figure 268 is a diagram showing the amino acid sequence of h2B1_Fab_HC_4 (signal sequence (1 to 23), variable region (24 to 146), and constant region (147 to 476)) (SEQ ID NO: 237).

[Figure 269] Figure 269 includes diagrams showing results of testing the binding properties of C-terminally Lys-added CDR-modified hybrid-type and CDR-modified hybrid-type anti-GPRC5D-anti-CD3 bispecific molecules against cells expressing endogenous human GPRC5D by flow cytometry. The vertical axis represents the mean fluorescence intensity assayed by flow cytometry. A, B, and C are diagrams showing the binding properties of (C5D-0004 and C5D-0014), (C5D-0005 and C5D-0015) and (C5D-0006 and C5D-0016) respectively.

[Figure 270] Figure 270 includes diagrams showing results of testing the binding properties of C-terminally Lys-added CDR-modified hybrid-type and CDR-modified hybrid-type anti-GPRC5D-anti-CD3 bispecific molecules against cells expressing cynomolgus monkey GPRC5D by flow cytometry. The vertical axis represents the mean fluorescence intensity assayed by flow cytometry. A, B, and C are diagrams showing the binding properties of (C5D-0004 and C5D-0014), (C5D-0005 and C5D-0015) and (C5D-0006 and C5D-0016) respectively.

[Figure 271] Figure 271 includes diagrams showing results of testing the binding activity of C-terminally Lys-added CDR-modified hybrid-type and CDR-modified hybrid-type anti-GPRC5D-anti-CD3 bispecific molecules against cells expressing human CD3 by flow cytometry. The vertical axis represents the mean fluorescence intensity assayed by flow cytometry. A, B, and C are diagrams showing the binding properties of (C5D-0004 and C5D-0014), (C5D-0005 and C5D-0015) and (C5D-0006 and C5D-0016) respectively.

[Figure 272] Figure 272 includes diagrams showing results of testing the binding properties of C-terminally Lys-added CDR-modified hybrid-type and CDR-modified hybrid-type anti-GPRC5D-anti-CD3 bispecific molecules against cells expressing human CD3 by flow cytometry. The vertical axis represents the mean fluorescence intensity assayed by flow cytometry. A, B, and C are diagrams showing the binding properties of (C5D-0004 and C5D-0014), (C5D-0005 and C5D-0015) and (C5D-0006 and C5D-0016) respectively.

[Figure 273-1] Figure 273-1 includes diagrams showing the cytotoxic activity of C-terminally Lys-added CDR-modified hybrid-type and CDR-modified hybrid-type anti-GPRC5D-anti-CD3 bispecific molecules. A and B are diagrams showing the cytocidal activity of C5D-0004 and C5D-0014 respectively.

[Figure 273-2] Figure 273-2 includes diagrams showing the cytotoxic activity of C-terminally Lys-added CDR-modified hybrid-type and CDR-modified hybrid-type anti-GPRC5D-anti-CD3 bispecific molecules. C and D are diagrams showing the cytocidal activity of C5D-0005 and C5D-0015 respectively.

[Figure 273-3] Figure 273-3 includes diagrams showing the cytotoxic activity of C-terminally Lys-added CDR-modified hybrid-type and CDR-modified hybrid-type anti-GPRC5D-anti-CD3 bispecific molecules. E and F are diagrams showing the cytocidal activity of C5D-0006 and C5D-0016 respectively.

[Figure 274] Figure 274 is a diagram showing the anti-tumor activity of the CDR-modified hybrid-type anti-GPRC5D-anti-CD3 bispecific molecule in a human PBMC and cancer cells co-grafting model.

[Figure 275] Figure 275 includes diagrams showing the anti-tumor activity of the C-terminally Lys-added CDR-modified hybrid-type and CDR-modified hybrid-type anti-GPRC5D-anti-CD3 bispecific molecules in a human PBMC-migration model. A and B are diagrams showing the tumor regression activity of C5D-0004 and C5D-0014 respectively.

[Figure 276] Figure 276 shows the amino acid sequence of the CDR-modified heavy chain CDR2 (SEQ ID NO: 238).

[Figure 277] Figure 277 shows the amino acid sequence of the CDR-modified light chain CDR2 (SEQ ID NO: 239).

[Figure 278] Figure 278 shows the amino acid sequence of the CDR-modified heavy chain variable region of C3E-7034 (SEQ ID NO: 240).

[Figure 279] Figure 279 shows the amino acid sequence of the CDR-modified light chain variable region of C3E-7034 (SEQ ID NO: 241).

[Figure 280] Figure 280 shows the amino acid sequence of the CDR-modified light chain variable region of C3E-7035 (SEQ ID NO: 242).

[Figure 281] Figure 281 shows the amino acid sequence of the CDR-modified light chain variable region of C3E-7036 (SEQ ID NO: 243).

[Figure 282] Figure 282 shows the amino acid sequence of C3E-7078 (1 to 269). VH (2 to 119), VL (135 to 243), and FLAG-His tag(244 to 269) (SEQ ID NO: 244).

[Figure 283] Figure 283 shows the amino acid sequence of C3E-7085 (1 to 267). VH (2 to 119), VL (135 to 241), and FLAG-His tag (242 to 267) (SEQ ID NO: 245).

[Figure 284] Figure 284 shows the amino acid sequence of C3E-7086 (1 to 269). VH (2 to 119), VL (135 to 243), and FLAG-His tag (244 to 269) (SEQ ID NO: 246).

[Figure 285] Figure 285 shows the amino acid sequence of C3E-7087 (1 to 269). VH (2 to 119), VL (135 to 243), and FLAG-His tag (244 to 269) (SEQ ID NO: 247).

[Figure 286] Figure 286 shows the amino acid sequence of C3E-7088 (1 to 269). VH (2 to 119), VL (135 to 243), and FLAG-His tag (244 to 269) (SEQ ID NO: 248).

[Figure 287] Figure 287 shows the amino acid sequence of C3E-7089 (1 to 269). VH (2 to 119), VL (135 to 243), and FLAG-His tag (244 to 269) (SEQ ID NO: 249).

[Figure 288] Figure 288 shows the amino acid sequence of C3E-7090 (1 to 269). VH (2 to 119), VL (135 to 243), and FLAG-His tag (244 to 269) (SEQ ID NO: 250).

[Figure 289] Figure 289 shows the amino acid sequence of C3E-7091 (1 to 269). VH (2 to 119), VL (135 to 243), and FLAG-His tag (244 to 269) (SEQ ID NO: 251).

[Figure 290] Figure 290 shows the amino acid sequence of C3E-7092 (1 to 269). VH (2 to 119), VL (135 to 243), and FLAG-His tag (244 to 269) (SEQ ID NO: 252).

[Figure 291] Figure 291 shows the amino acid sequence of C3E-7093 (1 to 269). VH (2 to 119), VL(135 to 243), and FLAG-His tag (244 to 269) (SEQ ID NO: 253).

[Figure 292] Figure 292 shows the amino acid sequence of C3E-7094 (1 to 269). VH (2 to 119), VL (135 to 243), and FLAG-His tag (244 to 269) (SEQ ID NO: 254).

[Figure 293] Figure 293 shows the amino acid sequence of C3E-7095 (1 to 269). VH (2 to 119), VL (135 to 243), and FLAG-His tag (244 to 269) (SEQ ID NO: 255).

[Description of Embodiments]

1. Definitions

In the present invention, the term "gene" means a nucleotide including a nucleotide sequence encoding the amino acids of a protein, or its complementary strand. The meaning of "gene" includes, for example, a polynucleotide, an oligonucleotide, DNA, mRNA, cDNA, and cRNA as the polynucleotide including a nucleotide sequence encoding the amino acids of a protein, or its complementary strand. Such a gene is a single-stranded, double-stranded, or triple- or more stranded nucleotide. The meaning of "gene" includes an association of DNA and RNA strands, a mixture of ribonucleotides (RNAs) and deoxyribonucleotides (DNAs) on one nucleotide strand, and a double-stranded or triple- or more stranded nucleotide comprising such a nucleotide strand. In the present invention, base sequence has the same meaning as nucleotide sequence.

In the present invention, the terms "polynucleotide," "nucleic acid," and "nucleic acid molecule" have the same meaning, and the meaning of "polynucleotide" also includes, for example, DNA, RNA, a probe, an oligonucleotide, and a primer. Such a polynucleotide is a single-stranded, double-stranded, or triple- or more stranded polynucleotide. The meaning of "polynucleotide" also includes an association of DNA and RNA strands, a mixture of ribonucleotides (RNAs) and deoxyribonucleotides (DNAs) on one polynucleotide strand, and an association of two strands or three or more strands comprising such a polynucleotide strand.

In the present invention, the terms "polypeptide", "peptide", and "protein" have the same meaning.

In the present invention, the term "antigen" is used to mean "immunogen".

In the present invention, the term "cell" also includes, for example, various cells derived from individual animals, subcultured cells, primary cultured cells, cell lines, recombinant cells, and microbial cells.

In the present invention, the term "antibody" has the same meaning as immunoglobulin. However, the "antibody" used for the anti-GPRC5D antibody of the present invention or the anti-CD3 antibody of the present invention means an immunoglobulin having constant and variable regions. The antibody is not particularly limited and may be a natural immunoglobulin or may be an immunoglobulin produced by partial or complete synthesis. The anti-GPRC5D antibody and/or the anti-CD3 antibody of the present invention is comprised as a part of the "molecule" described later.

The basic structure of a quaternary antibody is constituted by two identical light (L) chains and two identical heavy (H) chains. Each light chain is linked to the heavy chain by one covalent disulfide bond. The two heavy chains are linked to each other by one or a plurality of disulfide bonds according to the isotypes of the heavy chains. Each of the light and heavy chains has regularly spaced intrachain disulfide bonds. Each of the heavy and light chains contains a constant region which exhibits a very high degree of amino acid sequence similarity and a variable region which exhibits a low degree of amino acid sequence similarity. The light chain has a variable region (VL) at the amino terminus followed by a constant region (CL). The heavy chain has a variable region (VH) at the amino terminus followed by three constant regions (CHI, CH2, and CH3). VL and VH are paired with each other, and CL is aligned with the first constant region (CH1) of the heavy chain. The pair of VL and VH forms a single antigen-binding site.

Fab is composed of heavy chain CH1 followed by VH, and light chain CL followed by VL. VH and VL each contain complementarity determining regions (CDRs).

Fc is constituted by the carboxyl-terminal regions of the heavy chain constant regions and is a dimer containing CH2 and CH3. The Fc of the present invention may be Fc having a natural sequence (referred to as natural form Fc) or may be a mutated form of Fc in which a mutation has been applied to the natural sequence (referred to as an Fc mutation).

Examples of the Fc mutation include but are not limited to: a modified Fc region contained in a heteromultimer (comprising a heterodimeric Fc region) having increased stability disclosed in WO2013/063702; Fc comprising the CH3 domain of an immunoglobulin derived from an IgG antibody having "protuberance" and "cavity" contained in the heteromultimer disclosed in WO96/27011; Fc comprising CH3 domain contained in a heterodimer which is electrostatically favorable, achieved by replacing one or more amino acid residues with a charged amino acid disclosed in WO2009/089004; heterodimeric Fc regions contained in a heterodimer using a conformational variant and/or pI (Isoelectric point) variant disclosed in WO2014/110601; or heterodimeric Fc comprising CH3 domain comprising a modification to eradicate or reduce that binds to protein A disclosed in WO2010/151792.

The variable region is composed of regions, called hypervariable regions (HVRs), having extreme variability, and relatively invariable regions, called framework regions (FRs), interrupted by the hypervariable regions. The natural heavy and light chain variable regions each contain four FRs connected by three hypervariable regions. The hypervariable regions of each chain are kept in close proximity together with the hypervariable regions of another chain by FRs and contribute to the formation of an antigen-binding site in the antibody.

The heavy and light chains of an antibody molecule are known to each have three complementarity determining regions (CDRs). The complementarity determining regions are also called hypervariable domains. These regions are located in the variable regions of the antibody heavy and light chains. These sites have a particularly highly variable primary structure and are usually separated at three positions on the respective primary structures of heavy and light chain polypeptide strands. In the present invention, the complementarity determining regions of the antibody are referred to as heavy chain CDR1 (CDRH1), heavy chain CDR2 (CDRH2), and heavy chain CDR3 (CDRH3) from the amino terminus of the heavy chain amino acid sequence for the complementarity determining regions of the heavy chain, and as light chain CDR1 (CDRL1), light chain CDR2 (CDRL2), and light chain CDR3 (CDRL3) from the amino terminus of the light chain amino acid sequence for the complementarity determining regions of the light chain. These sites are proximal to each other on the three-dimensional structure and determine specificity for the antigen to be bound.

In the present invention, the positions and lengths of CDRs were determined according to the definition of IMGT (Developmental and Comparative Immunology 27 (2003) 55-77).

Framework regions (FRs) are variable regions except for the CDR residues. Each variable region generally has four FRs: FR1, FR2, FR3, and FR4. Heavy and light chain FRs are referred to as FRH1, FRH2, FRH3, and FRH4, and FRL1, FRL2, FRL3, and FRL4, respectively.

The CDRs and the FRs contained in the heavy and light chains are positioned as FRH1-CDRH1-FRH2-CDRH2-FRH3-CDRH3-FRH4 and FRL1-CDRL1-FRL2-CDRL2-FRL3-CDRL3-FRL4 in this order from the amino terminus toward the carboxyl terminus.

The positions of CDRs and FRs can also be determined according to various definitions well known in the art, for example, the definitions by Kabat, Chothia, AbM, contact, etc., with the exception of IMGT.

In the present invention, the term "antigen-binding fragment of the antibody" means a partial fragment of an antibody having binding activity with an antigen, constituted by heavy and light chain variable regions. Examples of the "antigen-binding fragment of the antibody" can include but are not limited to antigen-binding fragments such as Fab, $F(ab')_2$, scFv, Fab', Fv, and single-domain antibodies (sdAb). Such an antigen-binding fragment of the antibody may be obtained by treating a full-length molecule of the antibody protein with an enzyme such as papain or pepsin, or may be a recombinant protein produced in an appropriate host cell using a recombinant gene.

In the present invention, the "site" to which an antibody binds, i.e., the "site" recognized by an antibody, means a partial peptide or partial higher order structure on an antigen bound or recognized by the antibody.

In the present invention, such a site is also referred to as an epitope or an antibody binding site.

In the present invention, the term "antibody mutant" means a polypeptide that has an amino acid sequence derived from the amino acid sequence of the original antibody by the substitution, deletion, and/or addition (the addition includes insertion) (hereinafter, collectively referred to as a "mutation") of amino acid(s) and binds to the antigen. The number of mutated amino acids in such an antibody mutant is 1 to 2, 3, 4, 5, 6, 7, 8, 9, 10, 12, 15, 20, 25, 30, 40, or 50. Such an antibody mutant is also encompassed by the "antibody" of the present invention.

In the present invention, the term "several" in "1 to several" refers to 2 to 10.

In the present specification, the term "molecule" is a molecule comprising the aforementioned antibody or antigen-binding fragment of the antibody and may be a molecule which is multispecific formed by an antibody or a plurality of antigen-binding fragments derived therefrom.

In the present specification, the terms "molecule which is multispecific" and "multispecific molecule" have the same meaning. Such a multispecific molecule is not particularly limited as long as the molecule is capable of binding to a plurality of epitopes different from each other on one molecule, and/or epitopes different from each other on two or more molecules. The molecule which is multispecific also comprises an antibody comprising heavy chain variable (VH) and light chain variable (VL) regions. Examples of such a multispecific molecule include, but are not limited to, a full-length antibody molecule having two or more different types of heavy chains and light chains, i.e., an IgG-type multispecific molecule, and a molecule consisting of two or more types of antigen-binding fragments having VLs and VHs, i.e., a molecule derived by a combination of Fab, Fab', Fv, scFv, sdAb, etc. (i.e., tandem scFv, diabodies, single chain diabodies, and triabodies). In addition, a molecule formed by genetically or chemically linking a protein having an antigen that binds to an antigen-binding fragment without having an immunoglobulin skeleton is also included in the multispecific molecule.

Examples of activities or properties exerted by the anti-CD3 antibody of the present invention or antigen-binding fragment of the antibody, or the multispecific molecule of the present invention, can include biological activities and physicochemical properties and can specifically include various biological activities, binding activity against an antigen or an epitope, stability during production or storage, and thermal stability.

In the present invention, the phrase "hybridizing under stringent conditions" means hybridization under conditions involving hybridization at 65°C in a solution containing 5 × SSC, followed by washing at 65°C for 20 minutes in an aqueous solution containing 2 × SSC-0.1% SDS, at 65°C for 20 minutes in an aqueous solution containing 0.5 × SSC-0.1% SDS, and at 65°C for 20 minutes in an aqueous solution containing 0.2 × SSC-0.1% SDS, or hybridization under conditions equivalent thereto. SSC means an aqueous solution of 150 mM NaCl-15 mM sodium citrate, and n × SSC means SSC with an n-fold concentration.

In the present invention, the term "cytotoxicity" refers to a pathological change brought about to cells in some kind of way, and means not only direct trauma, but every structural or functional damage to cells, including DNA cleavage, formation of base dimers, chromosomal breakage, damage on mitotic apparatus, and reduction in the various enzyme activity.

In the present invention, the term "cytotoxic activity" means activity that causes the cell damage mentioned above.

In the present invention, the term "antibody dependent cellular cytotoxicity activity", also called "ADCC activity", means the effect or activity of damaging target cells such as tumor cells by NK cells via antibodies.

In the present invention, the term "cytotoxic activity by the redirection of T cells" means that cell damage is caused via a multispecific molecule comprising an anti-target antigen antibody such as an anti-tumor antigen and the anti-CD3 antibody. Ideally, the term means that the anti-tumor antigen antibody binds to target tumor cells while the anti-CD3 antibody binds to T cells so that the target tumor cells and the T cells come close to each other to induce T cell activation-mediated cell damage. The molecule can be contained in a pharmaceutical composition.

In the present invention, the terms "naturally occurring amino acid" and "naturally occurring amino acid residue" mean Ala (A), Arg (R), Asn (N), Asp (D), Cys (C), Gln (Q), Glu (E), Gly (G), His (H), Ile (I), Leu (L), Lys (K), Met (M), Phe (F), Pro (P), Ser (S), Thr (T), Trp (W), Tyr (Y), and Val (V) and their residues, and are also referred to as a "natural amino acid" or a "natural amino acid residue".

2. Antigenic protein

2-1. GPRC5D antigen

In the present invention, the term "GPRC5D" has the same meaning as a GPRC5D protein.

GPRC5D is classified into group 5 of the G-protein coupled receptor family C and is one of the human GPCR proteins newly found by the homology search of the EST database using the amino acid sequences of a series of human GPCRs (Non Patent Literature 1). This protein has been registered under GenBank deposition Nos: AF209923, NM_018654, and NP_0611124. However, the physiological functions or physiological ligand of GPRC5D, the subtype of G protein ($\alpha$ subunit) to be coupled therewith, etc. have not yet been revealed.

2-2. CD3 antigen

In the present invention, the term "CD3" has the same meaning as CD3 protein.

CD3 is expressed, as a portion of a multimolecular T cell receptor complex, on T cells and is a complex of 5 types of polypeptides ($\gamma$, $\delta$, $\varepsilon$, $\zeta$, and $\eta$ chains; molecular weights: 25000 to 28000, 21000, 20000, 16000, and 22000, respectively).

Examples of the CD3 complex include $\gamma$, $\delta$, $\varepsilon$, $\zeta$, and $\eta$ chains. These are also called subunits. Anti-CD3 antibodies bind to T cells to induce T cell activation-mediated cell damage. Many anti-CD3 antibodies bind to CD3$\varepsilon$.

The nucleotide sequence of a cDNA encoding human CD3$\varepsilon$ is registered in GenBank under Accession No. NM_000733.3. The nucleotide sequence of a cDNA encoding cynomolgus monkey CD3 is registered in GenBank under Accession No. NM_001283615.1. The amino acid sequence of human CD3$\varepsilon$ is described in SEQ ID NO: 189 of the Sequence Listing.

2-3. Preparation of antigenic protein

Each aforementioned antigenic protein GPRC5D or CD3 (hereinafter, GPRC5D and CD3 are also collectively referred to as the antigenic protein) used in the present invention can be prepared by purification and isolation from animal tissues (including body fluids), cells derived from the tissues, or cultures of the cells, gene recombination, *in vitro* translation, chemical synthesis, etc.

The cDNA of the antigenic protein can be obtained by, for example, a so-called PCR method which performs a polymerase chain reaction (hereinafter, referred to as "PCR") (Saiki, R.K., et al., Science (1988) 239, 487-489) using a cDNA library of organs expressing the mRNA of the antigenic protein as a template and using primers capable of specifically amplifying the cDNA of the antigenic protein.

A polynucleotide consisting of a nucleotide sequence complementary to the nucleotide sequence encoding the antigenic protein expressed in a human or a rat hybridizing under stringent conditions with a polynucleotide encoding a protein having biological activity equivalent to the antigenic protein is also included in the cDNA of the antigenic protein.

In addition, splice variants transcribed from the gene loci of the antigenic protein expressed in a human or a rat, or polynucleotides hybridizing under stringent conditions and encoding a protein having biological activities equivalent to the antigenic protein are also included in the cDNA of the antigenic protein.

A nucleotide sequence encoding a protein that consists of an amino acid sequence derived from the amino acid sequence of the human or rat antigenic protein or the amino acid sequence thereof except for a signal sequence by the substitution, deletion, or addition of 1 to several amino acid(s) and has biological activities equivalent to the antigenic protein is also included in the nucleotide sequence of the antigenic protein gene.

A protein that consists of an amino acid sequence encoded by a splicing variant transcribed from the gene loci of the human or rat antigenic protein or an amino acid sequence derived from the amino acid sequence by the substitution, deletion, or addition of 1 to several amino acid(s) and has biological activities equivalent to the antigenic protein is also included in the antigenic protein.

2-4 Binding specificity to antigenic protein

The anti-GPRC5D antibody of the present invention or antigen-binding fragment thereof, etc. recognizes human GPRC5D. In other words, the anti-GPRC5D antibody of the present invention or antigen-binding fragment thereof, etc. binds to a GPRC5D antigen and preferably binds to human GPRC5D and monkey GPRC5D, more preferably human GPRC5D and cynomolgus monkey GPRC5D. The humanized anti-GPRC5D antibody h2B1 antibody and the human antibody C3048 of the present invention described later further bind to cynomolgus monkey GPRC5D in addition to human GPRC5D.

The anti-CD3 antibody or antigen-binding fragment thereof, etc. contained in the multispecific molecule of the present invention recognizes a CD3 antigen, i.e., binds thereto. The anti-CD3 antibody or antigen-binding fragment thereof, etc. contained in the multispecific molecule of the present invention preferably binds to human CD3, monkey CD3, and the like and more preferably binds to human CD3 and cynomolgus monkey CD3.

The antibody that binds to the human and cynomolgus monkey antigenic proteins or the antigen-binding fragment thereof can be subjected to various tests on efficacy or safety using primates, particularly, cynomolgus monkeys, useful for the nonclinical development (preclinical development) of pharmaceutical products, and is thus preferred.

Meanwhile, preferably, the anti-GPRC5D antibody of the present invention does not bind to mouse and/or rat GPRC5D. Therefore, for example, various assays or immunohistochemical tests using human GPRC5D gene-transfected mouse cells, tissues, or individuals (including transgenic animals, knockout animals, and knock-in animals) and the antibody or the multispecific molecule of the present invention, etc. can be carried out without being influenced by GPRC5D of the host mice and/or rats. Thus, the antibody or the multispecific molecule of the present invention, etc. is preferred for the research and nonclinical development, using mice, of drugs, animal drugs, or diagnostic drugs, etc., including the antibody or the multispecific molecule of the present invention, etc.

Likewise, preferably, the anti-CD3 antibody contained in the multispecific molecule of the present invention does not bind to mouse and/or rat CD3. Therefore, for example, various assays or immunohistochemical tests using human CD3 gene-transfected mouse cells, tissues, or individuals (including transgenic animals, knockout animals, and knock-in animals) and the antibody or the multispecific molecule of the present invention, etc. can be carried out without being influenced by CD3 of the host mice and/or rats. Thus, the antibody or the multispecific molecule of the present invention, etc. is preferred for the research and nonclinical development, using mice, of drugs, animal drugs, or diagnostic drugs, etc., containing the antibody or the multispecific molecule of the present invention, etc.

In the present invention, the term "recognition", i.e., "binding", means binding which is not non-specific adsorption. Examples of criteria for determination of whether recognition is achieved or not, i.e., binding is achieved or not, can include a dissociation constant (hereinafter, referred to as "$K_D$"). Preferably, the antibody, etc. of the present invention has a $K_D$ value of $1 \times 10^{-5}$ M or lower, $5 \times 10^{-6}$ M or lower, $2 \times 10^{-6}$ M or lower, or $1 \times 10^{-6}$ M or lower for CD3.

In the present invention, the binding of the antibody to the antigen can be assayed or determined using a biomolecular interaction analysis system (e.g., SPR or BLI), ELISA, or RIA, or the like. The binding of the antibody to the antigen expressed on cell surface can be assayed by flow cytometry or the like.

The SPR (surface plasmon resonance analysis) method is used as an analysis approach of determining a dissociation constant ($K_D$ value), etc. as an index for affinity by measuring an association rate constant (Ka value) and a dissociation rate constant (Kd value) by kinetic analysis. Examples of equipment used in the SPR analysis can include BIAcore(TM) (manufactured by GE Healthcare Bio-Sciences Corp.), ProteOn(TM) (manufactured by Bio-Rad Laboratories, Inc.), SPR-Navi(TM) (manufactured by BioNavis Oy Ltd.), Spreeta(TM) (manufactured by Texas Instruments Inc.), SPRi-Plex II(TM) (manufactured by Horiba, Ltd.), and Autolab SPR(TM) (manufactured by Metrohm Japan Ltd.).

BLI (biolayer interferometry) is a method which involves measuring biomolecular interaction using biolayer interference. Examples of equipment used in BLI interaction analysis include Octet system (manufactured by Pall ForteBio Corp.).

The ELISA is a method which involves capturing an antigen or an antibody of interest contained in a sample solution using a specific antibody or antigen, while detecting and quantifying the antigen or antibody of interest through the use of enzymatic reaction. An enzyme-labeled antigen or antibody is incorporated into the reaction system, and the enzyme activity is detected. For the enzyme activity detection, a substrate whose absorption spectrum is changed by the reaction is used, and the absorption spectrum is digitized by absorbance measurement.

The Cell-ELISA is a method which involves capturing an analyte on cell surface on a cell basis, while detecting and quantifying the analyte through the use of enzymatic reaction.

The RIA (radio immunoassay) can quantify an antibody by labeling the antibody with a radioactive material and measuring radioactivity from the antibody.

The flow cytometry is an approach of optically analyzing individual cells by dispersing cells into a fluid and flowing a thin stream of the fluid. A fluorescent dye-labeled antibody binds to a cell surface antigen through antigen-antibody reaction, and fluorescence intensity from the labeled antibody bound with cells is measured to quantify the antigen binding activity of the antibody.

## 3. Anti-GPRC5D antibody

### 3-1. Type of anti-GPRC5D antibody

The anti-GPRC5D antibody of the present invention may be either a monoclonal or polyclonal antibody. Examples of the polyclonal antibody can include a mixture of a plurality of antibodies differing in a portion or the whole of CDR sets. Examples of the monoclonal antibody can include non-human animal-derived antibodies (non-human animal antibodies), human antibodies, chimeric antibodies, and humanized antibodies, etc.

Examples of the non-human animal antibody can include antibodies derived from vertebrates such as mammals and birds. Examples of the mammal-derived antibody can include rodent-derived antibodies such as mouse antibodies and rat antibodies. Examples of the bird-derived antibody can include chicken antibodies. Examples of the anti-human GPRC5D rat monoclonal antibody can include 2A4, 2B1 and 7B4 (Example 1) of the present invention.

The amino acid sequence of the heavy chain variable region of 2A4 is represented by SEQ ID NO: 5 of the Sequence Listing. The amino acid sequence of the heavy chain variable region of 2B1 is represented by SEQ ID NO: 7 of the Sequence Listing. The amino acid sequence of the heavy chain variable region of 7B4 is represented by SEQ ID NO: 9 of the Sequence Listing.

The amino acid sequence of the light chain variable region of 2A4 is represented by SEQ ID NO: 12 of the Sequence Listing. The amino acid sequence of the light chain variable region of 2B1 is represented by SEQ ID NO: 14 of the Sequence Listing. The amino acid sequence of the light chain variable region of 7B4 is represented by SEQ ID NO: 16 of the Sequence Listing.

2A4, 2B1, and 7B4 have ADCC activity (Example 2).

Examples of the chimeric antibody can include an antibody comprising non-human animal antibody-derived variable regions bound with human antibody (human immunoglobulin) constant regions.

Examples of the chimeric antibody derived from the rat anti-human GPRC5D antibody 2A4 can include an antibody consisting of a light chain comprising a light chain variable region consisting of amino acid residues 21 to 127 of SEQ ID NO: 22 and a heavy chain comprising a heavy chain variable region consisting of amino acid residues 20 to 141 of SEQ ID NO: 26. One example of such a 2A4-derived chimeric antibody can include an antibody consisting of a light chain consisting of amino acid residues 21 to 234 of SEQ ID NO: 22 and a heavy chain consisting of amino acid residues 20 to 471 of SEQ ID NO: 26. In the present specification, the antibody is referred to as c2A4.

Examples of the chimeric antibody derived from the rat anti-human GPRC5D antibody 2B1 can include an antibody consisting of a light chain comprising a light chain variable region consisting of amino acid residues 21 to 127 of SEQ ID NO: 30 and a heavy chain comprising a heavy chain variable region consisting of amino acid residues 20 to 142 of SEQ ID NO: 34. One example of such a 2B1-derived chimeric antibody can include an antibody consisting of a light chain consisting of amino acid residues 21 to 234 of SEQ ID NO: 30 and a heavy chain consisting of amino acid residues 20 to 472 of SEQ ID NO: 34. In the present specification, the antibody is referred to as c2B1.

Examples of the chimeric antibody derived from the rat anti-human GPRC5D antibody 7B4 can include an antibody consisting of a light chain comprising a light chain variable region consisting of amino acid residues 21 to 127 of SEQ ID NO: 38 and a heavy chain comprising a heavy chain variable region consisting of amino acid residues 20 to 142 of SEQ ID NO: 42. One example of such a 7B4-derived chimeric antibody can include an antibody consisting of a light chain consisting of amino acid residues 21 to 233 of SEQ ID NO: 38 and a heavy chain consisting of amino acid residues 20 to 472 of SEQ ID NO: 42. In the present specification, the antibody is referred to as c7B4.

Examples of the humanized antibody can include a human-derived antibody containing only complementarity determining regions (CDRs) (Nature (1986) 321, 522-525), a human antibody grafted with the CDR sequences and with some amino acid residues of framework regions by CDR grafting (International Publication No. WO1990/007861)), and an antibody having human antibody amino acid(s) replaced for one or two or more non-human animal antibody-derived amino acid(s) in any of these humanized antibodies.

The humanized antibody derived from each chimeric antibody mentioned above maintains all of 6 CDR sequences derived from the chimeric antibody mentioned above and by extension, the rat antibody, and has ADCC activity. Thus, examples of the antibody that maintains all of 6 CDR sequences shown below include rat antibodies, chimeric antibodies, and humanized antibodies.

The heavy chain variable region of the humanized antibody derived from 2A4 mentioned above maintains
heavy chain CDR1 consisting of an amino acid sequence represented by SEQ ID NO: 45 (GYTFTSYY),
heavy chain CDR2 consisting of an amino acid sequence represented by SEQ ID NO: 46 (VYPGYGGT), and
heavy chain CDR3 consisting of an amino acid sequence represented by SEQ ID NO: 47 (ARRKGIIRGPGYFDY).
The light chain variable region thereof maintains
light chain CDR1 consisting of an amino acid sequence represented by SEQ ID NO: 54 (EGISNS),
light chain CDR2 consisting of an amino acid sequence represented by SEQ ID NO: 55 (GAS), and
light chain CDR3 consisting of an amino acid sequence represented by SEQ ID NO: 56 (QQGYKYPPT).

The heavy chain variable region of the humanized antibody derived from 2B1 mentioned above maintains
heavy chain CDR1 consisting of an amino acid sequence represented by SEQ ID NO: 48 (GFSLNTYDMG),
heavy chain CDR2 consisting of an amino acid sequence represented by SEQ ID NO: 49 (IWWDDDK), and
heavy chain CDR3 consisting of an amino acid sequence represented by SEQ ID NO: 50 (ARIETVRVSRKGFAH).
The light chain variable region thereof maintains
light chain CDR1 consisting of an amino acid sequence represented by SEQ ID NO: 57 (QSVGIN),

light chain CDR2 consisting of an amino acid sequence represented by SEQ ID NO: 58 (GAS), and
light chain CDR3 consisting of an amino acid sequence represented by SEQ ID NO: 59 (LQHGSIPPT).

The heavy chain variable region of the humanized antibody derived from 7B4 mentioned above maintains
heavy chain CDR1 consisting of an amino acid sequence represented by SEQ ID NO: 51 (GYTITSGYD),
heavy chain CDR2 consisting of an amino acid sequence represented by SEQ ID NO: 52 (MSYRGST), and
heavy chain CDR3 consisting of an amino acid sequence represented by SEQ ID NO: 53 (ALTRTYWYNYYYVLDA).
The light chain variable region thereof maintains
light chain CDR1 consisting of an amino acid sequence represented by SEQ ID NO: 60 (QNINKY),
light chain CDR2 consisting of an amino acid sequence represented by SEQ ID NO: 61 (NTN), and
light chain CDR3 consisting of an amino acid sequence represented by SEQ ID NO: 62 (LQRNSWYT).

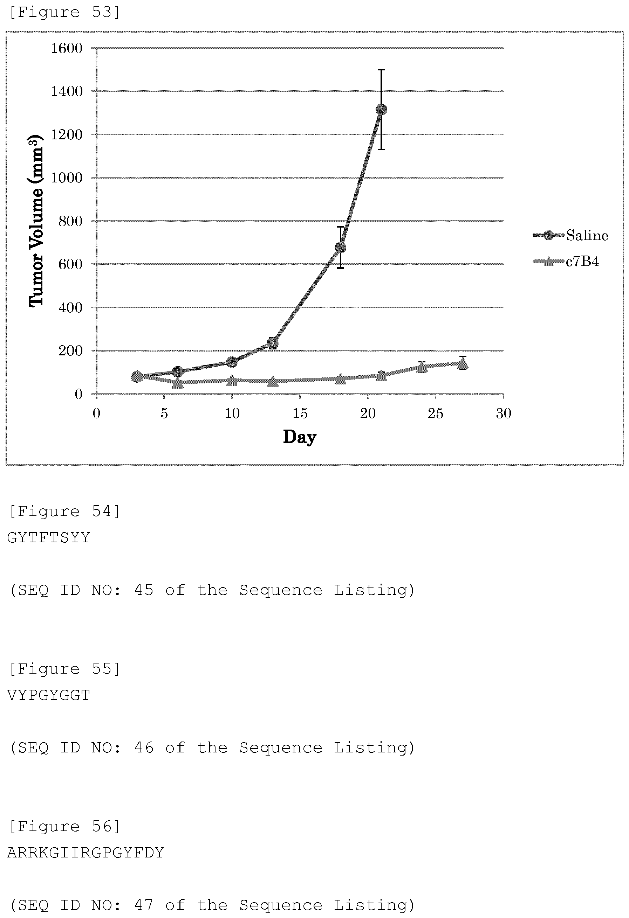

The amino acid sequences of the CDRs mentioned above are also described in Figures 54 to 71.

In the present invention, the positions and lengths of CDRs were determined according to the definition of IMGT (Developmental and Comparative Immunology 27 (2003) 55-77).

Preferred examples of the humanized antibody can include an antibody comprising
a light chain variable region comprising any one amino acid sequence represented by
amino acid residues 21 to 127 of an amino acid sequence represented by SEQ ID NO: 64,
amino acid residues 21 to 127 of an amino acid sequence represented by SEQ ID NO: 66,
amino acid residues 21 to 127 of an amino acid sequence represented by SEQ ID NO: 68,
amino acid residues 21 to 127 of an amino acid sequence represented by SEQ ID NO: 70, and
amino acid residues 21 to 127 of an amino acid sequence represented by SEQ ID NO: 72, and
a heavy chain variable region comprising any one amino acid sequence represented by
amino acid residues 21 to 142 of an amino acid sequence represented by SEQ ID NO: 74,
amino acid residues 21 to 142 of an amino acid sequence represented by SEQ ID NO: 76,
amino acid residues 21 to 142 of an amino acid sequence represented by SEQ ID NO: 78, and
amino acid residues 21 to 142 of an amino acid sequence represented by SEQ ID NO: 80.
Alternative preferred examples of the humanized antibody can include an antibody comprising
a light chain variable region comprising an amino acid sequence represented by
amino acid residues 21 to 126 of an amino acid sequence represented by SEQ ID NO: 82, or
amino acid residues 21 to 126 of an amino acid sequence represented by SEQ ID NO: 84, and
a heavy chain variable region comprising any one amino acid sequence represented by
amino acid residues 20 to 142 of an amino acid sequence represented by SEQ ID NO: 86,
amino acid residues 20 to 142 of an amino acid sequence represented by SEQ ID NO: 88,
amino acid residues 20 to 142 of an amino acid sequence represented by SEQ ID NO: 90, and
amino acid residues 20 to 142 of an amino acid sequence represented by SEQ ID NO: 92.

Specific preferred examples of the humanized antibody can include an antibody comprising any one combination of a heavy chain variable region and a light chain variable region of

- a heavy chain variable region comprising an amino acid sequence represented by amino acid residues 21 to 142 of the amino acid sequence represented by SEQ ID NO: 74, and a light chain variable region comprising an amino acid sequence represented by amino acid residues 21 to 127 of the amino acid sequence represented by SEQ ID NO: 64,
- a heavy chain variable region comprising an amino acid sequence represented by amino acid residues 21 to 142 of the amino acid sequence represented by SEQ ID NO: 74, and a light chain variable region comprising an amino acid sequence represented by amino acid residues 21 to 127 of the amino acid sequence represented by SEQ ID NO: 66,
- a heavy chain variable region comprising an amino acid sequence represented by amino acid residues 21 to 142 of the amino acid sequence represented by SEQ ID NO: 76, and a light chain variable region comprising an amino acid sequence represented by amino acid residues 21 to 127 of the amino acid sequence represented by SEQ ID NO: 66,
- a heavy chain variable region comprising an amino acid sequence represented by amino acid residues 21 to 142 of the amino acid sequence represented by SEQ ID NO: 76, and a light chain variable region comprising an amino acid sequence represented by amino acid residues 21 to 127 of the amino acid sequence represented by SEQ ID NO: 68,
- a heavy chain variable region comprising an amino acid sequence represented by amino acid residues 21 to 142 of the amino acid sequence represented by SEQ ID NO: 76, and a light chain variable region comprising an amino acid sequence represented by amino acid residues 21 to 127 of the amino acid sequence represented by SEQ ID NO: 70,
- a heavy chain variable region comprising an amino acid sequence represented by amino acid residues 21 to 142 of the amino acid sequence represented by SEQ ID NO: 76, and a light chain variable region comprising an amino acid sequence represented by amino acid residues 21 to 127 of the amino acid sequence represented by SEQ ID NO: 72,

- a heavy chain variable region comprising an amino acid sequence represented by amino acid residues 21 to 142 of the amino acid sequence represented by SEQ ID NO: 78, and a light chain variable region comprising an amino acid sequence represented by amino acid residues 21 to 127 of the amino acid sequence represented by SEQ ID NO: 68,
- a heavy chain variable region comprising an amino acid sequence represented by amino acid residues 21 to 142 of the amino acid sequence represented by SEQ ID NO: 78, and a light chain variable region comprising an amino acid sequence represented by amino acid residues 21 to 127 of the amino acid sequence represented by SEQ ID NO: 70,
- a heavy chain variable region comprising an amino acid sequence represented by amino acid residues 21 to 142 of the amino acid sequence represented by SEQ ID NO: 78, and a light chain variable region comprising an amino acid sequence represented by amino acid residues 21 to 127 of the amino acid sequence represented by SEQ ID NO: 72,
- a heavy chain variable region comprising an amino acid sequence represented by amino acid residues 21 to 142 of the amino acid sequence represented by SEQ ID NO: 80, and a light chain variable region comprising an amino acid sequence represented by amino acid residues 21 to 127 of the amino acid sequence represented by SEQ ID NO: 64,
- a heavy chain variable region comprising an amino acid sequence represented by amino acid residues 21 to 142 of the amino acid sequence represented by SEQ ID NO: 80, and a light chain variable region comprising an amino acid sequence represented by amino acid residues 21 to 127 of the amino acid sequence represented by SEQ ID NO: 68,
- a heavy chain variable region comprising an amino acid sequence represented by amino acid residues 21 to 142 of the amino acid sequence represented by SEQ ID NO: 80, and a light chain variable region comprising an amino acid sequence represented by amino acid residues 21 to 127 of the amino acid sequence represented by SEQ ID NO: 70, or
- a heavy chain variable region comprising an amino acid sequence represented by amino acid residues 21 to 142 of the amino acid sequence represented by SEQ ID NO: 80, and a light chain variable region comprising an amino acid sequence represented by amino acid residues 21 to 127 of the amino acid sequence represented by SEQ ID NO: 72.

Alternative specific preferred examples of the humanized antibody can include an antibody comprising any one combination of a heavy chain variable region and a light chain variable region of

- a heavy chain variable region comprising an amino acid sequence represented by amino acid residues 20 to 142 of the amino acid sequence represented by SEQ ID NO: 86, and a light chain variable region comprising an amino acid sequence represented by amino acid residues 21 to 126 of the amino acid sequence represented by SEQ ID NO: 84,
- a heavy chain variable region comprising an amino acid sequence represented by amino acid residues 20 to 142 of the amino acid sequence represented by SEQ ID NO: 88, and a light chain variable region comprising an amino acid sequence represented by amino acid residues 21 to 126 of the amino acid sequence represented by SEQ ID NO: 84,
- a heavy chain variable region comprising an amino acid sequence represented by amino acid residues 20 to 142 of the amino acid sequence represented by SEQ ID NO: 90, and a light chain variable region comprising an amino acid sequence represented by amino acid residues 21 to 126 of the amino acid sequence represented by SEQ ID NO: 82,
- a heavy chain variable region comprising an amino acid sequence represented by amino acid residues 20 to 142 of the amino acid sequence represented by SEQ ID NO: 90, and a light chain variable region comprising an amino acid sequence represented by amino acid residues 21 to 126 of the amino acid sequence represented by SEQ ID NO: 84, or
- a heavy chain variable region comprising an amino acid sequence represented by amino acid residues 20 to 142 of the amino acid sequence represented by SEQ ID NO: 92, and a light chain variable region comprising an amino acid sequence represented by amino acid residues 21 to 126 of the amino acid sequence represented by SEQ ID NO: 82.

More preferred specific examples of the humanized antibody can include an antibody comprising any one combination of a heavy chain and a light chain of

- a heavy chain comprising amino acid residues 20 to 472 of the amino acid sequence represented by SEQ ID NO: 74, and a light chain comprising amino acid residues 21 to 234 of the amino acid sequence represented by SEQ ID NO: 64,
- a heavy chain comprising amino acid residues 20 to 472 of the amino acid sequence represented by SEQ ID NO: 74, and a light chain comprising amino acid residues 21 to 234 of the amino acid sequence represented by SEQ ID NO: 66,
- a heavy chain comprising amino acid residues 20 to 472 of the amino acid sequence represented by SEQ ID NO: 76, and a light chain comprising amino acid residues 21 to 234 of the amino acid sequence represented by SEQ ID NO: 66,
- a heavy chain comprising amino acid residues 20 to 472 of the amino acid sequence represented by SEQ ID NO: 76, and a light chain comprising amino acid residues 21 to 234 of the amino acid sequence represented by SEQ ID NO: 68,
- a heavy chain comprising amino acid residues 20 to 472 of the amino acid sequence represented by SEQ ID NO: 76, and a light chain comprising amino acid residues 21 to 234 of the amino acid sequence represented by SEQ ID NO: 70,
- a heavy chain comprising amino acid residues 20 to 472 of the amino acid sequence represented by SEQ ID NO: 76, and a light chain comprising amino acid residues 21 to 234 of the amino acid sequence represented by SEQ ID NO: 72,
- a heavy chain comprising amino acid residues 20 to 472 of the amino acid sequence represented by SEQ ID NO: 78, and a light chain comprising amino acid residues 21 to 234 of the amino acid sequence represented by SEQ ID NO: 68,
- a heavy chain comprising amino acid residues 20 to 472 of the amino acid sequence represented by SEQ ID NO: 78, and a light chain comprising amino acid residues 21 to 234 of the amino acid sequence represented by SEQ ID NO: 70,
- a heavy chain comprising amino acid residues 20 to 472 of the amino acid sequence represented by SEQ ID NO: 78, and a light chain comprising amino acid residues 21 to 234 of the amino acid sequence represented by SEQ ID NO: 72,
- a heavy chain comprising amino acid residues 20 to 472 of the amino acid sequence represented by SEQ ID NO: 80, and a light chain comprising amino acid residues 21 to 234 of the amino acid sequence represented by SEQ ID NO: 64,
- a heavy chain comprising amino acid residues 20 to 472 of the amino acid sequence represented by SEQ ID NO: 80, and a light chain comprising amino acid residues 21 to 234 of the amino acid sequence represented by SEQ ID NO: 68,
- a heavy chain comprising amino acid residues 20 to 472 of the amino acid sequence represented by SEQ ID NO: 80, and a light chain comprising amino acid residues 21 to 234 of the amino acid sequence represented by SEQ ID NO: 70, and
- a heavy chain comprising amino acid residues 20 to 472 of the amino acid sequence represented by SEQ ID NO: 80, and a light chain comprising amino acid residues 21 to 234 of the amino acid sequence represented by SEQ ID NO: 72.

Alternative more preferred specific examples of the humanized antibody can include an antibody comprising any one combination of a heavy chain and a light chain of

- a heavy chain comprising amino acid residues 20 to 472 of the amino acid sequence represented by SEQ ID NO: 86, and a light chain comprising amino acid residues 21 to 233 of the amino acid sequence represented by SEQ ID NO: 84,
- a heavy chain comprising amino acid residues 20 to 472 of the amino acid sequence represented by SEQ ID NO: 88, and a light chain comprising amino acid residues 21 to 233 of the amino acid sequence represented by SEQ ID NO: 84,
- a heavy chain comprising amino acid residues 20 to 472 of the amino acid sequence represented by SEQ ID NO: 90, and a light chain comprising amino acid residues 21 to 233 of the amino acid sequence represented by SEQ ID NO: 82,
- a heavy chain comprising amino acid residues 20 to 472 of the amino acid sequence represented by SEQ ID NO: 90, and a light chain comprising amino acid residues 21 to 233 of the amino acid sequence represented by SEQ ID NO: 84, or
- a heavy chain comprising amino acid residues 20 to 472 of the amino acid sequence represented by SEQ ID NO: 92, and a light chain comprising amino acid residues 21 to 233 of the amino acid sequence represented by SEQ ID NO: 82.

The rat antibodies, the chimeric antibodies, and the humanized antibodies mentioned above may each contain Fc.

Also, the rat antibodies, the chimeric antibodies, and the humanized antibodies mentioned above may each contain a human immunoglobulin heavy chain constant region.

Further more preferred examples of the humanized antibody can include an antibody comprising the aforementioned heavy chain variable regions and light chain variable regions, preferably the heavy chain variable regions and light chain variable regions of a humanized antibody derived from 2B1, and

i) a heavy chain constant region comprising an amino acid sequence represented by amino acid residues 147 to 475 of the amino acid sequence represented by SEQ ID NO: 199, and a light chain constant region comprising an amino acid sequence represented by amino acid residues 131 to 237 of the amino acid sequence represented by SEQ ID NO: 203,
ii) a heavy chain constant region comprising an amino acid sequence represented by amino acid residues 147 to 475 of the amino acid sequence represented by SEQ ID NO: 201, and a light chain constant region comprising an amino acid sequence represented by amino acid residues 131 to 237 of the amino acid sequence represented by SEQ ID NO: 205,
iii) a heavy chain constant region comprising an amino acid sequence represented by amino acid residues 147 to 475 of the amino acid sequence represented by SEQ ID NO: 215, and a light chain constant region comprising an amino acid sequence represented by amino acid residues 131 to 237 of the amino acid sequence represented by SEQ ID NO: 217,
or
iv) a heavy chain constant region comprising an amino acid sequence represented by amino acid residues 147 to 475 of the amino acid sequence represented by SEQ ID NO: 237, and a light chain constant region comprising an amino acid sequence represented by amino acid residues 131 to 237 of the amino acid sequence represented by SEQ ID NO: 217.

Particularly preferred examples of the humanized antibody include an antibody comprising a heavy chain variable region comprising an amino acid sequence represented by amino acid residues 20 to 142 of the amino acid sequence represented by SEQ ID NO: 76, and a light chain variable region comprising an amino acid sequence represented by amino acid residues 21 to 127 of the amino acid sequence represented by SEQ ID NO: 72, and

i) a heavy chain constant region comprising an amino acid sequence represented by amino acid residues 147 to 475 of the amino acid sequence represented by SEQ ID NO: 199, and a light chain constant region comprising an amino acid sequence represented by amino acid residues 131 to 237 of the amino acid sequence represented by SEQ ID NO: 203,
ii) a heavy chain constant region comprising an amino acid sequence represented by amino acid residues 147 to 475 of the amino acid sequence represented by SEQ ID NO: 201, and a light chain constant region comprising an amino acid sequence represented by amino acid residues 131 to 237 of the amino acid sequence represented by SEQ ID NO: 205,
iii) a heavy chain constant region comprising an amino acid sequence represented by amino acid residues 147 to 475 of the amino acid sequence represented by SEQ ID NO: 215, and a light chain constant region comprising an amino acid sequence represented by amino acid residues 131 to 237 of the amino acid sequence represented by SEQ ID NO: 217,
or
iv) a heavy chain constant region comprising an amino acid sequence represented by amino acid residues 147 to 476 of the amino acid sequence represented by SEQ ID NO: 237, and a light chain constant region comprising an amino acid sequence represented by amino acid residues 131 to 237 of the amino acid sequence represented by SEQ ID NO: 217.

Of these, a more preferable antibody includes an antibody comprising a heavy chain variable region comprising an amino acid sequence represented by amino acid residues 20 to 142 of the amino acid sequence represented by SEQ ID NO: 76, and a light chain variable region comprising an amino acid sequence represented by amino acid residues 21 to 127 of the amino acid sequence represented by SEQ ID NO: 72, and

iii) a heavy chain constant region comprising an amino acid sequence represented by amino acid residues 147 to 475 of the amino acid sequence represented by SEQ ID NO: 215, and a light chain constant region comprising an amino acid sequence represented by amino acid residues 131 to 237 of the amino acid sequence represented by SEQ ID NO: 217,

or
iv) a heavy chain constant region comprising an amino acid sequence represented by amino acid residues 147 to 476 of the amino acid sequence represented by SEQ ID NO: 237, and a light chain constant region comprising an amino acid sequence represented by amino acid residues 131 to 237 of the amino acid sequence represented by SEQ ID NO: 217.

Specific examples of such an antibody can include an antibody comprising
a heavy chain comprising an amino acid sequence represented by amino acid residues 24 to 475 of the amino acid sequence represented by SEQ ID NO: 215, and a light chain comprising an amino acid sequence represented by amino acid residues 24 to 237 of the amino acid sequence represented by SEQ ID NO: 217,
or
a heavy chain comprising an amino acid sequence represented by amino acid residues 24 to 476 of the amino acid sequence represented by SEQ ID NO: 237, and a light chain comprising an amino acid sequence represented by amino acid residues 24 to 237 of the amino acid sequence represented by SEQ ID NO: 217.

An alternative further more preferred example of the humanized antibody can include an antibody comprising the aforementioned heavy chain variable regions and light chain variable regions, preferably the heavy chain variable regions and light chain variable regions of a humanized antibody derived from 2B1, and that also binds to human GPRC5D which comprises a natural or Fc mutation.

A particularly preferred example of such an antibody can include an antibody comprising a heavy chain variable region comprising an amino acid sequence represented by amino acid residues 20 to 142 of the amino acid sequence represented by SEQ ID NO: 76, and a light chain variable region comprising an amino acid sequence represented by amino acid residues 21 to 127 of the amino acid sequence represented by SEQ ID NO: 72, as well as comprising a natural or Fc mutation. More specific examples of these can include an antibody comprising an amino acid sequence represented by amino acid residues 24 to 271 of the amino acid sequence represented by SEQ ID NO: 223, and a natural or Fc mutation.

The human antibody is not particularly limited as long as the antibody binds to human GPRC5D. Examples thereof can also include a human antibody that binds to the same site as the humanized antibody of the present invention. Examples thereof include a human antibody that binds to the same site as h2B1H2L5.

Examples of the human antibody of the present invention include an antibody including a heavy chain variable region and a light chain variable region described in any one of the following (① to ④:

①

a heavy chain variable region comprising
heavy chain CDR1 consisting of an amino acid sequence represented by SEQ ID NO: 111,
heavy chain CDR2 consisting of an amino acid sequence represented by SEQ ID NO: 112, and
heavy chain CDR3 consisting of an amino acid sequence represented by SEQ ID NO: 113,
and
a light chain variable region comprising
light chain CDR1 consisting of an amino acid sequence represented by SEQ ID NO: 114,
light chain CDR2 consisting of an amino acid sequence represented by SEQ ID NO: 115, and
light chain CDR3 consisting of an amino acid sequence represented by SEQ ID NO: 116,

②

a heavy chain variable region comprising
heavy chain CDR1 consisting of an amino acid sequence represented by SEQ ID NO: 117,
heavy chain CDR2 consisting of an amino acid sequence represented by SEQ ID NO: 118, and
heavy chain CDR3 consisting of an amino acid sequence represented by SEQ ID NO: 119,
and
a light chain variable region comprising
light chain CDR1 consisting of an amino acid sequence represented by SEQ ID NO: 120,
light chain CDR2 consisting of an amino acid sequence represented by SEQ ID NO: 121, and
light chain CDR3 consisting of an amino acid sequence represented by SEQ ID NO: 122,

③

a heavy chain variable region comprising

heavy chain CDR1 consisting of an amino acid sequence represented by SEQ ID NO: 123,
heavy chain CDR2 consisting of an amino acid sequence represented by SEQ ID NO: 124, and
heavy chain CDR3 consisting of an amino acid sequence represented by SEQ ID NO: 125,
and
a light chain variable region comprising
light chain CDR1 consisting of an amino acid sequence represented by SEQ ID NO: 126,
light chain CDR2 consisting of an amino acid sequence represented by SEQ ID NO: 127, and
light chain CDR3 consisting of an amino acid sequence represented by SEQ ID NO: 128, and

④

a heavy chain variable region comprising
heavy chain CDR1 consisting of an amino acid sequence represented by SEQ ID NO: 129,
heavy chain CDR2 consisting of an amino acid sequence represented by SEQ ID NO: 130, and
heavy chain CDR3 consisting of an amino acid sequence represented by SEQ ID NO: 131, and
a light chain variable region comprising
light chain CDR1 consisting of an amino acid sequence represented by SEQ ID NO: 132,
light chain CDR2 consisting of an amino acid sequence represented by SEQ ID NO: 133, and
light chain CDR3 consisting of an amino acid sequence represented by SEQ ID NO: 134.

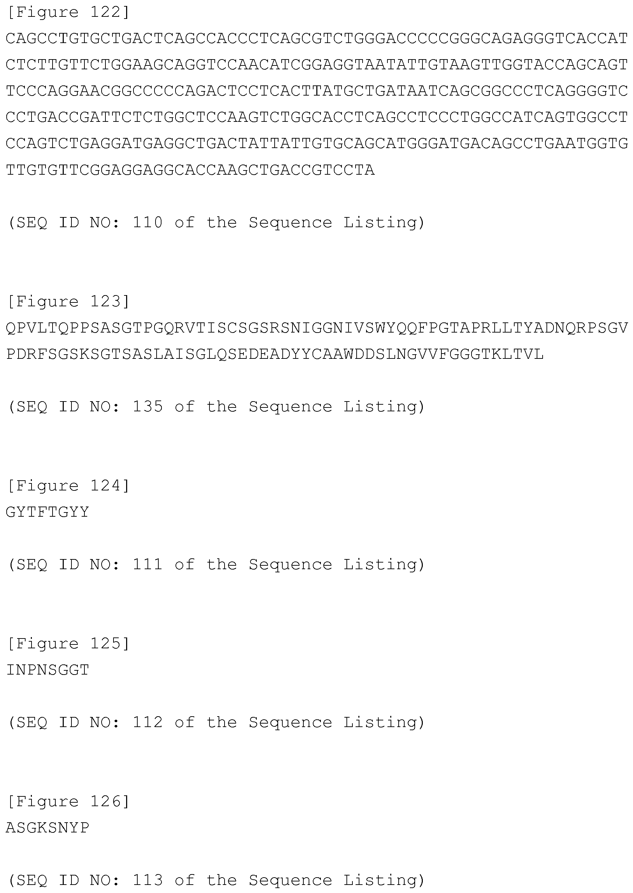

The amino acid sequences of the CDRs mentioned above are also described in Figures 124 to 147.

Preferred examples of the human antibody can include an antibody comprising
a heavy chain variable region comprising any one of
an amino acid sequence represented by SEQ ID NO: 97,
an amino acid sequence represented by SEQ ID NO: 101,
an amino acid sequence represented by SEQ ID NO: 105, and
an amino acid sequence represented by SEQ ID NO: 109,
and
a light chain variable region comprising any one of
an amino acid sequence represented by SEQ ID NO: 99,
an amino acid sequence represented by SEQ ID NO: 103,
an amino acid sequence represented by SEQ ID NO: 107, and
an amino acid sequence represented by SEQ ID NO: 135
or an antigen-binding fragment of the antibody.

Specific preferred examples of the human antibody can include an antibody comprising any one combination of a heavy chain variable region and a light chain variable region of

- a heavy chain variable region comprising the amino acid sequence represented by SEQ ID NO: 97, and a light chain variable region comprising the amino acid sequence represented by SEQ ID NO: 99,
- a heavy chain variable region comprising the amino acid sequence represented by SEQ ID NO: 101, and a light chain variable region comprising the amino acid sequence represented by SEQ ID NO: 103,
- a heavy chain variable region comprising the amino acid sequence represented by SEQ ID NO: 105, and a light chain variable region comprising the amino acid sequence represented by SEQ ID NO: 107, or
- a heavy chain variable region comprising the amino acid sequence represented by SEQ ID NO: 109, and a light chain variable region comprising the amino acid sequence represented by SEQ ID NO: 135. Such an antibody includes single chain antibody (also referred to as scFv) (Example 10)-4).

Another example of the human antibody includes an IgG type antibody comprising the aforementioned light and heavy chain variable regions linked by a human immunoglobulin heavy chain constant region (CHI and Fc region) or a human immunoglobulin light chain constant region (CL). Examples of such an IgG type human antibody include an antibody comprising any one combination of a heavy chain and a light chain of

- a heavy chain comprising the amino acid sequence represented by SEQ ID NO: 144, and a light chain comprising the amino acid sequence represented by SEQ ID NO: 145,
- a heavy chain comprising the amino acid sequence represented by SEQ ID NO: 146, and a light chain comprising the amino acid sequence represented by SEQ ID NO: 147,
- a heavy chain comprising the amino acid sequence represented by SEQ ID NO: 148, and a light chain comprising the amino acid sequence represented by SEQ ID NO: 149, or

- a heavy chain comprising the amino acid sequence represented by SEQ ID NO: 150, and a light chain comprising the amino acid sequence represented by SEQ ID NO: 151.

The anti-GPRC5D antibody of the present invention may be an antibody comprised of portions derived from a plurality of different antibodies as long as the antibody binds to human GPRC5D. Examples of such an antibody can include an antibody comprising heavy and/or light chains exchanged among a plurality of different antibodies, an antibody comprising full-length heavy and/or light chains exchanged there among, an antibody comprising only variable regions or only constant regions exchanged there among, and an antibody comprising all or some CDRs exchanged there among. The heavy and light chain variable regions of the chimeric antibody may be derived from different anti-GPRC5D antibodies of the present invention. Heavy chain CDR1 to CDR3 and light chain CDR1 to CDR3 in the heavy and light chain variable regions of the humanized antibody may be derived from two or more different anti-GPRC5D antibodies of the present invention. Heavy chain CDR1 to CDR3 and light chain CDR1 to CDR3 in the heavy and light chain variable regions of the human antibody may be a combination of CDRs carried by two or more different anti-GPRC5D antibodies of the present invention.

The anti-GPRC5D antibody of the present invention comprises a single chain immunoglobulin comprising full-length heavy and light chain sequences of the antibody linked via an appropriate linker (Lee, H-S, et al., Molecular Immunology (1999) 36, 61-71; and Shirrmann, T. et al., mAbs (2010), 2 (1), 1-4). Such a single chain immunoglobulin can be dimerized to thereby maintain a structure and activities similar to those of the antibody, which is originally a tetramer.

The anti-GPRC5D antibody of the present invention or antigen-binding fragment of the antibody also includes an antibody or an antigen-binding fragment of the antibody that comprises an amino acid sequence encoded by a nucleotide sequence contained in a polynucleotide hybridizing under stringent conditions to a complementary strand of a polynucleotide comprising a nucleotide sequence encoding an amino acid sequence contained in the anti-GPRC5D antibody of the present invention or antigen-binding fragment of the antibody, and binds to human GPRC5D.

The anti-GPRC5D antibody of the present invention or antigen-binding fragment of the antibody may be an antibody or an antigen-binding fragment of the antibody that comprises an amino acid sequence of a heavy chain variable region and/or an amino acid sequence of a light chain variable region 90%, 91%, 92%, 93%, 94%, 95%, 96%, 97%, 98%, or 99% or more identical to amino acid sequence(s) contained in the aforementioned antibody or antigen-binding fragment of the antibody according to any one of (8) to (12) and (18) to (20), and binds to human GPRC5D.

Compared to when the positions and lengths of light chain variable regions are determined according to the definition of IMGT, when the positions and lengths of light chain variable regions have been determined using a definition different from IMGT, one or two or more amino acid(s) may be contained, for example, arginine (R) and/or glycine (G), in the carboxyl termini of the amino acid sequences of the light chain variable regions determined according to the definition of IMGT. An antibody or an antigen-binding fragment thereof having such a light chain variable region is also encompassed by the antibody of the present invention or antigen-binding fragment thereof.

The ability of the antibody, etc. of the present invention to bind to GPRC5D, particularly, human and/or cynomolgus monkey GPRC5D, may be optimized by introducing a mutation to the antigen-binding fragment of the anti-GPRC5D antibody of the present invention. Specific examples of the method for introducing a mutation can comprise random mutagenesis using error-prone PCR, site directed amino acid mutagenesis using an NNK library, site directed mutagenesis using structure information, and combinations thereof.

3-2. Antibody mutant of anti-GPRC5D antibody

The antibody mutant of the anti-GPRC5D antibody of the present invention can preferably exhibit, for example, reduced sensitivity to protein degradation or oxidation, a preserved or improved biological activity or function or suppressed reduction or change in biological activity or function, an improved or adjusted ability to bind to the antigen, or physicochemical or functional properties imparted thereto. Proteins are known to vary in their function or activities due to change in a particular amino acid side chain present on the surface thereof. Such examples comprise the deamidation of an asparagine side chain and the isomerization of an aspartic acid side chain. An antibody derived from the anti-GPRC5D antibody of the present invention by replacement with another amino acid in order to prevent such change in the amino acid side chain is also included in the scope of the antibody mutant of the present invention.

Examples of the antibody mutant of the present invention can include an antibody having an amino acid sequence derived from the amino acid sequence of the original antibody by conservative amino acid substitution. The conservative amino acid substitution is a substitution that occurs in an amino acid group related to amino acid side chains.

Preferred amino acid groups are as follows: an acidic group such as aspartic acid and glutamic acid; a basic group such as lysine, arginine, and histidine; a nonpolar group such as alanine, valine, leucine, isoleucine, proline, phenylalanine, methionine, and tryptophan; and an uncharged polar family such as glycine, asparagine, glutamine, cysteine, serine, threonine, and tyrosine. Other preferred amino acid groups are as follows: an aliphatic hydroxy group such as serine and threonine; an amide-containing group such as asparagine and glutamine; an aliphatic group such as alanine, valine, leucine, and isoleucine; and an aromatic group such as phenylalanine, tryptophan, and tyrosine. Such amino acid substitution in the antibody mutant is preferably performed without reducing the antigen binding activity of the original antibody.

The anti-GPRC5D antibody of the present invention, the antigen-binding fragment thereof, the variant thereof, or the molecule of the present invention also encompasses: an antibody mutant, an antigen-binding fragment thereof, and a molecule comprising the antibody mutant or antigen-binding fragment, etc., having an amino acid sequence derived from the amino acid sequence of the antibody of the present invention such as 2A4, 2B1, or 7B4 by conservative amino acid substitution and/or any other mutation, and binds to human GPRC5D; a mouse antibody, a rat antibody, a chimeric antibody, a humanized antibody, a human antibody, an antigen-binding fragment thereof, and a molecule comprising these, having an amino acid sequence derived from any amino acid sequence of CDRH1 to CDRH3 and CDRL1 to CDRL3 derived from the antibody of the present invention containing 2A4, 2B1, or 7B4, or the like by conservative amino acid substitution and/or any other mutation, and binds to human GPRC5D; an antibody mutant, an antigen-binding fragment thereof, and a molecule comprising the antibody mutant or antigen-binding fragment, etc. having an amino acid sequence derived from the amino acid sequence of the antibody of the present invention such as C2037, C3048, C3015, or C3022 by conservative amino acid substitution, and binds to human GPRC5D; and a chimeric antibody, a human antibody, an antigen-binding fragment thereof, and a molecule comprising these, having an amino acid sequence of CDRH1 to CDRH3 and CDRL1 to CDRL3 derived from the antibody of the present invention containing C2037, C3048, C3015, or C3022, or the like by conservative amino acid substitution, and binds to human GPRC5D.

3-3. Antigen-binding fragment of anti-GPRC5D antibody

According to one aspect, the present invention provides an antigen-binding fragment of the anti-GPRC5D antibody of the present invention. The antigen-binding fragment of the anti-GPRC5D antibody of the present invention encompasses antigen-binding fragments of chimeric antibodies, humanized antibodies, or human antibodies. The antigen-binding fragment of the antibody means a fragment that maintains at least antigen binding activity among the functions of the antibody, or a modified form thereof. Examples of such functions of the antibody can generally include antigen binding activity, antigen activity-regulating activity (e.g., agonistic activity), activity of internalizing an antigen into cells, and activity of inhibiting or promoting the interaction between an antigen and its interacting substance.

The antigen-binding fragment of the antibody is not particularly limited as long as the fragment of the antibody maintains at least antigen binding activity among the activities of the antibody. Examples of such an antigen-binding fragment of the antibody include, but are not limited to, Fab, Fab', $F(ab')_2$, single chain Fab (scFab) comprising the carboxy terminus of a Fab light chain and the amino terminus of a Fab heavy chain linked via an appropriate linker, Fv, single chain Fv (scFv) comprising heavy and light chain Fvs linked via an appropriate linker, and single domain antibody (sdAb; also called nanobody) having a single heavy chain variable region and lacking a light chain sequence (Muyldemans S. et al., Protein Eng., (1994), 7 (9), 1129-35; and Hamers-Casterman C. et al., Nature (1993), 363 (6428), 446-448). The meaning of the antigen-binding fragment of the antibody of the present invention also encompasses a molecule comprising portions other than the antigen-binding fragment of the antibody of the present invention, such as scFab and scFv retaining a linker portion.

3-4 Modified form of anti-GPRC5D antibody or antigen-binding fragment thereof and conjugates

The present invention provides a modified form of the antibody or antigen-binding fragment thereof. The modified form of the antibody of the present invention or antigen-binding fragment thereof means an antibody of the present invention or an antigen-binding fragment thereof provided with chemical or biological modification. The chemically modified form includes, for example, a form having an amino acid skeleton conjugated with a chemical moiety, and a form having a chemically modified N-linked or O-linked carbohydrate chain. The biologically modified form includes, for example, a form that has undergone post-translational modification (e.g., N-linked or O-linked glycosylation, processing of an amino-terminal or carboxyl-terminal region, deamidation, isomerization of aspartic acid, or oxidation of methionine), and a form containing a methionine residue added to the amino terminus by expression using prokaryotic host cells. Such a modified form is also meant to include a form labeled to permit detection or isolation of the antibody or the antigen of the present invention, for example, an enzyme-labeled form, a fluorescently labeled form, or an affinity-labeled form. Such a modified form of the antibody of the present invention or antigen-binding fragment thereof is useful for improvement of the stability or blood retention of the original antibody of the present invention or the original antigen-binding fragment thereof, reduction in antigenicity, detection or isolation of the antibody or the antigen, etc.

Examples of the chemical moiety contained in the chemically modified form can include water-soluble polymers such as polyethylene glycol (PEG), ethylene glycol/propylene glycol copolymers, carboxymethylcellulose, dextran, and polyvinyl alcohol.

Examples of the biologically modified form can include a form modified by enzymatic treatment, cell treatment, or the like, a form fused with another peptide, such as a tag, added by gene recombination, and a form prepared from host cells expressing an endogenous or exogenous sugar chain-modifying enzyme.

Such a modification may be made at an arbitrary position or a desired position in the antibody or antigen-binding fragment thereof. Alternatively, the same or two or more different modifications may be made at one or two or more positions therein.

The deletion in these heavy chain sequences or the modification in the heavy or light chain sequences, however, has little influence on the ability of the antibody to bind to the antigen and its effector functions (complement activation, antibody dependent cytotoxic effects, etc.).

Thus, the present invention also encompasses an antibody that has received the deletion or the modification. Examples thereof can comprise a deletion variant derived from a heavy chain by the deletion of 1 or 2 amino acid(s) at its carboxyl terminus (Journal of Chromatography A, 705: 129-134 (1995)), an amidated form of the deletion variant having a heavy chain that lacks two amino acid residues (glycine and lysine) at the carboxy terminus and instead has an amidated proline residue at the carboxy terminus (Analytical Biochemistry, 360: 75-83 (2007)), and a modified antibody having a pyroglutamylated amino-terminal glutamine or glutamic acid residue in its heavy or light chain (International Publication No. WO2013/147153) (these are collectively referred to as "deletion variants"). However, the deletion variant at the carboxyl terminus of the antibody heavy or light chain according to the present invention is not limited to the types described above as long as the deletion variant maintains its antigen binding ability and effector functions. When the antibody according to the present invention comprises two or more chains (e.g., heavy chains), the two or more chains (e.g., heavy chains) may be heavy chains of any one type selected from a group consisting of full-length heavy chains and the deletion variants described above, or may be a combination of heavy chains of any two types selected therefrom. The quantitative ratio or the ratio of the number of molecules of each deletion variant may be influenced by the type of cultured mammalian cells producing the antibody according to the present invention, and culture conditions. Examples of such a case can include the deletion of one carboxyl-terminal amino acid residue each in both of the two heavy chains as main components of the antibody according to the present invention.

Even if one to several amino acid(s) derived from, for example, an expression vector and/or a signal sequence is added to the amino terminus and/or the carboxyl terminus (and a portion or the whole thereof is modified as described above) in the antibody of the present invention or antigen-binding fragment thereof (e.g., contained in the molecule, the multispecific molecule, the bispecific molecule, etc. of the present invention), as long as the desired antigen binding activity is maintained, it is contained in the scope of the modified form of the antibody of the present invention or the modified form of the antigen-binding fragment thereof, and the molecule comprising the modified form of such an antibody or the modified form of the antigen-binding fragment is included in the scope of the molecule of the present invention.

In the present invention, the "antibody or antigen-binding fragment thereof" also includes the meaning of the "modified form of the antibody or the antigen-binding fragment thereof'. The "antibody or the antigen-binding fragment thereof" contained in the molecule, the multispecific molecule, the bispecific molecule, etc. of the present invention also includes the meaning of the "modified form of the antibody or antigen-binding fragment thereof'.

Furthermore, it is possible to enhance antibody-dependent cytotoxic activity by adjusting glycosylation binding to the antibody of the present invention (glycosylation, defucoslyation, etc.). International Patent Nos. WO99/5432, WO00/61739, and WO02/31140 are known as art for adjusting glycosylation of an antibody, yet this art is not limited to these.

The present invention also encompasses conjugates formed by the aforementioned antibodies linked with other molecules via linkers (immunoconjugates). Examples of such an antibody-drug complex in which the antibody is conjugated with a radioactive material or a compound (drug) having a pharmacological action can include ADC (antibody-drug conjugate) ([Methods Mol Biol. (2013) 1045: 1-27; Nature Biotechnology (2005) 23, p.1137-1146)).

In addition, the present invention also encompasses conjugates comprising these antibodies connected to other functional polypeptides. Examples of such an antibody-peptide complex can include a complex of the antibody and an albumin-binding polypeptide (Protein Eng Des Sel. (2012) (2): 81-8).

These modified forms of the antibody, antibodies that have undergone glycosylation adjustment, and conjugates are encompassed by the antibody of the present invention. Antigen-binding fragments of these modified forms of the antibody, antibodies that have undergone glycosylation adjustment, and conjugates are encompassed by the antigen-binding fragment of the antibody of the present invention.

3-5. Antibody that binds to same site

An antibody that binds to a site on human GPRC5D bound by the antibody provided by the present invention or antigen-binding fragment of the antibody is also included in the antibody of the present invention or antigen-binding fragment of the antibody. The antibody that binds to the same site on the human GPRC5D antigen as a certain antibody means another antibody that binds to the same site on the antigen molecule recognized by the antibody. If a second antibody binds to a partial peptide or a partial three-dimensional structure on an antigen molecule bound by a first antibody, the first and second antibodies are determined as binding to the same site.

Alternatively, the first and second antibodies are determined as binding to the same site by confirming that the second antibody competes with the first antibody that binds to the antigen, i.e., the second antibody interferes with the binding of the first antibody to the antigen, even if the peptide sequence or three-dimensional structure of the specific binding site is not determined.

When the first and second antibodies bind to the same site, the second antibody has an exceedingly high probability of having the same activity as the first antibody.

The antibody binding site can be determined by a method well known by those skilled in the art, such as immunoassay. For example, a series of peptides are prepared by appropriately cleaving the amino acid sequence of the antigen from its carboxyl terminus or amino terminus, and the reactivity of the antibody thereto is studied to roughly determine a recognition site. Then, shorter peptides are synthesized, and the reactivity of the antibody to these peptides can be studied to thereby determine the binding site. The antigen fragment peptides can be prepared using a technique such as gene recombination or peptide synthesis.

3-6. Polynucleotide, vector, and cell of the present invention

The present invention also provides a polynucleotide comprising a nucleotide sequence (e.g., SEQ ID NO: 63, 65, 67, 69, 71, 73, 75, 77, 79, 81, 83, 85, 87, 89, or 91) encoding the amino acid sequence of the anti-GPRC5D antibody of the present invention or antigen-binding fragment thereof, a vector comprising the polynucleotide, a cell comprising the polynucleotide or the vector, and a cell producing the anti-GPRC5D antibody of the present invention or antigen-binding fragment thereof, etc. Such a polynucleotide, vector (a circular form such as a plasmid, and a noncircular form comprising vectors integrated in chromosomes), and cell are useful in the production of the anti-GPRC5D antibody or antigen-binding fragment thereof mentioned later.

The polynucleotide of the present invention may contain an arbitrary nucleotide sequence, in addition to the nucleotide sequence encoding the amino acid sequence of the anti-GPRC5D antibody or antigen-binding fragment thereof. For example, in addition to a polynucleotide comprising the nucleotide sequence (SEQ ID NO: 63, 65, 67, 69, 71, 73, 75, 77, 79, 81, 83, 85, 87, 89, 91, etc. of the Sequence Listing) encoding the amino acid sequence of the anti-GPRC5D antibody of the present invention or antigen-binding fragment thereof, a nucleotide sequence encoding the amino acid sequence of an activity signal transduction molecule peptide and/or a nucleotide sequence encoding the amino acid sequence of a co-stimulatory molecule are also part of the aspect of the polynucleotide of the present invention. The present invention also provides an artificial immunocyte having binding activity against tumor cells through a chimeric antigen receptor (CAR) expressed by the transfection of an immunocyte (e.g., a T cell, a NK cell, or a monocyte) with such a polynucleotide (hereinafter, also referred to as the artificial immunocyte of the present invention).

CAR is a chimeric protein having scFv comprising tandemly bound light and heavy chains derived from a monoclonal antibody that recognizes a surface antigen on tumor cells, and an activity signal transduction molecule (e.g., CD3ζ for T cell receptors or a receptor FcRγ for immunoglobulin molecules) at its amino terminus and carboxy terminus, respectively, between which an extracellular hinge domain, a transmembrane domain, a co-stimulatory molecule activating immunocytes, and the like are connected. In the cell of the present invention, the monoclonal antibody that recognizes a surface antigen on tumor cells is the anti-GPRC5D antibody of the present invention. The immunocyte of the present invention expressing CAR recognizes a GPRC5D protein on the surface of tumor via the anti-GPRC5D antibody of the present invention in the form of scFv while the activation of the immunocyte itself is induced by the intracellular activity signal transduction molecule to attack tumor cells.

In the case of using a T cell as the immunocyte to be transfected with the polynucleotide of the present invention, the chimeric antigen receptor (CAR) expressed on cell surface by the transfection of the T cell with this polynucleotide can cause a GPRC5D-expressing cell and the T cell to come close to each other to induce T cell activation-mediated cytotoxicity to the GPRC5D-expressing cell, i.e., cytotoxicity by the redirection of the T cell. The present invention also provides such a T cell expressing CAR (hereinafter, also referred to as the T cell of the present invention).

In other words, the T cell of the present invention can be redirected to tumor cells expressing GPRC5D to induce cytotoxicity to the tumor cells.

The activity signal transduction molecule is transfected into the immunocyte in order to transduce signals from an immunocyte receptor into the cell. In the case of using, for example, a T cell as the immunocyte, examples of the activity signal transduction molecule include CD3ζ, DAP12, and FcRγ.

The co-stimulatory molecule is transfected into the immunocyte in order to more strongly activate the immunocyte. In the case of using, for example, a T cell as the immunocyte, examples of the co-stimulatory molecule include CD2, CD27, CD28, CD49d, CD134, CD152, CD154, ICOS, 4-1BB, and RANKL.

4. Molecule having antigen binding activity

The molecule of the present invention comprises the anti-GPRC5D antibody of the present invention or antigen-binding fragment thereof.
Further, the molecule of the present invention can comprise, for example, a signal sequence, a tag for purification, etc., amino-terminal Gly, a drug linker portion of ADC, an albumin-binding polypeptide, a polymer such as PEG, an antibody other than the anti-GPRC5D antibody, an antigen-binding fragment thereof, and a protein having antigen binding activity without having an immunoglobulin skeleton, which will be described later. Examples of the antibody other than the anti-GPRC5D antibody include an anti-CD3 antibody. A multispecific molecule described later is included in the scope of the molecule of the present invention.

4-1. Multispecific molecule

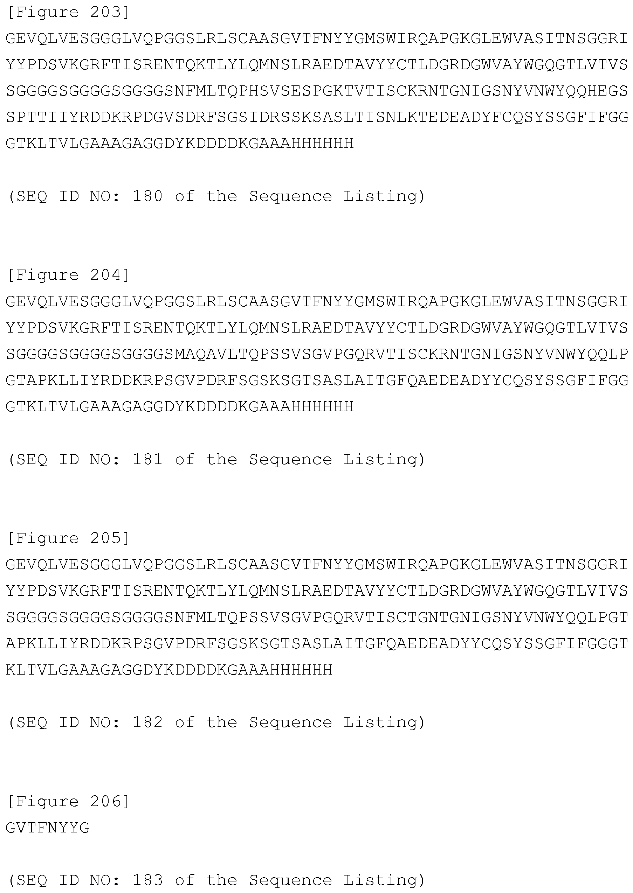

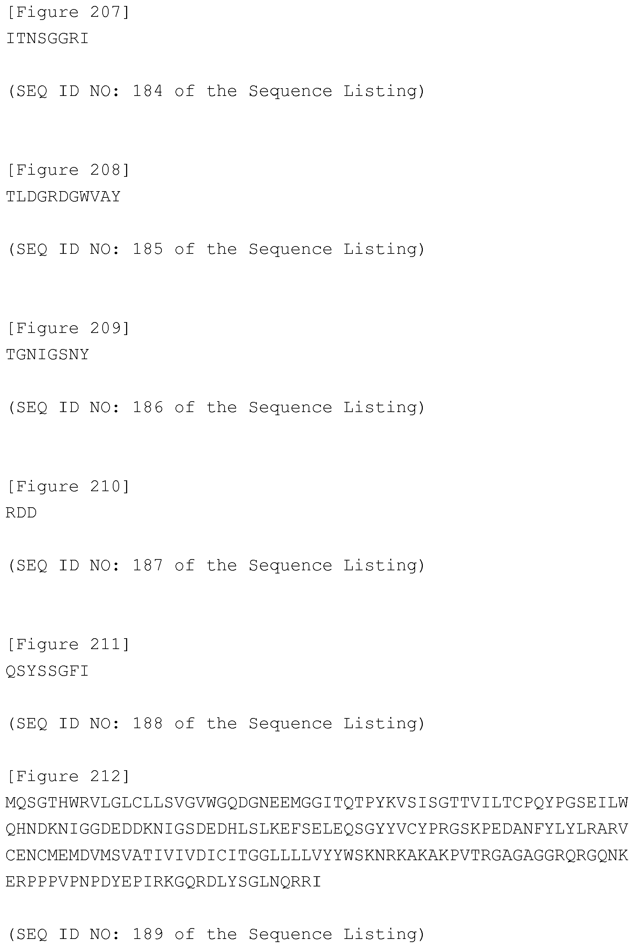

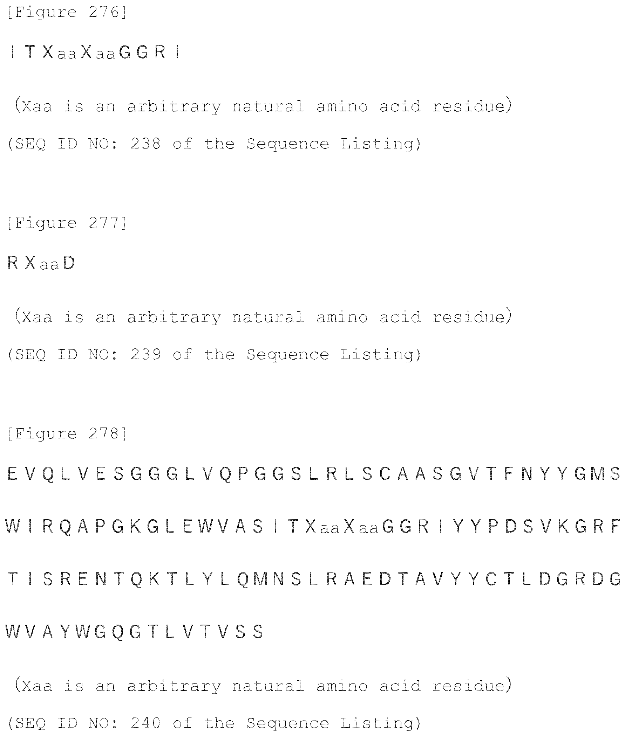

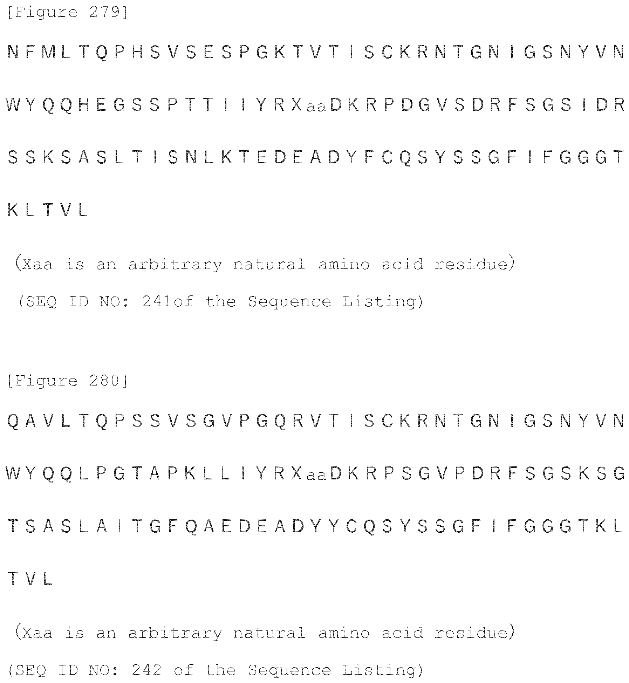

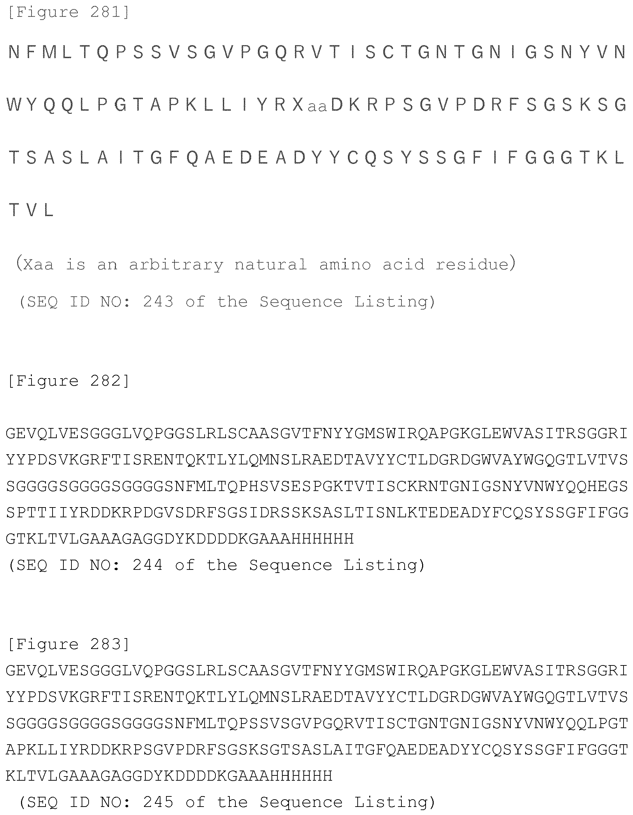

The multispecific molecule of the present invention is a molecule having two or more antigen-binding sites. Specifically, the multispecific molecule of the present invention is a molecule capable of binding to two or more epitopes different from each other on one molecule, or epitopes different from each other on two or more molecules, and comprises a plurality of antigen-binding fragments different from each other. Examples of such a multispecific molecule include, but are not limited to, IgG-type multispecific molecules and multispecific molecules having two or more types of variable regions, for example, antibody fragments such as tandem scFv, single chain diabodies, diabodies, and triabodies, and antibody fragments linked by a covalent bond or a noncovalent bond. The multispecific molecule may contain Fc.
The multispecific molecule of the present invention comprises the anti-GPRC5D antibody of the present invention or antigen-binding fragment of the antibody. The multispecific molecule of the present invention includes the anti-GPRC5D antibody of the present invention or antigen-binding fragment thereof, and 1 or 2 or more antibodies that are different from the anti-GPRC5D antibody and bind to an epitope absent in GPRC5D and present in another antigen, or antigen-binding fragment(s) of the antibody(ies).
Examples of the antigen-binding fragment of the anti-GPRC5D antibody can include Fab, F(ab)', Fv, scFv, and sdAb.
The multispecific molecule of the present invention specifically binds to GPRC5D or may further bind to a target such as an Fc receptor on effector cells.
Examples of the antibody different from the anti-GPRC5D antibody that can be contained in the multispecific molecule of the present invention include an anti-CD3 antibody.
As a preferred example, the anti-CD3 antibody or antigen-binding fragment thereof that can be contained in the multispecific molecule of the present invention retains
a heavy chain CDR1 comprising the amino acid sequence represented by SEQ ID NO: 183 (Figure 206) (GVTFNYYG),
a heavy chain CDR2 comprising the amino acid sequence represented by SEQ ID NO: 238 (Figure 276) (ITX$_{aa}$X$_{aa}$GGRI) (wherein each of the first X$_{aa}$ and the second X$_{aa}$ is an arbitrary natural amino acid residue; hereinafter, the first X$_{aa}$ and the second X$_{aa}$ in the heavy chain CDR2 are also referred to as X$_1$ and X$_2$, respectively), and
a CDRH3 comprising the amino acid sequence represented by SEQ ID NO: 185 (Figure 208) (TLDGRDGWVAY).
The light chain variable region contained in a preferred humanized anti-CD3 antibody of the present invention or antigen-binding fragment of the antibody retains
a light chain CDR1 comprising the amino acid sequence represented by SEQ ID NO: 186 (Figure 209) (TGNIGSNY),
a light chain CDR2 comprising the amino acid sequence represented by SEQ ID NO: 239 (Figure 277) (RX$_{aa}$D) (wherein X$_{aa}$ is an arbitrary natural amino acid residue; hereinafter, X$_{aa}$ in the light chain CDR2 is also referred to as X$_3$), and
a light chain CDR3 comprising the amino acid sequence represented by SEQ ID NO: 188 (Figure 211) (QSYSSGFI).
In the aforementioned heavy chain CDR2 (ITX$_1$X$_2$GGRI), preferably, X$_1$ is selected from a group consisting of (A, E, G, H, I, L, T, V, R, and S), and X$_2$ is S; or X$_1$ is N, and X$_2$ is selected from a group consisting of (E, R, F, Y, L, V, I, K, and T).
In the aforementioned light chain CDR2 (RX$_3$D), preferably, X$_3$ is selected from a group consisting of (Q, A, G, S, N, and D).
In the aforementioned heavy chain CDR2 (ITX$_1$X$_2$GGRI), more preferably, X$_1$ is selected from a group consisting of (R and S), and X$_2$ is S.
In the aforementioned light chain CDR2 (RX$_3$D), more preferably, X$_3$ is selected from a group consisting of (Q, A, G, S, N, and D).
Preferable examples of the multispecific molecule of the present invention comprise a heavy chain variable region comprising an amino acid sequence represented by SEQ ID NO: 240 (Figure 278) and a light chain variable region comprising an amino acid sequence represented by SEQ ID NO: 241 (Figure 279), SEQ ID NO: 242 (Figure 280), or SEQ ID NO: 243 (Figure 281), wherein
a first X$_{aa}$ of the amino acid sequence represented by SEQ ID NO: 240 is selected from a group consisting of (A, E, G, H, I, L, T, V, R, and S), and $X_a$ is S; or
the first $X_{aa}$ is N, and a second $X_{aa}$ is selected from a group consisting of (E, R, F, Y, L, V, I, K, and T).
$X_{aa}$ of any one of the amino acid sequences represented by SEQ ID Nos: 241, 242, and 243 is selected from a group consisting of (Q, A, G, S, N, and D).

More preferable examples of the anti-CD3 antibody or antigen-binding fragment thereof which can be contained in the multispecific molecule of the present invention include a molecule wherein the first $X_{aa}$ of SEQ ID NO: 240 is selected from a group consisting of (R, and S),
a second $X_{aa}$ is S, $X_{aa}$ of an amino acid sequence represented by one of SEQ ID NOs: 241, 242, and 243 is selected from a group consisting of (Q, A, G, S, N, and D).

Specific examples of the preferred humanized anti-CD3 antibody of the present invention or the antigen-binding fragment of the antibody include an antibody or an antigen-binding fragment of the antibody that comprises
a heavy chain variable region comprising
an amino acid sequence of heavy chain CDR1 represented by SEQ ID NO: 183 (GVTFNYYG),
an amino acid sequence of heavy chain CDR2 represented by SEQ ID NO: 184 (ITNSGGRI), and
an amino acid sequence of heavy chain CDR3 represented by SEQ ID NO: 185 (TLDGRDGWVAY), and
a light chain variable region comprising
an amino acid sequence of light chain CDR1 represented by SEQ ID NO: 186 (TGNIGSNY),
an amino acid sequence of light chain CDR2 represented by SEQ ID NO: 187 (RDD), and
an amino acid sequence of light chain CDR3 represented by SEQ ID NO: 188 (QSYSSGFI),
and binds to human CD3 and cynomolgus monkey CD3. The positions and lengths of these CDRs of the anti-CD3 antibody were determined according to the definition of IMGT.

The anti-CD3 antibody or the antigen-binding fragment thereof contained in the multispecific molecule of the present invention having the CDRs mentioned above, and their variable regions (hereinafter, also referred to as the anti-CD3 antibody, etc. of the present invention), bind to an Ig-like domain present in the extracellular region of the ε chain of the human CD3 complex. Furthermore, these also bind to an Ig-like domain present in the extracellular region of the ε chain of the cynomolgus monkey CD3 complex.

Epitopes present in the extracellular region of the ε chain of the human CD3 complex bound by the anti-CD3 antibody, etc. contained in the multispecific molecule of the present invention contain the following amino acids:
Ser55, Glu56, Leu58, Trp59, Asn65, Ile66, Ser77, Asp78, Arg101, Gly102, Ser103, Lys104, and Pro105.

Preferably, the anti-CD3 antibody, etc. contained in the multispecific molecule of the present invention can maintain binding with human CD3 by binding to an epitope region containing at least 7 amino acids selected from these 13 amino acids.

When an antibody is adjacent to these amino acids at a distance within 4 angstroms, such an antibody can be confirmed to have the same epitope specificity as that of the anti-CD3 antibody, etc. contained in the multispecific molecule of the present invention. On the other hand, among these epitope amino acids, Arg101, Gly102, Ser103, Lys104, and Pro105 are also epitope residues that interact with an anti-CD3 antibody OKT3 or UCHT1 known in the art (Lars Kjer-Nielsen et al., PNAS (2004); and Kelly L Arnett et al., PNAS (2004)). However, OKT3 or UCHT1 binds to human CD3, but does not bind to cynomolgus monkey CD3.

Such an antibody that binds to human CD3 and cynomolgus monkey CD3 or the antigen-binding fragment of the antibody contained in the multispecific molecule of the present invention can be subjected to various tests on efficacy or safety using primates, particularly, cynomolgus monkeys, useful for the nonclinical development (preclinical development) of pharmaceutical products, and is thus preferred. Also, the antibody, etc. that binds to human CD3 and cynomolgus monkey CD3 has cytotoxic activity and is useful, either alone or as the molecule of the present invention, in the treatment or prevention of diseases such as cancers in cynomolgus monkeys. The pharmaceutical composition will be described later.

The anti-CD3 antibody may be a nonhuman animal antibody, a chimeric antibody, a humanized antibody, or a human antibody. The anti-CD3 antibody is preferably a humanized antibody or a human antibody.

Examples of the antigen-binding fragment of the anti-CD3 antibody include Fab, F(ab)', Fv, scFv, and sdAb.

Examples of the anti-CD3 antibody or antigen-binding fragment of the antibody comprising the CDRs described above include a humanized antibody or an antigen-binding fragment of the antibody comprising a heavy chain variable region comprising the amino acid sequence represented by SEQ ID NO: 240 (Figure 278), and a light chain variable region comprising an amino acid sequence represented by any one of SEQ ID NOs: 241 (Figure 279), 242 (Figure 280), and 243 (Figure 281). Specific examples thereof include a humanized antibody or an antigen-binding fragment of the antibody comprising a heavy chain variable region comprising the amino acid sequence represented by SEQ ID NO: 155, and a light chain variable region comprising an amino acid sequence represented by any one of SEQ ID NOs: 156, 158, and 160.

Specific examples of the anti-CD3 antibody or the antigen-binding fragment of the antibody include an antibody or an antigen-binding fragment of the antibody comprising the amino acid sequence represented by SEQ ID NOs: 180, 181, and 182. More specific examples thereof include an antibody or an antigen-binding fragment of the antibody comprising an amino acid sequence represented by
an antibody or an antigen-binding fragment of the antibody comprising amino acid residues 2 to 243 of SEQ ID NO: 180,
an antibody or an antigen-binding fragment of the antibody comprising amino acid residues 2 to 243 of SEQ ID NO: 181, and
an antibody or an antigen-binding fragment of the antibody comprising amino acid residues 2 to 241 of SEQ ID NO: 182.

The anti-CD3 antibody mentioned above may be a humanized antibody or a human antibody comprising a human immunoglobulin constant region or Fc. Fc can be an Fc mutation.

Preferable examples of the multispecific molecule of the present invention including the anti-GPRC5D antibody or the antigen-binding fragment of the antibody and the anti-CD3 antibody or the antigen-binding fragment of the antibody can include a human anti-GPRC5D antibody or an antigen-binding fragment of the antibody derived from aforementioned 2B1, furthermore, as the anti-GPRC5D antibody or the antigen-binding fragment of the antibody, comprising

- a heavy chain variable region comprising an amino acid sequence represented by amino acid residues 25 to 142 of an amino acid sequence represented by SEQ ID NO: 207, and a light chain variable region comprising an amino acid sequence represented by amino acid residues 24 to 132 of an amino acid sequence represented by SEQ ID NO: 209,
- a heavy chain variable region comprising an amino acid sequence represented by amino acid residues 25 to 142 of an amino acid sequence represented by SEQ ID NO: 211, and a light chain variable region comprising an amino acid sequence represented by amino acid residues 24 to 130 of an amino acid sequence represented by SEQ ID NO: 213,
- a heavy chain variable region comprising an amino acid sequence represented by amino acid residues 2 to 119 of SEQ ID NO: 244, and a light chain variable region comprising an amino acid sequence represented by amino acid residues 135 to 243 of SEQ ID NO: 244,
- a heavy chain variable region comprising an amino acid sequence represented by amino acid residues 2 to 119 of SEQ ID NO: 245, and a light chain variable region comprising an amino acid sequence represented by amino acid residues 135 to 241 of SEQ ID NO: 245,
- a heavy chain variable region comprising an amino acid sequence represented by amino acid residues 2 to 119 of SEQ ID NO: 246, and a light chain variable region comprising an amino acid sequence represented by amino acid residues 135 to 243 of SEQ ID NO: 246,
- a heavy chain variable region comprising an amino acid sequence represented by amino acid residues 2 to 119 of SEQ ID NO: 247, and a light chain variable region comprising an amino acid sequence represented by amino acid residues 135 to 243 of SEQ ID NO: 247,
- a heavy chain variable region comprising an amino acid sequence represented by amino acid residues 2 to 119 of SEQ ID NO: 248, and a light chain variable region comprising an amino acid sequence represented by amino acid residues 135 to 243 of SEQ ID NO: 248,
- a heavy chain variable region comprising an amino acid sequence represented by amino acid residues 2 to 119 of SEQ ID NO: 249, and a light chain variable region comprising an amino acid sequence represented by amino acid residues 135 to 243 of SEQ ID NO: 249,
- a heavy chain variable region comprising an amino acid sequence represented by amino acid residues 2 to 119 SEQ ID NO: 250, and a light chain variable region comprising an amino acid sequence represented by amino acid residues 135 to 243 of SEQ ID NO: 250,
- a heavy chain variable region comprising an amino acid sequence represented by amino acid residues 2 to 119 of SEQ ID NO: 251, and a light chain variable region comprising an amino acid sequence represented by amino acid residues 135 to 243 of SEQ ID NO: 251,
- a heavy chain variable region comprising an amino acid sequence represented by amino acid residues 2 to 119 of SEQ ID NO: 252, and a light chain variable region comprising an amino acid sequence represented by amino acid residues 135 to 243 of SEQ ID NO: 252,
- a heavy chain variable region comprising an amino acid sequence represented by amino acid residues 2 to 119 of SEQ ID NO: 253, and a light chain variable region comprising an amino acid sequence represented by amino acid residues 135 to 243 of SEQ ID NO: 253,
- a heavy chain variable region comprising an amino acid sequence represented by amino acid residues 2 to 119 of SEQ ID NO: 254, and a light chain variable region comprising an amino acid sequence represented by amino acid residues 135 to 243 of SEQ ID NO: 254, or
- a heavy chain variable region comprising an amino acid sequence represented by amino acid residues 2 to 119 of SEQ ID NO: 255, and a light chain variable region comprising an amino acid sequence represented by amino acid residues 135 to 243 of SEQ ID NO: 255, and

comprises an antibody that binds to human CD3 and cynomolgus monkey CD3 or an antigen-binding fragment of the antibody.

A more preferable molecule from among these molecules wherein is one wherein the antibody that binds to human GPRC5D or the antigen-binding fragment of the antibody comprises a heavy chain variable region comprising an amino acid sequence represented by amino acid residues 20 to 142 of the amino acid sequence represented by SEQ ID NO: 76 and a light chain variable region comprising an amino acid sequence represented by amino acid residues 21 to 127 of the amino acid sequence represented by SEQ ID NO: 72, and

i) a heavy chain constant region comprising an amino acid sequence represented by amino acid residues 147 to 475 of the amino acid sequence represented by SEQ ID NO: 199, and a light chain constant region comprising an amino acid sequence represented by amino acid residues 131 to 237 of the amino acid sequence represented by SEQ ID NO: 203,
ii) a heavy chain constant region comprising an amino acid sequence represented by amino acid residues 147 to 475 of the amino acid sequence represented by SEQ ID NO: 201, and a light chain constant region comprising an amino acid sequence represented by amino acid residues 131 to 237 of the amino acid sequence represented by SEQ ID NO: 205,
iii) a heavy chain constant region comprising an amino acid sequence represented by amino acid residues 147 to 475 of the amino acid sequence represented by SEQ ID NO: 215, and a light chain constant region comprising an amino acid sequence represented by amino acid residues 131 to 237 of the amino acid sequence represented by SEQ ID NO: 217,
or
iv) a heavy chain constant region comprising an amino acid sequence represented by amino acid residues 147 to 476 of the amino acid sequence represented by SEQ ID NO: 237, and a light chain constant region comprising an amino acid sequence represented by amino acid residues 131 to 237 of the amino acid sequence represented by SEQ ID NO: 217, and the antibody that binds to human CD3 and cynomolgus monkey CD3 or the antigen-binding fragment of the antibody further comprises a Fc mutation.

An even more preferable molecule among these is a molecule wherein the antibody that binds to human GPRC5D or the antigen-binding fragment of the antibody is the antibody or the antigen-binding fragment of the antibody that binds to human GPRC5D, comprising the constant regions shown in the foregoing iii) or iv).

Specific preferable examples of the multispecific molecule of the present invention can comprise a molecule comprising:

the antigen-binding fragment of the antibody that binds to human GPRC5D comprising a heavy chain comprising an amino acid sequence represented by amino acid residues 24 to 475 of the amino acid sequence represented by SEQ ID NO: 215 and a light chain comprising an amino acid sequence represented by amino acid residues 24 to 237 of the amino acid sequence represented by SEQ ID NO: 217, and the antigen-binding fragment of the antibody that binds to human CD3 and cynomolgus monkey CD3 comprising an amino acid sequence represented by amino acid residues 24 to 499 of the amino acid sequence represented by SEQ ID NO: 219;
the antigen-binding fragment of the antibody that binds to human GPRC5D comprising a heavy chain comprising an amino acid sequence represented by amino acid residues 24 to 475 of the amino acid sequence represented by SEQ ID NO: 215 and a light chain comprising an amino acid sequence represented by amino acid residues 24 to 237 of the amino acid sequence represented by SEQ ID NO: 217, and the antigen-binding fragment of the antibody that binds to human CD3 and cynomolgus monkey CD3 comprising an amino acid sequence represented by amino acid residues 24 to 497 of the amino acid sequence represented by SEQ ID NO: 221;
the antigen-binding fragment of the antibody that binds to human GPRC5D comprising a heavy chain comprising an amino acid sequence represented by amino acid residues 24 to 475 of the amino acid sequence represented by SEQ ID NO: 215 and a light chain comprising an amino acid sequence represented by amino acid residues 24 to 237 of the amino acid sequence represented by SEQ ID NO: 217, and the antigen-binding fragment of the antibody that binds to human CD3 and cynomolgus monkey CD3 comprising an amino acid sequence represented by amino acid residues 24 to 497 of the amino acid sequence represented by SEQ ID NO: 225;
the antigen-binding fragment of the antibody that binds to human GPRC5D comprising a heavy chain comprising an amino acid sequence represented by amino acid residues 24 to 475 of the amino acid sequence represented by SEQ ID NO: 215 and a light chain comprising an amino acid sequence represented by amino acid residues 24 to 237 of the amino acid sequence represented by SEQ ID NO: 217, and the antigen-binding fragment of the antibody that binds to human CD3 and cynomolgus monkey CD3 comprising an amino acid sequence represented by amino acid residues 24 to 499 of the amino acid sequence represented by SEQ ID NO: 227;
the antigen-binding fragment of the antibody that binds to human GPRC5D comprising a heavy chain comprising an amino acid sequence represented by amino acid residues 24 to 475 of the amino acid sequence represented by SEQ ID NO: 215 and a light chain comprising an amino acid sequence represented by amino acid residues 24 to 237 of the amino acid sequence represented by SEQ ID NO: 217, and the antigen-binding fragment of the antibody that binds to human CD3 and cynomolgus monkey CD3 comprising an amino acid sequence represented by amino acid residues 24 to 499 of the amino acid sequence represented by SEQ ID NO: 229;
the antigen-binding fragment of the antibody that binds to human GPRC5D comprising a heavy chain comprising an amino acid sequence represented by amino acid residues 24 to 476 of the amino acid sequence represented by SEQ ID NO: 237 and a light chain comprising an amino acid sequence represented by amino acid residues 24 to 237 of the amino acid sequence represented by SEQ ID NO: 217, and the antigen-binding fragment of the antibody that binds to human CD3 and cynomolgus monkey CD3 comprising an amino acid sequence represented by amino acid residues 24 to 498 of the amino acid sequence represented by SEQ ID NO: 231;
the antigen-binding fragment of the antibody that binds to human GPRC5D comprising a heavy chain comprising an amino acid sequence represented by amino acid residues 24 to 476 of the amino acid sequence represented by SEQ ID NO: 237 and a light chain comprising an amino acid sequence represented by amino acid residues 24 to 237 of the amino acid sequence represented by SEQ ID NO: 217, and the antigen-binding fragment of the antibody that binds to human CD3 and cynomolgus monkey CD3 comprising an amino acid sequence represented by amino acid residues 24 to 500 of the amino acid sequence represented by SEQ ID NO: 233; or
the antigen-binding fragment of the antibody that binds to human GPRC5D comprising a heavy chain comprising an amino acid sequence represented by amino acid residues 24 to 476 of the amino acid sequence represented by SEQ ID NO: 237 and a light chain comprising an amino acid sequence represented by amino acid residues 24 to 237 of the amino acid sequence represented by SEQ ID NO: 217, and the antigen-binding fragment of the antibody that binds to human CD3 and cynomolgus monkey CD3 comprising an amino acid sequence represented by amino acid residues 24 to 500 of the amino acid sequence represented by SEQ ID NO: 235.

Specific examples of more preferable examples from among these of can include a molecule comprising
the antigen-binding fragment of the antibody that binds to human GPRC5D comprising a heavy chain comprising an amino acid sequence represented by amino acid residues 24 to 475 of the amino acid sequence represented by SEQ ID NO: 215 and a light chain comprising an amino acid sequence represented by amino acid residues 24 to 237 of the amino acid sequence represented by SEQ ID NO: 217, and the antigen-binding fragment of the antibody that binds to human CD3 and cynomolgus monkey CD3 comprising an amino acid sequence represented by amino acid residues 24 to 497 of the amino acid sequence represented by SEQ ID NO: 225;
the antigen-binding fragment of the antibody that binds to human GPRC5D comprising a heavy chain comprising an amino acid sequence represented by amino acid residues 24 to 475 of the amino acid sequence represented by SEQ ID NO: 215 and a light chain comprising an amino acid sequence represented by amino acid residues 24 to 237 of the amino acid sequence represented by SEQ ID NO: 217, and the antigen-binding fragment of the antibody that binds to human CD3 and cynomolgus monkey CD3 comprising an amino acid sequence represented by amino acid residues 24 to 499 of the amino acid sequence represented by SEQ ID NO: 227;
the antigen-binding fragment of the antibody that binds to human GPRC5D comprising a heavy chain comprising an amino acid sequence represented by amino acid residues 24 to 475 of the amino acid sequence represented by SEQ ID NO: 215 and a light chain comprising an amino acid sequence represented by amino acid residues 24 to 237 of the amino acid sequence represented by SEQ ID NO: 217, and the antigen-binding fragment of the antibody that binds to human CD3 and cynomolgus monkey CD3 comprising an amino acid sequence represented by amino acid residues 24 to 499 of the amino acid sequence represented by SEQ ID NO: 229;
the antigen-binding fragment of the antibody that binds to human GPRC5D comprising a heavy chain comprising an amino acid sequence represented by amino acid residues 24 to 476 of the amino acid sequence represented by SEQ ID NO: 237 and a light chain comprising an amino acid sequence represented by amino acid residues 24 to 237 of the amino acid sequence represented by SEQ ID NO: 217, and the antigen-binding fragment of the antibody that binds to human CD3 and cynomolgus monkey CD3 comprising an amino acid sequence represented by amino acid residues 24 to 498 of the amino acid sequence represented by SEQ ID NO: 231;
the antigen-binding fragment of the antibody that binds to human GPRC5D comprising a heavy chain comprising an amino acid sequence represented by amino acid residues 24 to 476 of the amino acid sequence represented by SEQ ID NO: 237 and a light chain comprising an amino acid sequence represented by amino acid residues 24 to 237 of the amino acid sequence represented by SEQ ID NO: 217, and the antigen-binding fragment of the antibody that binds to human CD3 and cynomolgus monkey CD3 comprising an amino acid sequence represented by amino acid residues 24 to 500 of the amino acid sequence represented by SEQ ID NO: 233; or
the antigen-binding fragment of the antibody that binds to human GPRC5D comprising a heavy chain comprising an amino acid sequence represented by amino acid residues 24 to 476 of the amino acid sequence represented by SEQ ID NO: 237 and a light chain comprising an amino acid sequence represented by amino acid residues 24 to 237 of the amino acid sequence represented by SEQ ID NO: 217, and the antigen-binding fragment of the antibody that binds to human CD3 and cynomolgus monkey CD3 comprising an amino acid sequence represented by amino acid residues 24 to 500 of the amino acid sequence represented by SEQ ID NO: 235.

A hybrid-type bispecific molecule to be mentioned below is included in the applicable form of these molecules.

An alternative example of a more preferable molecule of the multispecific molecule of the present invention can include a molecule wherein the antibody or the antigen-binding fragment of the antibody that binds to human GPRC5D derived from 2B1 preferably comprises a heavy chain variable region comprising an amino acid sequence represented by amino acid residues 20 to 142 of the amino acid sequence represented by SEQ ID NO: 76 and a light chain variable region comprising an amino acid sequence represented by amino acid residues 21 to 127 of the amino acid sequence represented by SEQ ID NO: 72, further comprising the constant regions according to the above i), ii), iii), or iv), and the antibody or the antigen-binding fragment of the antibody that binds to human CD3 and cynomolgus monkey CD3 further comprises

v) a heavy chain constant region comprising an amino acid sequence represented by amino acid residues 143 to 471 of the amino acid sequence represented by SEQ ID NO: 207, and a light chain constant region comprising an amino acid sequence represented by amino acid residues 24 to 132 of the amino acid sequence represented by SEQ ID NO: 209, or

vi) a heavy chain constant region comprising an amino acid sequence represented by amino acid residues 143 to 471 of the amino acid sequence represented by SEQ ID NO: 211, and a light chain constant region comprising an amino acid sequence represented by amino acid residues 131 to 236 of the amino acid sequence represented by SEQ ID NO: 213.

A more preferable molecule from among these molecules is one wherein the antibody or antigen-binding fragment of the antibody that binds to human GPRC5D is an antibody or an antigen-binding fragment of the antibody that binds to human GPRC5D, comprising a constant region indicated by the above iii) or iv).

A more specific preferable example of the multispecific molecule of the present invention can comprise

the antigen-binding fragment of the antibody that binds to human GPRC5D comprising a heavy chain comprising an amino acid sequence represented by amino acid residues 24 to 475 of the amino acid sequence represented by SEQ ID NO: 199 and a light chain comprising an amino acid sequence represented by amino acid residues 24 to 237 of the amino acid sequence represented by SEQ ID NO: 203, and the antigen-binding fragment of the antibody that binds to human CD3 and cynomolgus monkey CD3 comprising a heavy chain comprising an amino acid sequence represented by amino acid residues 25 to 471 of the amino acid sequence represented by SEQ ID NO: 207 and a light chain comprising an amino acid sequence represented by amino acid residues 24 to 238 of the amino acid sequence represented by SEQ ID NO: 209

or

the antigen-binding fragment of the antibody that binds to human GPRC5D comprising a heavy chain comprising an amino acid sequence represented by amino acid residues 24 to 475 of the amino acid sequence represented by SEQ ID NO: 201 and a light chain comprising an amino acid sequence represented by amino acid residues 24 to 237 of the amino acid sequence represented by SEQ ID NO: 205, and the antigen-binding fragment of the antibody that binds to human CD3 and cynomolgus monkey CD3 comprising a heavy chain comprising an amino acid sequence represented by amino acid residues 25 to 471 of the amino acid sequence represented by SEQ ID NO: 211 and a light chain comprising an amino acid sequence represented by amino acid residues 24 to 236 of the amino acid sequence represented by SEQ ID NO: 213.

An FSA-type bispecific molecule to be mentioned below is included in the applicable form of these molecules.

A further alternative preferable example of the multispecific molecule of the present invention a molecule according to claim 55, wherein the antibody that binds to human GPRC5D or an antigen-binding fragment of the antibody comprises a heavy chain variable region comprising an amino acid sequence represented by amino acid residues 20 to 142 of the amino acid sequence represented by SEQ ID NO: 76 and a light chain variable region comprising an amino acid sequence represented by amino acid residues 21 to 127 of the amino acid sequence represented by SEQ ID NO: 72, and a further Fc mutation, and the antibody that binds to human CD3 and cynomolgus monkey CD3 or the antigen-binding fragment of the antibody further comprises an Fc mutation.

A specific preferable example of such a multispecific molecule of the present invention can include a molecule comprising

the antibody that binds to human GPRC5D or the antigen-binding fragment of the antibody comprising an amino acid sequence represented by amino acid residues 24 to 271 of the amino acid sequence represented by SEQ ID NO: 223, and an Fc mutation; and the antibody that binds to human CD3 and cynomolgus monkey CD3 or the antigen-binding fragment of the antibody comprising an amino acid sequence represented by amino acid residues 24 to 266 of the amino acid sequence represented by SEQ ID NO: 219, and an Fc mutation

or

the antibody that binds to human GPRC5D or the antigen-binding fragment of the antibody comprising an amino acid sequence represented by amino acid residues 24 to 271 of the amino acid sequence represented by SEQ ID NO: 223, and an Fc mutation; and the antibody that binds to human CD3 and cynomolgus monkey CD3 or the antigen-binding fragment of the antibody comprising an amino acid sequence represented by amino acid residues 24 to 264 of the amino acid sequence represented by SEQ ID NO: 221, and an Fc mutation.

A dual-type bispecific molecule to be mentioned below is comprised in the applicable form of these molecules.

The molecule of the present invention also encompasses a molecule comprising a moiety of an anti-CD3 antibody or an antigen-binding fragment thereof that comprises an amino acid sequence encoded by a nucleotide sequence contained in a polynucleotide hybridizing under stringent conditions to a complementary strand of a polynucleotide comprising a nucleotide sequence encoding an amino acid sequence contained in the anti-CD3 antibody mentioned above or antigen-binding fragment of the antibody, and binds to human CD3 and cynomolgus monkey CD3, and a moiety of an anti-GPRC5D antibody or an antigen-binding fragment thereof that binds to human GPRC5D and preferably further binds to cynomolgus monkey GPRC5D.

The molecule of the present invention also encompasses a molecule comprising a moiety of an anti-CD3 antibody or an antigen-binding fragment thereof that comprises an amino acid sequence of a heavy chain variable region and/or an amino acid sequence of a light chain variable region 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% or more identical to the amino acid sequence of the heavy chain variable region and/or the amino acid sequence of the light chain variable region contained in the anti-CD3 antibody mentioned above or antigen-binding fragment of the antibody, and binds to human CD3 and cynomolgus monkey CD3, and a moiety of an anti-GPRC5D antibody or an antigen-binding fragment thereof that binds to human GPRC5D and preferably further binds to cynomolgus monkey GPRC5D.

The molecule of the present invention also encompasses a molecule that comprises an anti-CD3 antibody or an antigen-binding fragment of the antibody having an amino acid sequence derived from an amino acid sequence contained in the anti-CD3 antibody or antigen-binding fragment of the antibody by the substitution, deletion, or modification of 1 to several amino acid(s), and binds to human CD3 and cynomolgus monkey CD3, and to human GPRC5D, and preferably further binds to cynomolgus monkey GPRC5D. Examples of such an anti-CD3 antibody or antigen-binding fragment of the antibody can comprise a deletion variant derived from a heavy chain by the deletion of 1 or 2 amino acid(s) at its carboxyl terminus (Journal of Chromatography A, 705: 129-134 (1995)), an amidated form of the deletion variant having a heavy chain that lacks two amino acid residues (glycine and lysine) at the carboxy terminus and instead has an amidated proline residue at the carboxy terminus (Analytical Biochemistry, 360: 75-83 (2007)), and a modified antibody having a pyroglutamylated amino-terminal glutamine or glutamic acid residue in its heavy or light chain (International Publication No. WO2013/147153) (these are collectively referred to as "deletion variants"). However, the deletion variant at the carboxyl terminus of the heavy or light chain of the anti-CD3 antibody or the antigen-binding fragment of the antibody contained in the molecule of the present invention is not limited to the types described above as long as the deletion variant maintains its antigen binding ability and the effector functions. When the antibody contained in the molecule of the present invention comprises two or more chains (e.g., heavy chains), the two or more chains (e.g., heavy chains) may be heavy chains of any one type selected from a group consisting of the full-length heavy chains and the deletion variants described above, or may be a combination of heavy chains of any two or more types selected therefrom. The quantitative ratio of each deletion variant or the ratio of the number of molecules thereof may be influenced by the type of cultured mammalian cells producing the molecule of the present invention, and culture conditions. Examples of such a case can include the deletion of one carboxyl-terminal amino acid residue each in both of the two heavy chains as main components of the molecule of the present invention.

## 4-2. Bispecific molecule of the present invention

Preferred examples of the multispecific molecule of the present invention can include bispecific molecules.

The term "bispecific" means the capability of binding to two epitopes different from each other on one molecule, or epitopes different from each other on two or more molecules. An antibody or an antigen-binding fragment having such bispecificity is encompassed by the present invention.

The bispecific molecule of the present invention binds to GPRC5D and binds to an epitope that is absent in GPRC5D and present in another antigen. More specifically, such a bispecific molecule (i) binds to an epitope on GPRC5D (epitope 1) and (ii) binds to an epitope different from the epitope 1 on GPRC5D (epitope 2), or binds to an epitope on an antigen other than GPRC5D (epitope 3).

For example, in a tandem scFv-type bispecific molecule typified by BiTE, an antigen-binding site in the heavy chain variable region of a first antibody and an antigen-binding site in the light chain variable region of the first antibody are linked either via a linker or directly without a linker to form a first polypeptide. Also, an antigen-binding site in the heavy chain variable region of a second antibody and an antigen-binding site in the light chain variable region of the second antibody are linked either via a linker or directly without a linker to form a second polypeptide. The first polypeptide and the second polypeptide are linked either via a linker or directly without a linker. Alternatively, the first polypeptide and the second polypeptide may be linked via an additional molecule.

In a diabody-type bispecific molecule, an antigen-binding site in the heavy chain variable region of a first antibody and an antigen-binding site in the light chain variable region of a second antibody are linked either via a linker or directly without a linker. Also, an antigen-binding site in the light chain variable region of the first antibody and an antigen-binding site in the heavy chain variable region of the second antibody are linked either via a linker or directly without a linker. Also, a bispecific molecule may be prepared by the further dimerization of diabody-type bispecific molecules. In addition, the diabody-type bispecific molecule may be linked to one single chain or both chains of Fc via a linker (diabody-Fc-type bispecific molecule).

In a dual scFv-type bispecific molecule, two scFvs that binds to different epitopes are linked to two chains of dimeric Fc, respectively, either via linkers or directly without linkers. Alternatively, two types of scFvs that binds to different epitopes are linked to CH and CL, respectively, via linkers and further linked to two chains of dimeric Fc, respectively, via linkers. A dual scFv-type bispecific molecule is also described as a dual-type bispecific molecule or a dual-type.

In an IgG-type bispecific molecule, two Fabs that binds to different epitopes are linked to two chains of dimeric Fc, respectively, either via linkers or directly without linkers. An IgG-type bispecific molecule is also described as a full-size antibody (FSA)-type bispecific molecule or an FSA-type.

Alternatively, the bispecific molecule of the present invention may be a bispecific molecule in which Fab of a first antibody and scFv of a second antibody are linked to two chains of dimeric Fc, respectively, either via linkers or directly without linkers. Such a bispecific antibody is described as a hybrid-type bispecific antibody or a hybrid-type.

The scFv and the Fab contained in the bispecific molecule of the present invention are preferably scFv and Fab of a humanized antibody or a human antibody, and the Fc is preferably Fc of a human antibody.

The linker also comprises a single chain polypeptide or a single chain oligopeptide, or synthetic products such as PEG, nucleotides, sugar chains, and compounds. In addition, any linker known in the art may be used without particular limitations as long as the linker links two polypeptides.

The length of the linker is 5 to 30 amino acids for, for example, a peptide linker. When the bispecific molecule contains a plurality of linkers, all the peptide linkers used may have the same length or the peptide linkers used may have different lengths.

Examples of the peptide linker include a (Gly·Gly·Gly·Gly·Ser) repeat. 1 to several amino acid residues other than Gly and Ser may be added thereto.

Examples of the antibody that binds to an epitope on an antigen other than GPRC5D (epitope 3) or antigen-binding fragment thereof contained in the bispecific molecule of the present invention can comprise the aforementioned anti-CD3 antibody or antigen-binding fragment thereof.

Examples of the bispecific molecule of the present invention can include a molecule in which the anti-CD3 antibody or antigen-binding fragment of the antibody is bound with the anti-GPRC5D antibody of the present invention or antigen-binding fragment of the antibody via a linker or without a linker. Preferred examples of such a molecule can include a molecule in which the anti-CD3 antibody or antigen-binding fragment of the antibody and the anti-GPRC5D antibody of the present invention or antigen-binding fragment of the antibody, each of which is scFv, are bound with each other via a linker or without a linker.

Preferred specific examples of such a molecule can include a molecule that has an amino acid sequence represented by any one of SEQ ID NOs: 171 to 179, and binds to human CD3 and cynomolgus monkey CD3, and to human GPRC5D, and preferably further binds to cynomolgus monkey GPRC5D.

5. Production of antibody and molecule

5-1. Method using hybridoma

The anti-GPRC5D antibody of the present invention can be obtained by use of a routine method which involves immunizing an animal with GPRC5D or an arbitrary polypeptide selected from the amino acid sequence of GPRC5D and collecting and purifying the antibody produced *in vivo.* According to a method known in the art (e.g., Kohler and Milstein, Nature (1975) 256, p. 495-497; and Kennet, R. ed., Monoclonal Antibodies, p. 365-367, Plenum Press, N.Y. (1980)), antibody-producing cells that produce antibodies against GPRC5D are fused with myeloma cells to thereby establish hybridomas, from which monoclonal antibodies can also be obtained. Specific examples of such a method are described in, for example, International Publication No. WO09/48072 pamphlet (published on April 16, 2009).

The organism species of the antigen GPRC5D is not limited to a human, and the animal can also be immunized with GPRC5D derived from a nonhuman animal such as a mouse or a rat. In this case, the obtained antibody that binds to nonhuman GPRC5D can be tested for its cross-reactivity with human GPRC5D to thereby select an antibody applicable to human diseases.

The anti-CD3 antibody that can be contained in the molecule of the present invention can also be obtained by use of a routine method which involves immunizing an animal with CD3 or an arbitrary polypeptide selected from the amino acid sequence of CD3 and collecting and purifying the antibody produced *in vivo.* According to a method known in the art, antibody-producing cells that produce antibodies against CD3 are fused with myeloma cells to thereby establish hybridomas, from which monoclonal antibodies can also be obtained.

The organism species of the antigen CD3 is not limited to a human, and the animal can also be immunized with CD3 derived from a nonhuman animal such as a mouse or a rat. The obtained antibody that binds to nonhuman CD3 can be tested for its cross-reactivity with human CD3 to thereby select an antibody applicable to human diseases.

5-2. Cell immunization method

Cells expressing the native antigen, cells expressing the recombinant antigen or its fragment, or the like, can be used as immunogens to thereby prepare an antibody by the hybridoma method described above.

Examples of the cells expressing the native GPRC5D can include human plasma cells, human multiple myeloma patient-derived primary cultured cells, and human multiple myeloma patient-derived cultured cell lines. Examples of the cells expressing the native CD3 can include human thymus cells and T lymphocytes.

Such cells are used in an amount of $1 \times 10^5$ to $1 \times 10^9$ cells, preferably $1 \times 10^6$ to $1 \times 10^8$ cells, more preferably 0.5 to $2 \times 10^7$ cells, even more preferably $1 \times 10^7$ cells, per immunization shot. The number of cells used for immunization can be changed according to the expression level of antigen. The immunogens are generally administered intraperitoneally and may be administered through an intradermal route or the like.

5-3. DNA immunization method

The anti-GPRC5D antibody of the present invention and the anti-CD3 antibody that can be contained in the molecule of the present invention (hereinafter, these antibodies are also collectively referred to as the antibody of the present invention) can also be obtained by use of a DNA immunization method. This method involves transfecting an animal (e.g., mouse or rat) individual with an antigen expression plasmid and expressing the antigen in the individual to thereby induce immunity against the antigen. Examples of the transfection approach include a method of directly injecting the plasmid to the muscle, a method of injecting a transfection reagent such as a liposome or polyethylenimine to the vein, an approach using a viral vector, an approach of injecting gold particles attached with the plasmid using a gene gun, and a hydrodynamic method of rapidly injecting a plasmid solution in a large amount to the vein.

Actual examples of the rat anti-human GPRC5D antibody thus established can include 2A4, 2B1, and 7B4. The amino acid sequence of the heavy chain variable region of 2A4 is represented by SEQ ID NO: 5 of the Sequence Listing. The amino acid sequence of the light chain variable region of 2A4 is represented by SEQ ID NO: 12 of the Sequence Listing. The amino acid sequence of the heavy chain variable region of 2B1 is represented by SEQ ID NO: 7 of the Sequence Listing. The amino acid sequence of the light chain variable region of 2B1 is represented by SEQ ID NO: 14 of the Sequence Listing. The amino acid sequence of the heavy chain variable region of 7B4 is represented by SEQ ID NO: 9 of the Sequence Listing. The amino acid sequence of the light chain variable region of 7B4 is represented by SEQ ID NO: 16 of the Sequence Listing.

5-4. Designing humanized antibody

Examples of the humanized antibody can include, but are not limited to, a human-derived antibody having CDRs replaced only with the CDRs of a non-human animal antibody (see Nature (1986), 321, p. 522-525), a human antibody grafted with the CDR sequences and with some amino acid residues of framework regions by CDR grafting (see WO90/07861 and US6972323), and an antibody having human antibody amino acid(s) replaced for one or two or more non-human animal antibody-derived amino acid(s) in any of these humanized antibodies.

5-5. Designing human antibody

The human antibody means an antibody consisting of the amino acid sequence of a human-derived antibody. The human antibody can be obtained by a method using human antibody-producing mice carrying human genomic DNA fragments comprising human antibody heavy and light chain genes (see e.g., Tomizuka, K.et al., Nature Genetics (1997) 16, 133-143,; Kuroiwa, Y.et.al., Nuc. Acids Res. (1998) 26, 3447-3448; Yoshida, H. et. al., Animal Cell Technology: Basic and Applied Aspects vol.10, 69-73 (Kitagawa, Y., Matuda, T. and Iijima, S. eds.), Kluwer Academic Publishers, 1999.; Tomizuka, K.et.al., Proc. Natl. Acad. Sci. USA (2000) 97, 722-727).

Specifically, such human antibody-producing animals can be prepared by disrupting the endogenous immunoglobulin heavy and light chain gene loci of non-human mammals and instead introducing thereto human immunoglobulin heavy and light chain gene loci via yeast artificial chromosome (YAC) vectors or the like. Alternatively, eukaryotic cells may be transformed with cDNAs encoding the heavy and light chains, respectively, of such a human antibody, preferably with vectors comprising the cDNAs, by a gene recombination technique. The transformed cells producing a recombinant human monoclonal antibody can be cultured. This antibody can be obtained from the culture supernatant.

In this context, for example, eukaryotic cells, preferably mammalian cells such as HEK293F cells or CHO cells, can be used as the hosts.

Also, a method for obtaining a phage display-derived human antibody selected from a human antibody library is also known. For example, a phage display method can be used, which involves allowing the variable regions of a human antibody to be expressed as scFv on phage surface and selecting a phage that binds to the antigen. The phage selected on the basis of its ability to bind to the antigen can be subjected to gene analysis to thereby determine DNA sequences encoding the variable regions of the human antibody that binds to the antigen. If the DNA sequence of scFv that binds to the antigen is determined, an expression vector having this sequence can be prepared and introduced to appropriate hosts to allow them to express the human antibody (WO92/01047, WO92/20791, WO93/06213, WO93/11236, WO93/19172, WO95/01438, WO95/15388, Annu. Rev. Immunol (1994) 12, 433-455).

The method for constructing the human antibody phage library is well known. Genes of human antibody variable regions are amplified using cDNAs collected from human blood, spleen, or lymph node as templates using primers with reference to, for example, J Biol Chem, 274 (26), 18218-30, (1999) or Methods Mol Biol, 178, 59-71, (2002). The amplified variable region genes can be used to prepare scFv with reference to J Immunol Methods, 201 (1), 35-55 (1997).

5-6. Production of antigen-binding fragment of antibody

The antigen-binding fragment of the antibody can be produced by modifying the antibody by a genetic engineering method, followed by expression in appropriate cultured cells.

The method for preparing, for example, scFv, as the antigen-binding fragment of the antibody is well known in the art (see e.g., U.S. Patent Nos. 4,946,778, 5,260,203, 5,091,513, and 5,455,030). In this scFv, a heavy chain variable region and a light chain variable region are linked via a linker that prevents them from forming a conjugate, preferably a polypeptide linker (Huston, J.S. et al., PNAS (1988), 85, 5879-5883). The heavy chain variable region and the light chain variable region in scFv may be derived from the same antibody or may be derived from different antibodies.

For example, an arbitrary single chain peptide consisting of 5 to 30 residues is used as the polypeptide linker that links these variable regions.

In order to obtain scFv-encoding DNA, of the sequences of DNA encoding the heavy chain or heavy chain variable region of the antibody and DNA encoding the light chain or light chain variable region thereof, each DNA portion encoding the whole of these sequences or the desired amino acid sequence is used as a template and amplified by PCR using a primer pair flanking both ends of the template. Subsequently, DNA encoding the polypeptide linker moiety is further amplified in combination with a primer pair flanking both ends of the DNA so that the obtained fragment can be linked at its ends to the heavy and light chain DNAs, respectively. Alternatively, the DNA encoding the whole scFv region may be obtained by net synthesis.

The scFv-encoding DNA can be used to thereby prepare, according to a routine method, an expression vector containing the DNA and host cells transformed with the expression vector. In addition, the host cells can be cultured, and the scFv can be recovered from the cultures according to a routine method.

Also in order to obtain any other antigen-binding fragment of the antibody, a gene encoding an antigen-binding fragment is obtained according to the method described above and introduced into cells. The antigen-binding fragment of interest can be recovered from cultures of the cells.

The antibody of the present invention may be multimerized to thereby enhance its affinity for the antigen. In this case, antibodies of the same type may be multimerized, or a plurality of antibodies recognizing a plurality of epitopes, respectively, of the same antigen may be multimerized. Examples of methods for multimerizing these antibodies can include the binding of two scFvs to an IgG CH3 domain, the binding thereof to streptavidin, and the introduction of a helix-turn-helix motif.

5-7. Gene recombination

In order to prepare the antibody of the present invention, a polynucleotide (heavy chain nucleotide) comprising a nucleotide sequence encoding the amino acid sequence of its heavy chain and a polynucleotide (light chain nucleotide) comprising a nucleotide sequence encoding the amino acid sequence of its light chain, or a vector having an insert of the heavy chain nucleotide and a vector having an insert of the light chain nucleotide are introduced into host cells, and then the cells are cultured, and the antibody can be recovered from the cultures. The heavy chain nucleotide and the light chain nucleotide may be inserted in one vector.

Prokaryotic or eukaryotic cells can be used as the host cells. In the case of using host eukaryotic cells, animal cells, plant cells, or eukaryotic microbes can be used.

Examples of the animal cells can include mammal-derived cells, i.e., human embryonic kidney cells HEK293F cells (Subedi GP et al., J Vis Exp. (2015) 106), monkey kidney-derived COS cells (Gluzman, Y. Cell (1981), 23, 175-182, ATCC CRL-1650), mouse fibroblast NIH3T3 (ATCC No. CRL-1658), Chinese hamster ovary cells (CHO cells, ATCC CCL-61), dihydrofolate reductase-deficient lines thereof (CHOdhfr-; Urlaub, G. and Chasin, L.A. PNAS (1980), 77, 4126-4220), cells derived from birds such as chickens, and cells derived from insects.

Also, cells modified to enhance the biological activities of antibodies by the modification of sugar chain structures can be used as the hosts. For example, CHO cells modified such that the proportion of sugar chains with fucose unbound with N-acetylglucosamine at their reducing ends is 20% or more among complex-type N-glycoside-linked sugar chains that binds to the Fc region of the antibody, may be used to prepare an antibody having enhanced ADCC activity or CDC activity (International Publication No. WO02/31140).

Examples of the eukaryotic microbes can include yeasts. Examples of the prokaryotic cells can include *E. coli* and *Bacillus subtilis.*

A signal peptide for the secretion of the antibody of the present invention (monoclonal antibody derived from each animal, rat antibody, mouse antibody, chimeric antibody, humanized antibody, human antibody, etc.) is not limited to the secretory signal of an antibody of the same species, the same type, and the same subtype as the antibody of the present invention or to the secretory signal of the antibody of the present invention itself. Any secretory signal of an antibody of different type or subtype therefrom or any secretory signal of a protein derived from a different eukaryotic species therefrom or a prokaryotic species can be selected and used. The signal peptide is usually not contained in the nucleotide sequences and amino acid sequences of most of mature light chains or mature heavy chains. A secreted antibody, etc. containing the signal peptide is also encompassed by the antibody, etc. of the present invention or the molecule of the present invention.

The obtained antibody, antigen-binding fragment of the antibody, and molecule can be purified until homogeneous so as not to contain other proteins. Usual protein separation and purification methods can be used for the separation and purification of the antibody, antigen-binding fragment of the antibody, and molecule.

The antibody can be separated and purified by appropriately selected or combined approach(es), for example, chromatography columns, filters, ultrafiltration, salting out, dialysis, preparative polyacrylamide gel electrophoresis, and/or isoelectric focusing, though the separation and purification method is not limited thereto.

The separation and purification method can be preferably performed, for example, by preparing an expression vector using a DNA sequence encoding a His tag or a FLAG tag added to the carboxyl terminus of an antibody variable region, transforming cells with this vector, then culturing the cells to express the antibody and the antigen-binding fragment of the antibody, and extracting the culture supernatant after the completion of the culture, followed by purification using metal (e.g., Ni or Co) affinity chromatography, anti-FLAG tag antibody columns, gel filtration, ionexchange chromatography, or the like.

The expressed antibody and the antigen-binding fragment of the antibody containing an amino acid sequence of a tag such as a His tag or a FLAG tag is also encompassed by the antibody of the present invention, antigen-binding fragment of the antibody, or the molecule of the present invention.

5-8. Production of polyclonal antibody

The antibody of the present invention may be a polyclonal antibody. The polyclonal antibody can be recovered from cultures of mixed-cultured different antibody-producing cells (WO2004/061104). Alternatively, separately prepared antibodies may be mixed. Antiserum, which is one aspect of the polyclonal antibody, can be prepared by immunizing animals with a desired antigen and recovering serum from the animals according to a standard method.

5-9. Production of artificial immunocyte provided with binding specificity for tumor cell

An artificial immunocyte provided with binding specificity for tumor cells can be produced by imparting antigen specificity to an immunocyte by transfection with, for example, the gene of the anti-GPRC5D antibody of the present invention. Examples of the immunocyte include T cells, NK cells, and monocytes.

The case of using a T cell as the immunocyte will be described as an example. The T cell can be induced by culturing mononuclear cells recovered from human peripheral blood by a method such as a specific gravity centrifugal method, in a medium in the presence of an anti-CD3 antibody, IL-2, IL-12, or further, an anti-IL-4 antibody or IFN-$\gamma$.

Next, this T cell is transfected with a chimeric antigen receptor (CAR) gene comprising the gene of the anti-GPRC5D antibody of the present invention as a component. The CAR gene is typically constituted by a gene of an antibody that recognizes a surface antigen on tumor cells (in the present invention, the anti-GPRC5D antibody) and a gene encoding a co-stimulatory molecule necessary for T cell activation (e.g., co-stimulators such as T cell receptor $\zeta$

chain and CD28). The CAR gene comprising the gene of the anti-GPRC5D antibody can be transfected to the T cell using various viral vectors. Examples of such a vector include lentivirus vectors, retrovirus vectors, adenovirus vectors, adeno-associated virus vectors, Sendai virus vectors, and liposomes. A recombinant virus can be prepared from the viral vector having an insert of the gene of the anti-GPRC5D antibody and transfected to the antigen-nonspecifically activated T cell mentioned above. The T cell thus transfected with the gene can be cultured to obtain a T cell provided with specificity for tumor cells.

Whether the T cell of the present invention capable of inducing cytotoxicity by redirection, obtained by the method of the present invention, has the antigen specificity thus imparted thereto can be confirmed by coculturing the T cell with inactivated cells obtained by the mitomycin C treatment of antigen-positive tumor cells known to express GPRC5D, and then measuring the amount of IFN-γ or IL-2 in the culture supernatant.

5-10. Production of multispecific molecule and bispecific molecule

Examples of the method for preparing the multispecific molecule and the bispecific molecule of the present invention include a method which involves introducing expression plasmids to host cells, followed by transient expression, a method which involves introducing plasmids to host cells and then selecting a stably expressing cell line by drug selection, followed by constitutive expression, and a method which involves preparing respective antibodies or antigen-binding fragments by any of the methods described above, and then chemically linking these antibodies or fragments using a synthetic peptide linker.

As for the single chain antibody (scFv), examples of the preparation method include a method which involves linking two scFvs via a peptide linker (tandem scFv), a method which involves interchanging the respective domains of two antibodies differing in specificity, and forming a dimer by a noncovalent bond (diabody), a method which involves interchanging the respective domains of two antibodies differing in specificity, and forming a single chain (single chain diabody), and a method which involves preparing single chain diabodies and then forming a dimer by a noncovalent bond (TandAb, U.S. Patent No. 7129330).

The present invention also provides a gene encoding the antibody of the present invention or antigen-binding fragment of the antibody, or a modified form of the antigen, etc., a recombinant vector having an insert of the gene, a cell transfected with the gene or vector, and even a cell producing the antibody of the present invention.

6. Pharmaceutical composition

The present invention provides the anti-GPRC5D antibody or antigen-binding fragment thereof, the polynucleotide, vector, cell, and artificial immunocyte of the present invention, and/or a pharmaceutical composition comprising the molecule comprising at least one of them, as an active ingredient (hereinafter, also referred to as the pharmaceutical composition of the present invention).

The pharmaceutical composition of the present invention is useful in the treatment or prevention of various diseases related to abnormal or increased GPRC5D signals due to overexpression of GPRC5D or its ligand or GPRC5D mutations or gene amplification (hereinafter, these diseases are referred to as "GPRC5D-related diseases"), particularly, various cancers.

Examples of causes of the initiation or exacerbation of such cancers to be treated or prevented can include high expression of GPRC5D, single nucleotide polymorphism (SNP) in an intron of the GPRC5D gene, missense mutations that constitutively activate GPRC5D, and amplification or overexpression of the GPRC5D gene.

Also, the molecule of the present invention or the pharmaceutical composition of the present invention can include cytotoxicity to cells expressing GPRC5D by the redirection of immunocytes such as T cells to the cells. Therefore, the present invention provides a method for inducing cytotoxicity to cells expressing GPRC5D by the redirection of immunocytes such as T cells to the cells, including a step of administering the molecule of the present invention or the pharmaceutical composition of the present invention.

Examples of the cancer types to be treated or prevented with the pharmaceutical composition of the present invention can include cancers expressing the GPRC5D protein, for example, breast cancer, endometrial cancer, ovary cancer, lung cancer (e.g., non-small cell lung cancer), stomach cancer, prostate cancer, kidney cancer, liver cancer, pancreatic cancer, colorectal cancer, esophageal cancer, urinary bladder cancer, uterine cervix cancer, blood cancer, lymphoma, and malignant melanoma. Preferred examples thereof can include multiple myeloma expressing the GPRC5D protein.

The treatment or prevention of a GPRC5D-related disease includes, but is not limited to, the prevention of the onset of the disease in an individual expressing the GPRC5D protein, the suppression or inhibition of exacerbation or progression thereof, the alleviation of one or two or more symptoms exhibited by an individual affected with the disease, the suppression or remission of exacerbation or progression thereof, the treatment or prevention of a secondary disease in an individual affected with the disease, etc.

The pharmaceutical composition of the present invention can comprise a therapeutically or prophylactically effective amount of the anti-GPRC5D antibody or the antigen-binding fragment thereof, and/or a molecule comprising at least one of them as an active ingredient, and further contain a pharmaceutically acceptable diluent, vehicle, solubilizer, emulsifier, preservative, and/or additive.

The term "therapeutically or prophylactically effective amount" means an amount that exerts therapeutic or prophylactic effects on a particular disease by means of a particular dosage form and administration route, and is used interchangeably with a "pharmacologically effective amount".

The pharmaceutical composition of the present invention may comprise materials for changing, maintaining, or retaining pH, osmotic pressure, viscosity, transparency, color, tonicity, sterility, or the stability, solubility, sustained release, absorbability, permeability, dosage form, strength, properties, shape, etc., of the composition or the antibody comprised therein (hereinafter, referred to as "pharmaceutical materials"). The pharmaceutical materials are not particularly limited as long as the materials are pharmacologically acceptable. For example, no or low toxicity is a property preferably possessed by these pharmaceutical materials.

Examples of the pharmaceutical materials can include, but are not limited to, the following: amino acids such as glycine, alanine, glutamine, asparagine, histidine, arginine, and lysine; antimicrobial agents; antioxidants such as ascorbic acid, sodium sulfate, and sodium bisulfite; buffers such as phosphate, citrate, or borate buffers, sodium bicarbonate, and Tris-HCl solutions; fillers such as mannitol and glycine; chelating agents such as ethylenediaminetetraacetic acid (EDTA); complexing agents such as caffeine, polyvinylpyrrolidine, β-cyclodextrin, and hydroxypropyl-β-cyclodextrin; bulking agents such as glucose, mannose, and dextrin; other hydrocarbons such as monosaccharides, disaccharides, glucose, mannose, and dextrin; coloring agents; corrigents; diluents; emulsifiers; hydrophilic polymers such as polyvinylpyrrolidine; low-molecular-weight polypeptides; salt-forming counterions; antiseptics such as benzalkonium chloride, benzoic acid, salicylic acid, thimerosal, phenethyl alcohol, methylparaben, propylparaben, chlorhexidine, sorbic acid, and hydrogen peroxide; solvents such as glycerin, propylene glycol, and polyethylene glycol; sugar alcohols such as mannitol and sorbitol; suspending agents; surfactants such as PEG, sorbitan ester, polysorbates such as polysorbate 20 and polysorbate 80, triton, tromethamine, lecithin, and cholesterol; stability enhancers such as sucrose and sorbitol; elasticity enhancers such as sodium chloride, potassium chloride, mannitol, and sorbitol; transport agents; diluents; excipients; and/or pharmaceutical additives.

The amount of these pharmaceutical materials added is 0.001 to 1000 times, preferably 0.01 to 100 times, more preferably 0.1 to 10 times the weight of the anti-GPRC5D antibody or the antigen-binding fragment thereof, and/or the molecule comprising at least one of them.

An immunoliposome comprising the anti-GPRC5D antibody or the antigen-binding fragment thereof, and/or the molecule comprising at least one of them, encapsulated in a liposome, or a pharmaceutical composition comprising a modified antibody form including the antibody conjugated with a liposome (U.S. Patent No. 6214388, etc.) is also included in the pharmaceutical composition of the present invention.

The excipients or vehicles are not particularly limited as long as they are liquid or solid materials, and are substances usually used in injectable water, saline, artificial cerebrospinal fluids, and other preparations for oral or parenteral administration. Examples of saline can include neutral saline and serum albumin-containing saline.

Examples of buffers can include a Tris buffer adjusted to bring the final pH of the pharmaceutical composition to 7.0 to 8.5, an acetate buffer adjusted to bring the final pH thereof to 4.0 to 5.5, a citrate buffer adjusted to bring the final pH thereof to 5.0 to 8.0, and a histidine buffer adjusted to bring the final pH thereof to 5.0 to 8.0.

The pharmaceutical composition of the present invention is a solid, a liquid, a suspension, or the like. Another example of the pharmaceutical composition of the present invention can include freeze-dried preparations. The freeze-dried preparations can be formed using an excipient such as sucrose.

The administration route of the pharmaceutical composition of the present invention may be any of enteral administration, local administration, and parenteral administration. Examples thereof can include intravenous administration, intraarterial administration, intramuscular administration, intradermal administration, hypodermic administration, intraperitoneal administration, transdermal administration, intraosseous administration, and intraarticular administration.

The composition of such a pharmaceutical composition can be determined according to the administration method, the binding affinity of the antibody for the GPRC5D protein, etc.

The dose of the pharmaceutical composition of the present invention is not limited as long as the dose is a pharmacologically effective amount. The dose can be appropriately determined according to the species of an individual, the type of disease, symptoms, sex, age, pre-existing conditions, the binding affinity of the antibody for the GPRC5D protein or its biological activity, and other factors. Usually, a dose of 0.01 to 1000 mg/kg, preferably 0.1 to 100 mg/kg, can be administered once every day for a period of 180 days or twice or three or more times a day.

Examples of the form of the pharmaceutical composition can include injections (including freeze-dried preparations and drops), suppositories, transnasal absorption preparations, transdermal absorption preparations, sublingual formulations, capsules, tablets, ointments, granules, aerosols, pills, powders, suspensions, emulsions, eye drops, and biological implant formulations.

The pharmaceutical composition of the present invention can be administered concurrently with or separately from an additional drug. For example, the pharmaceutical composition of the present invention may be administered after administration of the additional drug, or the additional drug may be administered after administration of the pharmaceutical composition. Alternatively, the pharmaceutical composition and the additional drug may be administered concurrently. Examples of the additional drug can include various anticancer agents such as chemotherapeutics and radiation therapeutics. These cases are collectively referred to as "combined use with an additional drug" of the antibody of the present invention. A pharmaceutical composition comprising the active ingredient of the pharmaceutical composition of the present invention as well as an additional drug is also included in the present invention.

The present invention provides a method for treating or preventing GPRC5D-related diseases such as cancer, use of the antibody of the present invention for preparing a pharmaceutical composition for treatment or prevention of the diseases, and use of the antibody of the present invention for treating or preventing the diseases. The present invention also encompasses a kit for treatment or prevention comprising the antibody of the present invention.

[Examples]

Hereinafter, the present invention will be further specifically described with reference to the Examples. However, the present invention is not intended to be limited to them.

Procedures related to gene manipulation in the Examples below were performed according to the methods described in "Molecular Cloning" (Sambrook, J., Fritsch, E.F. and Maniatis, T., eds., Cold Spring Harbor Laboratory Press, 1989) or the methods described in other experimental manuals used by those skilled in the art, or using commercially available reagents or kits according to the instruction manuals, unless otherwise specified.

Example 1. Preparation of rat anti-human GPRC5D antibody

1)-1 Immunization using human GPRC5D expression vector

1)-1-1 Construction of human GPRC5D expression vector (pcDNA3.1-DEST-hGPRC5D)

pcDNA3.1-DEST engineered as a destination vector was prepared from pcDNA3.1(+) using Gateway Vector Convension System (Thermo Fisher Scientific Inc.). A cDNA encoding the human GPRC5D protein (NP_061124.1) was cloned into the pcDNA3.1-DEST vector using Gateway LR Clonase Enzyme mix (Life Technologies Corp.) to construct a human GPRC5D expression vector pcDNA3.1-DEST-hGPRC5D. For the large-scale preparation of the human GPRC5D expression vector, Endofree Plasmid Giga Kit (Qiagen N.V.) was used.

1)-1-2 Rat immunization

For immunization, WKY/Izm female rats (Japan SLC, Inc.) were used. First, both lower thighs of each rat were pretreated with hyaluronidase (Sigma-Aldrich Corp.). Then, the pcDNA3.1-DEST-hGPRC5D was intramuscularly injected to these sites. Subsequently, the *in vivo* electroporation of these sites was carried out using ECM830 (BTX) and a needle electrode. The same *in vivo* electroporation as above was repeated once per approximately two weeks. Then, the lymph node or the spleen was collected from the rat and used in hybridoma preparation.

1)-2 Hybridoma preparation

The lymph node cells or the spleen cells were electrically fused with mouse myeloma SP2/0-ag14 cells (ATCC, No. CRL-1 581) using LF301 Cell Fusion Unit (BEX Co., Ltd.). The fused cells were diluted with ClonaCell-HY Selection Medium D (StemCell Technologies Inc.) and cultured. Hybridoma colonies that appeared were recovered to thereby prepare monoclonal hybridomas. Each hybridoma colony thus recovered was cultured using ClonaCell-HY Selection Medium E (StemCell Technologies Inc.), and the obtained hybridoma culture supernatant was used to screen for an anti-human GPRC5D antibody-producing hybridoma.

1)-3 Antibody screening by Cell-ELISA

1)-3-1 Primary screening by Cell-ELISA

Cell line HEK293α cells (HEK293 cells stably transfected with integrin αv and integrin β3 expression vectors) were adjusted to $5 \times 10^5$ cells/mL in a DMEM medium containing 10% FBS. pcDNA3.1-DEST-hGPRC5D or a control pcDNA3.1-DEST was transfected thereto according to transfection procedures using Lipofectamine 2000 (Thermo Fisher Scientific Inc.). The resulting cells were dispensed in an amount of 100 μL/well to a 96-well plate (Corning Inc.) and cultured overnight at 37°C under 5% $CO_2$ conditions in a DMEM medium containing 10% FBS. The obtained transfected cells were used in the attached state in Cell-ELISA.

1)-3-2 Cell-ELISA

After removal of the culture supernatant from the expression vector-transfected HEK293α cells prepared in Example 1)-1-1, each hybridoma culture supernatant was added to the pcDNA3.1-DEST-hGPRC5D, or pcDNA3.1-DEST-transfected HEK293α cells, and the plate was left standing at 4°C for 1 hour. The cells in the wells were washed once with PBS containing 5% FBS. Then, Anti-Rat IgG, HRP-Linked Whole Ab Goat (GE Healthcare Bio-Sciences Corp.) diluted 500-fold with PBS containing 5% FBS was added thereto, and the plate was left standing at 4°C for 1 hour. The cells in the wells were washed twice with PBS containing 5% FBS. Then, an OPD chromogenic solution (OPD solution (o-phenylenediamine dihydrochloride (Wako Pure Chemicals Industries, Ltd.) and $H_2O_2$ dissolved at concentrations of 0.4 mg/mL and 0.6% (v/v), respectively, in 0.05 M trisodium citrate and 0.1 M disodium hydrogen phosphate dodecahydrate, pH 4.5)) was added thereto at a concentration of 100 μL/well. Color reaction was performed with occasional stirring and stopped by the addition of 1 M HCL at a concentration of 100 μL/well. Then, the absorbance was measured at 490 nm using a plate reader (ENVISION; PerkinElmer, Inc.). In order to select a hybridoma producing an antibody specifically that binds to human GPRC5D expressed on cell membrane surface, hybridomas that yielded a culture supernatant exhibiting higher absorbance for the pcDNA3.1-DEST-hGPRC5D expression vector-transfected HEK293α cells compared with the pcDNA3.1-DEST-transfected HEK293α cells of control were selected as anti-human GPRC5D antibody production-positive hybridomas.

1)-4 Screening antibody by flow cytometry

1)-4-1 Preparation of antigen gene-expressing cell for flow cytometry analysis

HEK293T cells (Thermo Fisher Scientific Inc.) were inoculated at a concentration of $4 \times 10^5$ cells/mL to a 225-$cm^2$ flask and cultured overnight at 37°C under 5% $CO_2$ conditions in a DMEM medium containing 10% FBS. On the next day, pcDNA3.1-DEST-hGPRC5D or a control pcDNA3.1-DEST was transfected to the HEK293T cells using Lipofectamine LTX (Thermo Fisher Scientific Inc.), and the cells were further cultured overnight at 37°C under 5% $CO_2$ conditions. On the next day, the expression vector-transfected HEK293T cells were treated with TrypLE Express (Thermo Fisher Scientific Inc.), washed with DMEM containing 10% FBS, and then adjusted to a concentration of $5 \times 10^6$ cells/mL in PBS containing 5% FBS. The obtained cell suspension was used in flow cytometry analysis.

1)-4-2 Flow cytometry analysis

The human GPRC5D binding specificity of the antibody produced by each hybridoma determined to be positive by Cell-ELISA in Example 1)-3 was further confirmed by flow cytometry. Each HEK293T cell suspension prepared in Example 1)-4-1 was inoculated at a concentration of 100 μL/well to a 96-well U-bottomed microplate and centrifuged to remove a supernatant. The pcDNA3.1-DEST-hGPRC5D transfected HEK293T cells or the pcDNA3.1-DEST-transfected HEK293T cells were suspended by the addition of the hybridoma culture supernatant and left standing at 4°C for 1 hour. The cells were washed once with PBS containing 5% FBS, then suspended by the addition of PE Goat Anti-Rat Ab (BD) diluted 100-fold with PBS containing 5% FBS, and left standing at 4°C for 30 minutes. The cells were washed twice with PBS containing 5% FBS and then resuspended in PBS containing 5% FBS, followed by detection using a flow cytometer (FACSCanto(TM) II:BD). The data was analyzed using Flowjo (Tree Star, Inc.). The PE fluorescence intensity of the cell fraction was plotted to a histogram. Hybridomas that yielded a sample exhibiting a shift to stronger fluorescence intensity in the histogram of the pcDNA3.1-DEST-hGPRC5D transfected HEK293T cells compared with the fluorescence intensity histogram of the control pcDNA3.1-DEST-transfected HEK293T cells were selected as hybridomas producing the anti-human GPRC5D antibody.

1)-5 Antibody screening by ADCC assay

1)-5-1 Preparation of cell stably expressing β-galactosidase

HEK293FT cells (Thermo Fisher Scientific Inc.) were transfected with pLenti6/V5-GW/LacZ and ViraPower(TM) Packaging Mix (Thermo Fisher Scientific Inc.) according to the attached protocols to prepare a recombinant lentivirus expressing the β-galactosidase gene. HEK293T cells were infected by the obtained recombinant lentivirus according to the protocol of ViraPower Lentiviral Expression Systems (Thermo Fisher Scientific Inc.). Virus-infected cells were selected

using 10 μg/mL Blasticidin (Thermo Fisher Scientific Inc.) to obtain a line stably expressing β-galactosidase. These HEK293T cells stably expressing β-galactosidase (hereinafter, referred to as "293T-lacZ cells") were used as target cells in the assay of ADCC activity.

1)-5-2 Preparation of target cell

The 293T-lacZ cells obtained in Example 1)-5-1 were inoculated at a concentration of $5 \times 10^5$ cells/ml to a 75-$cm^2$ flask and cultured overnight at 37°C under 5% $CO_2$ conditions in a DMEM medium containing 10% FBS. On the next day, pcDNA3.1-DEST-hGPRC5D or a control pcDNA3.1-DEST was transfected to the 293T-lacZ cells using Lipofectamine LTX (Thermo Fisher Scientific Inc.), and the cells were further cultured overnight at 37°C under 5% $CO_2$ conditions. On the next day, the expression vector-transfected 293T-lacZ cells were treated with TrypLE Express (Thermo Fisher Scientific Inc.) and washed twice with a phenol red-free RPMI1640 medium containing 5% FBS (Thermo Fisher Scientific Inc.) (hereinafter, referred to as a "medium for ADCC"). The number of live cells was counted by the trypan blue dye exclusion test. The cells were resuspended to $1 \times 10^5$ cells/mL in a medium for ADCC and used as target cells.

1)-5-3 Preparation of effector cell

Human peripheral blood mononuclear cells (PBMC) collected from the blood of a volunteer according to the standard method using Ficoll-Paque PLUS (GE Healthcare Bio-Sciences Corp.) were suspended in a phenol red-free RPMI1640 medium containing 10% FBS (Thermo Fisher Scientific Inc.), centrifuged, and then resuspended. The number of live cells was counted by the trypan blue dye exclusion test. After centrifugation, the medium was removed, and the cells were suspended and adjusted to $2 \times 10^6$ cells/mL in a medium for ADCC and used as effector cells.

1)-5-4 Preparation of hybridoma culture supernatant

The concentration of each rat anti-GPRC5D antibody-producing hybridoma culture supernatant obtained in Example 1)-4 was measured using FastELISA IgG ELISA Quantification Kit (RD-Biotech) and adjusted to 10 μg/mL (final concentration) with ClonaCell-HY Selection Medium E (StemCell Technologies Inc.).

1)-5-5 ADCC assay

The 293T-lacZ cells obtained in Example 1)-5-2 were added at a concentration of 50 μl/well to a 96-well U-bottomed microplate. Each hybridoma culture supernatant prepared in Example 1)-5-4, or a mouse anti-GPRC5D IgG2b antibody (R&D Systems, Inc.) for a positive control or a mouse control antibody (mIgG2b) (R&D Systems, Inc.) for a negative control adjusted to 10 μg/mL (final concentration) was added thereto at a concentration of 50 μl/well, and the plate was left standing at 4°C for 1 hour. The effector cells prepared in Example 1)-5-3 were further added thereto at a concentration of 50 μl/well. The plate was centrifuged at 1200 rpm at room temperature for 5 minutes, followed by culturing at 37°C for 18 hours under 5% $CO_2$ conditions. 50 μl of the supernatant in each well was recovered into a white plate (Corning Inc.). A solution of β-Glo assay system (Promega Corp.) was added thereto in an amount of 50 μl/well. The luminescence intensity was measured using a plate reader (ENVISION; PerkinElmer, Inc.). The percentage of cells lysed by ADCC activity was calculated according to the following expression:

$$\text{Percentage of cells lysed (\%)} = (A - B) / (C - B) \times 100$$

A: Count of sample wells
B: Average of spontaneous release (wells supplemented with neither the antibody nor the effector cells) counts (n = 3). 50 μL of a medium for ADCC was added during addition of the hybridoma culture supernatant and during addition of the effector cells. Otherwise, the same operation was performed for the sample wells.
C: Average of maximum release (wells containing target cells lysed in a surfactant) counts (n = 3). 50 μl of a medium for ADCC was added during addition of the hybridoma culture supernatant and during addition of the effector cells. For the assay, 150 μL of the β-Glo assay system solution was added to each well containing the cells and mixed therewith. A 100 μL aliquot thereof was added to a white plate to carry out the assay.

The ADCC activity against the pcDNA3.1-DEST-hGPRC5D-transfected 293T-lacZ cells or the pcDNA3.1-DEST-transfected 293T-lacZ cells was calculated according to the method described above to select hybridoma clones that exhibited ADCC activity specific for the pcDNA3.1-DEST-hGPRC5D-transfected 293T-lacZ cells and produced an antibody exhibiting ADCC activity higher than that of the positive control antibody.

1)-6 Isotyping of antibody

2A4, 2B1 and 7B4 which were suggestive of strongly binding to human GPRC5D and having high ADCC activity in Example 1)-5 were selected from among the rat anti-GPRC5D antibody-producing hybridomas obtained in Example 1)-4, and identified by antibody isotyping. The isotypes were determined using Rat monoclonal isotyping test kit (AbD Serotec). As a result, all the isotypes of the rat anti-GPRC5D monoclonal antibodies 2A4, 2B1, and 7B4 were confirmed to be IgG2b and κ chains.

1)-7 Preparation of monoclonal antibody

The rat anti-GPRC5D monoclonal antibody was purified from the hybridoma culture supernatant. First, the 2A4, 2B1 and 7B4-producing hybridoma was allowed to grow into a sufficient amount in ClonaCell-HY Selection Medium E (StemCell Technologies Inc.). Then, the medium was replaced with a Hybridoma SFM (Thermo Fisher Scientific Inc.) containing 5 μg/mL gentamicin (Thermo Fisher Scientific Inc.) supplemented with 20% Ultra Low IgG FBS (Thermo Fisher Scientific Inc.), followed by culturing for 5 days. This culture supernatant was recovered and sterilized through a 0.45 μm filter.

Each antibody was purified from the hybridoma supernatant in one step by protein G affinity chromatography (at 4 to 6°C). A buffer replacement step after the protein G affinity chromatography purification was carried out at 4 to 6°C. First, the hybridoma culture supernatant was applied to a column packed with protein G (GE Healthcare Bio-Sciences Corp.) equilibrated with PBS. After entry of the whole culture supernatant solution into the column, the column was washed with PBS in an amount of twice or more the column volume. Next, antibody-containing fractions were collected by elution with an aqueous solution of 0.1 M glycine/hydrochloric acid (pH 2.7). The collected fractions were adjusted to pH 7.0 to 7.5 by the addition of 1 M Tris-HCl (pH 9.0). Then, the buffer was replaced with HBSor (25 mM histidine/5% sorbitol, pH 6.0) using Centrifugal UF Filter Device VIVASPIN20 (molecular weight cutoff: UF30K, Sartorius Japan K.K., at 4 to 6°C) while the antibody was concentrated and adjusted to an antibody concentration of 1 mg/mL or higher. Finally, the concentrate was filtered through Minisart-Plus filter (Sartorius Japan K.K.) and used as a purified sample.

(Example 2) *In vitro* evaluation of rat anti-GPRC5D antibodies (2A4, 2B1, and 7B4)

2)-1 Study on binding activity of obtained rat anti-GPRC5D antibodies (2A4, 2B1, and 7B4) to human GPRC5D by flow cytometry

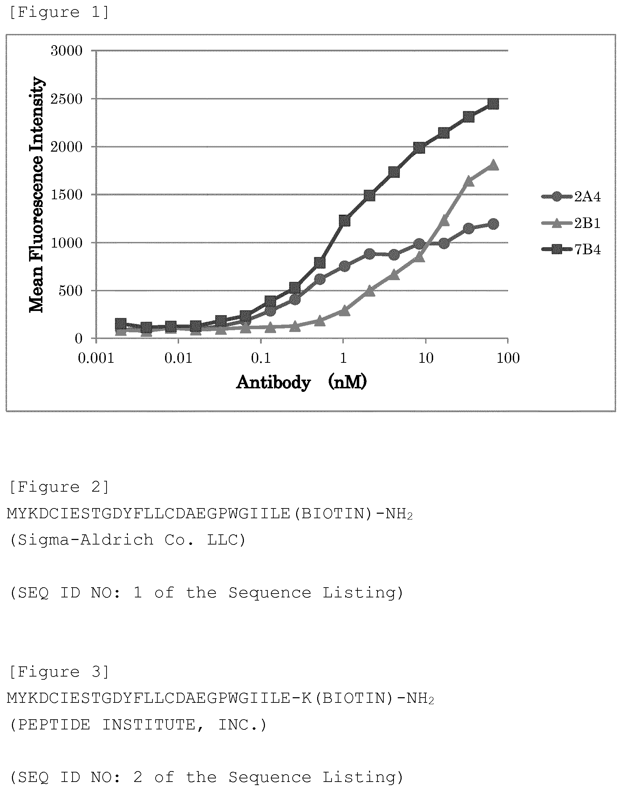

Human multiple myeloma cell line KHM-1B cells (JCRB Cell Bank) expressing GPRC5D were adjusted to a concentration of $5 \times 10^6$ cells/mL with PBS containing 5% FBS, inoculated in an amount of 100 μL/well to a 96-well U-bottomed microplate, and centrifuged to remove a supernatant. Each rat anti-GPRC5D antibody (2A4, 2B1, and 7B4) adjusted to 0.32 ng/mL to 10 μg/mL in Example 1)-7 was added thereto in an amount of 100 μL/well, and the plate was left standing at 4°C for 1 hour. The cells were washed twice with PBS containing 5% FBS. Then, PE Goat Anti-Rat Ab (Becton, Dickinson and Company) diluted 100-fold with PBS containing 5% FBS was added thereto at a concentration of 100 μL/well, and the plate was left standing at 4°C for 1 hour. The cells were washed twice with PBS containing 5% FBS and then resuspended in PBS containing 5% FBS, followed by detection using a flow cytometer (FACSCanto(TM) II; Becton, Dickinson and Company). The data was analyzed using Flowjo (Tree Star, Inc.). The PE fluorescence intensity of the cell fraction was plotted to a histogram, and the mean fluorescence intensity (MFI) was calculated. As shown in Figure 1, 2A4, 2B1, and 7B4 were found to bind to human GPRC5D (2A4 MFI (max): 1153, 2A4 Kd: 0.5 nM, 2B1 MFI (max): 2386, 2B1 Kd: 14.8 nM, 7B4 MFI (max): 2492, 7B4 Kd: 1.3 nM). Flow cytometry was also carried out using human multiple myeloma cell line KMS-34 cells (JCRB Cell Bank) expressing GPRC5D and produced similar results (2A4 MFI (max): 2064, 2A4 Kd: 1.4 nM, 2B1 MFI (max): 3157, 2B1 Kd: 12.7 nM, 7B4 MFI (max): 4471, 7B4 Kd: 2.1 nM).

2)-2 Identification of epitope for obtained rat anti-GPRC5D antibodies (2A4, 2B1, and 7B4)

2)-2-1 Identification of epitope by ELISA

Epitopes bound by the obtained rat anti-GPRC5D antibodies (2A4, 2B1, and 7B4) were identified using a human GPRC5D amino-terminal peptide MYKDCIESTGDYFLLCDAEGPWGIILE(Biotin)-$NH_2$ (Sigma-Aldrich Corp.) (SEQ ID NO: 1 of the Sequence Listing; Figure 2) having a biotinylated carboxy-terminal peptide and lacking an intramolecularly formed disulfide bond, and a human GPRC5D amino-terminal peptide MYKDCIESTGDYFLLCDAEGPWGIILE-K(Biotin)-$NH_2$ (Peptide Institute, Inc.) (SEQ ID NO: 2 of the Sequence Listing; Figure 3) having a lysine-containing biotinylated carboxy terminus and having an intramolecularly formed disulfide bond. Each peptide diluted with PBS was added to Nunc Immobilizer (Thermo Fisher Scientific Inc.), and the plate was left standing at room temperature for 1 hour. After washing three times with PBST, FBS containing 1% BSA was added thereto, and the plate was left standing at room temperature for 1 hour. After washing three times with PBST, each rat anti-GPRC5D antibody (2A4, 2B1, and 7B4) prepared in Example 1)-7 and diluted to 0.1 ng/mL to 1 μg/mL with PBS was added thereto, and the plate was left standing at room temperature for 1 hour. After washing three times with PBST, Anti-Rat IgG, HRP-Linked Whole Ab Goat (GE Healthcare Bio-Sciences Corp.) diluted 500-fold with PBS was added thereto, and the plate was left standing at room temperature for 1 hour. After washing three times with PBST, SuperSignal(TM) ELISA Pico Chemiluminescent Substrate (Thermo Fisher Scientific Inc.) was added thereto, and the luminescence was measured using a plate reader (ENVISION; PerkinElmer, Inc.). As a result, the 2B1 antibody bound to the amino-terminal peptide sequence of human GPRC5D whereas the 2A4 and 7B4 antibodies did not bind thereto, regardless of the presence or absence of a disulfide bond. These results suggested that the epitope for the 2B1 antibody was present in the amino-terminal region of human GPRC5D while the epitopes for the 2A4 and 7B4 antibodies were present in regions other than the amino terminus.

2)-2-2 Identification of epitope by flow cytometry

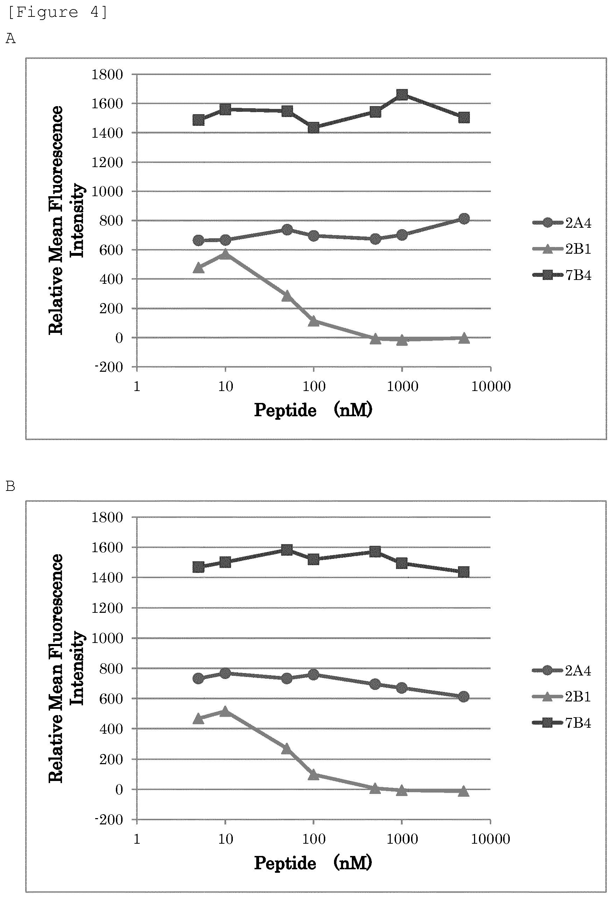

Human multiple myeloma cell line KMS-34 cells expressing GPRC5D were adjusted to a concentration of $2 \times 10^6$ cells/mL with PBS containing 5% FBS, inoculated in an amount of 100 μL/well to a 96-well U-bottomed microplate, and centrifuged to remove a supernatant. Each rat anti-GPRC5D antibody (2A4, 2B1, and 7B4) adjusted to 7.5 μg/mL in Example 1)-7, or Rat IgG2b isotype control antibody (Medical & Biological Laboratories Co., Ltd. (MBL)) was added thereto in an amount of 100 μL/well. The two types of peptides used in Example 2)-2-1 were each adjusted to 17 ng/mL to 34 μg/mL with PBS and added in an amount of 100 μL/well to the wells containing the antibody dilution, and the plate was left standing at 4°C for 1 hour. The cells were washed twice with PBS containing 5% FBS. Then, PE Goat Anti-Rat Ab (Becton, Dickinson and Company) diluted 100-fold with PBS containing 5% FBS was added thereto in an amount of 100 μL/well, and the plate was left standing at 4°C for 1 hour. The cells were washed twice with PBS containing 5% FBS and then resuspended in PBS containing 5% FBS, followed by detection using a flow cytometer (FACSCanto(TM) II; Becton, Dickinson and Company). The data was analyzed using Flowjo (Tree Star, Inc.). The PE fluorescence intensity of the cell fraction was plotted to a histogram, and the mean fluorescence intensity (MFI) was calculated. The MFI value of the control antibody was subtracted from the MFI value of the GPRC5D antibody to calculate a relative value of MFI (rMFI). As shown in Figure 4 (intramolecular disulfide bond was present (A) or absent (B)), the binding of 2B1 was found to be inhibited by the addition of the amino-terminal peptide of human GPRC5D, regardless of the presence or absence of a disulfide bond. By contrast, the binding of the 2A4 and 7B4 antibodies was found not to be inhibited by the addition of the amino-terminal peptide of human GPRC5D. These results suggested that the epitope for the 2B1 antibody was present in the amino-terminal region of human GPRC5D while the epitopes for the 2A4 and 7B4 antibodies were present in regions other than the amino terminus.

2)-3 ADCC activity evaluation of obtained rat anti-GPRC5D antibodies (2A4, 2B1, and 7B4)

2)-3-1 Preparation of target cell

Human multiple myeloma cell line KHM-1B cells were adjusted to a concentration of $2 \times 10^6$ cells/mL with an RPMI1640 medium containing 10% FBS (Thermo Fisher Scientific Inc.). 100 μL of Chromium-51 Radionuclide (PerkinElmer, Inc.) was added per mL of the cell suspension, and the cells were cultured at 37°C for 2 hours under 5% $CO_2$ conditions. The cells were washed three times with an RPMI1640 medium containing 10% FBS, then resuspended to $2 \times 10^5$ cells/mL in an RPMI1640 medium containing 10% FBS, and used as target cells.

2)-3-2 Preparation of effector cell

PBMC prepared in Example 1)-5-3 was differentiated into NK cells using BINKIT (Biotherapy Institute of Japan). The NK cells were adjusted to $1 \times 10^6$ cells/mL with an RPMI1640 medium containing 10% FBS and used as effector cells.

2)-3-3 ADCC assay

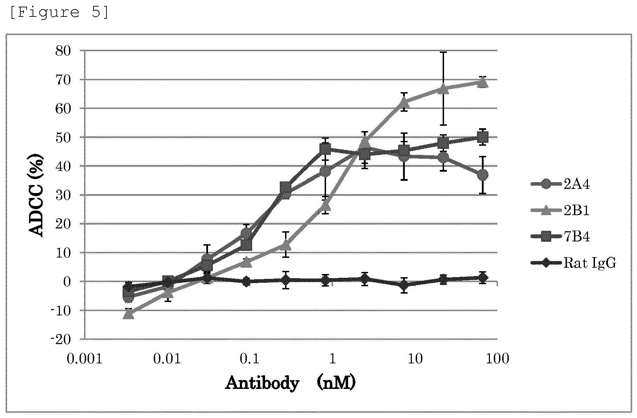

The KHM-1B cells prepared in Example 2)-3-1 were added at a concentration of 50 μL/well to a 96-well U-bottomed microplate. Each obtained rat anti-GPRC5D antibody (2A4, 2B1, and 7B4) and a rat control antibody (rIgG2b) adjusted to 0.5081 ng/mL to 10 μg/mL (final concentration) was added thereto in an amount of 50 μL/well, and the plate was left standing at 4°C for 30 minutes. The effector cells prepared in Example 2)-3-2 were further added thereto in an amount of 100 μL/well. After centrifugation at room temperature at 1200 rpm for 3 minutes, the cells were cultured at 37°C for 4 hours under 5% $CO_2$ conditions. A 50 μL aliquot of the supernatant was recovered into LumaPlate (PerkinElmer, Inc.) and dried overnight at 50°C, followed by measurement using a plate reader (TopCount; PerkinElmer, Inc.). The percentage of cells lysed by ADCC activity was calculated according to Example 1)-5-5. As shown in Figure 5, 2A4, 2B1, and 7B4 were found to have ADCC activity.

(Example 3) Sequencing of cDNAs encoding variable regions of rat anti-GPRC5D antibodies (2A4, 2B1, and 7B4)

3)-1 Sequencing of cDNAs encoding variable regions of 2A4

3)-1-1 Preparation of total RNA from 2A4-producing hybridoma

In order to amplify the cDNAs encoding the variable regions of 2A4, total RNA was prepared from the 2A4-producing hybridoma using TRIzol Reagent (Ambion/Thermo Fisher Scientific Inc.).

3)-1-2 Synthesis of cDNA (5'-RACE-Ready cDNA)

cDNAs (5'-RACE-Ready cDNAs) were synthesized using approximately 1 μg of the total RNA prepared in Example 3)-1-1, and SMARTer RACE cDNA Amplification Kit (Clontech Laboratories, Inc.).

3)-1-3 Amplification and sequencing of cDNA encoding heavy chain variable region of 2A4 by 5'-RACE PCR

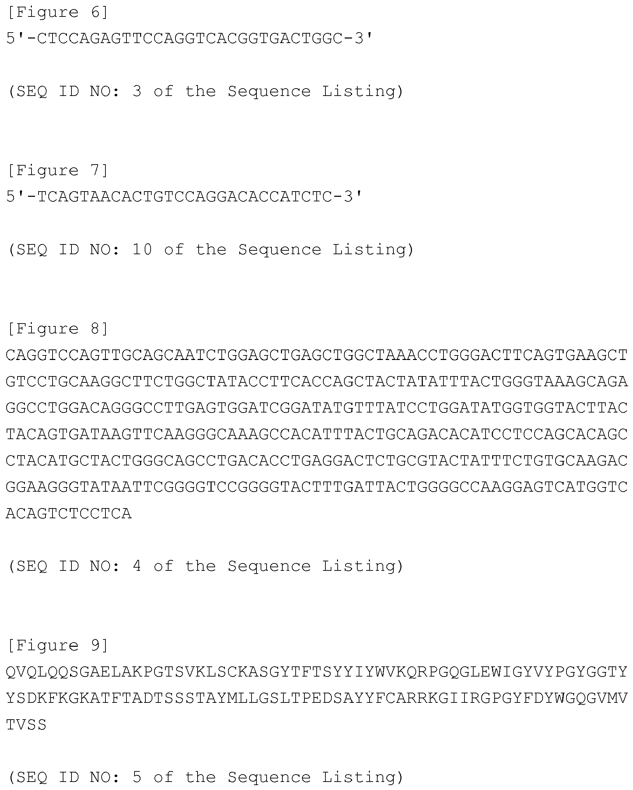

The primers used for the PCR amplification of the variable region-encoding cDNA of the heavy chain gene of 2A4 were oligonucleotides having the sequences of UPM (Universal Primer A Mix; attached to SMARTer RACE cDNA Amplification Kit) and 5'-CTCCAGAGTTCCAGGTCACGGTGACTGGC-3' (RG2AR3: SEQ ID NO: 3 (Figure 6)). The UPM used was attached to SMARTer RACE cDNA Amplification Kit (Clontech Laboratories, Inc.), while RG2AR3 was designed from the sequences of rat heavy chain constant regions registered in the database.
The cDNA encoding the heavy chain variable region of 2A4 was amplified by 5'-RACE PCR using this primer set and the cDNAs (5'-RACE-Ready cDNAs) synthesized in Example 3)-1-2 as templates. This PCR was carried out on the Touchdown PCR program according to the manual of SMARTer RACE cDNA Amplification Kit (Clontech Laboratories, Inc.).
The heavy chain variable region-encoding cDNA amplified by 5'-RACE PCR was purified using MinElute PCR Purification Kit (Qiagen N.V.) and then cloned using Zero Blunt TOPO PCR Cloning Kit (Invitrogen Corp.). The nucleotide sequence of the cloned heavy chain variable region-encoding cDNA was subjected to sequence analysis.
The determined nucleotide sequence of the cDNA encoding the heavy chain variable region of 2A4 is shown in SEQ ID NO: 4 (Figure 8), and the amino acid sequence thereof is shown in SEQ ID NO: 5 (Figure 9).

3)-1-4 Amplification and sequencing of cDNA encoding light chain variable region of 2A4 by 5'-RACE PCR

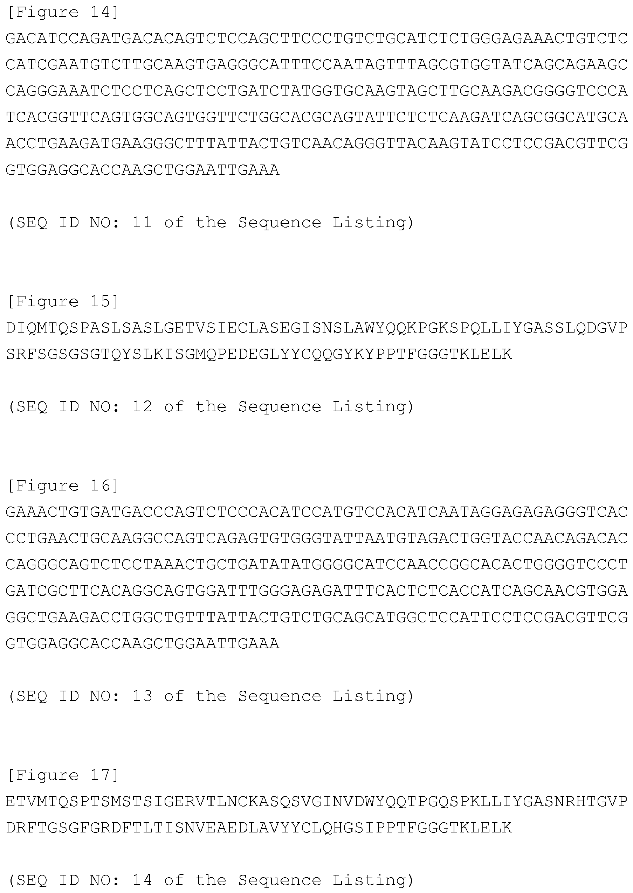

The primers used for the PCR amplification of the variable region-encoding cDNA of the light chain gene of 2A4 were oligonucleotides having the sequences of UPM (Universal Primer A Mix; attached to SMARTer RACE cDNA Amplification Kit) and 5'-TCAGTAACACTGTCCAGGACACCATCTC-3' (RKR5: SEQ ID NO: 10 (Figure 7)). The UPM used was attached to SMARTer RACE cDNA Amplification Kit (Clontech Laboratories, Inc.), while RKR5 was designed from the sequences of rat light chain constant regions registered in the database.
The cDNA encoding the light chain variable region of 2A4 was amplified by 5'-RACE PCR using this primer set and the cDNAs (5'-RACE-Ready cDNAs) synthesized in Example 3)-1-2 as templates. This PCR was carried out on the Touchdown PCR program according to the manual of SMARTer RACE cDNA Amplification Kit (Clontech Laboratories, Inc.).
The light chain variable region-encoding cDNA amplified by 5'-RACE PCR was purified using MinElute PCR Purification Kit (Qiagen N.V.) and then cloned using Zero Blunt TOPO PCR Cloning Kit (Invitrogen Corp.). The nucleotide sequence of the cloned light chain variable region-encoding cDNA was subjected to sequence analysis.
The determined nucleotide sequence of the cDNA encoding the light chain variable region of 2A4 is shown in SEQ ID NO: 11 (Figure 14), and the amino acid sequence thereof is shown in SEQ ID NO: 12 (Figure 15).

3)-2 Sequencing of cDNAs encoding variable regions of 2B1

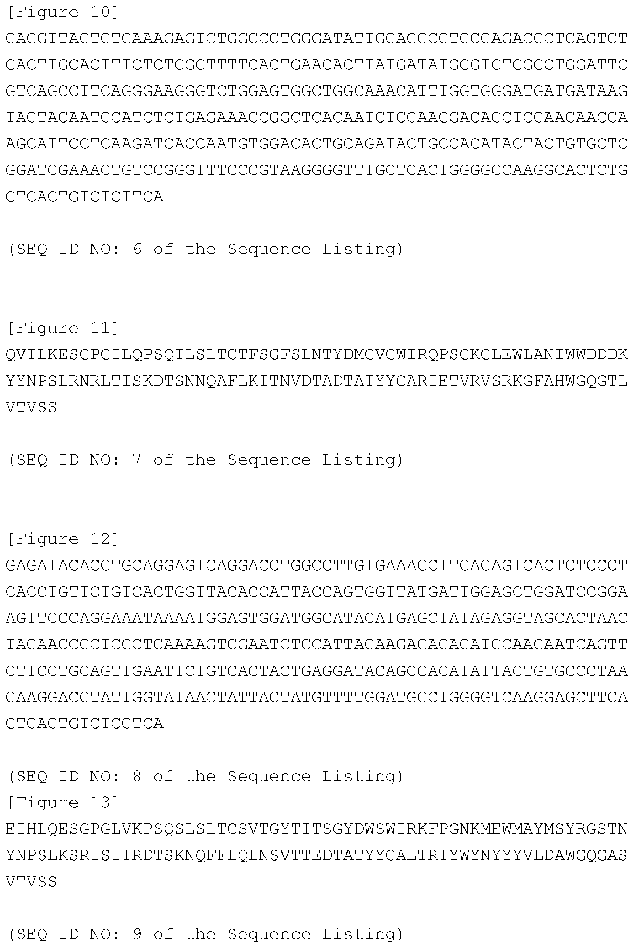

Sequences were determined in the same way as in Example 3)-1.
The determined nucleotide sequence of the cDNA encoding the heavy chain variable region of 2B1 is shown in SEQ ID NO: 6 (Figure 10), and the amino acid sequence thereof is shown in SEQ ID NO: 7 (Figure 11). The nucleotide sequence of the cDNA encoding the light chain variable region is shown in SEQ ID NO: 13 (Figure 16), and the amino acid sequence thereof is shown in SEQ ID NO: 14 (Figure 17).

3)-3 Sequencing of cDNAs encoding variable regions of 7B4

Sequences were determined in the same way as in Example 3)-1.

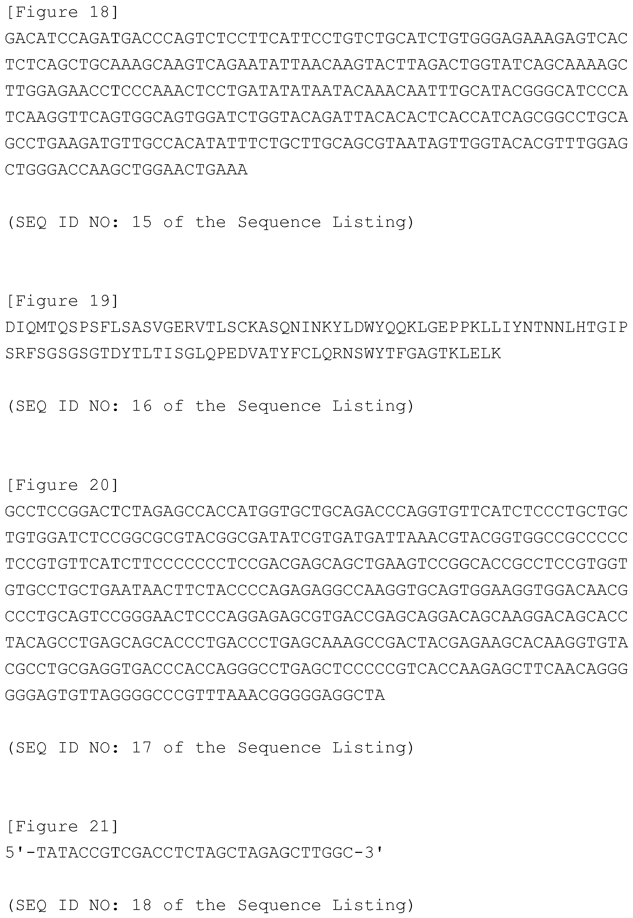

The determined nucleotide sequence of the cDNA encoding the heavy chain variable region of 7B4 is shown in SEQ ID NO: 8 (Figure 12), and the amino acid sequence thereof is shown in SEQ ID NO: 9 (Figure 13). The nucleotide sequence of the cDNA encoding the light chain variable region is shown in SEQ ID NO: 15 (Figure 18), and the amino acid sequence thereof is shown in SEQ ID NO: 16 (Figure 19).

(Example 4) Preparation of human chimeric anti-GPRC5D antibodies (c2A4, c2B1, and c7B4)

4)-1 Construction of chimeric and humanized light chain expression vector pCMA-LK

A plasmid pcDNA3.3-TOPO/LacZ (Invitrogen Corp.) was digested with restriction enzymes XbaI and PmeI. The obtained fragment of approximately 5.4 kb was fused with a DNA fragment comprising a DNA sequence (shown in SEQ ID NO: 17 (Figure 20) of the Sequence Listing) encoding a human light chain secretory signal and a human κ chain constant region using an In-Fusion Advantage PCR cloning kit (Clontech Laboratories, Inc.) to prepare pcDNA3.3/LK.

PCR was performed with pcDNA3.3/LK as a template using a primer set shown below. The obtained fragment of approximately 3.8 kb was phosphorylated and then self-ligated to construct a chimeric and humanized light chain expression vector pCMA-LK having the nucleotide sequence encoding a signal sequence, a cloning site, and the human κ chain constant region, downstream of the CMV promoter.

Primer set

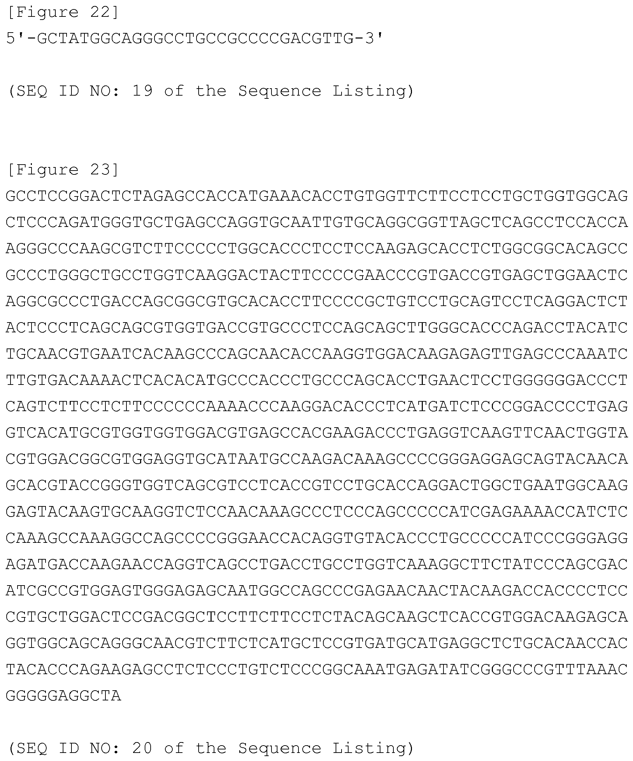

5'-TATACCGTCGACCTCTAGCTAGAGCTTGGC-3' (3.3-F1: SEQ ID NO: 18 of the Sequence Listing; Figure 21)
5'-GCTATGGCAGGGCCTGCCGCCCCGACGTTG-3' (3.3-R1: SEQ ID NO: 19 of the Sequence Listing; Figure 22)

4)-2 Construction of chimeric and humanized IgG1 type heavy chain expression vector pCMA-G1

pCMA-LK was digested with XbaI and PmeI. The obtained DNA fragment except for the DNA sequence encoding the light chain secretory signal and the human κ chain constant region was fused with a DNA fragment comprising a DNA sequence (shown in SEQ ID NO: 20 (Figure 23) of the Sequence Listing) encoding the amino acids of a human heavy chain signal sequence and a human IgG1 constant region using In-Fusion Advantage PCR cloning kit (Clontech Laboratories, Inc.) to construct a chimeric and humanized IgG1 type heavy chain expression vector pCMA-G1 having the nucleotide sequence encoding a signal sequence, a cloning site, and the human IgG1 heavy chain constant region, downstream of the CMV promoter.

4)-3 Construction of c2A4 light chain expression vector

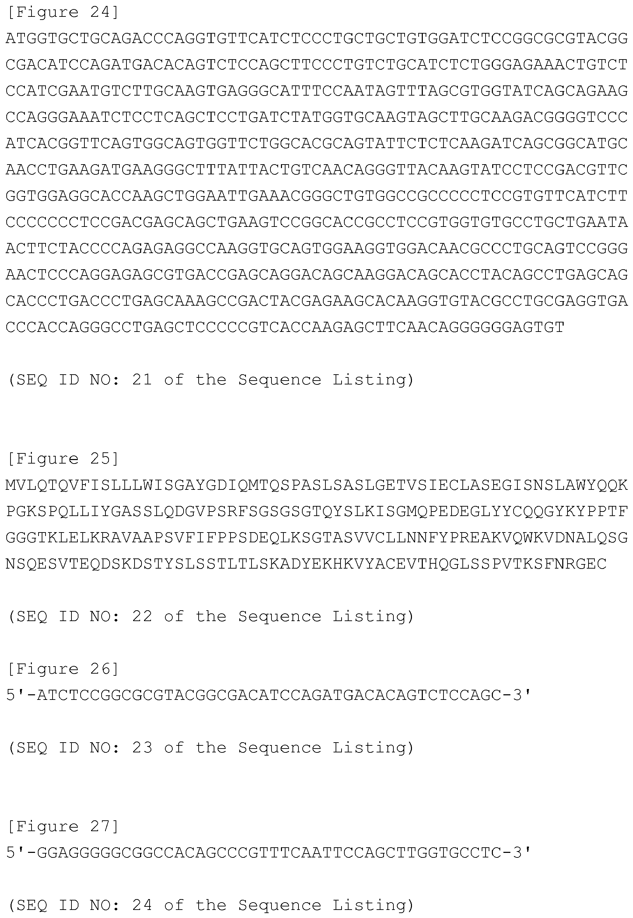

A DNA fragment comprising a light chain variable region-encoding cDNA was amplified by PCR using the 2A4 light chain variable region-encoding cDNA obtained in Example 3) as a template and a primer set shown below, and inserted to the restriction enzyme BsiWI-cleaved site of the chimeric and humanized antibody light chain expression vector pCMA-LK using In-Fusion Advantage PCR cloning kit (Clontech Laboratories, Inc.) to construct a c2A4 light chain expression vector. The obtained expression vector was designated as "pCMA-LK/c2A4". The nucleotide sequence of the c2A4 light chain and the amino acid sequence of this light chain are shown in SEQ ID NOs: 21 and 22 (Figures 24 and 25), respectively, of the Sequence Listing.

Primer set for c2A4 light chain

5'-ATCTCCGGCGCGT ACGGCGACA TCCAGA TGACACAGTCTCCAGC-3' (c2A4-LF: SEQ ID NO: 23 of the Sequence Listing; Figure 26)
5'-GGAGGGGGCGGCCACAGCCCGTTTCAA TTCCAGCTTGGTGCCTC-3' (c2A4-LR: SEQ ID NO: 24 of the Sequence Listing; Figure 27)

4)-4 Construction of c2A4 heavy chain expression vector

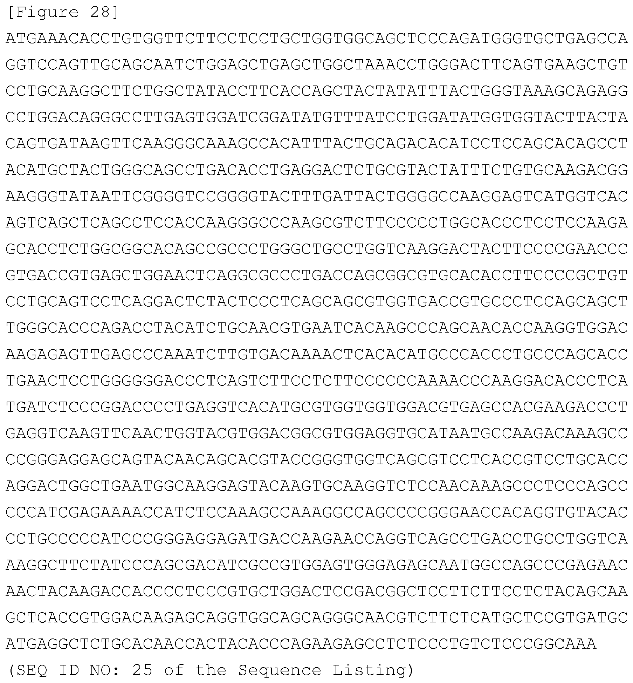

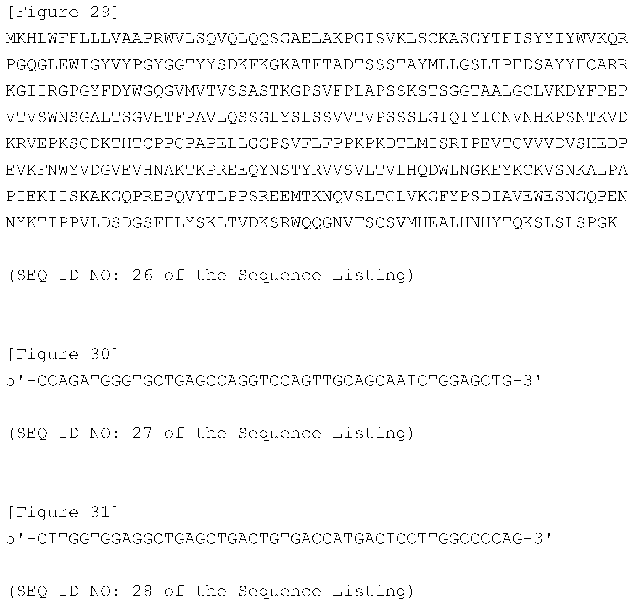

A DNA fragment comprising a heavy chain variable region-encoding cDNA was amplified by PCR using the 2A4 heavy chain variable region-encoding cDNA obtained in Example 3) as a template and a primer set shown below, and inserted to the restriction enzyme BlpI-cleaved site of the chimeric and humanized antibody heavy chain expression vector pCMA-G1 using In-Fusion Advantage PCR cloning kit (Clontech Laboratories, Inc.) to construct a c2A4 heavy chain expression vector. The obtained expression vector was designated as "pCMA-G1/c2A4". The nucleotide sequence of the c2A4 heavy chain and the amino acid sequence of this heavy chain are shown in SEQ ID NOs: 25 and 26 (Figures 28 and 29), respectively, of the Sequence Listing.
Primer set for c2A4 heavy chain

5'-CCAGATGGGTGCTGAGCCAGGTCCAGTTGCAGCAATCTGGAGCTG-3' (c2A4-HF: SEQ ID NO: 27 of the Sequence Listing; Figure 30)
5'-CTTGGTGGAGGCTGAGCTGACTGTGACCATGACTCCTTGGCCCCAG -3' (c2A4-HR: SEQ ID NO: 28 of the Sequence Listing; Figure 31

4)-5 Construction of c2B1 light chain expression vector

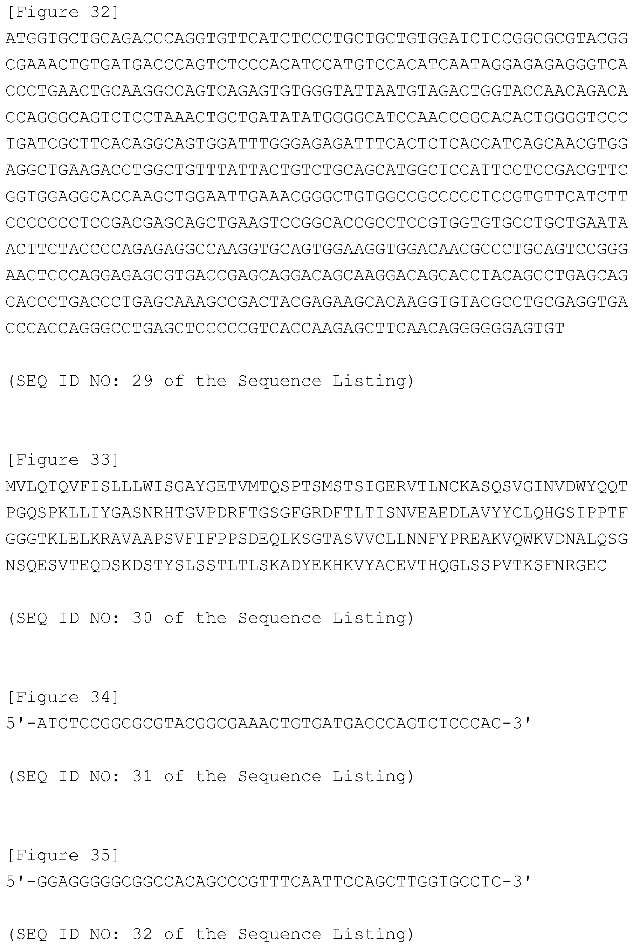

A DNA fragment comprising a light chain variable region-encoding cDNA was amplified by PCR using the 2B1 light chain variable region-encoding cDNA obtained in Example 3) as a template and a primer set shown below. A c2B1 light chain expression vector was constructed in the same way as in Example 4)-3. The obtained expression vector was designated as "pCMA-LK/c2B1 The nucleotide sequence of the c2B1 light chain and the amino acid sequence of this light chain are shown in SEQ ID NOs: 29 and 30 (Figures 32 and 33), respectively, of the Sequence Listing.
Primer set for c2B1 light chain

5'-ATCTCCGGCGCGT ACGGCGAAACTGTGA TGACCCAGTCTCCCAC-3' (c2B1-LF: SEQ ID NO: 31 of the Sequence Listing; Figure 34)
5'-GGAGGGGGCGGCCACAGCCCGTTTCAA TTCCAGCTTGGTGCCTC-3' (c2B1-LR: SEQ ID NO: 32 of the Sequence Listing; Figure 35)

4)-6 Construction of c2B1 heavy chain expression vector

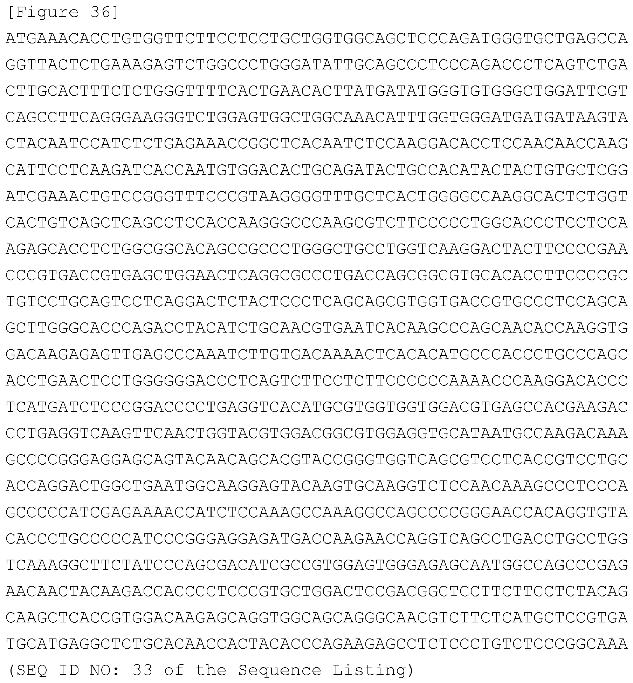

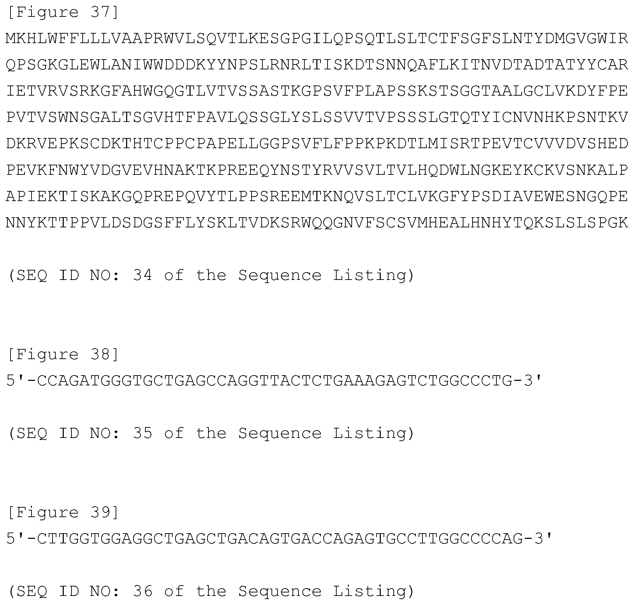

A DNA fragment comprising a heavy chain variable region-encoding cDNA was amplified by PCR using the 2B1 heavy chain variable region-encoding cDNA obtained in Example 3) as a template and a primer set shown below. A c2B1 heavy chain expression vector was constructed in the same way as in Example 4)-4. The obtained expression vector was designated as "pCMA-G1/c2B1". The nucleotide sequence encoding the c2B1 heavy chain and the amino acid sequence of this heavy chain are shown in SEQ ID NOs: 33 and 34 (Figures 36 and 37), respectively, of the Sequence Listing.
Primer set for c2B1 heavy chain

5'-CCAGATGGGTGCTGAGCCAGGTTACTCTGAAAGAGTCTGGCCCTG-3' (c2B1-HF: SEQ ID NO: 35 of the Sequence Listing; Figure 38)
5'-CTTGGTGGAGGCTGAGCTGACAGTGACCAGAGTGCCTTGGCCCCA G-3' (c2B1-HR: SEQ ID NO: 36 of the Sequence Listing; Figure 39)

4)-7 Construction of c7B4 light chain expression vector

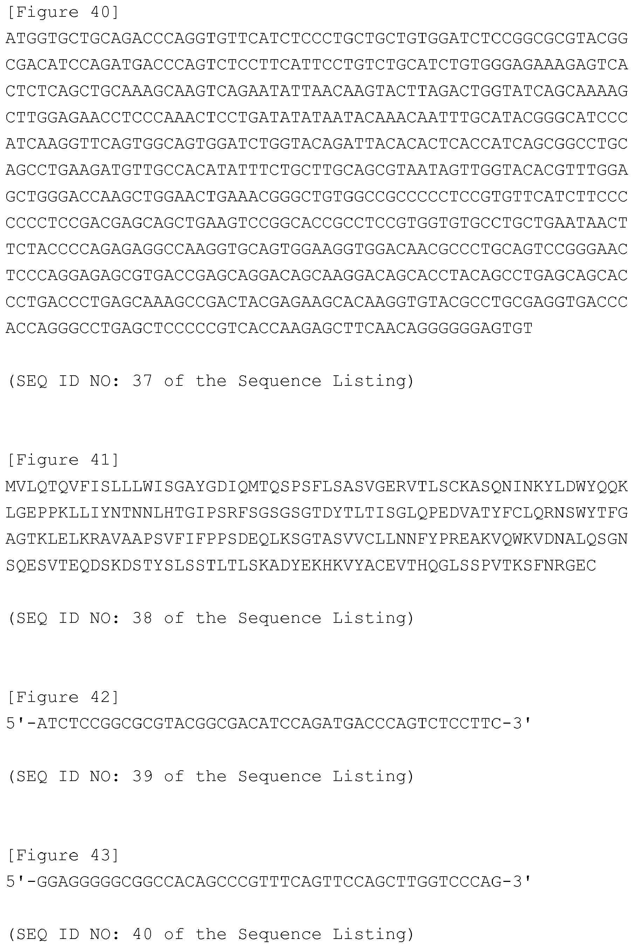

A DNA fragment comprising a light chain variable region-encoding cDNA was amplified by PCR using the 7B4 light chain variable region-encoding cDNA obtained in Example 3) as a template and a primer set shown below. A c7B4 light chain expression vector was constructed in the same way as in Example 4)-3. The obtained expression vector was designated as "pCMA-LK/c7B4". The nucleotide sequence of the c7B4 light chain and the amino acid sequence of this light chain are shown in SEQ ID NOs: 37 and 38 (Figures 40 and 41), respectively, of the Sequence Listing.
Primer set for c7B4 light chain

5'-ATCTCCGGCGCGTACGGCGACATCCAGATGACCCAGTCTCCTTC-3' (c7B4-LF: SEQ ID NO: 39 of the Sequence Listing; Figure 42)
5'-GGAGGGGGCGGCCACAGCCCGTTTCAGTTCCAGCTTGGTCCCAG-3 ' (c7B4-LR: SEQ ID NO: 40 of the Sequence Listing; Figure 43)

4)-8 Construction of c7B4 heavy chain expression vector

A DNA fragment comprising a heavy chain variable region-encoding cDNA was amplified by PCR using the 7B4 heavy chain variable region-encoding cDNA obtained in Example 3) as a template and a primer set shown below.

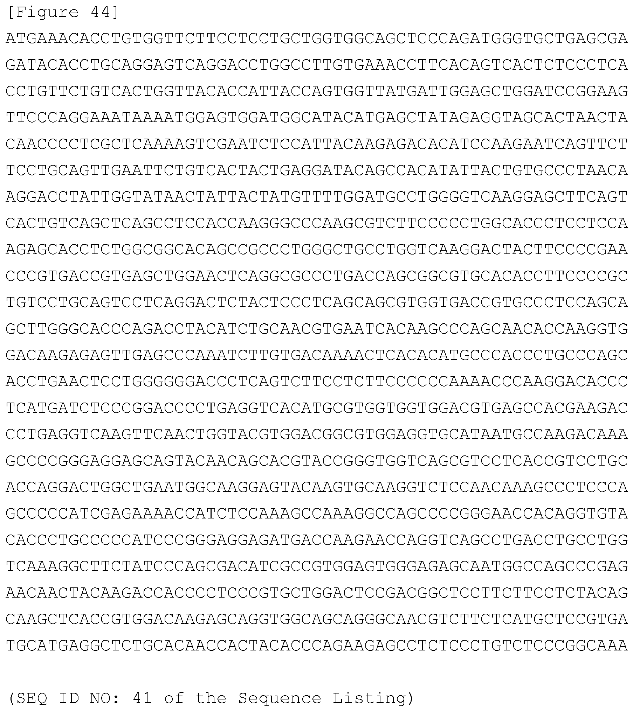

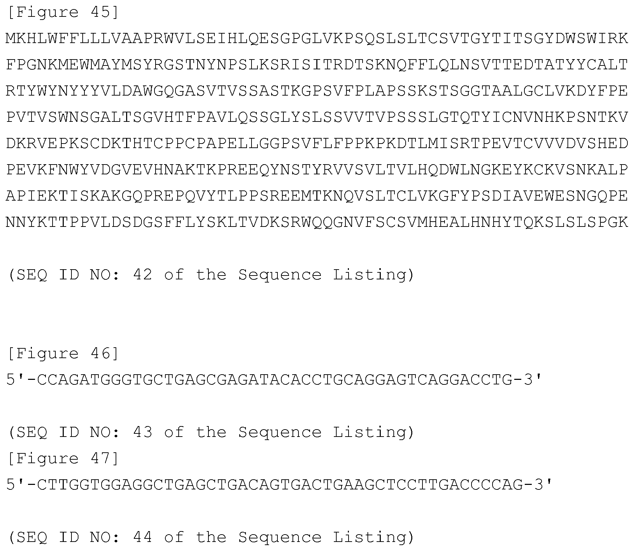

A c7B4 heavy chain expression vector was constructed in the same way as in Example 4)-4. The obtained expression vector was designated as "pCMA-G1/c7B4". The nucleotide sequence of the c7B4 heavy chain and the amino acid sequence of this heavy chain are shown in SEQ ID NOs: 41 and 42 (Figures 44 and 45), respectively, of the Sequence Listing.

Primer set for c7B4 heavy chain

5'-CCAGATGGGTGCTGAGCGAGATACACCTGCAGGAGTCAGGACCTG -3' (c7B4-HF: SEQ ID NO: 43 of the Sequence Listing; Figure 46)

5'-CTTGGTGGAGGCTGAGCTGACAGTGACTGAAGCTCCTTGACCCCA G-3' (c7B4-HR: SEQ ID NO: 44 of the Sequence Listing; Figure 47)

4)-9 Preparation of human chimeric anti-GPRC5D antibody

4)-9-1 Production of human chimeric anti-GPRC5D antibody

FreeStyle 293F cells (Invitrogen Corp.) were subcultured and cultured according to the manual. 1.2 $\times$ $10^9$ FreeStyle 293F cells (Invitrogen Corp.) in the logarithmic growth phase were inoculated to a 3-L Fernbach Erlenmeyer Flask (Corning Inc.), adjusted to 2.0 $\times$ $10^6$ cells/ml by dilution with FreeStyle 293 expression medium (Invitrogen Corp.), and then shake-cultured at 90 rpm at 37°C for 1 hour in an 8% $CO_2$ incubator. 1.8 mg of polyethyleneimine (Polysciences #24765) was dissolved in 20 ml of Opti-Pro SFM medium (Invitrogen Corp.). Next, each heavy chain expression vector (0.24 mg) and light chain expression vector (0.36 mg) prepared using NucleoBond Xtra (Takara Bio Inc.) were added to 20 ml of Opti-Pro SFM medium (Invitrogen Corp.). 20 ml of the expression vector/Opti-Pro SFM mixed solution was added to 20 ml of the polyethyleneimine/Opti-Pro SFM mixed solution, and the mixture was gently stirred, left standing for 5 minutes, and then added to the FreeStyle 293F cells. The cells were shake-cultured at 90 rpm at 37°C for 4 hours in an 8% $CO_2$ incubator. Then, 600 ml of EX-CELL VPRO medium (SAFC Biosciences Inc.), 18 ml of GlutaMAX I (Gibco/Thermo Fisher Scientific Inc.), and 30 ml of Yeastolate Ultrafiltrate (Gibco/Thermo Fisher Scientific Inc.) were added thereto. The cells were shake-cultured at 90 rpm at 37°C for 7 days in an 8% $CO_2$ incubator, and the obtained culture supernatant was filtered through Disposable Capsule Filter (Advantec #CCS-045-E1H).

The human chimeric 2A4 obtained by the combination of pCMA-G1/c2A4 and pCMA-LK/c2A4 was designated as "c2A4". The human chimeric 2B1 obtained by the combination of pCMA-G1/c2B1 and pCMA-LK/c2B1 was designated as "c2B1". The human chimeric 7B4 obtained by the combination of pCMA-G1/c7B4 and pCMA-LK/c7B4 was designated as "c7B4".

4)-9-2 Purification of human chimeric anti-GPRC5D antibody

Each antibody was purified from the culture supernatant obtained in Example 4)-9-1 in one step by rProtein A affinity chromatography (at 4 to 6°C). A buffer replacement step after the rProtein A affinity chromatography purification was carried out at 4 to 6°C. The culture supernatant was applied to a column packed with MabSelectSuRe (manufactured by GE Healthcare Bio-Sciences Corp.) equilibrated with PBS. After entry of the whole culture solution into the column, the column was washed with PBS in an amount of twice or more the column volume. Next, antibody-containing fractions were collected by elution with a 2 M arginine hydrochloride solution (pH 4.0). The collected fractions were buffer-replaced with HBSor (25 mM histidine/5% sorbitol, pH 6.0) by dialysis (Thermo Fisher Scientific Inc., Slide-A-Lyzer Dialysis Cassette). The antibody was concentrated and adjusted to an IgG concentration of 10 mg/ml or higher using Centrifugal UF Filter Device VIVASPIN20 (molecular weight cutoff: UF10K, Sartorius Japan K.K., 4°C), and used as a purified sample. Finally, this purified sample was filtered through Minisart-Plus filter (Sartorius Japan K.K.).

(Example 5) *In vitro* activity of human chimeric anti-GPRC5D antibodies (c2A4, c2B1, and c7B4)

5)-1 Study on binding property of human chimeric anti-GPRC5D antibodies (c2A4, c2B1, and c7B4) to human GPRC5D by flow cytometry

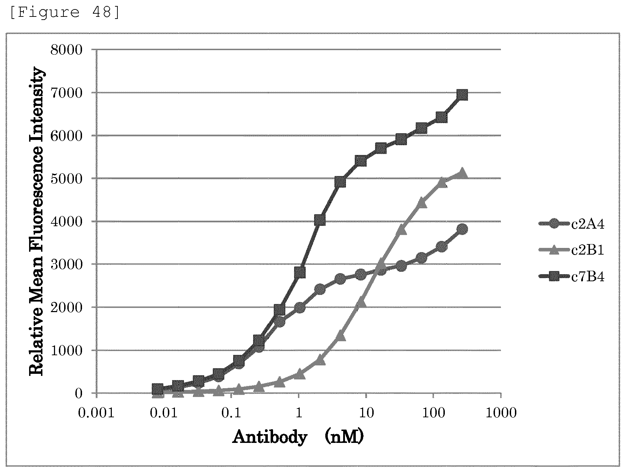

Human multiple myeloma cell line KHM-1B cells expressing GPRC5D were adjusted to a concentration of 5 $\times$ $10^6$ cells/mL with PBS containing 5% FBS, inoculated in an amount of 100 μL/well to a 96-well U-bottomed microplate, and centrifuged to remove a supernatant. Each human chimeric anti-GPRC5D antibody (c2A4, c2B1, and c7B4) or Human IgG isotype control antibody (Calbiochem/Merck Millipore Corp.) adjusted to 1.2 ng/mL to 40 μg/mL was added thereto in an amount of 100 μL/well, and the plate was left standing at 4°C for 1 hour. The cells were washed twice with PBS containing 5% FBS. Then, R-Phycoerythrin AffiniPure F(ab')2 Fragment Goat Anti-Human IgG, Fcγ Fragment Specific (Jackson ImmunoResearch Laboratories, Inc.) diluted 100-fold with PBS containing 5% FBS was added thereto in an amount of 100 μL/well, and the plate was left standing at 4°C for 1 hour. The cells were washed twice with PBS containing 5% FBS and then resuspended in PBS containing 5% FBS, followed by detection using a flow cytometer (FACSCanto(TM) II; Becton, Dickinson and Company). The data was analyzed using Flowjo (Tree Star, Inc.). The PE fluorescence intensity of the cell fraction was plotted to a histogram, and the mean fluorescence intensity (MFI) was calculated. The MFI value of the control antibody was subtracted from the MFI value of the GPRC5D antibody to calculate a relative value of MFI (I). As shown in Figure 48, c2A4, c2B1, and c7B4 were found to bind to human GPRC5D.

5)-2 Study on cross-reactivity of human chimeric anti-GPRC5D antibodies (c2A4, c2B1, and c7B4) with cynomolgus monkey GPRC5D

5)-2-1 Construction of cynomolgus monkey GPRC5D expression vector (pcDNA3.1-DEST -cGPRC5D)

A cDNA encoding the cynomolgus monkey GPRC5D protein (XP_005570249.1) was cloned into the pcDNA3.1-DEST vector prepared in Example 1)-1-1 using Gateway LR Clonase Enzyme mix (Life Technologies Corp.) to construct a cynomolgus monkey GPRC5D expression vector pcDNA3.1-DEST-cGPRC5D. For the large-scale preparation of the cynomolgus monkey GPRC5D expression vector, Endofree Plasmid Giga Kit (Qiagen N.V.) was used.

5)-2-2 Preparation of cynomolgus monkey GPRC5D-expressing cell line

A multiple myeloma cell line KMS-11 (JCRB Cell Bank) not expressing human GPRC5D was inoculated at a concentration of $3.7 \times 10^5$ cells/mL to a 75-cm$^2$ flask using an RPMI1640 medium containing 10% FBS. pcDNA3.1-DEST-cGPRC5D was transfected to the KMS-11 cells using Lipofectamine 2000 (Thermo Fisher Scientific Inc.), and the cells were cultured at 37°C for 2 days under 5% $CO_2$ conditions. The cultured expression vector-transfected KMS-11 cells were cultured at a concentration of $1 \times 10^6$ cells/mL in an RPMI1640 medium containing 10% FBS and 1 mg/mL Geneticin (Thermo Fisher Scientific Inc.) for drug screening. Bulk cells were prepared as single clones by the limiting dilution method using ClonaCell-HY Selection Medium E medium (StemCell Technologies Inc.) containing 1 mg/mL Geneticin (Thermo Fisher Scientific Inc.) to establish a cynomolgus monkey GPRC5D-expressing multiple myeloma cell line KMS-11_cGPRC5D.

5)-2-3 Study on binding property of human chimeric anti-GPRC5D antibodies (c2A4, c2B1, and c7B4) to cynomolgus monkey GPRC5D by flow cytometry

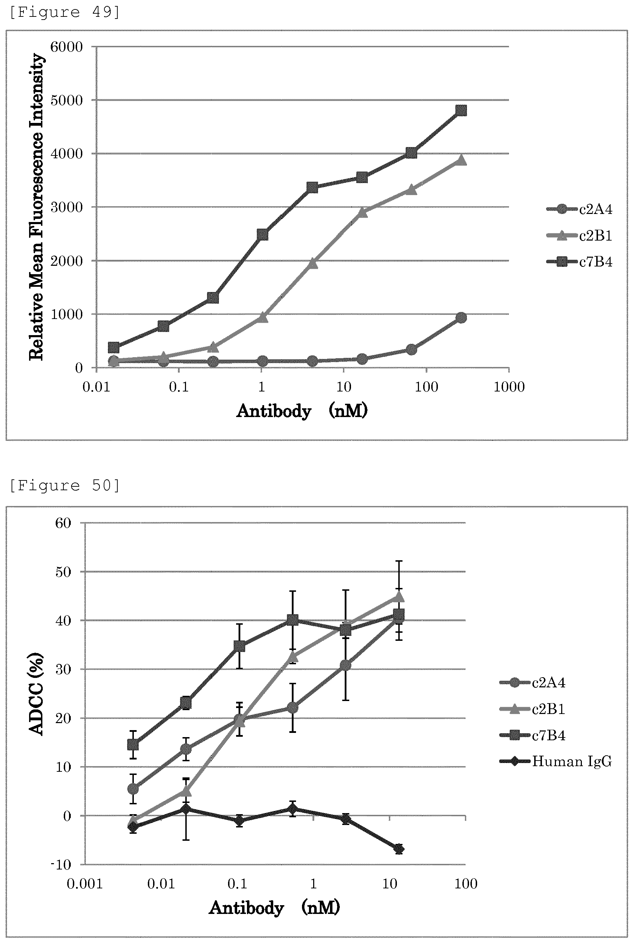

The KMS-11_cGPRC5D cells prepared in Example 5)-2-2 were adjusted to a concentration of $5 \times 10^6$ cells/mL with PBS containing 5% FBS, inoculated in an amount of 100 μL/well to a 96-well U-bottomed microplate, and centrifuged to remove a supernatant. Each human chimeric anti-GPRC5D antibody (c2A4, c2B1, and c7B4) or Human IgG isotype control antibody (Calbiochem/Merck Millipore Corp.) adjusted to 1.2 ng/mL to 40 μg/mL was added thereto in an amount of 100 μL/well, and the plate was left standing at 4°C for 1 hour. The cells were washed twice with PBS containing 5% FBS. Then, R-Phycoerythrin AffiniPure F(ab')2 Fragment Goat Anti-Human IgG, Fcγ Fragment Specific (Jackson ImmunoResearch Laboratories, Inc.) diluted 100-fold with PBS containing 5% FBS was added thereto in an amount of 100 μL/well, and the plate was left standing at 4°C for 1 hour. The cells were washed twice with PBS containing 5% FBS and then resuspended in PBS containing 5% FBS, followed by detection using a flow cytometer (FACSCanto(TM) II; Becton, Dickinson and Company). The data was analyzed using Flowjo (Tree Star, Inc.). The PE fluorescence intensity of the cell fraction was plotted to a histogram, and the mean fluorescence intensity (MFI) was calculated. The MFI value of the control antibody was subtracted from the MFI value of the GPRC5D antibody to calculate a relative value of MFI (rMFI). As shown in Figure 49, c2A4, c2B1, and c7B4 were found to bind to cynomolgus monkey GPRC5D. The antibody, etc. that binds to human GPRC5D and cynomolgus monkey GPRC5D can be subjected to various tests on efficacy or safety using primates, particularly, cynomolgus monkeys, useful for the nonclinical development (preclinical development) of pharmaceutical products, and are thus preferred. Also, the antibody, etc. that binds to human GPRC5D and cynomolgus monkey GPRC5D have cytotoxic activity and are useful, either alone or as the molecule of the present invention, in the treatment or prevention of diseases such as cancers in cynomolgus monkeys.

As a result of studying the cross-reactivity of c2A4, c2B1, and c7B4 with rat GPRC5D and mouse GPRC5D in the same way as in Example 5)-2, none of c2A4, c2B1, and c7B4 bound to rat GPRC5D and mouse GPRC5D. By virtue of these antibodies c2A4, c2B1, and c7B4, various assays, immunohistochemical tests, etc. using human GPRC5D gene-transfected mouse or rat cells, tissues, or individuals (including transgenic animals, knockout animals, and knock-in animals) and the antibody, etc. can be carried out without being influenced by GPRC5D of the host mice. Thus, these antibodies are preferred for research and nonclinical development using mice or rats, of drugs, animal drugs, or diagnostic drugs, etc., including the antibody, etc.

5)-3 ADCC activity of human chimeric anti-GPRC5D antibodies (c2A4, c2B1, and c7B4)

The KHM-1B cells prepared in Example 2)-3-1 were added in an amount of 50 μL/well to a 96-well U-bottomed microplate. Each purified human chimeric anti-GPRC5D antibody (c2A4, c2B1, and c7B4) prepared in Example 4 and a human control antibody (hIgG1) (Calbiochem/Merck Millipore Corp.) adjusted to 0.64 ng/mL to 2 μg/mL (final concentration) was added thereto in an amount of 50 μL/well, and the plate was left standing at 4°C for 30 minutes. The effector cells (adjusted to $3 \times 10^6$ cells/mL) prepared in Example 1)-5-3 were further added thereto at a concentration of 100 μL/well. After centrifugation at room temperature at 1200 rpm for 3 minutes, the cells were cultured at 37°C for 4 hours under 5% $CO_2$ conditions. A 50 μL aliquot of the supernatant was recovered into LumaPlate (PerkinElmer, Inc.) and dried overnight at 50°C, followed by measurement using a plate reader (TopCount; PerkinElmer, Inc.). The percentage of cells lysed by ADCC activity was calculated according to Example 1)-5-5. As shown in Figure 50, c2A4, c2B1, and c7B4 were found to have ADCC activity.

(Example 6) *In vivo* activity of human chimeric anti-GPRC5D antibodies (c2A4, c2B1, and c7B4)

$1 \times 10^7$ cells of a human multiple myeloma cell line KHM-1B were suspended in 100% Matrigel (Becton, Dickinson and Company) and subcutaneously grafted to the axillary region of each BALB/c-nu/nu mouse (CanN.Cg-Foxn1nu/CrlCrlj, purchased from Charles River Laboratories Japan Inc.). Three and 10 days after grafting, each human chimeric anti-GPRC5D antibody (c2A4, c2B1, and c7B4) was administered at a dose of 10 mg/kg to the tail veins of the cancer-bearing mice (n = 12 or 11). The major axis and minor axis of the grafted tumors were measured twice a week using an electronic digital caliper (manufactured by Mitsutoyo Corp.). The tumor volume was calculated according to the following expression:

$$\text{Tumor volume (mm}^3\text{)} = 1/2 \times \text{Minor axis (mm)} \times \text{Minor axis (mm)} \times \text{Major axis (mm)}$$

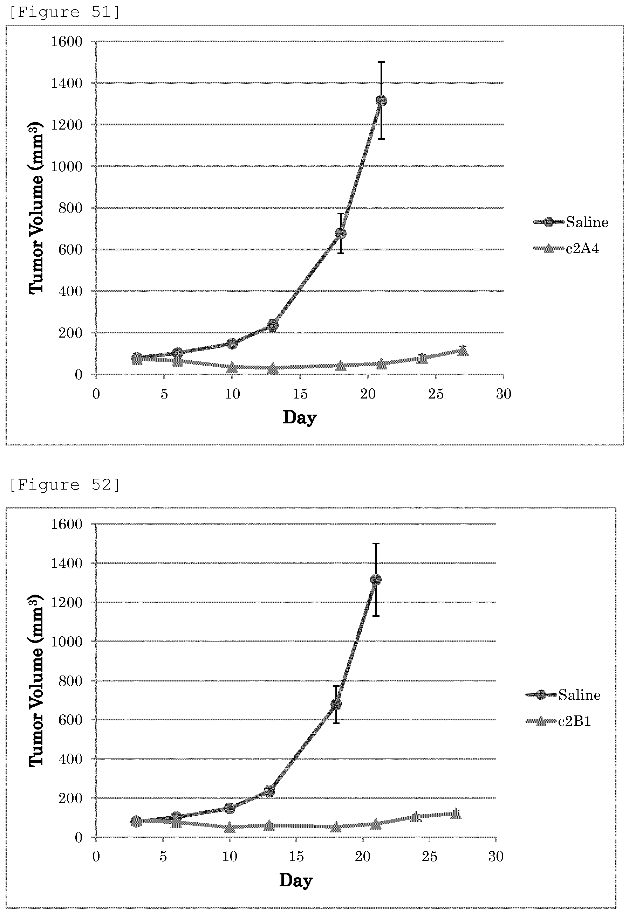

The results of the c2A4 antibody are shown in Figure 51. The percentage of tumor growth inhibition at 21 days after grafting was 96%.
The results of the c2B1 antibody are shown in Figure 52. The percentage of tumor growth inhibition at 21 days after grafting was 95%.
The results of the c7B4 antibody are shown in Figure 53. The percentage of tumor growth inhibition at 21 days after grafting was 94%.

(Example 7) Design of humanized versions (h2B1 and h7B4) of human chimeric anti-GPRC5D antibodies (c2B1 and c7B4)

7)-1 Design of humanized form of anti-GPRC5D antibody 2B1

7)-1-1 Molecular modeling of 2B1 variable regions

The molecular modeling of the 2B1 variable regions was carried out by a method generally known as homology modeling (Methods in Enzymology, 203, 121-153, (1991)). The variable regions of 2B1 determined above were compared with the primary sequences (three-dimensional structures derived from X-ray crystal structures that are available) of human immunoglobulin variable regions registered in Protein Data Bank (Nuc. Acid Res. 35, D301-D303 (2007)). As a result, 3MBX was selected because it had the highest sequence identity to the heavy and light chain variable regions of 2B1. The three-dimensional structures of framework regions were prepared as a "framework model" by combining the coordinates of 3MBX corresponding to the heavy and light chains of 2B1. Subsequently, the typical conformation of each CDR was incorporated into the framework model. Finally, energy calculation for excluding disadvantageous interatomic contact was conducted in order to obtain possible molecular models of the 2B1 variable regions in terms of energy. These procedures were performed using a commercially available protein three-dimensional structure analysis program BioLuminate (manufactured by Schrodinger, LLC).

7)-1-2 Design of amino acid sequence of humanized h2B1

Humanized h2B1 was constructed by a method generally known as CDR grafting (Proc. Natl. Acad. Sci. USA 86, 10029-10033 (1989)). An acceptor antibody was selected on the basis of the identity of amino acids in framework regions.

The sequences of the framework regions of 2B1 were compared with the framework regions of human subgroup consensus sequences or germline sequences specified by KABAT et al. (Sequences of Proteins of Immunological Interest, 5th Ed. Public Health Service National Institutes of Health, Bethesda, MD. (1991)). As a result, human germline sequences IGHV2_5x08 and IGHJ1x01 and a human gamma chain subgroup 2 consensus sequence were selected as a heavy chain acceptor while human germline sequences IGKV1_8x01 and IGKJ4x01 and a human kappa chain subgroup 4 consensus sequence were selected as light chain acceptor due to their high sequence identity as to framework regions. The amino acid residues of the framework regions of the acceptors were aligned with the amino acid residues of the 2B1 framework regions to identify the positions of amino acids that did not match therebetween. The positions of these residues were analyzed using the three-dimensional model of 2B1 constructed above. Then, the donor residues to be grafted onto the acceptors were selected according to the criteria provided by Queen et al. (Proc. Natl. Acad. Sci. USA 86, 10029-10033 (1989)). Some donor residues thus selected were transferred to the acceptor antibody to construct the humanized h2B1 sequence as described in Examples below. The heavy chain was not limited by donor residues, and depending on a site, residues of a gamma chain subgroup 1 consensus sequence were transferred thereto.

7)-1-3 Humanization of 2B1 heavy chain

7)-1-3-1 Humanized h2B1_H1 type heavy chain

A humanized h2B1 heavy chain designed from the chimeric c2B1 heavy chain shown in SEQ ID NO: 34 by the replacement in the variable region of threonine at amino acid position 3 with glutamine, lysine at amino acid position 5 with valine, proline at amino acid position 9 with glycine, isoleucine at amino acid position 11 with leucine, leucine at amino acid position 12 with valine, glutamine at amino acid position 13 with lysine, serine at amino acid position 43 with proline, leucine at amino acid position 50 with isoleucine, alanine at amino acid position 51 with glycine, arginine at amino acid position 66 with lysine, asparagine at amino acid position 67 with serine, leucine at amino acid position 69 with valine, lysine at amino acid position 73 with valine, asparagine at amino acid position 77 with lysine, phenylalanine at amino acid position 81 with serine, isoleucine at amino acid position 84 with leucine, threonine at amino acid position 85 with serine, asparagine at amino acid position 86 with serine, aspartic acid at amino acid position 88 with threonine, threonine at amino acid position 89 with alanine, and threonine at amino acid position 94 with valine was designated as "humanized h2B1_H1 type heavy chain" (also referred to as "h2B1_H1").

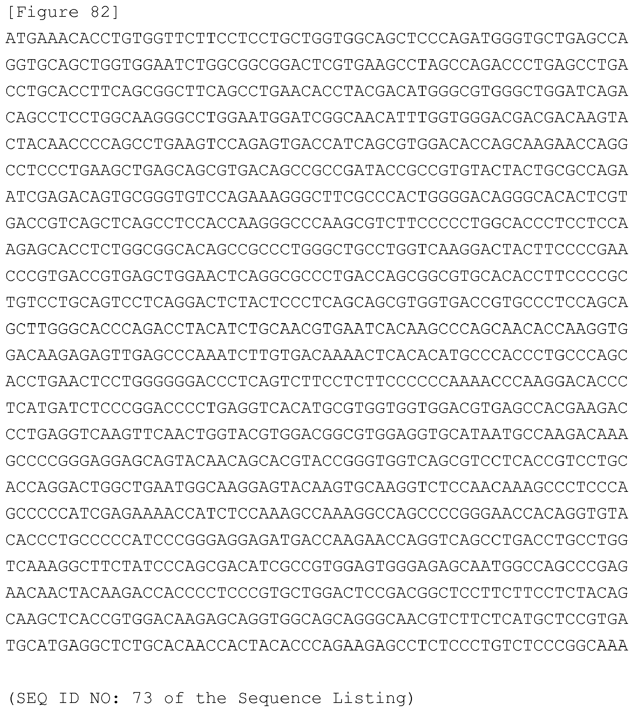

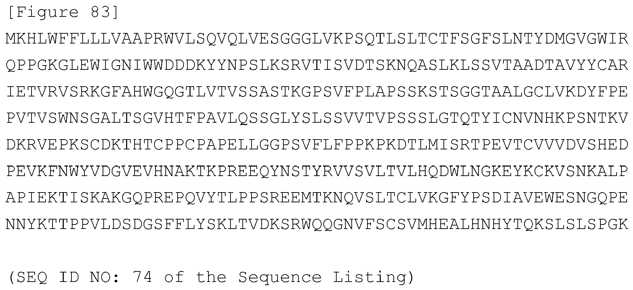

The amino acid sequence of the humanized h2B1_H1 type heavy chain is described in SEQ ID NO: 74 (Figure 83) of the Sequence Listing. A nucleotide sequence encoding the amino acid sequence of SEQ ID NO: 74 is described in SEQ ID NO: 73 (Figure 82) of the Sequence Listing.

7)-1-3-2 Humanized h2B1_H2 type heavy chain

A humanized h2B1 heavy chain designed from the chimeric c2B1 heavy chain shown in SEQ ID NO: 34 by the replacement in the variable region of threonine at amino acid position 3 with glutamine, lysine at amino acid position 5 with valine, proline at amino acid position 9 with glycine, isoleucine at amino acid position 11 with leucine, leucine at amino acid position 12 with valine, glutamine at amino acid position 13 with lysine, serine at amino acid position 43 with proline, leucine at amino acid position 50 with isoleucine, alanine at amino acid position 51 with glycine, arginine at amino acid position 66 with lysine, asparagine at amino acid position 67 with serine, leucine at amino acid position 69 with valine, phenylalanine at amino acid position 81 with serine, isoleucine at amino acid position 84 with leucine, threonine at amino acid position 85 with serine, asparagine at amino acid position 86 with serine, aspartic acid at amino acid position 88 with threonine, threonine at amino acid position 89 with alanine, and threonine at amino acid position 94 with valine was designated as "humanized h2B1_H2 type heavy chain" (also referred to as "h2B1_H2").

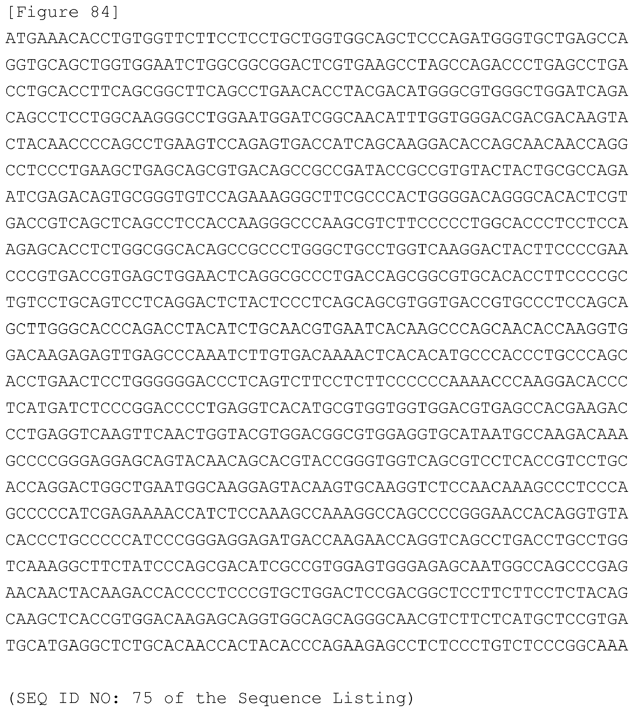

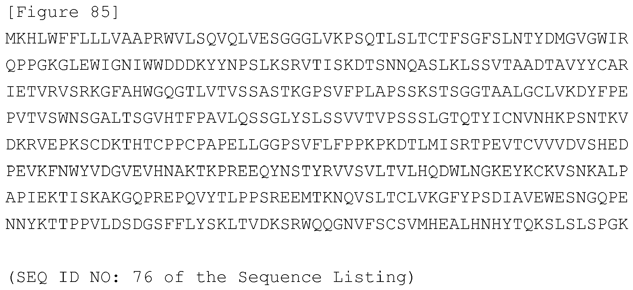

The amino acid sequence of the humanized h2B1_H2 type heavy chain is described in SEQ ID NO: 76 (Figure 85) of the Sequence Listing. A nucleotide sequence encoding the amino acid sequence of SEQ ID NO: 76 is described in SEQ ID NO: 75 (Figure 84) of the Sequence Listing.

7)-1-3-3 Humanized h2B1_H3 type heavy chain

A humanized h2B1 heavy chain designed from the chimeric c2B1 heavy chain shown in SEQ ID NO: 34 by the replacement in the variable region of threonine at amino acid position 3 with glutamine, lysine at amino acid position 5 with valine, proline at amino acid position 9 with glycine, isoleucine at amino acid position 11 with leucine, leucine at amino acid position 12 with valine, glutamine at amino acid position 13 with lysine, serine at amino acid position 43 with proline, leucine at amino acid position 50 with isoleucine, arginine at amino acid position 66 with lysine, asparagine at amino acid position 67 with serine, leucine at amino acid position 69 with valine, phenylalanine at amino acid position 81 with serine, isoleucine at amino acid position 84 with leucine, threonine at amino acid position 85 with serine, asparagine at amino acid position 86 with serine, aspartic acid at amino acid position 88 with threonine, threonine at amino acid position 89 with alanine, and threonine at amino acid position 94 with valine was designated as "humanized h2B1_H3 type heavy chain" (also referred to as "h2B1_H3").

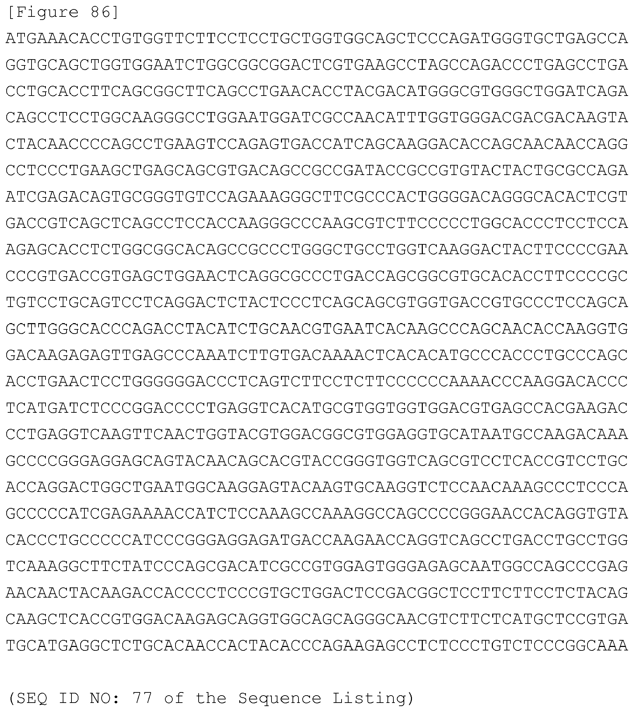

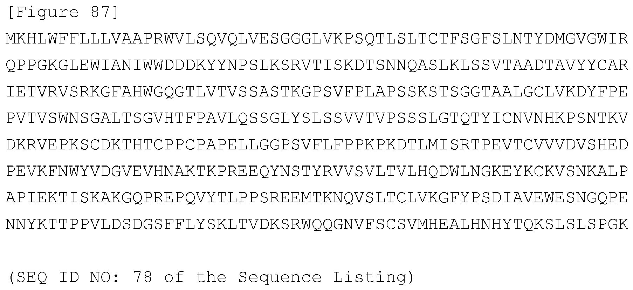

The amino acid sequence of the humanized h2B1_H3 type heavy chain is described in SEQ ID NO: 78 (Figure 87) of the Sequence Listing. A nucleotide sequence encoding the amino acid sequence of SEQ ID NO: 78 is described in SEQ ID NO: 77 (Figure 86) of the Sequence Listing.

7)-1-3-4 Humanized h2B1_H4 type heavy chain

A humanized h2B1 heavy chain designed from the chimeric c2B1 heavy chain shown in SEQ ID NO: 34 by the replacement in the variable region of glycine at amino acid position 10 with alanine, isoleucine at amino acid position 11 with leucine, leucine at amino acid position 12 with valine, glutamine at amino acid position 13 with lysine, serine at amino acid position 15 with threonine, serine at amino acid position 19 with threonine, serine at amino acid position 43 with proline, asparagine at amino acid position 62 with serine, arginine at amino acid position 66 with lysine, asparagine at amino acid position 67 with serine, serine at amino acid position 72 with threonine, phenylalanine at amino acid position 81 with valine, lysine at amino acid position 83 with threonine, isoleucine at amino acid position 84 with methionine, valine at amino acid position 87 with methionine, threonine at amino acid position 89 with proline, and alanine at amino acid position 90 with valine was designated as "humanized h2B1_H4 type heavy chain" (also referred to as "h2B1_H4").

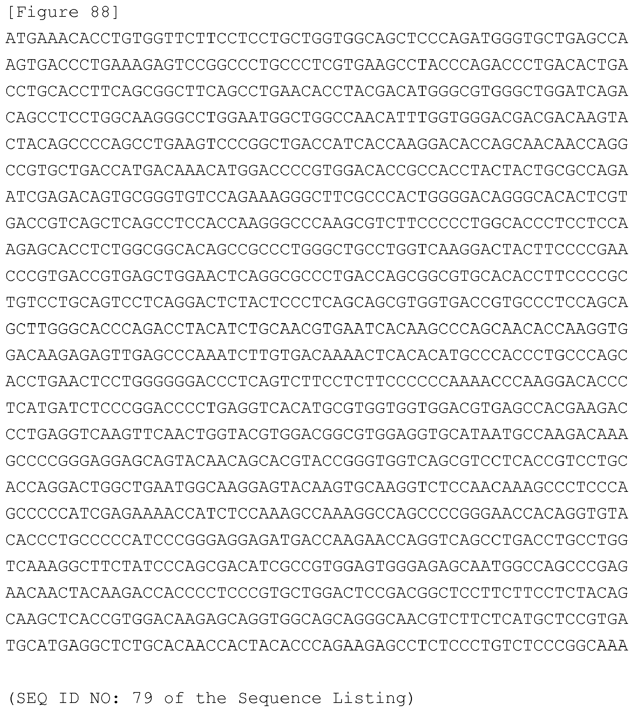

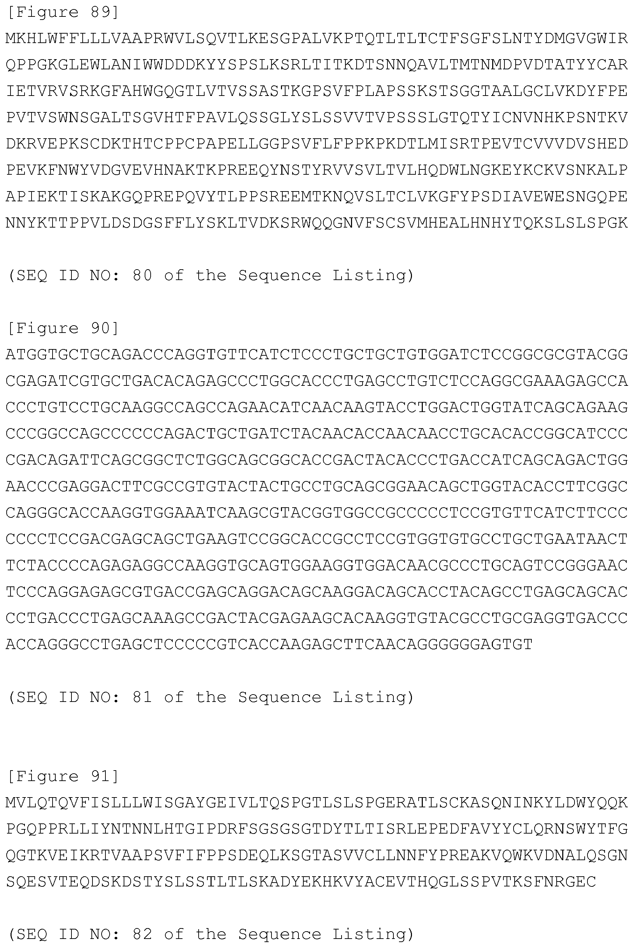

The amino acid sequence of the humanized h2B1_H4 type heavy chain is described in SEQ ID NO: 80 (Figure 89) of the Sequence Listing. A nucleotide sequence encoding the amino acid sequence of SEQ ID NO: 80 is described in SEQ ID NO: 79 (Figure 88) of the Sequence Listing.

7)-1-4 Humanization of 2B1 light chain

7)-1-4-1 Humanized h2B1_L1 type light chain

A humanized h2B1 light chain designed from the chimeric c2B1 light chain shown in SEQ ID NO: 30 by the replacement in the variable region of glutamic acid at amino acid position 1 with aspartic acid, threonine at amino acid position 9 with aspartic acid, methionine at amino acid position 11 with leucine, serine at amino acid position 12 with alanine, threonine at amino acid position 13 with valine, isoleucine at amino acid position 15 with leucine, valine at amino acid position 19 with alanine, leucine at amino acid position 21 with isoleucine, threonine at amino acid position 39 with lysine, serine at amino acid position 43 with proline, threonine at amino acid position 63 with serine, phenylalanine at amino acid position 67 with serine, asparagine at amino acid position 77 with serine, valine at amino acid position 78 with leucine, glutamic acid at amino acid position 79 with glutamine, leucine at amino acid position 83 with valine, glycine at amino acid position 100 with glutamine, leucine at amino acid position 104 with valine, and leucine at amino acid position 106 with isoleucine was designated as "humanized h2B1_L1 type light chain" (also referred to as "h2B1_L1").

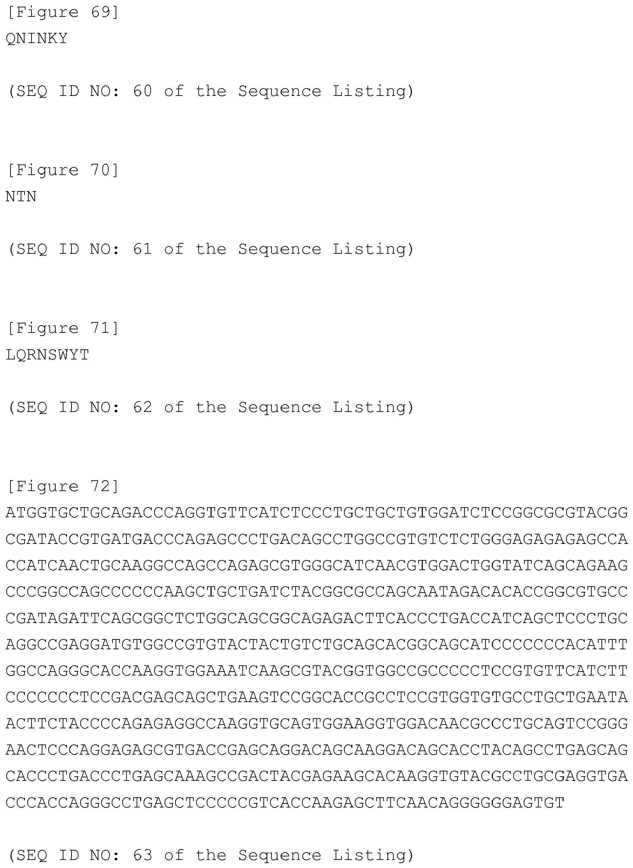

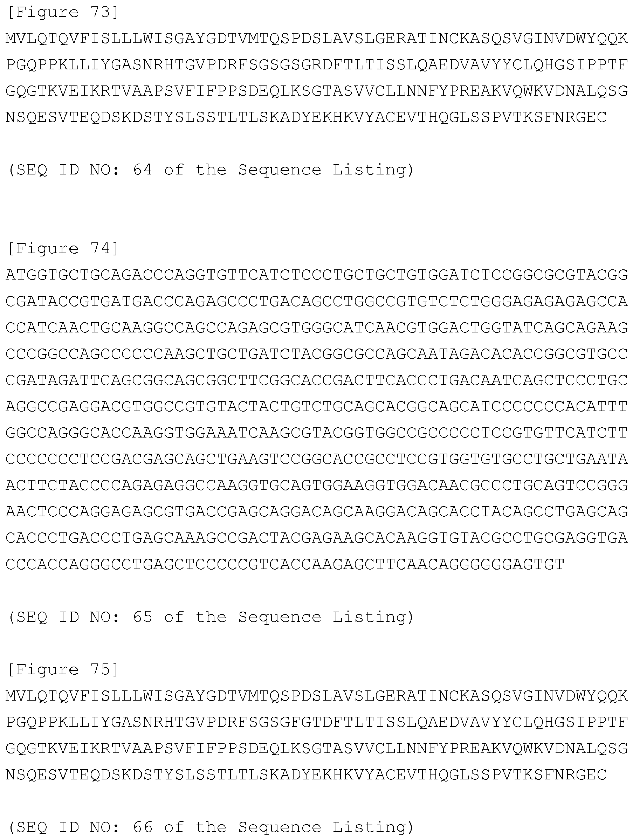

The amino acid sequence of the humanized h2B1_L1 type light chain is described in SEQ ID NO: 64 (Figure 73) of the Sequence Listing. A nucleotide sequence encoding the amino acid sequence of SEQ ID NO: 64 is described in SEQ ID NO: 63 (Figure 72) of the Sequence Listing.

7)-1-4-2 Humanized h2B1_L2 type light chain

A humanized h2B1 light chain designed from the chimeric c2B1 light chain shown in SEQ ID NO: 30 by the replacement in the variable region of glutamic acid at amino acid position 1 with aspartic acid, threonine at amino acid position 9 with aspartic acid, methionine at amino acid position 11 with leucine, serine at amino acid position 12 with alanine, threonine at amino acid position 13 with valine, isoleucine at amino acid position 15 with leucine, valine at amino acid position 19 with alanine, leucine at amino acid position 21 with isoleucine, threonine at amino acid position 39 with lysine, serine at amino acid position 43 with proline, threonine at amino acid position 63 with serine, arginine at amino acid position 69 with threonine, asparagine at amino acid position 77 with serine, valine at amino acid position 78 with leucine, glutamic acid at amino acid position 79 with glutamine, leucine at amino acid position 83 with valine, glycine at amino acid position 100 with glutamine, leucine at amino acid position 104 with valine, and leucine at amino acid position 106 with isoleucine was designated as "humanized h2B1_L2 type light chain" (also referred to as "h2B1_L2").

The amino acid sequence of the humanized h2B1_L2 type light chain is described in SEQ ID NO: 66 (Figure 75) of the Sequence Listing. A nucleotide sequence encoding the amino acid sequence of SEQ ID NO: 66 is described in SEQ ID NO: 65 (Figure 74) of the Sequence Listing.

7)-1-4-3 Humanized h2B1_L3 type light chain

A humanized h2B1 light chain designed from the chimeric c2B1 light chain shown in SEQ ID NO: 30 by the replacement in the variable region of glutamic acid at amino acid position 1 with aspartic acid, threonine at amino acid position 9 with aspartic acid, methionine at amino acid position 11 with leucine, serine at amino acid position 12 with alanine, threonine at amino acid position 13 with valine, isoleucine at amino acid position 15 with leucine, valine at amino acid position 19 with alanine, leucine at amino acid position 21 with isoleucine, threonine at amino acid position 39 with lysine, serine at amino acid position 43 with proline, threonine at amino acid position 63 with serine, asparagine at amino acid position 77 with serine, valine at amino acid position 78 with leucine, glutamic acid at amino acid position 79 with glutamine, leucine at amino acid position 83 with valine, glycine at amino acid position 100 with glutamine, leucine at amino acid position 104 with valine, and leucine at amino acid position 106 with isoleucine was designated as "humanized h2B1_L3 type light chain" (also referred to as "h2B1_L3").

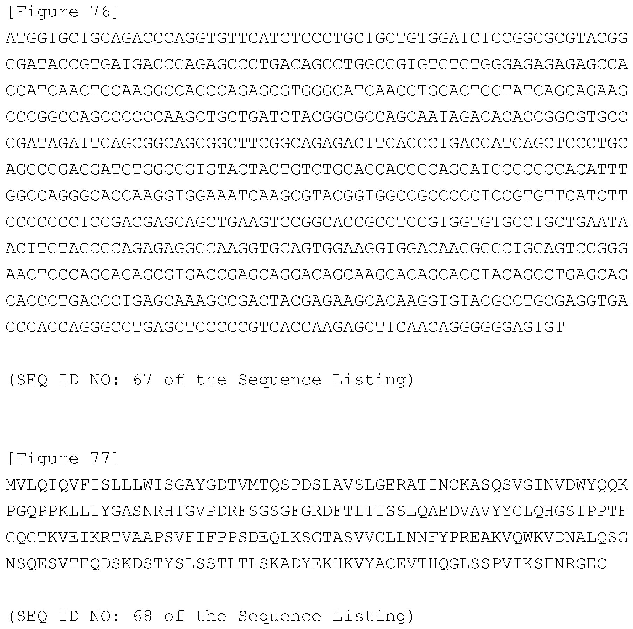

The amino acid sequence of the humanized h2B1_L3 type light chain is described in SEQ ID NO: 68 (Figure 77) of the Sequence Listing. A nucleotide sequence encoding the amino acid sequence of SEQ ID NO: 68 is described in SEQ ID NO: 67 (Figure 76) of the Sequence Listing.

7)-1-4-4 Humanized h2B1_L4 type light chain

A humanized h2B1 light chain designed from the chimeric c2B1 light chain shown in SEQ ID NO: 30 by the replacement in the variable region of glutamic acid at amino acid position 1 with aspartic acid, threonine at amino acid position 9 with aspartic acid, methionine at amino acid position 11 with leucine, serine at amino acid position 12 with alanine, threonine at amino acid position 13 with valine, isoleucine at amino acid position 15 with leucine, valine at amino acid position 19 with alanine, leucine at amino acid position 21 with isoleucine, threonine at amino acid position 39 with lysine, threonine at amino acid position 63 with serine, asparagine at amino acid position 77 with serine, valine at amino acid position 78 with leucine, glutamic acid at amino acid position 79 with glutamine, leucine at amino acid position 83 with valine, glycine at amino acid position 100 with glutamine, leucine at amino acid position 104 with valine, and leucine at amino acid position 106 with isoleucine was designated as "humanized h2B1_L4 type light chain" (also referred to as "h2B1_L4").

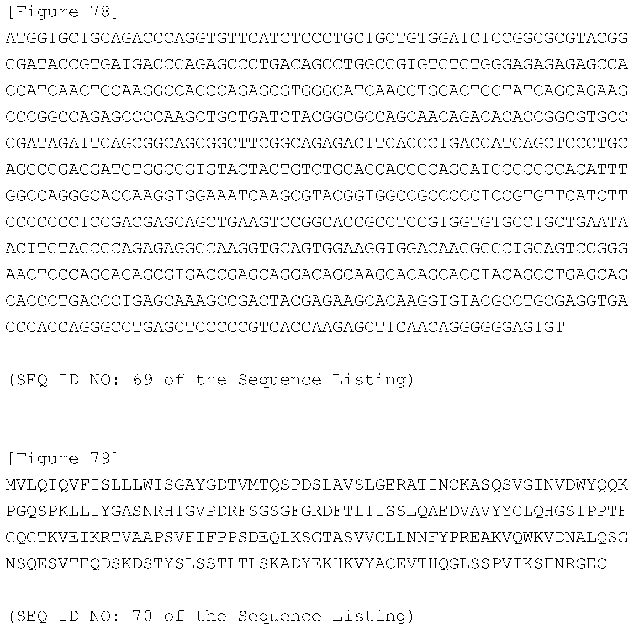

The amino acid sequence of the humanized h2B1_L4 type light chain is described in SEQ ID NO: 70 (Figure 79) of the Sequence Listing. A nucleotide sequence encoding the amino acid sequence of SEQ ID NO: 70 is described in SEQ ID NO: 69 (Figure 78) of the Sequence Listing.

7)-1-4-5 Humanized h2B1_L5 type light chain

A humanized h2B1 light chain designed from the chimeric c2B1 light chain shown in SEQ ID NO: 30 by the replacement in the variable region of glutamic acid at amino acid position 1 with alanine, valine at amino acid position 3 with arginine, threonine at amino acid position 9 with serine, methionine at amino acid position 11 with phenylalanine, threonine at amino acid position 13 with alanine, isoleucine at amino acid position 15 with threonine, glutamic acid at amino acid position 17 with aspartic acid, leucine at amino acid position 21 with isoleucine, asparagine at amino acid position 22 with threonine, threonine at amino acid position 39 with lysine, glutamine at amino acid position 42 with lysine, aspartic acid at amino acid position 60 with serine, threonine at amino acid position 63 with serine, asparagine at amino acid position 77 with serine, valine at amino acid position 78 with leucine, glutamic acid at amino acid position 79 with glutamine, alanine at amino acid position 80 with serine, leucine at amino acid position 83 with phenylalanine, valine at amino acid position 85 with threonine, leucine at amino acid position 104 with valine, and leucine at amino acid position 106 with isoleucine was designated as "humanized h2B1_L5 type light chain" (also referred to as "h2B1_L5").

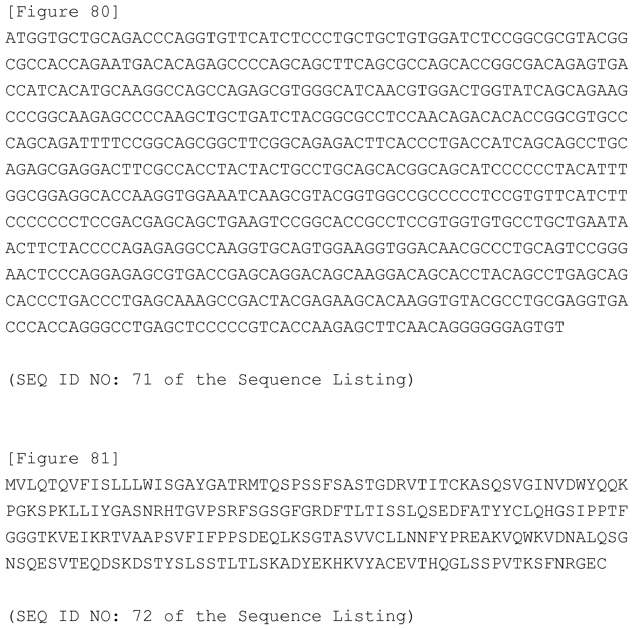

The amino acid sequence of the humanized h2B1_L5 type light chain is described in SEQ ID NO: 72 (Figure 81) of the Sequence Listing. A nucleotide sequence encoding the amino acid sequence of SEQ ID NO: 72 is described in SEQ ID NO: 71 (Figure 80) of the Sequence Listing.

7)-2 Design of humanized form of anti-GPRC5D antibody 7B4

7)-2-1 Molecular modeling of 7B4 variable regions

The molecular modeling of the 7B4 variable regions was carried out by a method generally known as homology modeling (Methods in Enzymology, 203, 121-153, (1991)). The variable regions of 7B4 determined above were compared with the primary sequences (three-dimensional structures derived from X-ray crystal structures that are available) of human immunoglobulin variable regions registered in Protein Data Bank (Nuc. Acid Res. 35, D301-D303 (2007)). As a result, 1BGX was selected because it had the highest sequence identity to the heavy and light chain variable regions of 7B4. The three-dimensional structures of framework regions were prepared as a "framework model" by combining the coordinates of 1BGX corresponding to the heavy and light chains of 7B4. Subsequently, the typical conformation of each CDR was incorporated into the framework model. Finally, energy calculation for excluding disadvantageous interatomic contact was conducted in order to obtain possible molecular models of the 7B4 variable regions in terms of energy. These procedures were performed using a commercially available protein three-dimensional structure analysis program BioLuminate (Schrodinger, LLC).

7)-2-2 Design of amino acid sequence of humanized h7B4

Humanized h7B4 was constructed by a method generally known as CDR grafting (Proc. Natl. Acad. Sci. USA 86, 10029-10033 (1989)). An acceptor antibody was selected on the basis of the identity of amino acids in framework regions.

The sequences of the framework regions of 7B4 were compared with the framework regions of human subgroup consensus sequences or germline sequences specified by KABAT et al. (Sequences of Proteins of Immunological Interest, 5th Ed. Public Health Service National Institutes of Health, Bethesda, MD. (1991)). As a result, a human gamma chain subgroup 2 consensus sequence was selected as a heavy chain acceptor while a human kappa chain subgroup 3 consensus sequence was selected as light chain acceptor due to their high sequence identity as to framework regions. The amino acid residues of the framework regions of the acceptors were aligned with the amino acid residues of the 7B4 framework regions to identify the positions of amino acids that did not match there-between. The positions of these residues were analyzed using the three-dimensional model of 7B4 constructed above. Then, the donor residues to be grafted onto the acceptors were selected according to the criteria provided by Queen et al. (Proc. Natl. Acad. Sci. USA 86, 10029-10033 (1989)). Some donor residues thus selected were transferred to the acceptor antibody to construct the humanized h7B4 sequence as described in Examples below. The light chain was not limited by donor residues, and depending on a site, residues of a kappa chain subgroup 1 consensus sequence were transferred thereto.

7)-2-3 Humanization of 7B4 heavy chain

7)-2-3-1 Humanized h7B4_H1 type heavy chain

A humanized h7B4 heavy chain designed from the chimeric c7B4 heavy chain shown in SEQ ID NO: 42 by the replacement in the variable region of glutamic acid at amino acid position 1 with glutamine, isoleucine at amino acid position 2 with valine, histidine at amino acid position 3 with glutamine, serine at amino acid position 17 with threonine, serine at amino acid position 23 with threonine, threonine at amino acid position 25 with serine, lysine at amino acid position 40 with glutamine, phenylalanine at amino acid position 41 with proline, asparagine at amino acid position 44 with lysine, lysine at amino acid position 45 with glycine, methionine at amino acid position 46 with leucine, methionine at amino acid position 49 with isoleucine, isoleucine at amino acid position 68 with valine, serine at amino acid position 69 with threonine, threonine at amino acid position 71 with serine, phenylalanine at amino acid position 80 with serine, glutamine at amino acid position 82 with lysine, asparagine at amino acid position 84 with serine, threonine at amino acid position 88 with alanine, glutamic acid at amino acid position 89 with alanine, threonine at amino acid position 93 with valine, alanine at amino acid position 117 with threonine, and serine at amino acid position 118 with leucine was designated as "humanized h7B4_H1 type heavy chain" (also referred to as "h7B4_H1").

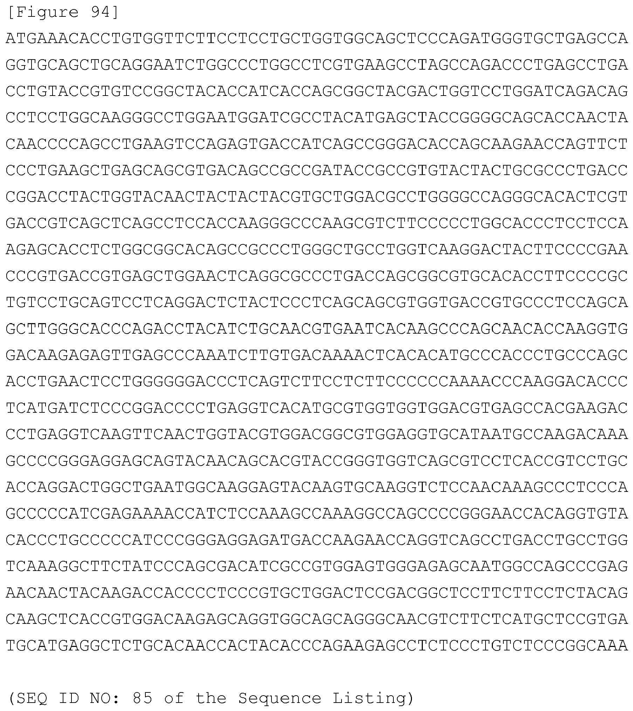

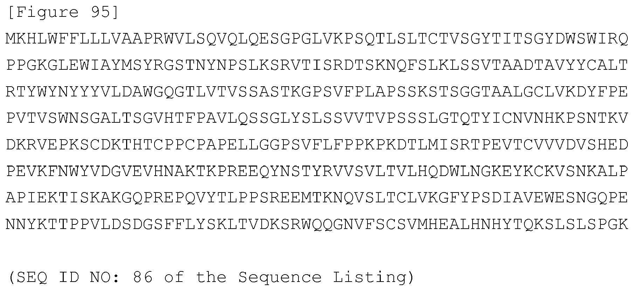

The amino acid sequence of the humanized h7B4_H1 type heavy chain is described in SEQ ID NO: 86 (Figure 95) of the Sequence Listing. A nucleotide sequence encoding the amino acid sequence of SEQ ID NO: 86 is described in SEQ ID NO: 85 (Figure 94) of the Sequence Listing.

7)-2-3-2 Humanized h7B4_H2 type heavy chain

A humanized h7B4 heavy chain designed from the chimeric c7B4 heavy chain shown in SEQ ID NO: 42 by the replacement in the variable region of histidine at amino acid position 3 with glutamine, serine at amino acid position 17 with threonine, serine at amino acid position 23 with threonine, threonine at amino acid position 25 with serine, lysine at amino acid position 40 with glutamine, phenylalanine at amino acid position 41 with proline, asparagine at amino acid position 44 with lysine, lysine at amino acid position 45 with glycine, methionine at amino acid position 46 with leucine, methionine at amino acid position 49 with isoleucine, alanine at amino acid position 50 with glycine, isoleucine at amino acid position 68 with valine, serine at amino acid position 69 with threonine, threonine at amino acid position 71 with serine, phenylalanine at amino acid position 80 with serine, glutamine at amino acid position 82 with lysine, asparagine at amino acid position 84 with serine, threonine at amino acid position 88 with alanine, glutamic acid at amino acid position 89 with alanine, threonine at amino acid position 93 with valine, alanine at amino acid position 117 with threonine, and serine at amino acid position 118 with leucine was designated as "humanized h7B4_H2 type heavy chain" (also referred to as "h7B4_H2").

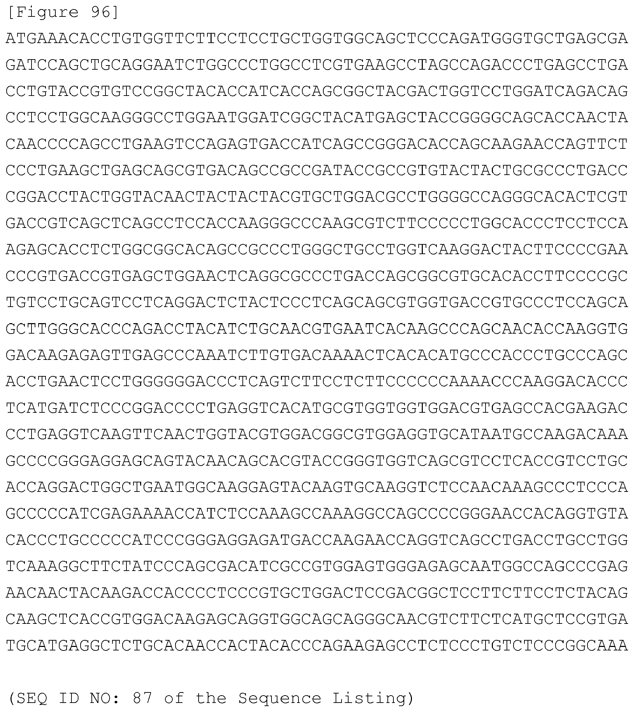

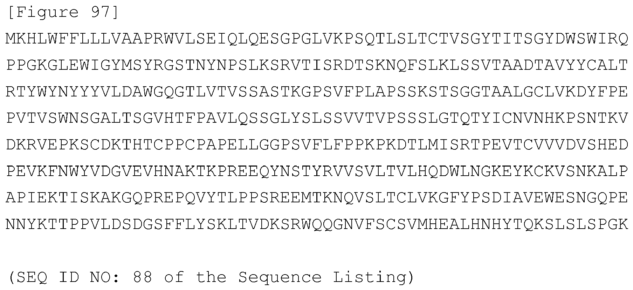

The amino acid sequence of the humanized h7B4_H2 type heavy chain is described in SEQ ID NO: 88 (Figure 97) of the Sequence Listing. A nucleotide sequence encoding the amino acid sequence of SEQ ID NO: 88 is described in SEQ ID NO: 87 (Figure 96) of the Sequence Listing.

7)-2-3-3 Humanized h7B4_H3 type heavy chain

A humanized h7B4 heavy chain designed from the chimeric c7B4 heavy chain shown in SEQ ID NO: 42 by the replacement in the variable region of histidine at amino acid position 3 with glutamine, serine at amino acid position 17 with threonine, serine at amino acid position 23 with threonine, threonine at amino acid position 25 with serine, lysine at amino acid position 40 with glutamine, phenylalanine at amino acid position 41 with proline, asparagine at amino acid position 44 with lysine, lysine at amino acid position 45 with glycine, methionine at amino acid position 46 with leucine, methionine at amino acid position 49 with isoleucine, isoleucine at amino acid position 68 with valine, serine at amino acid position 69 with threonine, threonine at amino acid position 71 with serine, phenylalanine at amino acid position 80 with serine, glutamine at amino acid position 82 with lysine, asparagine at amino acid position 84 with serine, threonine at amino acid position 88 with alanine, glutamic acid at amino acid position 89 with alanine, threonine at amino acid position 93 with valine, alanine at amino acid position 117 with threonine, and serine at amino acid position 118 with leucine was designated as "humanized h7B4_H3 type heavy chain" (also referred to as "h7B4_H3").

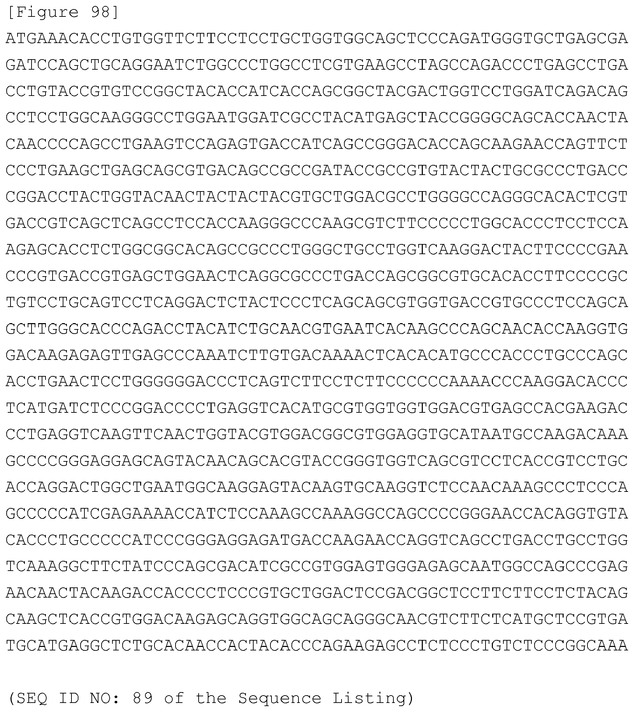

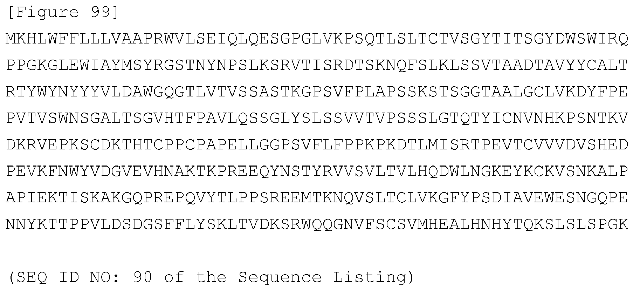

The amino acid sequence of the humanized h7B4_H3 type heavy chain is described in SEQ ID NO: 90 (Figure 99) of the Sequence Listing. A nucleotide sequence encoding the amino acid sequence of SEQ ID NO: 90 is described in SEQ ID NO: 89 (Figure 98) of the Sequence Listing.

7)-2-3-4 Humanized h7B4_H5 type heavy chain

A humanized h7B4 heavy chain designed from the chimeric c7B4 heavy chain shown in SEQ ID NO: 42 by the replacement in the variable region of histidine at amino acid position 3 with glutamine, serine at amino acid position 17 with threonine, serine at amino acid position 23 with threonine, threonine at amino acid position 25 with serine, phenylalanine at amino acid position 41 with proline, methionine at amino acid position 49 with isoleucine, isoleucine at amino acid position 68 with valine, serine at amino acid position 69 with threonine, threonine at amino acid position 71 with serine, phenylalanine at amino acid position 80 with serine, glutamine at amino acid position 82 with lysine, asparagine at amino acid position 84 with serine, threonine at amino acid position 88 with alanine, glutamic acid at amino acid position 89 with alanine, threonine at amino acid position 93 with valine, alanine at amino acid position 117 with threonine, and serine at amino acid position 118 with leucine was designated as "humanized h7B4_H5 type heavy chain" (also referred to as "h7B4_H5").

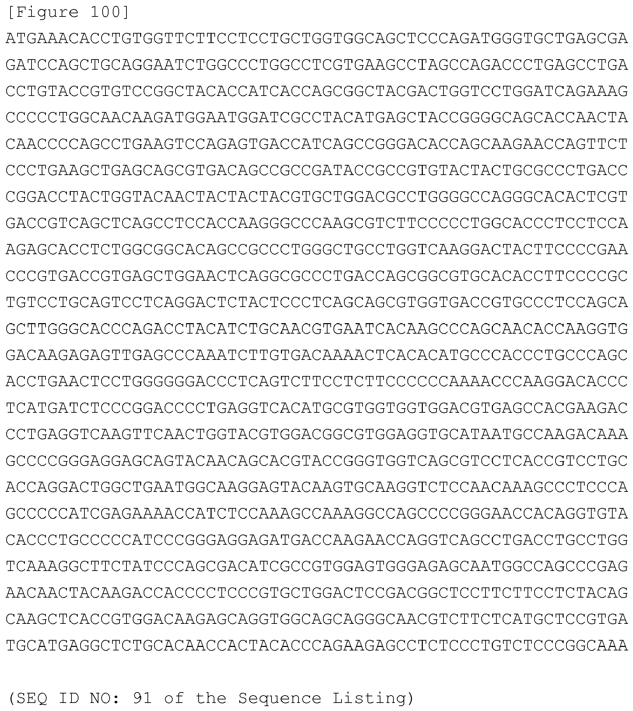

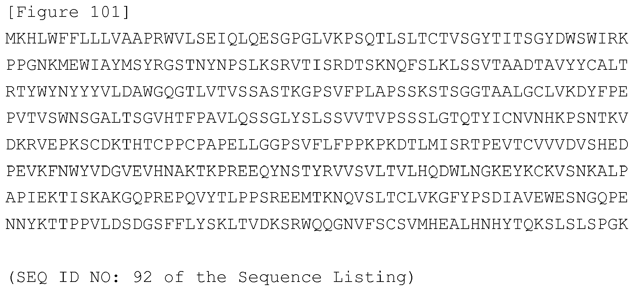

The amino acid sequence of the humanized h7B4_H5 type heavy chain is described in SEQ ID NO: 92 (Figure 101) of the Sequence Listing. A nucleotide sequence encoding the amino acid sequence of SEQ ID NO: 92 is described in SEQ ID NO: 91 (Figure 100) of the Sequence Listing.

7)-2-4 Humanization of 7B4 light chain

7)-2-4-1 Humanized h7B4_L1 type light chain

A humanized h7B4 light chain designed from the chimeric c7B4 light chain shown in SEQ ID NO: 38 by the replacement in the variable region of aspartic acid at amino acid position 1 with glutamic acid, glutamine at amino acid position 3 with valine, methionine at amino acid position 4 with leucine, serine at amino acid position 9 with glycine, phenylalanine at amino acid position 10 with threonine, alanine at amino acid position 13 with leucine, valine at amino acid position 15 with proline, valine at amino acid position 19 with alanine, leucine at amino acid position 40 with proline, glutamic acid at amino acid position 42 with glutamine, lysine at amino acid position 45 with arginine, serine at amino acid position 60 with aspartic acid, glycine at amino acid position 77 with arginine, glutamine at amino acid position 79 with glutamic acid, valine at amino acid position 83 with phenylalanine, threonine at amino acid position 85 with valine, phenylalanine at amino acid position 87 with tyrosine, alanine at amino acid position 99 with glutamine, leucine at amino acid position 103 with valine, and leucine at amino acid position 105 with isoleucine was designated as "humanized h7B4_L1 type light chain" (also referred to as "h7B4_L1").

The amino acid sequence of the humanized h7B4_L1 type light chain is described in SEQ ID NO: 82 (Figure 91) of the Sequence Listing. A nucleotide sequence encoding the amino acid sequence of SEQ ID NO: 82 is described in SEQ ID NO: 81 (Figure 90) of the Sequence Listing.

7)-2-4-2 Humanized h7B4_L2 type light chain

A humanized h7B4 light chain designed from the chimeric c7B4 light chain shown in SEQ ID NO: 38 by the replacement in the variable region of phenylalanine at amino acid position 10 with serine, alanine at amino acid position 13 with leucine, valine at amino acid position 15 with proline, valine at amino acid position 19 with alanine, leucine at amino acid position 40 with proline, glutamic acid at amino acid position 42 with glutamine, lysine at amino acid position 45 with arginine, serine at amino acid position 60 with aspartic acid, glycine at amino acid position 77 with arginine, glutamine at amino acid position 79 with glutamic acid, valine at amino acid position 83 with phenylalanine, threonine at amino acid position 85 with valine, phenylalanine at amino acid position 87 with tyrosine, alanine at amino acid position 99 with glutamine, leucine at amino acid position 103 with valine, and leucine at amino acid position 105 with isoleucine was designated as "humanized h7B4_L2 type light chain" (also referred to as "h7B4_L2").

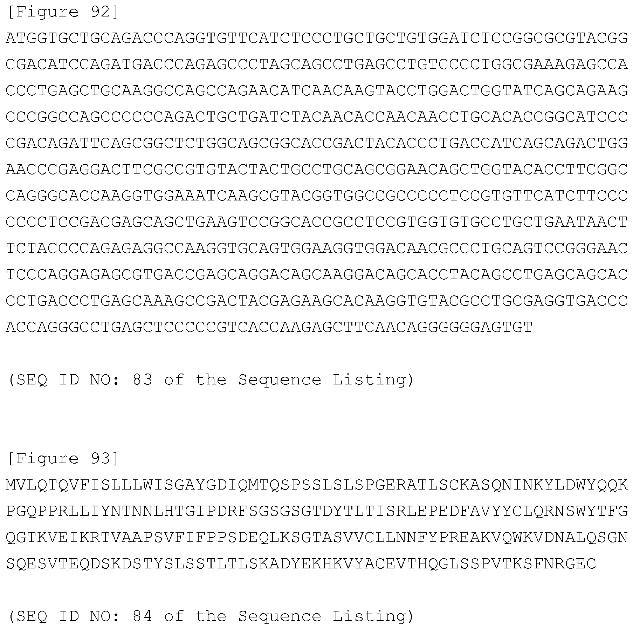

The amino acid sequence of the humanized h7B4_L2 type light chain is described in SEQ ID NO: 84 (Figure 93) of the Sequence Listing. A nucleotide sequence encoding the amino acid sequence of SEQ ID NO: 84 is described in SEQ ID NO: 83 (Figure 92) of the Sequence Listing.

(Example 8) Construction of expression vectors for humanized antibodies (h2B1 and h7B4) of rat anti-human GPRC5D antibodies (2B1 and 7B4) and preparation of antibodies

8)-1 Construction of h2B1 heavy chain expression vector

8)-1-1 Construction of h2B1_H1 type heavy chain

A DNA fragment comprising the h2B1_H1 variable region-encoding DNA sequence represented by nucleotide positions 58 to 426 of the h2B1_H1 nucleotide sequence represented by SEQ ID NO: 73 was synthesized (GeneArt Artificial Gene Synthesis Service). A h2B1_H1 expression vector was constructed in the same way as in Example 8)-1-1. The obtained expression vector was designated as "pCMA/h2B1_H1".

8)-1-2 Construction of h2B1_H2 type heavy chain

A DNA fragment comprising the h2B1_H2 variable region-encoding DNA sequence represented by nucleotide positions 58 to 426 of the h2B1_H2 nucleotide sequence represented by SEQ ID NO: 75 was synthesized (GeneArt Artificial Gene Synthesis Service). The synthesized DNA fragment was amplified by PCR and inserted to the restriction enzyme BlpI-cleaved site of the chimeric and humanized antibody heavy chain expression vector pCMA-G1 using In-Fusion HD PCR cloning kit (Clontech Laboratories, Inc.) to construct a h2B1_H2 expression vector. The obtained expression vector was designated as "pCMA/h2B1_H2".

8)-1-3 Construction of h2B1_H3 type heavy chain

A DNA fragment comprising the h2B1_H3 variable region-encoding DNA sequence represented by nucleotide positions 58 to 426 of the h2B1_H3 nucleotide sequence represented by SEQ ID NO: 77 was synthesized (GeneArt Artificial Gene Synthesis Service). A h2B1_H3 expression vector was constructed in the same way as in Example 8)-1-1. The obtained expression vector was designated as "pCMA/h2B1_H3".

8)-1-4 Construction of h2B1_H4 type heavy chain

A DNA fragment comprising the h2B1_H4 variable region-encoding DNA sequence represented by nucleotide positions 58 to 426 of the h2B1_H4 nucleotide sequence represented by SEQ ID NO: 79 was synthesized (GeneArt Artificial Gene Synthesis Service). A h2B1_H4 expression vector was constructed in the same way as in Example 8)-1-1. The obtained expression vector was designated as "pCMA/h2B1_H4".

8)-2 Construction of h2B1 light chain expression vector

8)-2-1 Construction of h2B1_L1 type light chain

A DNA fragment comprising the h2B1_L1 variable region-encoding DNA sequence represented by nucleotide positions 61 to 381 of the h2B1_L1 nucleotide sequence represented by SEQ ID NO: 63 was synthesized (GeneArt Gene Synthesis Service). The synthesized DNA fragment was amplified by PCR and inserted to the restriction enzyme BsiWI-cleaved site of the chimeric and humanized antibody light chain expression vector pCMA-LK using In-Fusion HD PCR cloning kit (Clontech Laboratories, Inc.) to construct a h2B1_L1 expression vector. The obtained expression vector was designated as "pCMA/h2B1_L1".

8)-2-2 Construction of h2B1_L2 type light chain

A DNA fragment comprising the h2B1_L2 variable region-encoding DNA sequence represented by nucleotide positions 61 to 381 of the h2B1_L2 nucleotide sequence represented by SEQ ID NO: 65 was synthesized (GeneArt Gene Synthesis Service). A h2B1_L2 expression vector was constructed in the same way as in Example 8)-2-1. The obtained expression vector was designated as "pCMA/h2B1_L2".

8)-2-3 Construction of h2B1_L3 type light chain

A DNA fragment comprising the h2B1_L3 variable region-encoding DNA sequence represented by nucleotide positions 61 to 381 of the h2B1_L3 nucleotide sequence represented by SEQ ID NO: 67 was synthesized (GeneArt Gene Synthesis Service). A h2B1_L3 expression vector was constructed in the same way as in Example 8)-2-1. The obtained expression vector was designated as "pCMA/h2B1_L3".

8)-2-4 Construction of h2B1_L4 type light chain

A DNA fragment comprising the h2B1_L4 variable region-encoding DNA sequence represented by nucleotide positions 61 to 381 of the h2B1_L4 nucleotide sequence represented by SEQ ID NO: 69 was synthesized (GeneArt Gene Synthesis Service). A h2B1_L4 expression vector was constructed in the same way as in Example 8)-2-1. The obtained expression vector was designated as "pCMA/h2B1_L4".

8)-2-5 Construction of h2B1_L5 type light chain

A DNA fragment comprising the h2B1_L5 variable region-encoding DNA sequence represented by nucleotide positions 61 to 381 of the h2B1_L5 nucleotide sequence represented by SEQ ID NO: 71 was synthesized (GeneArt Gene Synthesis Service). A h2B1_L5 expression vector was constructed in the same way as in Example 8)-2-1. The obtained expression vector was designated as "pCMA/h2B1_L5".

8)-3 Construction of h7B4 heavy chain expression vector

8)-3-1 Construction of h7B4_H1 type heavy chain

A DNA fragment comprising the h7B4_H1 variable region-encoding DNA sequence represented by nucleotide positions 58 to 426 of the h7B4_H1 nucleotide sequence represented by SEQ ID NO: 85 was synthesized (GeneArt Artificial Gene Synthesis Service). A h7B4_H1 expression vector was constructed in the same way as in Example 8)-1-1. The obtained expression vector was designated as "pCMA/h7B4_H1".

8)-3-2 Construction of h7B4_H2 type heavy chain

A DNA fragment comprising the h7B4_H2 variable region-encoding DNA sequence represented by nucleotide positions 58 to 426 of the h7B4_H2 nucleotide sequence represented by SEQ ID NO: 87 was synthesized (GeneArt Artificial Gene Synthesis Service). A h7B4_H2 expression vector was constructed in the same way as in Example 8)-1-1. The obtained expression vector was designated as "pCMA/h7B4_H2".

8)-3-3 Construction of h7B4_H3 type heavy chain

A DNA fragment comprising the h7B4_H3 variable region-encoding DNA sequence represented by nucleotide positions 58 to 426 of the h7B4_H3 nucleotide sequence represented by SEQ ID NO: 89 was synthesized (GeneArt Artificial Gene Synthesis Service). A h7B4_H3 expression vector was constructed in the same way as in Example 8)-1-1. The obtained expression vector was designated as "pCMA/h7B4_H3".

8)-3-4 Construction of h7B4_H5 type heavy chain

A DNA fragment comprising the h7B4_H5 variable region-encoding DNA sequence represented by nucleotide positions 58 to 426 of the h7B4_H5 nucleotide sequence represented by SEQ ID NO: 91 was synthesized (GeneArt Artificial Gene Synthesis Service). A h7B4_H5 expression vector was constructed in the same way as in Example 8)-1-1. The obtained expression vector was designated as "pCMA/h7B4_H5".

8)-4 Construction of h7B4 light chain expression vector

8)-4-1 Construction of h7B4_L1 type light chain

A DNA fragment comprising the h7B4_L1 variable region-encoding DNA sequence represented by nucleotide positions 61 to 378 of the h7B4_L1 nucleotide sequence represented by SEQ ID NO: 90 was synthesized (GeneArt Gene Synthesis Service). A h7B4_L1 expression vector was constructed in the same way as in Example 8)-2-1. The obtained expression vector was designated as "pCMA/h7B4_L1".

8)-4-2 Construction of h7B4_L2 type light chain

A DNA fragment comprising the h7B4_L2 variable region-encoding DNA sequence represented by nucleotide positions 61 to 378 of the h7B4_L2 nucleotide sequence represented by SEQ ID NO: 92 was synthesized (GeneArt Gene Synthesis Service). A h7B4_L2 expression vector was constructed in the same way as in Example 8)-2-1. The obtained expression vector was designated as "pCMA/h7B4_L2".

8)-5 Preparation of humanized antibodies (h2B1 and h7B4) (FreeStyle 293F cells)

8)-5-1 Small-scale production of humanized antibodies (h2B1 and h7B4)

FreeStyle 293F cells (Invitrogen Corp.) were subcultured and cultured according to the manual.

$1 \times 10^7$ FreeStyle 293F cells (Invitrogen Corp.) in the logarithmic growth phase were diluted to 9.6 mL with FreeStyle 293 expression medium (Invitrogen Corp.), then inoculated to 30 mL Square Storage Bottle (Nalgene/Thermo Fisher Scientific Inc.), and shake-cultured at 90 rpm at 37°C for 1 hour in an 8% $CO_2$ incubator. 30 $\mu$g of polyethyleneimine (Polysciences #24765) was dissolved in 200 $\mu$L of Opti-Pro SFM (Invitrogen Corp.). Next, each heavy chain expression vector (4 $\mu$g) and light chain expression vector (6 $\mu$g) prepared using PureLink HiPure Plasmid kit (Invitrogen Corp.) were added to 200 $\mu$L of Opti-Pro SFM (Invitrogen Corp.). 200 $\mu$L of the expression vector/Opti-Pro SFM mixed solution was added to 200 $\mu$L of the polyethyleneimine/Opti-Pro SFM mixed solution, and the mixture was gently stirred, further left standing for 5 minutes, and then added to the FreeStyle 293F cells. The cells were shake-cultured at 90 rpm at 37°C for 7 days in an 8% $CO_2$ incubator, and the obtained culture supernatant was filtered through Minisart-Plus filter (Sartorius Japan K.K.) and used as a sample for evaluation.

h2B1_H1/L1 was obtained by the combination of pCMA/h2B1_H1 and pCMA/h2B1_L1. h2B1_H1/L2 was obtained by the combination of pCMA/h2B1_H1 and pCMA/h2B1_L2. h2B1_H2/L2 was obtained by the combination of pCMA/h2B1_H2 and pCMA/h2B1_L2. h2B1_H2/L3 was obtained by the combination of pCMA/h2B1_H2 and pCMA/h2B1_L3. h2B1_H2/L4 was obtained by the combination of pCMA/h2B1_H2 and pCMA/h2B1_L4. h2B1_H2/L5 was obtained by the combination of pCMA/h2B1_H2 and pCMA/h2B1_L5. h2B1_H3/L3 was obtained by the combination of pCMA/h2B1_H3 and pCMA/h2B1_L3. h2B1_H3/L4 was obtained by the combination of pCMA/h2B1_H3 and pCMA/h2B1_L4. h2B1_H3/L5 was obtained by the combination of pCMA/h2B1_H3 and pCMA/h2B1_L5. h2B1_H4/L1 was obtained by the combination of pCMA/h2B1_H4 and pCMA/h2B1_L1. h2B1_H4/L3 was obtained by the combination of pCMA/h2B1_H4 and pCMA/h2B1_L3. h2B1_H4/L4 was obtained by the combination of pCMA/h2B1_H4 and pCMA/h2B1_L4. h2B1_H4/L5 was obtained by the combination of pCMA/h2B1_H4 and pCMA/h2B1_L5. h7B4_H1/L2 was obtained by the combination of pCMA/h7B4_H1 and pCMA/h7B4_L2. h7B4_H2/L2 was obtained by the combination of pCMA/h7B4_H2 and pCMA/h7B4_L2. h7B4_H3/L1 was obtained by the combination of pCMA/h7B4_H3 and pCMA/h7B4_L1. h7B4_H3/L2 was obtained by the combination of pCMA/h7B4_H3 and pCMA/h7B4_L2. h7B4_H5/L1 was obtained by the combination of pCMA/h7B4_H5 and pCMA/h7B4_L1.

8)-5-2 Production of humanized antibodies (h2B1 and h7B4)

Humanized antibodies were produced in the same way as in Example 4)-9-1. Specifically, h2B1_H1/L1 was obtained by the combination of pCMA/h2B1_H1 and pCMA/h2B1_L1. h2B1_H2/L5 was obtained by the combination of pCMA/h2B1_H2 and pCMA/h2B1_L5. h2B1_H4/L5 was obtained by the combination of pCMA/h2B1_H4 and pCMA/h2B1_L5. h7B4_H1/L2 was obtained by the combination of pCMA/h7B4_H1 and pCMA/h7B4_L2. h7B4_H3/L1 was obtained by the combination of pCMA/h7B4_H3 and pCMA/h7B4_L1.

8)-5-3 Purification of humanized antibodies (h2B1 and h7B4)

Each antibody was purified from the culture supernatant obtained in Example 8)-5-2 by two steps using rProtein A affinity chromatography (at 4 to 6°C) and ceramic hydroxyapatite (at room temperature). Buffer replacement steps after the rProtein A affinity chromatography purification and after the ceramic hydroxyapatite purification were carried out at 4 to 6°C. The culture supernatant was applied to MabSelect SuRe (manufactured by GE Healthcare Bio-Sciences Corp., HiTrap column) equilibrated with PBS. After entry of the whole culture supernatant in the column, the column was washed with PBS in an amount at least twice the column volume. Next, antibody-containing fractions were collected by elution with a 2 M arginine hydrochloride solution (pH 4.0). The fractions were buffer-replaced with PBS by dialysis (Thermo Fisher Scientific Inc., Slide-A-Lyzer Dialysis Cassette) and then diluted 5-fold with a buffer of 5 mM sodium phosphate and 50 mM MES (pH 7.0). The resulting antibody solution was applied to a ceramic hydroxyapatite column (Bio-Rad Laboratories, Inc., Bio-Scale CHT Type-1 Hydroxyapatite Column) equilibrated with a buffer of 5 mM NaPi, 50 mM MES, and 30 mM NaCl (pH 7.0). Antibody-containing fractions were collected by linear concentration gradient elution using sodium chloride. The fractions were buffer-replaced with HBSor (25 mM histidine and 5% sorbitol, pH 6.0) by dialysis (Thermo Fisher Scientific Inc., Slide-A-Lyzer Dialysis Cassette). The fractions were concentrated and adjusted to an IgG concentration of 10 mg/ml or higher using Centrifugal UF Filter Device VIVASPIN 20 (molecular weight cutoff: UF10K, Sartorius Japan K.K., at 4°C). Finally, the antibody solution was filtered through Minisart-Plus filter (Sartorius Japan K.K.) and used as a purified sample.

(Example 9) *In vitro* activity evaluation of humanized anti-GPRC5D antibody

9)-1 Evaluation of binding activity of humanized anti-GPRC5D antibodies (h2B1_H1/L1 to h2B1_H4/L5 and h7B4_H1/L2 to h7B4_H5/L1) against human GPRC5D by flow cytometry

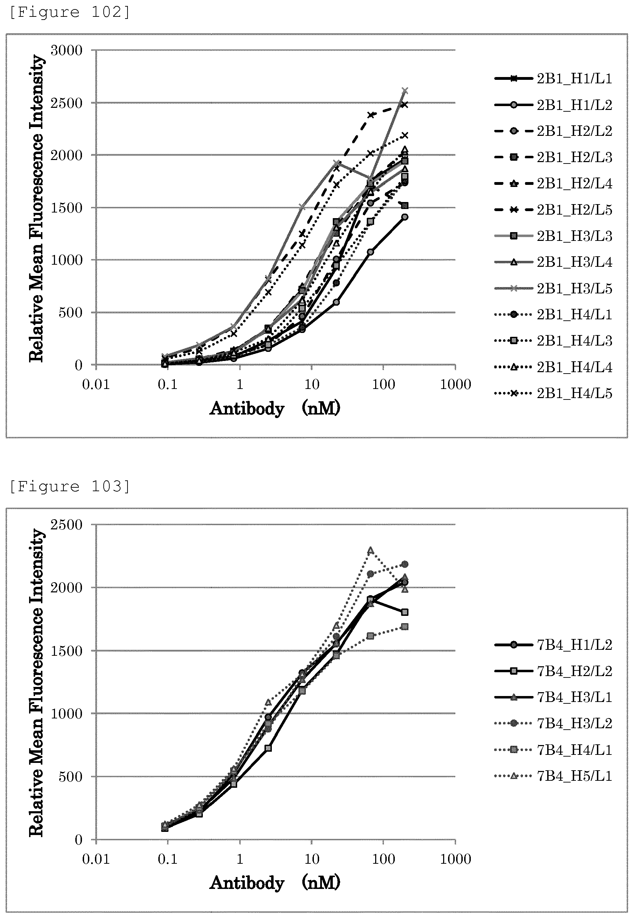

Human multiple myeloma cell line KHM-1B cells expressing GPRC5D were adjusted to a concentration of $2 \times 10^6$ cells/mL with PBS containing 5% FBS, inoculated in an amount of 100 μL/well to a 96-well U-bottomed microplate, and centrifuged to remove a supernatant. The culture supernatant of each humanized anti-GPRC5D antibody obtained in Example 8)-3-1 or Human IgG isotype control antibody (Calbiochem/Merck Millipore Corp.) adjusted to 14 ng/mL to 30 μg/mL was added thereto in an amount of 100 μL/well, and the plate was left standing at 4°C for 1 hour. The cells were washed twice with PBS containing 5% FBS. Then, R-Phycoerythrin AffiniPure F(ab')2 Fragment Goat Anti-Human IgG, Fcγ Fragment Specific (Jackson ImmunoResearch Laboratories, Inc.) diluted 100-fold with PBS containing 5% FBS was added thereto in an amount of 100 μL/well, and the plate was left standing at 4°C for 1 hour. The cells were washed twice with PBS containing 5% FBS and then resuspended in PBS containing 5% FBS, followed by detection using a flow cytometer (FACSCanto(TM) II; Becton, Dickinson and Company). The data was analyzed using Flowjo (Tree Star, Inc.). The PE fluorescence intensity of the cell fraction was plotted to a histogram, and the mean fluorescence intensity (MFI) was calculated. The MFI value of the control antibody was subtracted from the MFI value of the GPRC5D antibody to calculate a relative value of MFI (rMFI). Figure 102 shows the results about the humanized anti-GPRC5D antibodies h2B1_H1/L1 to h2B1_H4/L5, and Figure 103 shows the results about the humanized anti-GPRC5D antibodies h7B4_H1/L2 to h7B4_H5/L1. As shown in Figures 102 and 103, these humanized anti-GPRC5D antibodies were found to bind to human GPRC5D.

9)-2 Evaluation of binding property of humanized anti-GPRC5D antibodies (h2B1_H1/L1 to h2B1_H4/L5 and h7B4_H1/L2 to h7B4_H5/L1) to cynomolgus monkey GPRC5D by flow cytometry

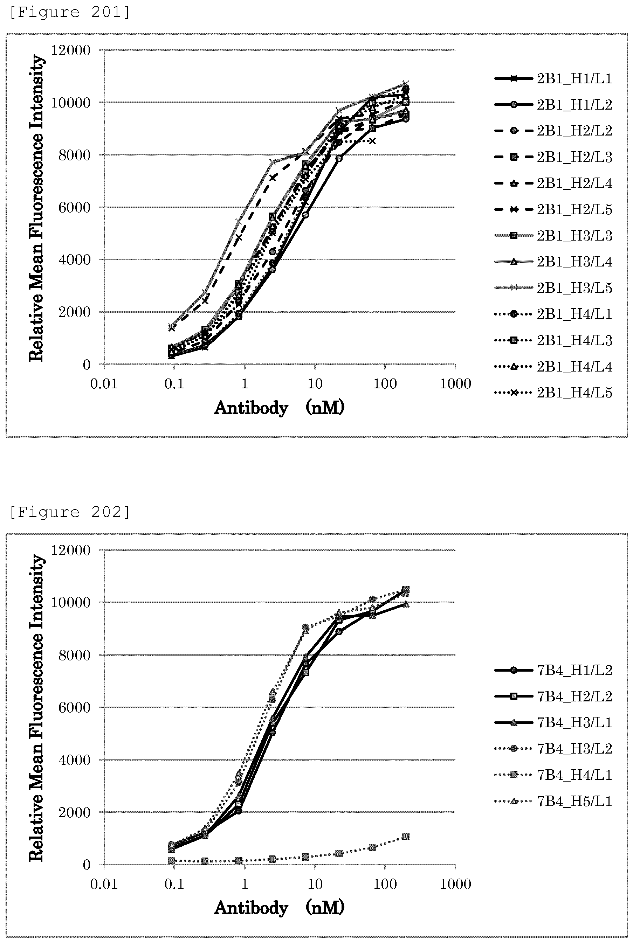

Staining and analysis were carried out in the same way as in Example 9)-1 using the KMS-11_cGPRC5D cells prepared in Example 5)-2-2. As shown in Figures 201 and 202, these humanized anti-GPRC5D antibodies were found to bind to cynomolgus monkey GPRC5D.

9)-3 ADCC activity evaluation of humanized anti-GPRC5D antibodies (h2B1_H1/L1, h2B1_H2/L5, h2B1_H4/L5, h7B4_H1/L2, and h7B4_H3/L1)

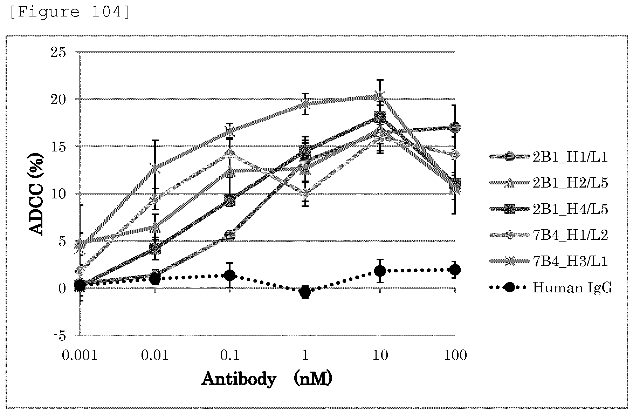

The KHM-1B cells prepared in Example 2)-3-1 were added in an amount of 50 μL/well to a 96-well U-bottomed microplate. Each humanized anti-GPRC5D antibody (h2B1_H1/L1, h2B1_H2/L5, h2B1_H4/L5, h7B4_H1/L2 and h7B4_H3/L1) and human control antibody (hIgG1) (Calbiochem/Merck Millipore Corp.) adjusted to 0.15 ng/mL to 15 μg/mL (final concentration) was added thereto in an amount of 50 μL/well, and the plate was left standing at 4°C for 30 minutes. The effector cells prepared in Example 1)-5-3 were further added thereto in an amount of 100 μL/well. After centrifugation at room temperature at 1200 rpm for 3 minutes, the cells were cultured at 37°C for 4 hours under 5% $CO_2$ conditions. A 50 μL aliquot of the supernatant was recovered into LumaPlate (PerkinElmer, Inc.) and dried overnight at 50°C, followed by measurement using a plate reader (TopCount; PerkinElmer, Inc.). The percentage of cells lysed by ADCC activity was calculated according to Example 1)-5-5. As shown in Figure 104, h2B1_H1/L1, h2B1_H2/L5, h2B1_H4/L5, h7B4_H1/L2, and h7B4_H3/L1 were found to have ADCC activity.

(Example 10) Obtainment of anti-GPRC5D antibody derived from human antibody phage library, and binding activity evaluation

10)-1 Isolation of scFv having GPRC5D binding activity

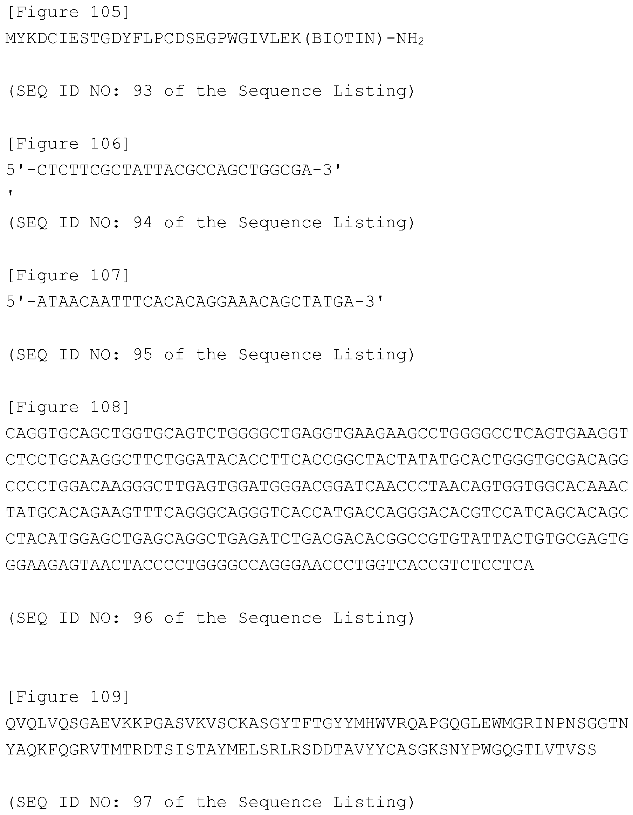

scFv that binds to human GPRC5D and cynomolgus monkey GPRC5D was isolated from a human antibody phage library. Phages were added to Dynabeads Streptavidin M-280 (Thermo Fisher Scientific Inc.) on which the amino-terminal peptide (synthesized by Peptide Institute, Inc.) of human (SEQ ID NO: 2 of the Sequence Listing; Figure 3) or cynomolgus monkey GPRC5D having a biotinylated carboxy terminus was immobilized. Unbound phages were removed by washing operation using a magnet stand (DynaMag-2, Thermo Fisher Scientific Inc.). The amino-terminal peptide of cynomolgus monkey GPRC5D used had the following sequence:
Amino-terminal peptide of cynomolgus monkey GPRC5D: MYKDCIESTGDYFLPCDSEGPWGIVLEK(Biotin)-$NH_2$ (SEQ ID NO: 93 of the Sequence Listing; Figure 105)
Then, the phages bound with the GPRC5D amino-terminal peptide were infected with *E. coli* (XL-1 Blue, Agilent Technologies, Inc.), and the phages bound with the GPRC5D amino-terminal peptide were recovered and amplified. Alternatively, phages were added to Expi293F cells (Thermo Fisher Scientific Inc.) caused to transiently express human or cynomolgus monkey GPRC5D using the GPRC5D expression vector prepared in Example 1)-1-1 or 5)-2-1. Unbound phages were removed by washing operation. Then, the phages bound with the GPRC5D amino-terminal peptide were infected with *E. coli,* and the phages bound with the GPRC5D amino-terminal peptide were recovered and amplified. A total of 3 rounds of panning were carried out for the peptide or the Expi293F cells caused to transiently express human or cynomolgus monkey GPRC5D. After transfer from the polyclonal phagemid to an expression vector for *E. coli* to add FLAG and His tags to the carboxyl terminus of scFv, *E. coli* was transformed with the expression vector, and scFv was expressed in the presence of IPTG (isopropyl-β-D-thiogalactopyranoside) (Sigma-Aldrich Corp.) and subjected to screening by ELISA.

10)-2 Screening for GPRC5D-binding scFv by ELISA

NeutrAvidin (Life Technologies Corp.) diluted to 1 μg/mL with PBS (0.01 M phosphate-buffered saline (pH 7.4) containing 0.138 M sodium chloride and 0.0027 M potassium chloride, Sigma-Aldrich Corp.) was added in an amount of 50 μL/well to 384-well Maxi-sorp plate (Black, Nunc/Thermo Fisher Scientific Inc.), and the plate was left standing overnight at 4°C for immobilization. After washing three times with PBS containing 0.05% Tween-20 (Bio-Rad Laboratories, Inc.) (ELISA buffer), the amino-terminal peptide of the biotinylated human or cynomolgus monkey GPRC5D (also used in Example 10)-1) diluted to 1 μg/mL with PBS was added thereto, and the plate was shaken at room temperature for 1 hour. After washing three times with an ELISA buffer, the plate was blocked with Blocker Casein (Thermo Fisher Scientific Inc.) and washed three times with an ELISA buffer. Then, the culture solution of the scFv-expressing *E. coli* was added to the plate and reacted at room temperature for 2 hours. After washing three times with an ELISA buffer, a horseradish peroxidase (HRP)-labeled anti-FLAG antibody (Sigma-Aldrich Corp.) diluted 5000-fold with an ELISA buffer was added thereto in an amount of 50 μL/well and reacted at room temperature for 1 hour. After washing five times with ELISA buffer, SuperSignal Pico ELISA Chemiluminescent substrate (Thermo Fisher Scientific Inc.) was added. After 10 minutes, the chemiluminescence was measured using a plate reader (Envision 2104 Multilabel Reader, PerkinElmer, Inc.), and GPRC5D-binding scFv was selected.

10)-3 Sequencing of ELISA positive clone

The nucleotide sequences of the heavy and light chain variable regions of ELISA positive clones (C2037, C3048, C3015, and C3022) were analyzed by the dye terminator method (BigDye(R) Terminator v3.1, Thermo Fisher Scientific Inc.). The primer sequences used in the sequence analysis were as follows:

Primer A: 5'-CTCTTCGCTATTACGCCAGCTGGCGA-3' (SEQ ID NO: 94 of the Sequence Listing; Figure 106)
Primer B: 5'-ATAACAATTTCACACAGGAAACAGCTATGA-3' (SEQ ID NO: 95 of the Sequence Listing; Figure 107)

The variable region-encoding nucleotide sequences of the genes of the C2037 antibody, the C3048 antibody, the C3015 antibody, and the C3022 antibody were determined by the analysis.
The determined nucleotide sequence of the cDNA encoding the heavy chain variable region of C2037 is represented by SEQ ID NO: 96 (Figure 108), and the amino acid sequence thereof is represented by SEQ ID NO: 97 (Figure 109).

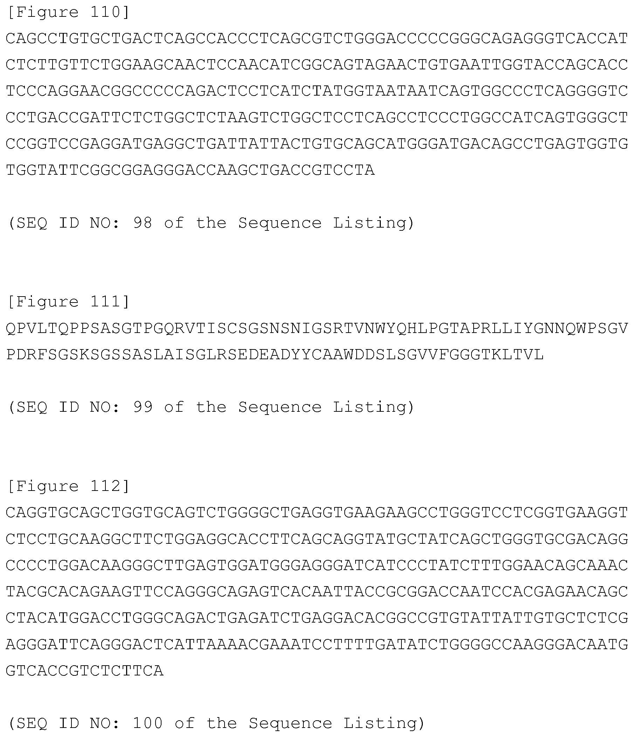

The determined nucleotide sequence of the cDNA encoding the light chain variable region of C2037 is represented by SEQ ID NO: 98 (Figure 110), and the amino acid sequence thereof is represented by SEQ ID NO: 99 (Figure 111).

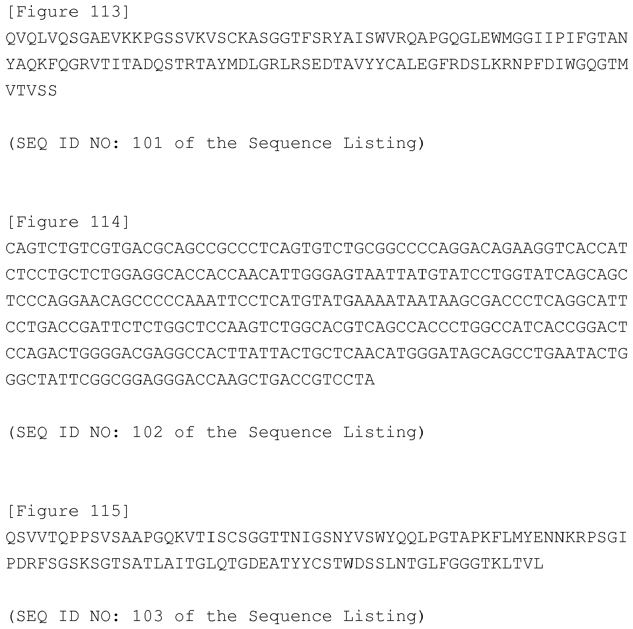

The determined nucleotide sequence of the cDNA encoding the heavy chain variable region of C3048 is represented by SEQ ID NO: 100 (Figure 112), and the amino acid sequence thereof is represented by SEQ ID NO: 101 (Figure 113).

The determined nucleotide sequence of the cDNA encoding the light chain variable region of C3048 is represented by SEQ ID NO: 102 (Figure 114), and the amino acid sequence thereof is represented by SEQ ID NO: 103 (Figure 115).

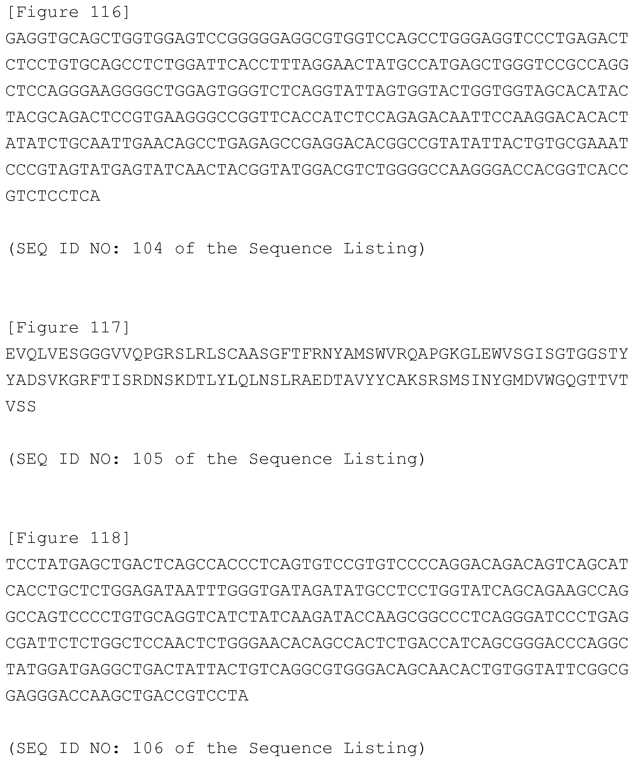

The determined nucleotide sequence of the cDNA encoding the heavy chain variable region of C3015 is represented by SEQ ID NO: 104 (Figure 116), and the amino acid sequence thereof is represented by SEQ ID NO: 105 (Figure 117).

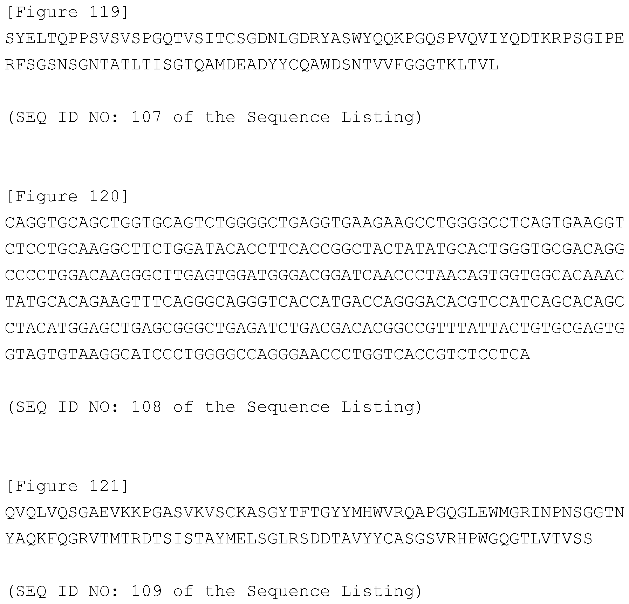

The determined nucleotide sequence of the cDNA encoding the light chain variable region of C3015 is represented by SEQ ID NO: 106 (Figure 118), and the amino acid sequence thereof is represented by SEQ ID NO: 107 (Figure 119).

The determined nucleotide sequence of the cDNA encoding the heavy chain variable region of C3022 is represented by SEQ ID NO: 108 (Figure 120), and the amino acid sequence thereof is represented by SEQ ID NO: 109 (Figure 121).

The determined nucleotide sequence of the cDNA encoding the light chain variable region of C3022 is represented by SEQ ID NO: 110 (Figure 122), and the amino acid sequence thereof is represented by SEQ ID NO: 135 (Figure 123).

10)-4 Expression and purification of scFv

The C2037 antibody, C3048 antibody, C3015 antibody, or C3022 antibody scFv was inserted to an expression vector for animal cells such as pcDNA3.1 (Thermo Fisher Scientific Inc.) to construct an scFv expression vector for animal cells. The scFv expression vector for animal cells was transfected to Expi293F cells (Thermo Fisher Scientific Inc.), for transient expression. If necessary, the scFv was purified from the culture supernatant using a His Trap column (GE Healthcare Bio-Sciences Corp.) and a gel filtration column (Superdex 200 Increase, GE Healthcare Bio-Sciences Corp.). The buffer solution containing the scFv dissolved therein was replaced with PBS, and the resulting solution was subjected to the following step "10)-6".

10)-5 Conversion to full-length IgG and expression and purification of IgG

A full-length IgG form containing C2037, C3048, C3015, or C3022 was prepared by the following method.

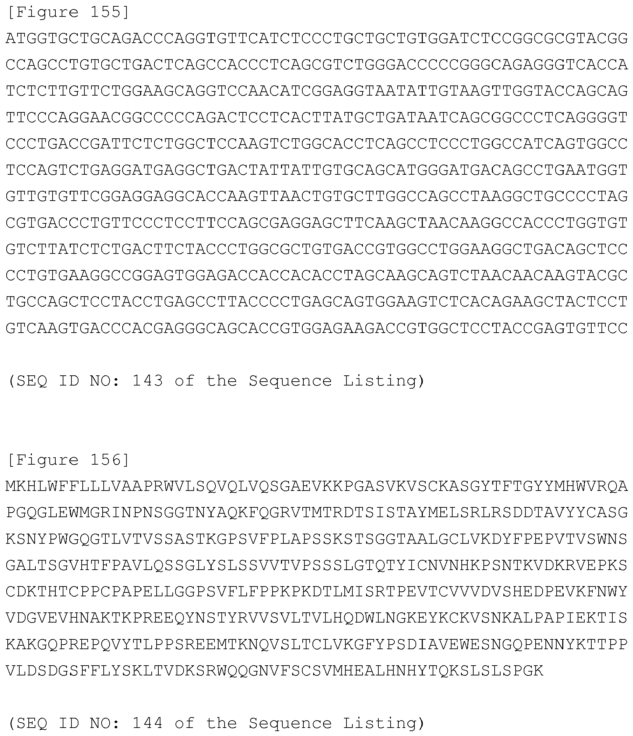

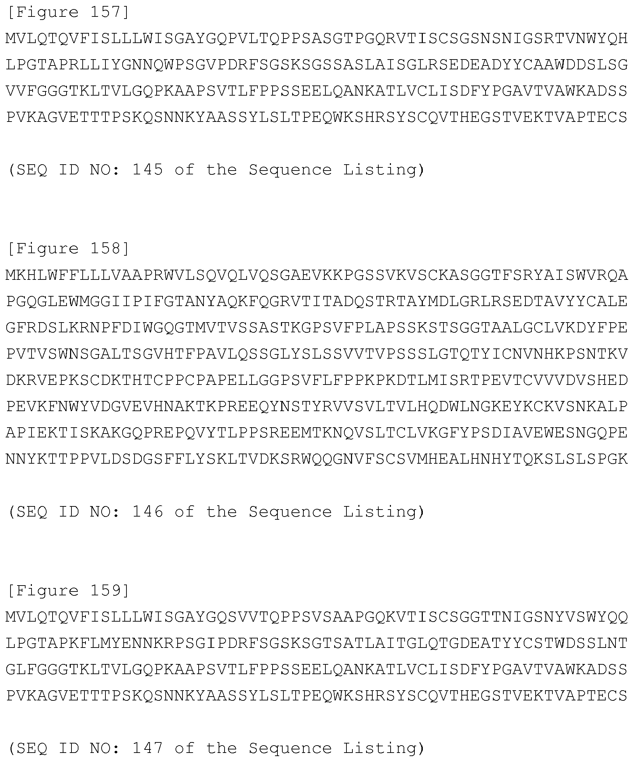

The nucleotide sequences encoding the heavy and light chain variable regions of each antibody identified in Example 10)-3 were linked to a nucleotide sequence encoding a human $IgG_1$ heavy chain constant region (CH1 + Fc region: amino acid sequence positions 135 to 464 of the amino acid sequence represented by SEQ ID NO: 144 (Figure 156) of the Sequence Listing) or a nucleotide sequence encoding a human $IgG_1$ light chain constant region (CL: amino acid sequence positions 131 to 236 of the amino acid sequence represented by SEQ ID NO: 145 (Figure 157) of the Sequence Listing), respectively, by a routine method. The constructs were inserted to an expression vector for animal cells such as pcDNA3.1 (Thermo Fisher Scientific Inc.) to construct an IgG expression vector for animal cells.

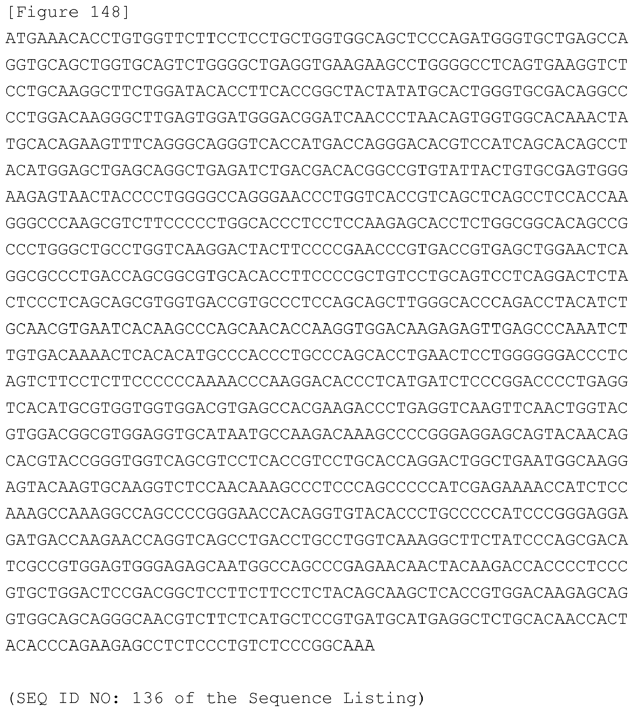

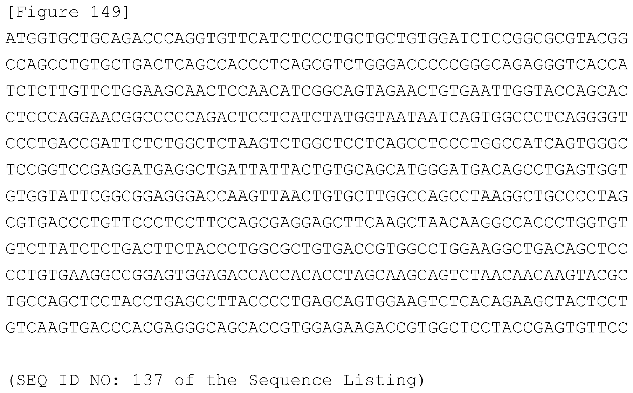

The nucleotide sequence of the constructed IgG expression vector was re-analyzed. It was confirmed that the nucleotide sequence of the full-length heavy chain of the C2037 antibody was the nucleotide sequence represented by SEQ ID NO: 136 (Figure 148) of the Sequence Listing, and the nucleotide sequence of the full-length light chain was the nucleotide sequence represented by SEQ ID NO: 137 (Figure 149) of the Sequence Listing.

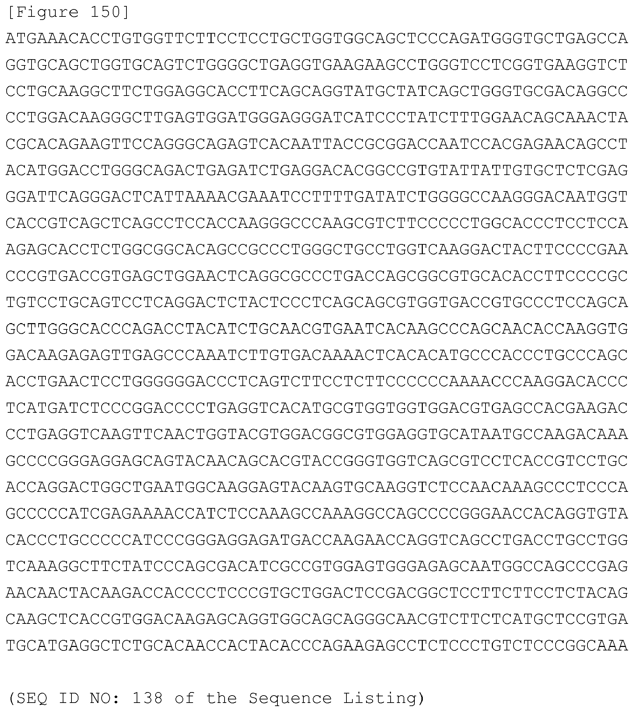

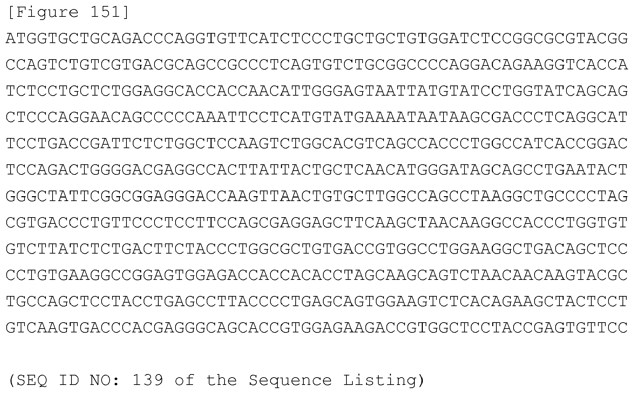

It was confirmed that the nucleotide sequence of the full-length heavy chain of the C3048 antibody was the nucleotide sequence represented by SEQ ID NO: 138 (Figure 150) of the Sequence Listing, and the nucleotide sequence of the full-length light chain was the nucleotide sequence represented by SEQ ID NO: 139 (Figure 151) of the Sequence Listing.

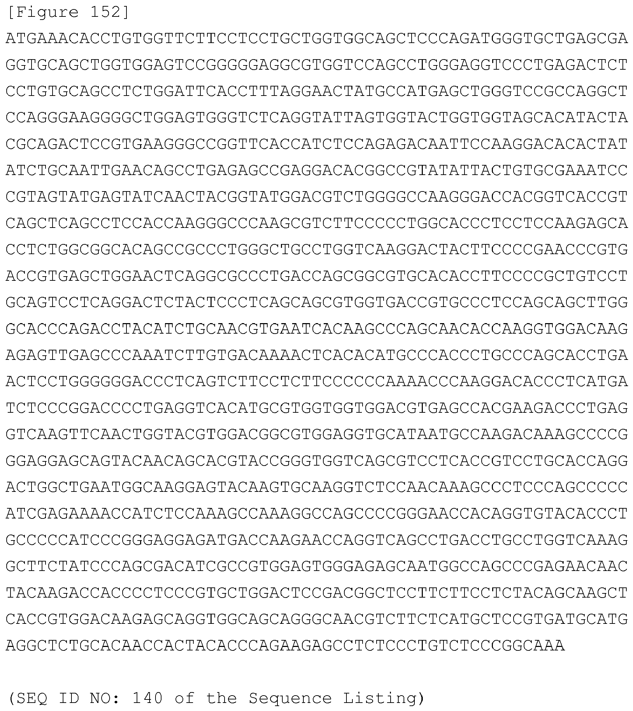

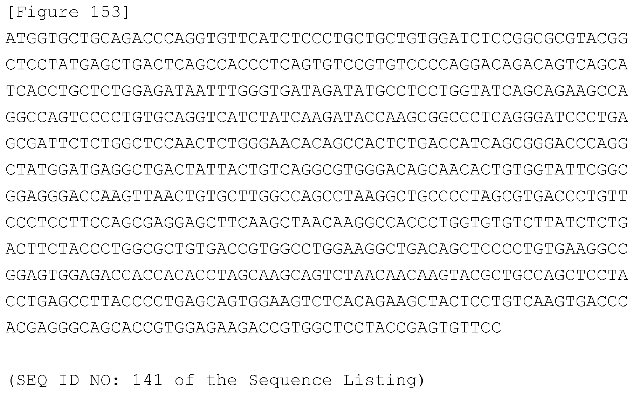

It was confirmed that the nucleotide sequence of the full-length heavy chain of the C3015 antibody was the nucleotide sequence represented by SEQ ID NO: 140 (Figure 152) of the Sequence Listing, and the nucleotide sequence of the full-length light chain was the nucleotide sequence represented by SEQ ID NO: 141 (Figure 153) of the Sequence Listing.

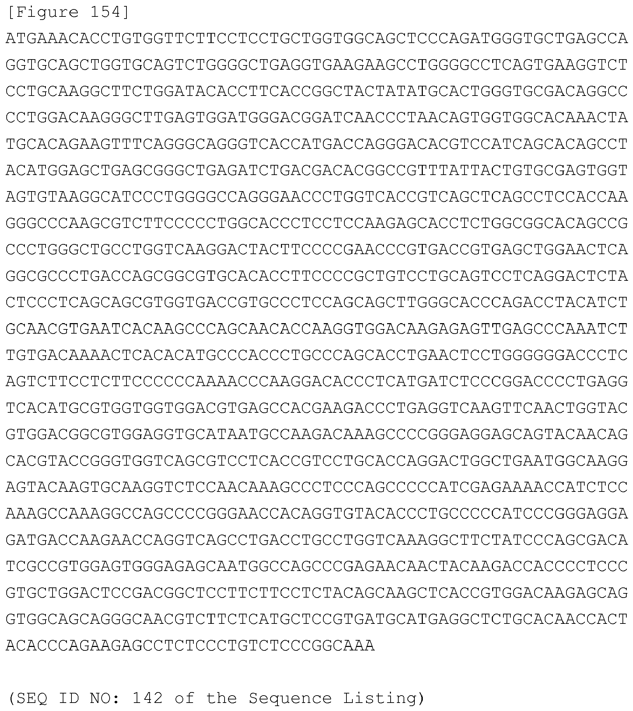

It was confirmed that the nucleotide sequence of the full-length heavy chain of the C3022 antibody was the nucleotide sequence represented by SEQ ID NO: 142 (Figure 154) of the Sequence Listing, and the nucleotide sequence of the full-length light chain was the nucleotide sequence represented by SEQ ID NO: 143 (Figure 155) of the Sequence Listing.

The amino acid sequences of the full-length heavy and light chains of the C2037, C3048, C3015, and C3022 antibodies encoded by these sequences were determined from the nucleotide sequences.

The amino acid sequence of the heavy chain of the C2037 antibody was the amino acid sequence represented by SEQ ID NO: 144 (Figure 156) of the Sequence Listing, and the amino acid sequence of the light chain was the amino acid sequence represented by SEQ ID NO: 145 (Figure 157) of the Sequence Listing.

The amino acid sequence of the heavy chain of the C3048 antibody was the amino acid sequence represented by SEQ ID NO: 146 (Figure 158) of the Sequence Listing, and the amino acid sequence of the light chain was the amino acid sequence represented by SEQ ID NO: 147 (Figure 159) of the Sequence Listing.

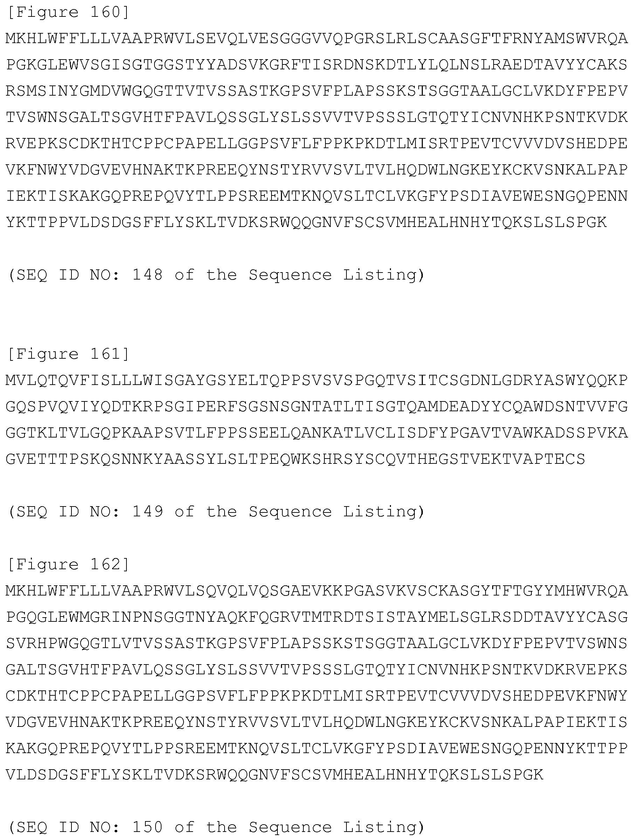

The amino acid sequence of the heavy chain of the C3015 antibody was the amino acid sequence represented by SEQ ID NO: 148 (Figure 160) of the Sequence Listing, and the amino acid sequence of the light chain was the amino acid sequence represented by SEQ ID NO: 149 (Figure 161) of the Sequence Listing.

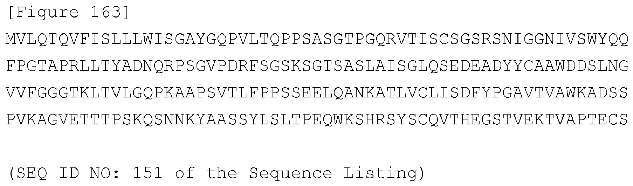

The amino acid sequence of the heavy chain of the C3022 antibody was the amino acid sequence represented by SEQ ID NO: 150 (Figure 162) of the Sequence Listing, and the amino acid sequence of the light chain was the amino acid sequence represented by SEQ ID NO: 151 (Figure 163) of the Sequence Listing.

The IgG form of the C2037, C3048, C3015, or C3022 antibody was transiently expressed by the transfection of FreeStyle 293F cells (Thermo Fisher Scientific Inc.) with the IgG expression vector for animal cells. If necessary, the IgG form was purified using a protein A affinity column (HiTrap Mab Select SuRe, GE Healthcare Bio-Sciences Corp.). Then, the buffer solution containing the IgG dissolved therein was replaced with PBS using Vivaspin 20 (7k MWCO, GE Healthcare Bio-Sciences Corp.), and the resulting solution was subjected to the following steps 10)-7-7 and 10)-9.

10)-6 Confirmation of binding of scFv to GPRC5D by ELISA

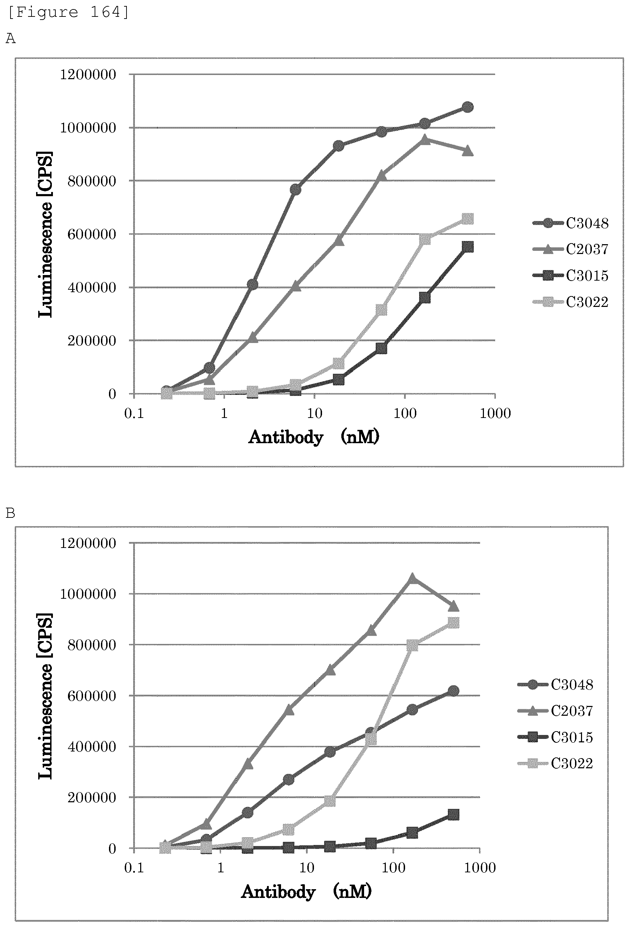

NeutrAvidin diluted to 1 μg/mL with PBS was added in an amount of 50 μL/well to 96-well Maxi-sorp plate (Black, Nunc/Thermo Fisher Scientific Inc.), and the plate was left standing overnight at 4°C for immobilization. After washing three times with an ELISA buffer, the amino-terminal peptide of the biotinylated human or cynomolgus monkey GPRC5D (used in Example 10)-1) diluted to 1 μg/mL with PBS was added thereto, and the plate was shaken at room temperature for 1 hour. After washing three times with an ELISA buffer, the plate was blocked with Blocker Casein and washed three times with an ELISA buffer. Then, the C2037, C3048, C3015, or C3022 scFv was added to the plate and reacted at room temperature for 2 hours. After washing three times with an ELISA buffer, a horseradish peroxidase (HRP)-labeled anti-FLAG antibody diluted 5000-fold with an ELISA buffer was added thereto in an amount of 50 μL/well and reacted at room temperature for 1 hour. After washing five times with an ELISA buffer, SuperSignal Pico ELISA Chemiluminescent substrate was added. After 10 minutes, the chemiluminescence was measured using a plate reader. As a result, the C2037, C3048, C3015, and C3022 scFvs were found to bind to the amino-terminal peptides of human GPRC5D (Figure 164A) and cynomolgus monkey GPRC5D (Figure 164B).

10)-7 Confirmation of binding of IgG to GPRC5D by ELISA

NeutrAvidin diluted to 1 μg/mL with PBS was added in an amount of 50 μL/well to 96-well Maxi-sorp plate, and the plate was left standing overnight at 4°C for immobilization. After washing three times with an ELISA buffer, the amino-terminal peptide of the biotinylated human or cynomolgus monkey GPRC5D (used in Example 10)-1) diluted to 1 μg/mL with PBS was added thereto, and the plate was shaken at room temperature for 1 hour. After washing three times with an ELISA buffer, the plate was blocked with Blocker Casein and washed three times with an ELISA buffer. Then, the culture solution of the IgG-expressing FreeStyle 293F cells was added to the plate and reacted at room temperature for 2 hours. After washing three times with an ELISA buffer, a horseradish peroxidase (HRP)-labeled anti-human Fab antibody (Jackson ImmunoResearch Laboratories, Inc.) diluted 2500-fold with ELISA buffer was added thereto in an amount of 50 μL/well and reacted at room temperature for 1 hour. After washing five times with an ELISA buffer, SuperSignal Pico ELISA Chemiluminescent substrate was added. After 10 minutes, the chemiluminescence was measured using a plate reader.

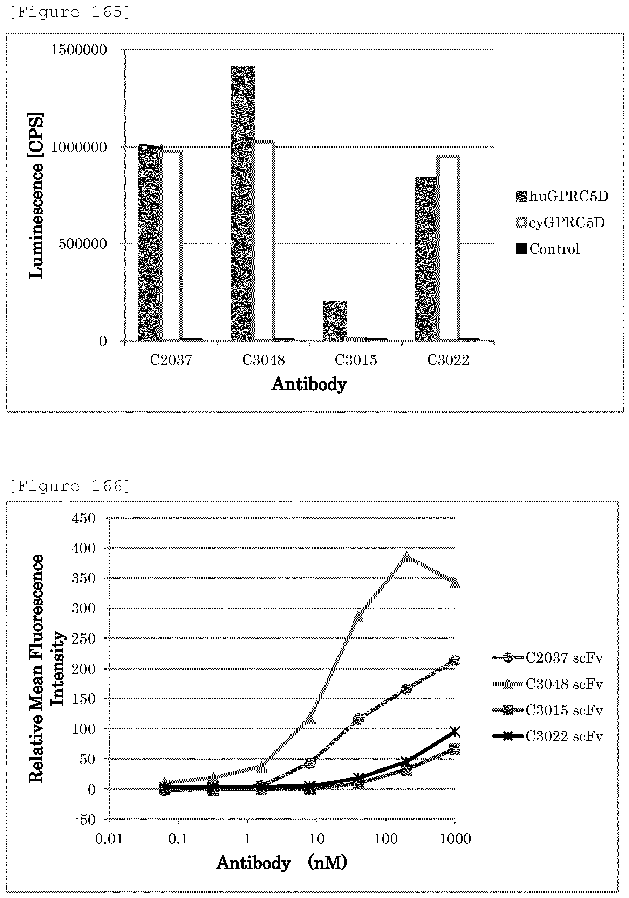

As a result, the C2037, C3048, C3015 and C3022 IgG forms were found to bind to the amino-terminal peptides of human GPRC5D and cynomolgus monkey GPRC5D (Figure 165).

10)-8 Binding of scFv to endogenous human GPRC5D-expressing cell (KMS-34)

The KMS-34 cells were recovered by centrifugation, washed twice with a FACS buffer (PBS containing 0.5% BSA and 2 mM EDTA, pH 7.4), and then suspended in the same solution as above. The C2037, C3048, C3015 or C3022 scFv was added to the obtained cell suspension, and the mixture was left standing at 4°C for 2 hours. After washing twice with a FACS buffer, the cells were suspended by the addition of an anti-FLAG antibody (Sigma-Aldrich Corp.), and the mixture was further left standing at 4°C for 1 hour. After washing twice with a FACS buffer, the cells were suspended by the addition of Alexa 488-labeled anti-mouse IgG antibody (Jackson ImmunoResearch Laboratories, Inc.), and the mixture was further left standing at 4°C for 1 hour. After washing twice with a FACS buffer, the cells were fixed in 1% PFA (prepared from a 32% paraformaldehyde solution (Electron Microscopy Sciences)), followed by detection using a flow cytometer (FACSCanto(TM) II; Becton, Dickinson and Company). The data was analyzed using Flowjo (Tree Star, Inc.).

As a result, the C2037, C3048, C3015, and C3022 scFvs were found to bind to human GPRC5D-expressing cells (Figure 166).

10)-9 Binding of IgG to endogenous human GPRC5D-expressing cell (KHM-1B)

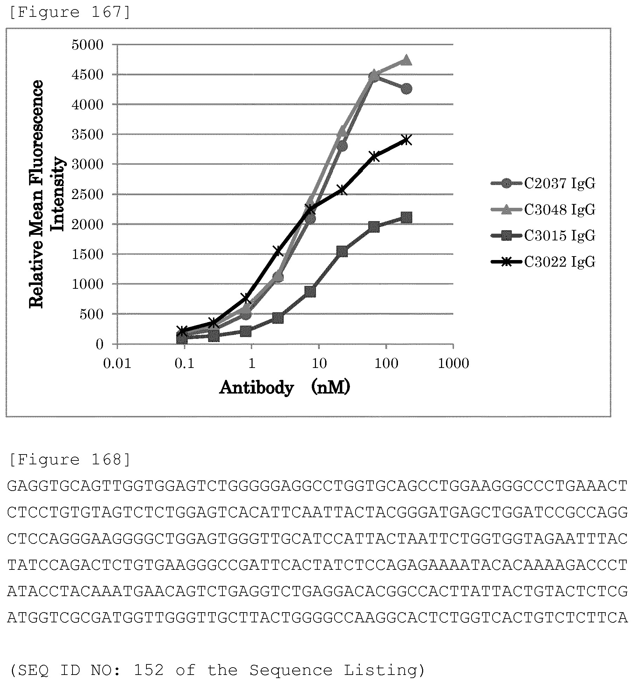

Human multiple myeloma cell line KHM-1B cells expressing GPRC5D were adjusted to a concentration of 2 × $10^6$ cells/mL with PBS containing 5% FBS, inoculated in an amount of 100 μL/well to a 96-well U-bottomed microplate, and centrifuged to remove the supernatant. The culture supernatant of each human anti-GPRC5D antibody (full-length IgG form containing C2037, C3048, C3015, and C3022) obtained in Example 10)-5 or Human IgG isotype control antibody (Calbiochem/Merck Millipore Corp.) adjusted to 14 ng/mL to 30 μg/mL was added thereto in an amount of 100 μL/well, and the plate was left standing at 4°C for 1 hour. The cells were washed twice with PBS containing 5% FBS. Then, R-Phycoerythrin AffiniPure F(ab')2 Fragment Goat Anti-Human IgG, Fcγ Fragment Specific (Jackson ImmunoResearch Laboratories, Inc.) diluted 100-fold with PBS containing 5% FBS was added thereto in an amount of 100 μL/well, and the plate was left standing at 4°C for 1 hour. The cells were washed twice with PBS containing 5% FBS and then resuspended in PBS containing 5% FBS, followed by detection using a flow cytometer (FACSCanto(TM) II; Becton, Dickinson and Company). The data was analyzed using Flowjo (Tree Star, Inc.). The PE fluorescence intensity of the cell fraction was plotted to a histogram, and the mean fluorescence intensity (MFI) was calculated. The MFI value of the control antibody was subtracted from the MFI value of the GPRC5D antibody to calculate a relative value of MFI (rMFI). As a result, the C2037, C3048, C3015, and C3022 IgG forms were found to bind to human GPRC5D-expressing cells (Figure 167).

10)-10 Preparation and evaluation of modified forms of C3022 and C3048

10)-10-1 Obtainment of modified forms

A library was constructed using a method for introducing a mutation by PCR templates (Zaccolo, et al., J. Mol. Biol. (1996) 255, 589-603) using the C3022 and C3048 genes, or a method which involves synthesizing oligomers for all CDR residues, wherein each residue was mutated to 19 types of amino acids other than wild-type amino acids to construct a library (oligo-based library). The library was screened for clones having high binding capacity, and their nucleotide sequences were determined. The identified high binding capacity mutations were combined to obtain a high binding capacity mutant E1018 of C3022 and a high binding capacity mutant D1012 of C3048.

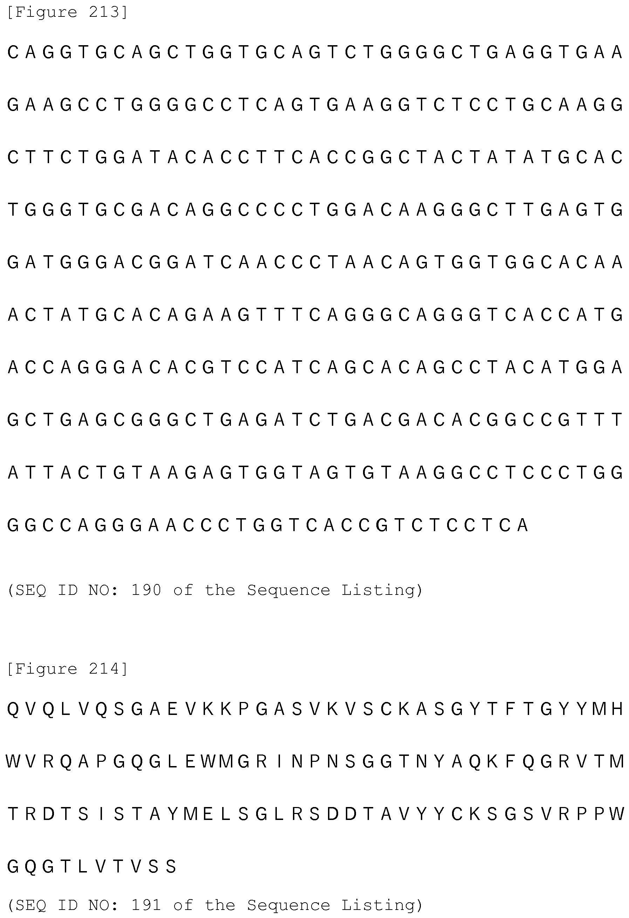

The nucleotide sequence of the cDNA encoding the heavy chain variable region of the obtained E1018 is represented by SEQ ID NO: 190 (Figure 213), and the amino acid sequence thereof is represented by SEQ ID NO: 191 (Figure 214).

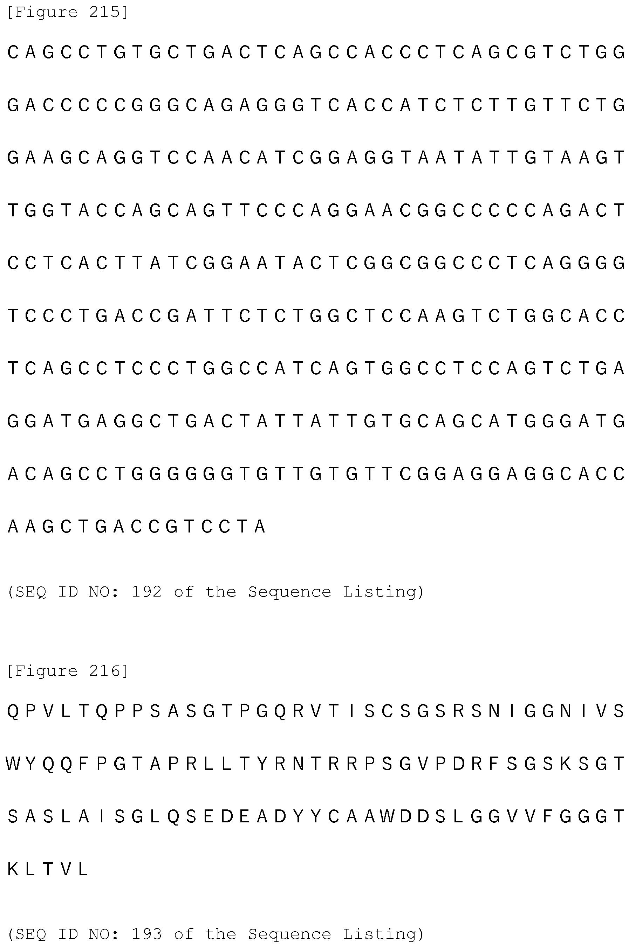

The nucleotide sequence of the cDNA encoding the light chain variable region of the obtained E1018 is represented by SEQ ID NO: 192 (Figure 215), and the amino acid sequence thereof is represented by SEQ ID NO: 193 (Figure 216).

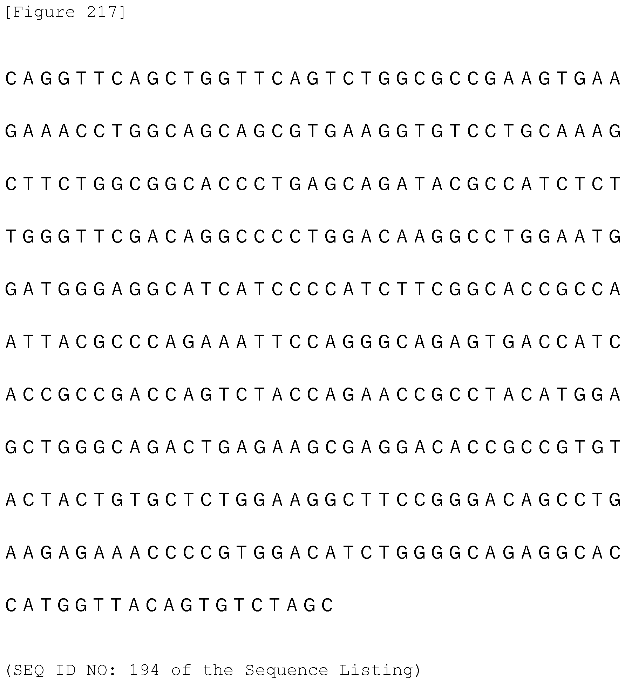

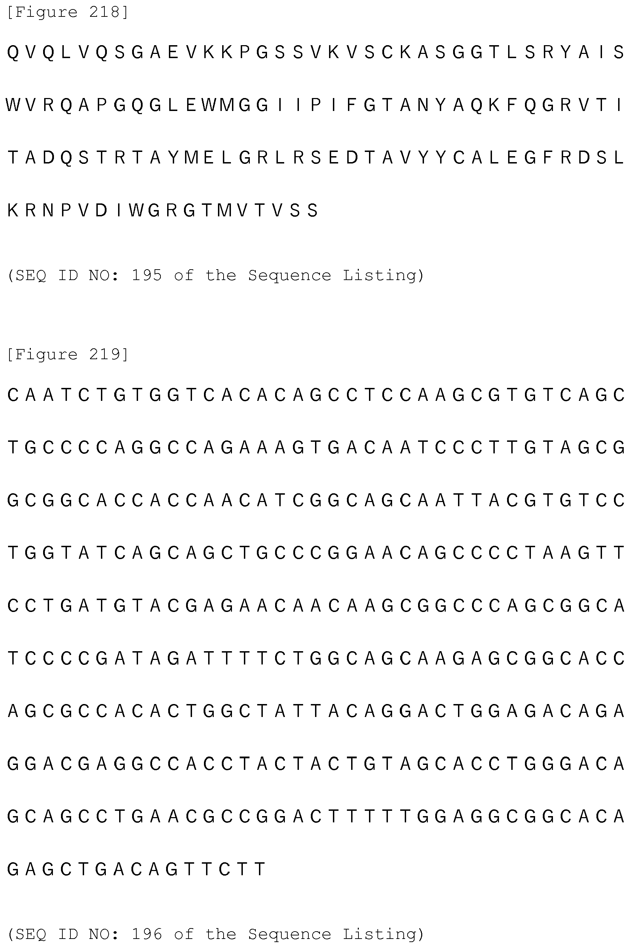

The nucleotide sequence of the cDNA encoding the heavy chain variable region of the obtained D1012 is represented by SEQ ID NO: 194 (Figure 217), and the amino acid sequence thereof is represented by SEQ ID NO: 195 (Figure 218).

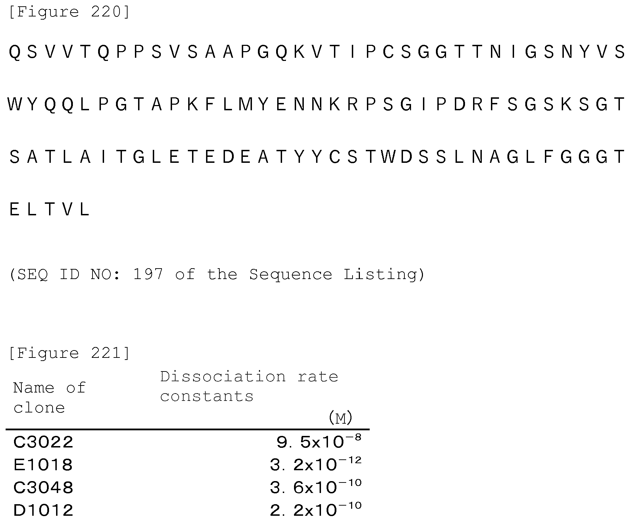

The nucleotide sequence of the cDNA encoding the light chain variable region of the obtained D1012 is represented by SEQ ID NO: 196 (Figure 219), and the amino acid sequence thereof is represented by SEQ ID NO: 197 (Figure 220).

10)-10-2 Confirmation of that binds to GPRC5D using Biacore

The binding activity of the anti-GPRC5D antibodies to the amino-terminal peptide of human GPRC5D was tested by SPR using Biacore T200. The amino-terminal peptide of biotinylated human GPRC5D diluted to 2 nM with HBS-EP+ (manufactured by GE Healthcare Bio-Sciences Corp.) was immobilized on Sensor Chip CAP (manufactured by GE Healthcare Bio-Sciences Corp.) by contact at a rate of 10 μL/min for 180 seconds. Then, Kd was calculated by kinetic analysis using a plurality of concentrations of each scFv diluted with HBS-EP+ as analytes. As a result, the E1018 and D1012 scFvs were found to bind to the amino-terminal peptide of human GPRC5D more strongly than C3022 and C3048, respectively (Figure 221).

(Example 11) Construction of anti-CD3 antibody expression vector

11)-1 Construction of rat anti-CD3 scFv antibody expression vector

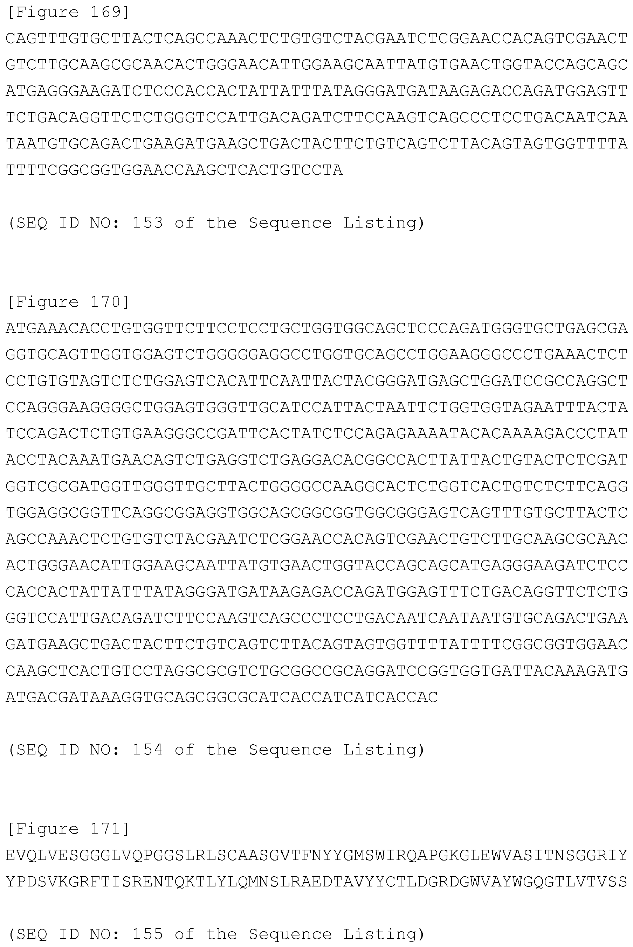

A rat anti-CD3 monoclonal antibody-producing hybridoma was prepared from lymph node or spleen of a rat immunized by the DNA immunization method. cDNAs encoding VH and VL of the monoclonal antibody were sequenced from the hybridoma, and a single chain Fv expression vector was prepared. Specifically, the VH DNA fragment of SEQ ID NO: 152 (Figure 168) amplified by PCR, the DNA fragment of the linker to be inserted between VH and VL, and a DNA fragment amplified by PCR in which a DNA sequence encoding a FLAG-His tag was added to a region containing the VL DNA sequence of SEQ ID NO: 153 (Figure 169) at the carboxyl terminus, were fused using In-Fusion HD cloning kit (Clontech Laboratories, Inc.) to prepare a single chain antibody expression vector pC3E-7000 containing the nucleotide sequence of SEQ ID NO: 154 (Figure 170) in ORF.

11)-2 Construction of humanized anti-CD3 scFv antibody expression vector pC3E-7034

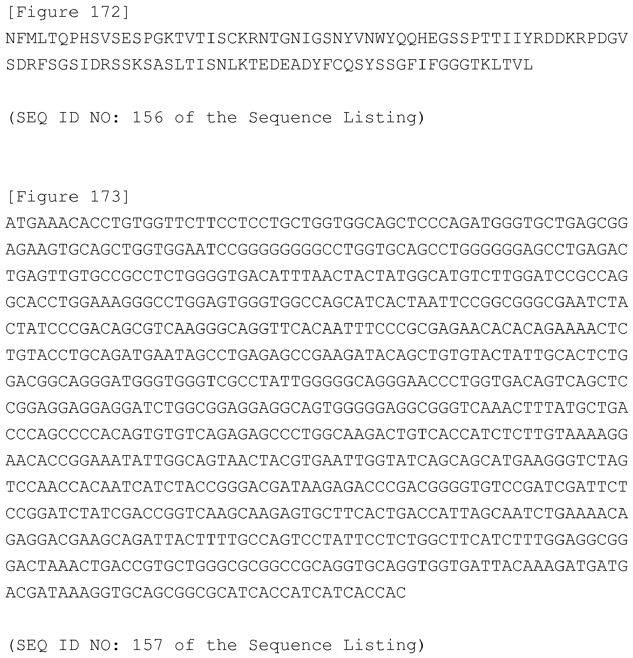

A DNA fragment comprising a scFv DNA sequence connecting a region containing SEQ ID NO: 156 (Figure 172) to the carboxyl terminus of SEQ ID NO: 155 (Figure 171) via a 15-amino acid flexible linker, and 15-base additional sequences upstream and downstream thereof was synthesized (GeneArt Artificial Gene Synthesis Service). A region containing the C3E-7034 DNA and its upstream and downstream additional sequences was amplified by PCR using this DNA fragment as a template to obtain an insert DNA fragment. A vector region except for an scFv region was amplified by PCR using the expression vector pC3E-7000 prepared in Example 11)-1 as a template to obtain a vector fragment. These DNA fragments were fused using In-Fusion HD cloning kit (Clontech Laboratories, Inc.) to construct an expression vector containing the nucleotide sequence of SEQ ID NO: 157 (Figure 173) in ORF. The obtained expression vector was designated as "pC3E-7034".

11)-3 Construction of humanized anti-CD3 scFv antibody expression vector pC3E-7035

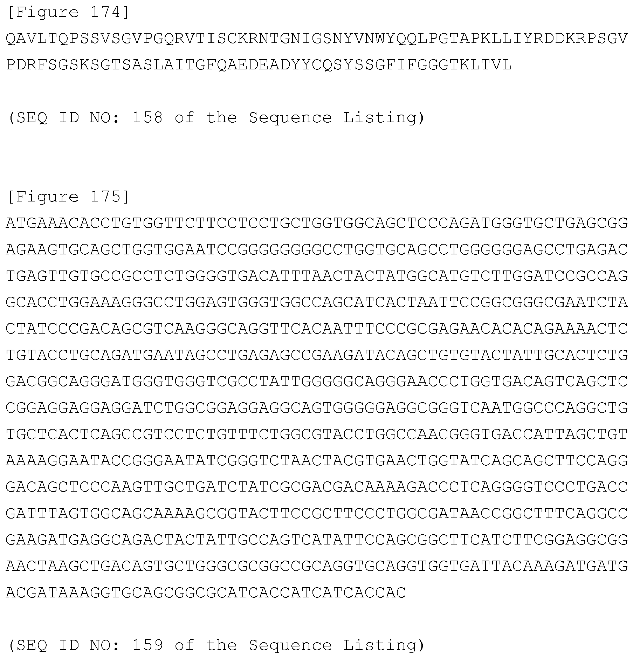

A DNA fragment comprising a scFv DNA sequence connecting SEQ ID NO: 158 (Figure 174) to the carboxyl terminus of SEQ ID NO: 155 (Figure 171) via a 17-amino acid flexible linker, and 15-base additional sequences upstream and downstream thereof was synthesized (GeneArt Artificial Gene Synthesis Service). An expression vector containing the nucleotide sequence of SEQ ID NO: 159 (Figure 175) in ORF was constructed in the same way as in Example 11)-2. The obtained expression vector was designated as "pC3E-7035".

11)-4 Construction of humanized anti-CD3 scFv antibody expression vector pC3E-7036

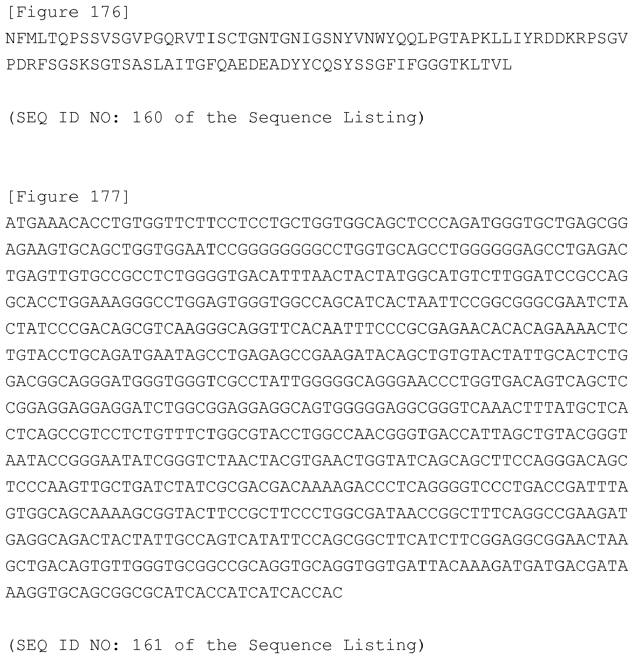

A DNA fragment comprising a scFv DNA sequence connecting SEQ ID NO: 160 (Figure 176) to the carboxyl terminus of SEQ ID NO: 155 (Figure 171) via a 15-amino acid flexible linker, and 15-base additional sequences upstream and downstream thereof was synthesized (GeneArt Artificial Gene Synthesis Service). A C3E-7036 expression vector containing the nucleotide sequence of SEQ ID NO: 161 (Figure 177) in ORF was constructed in the same way as in Example 11)-2. The obtained expression vector was designated as "pC3E-7036".

(Example 12) Preparation of anti-GPRC5D-anti-CD3 bispecific molecule

12)-1 Preparation of anti-GPRC5D-anti-CD3 bispecific molecule expression vector

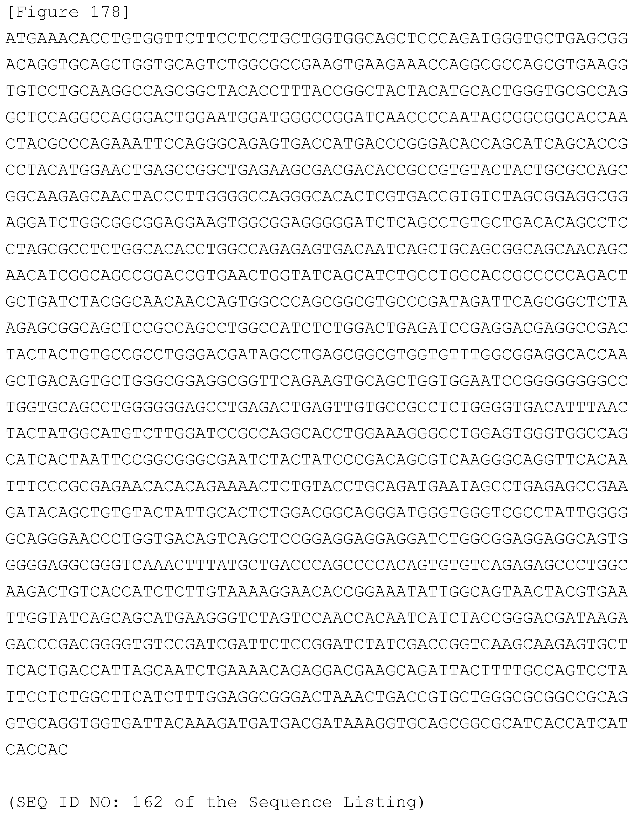

A DNA sequence of a region containing the C2037 antibody scFv, a portion of a human antibody heavy chain signal sequence, and an added linker to link scFvs was amplified by PCR using the DNA sequence of the C2037 antibody scFv prepared in Example 10)-4 as a template to obtain an insert DNA. Also, the whole vector region containing the CD3 scFv DNA was amplified by PCR using the expression vector pC3E-7034 prepared in Example 11)-2 as a template and primers encoding a signal sequence and the amino-terminal sequence of the CD3 scFv antibody to obtain a vector fragment. These DNA fragments were fused using In-Fusion HD cloning kit (Clontech Laboratories, Inc.) to construct an anti-GPRC5D-anti-CD3 bispecific molecule expression vector containing the nucleotide sequence of SEQ ID NO: 162 (Figure 178) in ORF. The obtained expression vector was designated as "pC2037-C3E-7034".

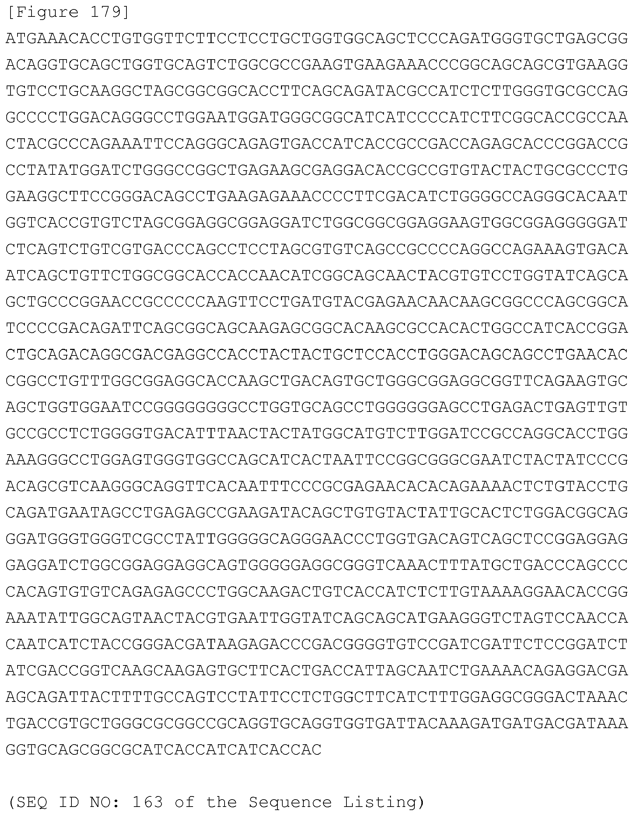

An anti-GPRC5D-anti-CD3 bispecific molecule expression vector containing the nucleotide sequence of SEQ ID NO: 163 (Figure 179) in ORF was constructed in the same way as above using the C3048 antibody scFv and pC3E-7034 as templates. The obtained expression vector was designated as "pC3048-C3E-7034".

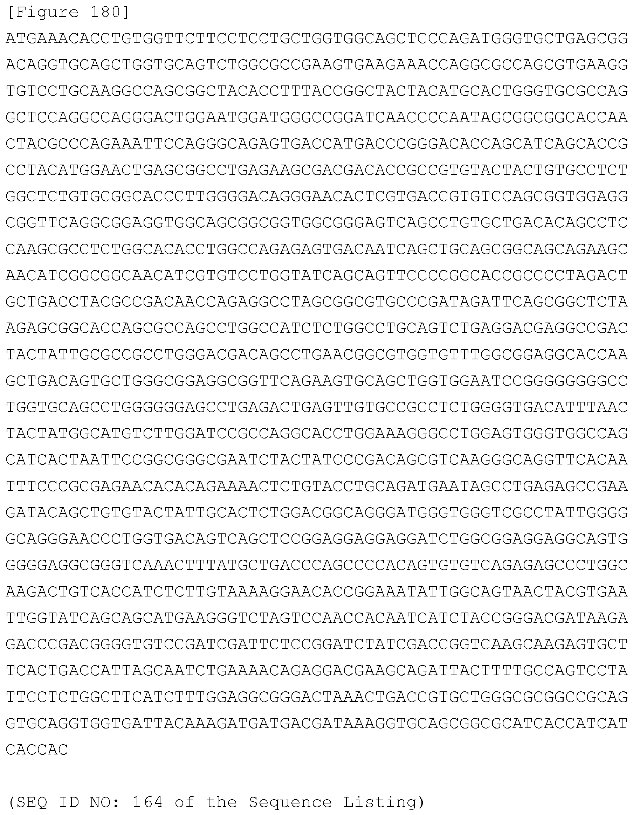

An anti-GPRC5D-anti-CD3 bispecific molecule expression vector containing the nucleotide sequence of SEQ ID NO: 164 (Figure 180) in ORF was constructed in the same way as above the C3022 antibody scFv and pC3E-7034 as templates. The obtained expression vector was designated as "pC3022-C3E-7034".

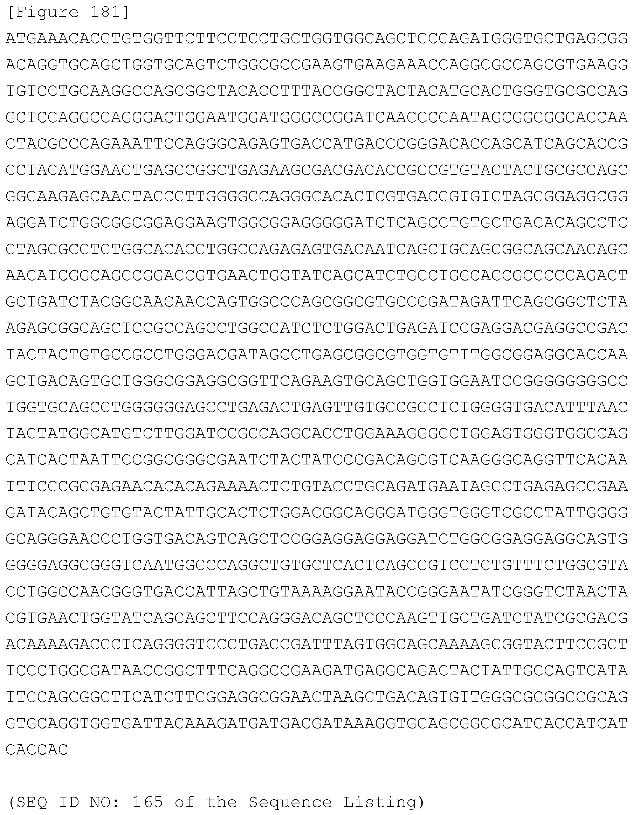

An anti-GPRC5D-anti-CD3 bispecific molecule expression vector containing the nucleotide sequence of SEQ ID NO: 165 (Figure 181) in ORF was constructed in the same way as above the C2037 antibody scFv and pC3E-7035 as templates. The obtained expression vector was designated as "pC2037-C3E-7035".

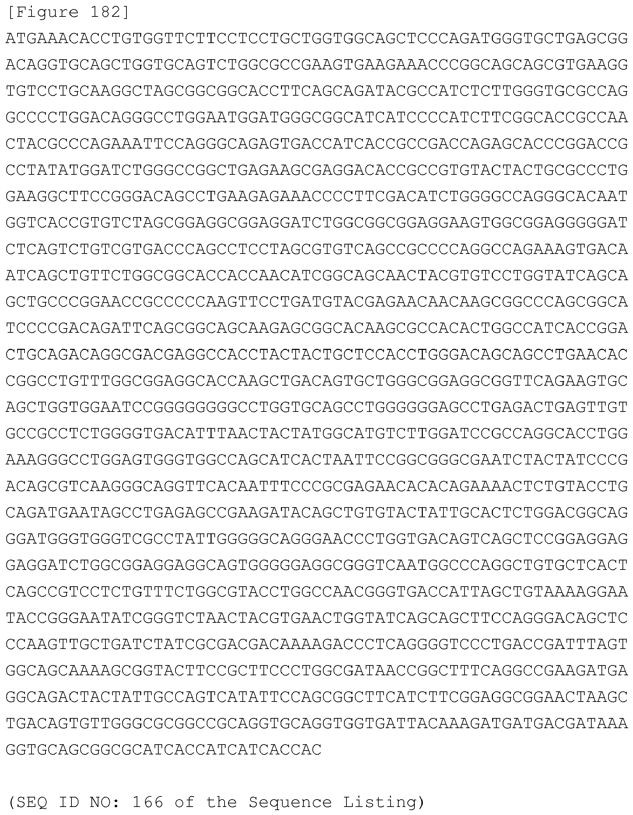

An anti-GPRC5D-anti-CD3 bispecific molecule expression vector containing the nucleotide sequence of SEQ ID NO: 166 (Figure 182) in ORF was constructed in the same way as above using the C3048 antibody scFv and pC3E-7035 as templates. The obtained expression vector was designated as " pC3048-C3E-7035".

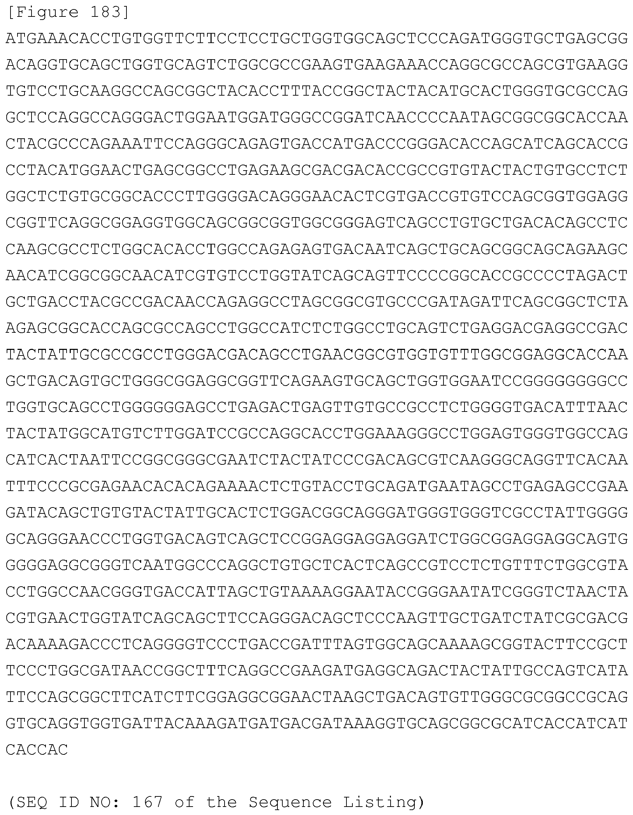

An anti-GPRC5D-anti-CD3 bispecific molecule expression vector containing the nucleotide sequence of SEQ ID NO: 167 (Figure 183) in ORF was constructed in the same way as above using the C3022 antibody scFv and pC3E-7035 as templates. The obtained expression vector was designated as "pC3022-C3E-7035".

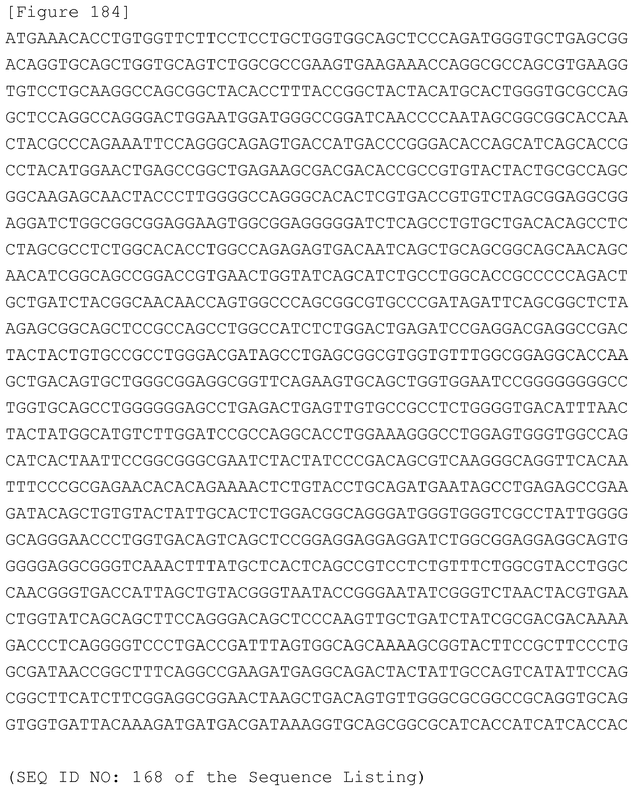

An anti-GPRC5D-anti-CD3 bispecific molecule expression vector containing the nucleotide sequence of SEQ ID NO: 168 (Figure 184) in ORF was constructed in the same way as above using the C2037 antibody scFv and pC3E-7036 as templates. The obtained expression vector was designated as "pC2037-C3E-7036".

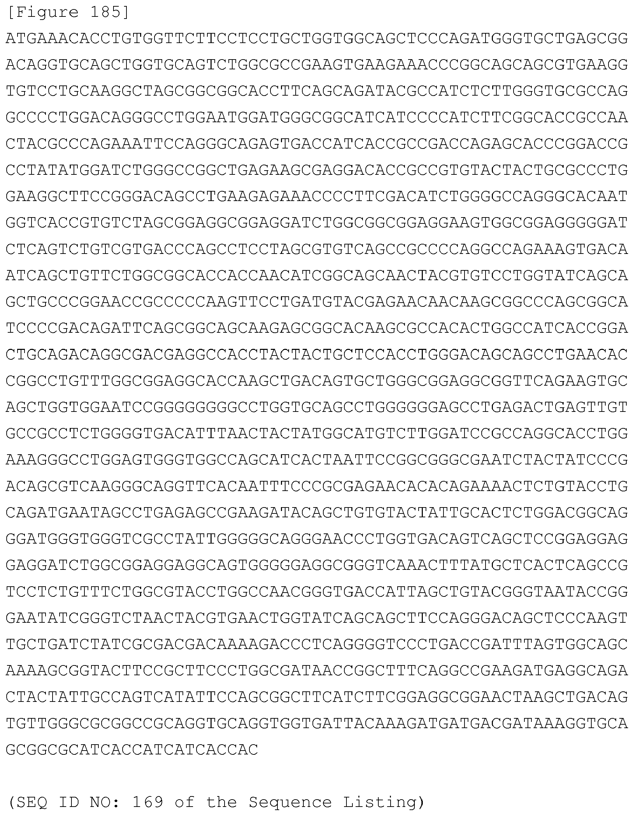

An anti-GPRC5D-anti-CD3 bispecific molecule expression vector containing the nucleotide sequence of SEQ ID NO: 169 (Figure 185) in ORF was constructed in the same way as above using the C3048 antibody scFv and pC3E-7036 as templates. The obtained expression vector was designated as "pC3048-C3E-7036".

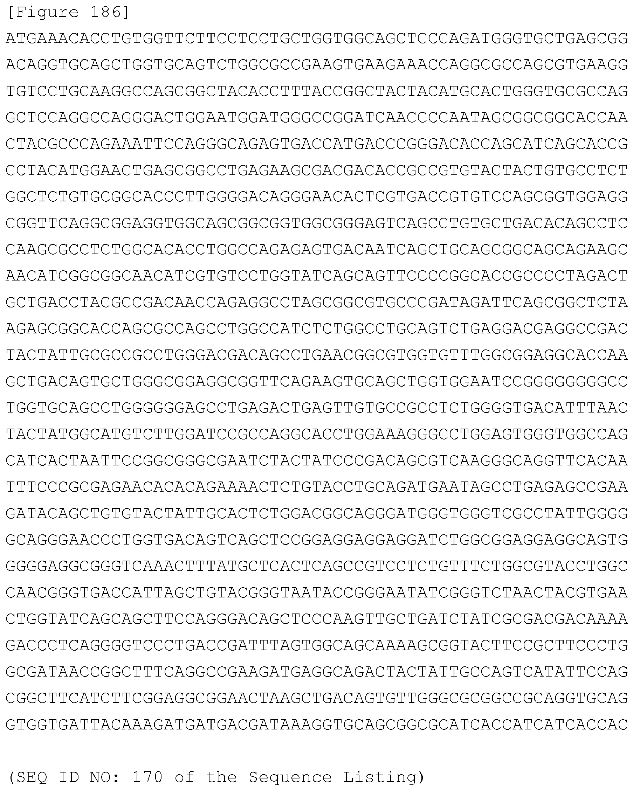

An anti-GPRC5D-anti-CD3 bispecific molecule expression vector containing the nucleotide sequence of SEQ ID NO: 170 (Figure 186) in ORF was constructed in the same way as above using the C3022 antibody scFv and pC3E-7036 as templates. The obtained expression vector was designated as "pC3022-C3E-7036".

12)-2 Expression and purification of anti-GPRC5D-anti-CD3 bispecific molecule

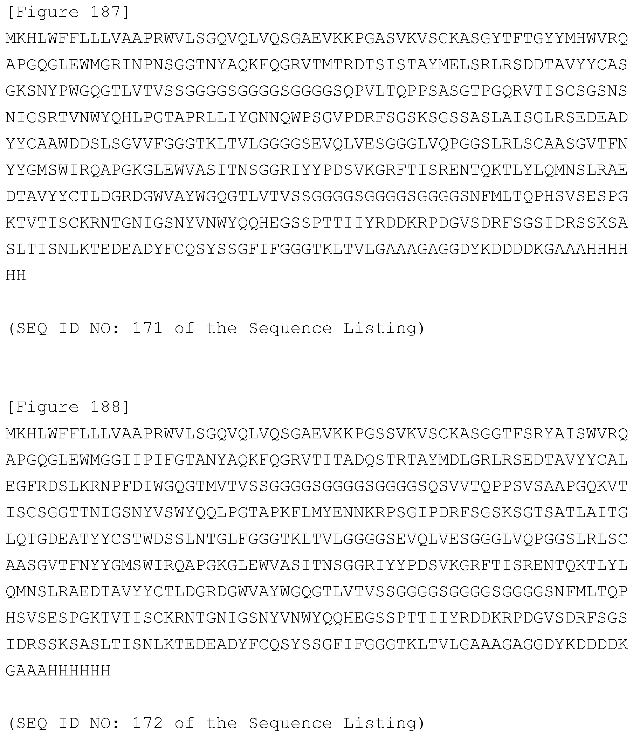

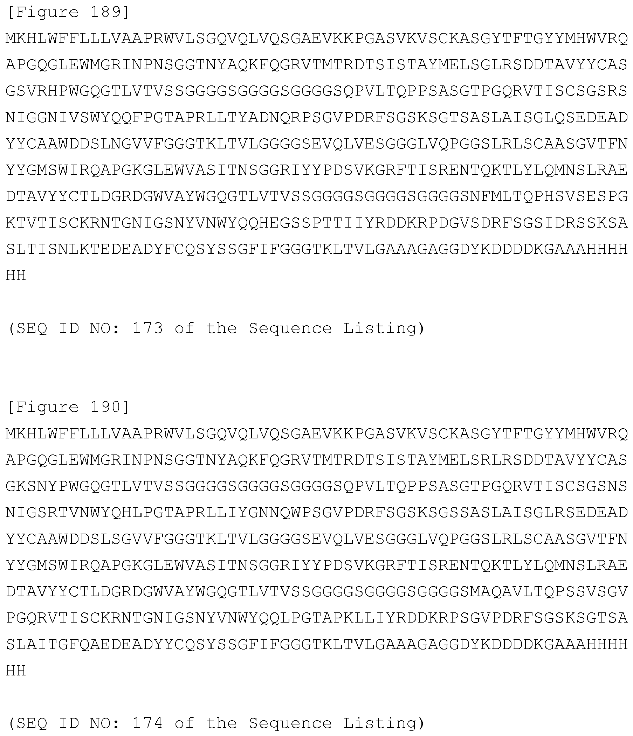

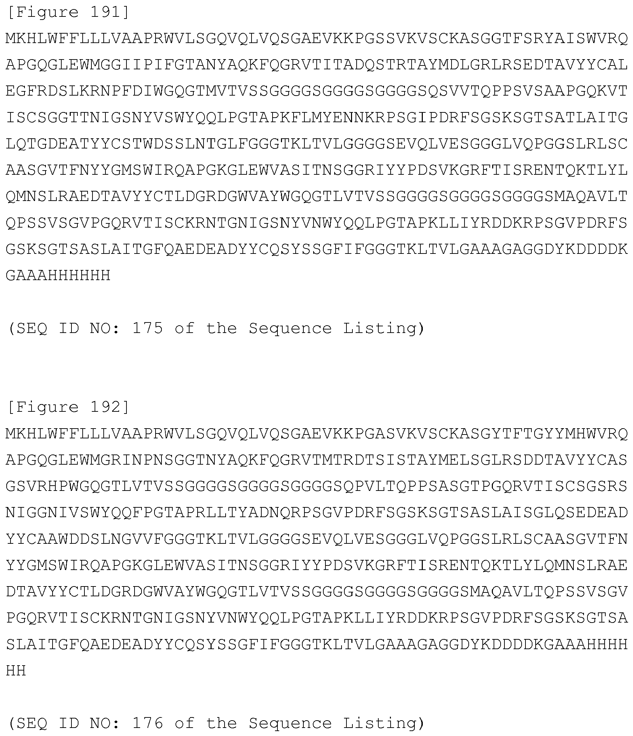

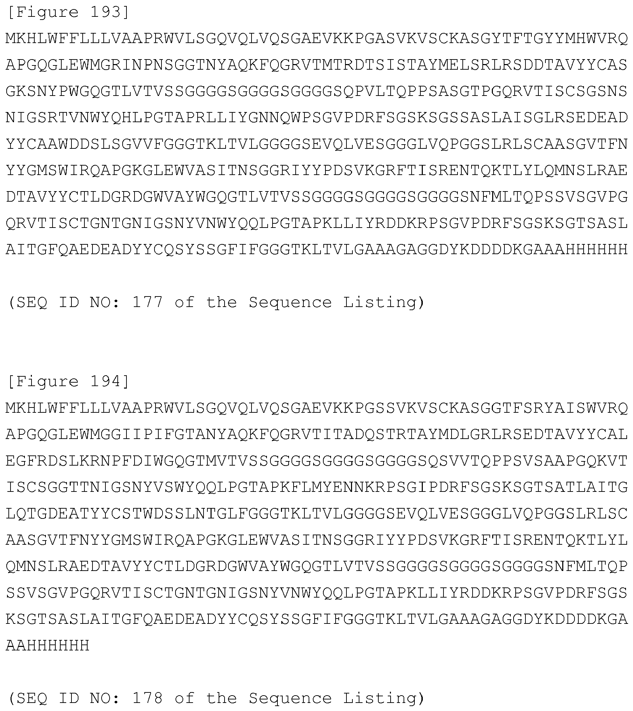

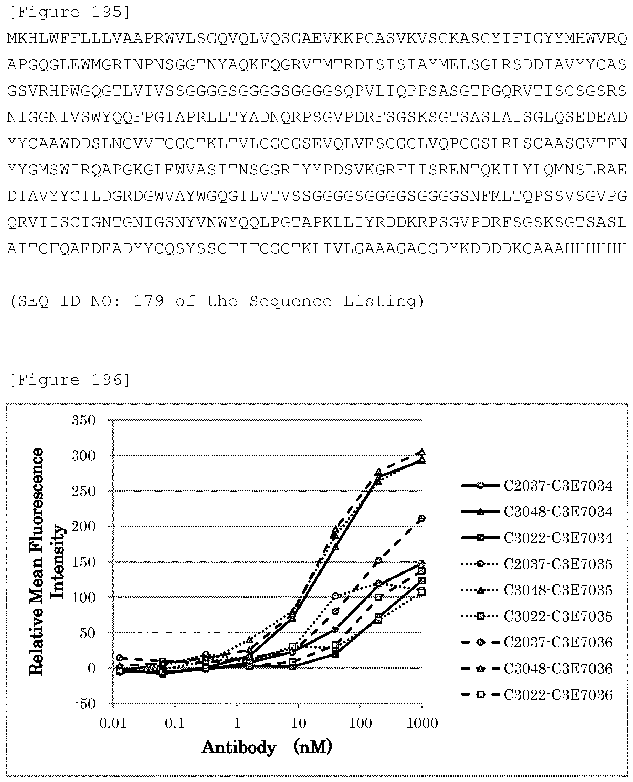

C2037-C3E-7034 to C3022-C3E-7036 were expressed and purified in the same way as in Example 10)-4. The amino acid sequence of C2037-C3E-7034 is described in SEQ ID NO: 171 (Figure 187). The amino acid sequence of C3048-C3E-7034 is described in SEQ ID NO: 172 (Figure 188). The amino acid sequence of C3022-C3E-7034 is described in SEQ ID NO: 173 (Figure 189). The amino acid sequence of C2037-C3E-7035 is described in SEQ ID NO: 174 (Figure 190). The amino acid sequence of C3048-C3E-7035 is described in SEQ ID NO: 175 (Figure 191). The amino acid sequence of C3022-C3E-7035 is described in SEQ ID NO: 176 (Figure 192). The amino acid sequence of C2037-C3E-7036 is described in SEQ ID NO: 177 (Figure 193). The amino acid sequence of C3048-C3E-7036 is described in SEQ ID NO: 178 (Figure 194). The amino acid sequence of C3022-C3E-7036 is described in SEQ ID NO: 179 (Figure 195).

(Example 13) *In vitro* activity evaluation of anti-GPRC5D-anti-CD3 bispecific molecule

13)-1 Binding activity evaluation of anti-GPRC5D-anti-CD3 bispecific molecule by flow cytometry

13)-1-1 Binding of anti-GPRC5D-anti-CD3 bispecific molecule to endogenous human GPRC5D-expressing cell (A4/Fuk)

Lymphoma cell line A4/Fuk cells (JCRB Cell Bank) were adjusted to an appropriate concentration with PBS containing 5% FBS. LIVE/DEAD Fixable Near-IR Dead Cell Stain Kit was added to the cells, which were then left standing at 4°C for 30 minutes. The cells were washed twice with PBS containing 5% FBS, then adjusted to a concentration of $1 \times 10^6$ cells/mL with PBS containing 5% FBS, inoculated in an amount of 100 μL/well to a 96-well U-bottomed microplate, and centrifuged to remove a supernatant. Each anti-GPRC5D-anti-CD3 bispecific molecule (C2037-C3E-7034 to C3022-C3E-7036 prepared in Example 12) diluted with PBS containing 5% FBS were added thereto in an amount of 100 μL/well, and the plate was left standing at 4°C for 60 minutes. The cells were washed twice with PBS containing 5% FBS. Then, Penta-His Alexa Fluor 488 diluted with PBS containing 5% FBS was added thereto in an amount of 30 μL/well, and the plate was left standing at 4°C for 30 minutes. The cells were washed twice with PBS containing 5% FBS and then resuspended in PBS containing 5% FBS, followed by detection using a flow cytometer (FACSCanto(TM) II). The data was analyzed using Flowjo. The mean fluorescence intensity (MFI) of Alexa Fluor 488 in a fraction free from dead cells was calculated. The MFI value of the antibody-unsupplemented sample was subtracted from the MFI value of the antibody-supplemented sample to calculate a relative value of MFI (rMFI). As shown in Figure 196, these anti-GPRC5D-anti-CD3 bispecific antibodies were found to bind to endogenous human GPRC5D-expressing cells.

13)-1-2 Binding of anti-GPRC5D-anti-CD3 bispecific molecule to cynomolgus monkey GPRC5D-expressing cells

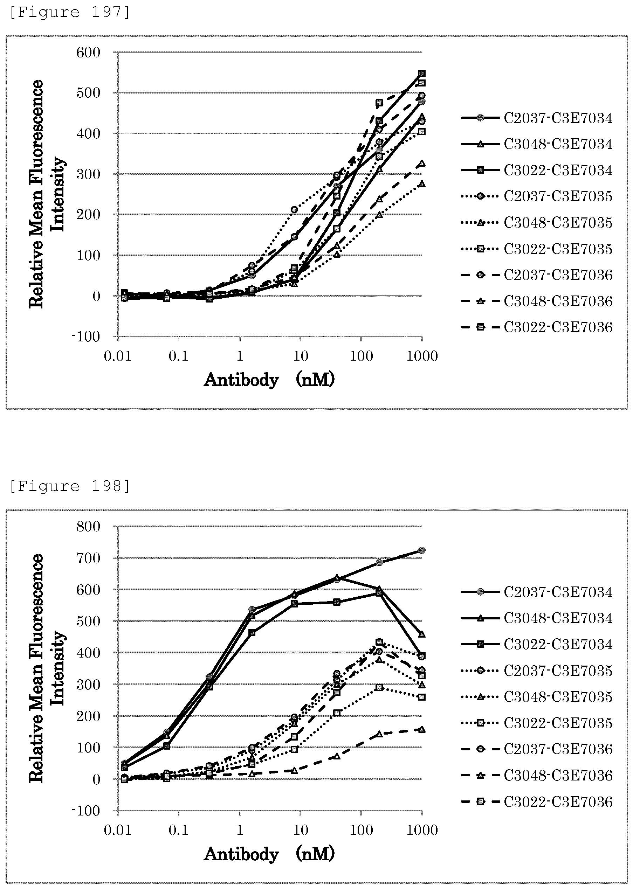

Staining and analysis were carried out in the same way as in Example 13)-1-1 using the KMS-11_cGPRC5D cells prepared in Example 5)-2-2. As shown in Figure 197, these anti-GPRC5D-anti-CD3 bispecific antibodies were found to bind to cynomolgus monkey GPRC5D-expressing cells.

13)-1-3 Binding of anti-GPRC5D-anti-CD3 bispecific molecule to human CD3 (PBMC)

Commercially available human PBMC (Cellular Technology Limited) was adjusted to an appropriate concentration with PBS containing 5% FBS. LIVE/DEAD Fixable Near-IR Dead Cell Stain Kit (Thermo Fisher Scientific Inc.) and an anti-CD19 antibody (Beckman Coulter Inc.) were added to the cells, which were then left standing at 4°C for 30 minutes. The cells were washed twice with PBS containing 5% FBS, then adjusted to a concentration of $1 \times 10^6$ cells/mL with PBS containing 5% FBS, inoculated in an amount of 100 μL/well to a 96-well U-bottomed microplate, and centrifuged to remove a supernatant. Each anti-GPRC5D-anti-CD3 bispecific molecule (C2037-C3E-7034 to C3022-C3E-7036 prepared in Example 12) diluted with PBS containing 5% FBS were added thereto in an amount of 100 μL/well, and the plate was left standing at 4°C for 60 minutes. The cells were washed twice with PBS containing 5% FBS. Then, Penta-His Alexa Fluor 488 (Qiagen N.V.) diluted with PBS containing 5% FBS was added thereto in an amount of 30 μL/well, and the plate was left standing at 4°C for 30 minutes. The cells were washed twice with PBS containing 5% FBS and then resuspended in PBS containing 5% FBS, followed by detection using a flow cytometer (FACSCanto(TM) II; Becton, Dickinson and Company). The data was analyzed using Flowjo (Tree Star, Inc.). The mean fluorescence intensity (MFI) of Alexa Fluor 488 in a fraction free from dead cells and CD19-positive cells was calculated. The MFI value of the antibody-unsupplemented sample was subtracted from the MFI value of the antibody-supplemented sample to calculate a relative value of MFI (rMFI). As shown in Figure 198, these anti-GPRC5D-anti-CD3 bispecific antibodies were found to bind to human CD3-expressing cells.

13)-1-4 Binding of anti-GPRC5D-anti-CD3 bispecific molecule to cynomolgus monkey CD3 (PBMC)

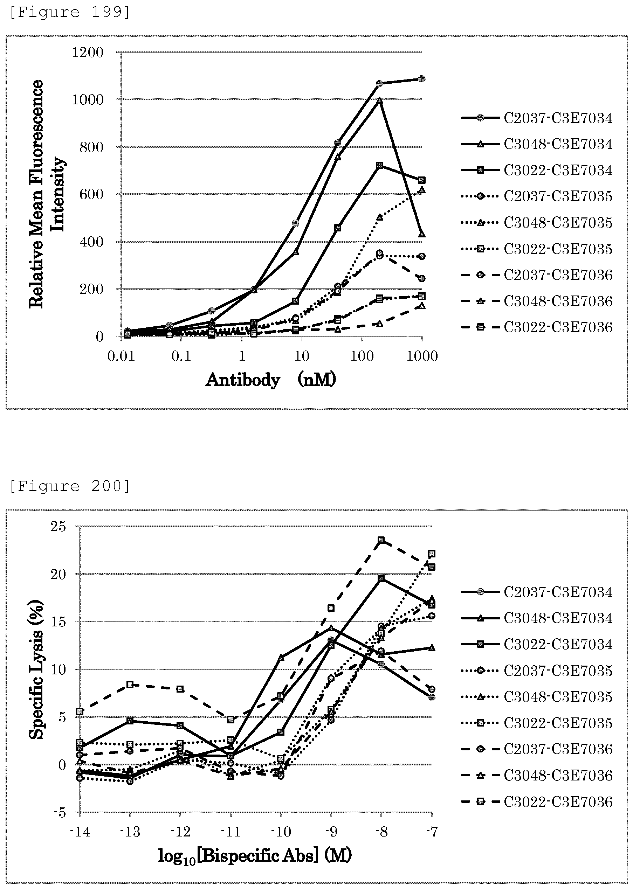

PBMC was collected from the blood of a cynomolgus monkey according to a standard method using SepMate (StemCell Technologies Inc.) and Lymphocyte Separation Solution (Nacalai Inc.). Using the collected cynomolgus monkey PBMC, staining and analysis were carried out in the same way as in Example 13)-1-3. As shown in Figure 199, these anti-GPRC5D-anti-CD3 bispecific antibodies were found to bind to cynomolgus monkey CD3-expressing cells.

13)-2 Cytotoxic activity evaluation of anti-GPRC5D-anti-CD3 bispecific molecule

13)-2-1 Preparation of target cell

A4/Fuk cells were adjusted to a concentration of $1 \times 10^6$ cells/mL with an RPMI1640 medium (Thermo Fisher Scientific Inc.) containing 10% FBS. 100 μL of Chromium-51 Radionuclide (PerkinElmer, Inc.) was added per mL of the cell suspension, and the cells were cultured at 37°C for 2 hours under 5% $CO_2$ conditions. The cells were washed twice with an RPMI1640 medium containing 10% FBS, then resuspended to $1 \times 10^5$ cells/mL in an RPMI1640 medium containing 10% FBS, and used as target cells.

13)-2-2 Preparation of effector cell

Commercially available frozen PBMC (Cellular Technology Limited) was thawed at 37°C, transferred to a solution of an RPMI1640 medium containing 10% FBS supplemented with Anti-aggregate Wash reagent (Cellular Technology Limited), washed twice, then adjusted to $1 \times 10^6$ cells/mL with an RPMI1640 medium containing 10% FBS, and used as effector cells.

13)-2-3 Cytotoxicity assay

The A4/Fuk cells obtained in Example 13)-2-1 were added at a concentration of 50 μL/well to a 96-well U-bottomed microplate. Each anti-GPRC5D-anti-CD3 bispecific molecule (prepared in Example 12) adjusted to varying concentrations was added thereto in an amount of 50 μL/well. The effector cells prepared in Example 13)-2-2 were added thereto in an amount of 100 μL/well. After centrifugation at room temperature at 1000 rpm for 1 minute, the cells were cultured at 37°C for 20 to 24 hours under 5% $CO_2$ conditions. A 50 μL aliquot of the supernatant was recovered into LumaPlate (PerkinElmer, Inc.) and dried at 50°C for approximately 2 hours, followed by measurement using a plate reader (TopCount; PerkinElmer, Inc.). The percentage of cells lysed was calculated according to the following expression:

$$\text{Percentage of cells lysed (\%)} = (A - B) / (C - B) \times 100$$

A: Count of sample wells
B: Average of background (antibody-unsupplemented wells) counts (n = 3). 50 μL of a medium for assay was added when adding the antibody. The other procedures were the same as in the case of the sample wells.
C: Average of maximum release (wells containing target cells lysed in a surfactant) counts (n = 3). 50 μL of a medium for assay was added when adding the antibody. 100 μL of the surfactant was added, and the 50 μL aliquot was transferred to LumaPlate, as with the sample well, and assayed.

As shown in Figure 200, these anti GPRC5D-anti-CD3 bispecific antibodies exhibited cytotoxic activity against the A4/Fuk cells.

(Example 14) Preparation of Fc-containing anti-GPRC5D-anti-CD3 bispecific molecule

14)-1 Preparation of Fc-containing anti-GPRC5D-anti-CD3 bispecific molecule expression vector

14)-1-1 Preparation of full-size antibody (FSA)-type bispecific molecule expression vector

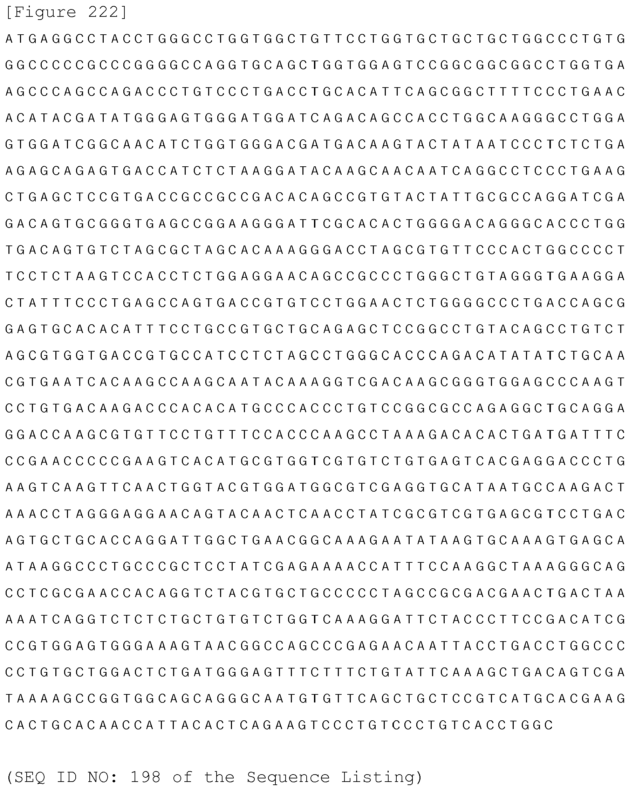

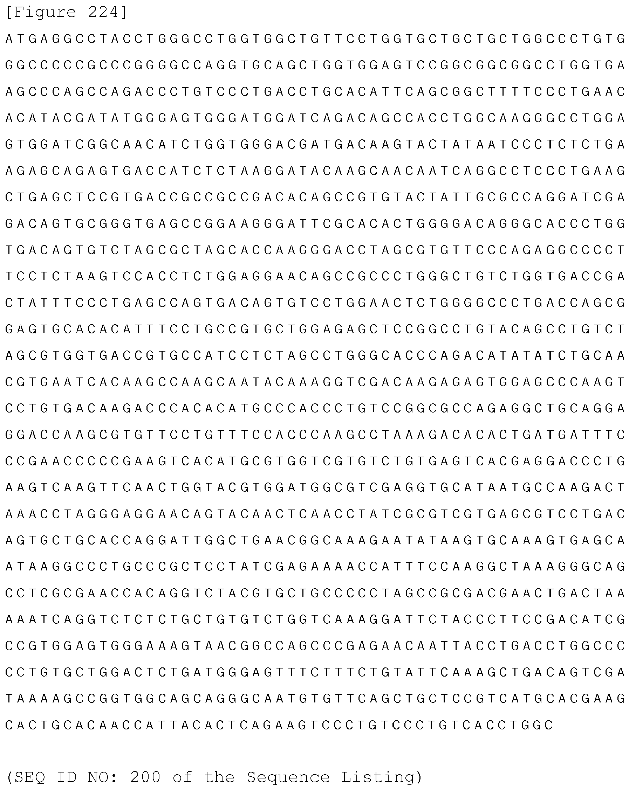

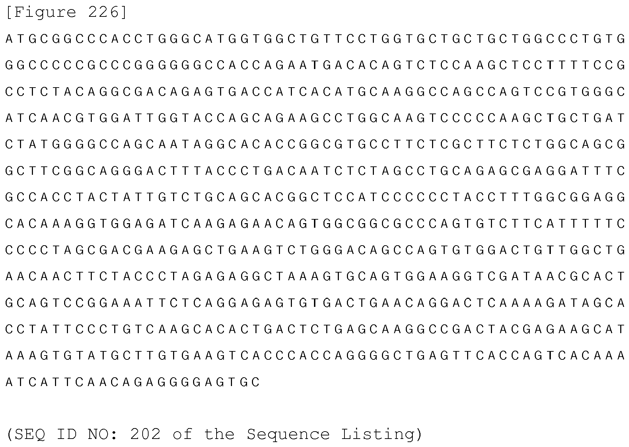

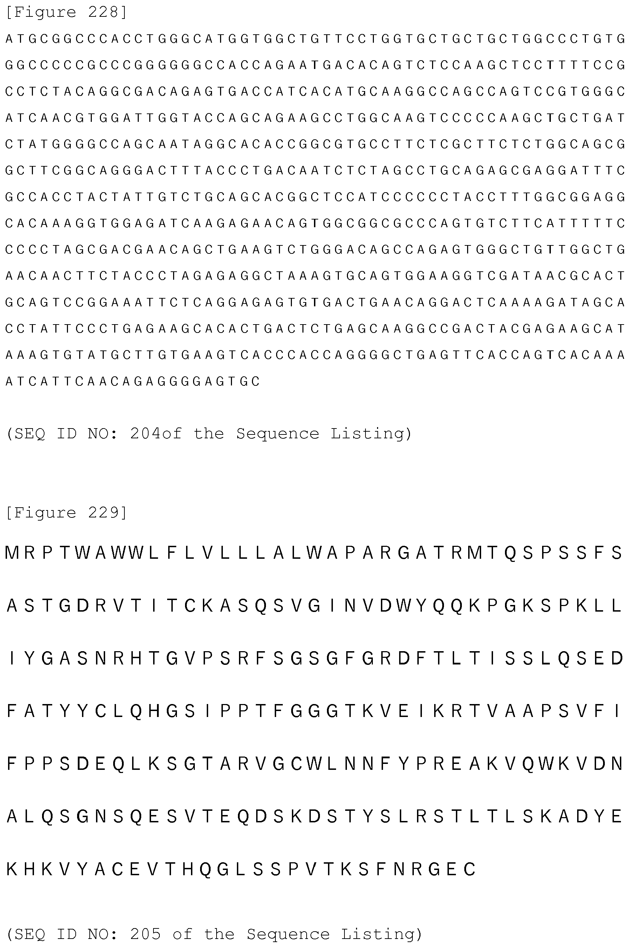

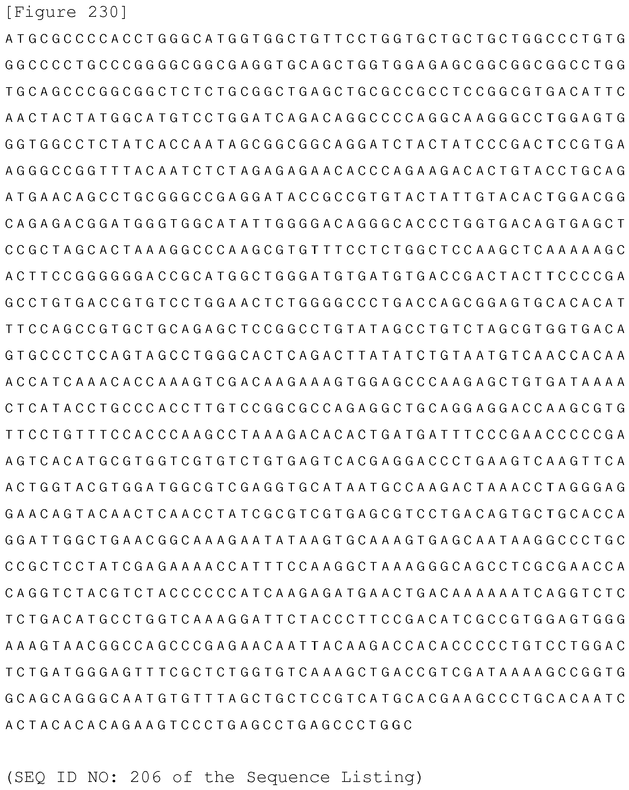

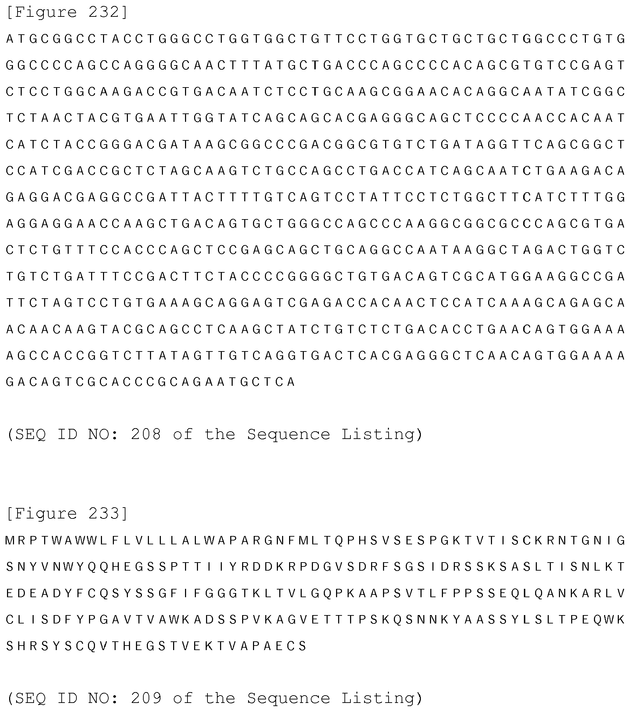

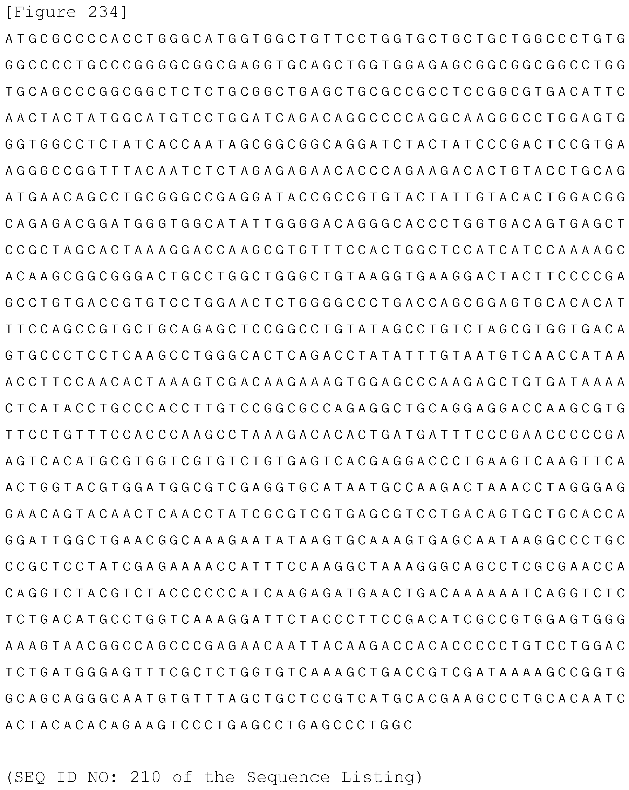

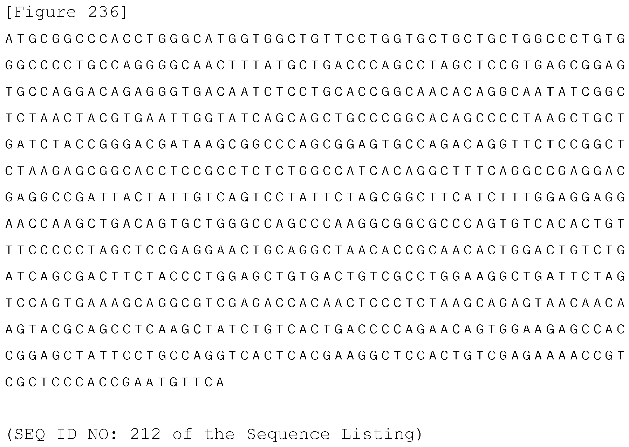

DNA encoding the humanized anti-GPRC5D antibody (h2B1) H2 type heavy chain variable region constructed in Example 8)-1-2 was net-synthesized (Genscript Custom Gene Synthesis Service). Expression vectors "pCL_#13540" and "pCL_#13543" were prepared by inserting the obtained heavy chain variable region, two types of human IgG-derived CH1 regions, and a Fc region with reduced effector functions and containing heteromultimer-forming mutations (WO2014/190441) to mammalian expression vector pTT5 (National Research Council, WO2009/137911). Also, the humanized anti-GPRC5D antibody (h2B1) L5 type light chain variable region constructed in Example 8)-2-5 was net-synthesized. The expression vectors "pCL_#12290" and "pCL_#12313" were prepared by inserting DNAs encoding the obtained light chain variable region and two types of human IgG-derived CL regions to mammalian expression vector pTT5.
Next, a DNA fragment encoding the heavy chain of the humanized anti-CD3 scFv (C3E-7034) constructed in Example 11)-2 was net-synthesized. "pCL_#13552" was prepared by inserting the obtained scFv heavy chain variable region, human IgG-derived CH1, and a Fc region with reduced effector functions and containing heteromultimer-forming mutations to mammalian expression vector pTT5. Also, a DNA fragment encoding the light chain of the humanized anti-CD3 scFv (C3E-7034) constructed in Example 11)-2 was net-synthesized. "pCL_#12287" was prepared by inserting the obtained scFv light chain variable region and human IgG-derived CL to mammalian expression vector pTT5. Likewise, a DNA sequence encoding the heavy chain of the humanized anti-CD3 scFv (C3E-7036) constructed in Example 11)-4 was net-synthesized. "pCL_#13541" was prepared by inserting DNA sequences encoding the obtained scFv heavy chain variable region, human IgG-derived CH1, and a Fc region with reduced effector functions and containing heteromultimer-forming mutations to mammalian expression vector pTT5. Also, a DNA fragment encoding the light chain of the humanized anti-CD3 scFv (C3E-7036) constructed in Example 11)-4 was net-synthesized. "pCL_#12321" was prepared by inserting DNA fragments encoding the obtained scFv light chain and human IgG-derived CL to mammalian expression vector pTT5.
The ORF sequences of pCL_#13540, pCL_#13543, pCL_#12290, pCL_#12313, pCL_#13552, pCL_#12287, pCL_#13541, and pCL_#12321 are represented by SEQ ID NO: 198 (Figure 222), SEQ ID NO: 200 (Figure 224), SEQ ID NO: 202 (Figure 226), SEQ ID NO: 204 (Figure 228), SEQ ID NO: 206 (Figure 230), SEQ ID NO: 208 (Figure 232), SEQ ID NO: 210 (Figure 234), and SEQ ID NO: 212 (Figure 236), respectively, of the Sequence Listing.

14)-1-2 Preparation of hybrid-type bispecific molecule expression vectors

An expression vector for mammalian cells having an insert of DNA fragments encoding the humanized anti-GPRC5D antibody (h2B1) H2 type heavy chain variable region, a human IgG-derived CH1 region, and a Fc region with reduced effector functions and containing heteromultimer-forming mutations was prepared and designated as "pCL_#13555". Also, an expression vector for mammalian cells having an insert of DNA fragments encoding the humanized anti-GPRC5D antibody (h2B1) L5 type light chain variable region and a human IgG-derived CL region was prepared and designated as "pCL_#12123".
Next, an expression vector for mammalian cells having an insert of DNA fragments encoding the humanized anti-CD3 scFv (C3E-7034) and a Fc region with reduced effector functions and containing heteromultimer-forming mutations was prepared and designated as "pCL_#13557". Also, an expression vector "pCL_#13561" for mammalian cells having an insert of DNA fragments encoding the humanized anti-CD3 scFv (C3E-7036) and a Fc region with reduced effector functions and containing heteromultimer-forming mutations was prepared.

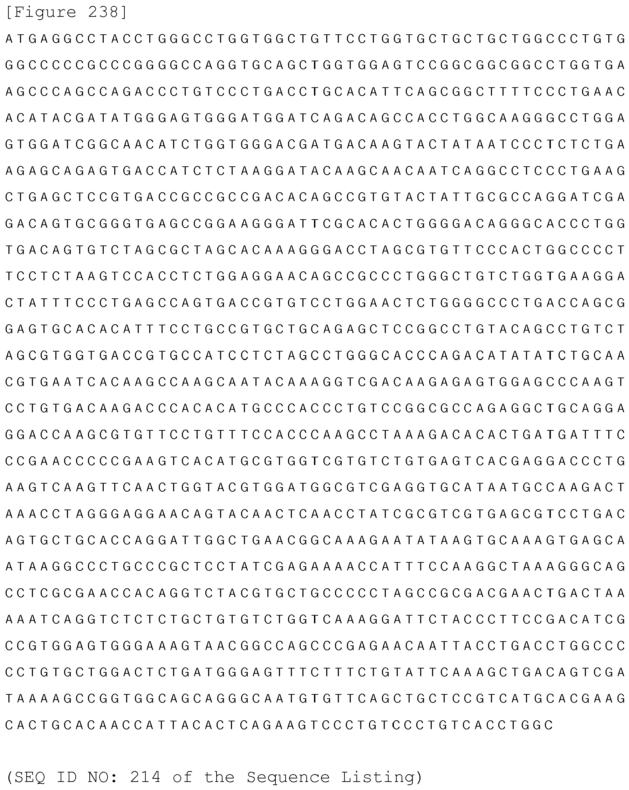

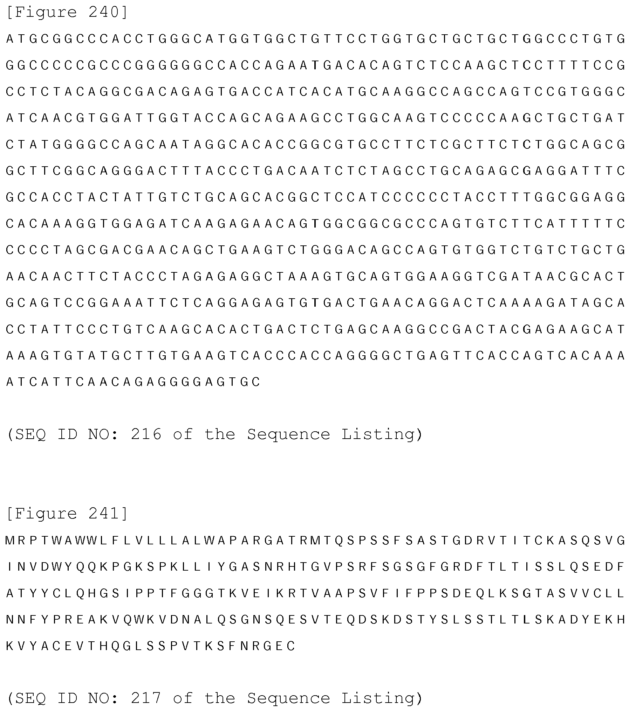

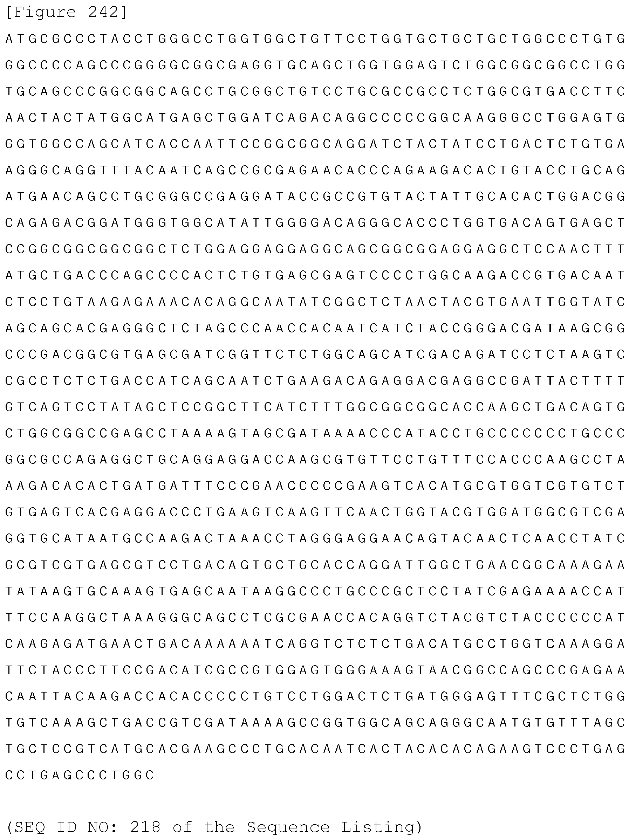

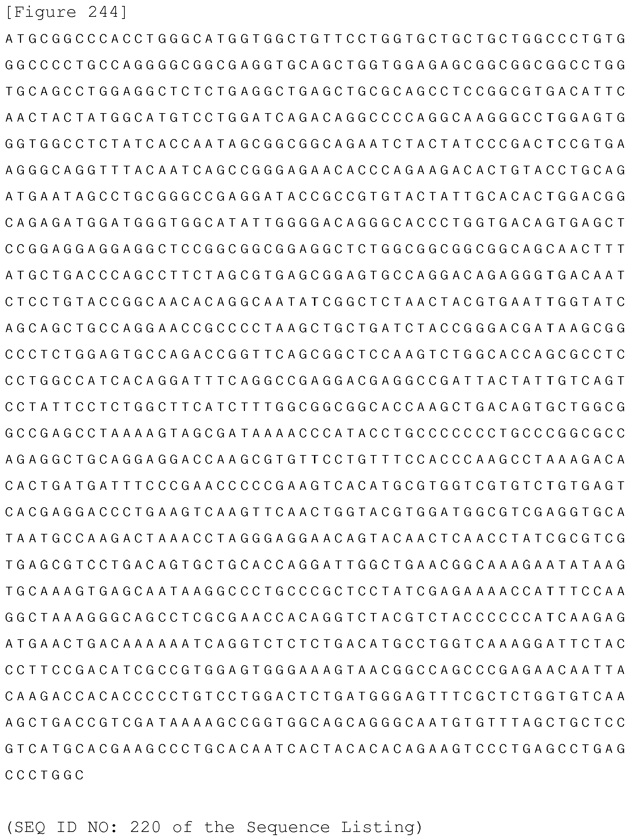

The ORF sequences of pCL_#13555, pCL_#12123, pCL_#13557, and pCL_#13561 are represented by SEQ ID NO: 214 (Figure 238), SEQ ID NO: 216 (Figure 240), SEQ ID NO: 218 (Figure 242), and SEQ ID NO: 220 (Figure 244), respectively, of the Sequence Listing.

14)-1-3 Preparation of dual-type bispecific molecule expression vector

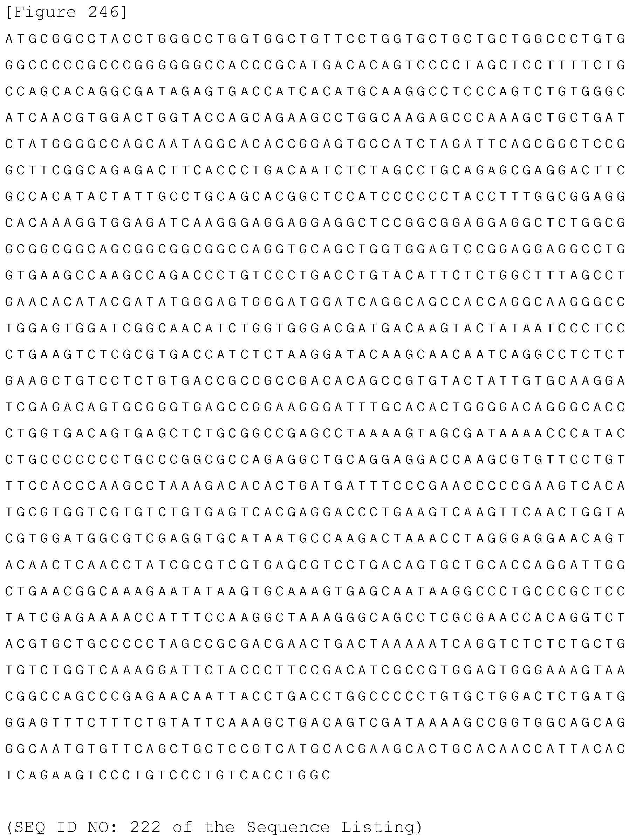

The humanized anti-GPRC5D antibody (h2B1) H2 type heavy chain variable region and L5 type light chain variable region constructed in Example 8)-1-2 were linked via a flexible linker consisting of 3 repeat sequences of GGGGS to prepare a single chain antibody (scFv). "pCL_#13563" was prepared by inserting DNA fragments encoding this anti-GPRC5D scFv and a Fc region with reduced effector functions and containing heteromultimer-forming mutations to mammalian expression vector pTT5. The ORF sequence of pCL_#13563 is represented by SEQ ID NO: 222 (Figure 246) of the Sequence Listing.

14)-2 Production of Fc-containing anti-GPRC5D-anti-CD3 bispecific molecule

CHO-3E7 cells were subcultured and cultured according to the manual (National Research Council Canada, Raymond C. et al., Methods (2011) 55 (1), 44-51). A culture medium of CHO-3E7 cells in the logarithmic growth phase was diluted to $2 \times 10^6$ cells/mL with a FreeStyle F17 medium (Invitrogen Corp.) containing 4 mM glutamine and 0.1% Kolliphor (Sigma-Aldrich Corp.) and used in the production of various bispecific antibodies.

14)-2-1 Production of full-size antibody (FSA)-type bispecific molecule

8000 μg of Polyethyleneimine max (PEImax, Polysciences) was dissolved in dispensed FreeStyle F17 medium to prepare a PEImax solution. 1000 μg of a mixture of the vectors pCL_#13552, pCL_#12287, pCL_#13540, and pCL_#12290 mixed at a ratio of 15:15:53:17, or the vectors pCL_#13541, pCL_#12321, pCL_#13543, and pCL_#12313 mixed at a ratio of 22:8:17:53, was added to an aliquot of the dispensed F17 medium to obtain a vector mixture. 1000 μg of a DNA mixture of pAKT and pGFP (both from National Research Council) mixed with already fragmented salmon sperm DNA (Sigma-Aldrich Corp.) was added to another aliquot of the dispensed F17 medium to create a DNA solution. The PEImax solution, the vector mixture, and the DNA solution were combined, gently stirred, left standing for 5 minutes, and then added to 2 L of CHO-3E7 cell suspension. The cells were shake-cultured at 37°C for 1 day in a 5% $CO_2$ incubator. 0.5 mM valproic acid (Sigma-Aldrich Corp.) and 0.1% (w/v) Tryptone N1 (Organotechnie) were then added. The cells were further shake-cultured at 32°C for 6 days. On day 7 after the start of the culture, the culture supernatant was recovered and filtered through a 0.2 μm filter (Sartorius Japan K.K.) to prepare a sample for evaluation.

pCL_#13552, pCL_#12287, pCL_#13540, and pCL_#12290 were used in the expression and preparation of a FSA-type bispecific molecule of C3E-7034 and h2B1 (v19159). pCL_#13541, pCL_#12321, pCL_#13543, and pCL_#12313 were used in the expression and preparation of a FSA-type bispecific molecule of C3E-7036 and h2B1 (v19140).

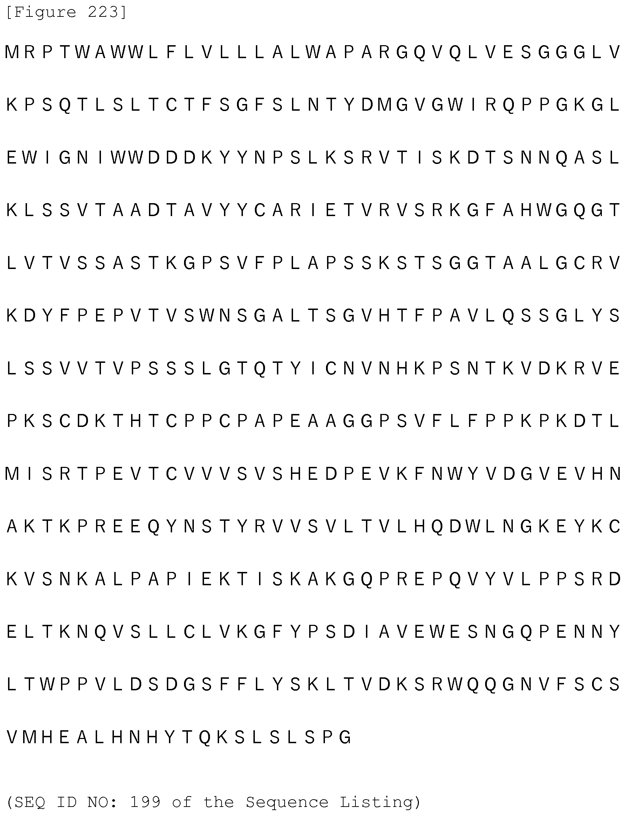

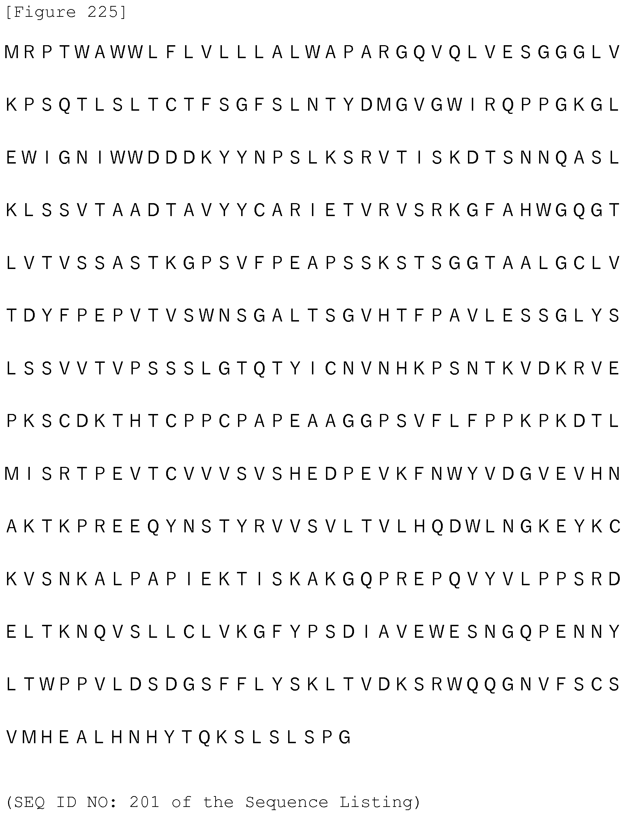

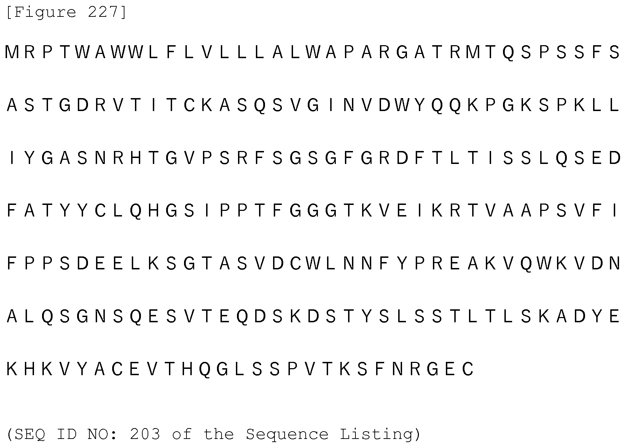

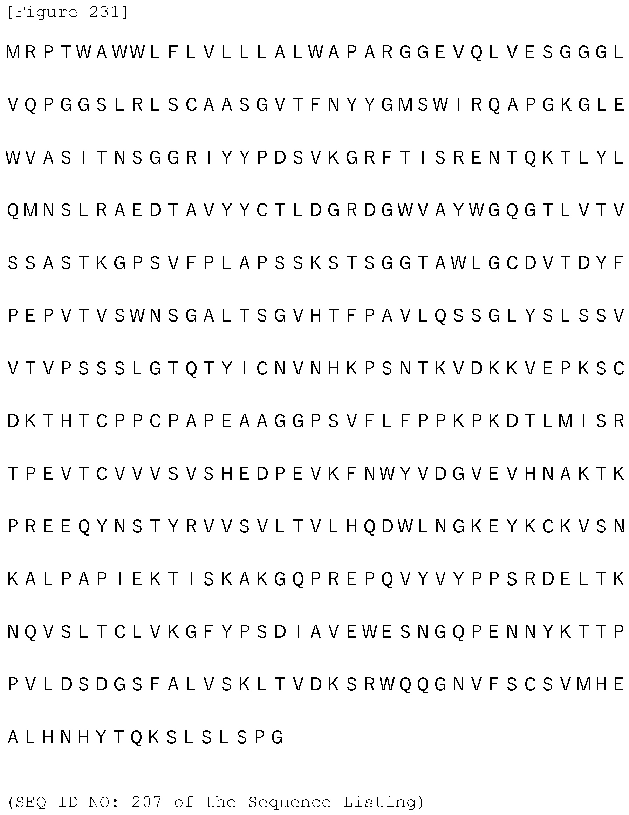

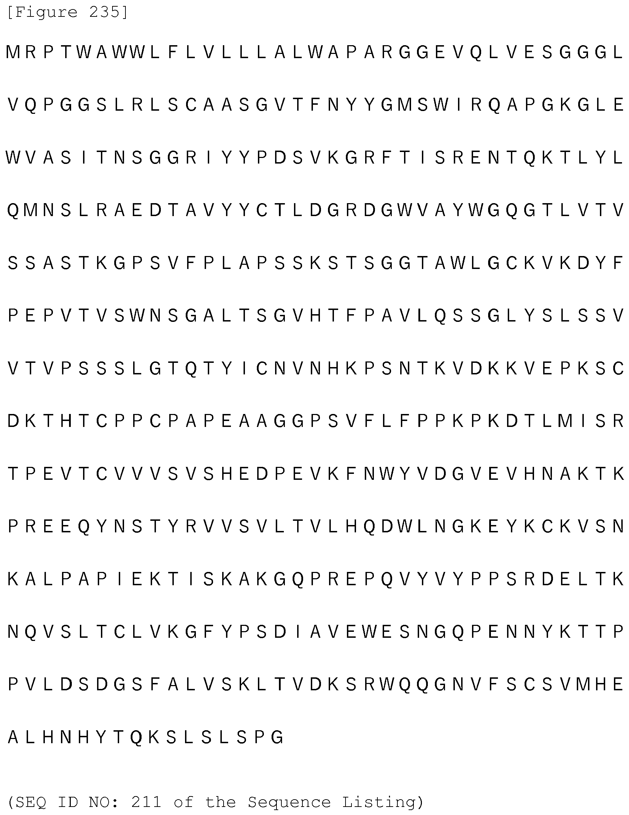

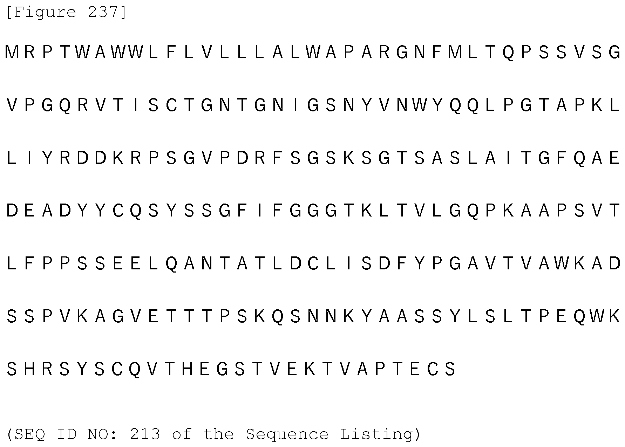

The amino acid sequences constituting v19159 obtained by expression from the respective vectors are represented by SEQ ID NOs: 207 (Figure 231), 209 (Figure 233), 199 (Figure 223), and 203 (Figure 227) of the Sequence Listing. The amino acid sequences constituting v19140 are represented by SEQ ID NOs: 211 (Figure 235), 213 (Figure 237), 201 (Figure 225), and 205 (Figure 229) of the Sequence Listing.

14)-2-2 Production of hybrid-type bispecific molecule

8000 μg of Polyethyleneimine max (PEImax, Polysciences) was dissolved in dispensed FreeStyle F17 medium to prepare a PEImax solution. 1000 μg of a mixture of the vectors pCL_#13557, pCL_#13555, and pCL_#12123 mixed at a ratio of 1:1:1.5, or the vectors pCL_#13561, pCL_#13555, and pCL_#12123 mixed at a ratio of 1:1:1.5, was added to an aliquot of the dispensed F17 medium to obtain a vector mixture. 1000 μg of a DNA mixture of pAKT and pGFP (both from National Research Council) mixed with already fragmented salmon sperm DNA (Sigma-Aldrich Corp.) was added to another aliquot of the dispensed F17 medium to obtain a DNA solution. The PEImax solution, the vector mixture, and the DNA solution were combined, gently stirred, left standing for 5 minutes, and then added to 2 L of CHO-3E7 cell suspension. The cells were shake-cultured at 37°C for 1 day in a 5% $CO_2$ incubator. 0.5 mM valproic acid (Sigma-Aldrich Corp.) and 0.1% (w/v) Tryptone N1 (Organotechnie) were then added. The cells were further shake-cultured at 32°C for 6 days. On day 7 after the start of the culture, the culture supernatant was recovered and filtered through a 0.2 μm filter (Sartorius Japan K.K.) to prepare a sample for evaluation.

pCL_#13557, pCL_#13555, and pCL_#12123 were used in the expression and preparation of a hybrid-type bispecific molecule of C3E-7034 and h2B1 (v19126). pCL_#13561, pCL_#13555, and pCL_#12123 were used in the expression and preparation of a hybrid-type bispecific molecule of C3E-7036 and h2B1 (v19125).

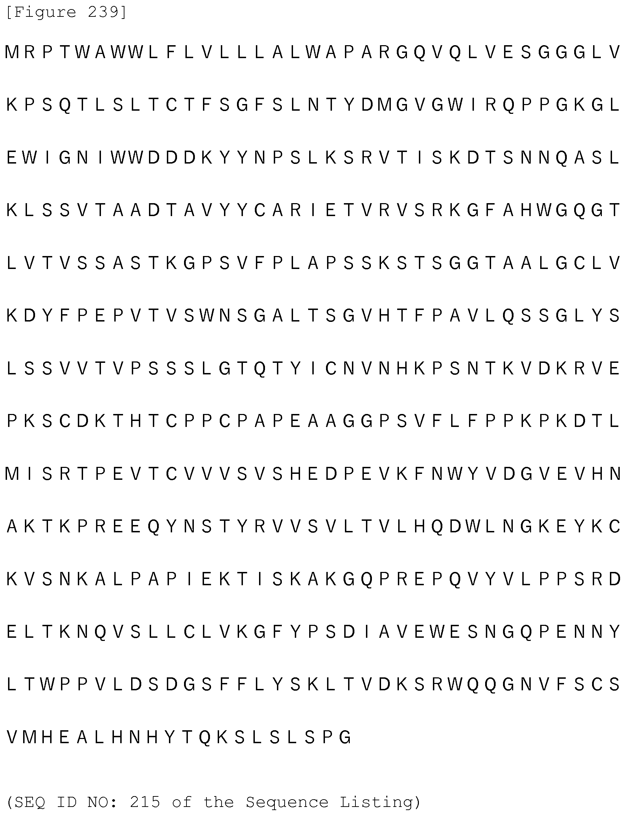

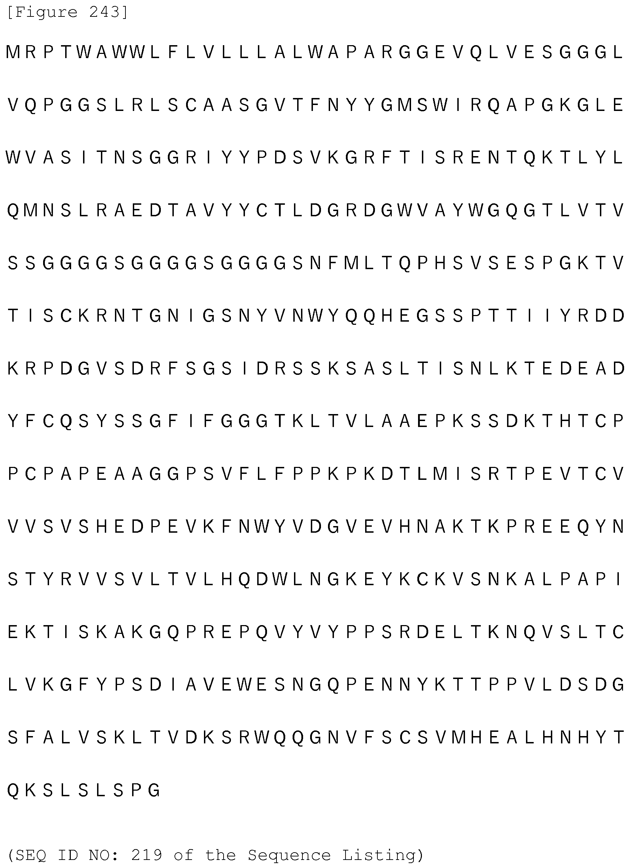

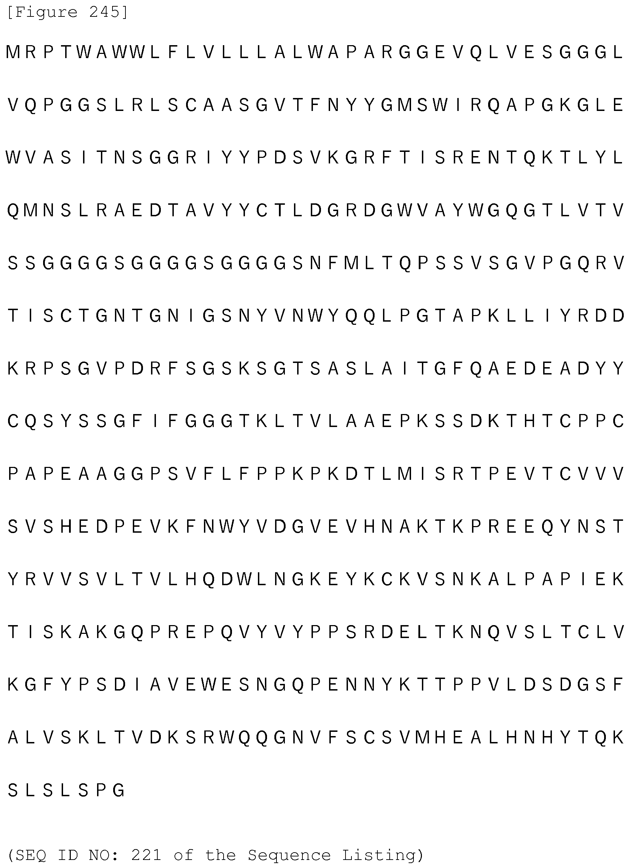

The amino acid sequences constituting v19126 obtained by expression from the respective vectors are represented by SEQ ID NOs: 219 (Figure 243), 215 (Figure 239), and 217 (Figure 241) of the Sequence Listing. The amino acid sequences constituting v19125 are represented by SEQ ID NOs: 221 (Figure 245), 215 (Figure 239), and 217 (Figure 241) of the Sequence Listing.

14)-2-3 Production of dual-type bispecific molecule

8000 μg of Polyethyleneimine max (PEImax, Polysciences) was dissolved in dispensed FreeStyle F17 medium to prepare a PEImax solution. 1000 μg of a mixture of the vectors pCL_#13557 and pCL_#13563 mixed at a ratio of 4:3, or the vectors pCL_#13561 and pCL_#13563 mixed at a ratio of 1:1, was added to an aliquot of the dispensed F17 medium to obtain a vector mixture. 1000 μg of a DNA mixture of pAKT and pGFP (both from National Research Council) mixed with already fragmented salmon sperm DNA (Sigma-Aldrich Corp.) was added to another aliquot of F17 medium to obtain a DNA solution. The PEImax solution, the vector mixture, and the DNA solution were combined, gently stirred, left standing for 5 minutes, and then added to 2 L of CHO-3E7 cell suspension. The cells were shake-cultured at 37°C for 1 day in a 5% CO2 incubator. 0.5 mM valproic acid (Sigma-Aldrich Corp.) and 0.1% (w/v) Tryptone N1 (Organotechnie) were then added. The cells were further shake-cultured at 32°C for 6 days. On day 7 after the start of the culture, the culture supernatant was recovered and filtered through a 0.2 μm filter (Sartorius Japan K.K.) to prepare a sample for evaluation.

pCL_#13557 and pCL_#13563 were used in the expression and preparation of a dual-scFv (dual)-type bispecific molecule of C3E-7034 and h2B1 (v19122). pCL_#13561 and pCL_#13563 were used in the expression and preparation of a dual-type bispecific molecule of C3E-7036 and h2B1 (v19121).

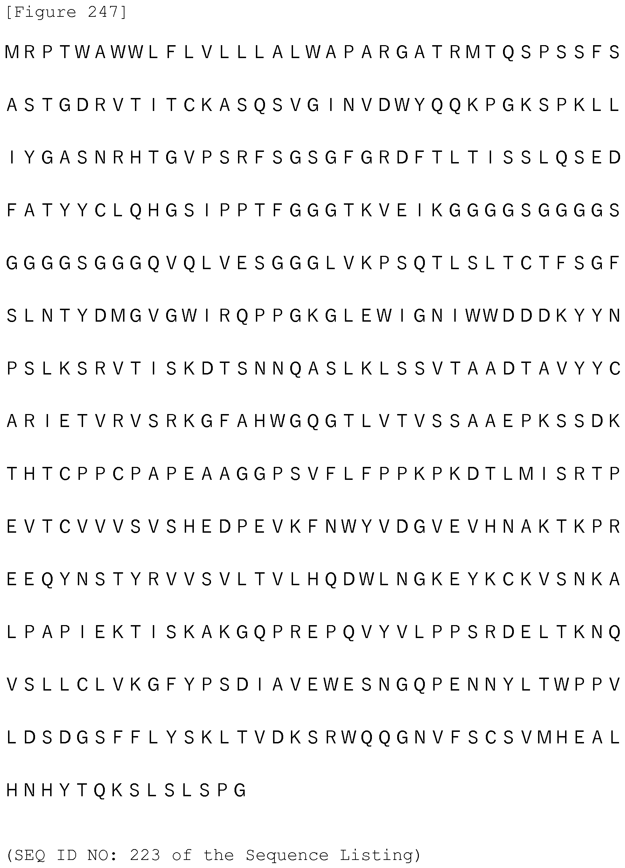

The amino acid sequences constituting v19122 obtained by expression from the respective vectors are represented by SEQ ID NOs: 219 (Figure 243) and 223 (Figure 247) of the Sequence Listing. The amino acid sequences constituting v19121 are represented by SEQ ID NOs: 221 (Figure 245) and 223 (Figure 247) of the Sequence Listing.

14)-3 Purification of Fc-containing anti-GPRC5D-anti-CD3 bispecific molecule

Each bispecific molecule was purified from the culture supernatant obtained in Example 14)-2 by two steps using protein A affinity chromatography and gel filtration chromatography.

The culture supernatant was applied to a MabSelect SuRe column (GE Healthcare Bio-Sciences Corp.) equilibrated with PBS (pH 7.4) to adsorb the bispecific molecule of interest thereon. Non-adsorbed components were removed with PBS. Then, the adsorbed component was eluted with an acetate buffer (pH 3.6). The eluted fractions were adjusted to neutral pH with a Tris buffer (pH 11), then concentrated, and applied to a gel filtration column Superdex 200 10/300 (GE Healthcare Bio-Sciences Corp.) equilibrated in advance with PBS (pH 7.4). The peak fractions obtained by gel filtration chromatography were analyzed by SDS capillary electrophoresis (LabChip-Caliper) to recover fractions corresponding to the heterodimer of interest. For the dual-type bispecific molecule, the recovered fractions were subsequently subjected to desalting column HiPrep 26/10 Desalting (GE Healthcare Bio-Sciences Corp.) equilibriated in advance with HBsor (25 mM histidine, 5% sorbitol, pH 6.0) for buffer-exchange to HBsor. The final recovered fractions were passed through a 0.2 μm filter to prepare a sterile purified sample. The identity of the purified sample was confirmed by mass spectrometry and SDS-polyacrylamide electrophoresis (SDS-PAGE) to be the correctly-assembled anti-GPRC5D-anti-CD3 bispecific molecule of interest.

(Example 15) *In vitro* activity evaluation of Fc-containing anti-GPRC5D-anti-CD3 bispecific molecule

15)-1 Evaluation of binding activity of Fc-containing anti-GPRC5D-anti-CD3 bispecific molecule by flow cytometry

15)-1-1 Binding of Fc-containing anti-GPRC5D-anti-CD3 bispecific molecule to endogenous human GPRC5D-expressing cells (KHM-1B)

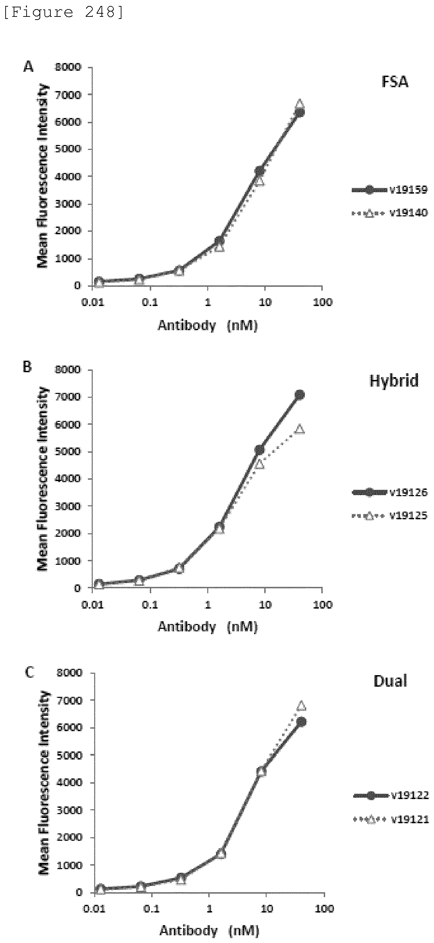

A human multiple myeloma cell line KHM-1B expressing GPRC5D was adjusted to an appropriate concentration with PBS containing 5% FBS. LIVE/DEAD Fixable Near-IR Dead Cell Stain Kit was added to the cells, which were then left standing at 4°C for 30 minutes. The cells were washed twice with PBS containing 5% FBS, then adjusted to a concentration of $1 \times 10^6$ cells/mL with PBS containing 5% FBS, inoculated in an amount of 100 μL/well to a 96-well U-bottomed microplate, and centrifuged to remove a supernatant. Each Fc-containing anti-GPRC5D-anti-CD3 bispecific molecule (prepared in Example 14) diluted with PBS containing 5% FBS was added thereto in an amount of 100 μL/well, and the plate was left standing at 4°C for 60 minutes. The cells were washed twice with PBS containing 5% FBS. Then, R-Phycoerythrin AffiniPure F(ab')2 Fragment Goat Anti-Human IgG, Fcγ Fragment Specific (Jackson ImmunoResearch Laboratories, Inc.) diluted 100-fold with PBS containing 5% FBS was added thereto in an amount of 100 μL/well, and the plate was left standing at 4°C for 1 hour. The cells were washed twice with PBS containing 5% FBS and then resuspended in PBS containing 5% FBS, followed by detection using a flow cytometer (FACSCantoI II). The data was analyzed using Flowjo. The PE fluorescence intensity of the fraction free from dead cells was plotted to a histogram. The mean fluorescence intensity (MFI) was calculated. As a result, the Fc-containing anti-GPRC5D-anti-CD3 bispecific antibodies were found to bind to human GPRC5D-expressing cells (Figure 248).

15)-1-2 Binding of Fc-containing anti-GPRC5D-anti-CD3 bispecific molecule to cynomolgus monkey GPRC5D-expressing cells

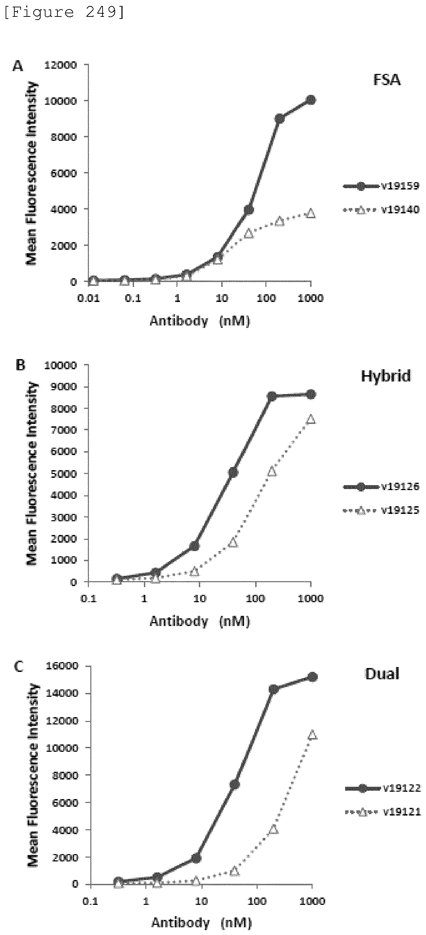

Staining and analysis were carried out in the same way as in Example 15)-1-1 using the KMS-11_cGPRC5D cells prepared in Example 5)-2-2. As shown in Figure 249, these Fc-containing anti-GPRC5D-anti-CD3 bispecific antibodies were found to bind to cynomolgus monkey GPRC5D-expressing cells.

15)-1-3 Binding of Fc-containing anti-GPRC5D-anti-CD3 bispecific molecule to human CD3 (PBMC)

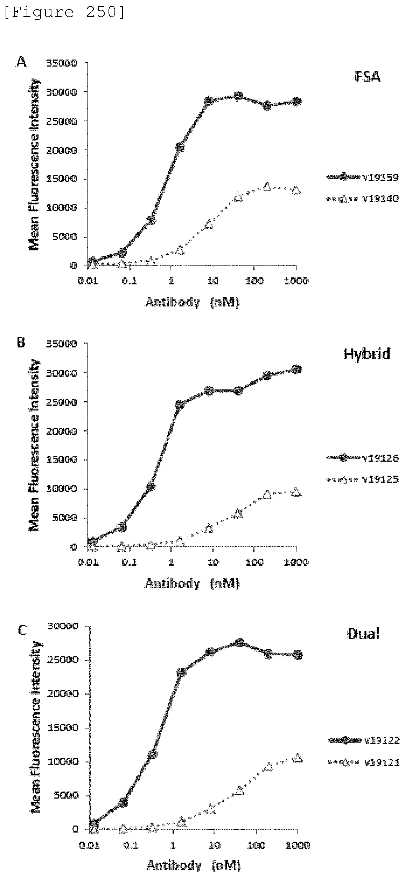

Commercially available human PBMC (Cellular Technology Limited) was adjusted to an appropriate concentration with PBS containing 5% FBS. LIVE/DEAD Fixable Near-IR Dead Cell Stain Kit (Thermo Fisher Scientific Inc.) and an anti-CD19 antibody (Beckman Coulter Inc.) were added to the cells, which were then left standing at 4°C for 30 minutes. The cells were washed twice with PBS containing 5% FBS, then adjusted to a concentration of $1 \times 10^6$ cells/mL with PBS containing 5% FBS, inoculated in an amount of 100 μL/well to a 96-well U-bottomed microplate, and centrifuged to remove a supernatant. Each Fc-containing anti-GPRC5D-anti-CD3 bispecific molecule (prepared in Example 14) diluted with PBS containing 5% FBS was added thereto in an amount of 100 μL/well, and the plate was left standing at 4°C for 60 minutes. The cells were washed twice with PBS containing 5% FBS. Then, R-Phycoerythrin AffiniPure F(ab')2 Fragment Goat Anti-Human IgG, Fcγ Fragment Specific (Jackson ImmunoResearch Laboratories, Inc.) diluted 100-fold with PBS containing 5% FBS was added thereto in an amount of 100 μL/well, and the plate was left standing at 4°C for 1 hour. The cells were washed twice with PBS containing 5% FBS and then resuspended in PBS containing 5% FBS, followed by detection using a flow cytometer (FACSCI(TM) II). The data was analyzed using Flowjo. The PE fluorescence intensity of the fraction free from dead cells was plotted to a histogram. The mean fluorescence intensity (MFI) was calculated. As a result, the Fc-containing anti-GPRC5D-anti-CD3 bispecific antibodies were found to bind to human CD3-expressing cells (Figure 250).

15)-1-4 Binding of Fc-containing anti-GPRC5D-anti-CD3 bispecific molecule to cynomolgus monkey CD3 (cynomolgus monkey PBMC)

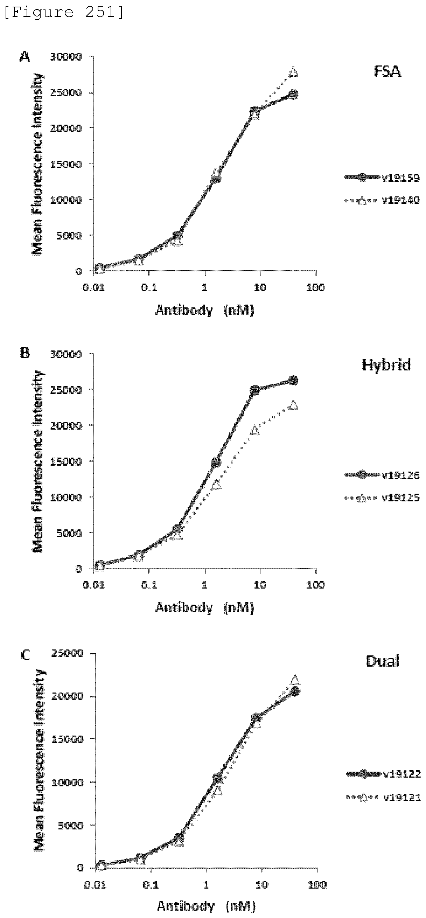

Staining and analysis were carried out in the same way as in Example 15)-1-3 using the cynomolgus monkey PBMC collected in the same way as in Example 13)-1-4. As shown in Figure 251, these Fc-containing anti-GPRC5D-anti-CD3 bispecific antibodies were found to bind to cynomolgus monkey CD3-expressing cells.

15)-2 Cytotoxic activity evaluation of Fc-containing anti-GPRC5D-anti-CD3 bispecific molecule

15)-2-1 Preparation of target cell

KHM-1B cells were prepared in the same way as in Example 13)-2-1 and used as target cells.

15)-2-2 Preparation of effector cell

Commercially available frozen PBMC (Cellular Technology Limited) was thawed at 37°C, transferred to a solution of an RPMI1640 medium containing 10% FBS supplemented with Anti-aggregate Wash reagent (Cellular Technology Limited), washed twice, then adjusted to 1.5 × 105 cells/mL with an RPMI1640 medium containing 10% FBS, and used as effector cells.

15)-2-3 Cytotoxicity assay

The KHM-1B cells obtained in Example 15)-2-1 were added at a concentration of 50 μL/well to a 96-well U-bottomed microplate. Each Fc-containing anti-GPRC5D-anti-CD3 bispecific molecule (prepared in Example 14) adjusted to varying concentrations was added thereto in an amount of 50 μL/well. The effector cells prepared in Example 15)-2-2 were added thereto in an amount of 100 μL/well. After centrifugation at room temperature at 1000 rpm for 1 minute, the cells were cultured at 37°C for 24 to 48 hours under 5% $CO_2$ conditions. A 50 μL aliquot of the supernatant was recovered into LumaPlate (PerkinElmer, Inc.) and dried at 50°C for approximately 2 hours, followed by measurement using a plate reader (TopCount; PerkinElmer, Inc.). The percentage of cells lysed was calculated according to the following expression:

$$\text{Percentage of cells lysed (\%)} = (A - B) / (C - B) \times 100$$

A: Count of sample well
B: Average of background (antibody-unsupplemented wells) counts (n = 3). 50 μL of a medium for assay was added when adding the antibody. The other procedures were the same as in the case of the sample well.
C: Average of maximum release (wells containing target cells lysed in a surfactant) counts (n = 3). 50 μL of a medium for assay was added when adding the antibody. 100 μL of the surfactant was added, and the 50 μL aliquot was transferred to LumaPlate, as with the sample well, and assayed.

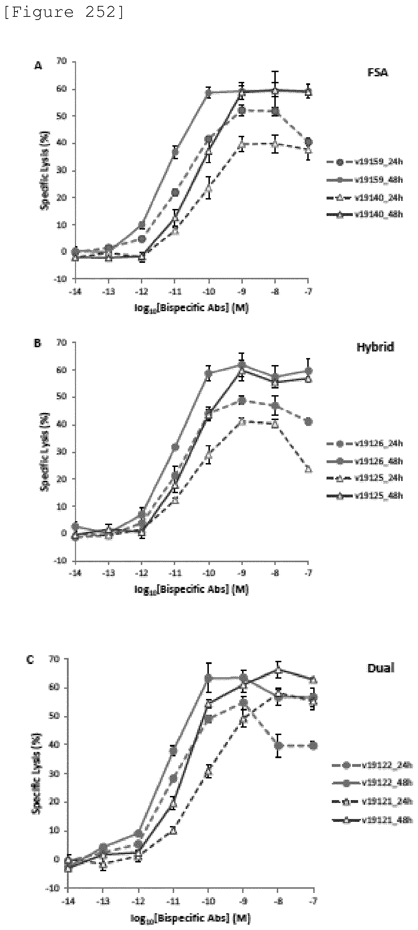

As shown in Figure 252, these Fc-containing anti-GPRC5D-anti-CD3 bispecific antibodies exhibited cytotoxic activity against the KHM-1B cells.

(Example 16) *In vivo* activity evaluation of Fc-containing anti-GPRC5D-anti-CD3 bispecific molecule

16)-1 *In vivo* activity in co-grafting model of human PBMC and cancer cells

A human multiple myeloma cell line KHM-1B (JCRB) and human PBMC (Cellular Technology Limited) were each adjusted to 5 × $10^7$ cells/mL with PBS containing 50% Matrigel (Corning Inc.) and subcutaneously co-grafted in an amount of 0.1 mL to each NOD-Scid mouse (female, 5 weeks old). After grafting, the mice were grouped, and each anti-GPRC5D-anti-CD3 bispecific molecule was administered (0.1 mg/kg) into the tail veins. The administration was carried out three times every day from the grafting day (day 0) to day 2. The major axis (mm) and minor axis (mm) of the tumors were measured over time from 1 week later (day 7) using an electronic digital caliper. The estimated tumor volume was calculated according to the following expression:

$$\text{Estimated tumor volume (mm}^3\text{)} = \text{Average estimated tumor volume of each individual}$$

$$\text{Estimated tumor volume of each individual} = \text{Major axis} \times \text{Minor axis}^2 / 2$$

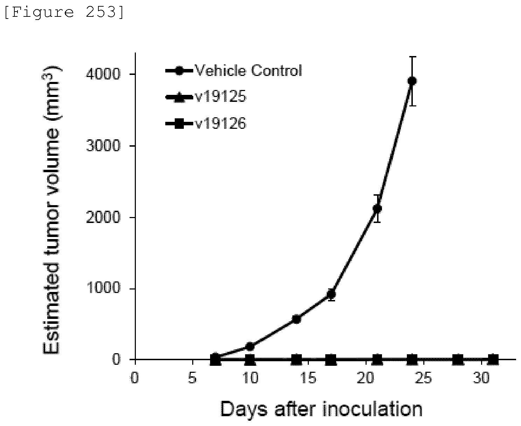

Anti-tumor efficacy was confirmed in each anti-GPRC5D-anti-CD3 bispecific molecule administration group (Figure 253).

16)-2 *In vivo* activity evaluation in human PBMC-migration model

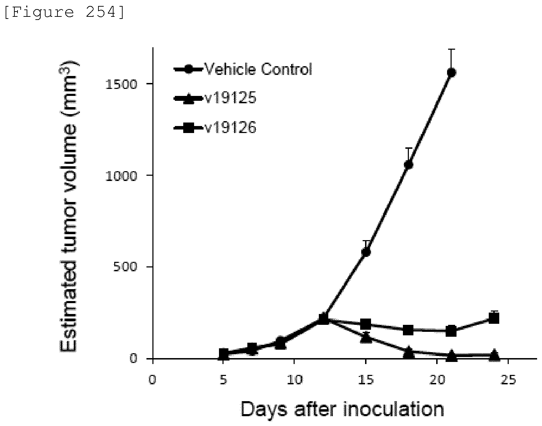

Human PBMC was adjusted to 5 × $10^7$ cells/mL with PBS and grafted in an amount of 0.2 mL into the tail vein of each NOG mouse (female, 6 weeks old) (day -4). On day 0, KHM-1B was adjusted to 3 × $10^7$ cells/mL each with PBS containing 50% Matrigel and subcutaneously grafted in an amount of 0.1 mL to the NOG mouse. When the estimated tumor volume of the mouse reached approximately 200 $mm^3$ (day 12), the mice were grouped according to their tumor volumes, and each anti-GPRC5D-anti-CD3 bispecific molecule was administered (1 mg/kg) into the tail veins. The administration was carried out on days 12, 15, and 18. The major axis (mm) and minor axis (mm) of the tumors were measured over time using an electronic digital caliper. The estimated tumor volume was calculated. As shown in the figure, tumor regression was exhibited in each anti-GPRC5D-anti-CD3 bispecific antibody molecule administration group. Particularly strong tumor regression efficacy was confirmed in the v19125 treatment group (Figure 254).

(Example 17) Preparation of CDR-modified hybrid-type anti-GPRC5D-anti-CD3 bispecific molecule

17)-1 Preparation of CDR-modified hybrid-type anti-GPRC5D-anti-CD3 bispecific molecule expression vector

17)-1-1 Preparation of CDR-modified hybrid-type anti-GPRC5D-anti-CD3 bispecific molecule (C5D-0004, C5D-0005, and C5D-0006) expression vectors

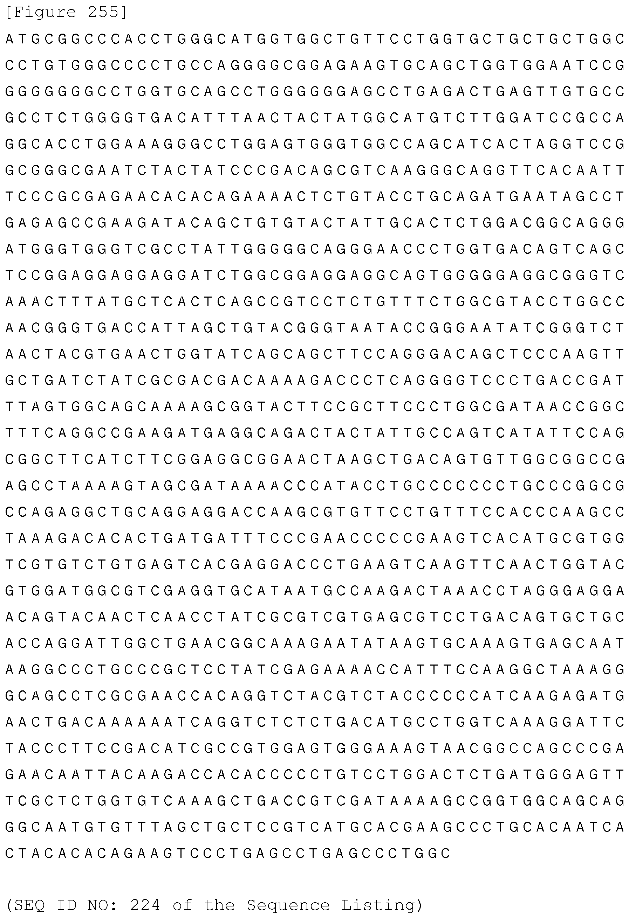

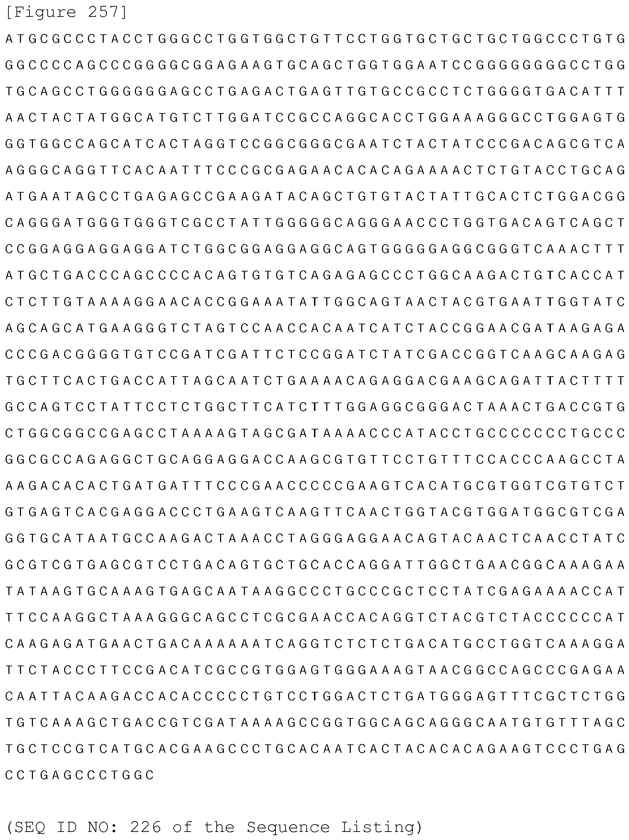

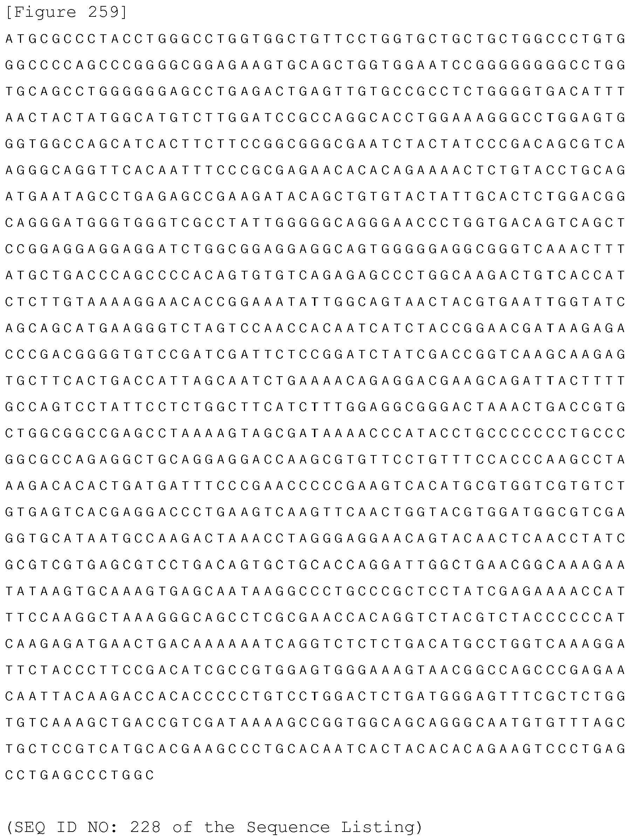

Among the hybrid-type bispecific molecule (v19125) expression vectors constructed in Example 14)-1-2, pCL_#13561 encoding the humanized anti-CD3 scFv-Fc was used as a template in site-directed mutagenesis to prepare a CDR-modified vector pC3E-8015 modifying Asn53 of H chain CDR2 to Arg. Likewise, among the hybrid-type bispecific molecule (v19126) expression vectors, pCL_#13557 encoding the humanized anti-CD3 scFv-Fc was used as a template in site-directed mutagenesis to prepare a CDR-modified vector pC3E-8017 modifying Asn53 of H chain CDR2 to Arg and L chain Asp52 to Asn. Also, pCL_#13557 was used as a template in site-directed mutagenesis to prepare a CDR-modified vector pC3E-8018 modifying Asn53 of H chain CDR2 to Ser and L chain Asp52 to Asn.
The ORF sequences of pC3E-8015, pC3E-8017, and pC3E-8018 are represented by SEQ ID NO: 224 (Figure 255), SEQ ID NO: 226 (Figure 257), and SEQ ID NO: 228 (Figure 259), respectively, of the Sequence Listing.

17)-1-2 Preparation of C-terminally Lys-added CDR-modified hybrid-type anti-GPRC5D-anti-CD3 bispecific molecule (C5D-0014, C5D-0015, and C5D-0016) expression vectors

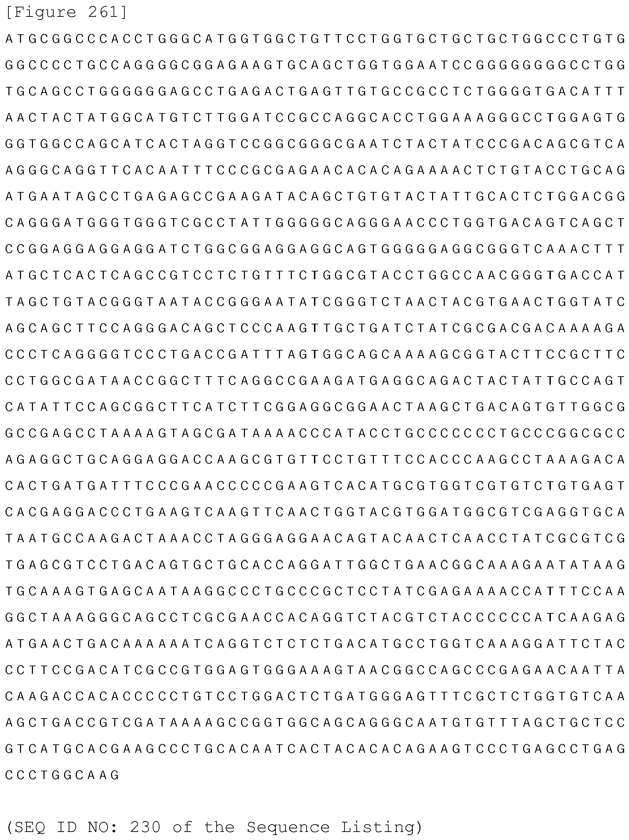

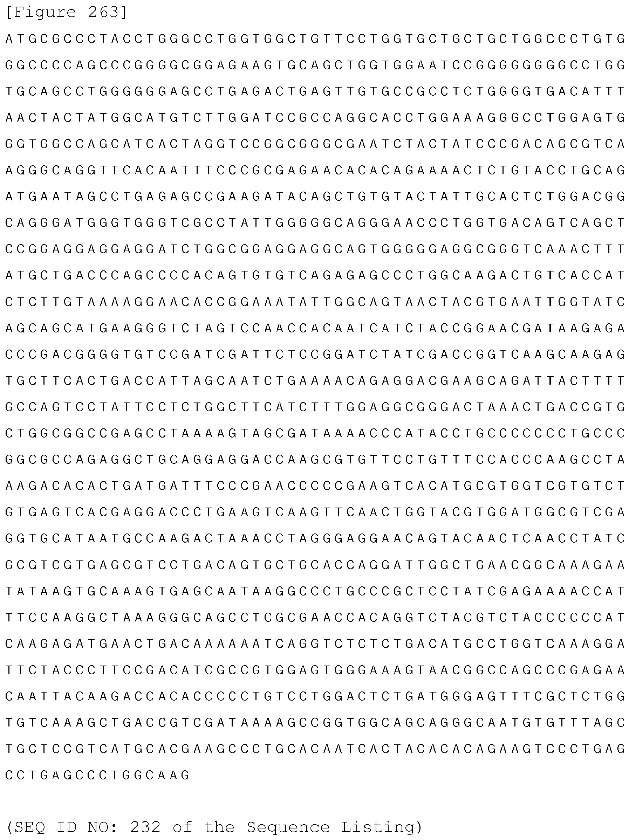

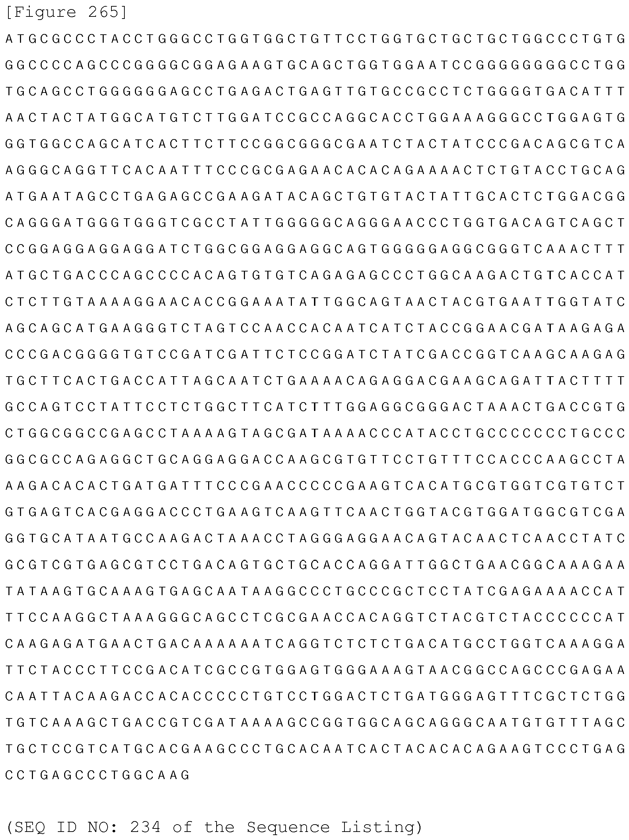

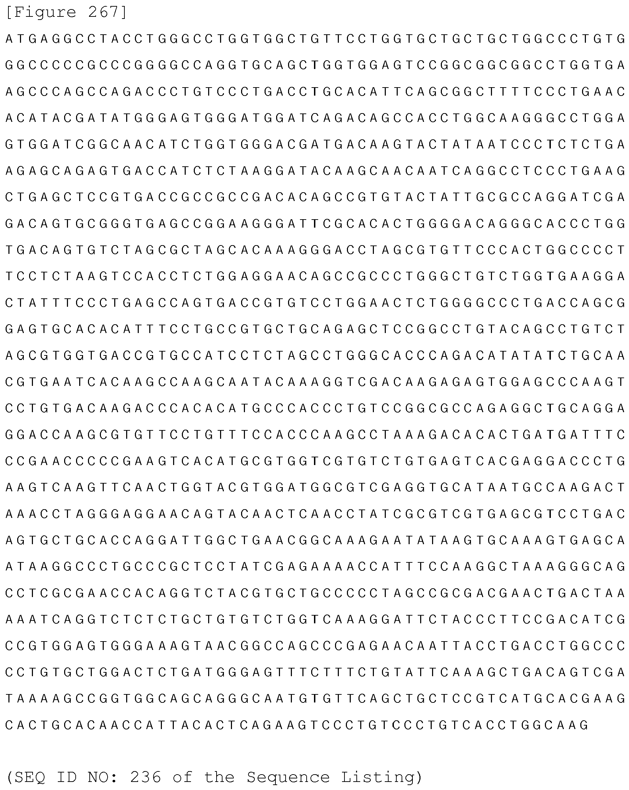

Among the hybrid-type bispecific molecule (C5D-0004) expression vectors constructed in Example 17)-1-1, pC3E-8015 encoding CDR-modified humanized anti-CD3 scFv-Fc was used as a template in site-directed mutagenesis to prepare a K-added CDR-modified vector pC3E-8025 containing Lys inserted to the C terminus of Fc. Likewise, pC3E-8017 and pC3E-8018 were used as a template in site-directed mutagenesis to prepare K-added CDR-modified vectors pC3E-8027 and pC3E-8028 containing Lys inserted in the C terminus of Fc.
Among the hybrid-type bispecific molecule (v19125 and v19126) expression vectors constructed in Example 14)-1-2, pCL_#13555 encoding the anti-GPRC5D Fab-Fc was used as a template in site-directed mutagenesis to prepare a K-added vector pTAA_#2 containing Lys inserted in the C terminus of Fc.
The ORF sequences of pC3E-8025, pC3E-8027, pC3E-8028, and pTAA_#2 are represented by SEQ ID NO: 230 (Figure 261), SEQ ID NO: 232 (Figure 263), SEQ ID NO: 234 (Figure 265), and SEQ ID NO: 236 (Figure 267), respectively, of the Sequence Listing.

17)-2 Production of CDR-modified hybrid-type anti-GPRC5D-anti-CD3 bispecific molecule

CHO-3E7 cells were subcultured and cultured according to the manual (National Research Council Canada, Raymond C. et al., Methods (2011) 55 (1), 44-51). The culture medium of the CHO-3E7 cells in the logarithmic growth phase was diluted to 2 × 10^6 cells/mL with BalanCD Transfectory CHO (Irvine Scientific) containing 4 mM glutamine and used in the production of various bispecific antibodies.
ExpiCHO-S cells were subcultured and cultured according to the manual (Thermo Fisher Scientific Inc.). The culture medium of the ExpiCHO-S cells in the logarithmic growth phase was diluted to 6 × 10^6 cells/mL with ExpiCHO Expression Medium (Thermo Fisher Scientific Inc.) and used in the production of various bispecific antibodies.

17)-2-1 Production of CDR-modified hybrid-type anti-GPRC5D-anti-CD3 bispecific antibodies (C5D-0004, C5D-0005, and C5D-0006)

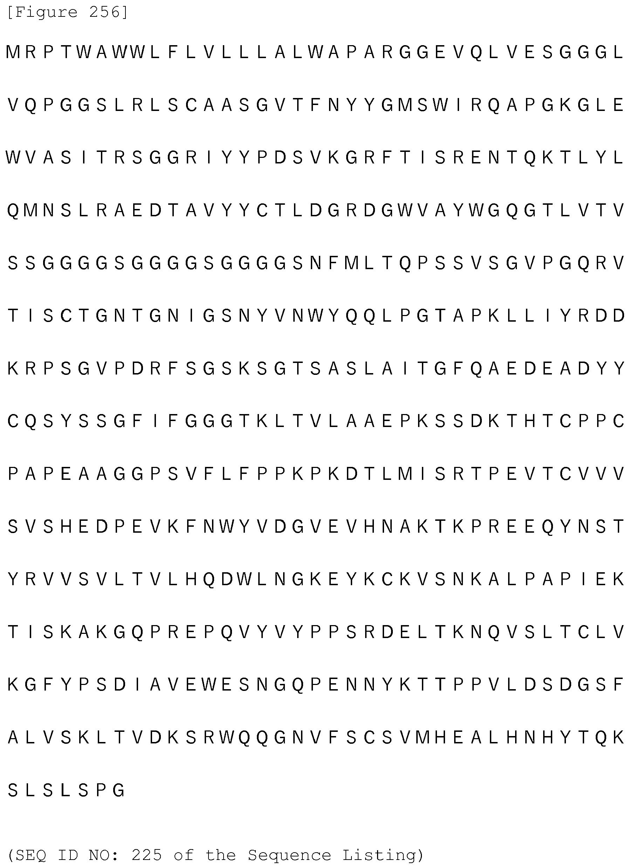

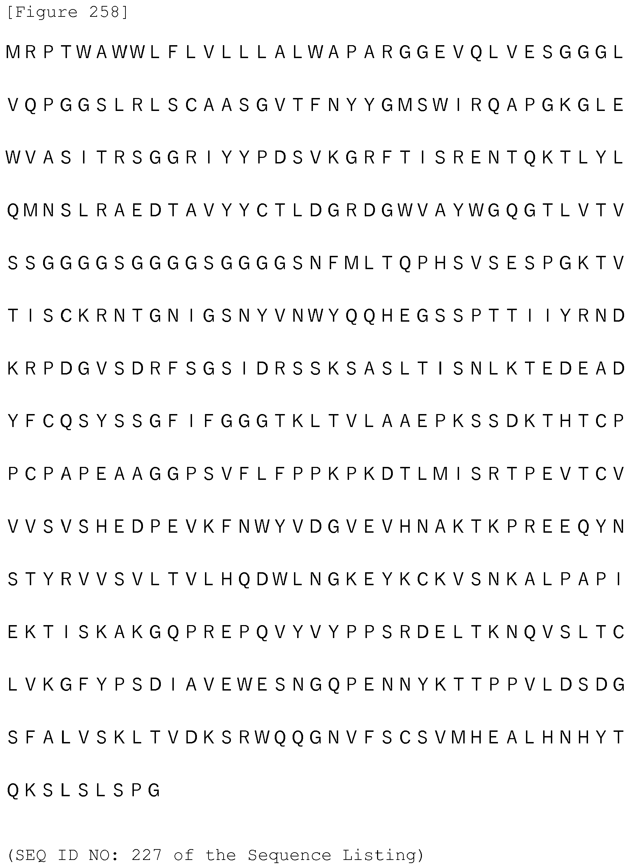

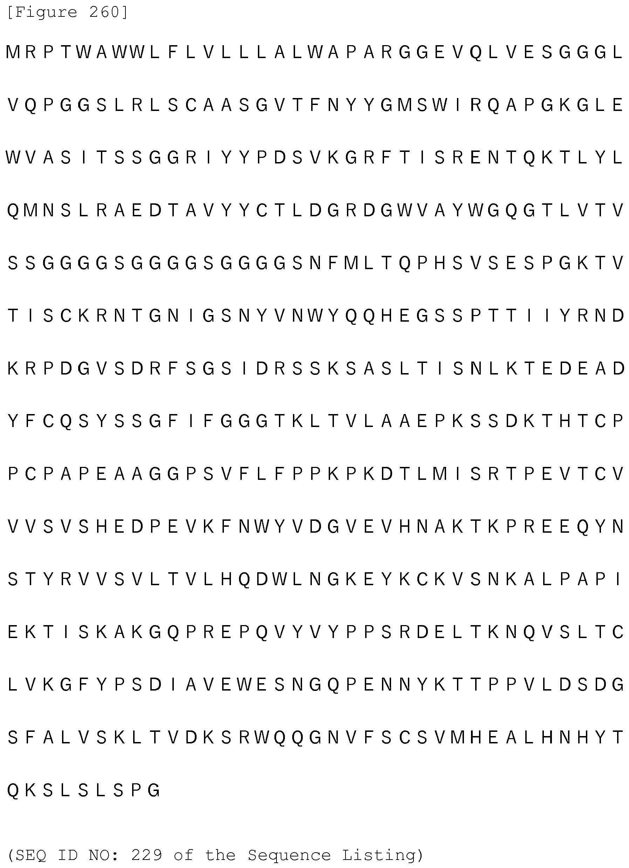

The hybrid-type anti-GPRC5D-anti-CD3 bispecific antibodies C5D-0004, C5D-0005, and C5D-0006 were expressed by culture using the ExpiCHO-S cells as a host. A method for transfecting the cells with the expression vectors and culture conditions were all carried out according to the manual attached to the product (Thermo Fisher Scientific Inc.). The culture was performed on a scale of 750 mL, and conditions of the Max titer protocol described in the manual were used for feed addition and a culture temperature. $O^n$ the 13th day after the start of culturing, the culture supernatant was recovered and filtered through a 0.2 μm filter (Sartorius Japan K.K.) to prepare a sample for evaluation.
A hybrid-type bispecific molecule C5D-0004 was obtained from the combination of pC3E-8015, pCL_#13555, and pCL_#12123. A hybrid-type bispecific molecule C5D-0005 was obtained from the combination of pC3E-8017, pCL_#13555, and pCL_#12123. A hybrid-type bispecific molecule C5D-0006 was obtained from the combination of pC3E-8018, pCL_#13555, and pCL_#12123.
The amino acid sequences constituting C5D-0004 obtained by expression from the respective vectors are represented by SEQ ID NOs: 225 (Figure 256), 215 (Figure 239), and 217 (Figure 241) of the Sequence Listing. The amino acid sequences constituting C5D-0005 are represented by SEQ ID NOs: 227 (Figure 258), 215 (Figure 239), and 217 (Figure 241) of the Sequence Listing. The amino acid sequences constituting C5D-0006 are represented by SEQ ID NOs: 229 (Figure 260), 215 (Figure 239), and 217 (Figure 241) of the Sequence Listing.

17)-2-2 Production of C-terminally Lys-added CDR-modified hybrid-type anti-GPRC5D-anti-CD3 bispecific antibodies (C5D-0014, C5D-0015, and C5D-0016)

800 μg of PEImax (Polysciences) was dissolved in 3 mL of an Opti-PRO SFM medium (Thermo Fisher Scientific Inc.) to prepare a PEImax solution. 100 μg of a vector mixture of pC3E-_8025, pTAA_#2, and pCL_#12123 mixed at a ratio of 1:1:1.5 and 100 μg of a DNA mixture of pAKT and pGFP mixed with already fragmented salmon sperm DNA was added to 3 mL of an Opti-PRO SFM medium. The PEImax solution, the vector mixture, and the DNA solution were combined, gently stirred, left standing for 5 minutes, and then added to 200 mL of the CHO-3E7 cell culture medium. The cells were shake-cultured at 37°C for 1 day in a 5% $CO_2$ incubator. Then, 22 mL of Transfectory Supplement (Irvine Scientific), 480 μL of Anti clumping supplement (Thermo Fisher Scientific Inc.), and 500 μL of valproic acid (Sigma-Aldrich Corp.) were added thereto. The cells were further shake-cultured at 32°C for 9 days. $O^n$ the 10th day after the start of culturing, the culture supernatant was recovered and filtered through a 0.2 μm filter (Sartorius Japan K.K.) to prepare a sample for evaluation.

A hybrid-type bispecific molecule C5D-0014 was obtained from the combination of pC3E-8025, pTAA_#2, and pCL_12123. A hybrid-type bispecific molecule C5D-0015 was obtained from the combination of pC3E-8027, pTAA_#2, and pCL_#12123. A hybrid-type bispecific molecule C5D-0016 was obtained from combination of pC3E-8028, pTAA_#2, and pCL_#12123.

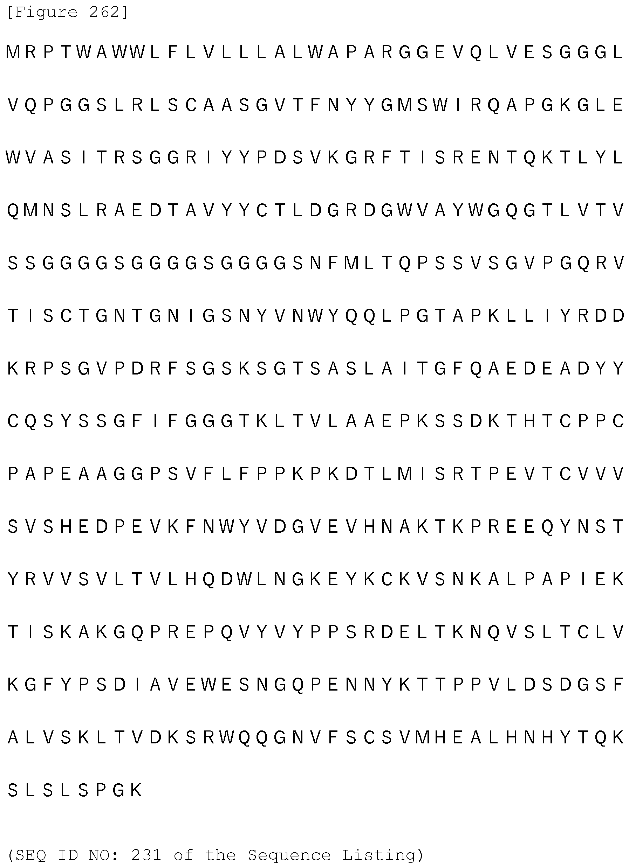

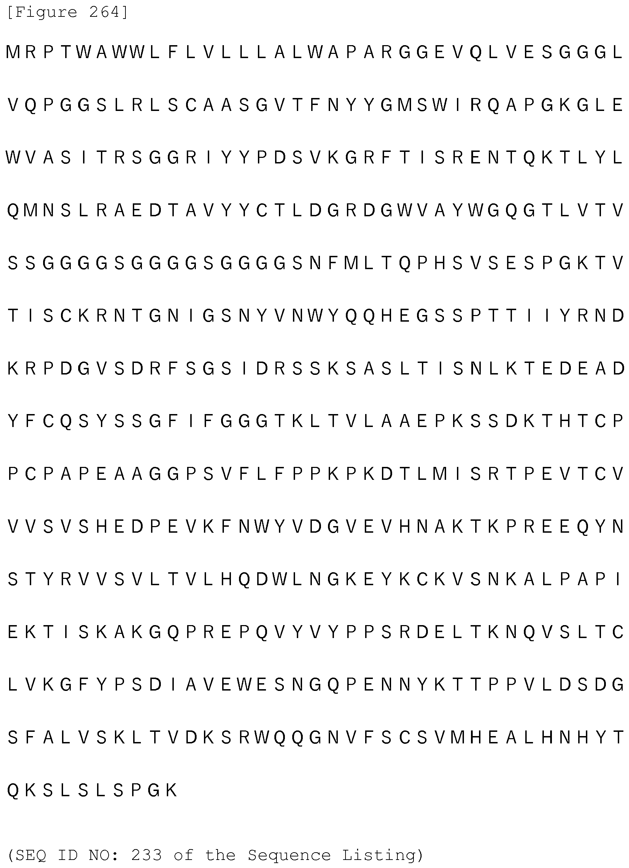

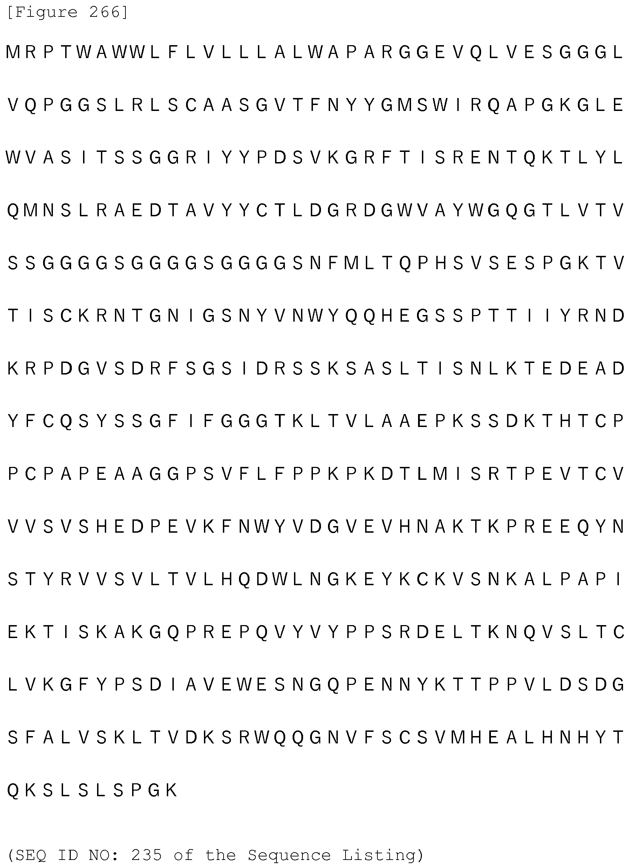

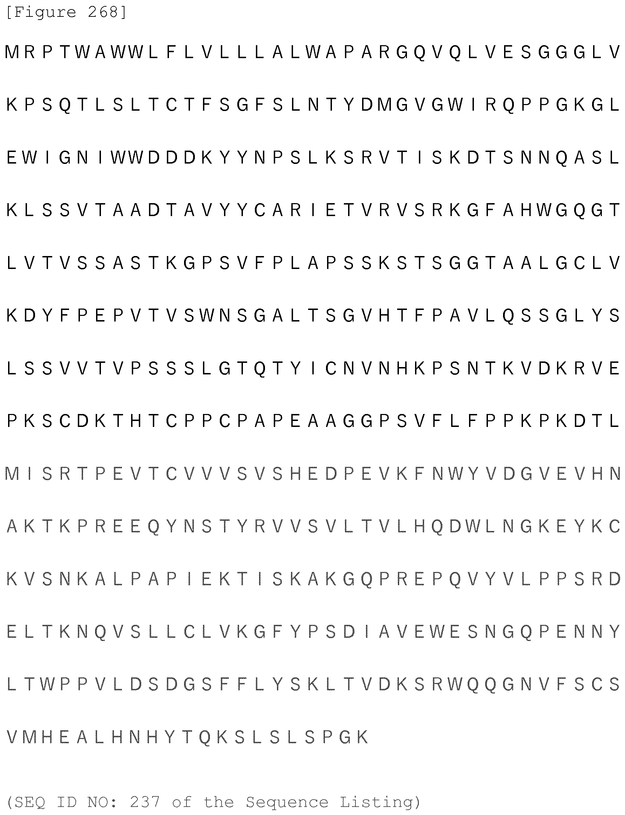

The amino acid sequences constituting C5D-0014 obtained by expression from the respective vectors are represented by SEQ ID NOs: 231 (Figure 262), 237 (Figure 268), and 217 (Figure 241) of the Sequence Listing. The amino acid sequences constituting C5D-0015 are represented by SEQ ID NOs: 233 (Figure 264), 237 (Figure 268), and 217 (Figure 241) of the Sequence Listing. The amino acid sequences constituting C5D-0016 are represented by SEQ ID NOs: 235 (Figure 266), 237 (Figure 268), and 217 (Figure 241) of the Sequence Listing.

17)-3 Purification of CDR-modified hybrid-type anti-GPRC5D-anti-CD3 bispecific antibodies

17)-3-1 Purification of CDR-modified hybrid-type anti-GPRC5D-anti-CD3 bispecific antibodies (C5D-0004, C5D-0005, and C5D-0006)

Each bispecific molecule was purified from the culture supernatant obtained in 17)-2-1 by three steps using protein A affinity chromatography, hydroxyapatite chromatography, and cation-exchange chromatography. Specifically, the culture supernatant was applied to a MabSelect SuRe column (GE Healthcare Bio-Sciences Corp.) equilibrated with PBS (pH 7.4) to adsorb the bispecific molecule of interest. Non-adsorbed components were removed with PBS. Then, the adsorbed component was eluted with a 100 mM acetate buffer (pH 3.5). The eluted fractions were immediately adjusted to neutral pH with a Tris buffer (pH 9.0), then dialyzed in a solution of 50 mM HEPES, 10 mM potassium phosphate, and a 100 mM sodium chloride, and applied to a hydroxyapatite column Bio-Scale CHT Type-I (Bio-Rad Laboratories, Inc.). The adsorbed bispecific molecule of interest was eluted by changing the sodium chloride concentration in the solvent from 0.1 M to 1 M by a linear concentration gradient. The obtained peak fractions were analyzed by SDS-PAGE to recover fractions corresponding to the bispecific molecule of interest. Next, the recovered fractions were buffer-replaced with 50 mM HEPES (pH 8.0) and a 20 mM sodium chloride solution and then applied to a cation-exchange column Mono S (GE Healthcare Bio-Sciences Corp.). The adsorbed bispecific molecule of interest was eluted by changing the sodium chloride concentration in the solvent from 20 mM to 1 M by a linear concentration gradient. The obtained peak fractions were analyzed by SDS-PAGE to recover fractions corresponding to the bispecific molecule of interest. The final recovered fractions were dialyzed by HBsor (25 mM histidine, 5% sorbitol, pH 6.0) and filtered through a filter to prepare a purified sample. The purified sample was confirmed by mass spectrometry, SDS-PAGE, and SEC analysis to unmistakably be the anti-GPRC5D-anti-CD3 bispecific molecule of interest.

17)-3-2 Purification of C-terminally Lys-added CDR-modified hybrid-type anti-GPRC5D-anti-CD3 bispecific antibodies (C5D-0014, C5D-0015, and C5D-0016)

Each bispecific molecule was purified from the culture supernatant obtained in 17)-2-2 in two steps using protein A affinity chromatography and hydroxyapatite chromatography. Specifically, the culture supernatant was applied to a MabSelect SuRe column (GE Healthcare Bio-Sciences Corp.) equilibrated with PBS (pH 7.4) to adsorb the bispecific molecule of interest. Non-adsorbed components were removed with PBS. Then, the adsorbed component was eluted with a 100 mM acetate buffer (pH 3.0). The eluted fractions were immediately adjusted to neutral pH with a Tris buffer (pH 9.5), then dialyzed in a solution of 50 mM HEPES, 10 mM potassium phosphate, and 100 mM sodium chloride, and applied to a hydroxyapatite column Bio-Scale CHT Type-I (Bio-Rad Laboratories, Inc.). The adsorbed bispecific molecule of interest was eluted by changing the sodium chloride concentration in the solvent from 0.1 M to 1 M by a linear concentration gradient. The obtained peak fractions were analyzed by SDS-PAGE to recover fractions corresponding to the bispecific molecule of interest. The final recovered fractions were dialyzed with HBsor (25 mM histidine, 5% sorbitol, pH 6.0) and filtered through a filter to prepare a purified sample. The purified sample was confirmed by mass spectrometry, SDS-PAGE, and SEC analysis to unmistakably be the anti-GPRC5D-anti-CD3 bispecific molecule of interest.

(Example 18) *In vitro* activity evaluation of C-terminally Lys-added CDR-modified hybrid-type and CDR-modified hybrid-type anti-GPRC5D-anti-CD3 bispecific antibodies

18)-1 Binding activity evaluation of C-terminally Lys-added CDR-modified hybrid-type and CDR-modified hybrid-type anti-GPRC5D-anti-CD3 bispecific antibodies by flow cytometry

18)-1-1 Binding of C-terminally Lys-added CDR-modified hybrid-type and CDR-modified hybrid-type anti-GPRC5D-anti-CD3 bispecific antibodies to endogenous human GPRC5D-expressing cells (KHM-1B)

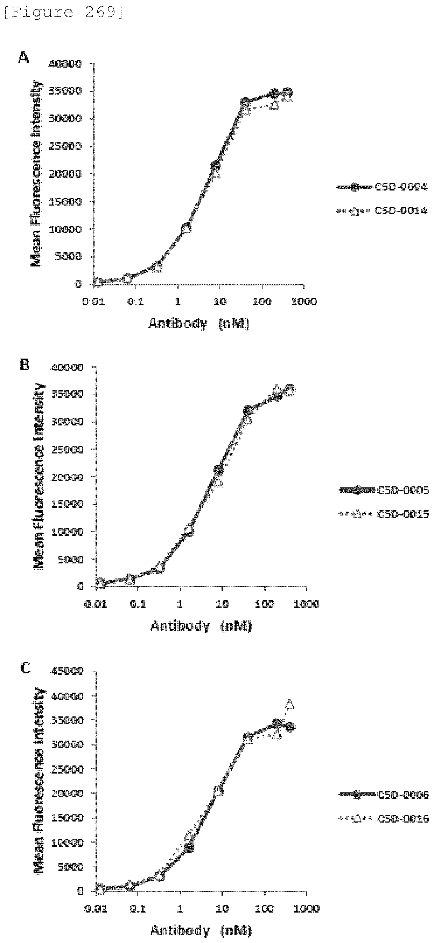

Cell preparation, staining, and analysis were carried out in the same way as in Example 15)-1-1. As a result, the anti-GPRC5D-anti-CD3 bispecific antibodies were found to bind to human GPRC5D-expressing cells (Figure 269).

18)-1-2-2 Binding of C-terminally Lys-added CDR-modified hybrid-type and CDR-modified hybrid-type anti-GPRC5D-anti-CD3 bispecific antibodies to cynomolgus monkey GPRC5D-expressing cells

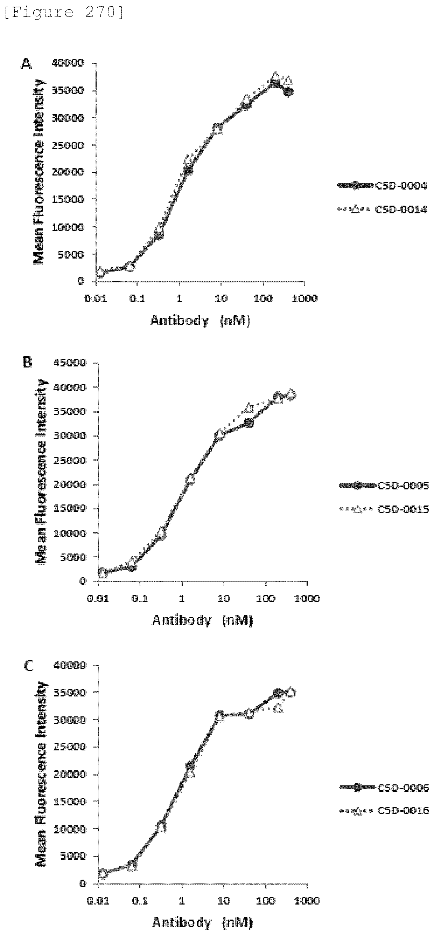

Cell preparation, staining, and analysis were carried out in the same way as in Example 15)-1-2. As shown in Figure 270, these anti-GPRC5D-anti-CD3 bispecific antibodies were found to bind to cynomolgus monkey GPRC5D-expressing cells.

18)-1-3 Binding of C-terminally Lys-added CDR-modified hybrid-type and CDR-modified hybrid-type anti-GPRC5D-anti-CD3 bispecific antibodies to human CD3 (PBMC)

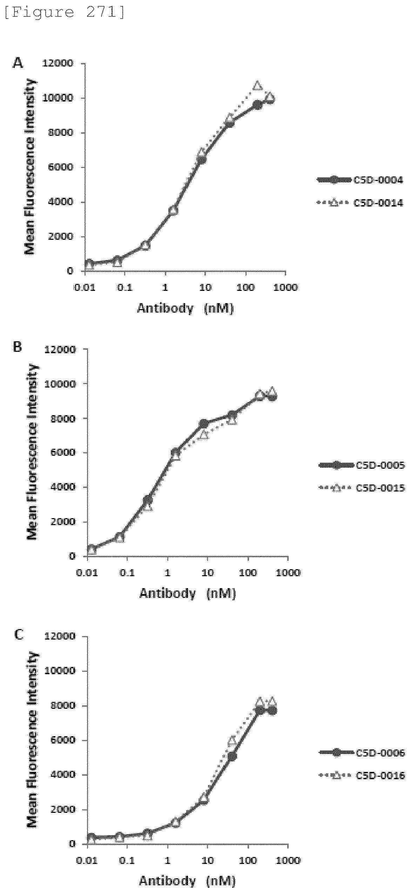

Cell preparation, staining, and analysis were carried out in the same way as in Example 15)-1-3. As a result, the anti-GPRC5D-anti-CD3 bispecific antibodies were found to bind to human CD3-expressing cells (Figure 271).

18)-1-4 Binding of C-terminally Lys-added CDR-modified hybrid-type and CDR-modified hybrid-type anti-GPRC5D-anti-CD3 bispecific antibodies to cynomolgus monkey CD3 (PBMC)

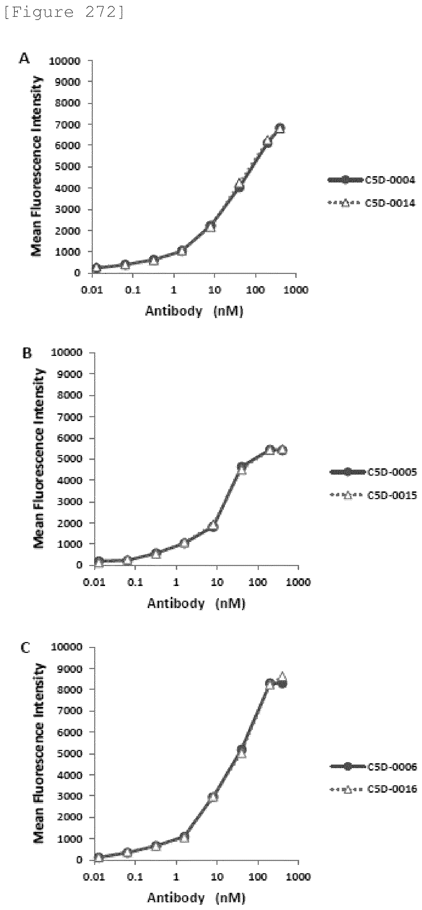

Cell preparation, staining, and analysis were carried out in the same way as in Example 15)-1-4. As shown in Figure 272, these anti-GPRC5D-anti-CD3 bispecific antibodies were found to bind to cynomolgus monkey CD3-expressing cells.

18)-2 Cytotoxic activity evaluation of C-terminally Lys-added CDR-modified hybrid-type and CDR-modified hybrid-type anti-GPRC5D-anti-CD3 bispecific antibodies

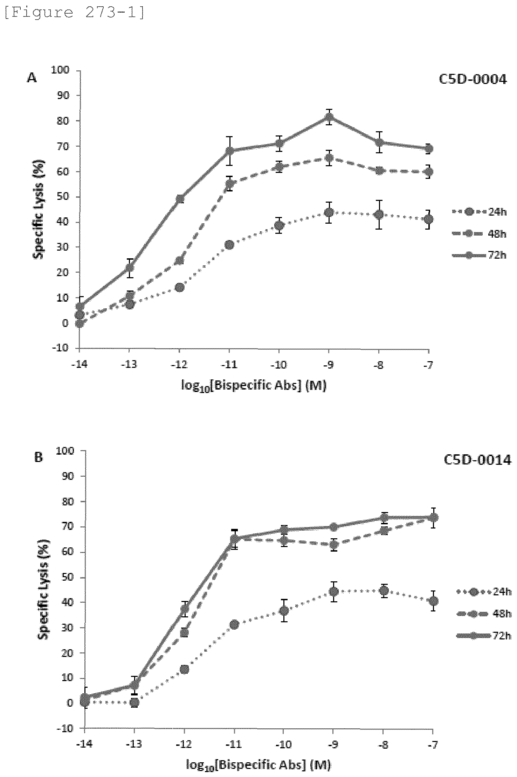

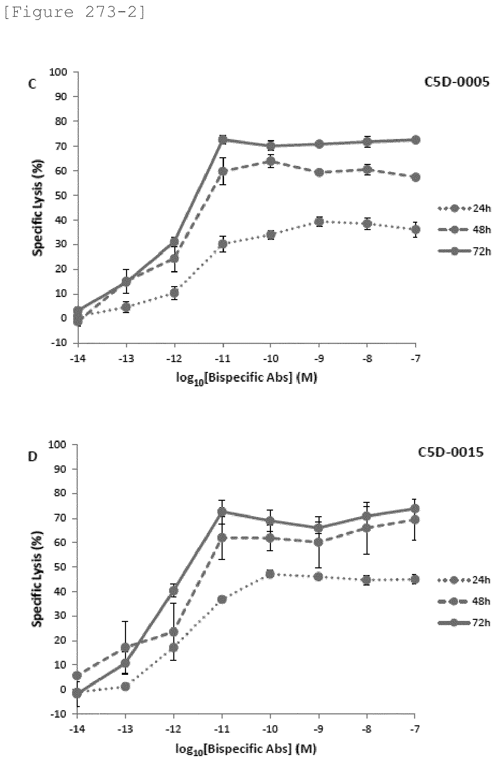

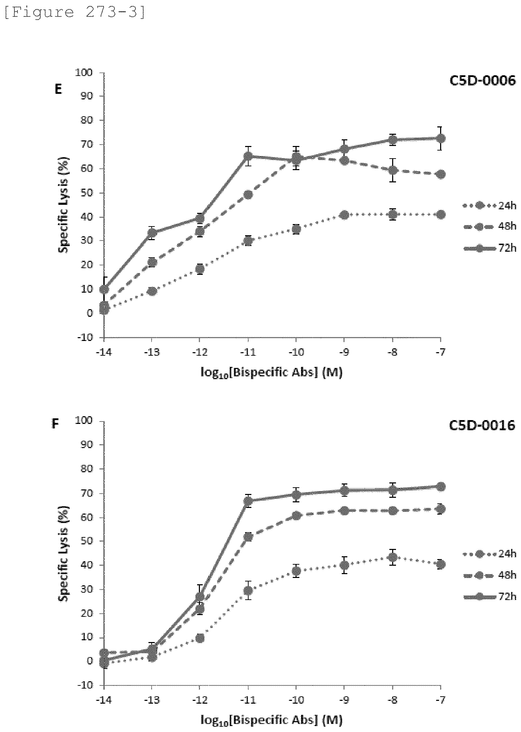

The cytotoxic activity assay was carried out in the same way as in Example 15)-2-3 except that the culture time was 24 to 72 hours. As shown in Figure 273, these anti-GPRC5D-anti-CD3 bispecific antibodies exhibited cytotoxic activity against KHM-1B cells.

(Example 19) *In vivo* activity evaluation of CDR-modified hybrid-type, and C-terminally Lys-added CDR-modified hybrid-type anti-GPRC5D-anti-CD3 bispecific molecule

19)-1 *In vivo* activity in co-grafting model of human PBMC and cancer cells

KHM-1B and human PBMC were each adjusted to $5 \times 10^7$ cells/mL with PBS containing 50% Matrigel and subcutaneously co-grafted in an amount of 0.1 mL to each NOD-Scid mouse (female, 5 weeks old). After grafting, the mice were grouped, and each anti-GPRC5D-anti-CD3 bispecific molecule was administered (1 $\mu$g/kg) into the tail veins. The administration was carried out three times every day from the grafting day (day 0) to day 2. The major axis (mm) and minor axis (mm) of the tumor were measured over time after one week (day 7) using an electronic digital caliper. The estimated tumor volume was calculated according to the following expression:

Estimated tumor volume ($mm^3$) = Average estimated tumor volume of each individual

$$\text{Estimated tumor volume of each individual} = \text{Major axis} \times \text{Minor axis}^2 / 2$$

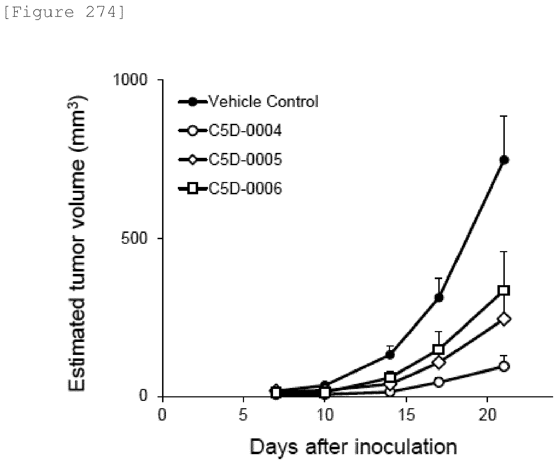

Anti-tumor efficacy was confirmed in each anti-GPRC5D-anti-CD3 bispecific molecule administration group (Figure 274).

19)-2 *In vivo* activity in human PBMC migration model

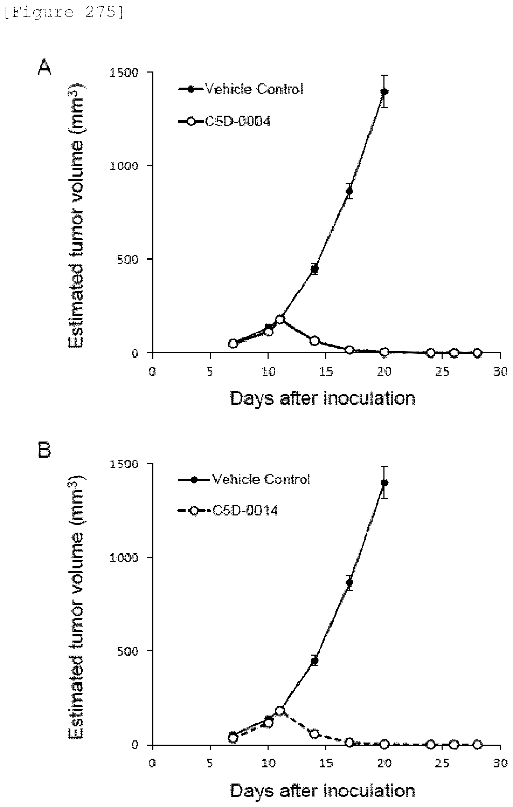

Human PBMC was adjusted to $5 \times 10^7$ cells/mL with PBS and grafted in an amount of 0.2 mL into the tail vein of each NOG mouse (female, 6 weeks old) (day -4). On day 0, KHM-1B was adjusted to $3 \times 10^7$ cells/mL with PBS containing 50% Matrigel and subcutaneously grafted in an amount of 0.1 mL to the NOG mouse. When the estimated tumor volume of the mouse reached approximately 200 $mm^3$ (day 11), the mice were grouped according to their tumor volumes, and each anti-GPRC5D-anti-CD3 bispecific molecule was administered (1 mg/kg) into the tail veins. The administration was carried out on days 11, 14, and 17. The major axis (mm) and minor axis (mm) of the tumor were measured over time using an electronic digital caliper. The estimated tumor volume was calculated. As shown in the figure, tumor regression was confirmed for C5D-0004 (Figure 275A) and C5D-0014 (Figure 275B).

[Sequence Listing Free Text]

SEQ ID NO: 1: Amino-terminal sequence of human GPRC5D (Figure 2)
SEQ ID NO: 2: Amino-terminal sequence of human GPRC5D (Figure 3)
SEQ ID NO: 3: Primer for the PCR amplification of the variable region-encoding cDNA of the heavy chain gene of 2A4
SEQ ID NO: 4: Nucleotide sequence of a cDNA encoding the heavy chain variable region of 2A4 (Figure 8)
SEQ ID NO: 5: Amino acid sequence of the heavy chain variable region of 2A4 (Figure 9)
SEQ ID NO: 6: Nucleotide sequence of a cDNA encoding the heavy chain variable region of 2B1 (Figure 10)
SEQ ID NO: 7: Amino acid sequence of the heavy chain variable region of 2B1 (Figure 11)
SEQ ID NO: 8: Nucleotide sequence of a cDNA encoding the heavy chain variable region of 7B4 (Figure 12)
SEQ ID NO: 9: Amino acid sequence of the heavy chain variable region of 7B4 (Figure 13)
SEQ ID NO: 10: Primer for the PCR amplification of the variable region-encoding cDNA of the light chain gene of 2A4
SEQ ID NO: 11: Nucleotide sequence of a cDNA encoding the light chain variable region of 2A4 (Figure 14)
SEQ ID NO: 12: Amino acid sequence of the light chain variable region of 2A4 (Figure 15)
SEQ ID NO: 13: Nucleotide sequence of a cDNA encoding the light chain variable region of 2B1 (Figure 16)
SEQ ID NO: 14: Amino acid sequence of the light chain variable region of 2B1 (Figure 17)
SEQ ID NO: 15: Nucleotide sequence of a cDNA encoding the light chain variable region of 7B4 (Figure 18)
SEQ ID NO: 16: Amino acid sequence of the light chain variable region of 7B4 (Figure 19)
SEQ ID NO: 17: DNA fragment comprising a DNA sequence encoding the amino acids of a human $\kappa$ chain secretory signal sequence and a human $\kappa$ chain constant region (Figure 20)
SEQ ID NO: 18: Primer F for a light chain expression vector (Figure 21)
SEQ ID NO: 19: Primer R for a light chain expression vector (Figure 22)
SEQ ID NO: 20: DNA fragment comprising a DNA sequence encoding the amino acids of a human heavy chain signal sequence and a human IgG1 constant region (Figure 23)
SEQ ID NO: 21: Nucleotide sequence of the light chain of human chimeric 2A4 (c2A4) (Figure 24)
SEQ ID NO: 22: Amino acid sequence of the light chain of human chimeric 2A4 (c2A4) (Figure 25)
SEQ ID NO: 23: Primer set F for the light chain of human chimeric 2A4 (Figure 26)
SEQ ID NO: 24: Primer set R for the light chain of human chimeric 2A4 (Figure 27)
SEQ ID NO: 25: Nucleotide sequence of the heavy chain of human chimeric 2A4 (c2A4) (Figure 28)
SEQ ID NO: 26: Amino acid sequence of the heavy chain of human chimeric 2A4 (c2A4) (Figure 29)
SEQ ID NO: 27: Primer set F for the heavy chain of human chimeric 2A4 (Figure 30)
SEQ ID NO: 28: Primer set R for the heavy chain of human chimeric 2A4 (Figure 31)
SEQ ID NO: 29: Nucleotide sequence of the light chain of human chimeric 2B1 (c2B1) (Figure 32)
SEQ ID NO: 30: Amino acid sequence of the light chain of human chimeric 2B1 (c2B1) (Figure 33)
SEQ ID NO: 31: Primer set F for the light chain of human chimeric 2B1 (Figure 34)

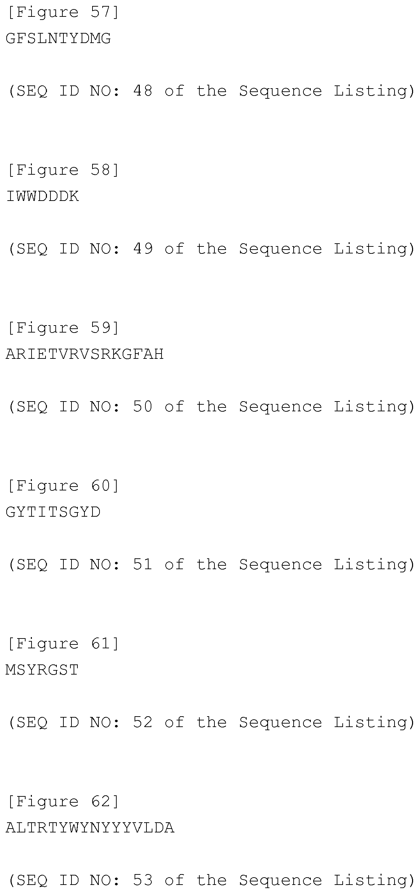

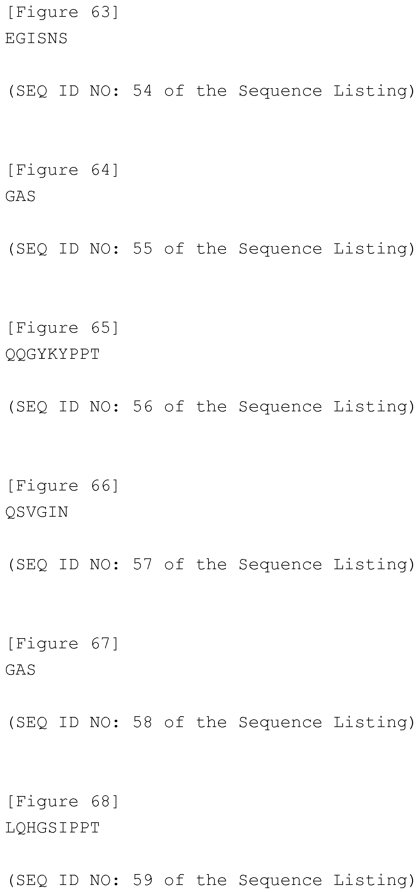

SEQ ID NO: 32: Primer set R for the light chain of human chimeric 2B1 (Figure 35)
SEQ ID NO: 33: Nucleotide sequence of the heavy chain of human chimeric 2B1 (c2B1) (Figure 36)
SEQ ID NO: 34: Amino acid sequence of the heavy chain of human chimeric 2B1 (c2B1) (Figure 37)
SEQ ID NO: 35: Primer set F for the heavy chain of human chimeric 2B1 (Figure 38)
SEQ ID NO: 36: Primer set R for the heavy chain of human chimeric 2B1 (Figure 39)
SEQ ID NO: 37: Nucleotide sequence of the light chain of human chimeric 7B4 (c7B4) (Figure 40)
SEQ ID NO: 38: Amino acid sequence of the light chain of human chimeric 7B4 (c7B4) (Figure 41)
SEQ ID NO: 39: Primer set F for the light chain of human chimeric 7B4 (Figure 42)
SEQ ID NO: 40: Primer set R for the light chain of human chimeric 7B4 (Figure 43)
SEQ ID NO: 41: Nucleotide sequence of the heavy chain of human chimeric 7B4 (c7B4) (Figure 44)
SEQ ID NO: 42: Amino acid sequence of the heavy chain of human chimeric 7B4 (c7B4) (Figure 45)
SEQ ID NO: 43: Primer set F for the heavy chain of human chimeric 7B4 (Figure 46)
SEQ ID NO: 44: Primer set R for the heavy chain of human chimeric 7B4 (Figure 47)
SEQ ID NO: 45: Amino acid sequence of the heavy chain CDR1 of the rat anti-GPRC5D antibody 2A4 (Figure 54)
SEQ ID NO: 46: Amino acid sequence of the heavy chain CDR2 of the rat anti-GPRC5D antibody 2A4 (Figure 55)
SEQ ID NO: 47: Amino acid sequence of the heavy chain CDR3 of the rat anti-GPRC5D antibody 2A4 (Figure 56)
SEQ ID NO: 48: Amino acid sequence of the heavy chain CDR1 of the rat anti-GPRC5D antibody 2B1 (Figure 57)
SEQ ID NO: 49: Amino acid sequence of the heavy chain CDR2 of the rat anti-GPRC5D antibody 2B1 (Figure 58)
SEQ ID NO: 50: Amino acid sequence of the heavy chain CDR3 of the rat anti-GPRC5D antibody 2B1 (Figure 59)
SEQ ID NO: 51: Amino acid sequence of the heavy chain CDR1 of the rat anti-GPRC5D antibody 7B4 (Figure 60)
SEQ ID NO: 52: Amino acid sequence of the heavy chain CDR2 of the rat anti-GPRC5D antibody 7B4 (Figure 61)
SEQ ID NO: 53: Amino acid sequence of the heavy chain CDR3 of the rat anti-GPRC5D antibody 7B4 (Figure 62)
SEQ ID NO: 54: Amino acid sequence of the light chain CDR1 of the rat anti-GPRC5D antibody 2A4 (Figure 63)
SEQ ID NO: 55: Amino acid sequence of the light chain CDR2 of the rat anti-GPRC5D antibody 2A4 (Figure 64)
SEQ ID NO: 56: Amino acid sequence of the light chain CDR3 of the rat anti-GPRC5D antibody 2A4 (Figure 65)
SEQ ID NO: 57: Amino acid sequence of the light chain CDR1 of the rat anti-GPRC5D antibody 2B1 (Figure 66)
SEQ ID NO: 58: Amino acid sequence of the light chain CDR2 of the rat anti-GPRC5D antibody 2B1 (Figure 67)
SEQ ID NO: 59: Amino acid sequence of the light chain CDR3 of the rat anti-GPRC5D antibody 2B1 (Figure 68)
SEQ ID NO: 60: Amino acid sequence of the light chain CDR1 of the rat anti-GPRC5D antibody 7B4 (Figure 69)
SEQ ID NO: 61: Amino acid sequence of the light chain CDR2 of the rat anti-GPRC5D antibody 7B4 (Figure 70)
SEQ ID NO: 62: Amino acid sequence of the light chain CDR3 of the rat anti-GPRC5D antibody 7B4 (Figure 71)
SEQ ID NO: 63: Nucleotide sequence of a humanized 2B1 light chain (h2B1_L1) (Figure 72). In this sequence, nucleotide positions 1 to 60 represent a signal sequence, which is usually not contained in the nucleotide sequences of most of mature h2B1_L1 light chains.
SEQ ID NO: 64: Amino acid sequence of the humanized 2B1 light chain (h2B1_L1) (Figure 73)
SEQ ID NO: 65: Nucleotide sequence of the humanized 2B1 light chain (h2B1_L2) (Figure 74)
SEQ ID NO: 66: Amino acid sequence of the humanized 2B1 light chain (h2B1_L2) (Figure 75)
SEQ ID NO: 67: Nucleotide sequence of the humanized 2B1 light chain (h2B1_L3) (Figure 76)
SEQ ID NO: 68: Amino acid sequence of the humanized 2B1 light chain (h2B1_L3) (Figure 77)
SEQ ID NO: 69: Nucleotide sequence of the humanized 2B1 light chain (h2B1_L4) (Figure 78)
SEQ ID NO: 70: Amino acid sequence of the humanized 2B1 light chain (h2B1_L4) (Figure 79)
SEQ ID NO: 71: Nucleotide sequence of the humanized 2B1 light chain (h2B1_L5) (Figure 80)
SEQ ID NO: 72: Amino acid sequence of the humanized 2B1 light chain (h2B1_L5) (Figure 81)
SEQ ID NO: 73: Nucleotide sequence of the humanized 2B1 heavy chain (h2B1_H1) (Figure 82)
SEQ ID NO: 74: Amino acid sequence of the humanized 2B1 heavy chain (h2B1_H1) (Figure 83)
SEQ ID NO: 75: Nucleotide sequence of the humanized 2B1 heavy chain (h2B1_H2) (Figure 84)
SEQ ID NO: 76: Amino acid sequence of the humanized 2B1 heavy chain (h2B1_H2) (Figure 85)
SEQ ID NO: 77: Nucleotide sequence of the humanized 2B1 heavy chain (h2B1_H3) (Figure 86)
SEQ ID NO: 78: Amino acid sequence of the humanized 2B1 heavy chain (h2B1_H3) (Figure 87)
SEQ ID NO: 79: Nucleotide sequence of the humanized 2B1 heavy chain (h2B1_H4) (Figure 88)
SEQ ID NO: 80: Amino acid sequence of the humanized 2B1 heavy chain (h2B1_H4) (Figure 89)
SEQ ID NO: 81: Nucleotide sequence of the humanized 7B4 light chain (h7B4_L1) (Figure 90)
SEQ ID NO: 82: Amino acid sequence of the humanized 7B4 light chain (h7B4_L1) (Figure 91)
SEQ ID NO: 83: Nucleotide sequence of the humanized 7B4 light chain (h7B4_L2) (Figure 92)
SEQ ID NO: 84: Amino acid sequence of the humanized 7B4 light chain (h7B4_L2) (Figure 93)
SEQ ID NO: 85: Nucleotide sequence of the humanized 7B4 heavy chain (h7B4_H1) (Figure 94)
SEQ ID NO: 86: Amino acid sequence of the humanized 7B4 heavy chain (h7B4_H1) (Figure 95)
SEQ ID NO: 87: Nucleotide sequence of the humanized 7B4 heavy chain (h7B4_H2) (Figure 96)

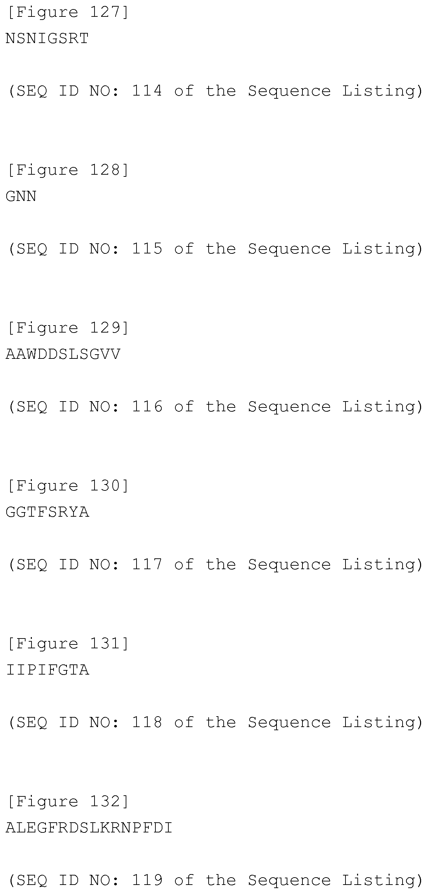

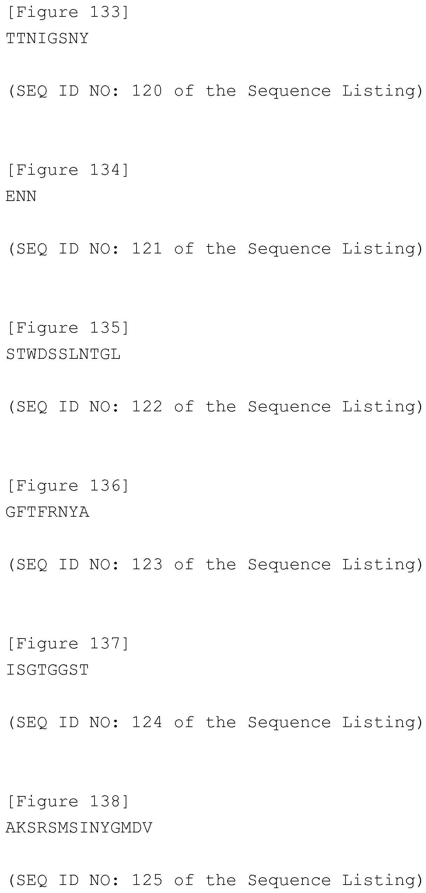

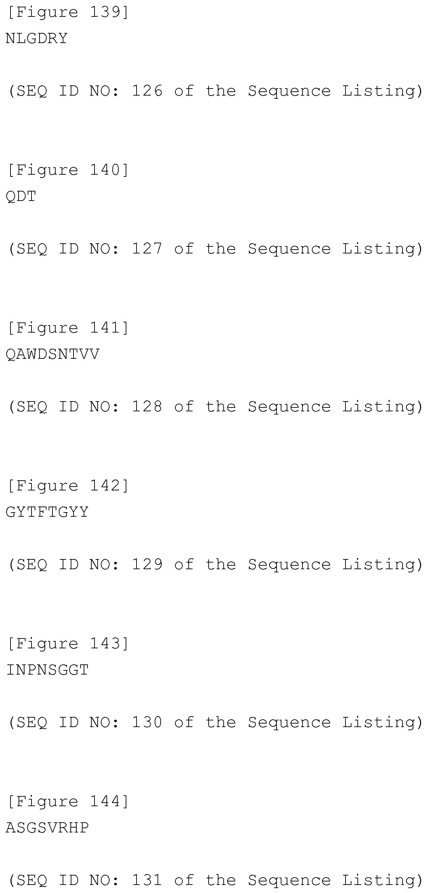

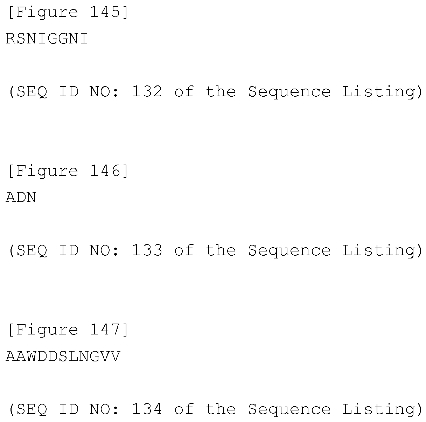

SEQ ID NO: 88: Amino acid sequence of the humanized 7B4 heavy chain (h7B4_H2) (Figure 97)
SEQ ID NO: 89: Nucleotide sequence of the humanized 7B4 heavy chain (h7B4_H3) (Figure 98)
SEQ ID NO: 90: Amino acid sequence of the humanized 7B4 heavy chain (h7B4_H3) (Figure 99)
SEQ ID NO: 91: Nucleotide sequence of the humanized 7B4 heavy chain (h7B4_H5) (Figure 100)
SEQ ID NO: 92: Amino acid sequence of the humanized 7B4 heavy chain (h7B4_H5) (Figure 101)
SEQ ID NO: 93: Amino acid sequence of the amino-terminal peptide of cynomolgus monkey GPRC5D (Figure 105)
SEQ ID NO: 94: Nucleotide sequence of primer A used in the sequence analysis of scFv (Figure 106)
SEQ ID NO: 95: Nucleotide sequence of primer B used in the sequence analysis of scFv (Figure 107)
SEQ ID NO: 96: Nucleotide sequence of the heavy chain variable region of C2037 (Figure 108)
SEQ ID NO: 97: Amino acid sequence of the heavy chain variable region of C2037 (Figure 109)
SEQ ID NO: 98: Nucleotide sequence of the light chain variable region of C2037 (Figure 110)
SEQ ID NO: 99: Amino acid sequence of the light chain variable region of C2037 (Figure 111)
SEQ ID NO: 100: Nucleotide sequence of the heavy chain variable region of C3048 (Figure 112)
SEQ ID NO: 101: Amino acid sequence of the heavy chain variable region of C3048 (Figure 113)
SEQ ID NO: 102: Nucleotide sequence of the light chain variable region of C3048 (Figure 114)
SEQ ID NO: 103: Amino acid sequence of the light chain variable region of C3048 (Figure 115)
SEQ ID NO: 104: Nucleotide sequence of the heavy chain variable region of C3015 (Figure 116)
SEQ ID NO: 105: Amino acid sequence of the heavy chain variable region of C3015 (Figure 117)
SEQ ID NO: 106: Nucleotide sequence of the light chain variable region of C3015 (Figure 118)
SEQ ID NO: 107: Amino acid sequence of the light chain variable region of C3015 (Figure 119)
SEQ ID NO: 108: Nucleotide sequence of the heavy chain variable region of C3022 (Figure 120)
SEQ ID NO: 109: Amino acid sequence of the heavy chain variable region of C3022 (Figure 121)
SEQ ID NO: 110: Nucleotide sequence of the light chain variable region of C3022 (Figure 122)
SEQ ID NO: 111: Amino acid sequence of the heavy chain CDR1 of C2037 (Figure 124)
SEQ ID NO: 112: Amino acid sequence of the heavy chain CDR2 of C2037 (Figure 125)
SEQ ID NO: 113: Amino acid sequence of the heavy chain CDR3 of C2037 (Figure 126)
SEQ ID NO: 114: Amino acid sequence of the light chain CDR1 of C2037 (Figure 127)
SEQ ID NO: 115: Amino acid sequence of the light chain CDR2 of C2037 (Figure 128)
SEQ ID NO: 116: Amino acid sequence of the light chain CDR3 of C2037 (Figure 129)
SEQ ID NO: 117: Amino acid sequence of the heavy chain CDR1 of C3048 (Figure 130)
SEQ ID NO: 118: Amino acid sequence of the heavy chain CDR2 of C3048 (Figure 131)
SEQ ID NO: 119: Amino acid sequence of the heavy chain CDR3 of C3048 (Figure 132)
SEQ ID NO: 120: Amino acid sequence of the light chain CDR1 of C3048 (Figure 133)
SEQ ID NO: 121: Amino acid sequence of the light chain CDR2 of C3048 (Figure 134)
SEQ ID NO: 122: Amino acid sequence of the light chain CDR3 of C3048 (Figure 135)
SEQ ID NO: 123: Amino acid sequence of the heavy chain CDR1 of C3015 (Figure 136)
SEQ ID NO: 124: Amino acid sequence of the heavy chain CDR2 of C3015 (Figure 137)
SEQ ID NO: 125: Amino acid sequence of the heavy chain CDR3 of C3015 (Figure 138)
SEQ ID NO: 126: Amino acid sequence of the light chain CDR1 of C3015 (Figure 139)
SEQ ID NO: 127: Amino acid sequence of the light chain CDR2 of C3015 (Figure 140)
SEQ ID NO: 128: Amino acid sequence of the light chain CDR3 of C3015 (Figure 141)
SEQ ID NO: 129: Amino acid sequence of the heavy chain CDR1 of C3022 (Figure 142)
SEQ ID NO: 130: Amino acid sequence of the heavy chain CDR2 of C3022 (Figure 143)
SEQ ID NO: 131: Amino acid sequence of the heavy chain CDR3 of C3022 (Figure 144)
SEQ ID NO: 132: Amino acid sequence of the light chain CDR1 of C3022 (Figure 145)
SEQ ID NO: 133: Amino acid sequence of the light chain CDR2 of C3022 (Figure 146)
SEQ ID NO: 134: Amino acid sequence of the light chain CDR3 of C3022 (Figure 147)
SEQ ID NO: 135: Amino acid sequence of the light chain variable region of C3022 (Figure 123)
SEQ ID NO: 136: Nucleotide sequence of the heavy chain of an IgG form of C2037 (Figure 148)
SEQ ID NO: 137: Nucleotide sequence of the light chain of an IgG form of C2037 (Figure 149)
SEQ ID NO: 138: Nucleotide sequence of the heavy chain of an IgG form of C3048 (Figure 150)
SEQ ID NO: 139: Nucleotide sequence of the light chain of an IgG form of C3048 (Figure 151)
SEQ ID NO: 140: Nucleotide sequence of the heavy chain of an IgG form of C3015 (Figure 152)
SEQ ID NO: 141: Nucleotide sequence of the light chain of an IgG form of C3015 (Figure 153)
SEQ ID NO: 142: Nucleotide sequence of the heavy chain of an IgG form of C3022 (Figure 154)
SEQ ID NO: 143: Nucleotide sequence of the light chain of an IgG form of C3022 (Figure 155)
SEQ ID NO: 144: Amino acid sequence of the heavy chain of an IgG form of C2037 (Figure 156)
SEQ ID NO: 145: Amino acid sequence of the light chain of an IgG form of C2037 (Figure 157)

SEQ ID NO: 146: Amino acid sequence of the heavy chain of an IgG form of C3048 (Figure 158)
SEQ ID NO: 147: Amino acid sequence of the light chain of an IgG form of C3048 (Figure 159)
SEQ ID NO: 148: Amino acid sequence of the heavy chain of an IgG form of C3015 (Figure 160)
SEQ ID NO: 149: Amino acid sequence of the light chain of an IgG form of C3015 (Figure 161)
SEQ ID NO: 150: Amino acid sequence of the heavy chain of an IgG form of C3022 (Figure 162)
SEQ ID NO: 151: Amino acid sequence of the light chain of the IgG form of C3022 (Figure 163)
SEQ ID NO: 152: Nucleotide sequence of the heavy chain variable region of a rat anti-CD3 antibody (Figure 168)
SEQ ID NO: 153: Nucleotide sequence of the light chain variable region of the rat anti-CD3 antibody (Figure 169)
SEQ ID NO: 154: Nucleotide sequence of C3E-7000 (Figure 170)
SEQ ID NO: 155: Amino acid sequence of the heavy chain variable region of C3E-7034 (Figure 171)
SEQ ID NO: 156: Amino acid sequence of the light chain variable region of C3E-7034 (Figure 172)
SEQ ID NO: 157: Nucleotide sequence of C3E-7034 (Figure 173)
SEQ ID NO: 158: Amino acid sequence of the light chain variable region of C3E-7035 (Figure 174)
SEQ ID NO: 159: Nucleotide sequence of C3E-7035 (Figure 175)
SEQ ID NO: 160: Amino acid sequence of the light chain variable region of C3E-7036 (Figure 176)
SEQ ID NO: 161: Nucleotide sequence of C3E-7036 (Figure 177)
SEQ ID NO: 162: Nucleotide sequence of C2037-C3E-7034 (Figure 178)
SEQ ID NO: 163: Nucleotide sequence of C3048-C3E-7034 (Figure 179)
SEQ ID NO: 164: Nucleotide sequence of C3022-C3E-7034 (Figure 180)
SEQ ID NO: 165: Nucleotide sequence of C2037-C3E-7035 (Figure 181)
SEQ ID NO: 166: Nucleotide sequence of C3048-C3E-7035 (Figure 182)
SEQ ID NO: 167: Nucleotide sequence of C3022-C3E-7035 (Figure 183)
SEQ ID NO: 168: Nucleotide sequence of C2037-C3E-7036 (Figure 184)
SEQ ID NO: 169: Nucleotide sequence of C3048-C3E-7036 (Figure 185)
SEQ ID NO: 170: Nucleotide sequence of C3022-C3E-7036 (Figure 186)
SEQ ID NO: 171: Amino acid sequence of C2037-C3E-7034 (Figure 187)
SEQ ID NO: 172: Amino acid sequence of C3048-C3E-7034 (Figure 188)
SEQ ID NO: 173: Amino acid sequence of C3022-C3E-7034 (Figure 189)
SEQ ID NO: 174: Amino acid sequence of C2037-C3E-7035 (Figure 190)
SEQ ID NO: 175: Amino acid sequence of C3048-C3E-7035 (Figure 191)
SEQ ID NO: 176: Amino acid sequence of C3022-C3E-7035 (Figure 192)
SEQ ID NO: 177: Amino acid sequence of C2037-C3E-7036 (Figure 193)
SEQ ID NO: 178: Amino acid sequence of C3048-C3E-7036 (Figure 194)
SEQ ID NO: 179: Amino acid sequence of C3022-C3E-7036 (Figure 195)
SEQ ID NO: 180: Amino acid sequence of C3E-7034 (Figure 203)
SEQ ID NO: 181: Amino acid sequence of C3E-7035 (Figure 204)
SEQ ID NO: 182: Amino acid sequence of C3E-7036 (Figure 205)
SEQ ID NO: 183: Amino acid sequence of the heavy chain CDR1 of C3E-7000 (Figure 206)
SEQ ID NO: 184: Amino acid sequence of the heavy chain CDR2 of C3E-7000 (Figure 207)
SEQ ID NO: 185: Amino acid sequence of the heavy chain CDR3 of C3E-7000 (Figure 208)
SEQ ID NO: 186: Amino acid sequence of the light chain CDR1 of C3E-7000 (Figure 209)
SEQ ID NO: 187: Amino acid sequence of the light chain CDR2 of C3E-7000 (Figure 210)
SEQ ID NO: 188: Amino acid sequence of the light chain CDR3 of C3E-7000 (Figure 211)
SEQ ID NO: 189: Amino acid sequence of human CD3ε (Figure 212)
SEQ ID NO: 190: Nucleotide sequence of the heavy chain variable region of E1018 (Figure 213)
SEQ ID NO: 191: Amino acid sequence of the heavy chain variable region of E1018 (Figure 214)
SEQ ID NO: 192: Nucleotide sequence of the light chain variable region of E1018 (Figure 215)
SEQ ID NO: 193: Amino acid sequence of the light chain variable region of E1018 (Figure 216)
SEQ ID NO: 194: Nucleotide sequence of the heavy chain variable region of D1012 (Figure 217)
SEQ ID NO: 195: Amino acid sequence of the heavy chain variable region of D1012 (Figure 218)
SEQ ID NO: 196: Nucleotide sequence of the light chain variable region of D1012 (Figure 219)
SEQ ID NO: 197: Amino acid sequence of the light chain variable region of D1012 (Figure 220)
SEQ ID NO: 198: Nucleotide sequence of h2B1 Fab HC_1 (Figure 222)
SEQ ID NO: 199: Amino acid sequence of h2B1 Fab HC_1 (Figure 223)
SEQ ID NO: 200: Nucleotide sequence of h2B1 Fab HC_2 (Figure 224)
SEQ ID NO: 201: Amino acid sequence of h2B1 Fab HC_2 (Figure 225)
SEQ ID NO: 202: Nucleotide sequence of h2B1 Fab LC_1 (Figure 226)
SEQ ID NO: 203: Amino acid sequence of h2B1 Fab LC_1 (Figure 227)

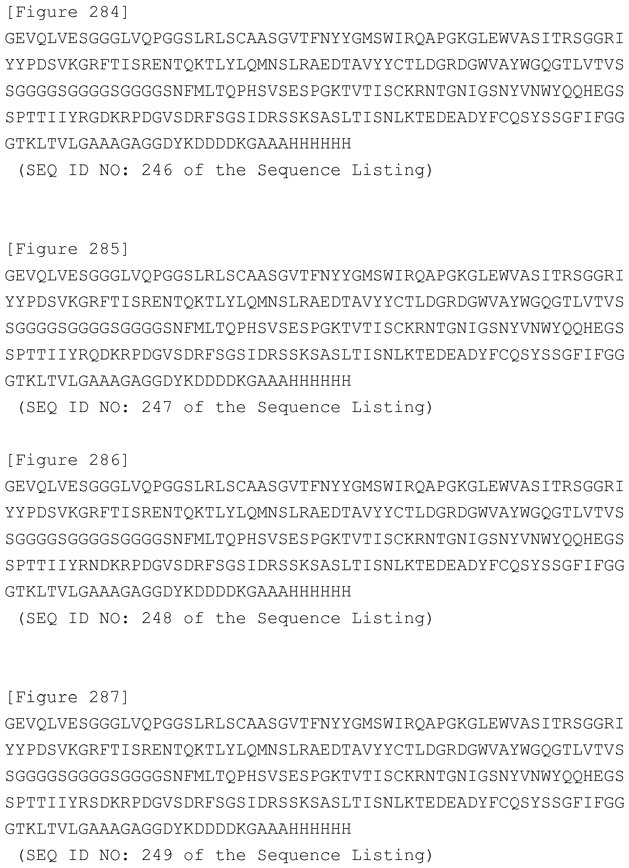

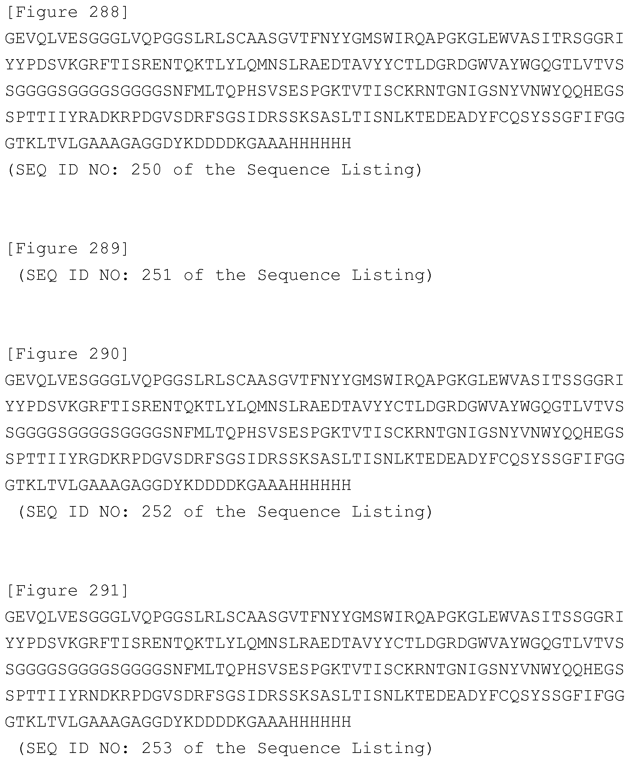

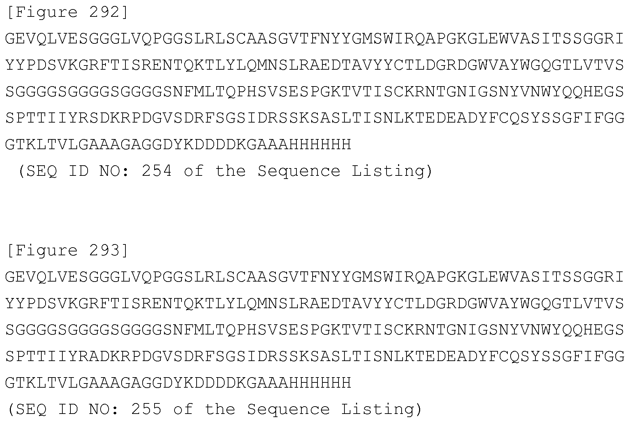

SEQ ID NO: 204: Nucleotide sequence of h2B1 Fab LC_2 (Figure 228)
SEQ ID NO: 205: Amino acid sequence of h2B1 Fab LC_2 (Figure 229)
SEQ ID NO: 206: Nucleotide sequence of C3E-7034 Fab HC (Figure 230)
SEQ ID NO: 207: Amino acid sequence of C3E-7034 Fab HC (Figure 231)
SEQ ID NO: 208: Nucleotide sequence of C3E-7034 Fab LC (Figure 232)
SEQ ID NO: 209: Amino acid sequence of C3E-7034 Fab LC (Figure 233)
SEQ ID NO: 210: Nucleotide sequence of C3E-7036 Fab HC (Figure 234)
SEQ ID NO: 211: Amino acid sequence of C3E-7036 Fab HC (Figure 235)
SEQ ID NO: 212: Nucleotide sequence of C3E-7036 Fab LC (Figure 236)
SEQ ID NO: 213: Amino acid sequence of C3E-7036 Fab LC (Figure 237)
SEQ ID NO: 214: Nucleotide sequence of h2B1 Fab HC_3 (Figure 238)
SEQ ID NO: 215: Amino acid sequence of h2B1 Fab HC_3 (Figure 239)
SEQ ID NO: 216: Nucleotide sequence of h2B1 Fab LC_3 (Figure 240)
SEQ ID NO: 217: Amino acid sequence of h2B1 Fab LC_3 (Figure 241)
SEQ ID NO: 218: Nucleotide sequence of C3E-7034 scFv Fc (Figure 242)
SEQ ID NO: 219: Amino acid sequence of C3E-7034 scFv Fc (Figure 243)
SEQ ID NO: 220: Nucleotide sequence of C3E-7036 scFv Fc (Figure 244)
SEQ ID NO: 221: Amino acid sequence of C3E-7036 scFv Fc (Figure 245)
SEQ ID NO: 222: Nucleotide sequence of h2B1 scFv Fc (Figure 246)
SEQ ID NO: 223: Amino acid sequence of h2B1 scFv Fc (Figure 247)
SEQ ID NO: 224: Nucleotide sequence of C3E-8015 (Figure 255)
SEQ ID NO: 225: Amino acid sequence of C3E-8015 (Figure 256)
SEQ ID NO: 226: Nucleotide sequence of C3E-8017 (Figure 257)
SEQ ID NO: 227: Amino acid sequence of C3E-8017 (Figure 258)
SEQ ID NO: 228: Nucleotide sequence of C3E-8018 (Figure 259)
SEQ ID NO: 229: Amino acid sequence of C3E-8018 (Figure 260)
SEQ ID NO: 230: Nucleotide sequence of C3E-8025 (Figure 261)
SEQ ID NO: 231: Amino acid sequence of C3E-8025 (Figure 262)
SEQ ID NO: 232: Nucleotide sequence of C3E-8027 (Figure 263)
SEQ ID NO: 233: Amino acid sequence of C3E-8027 (Figure 264)
SEQ ID NO: 234: Nucleotide sequence of C3E-8028 (Figure 265)
SEQ ID NO: 235: Amino acid sequence of C3E-8028 (Figure 266)
SEQ ID NO: 236: Nucleotide sequence of h2B1 Fab HC_4 (Figure 267)
SEQ ID NO: 237: Amino acid sequence of h2B1 Fab HC_4 (Figure 268)
SEQ ID NO: 238: Amino acid sequence of the heavy chain CDR2 of a CDR-modified form (Figure 276) wherein X is an arbitrary natural amino acid.
SEQ ID NO: 239: Amino acid sequence of the light chain CDR2 of the CDR-modified form (Figure 277) wherein X is an arbitrary natural amino acid.
SEQ ID NO: 240: Amino acid sequence of the heavy chain variable region of a CDR-modified form of C3E-7034 (Figure 278) wherein X is an arbitrary natural amino acid.
SEQ ID NO: 241: Amino acid sequence of the light chain variable region of the CDR-modified form of C3E-7034 (Figure 279) wherein X is an arbitrary natural amino acid.
SEQ ID NO: 242: Amino acid sequence of the light chain variable region of a CDR-modified form of C3E-7035 (Figure 280) wherein X is an arbitrary natural amino acid.
SEQ ID NO: 243: Amino acid sequence of the light chain variable region of a CDR-modified form of C3E-7036 (Figure 281) wherein X is an arbitrary natural amino acid.
SEQ ID NO: 244: Amino acid sequence of C3E-7078 (Figure 282)
SEQ ID NO: 245: Amino acid sequence of C3E-7085 (Figure 283)
SEQ ID NO: 246: Amino acid sequence of C3E-7086 (Figure 284)
SEQ ID NO: 247: Amino acid sequence of C3E-7087 (Figure 285)
SEQ ID NO: 248: Amino acid sequence of C3E-7088 (Figure 286)
SEQ ID NO: 249: Amino acid sequence of C3E-7089 (Figure 287)
SEQ ID NO: 250: Amino acid sequence of C3E-7090 (Figure 288)
SEQ ID NO: 251: Amino acid sequence of C3E-7091 (Figure 289)
SEQ ID NO: 252: Amino acid sequence of C3E-7092 (Figure 290)
SEQ ID NO: 253: Amino acid sequence of C3E-7093 (Figure 291)
SEQ ID NO: 254: Amino acid sequence of C3E-7094 (Figure 292)
SEQ ID NO: 255: Amino acid sequence of C3E-7095 (Figure 293)

## Claims

**1.** An antibody or an antigen-binding fragment of the antibody, wherein the antibody comprises a heavy chain variable region and a light chain variable region according to any one of the following (I) to (III):

(II)

a heavy chain variable region comprising
heavy chain CDR1 consisting of an amino acid sequence represented by SEQ ID NO: 48,
heavy chain CDR2 consisting of an amino acid sequence represented by SEQ ID NO: 49, and
heavy chain CDR3 consisting of an amino acid sequence represented by SEQ ID NO: 50,
and
a light chain variable region comprising
light chain CDR1 consisting of an amino acid sequence represented by SEQ ID NO: 57,
light chain CDR2 consisting of an amino acid sequence represented by SEQ ID NO: 58, and
light chain CDR3 consisting of an amino acid sequence represented by SEQ ID NO: 59,

(I)

a heavy chain variable region comprising
heavy chain CDR1 consisting of an amino acid sequence represented by SEQ ID NO: 45,
heavy chain CDR2 consisting of an amino acid sequence represented by SEQ ID NO: 46, and
heavy chain CDR3 consisting of an amino acid sequence represented by SEQ ID NO: 47,
and
a light chain variable region comprising
light chain CDR1 consisting of an amino acid sequence represented by SEQ ID NO: 54,
light chain CDR2 consisting of an amino acid sequence represented by SEQ ID NO: 55, and
light chain CDR3 consisting of an amino acid sequence represented by SEQ ID NO: 56,and

(III)

a heavy chain variable region comprising
heavy chain CDR1 consisting of an amino acid sequence represented by SEQ ID NO: 51,
heavy chain CDR2 consisting of an amino acid sequence represented by SEQ ID NO: 52, and
heavy chain CDR3 consisting of an amino acid sequence represented by SEQ ID NO: 53,
and
a light chain variable region comprising
light chain CDR1 consisting of an amino acid sequence represented by SEQ ID NO: 60,
light chain CDR2 consisting of an amino acid sequence represented by SEQ ID NO: 61, and
light chain CDR3 consisting of an amino acid sequence represented by SEQ ID NO: 62
and binds to human GPRC5D.

**2.** The antibody or the antigen-binding fragment of the antibody according to claim 1, wherein the heavy chain variable region and the light chain variable region are the heavy chain variable region and the light chain variable region according to (II).

**3.** The antibody or the antigen-binding fragment of the antibody according to claim 1, wherein the heavy chain variable region and the light chain variable region are the heavy chain variable region and the light chain variable region according to (I).

**4.** The antibody or the antigen-binding fragment of the antibody according to claim 1, wherein the heavy chain variable region and the light chain variable region are the heavy chain variable region and the light chain variable region according to (III).

5. The antibody or the antigen-binding fragment of the antibody according to any one of claims 1 to 4, wherein the antibody or the antigen-binding fragment is a chimeric antibody or an antigen-binding fragment of the antibody.

6. The antibody or the antigen-binding fragment of the antibody according to any one of claims 1 to 4, wherein the antibody or the antigen-binding fragment is a humanized antibody or an antigen-binding fragment of the antibody.

7. The antibody or the antigen-binding fragment of the antibody according to any one of claims 1 to 4, wherein the antibody or the antigen-binding fragment is a human antibody or an antigen-binding fragment of the antibody.

8. The antibody or the antigen-binding fragment of the antibody according to any one of claims 1, 2, or 6, wherein the antibody comprises a light chain variable region comprising any one amino acid sequence represented by

amino acid residues 21 to 127 of an amino acid sequence represented by SEQ ID NO: 64,
amino acid residues 21 to 127 of an amino acid sequence represented by SEQ ID NO: 66,
amino acid residues 21 to 127 of an amino acid sequence represented by SEQ ID NO: 68,
amino acid residues 21 to 127 of an amino acid sequence represented by SEQ ID NO: 70, and
amino acid residues 21 to 127 of an amino acid sequence represented by SEQ ID NO: 72, and
a heavy chain variable region comprising any one amino acid sequence represented by
amino acid residues 20 to 142 of an amino acid sequence represented by SEQ ID NO: 74,
amino acid residues 20 to 142 of an amino acid sequence represented by SEQ ID NO: 76,
amino acid residues 20 to 142 of an amino acid sequence represented by SEQ ID NO: 78, and
amino acid residues 20 to 142 of an amino acid sequence represented by SEQ ID NO: 80.

9. The antibody or the antigen-binding fragment of the antibody according to any one of claims 1, 2, 6, or 8, wherein the antibody comprises any one combination of a heavy chain variable region and a light chain variable region of

• a heavy chain variable region comprising an amino acid sequence represented by amino acid residues 20 to 142 of the amino acid sequence represented by SEQ ID NO: 74, and a light chain variable region comprising an amino acid sequence represented by amino acid residues 21 to 127 of the amino acid sequence represented by SEQ ID NO: 64,
• a heavy chain variable region comprising an amino acid sequence represented by amino acid residues 20 to 142 of the amino acid sequence represented by SEQ ID NO: 74, and a light chain variable region comprising an amino acid sequence represented by amino acid residues 21 to 127 of the amino acid sequence represented by SEQ ID NO: 66,
• a heavy chain variable region comprising an amino acid sequence represented by amino acid residues 20 to 142 of the amino acid sequence represented by SEQ ID NO: 76, and a light chain variable region comprising an amino acid sequence represented by amino acid residues 21 to 127 of the amino acid sequence represented by SEQ ID NO: 66,
• a heavy chain variable region comprising an amino acid sequence represented by amino acid residues 20 to 142 of the amino acid sequence represented by SEQ ID NO: 76, and a light chain variable region comprising an amino acid sequence represented by amino acid residues 21 to 127 of the amino acid sequence represented by SEQ ID NO: 68,
• a heavy chain variable region comprising an amino acid sequence represented by amino acid residues 20 to 142 of the amino acid sequence represented by SEQ ID NO: 76, and a light chain variable region comprising an amino acid sequence represented by amino acid residues 21 to 127 of the amino acid sequence represented by SEQ ID NO: 70,
• a heavy chain variable region comprising an amino acid sequence represented by amino acid residues 20 to 142 of the amino acid sequence represented by SEQ ID NO: 76, and a light chain variable region comprising an amino acid sequence represented by amino acid residues 21 to 127 of the amino acid sequence represented by SEQ ID NO: 72,
• a heavy chain variable region comprising an amino acid sequence represented by amino acid residues 20 to 142 of the amino acid sequence represented by SEQ ID NO: 78, and a light chain variable region comprising an amino acid sequence represented by amino acid residues 21 to 127 of the amino acid sequence represented by SEQ ID NO: 68,
• a heavy chain variable region comprising an amino acid sequence represented by amino acid residues 20 to 142 of the amino acid sequence represented by SEQ ID NO: 78, and a light chain variable region comprising an amino acid sequence represented by amino acid residues 21 to 127 of the amino acid sequence represented by SEQ ID NO: 70,

• a heavy chain variable region comprising an amino acid sequence represented by amino acid residues 20 to 142 of the amino acid sequence represented by SEQ ID NO: 78, and a light chain variable region comprising an amino acid sequence represented by amino acid residues 21 to 127 of the amino acid sequence represented by SEQ ID NO: 72,
• a heavy chain variable region comprising an amino acid sequence represented by amino acid residues 20 to 142 of the amino acid sequence represented by SEQ ID NO: 80, and a light chain variable region comprising an amino acid sequence represented by amino acid residues 21 to 127 of the amino acid sequence represented by SEQ ID NO: 64,
• a heavy chain variable region comprising an amino acid sequence represented by amino acid residues 20 to 142 of the amino acid sequence represented by SEQ ID NO: 80, and a light chain variable region comprising an amino acid sequence represented by amino acid residues 21 to 127 of the amino acid sequence represented by SEQ ID NO: 68,
• a heavy chain variable region comprising an amino acid sequence represented by amino acid residues 20 to 142 of the amino acid sequence represented by SEQ ID NO: 80, and a light chain variable region comprising an amino acid sequence represented by amino acid residues 21 to 127 of the amino acid sequence represented by SEQ ID NO: 70, or
• a heavy chain variable region comprising an amino acid sequence represented by amino acid residues 20 to 142 of the amino acid sequence represented by SEQ ID NO: 80, and a light chain variable region comprising an amino acid sequence represented by amino acid residues 21 to 127 of the amino acid sequence represented by SEQ ID NO: 72.

**10.** The antibody or antigen-binding fragment of the antibody according to any one of claims 1, 4, or 6, wherein the antibody comprises a light chain variable region comprising an amino acid sequence represented by

amino acid residues 21 to 126 of an amino acid sequence represented by SEQ ID NO: 82, or
amino acid residues 21 to 126 of an amino acid sequence represented by SEQ ID NO: 84, and
a heavy chain variable region comprising any one amino acid sequence represented by
amino acid residues 20 to 142 of an amino acid sequence represented by SEQ ID NO: 86,
amino acid residues 20 to 142 of an amino acid sequence represented by SEQ ID NO: 88,
amino acid residues 20 to 142 of an amino acid sequence represented by SEQ ID NO: 90, and
amino acid residues 20 to 142 of an amino acid sequence represented by SEQ ID NO: 92.

**11.** The antibody or the antigen-binding fragment of the antibody according to any one of claims 1, 4, 6, or 10, wherein the antibody comprises any one combination of a heavy chain variable region and a light chain variable region of

• a heavy chain variable region comprising an amino acid sequence represented by amino acid residues 20 to 142 of the amino acid sequence represented by SEQ ID NO: 86, and a light chain variable region comprising an amino acid sequence represented by amino acid residues 21 to 126 of the amino acid sequence represented by SEQ ID NO: 84,
• a heavy chain variable region comprising an amino acid sequence represented by amino acid residues 20 to 142 of the amino acid sequence represented by SEQ ID NO: 88, and a light chain variable region comprising an amino acid sequence represented by amino acid residues 21 to 126 of the amino acid sequence represented by SEQ ID NO: 84,
• a heavy chain variable region comprising an amino acid sequence represented by amino acid residues 20 to 142 of the amino acid sequence represented by SEQ ID NO: 90, and a light chain variable region comprising an amino acid sequence represented by amino acid residues 21 to 126 of the amino acid sequence represented by SEQ ID NO: 82,
• a heavy chain variable region comprising an amino acid sequence represented by amino acid residues 20 to 142 of the amino acid sequence represented by SEQ ID NO: 90, and a light chain variable region comprising an amino acid sequence represented by amino acid residues 21 to 126 of the amino acid sequence represented by SEQ ID NO: 84, or
• a heavy chain variable region comprising an amino acid sequence represented by amino acid residues 20 to 142 of the amino acid sequence represented by SEQ ID NO: 92, and a light chain variable region comprising an amino acid sequence represented by amino acid residues 21 to 126 of the amino acid sequence represented by SEQ ID NO: 82,

**12.** The antibody according to any one of claims 1 to 11, wherein the antibody comprises Fc.

**13.** An antibody or an antigen-binding fragment of the antibody, wherein the antibody binds to human GPRC5D and comprises a heavy chain variable region and a light chain variable region according to any one of the following ① to ④:

①

a heavy chain variable region comprising
heavy chain CDR1 consisting of an amino acid sequence represented by SEQ ID NO: 111,
heavy chain CDR2 consisting of an amino acid sequence represented by SEQ ID NO: 112, and
heavy chain CDR3 consisting of an amino acid sequence represented by SEQ ID NO: 113,
and
a light chain variable region comprising
light chain CDR1 consisting of an amino acid sequence represented by SEQ ID NO: 114,
light chain CDR2 consisting of an amino acid sequence represented by SEQ ID NO: 115, and
light chain CDR3 consisting of an amino acid sequence represented by SEQ ID NO: 116,

②

a heavy chain variable region comprising
heavy chain CDR1 consisting of an amino acid sequence represented by SEQ ID NO: 117,
heavy chain CDR2 consisting of an amino acid sequence represented by SEQ ID NO: 118, and
heavy chain CDR3 consisting of an amino acid sequence represented by SEQ ID NO: 119,
and
a light chain variable region comprising
light chain CDR1 consisting of an amino acid sequence represented by SEQ ID NO: 120,
light chain CDR2 consisting of an amino acid sequence represented by SEQ ID NO: 121, and
light chain CDR3 consisting of an amino acid sequence represented by SEQ ID NO: 122,

③

a heavy chain variable region comprising
heavy chain CDR1 consisting of an amino acid sequence represented by SEQ ID NO: 123,
heavy chain CDR2 consisting of an amino acid sequence represented by SEQ ID NO: 124, and
heavy chain CDR3 consisting of an amino acid sequence represented by SEQ ID NO: 125,
and
a light chain variable region comprising
light chain CDR1 consisting of an amino acid sequence represented by SEQ ID NO: 126,
light chain CDR2 consisting of an amino acid sequence represented by SEQ ID NO: 127, and
light chain CDR3 consisting of an amino acid sequence represented by SEQ ID NO: 128, and

④

a heavy chain variable region comprising
heavy chain CDR1 consisting of an amino acid sequence represented by SEQ ID NO: 129,
heavy chain CDR2 consisting of an amino acid sequence represented by SEQ ID NO: 130, and
heavy chain CDR3 consisting of an amino acid sequence represented by SEQ ID NO: 131,
and
a light chain variable region comprising
light chain CDR1 consisting of an amino acid sequence represented by SEQ ID NO: 132,
light chain CDR2 consisting of an amino acid sequence represented by SEQ ID NO: 133, and
light chain CDR3 consisting of an amino acid sequence represented by SEQ ID NO: 134.

**14.** The antibody or the antigen-binding fragment of the antibody according to claim 13, wherein the heavy chain variable region and the light chain variable region are the heavy chain variable region and the light chain variable region according to ①.

**15.** The antibody or the antigen-binding fragment of the antibody according to claim 13, wherein the heavy chain variable region and the light chain variable region are the heavy chain variable region and the light chain variable region according to ②.

**16.** The antibody or the antigen-binding fragment of the antibody according to claim 13, wherein the heavy chain variable region and the light chain variable region are the heavy chain variable region and the light chain variable region according to ③.

**17.** The antibody or the antigen-binding fragment of the antibody according to claim 13, wherein the heavy chain variable region and the light chain variable region are the heavy chain variable region and the light chain variable region according to ④.

**18.** The antibody or the antigen-binding fragment of the antibody according to any one of claims 13 to 17, wherein the antibody comprises
a heavy chain variable region comprising any one of

the amino acid sequence represented by SEQ ID NO: 97,
the amino acid sequence represented by SEQ ID NO: 101,
the amino acid sequence represented by SEQ ID NO: 105, and
the amino acid sequence represented by SEQ ID NO: 109,

and
a light chain variable region comprising any one of

the amino acid sequence represented by SEQ ID NO: 99,
the amino acid sequence represented by SEQ ID NO: 103,
the amino acid sequence represented by SEQ ID NO: 107, and
the amino acid sequence represented by SEQ ID NO: 135.

**19.** The antibody or the antigen-binding fragment of the antibody according to any one of claims 13 to 18, wherein the antibody comprises any one combination of a heavy chain variable region and a light chain variable region of

- a heavy chain variable region comprising the amino acid sequence represented by SEQ ID NO: 97, and a light chain variable region comprising the amino acid sequence represented by SEQ ID NO: 99,
- a heavy chain variable region comprising the amino acid sequence represented by SEQ ID NO: 101, and a light chain variable region comprising the amino acid sequence represented by SEQ ID NO: 103,
- a heavy chain variable region comprising the amino acid sequence represented by SEQ ID NO: 105, and a light chain variable region comprising the amino acid sequence represented by SEQ ID NO: 107, or
- a heavy chain variable region comprising the amino acid sequence represented by SEQ ID NO: 109, and a light chain variable region comprising the amino acid sequence represented by SEQ ID NO: 135.

**20.** An antibody or an antigen-binding fragment of the antibody, wherein the antibody comprises any one combination of a heavy chain and a light chain of

- a heavy chain comprising an amino acid sequence represented by SEQ ID NO: 144, and a light chain comprising an amino acid sequence represented by SEQ ID NO: 145,
- a heavy chain comprising an amino acid sequence represented by SEQ ID NO: 146, and a light chain comprising an amino acid sequence represented by SEQ ID NO: 147,
- a heavy chain comprising an amino acid sequence represented by SEQ ID NO: 148, and a light chain comprising an amino acid sequence represented by SEQ ID NO: 149, or
- a heavy chain comprising an amino acid sequence represented by SEQ ID NO: 150, and a light chain comprising an amino acid sequence represented by SEQ ID NO: 151.

**21.** An antibody or an antigen-binding fragment of the antibody, wherein the antibody or the antigen-binding fragment comprises an amino acid sequence encoded by a nucleotide sequence contained in a polynucleotide hybridizing under stringent conditions to a complementary strand of a polynucleotide comprising a nucleotide sequence encoding an amino acid sequence contained in the antibody or the antigen-binding fragment of the antibody according to any one of claims 8 to 12 and 18 to 20, and binds to human GPRC5D.

**22.** An antibody or an antigen-binding fragment of the antibody, wherein the antibody or the antigen-binding fragment comprises an amino acid sequence 90% or more identical to an amino acid sequence contained in the antibody or the antigen-binding fragment of the antibody according to any one of claims 8 to 12 and 18 to 20, and binds to human GPRC5D.

**23.** An antibody or an antigen-binding fragment of the antibody, wherein the antibody or the antigen-binding fragment comprises an amino acid sequence derived by the substitution, deletion, or addition of 1 to several amino acid(s) from an amino acid sequence contained in the antibody or the antigen-binding fragment of the antibody according to any one of claims 8 to 12 and 18 to 20, and binds to human GPRC5D.

**24.** An antibody or an antigen-binding fragment of the antibody, wherein the antibody or the antigen-binding fragment binds to a site on human GPRC5D bound by the antibody or the antigen-binding fragment of the antibody according to any one of claims 1 to 20.

**25.** An antibody or an antigen-binding fragment of the antibody, wherein the antibody or the antigen-binding fragment competes with the antibody or the antigen-binding fragment of the antibody according to any one of claims 1 to 20 for that binds to human GPRC5D.

**26.** The antibody or the antigen-binding fragment according to any one of claims 1 to 25, wherein the antibody or the antigen-binding fragment binds to cynomolgus monkey GPRC5D.

**27.** The antibody or the antigen-binding fragment of the antibody according to any one of claims 1 to 26, wherein the antigen-binding fragment is Fa', F(ab)', Fv, scFv, or sdAb.

**28.** An antibody or an antigen-binding fragment of the antibody, wherein the antibody binds to human GPRC5D, comprising a heavy chain variable region and a light chain variable region according to claim 2, 8 or 9, and

i) a heavy chain constant region comprising an amino acid sequence represented by amino acid residues 147 to 475 of an amino acid sequence represented by SEQ ID NO: 199, and a light chain constant region comprising an amino acid sequence represented by amino acid residues 131 to 237 of the amino acid sequence represented by SEQ ID NO: 203,
ii) a heavy chain constant region comprising an amino acid sequence represented by amino acid residues 147 to 475 of an amino acid sequence represented by SEQ ID NO: 201, and a light chain constant region comprising an amino acid sequence represented by amino acid residues 131 to 237 of an amino acid sequence represented by SEQ ID NO: 205,
iii) a heavy chain constant region comprising an amino acid sequence represented by amino acid residues 147 to 475 of an amino acid sequence represented by SEQ ID NO: 215, and a light chain constant region comprising an amino acid sequence represented by amino acid residues 131 to 237 of an amino acid sequence represented by SEQ ID NO: 217,
or
iv) a heavy chain constant region comprising an amino acid sequence represented by amino acid residues 147 to 476 of an amino acid sequence represented by SEQ ID NO: 237, and a light chain constant region comprising an amino acid sequence represented by amino acid residues 131 to 237 of an amino acid sequence represented by SEQ ID NO: 217.

**29.** An antibody or an antigen-binding fragment of the antibody, wherein the antibody binds to human GPRC5D, comprising a heavy chain variable region comprising an amino acid sequence represented by amino acid residues 20 to 142 of the amino acid sequence represented by SEQ ID NO: 76, and a light chain variable region comprising an amino acid sequence represented by amino acid residues 21 to 127 of the amino acid sequence represented by SEQ ID NO: 72, and

i) a heavy chain constant region comprising an amino acid sequence represented by amino acid residues 147 to 475 of the amino acid sequence represented by SEQ ID NO: 199, and a light chain constant region comprising an amino acid sequence represented by amino acid residues 131 to 237 of the amino acid sequence represented by SEQ ID NO: 203,
ii) a heavy chain constant region comprising an amino acid sequence represented by amino acid residues 147 to 475 of the amino acid sequence represented by SEQ ID NO: 201, and a light chain constant region comprising an amino acid sequence represented by amino acid residues 131 to 237 of the amino acid sequence represented by SEQ ID NO: 205,
iii) a heavy chain constant region comprising an amino acid sequence represented by amino acid residues 147 to 475 of the amino acid sequence represented by SEQ ID NO: 215, and a light chain constant region comprising an amino acid sequence represented by amino acid residues 131 to 237 of the amino acid sequence represented by SEQ ID NO: 217,

or

iv) a heavy chain constant region comprising an amino acid sequence represented by amino acid residues 147 to 476 of the amino acid sequence represented by SEQ ID NO: 237, and a light chain constant region comprising an amino acid sequence represented by amino acid residues 131 to 237 of the amino acid sequence represented by SEQ ID NO: 217.

**30.** An antibody or an antigen-binding fragment of the antibody, wherein the antibody binds to human GPRC5D, comprising a heavy chain variable region and a light chain variable region according to claim 2, 8 or 9, and an Fc mutation.

**31.** An antibody or an antigen-binding fragment of the antibody, wherein the antibody binds to human GPRC5D, comprising a heavy chain variable region comprising an amino acid sequence represented by amino acid residues 20 to 142 of the amino acid sequence represented by SEQ ID NO: 76 and a light chain variable region comprising an amino acid sequence represented by amino acid residues 21 to 127 of the amino acid sequence represented by SEQ ID NO: 72, and an Fc mutation.

**32.** A polynucleotide encoding the antibody or the antigen-binding fragment of the antibody according to any one of claims 1 to 31.

**33.** A vector comprising any one of the polynucleotides according to claim 32.

**34.** A cell comprising any one of the polynucleotides according to claim 32 or the vector according to claim 33, or for producing the antibody or the antigen-binding fragment of the antibody according to any one of claims 1 to 31.

**35.** An artificial immunocyte comprising any one of the polynucleotides according to claim 32 or the vector according to claim 33, or for expressing the antibody or the antigen-binding fragment of the antibody according to any one of claims 1 to 31 on the cell surface.

**36.** A method for producing an antibody or an antigen-binding fragment of the antibody which binds to human GPRC5D, comprising steps of: culturing the cell according to claim 34; and recovering an antibody or an antigen-binding fragment of the antibody which binds to human GPRC5D from the cultures.

**37.** An antibody or an antigen-binding fragment of the antibody which binds to human GPRC5D, the antibody or the antigen-binding fragment being obtained by the method according to claim 36.

**38.** A pharmaceutical composition for treatment and/or prevention, comprising the antibody or the antigen-binding fragment of the antibody according to any one of claims 1 to 31 and 37, the polynucleotide according to claim 32, the vector according to claim 33, or the artificial immunocyte according to claim 35 as an active ingredient.

**39.** The pharmaceutical composition according to claim 38, wherein the pharmaceutical composition is for the treatment and/or prevention of a cancer.

**40.** The pharmaceutical composition according to claim 39, wherein the cancer is breast cancer, endometrial cancer, ovary cancer, lung cancer, stomach cancer, prostate cancer, kidney cancer, liver cancer, pancreatic cancer, colorectal cancer, esophageal cancer, urinary bladder cancer, uterine cervix cancer, blood cancer, lymphoma, or malignant melanoma expressing a GPRC5D protein.

**41.** The pharmaceutical composition according to claim 40, wherein the cancer is multiple myeloma expressing a GPRC5D protein.

**42.** A molecule having antigen binding activity, comprising the antibody or the antigen-binding fragment of the antibody according to any one of claims 1 to 31 and 37.

**43.** The molecule according to claim 42, wherein the molecule is multispecific.

**44.** The molecule according to claim 42 or 43, wherein the molecule comprises the antibody that binds to human GPRC5D or the antigen-binding fragment of the antibody according to any one of claims 1 to 31 and 37, and

an antibody or an antigen-binding fragment of the antibody comprising
a heavy chain variable region comprising
heavy chain CDR1 comprising an amino acid sequence represented by SEQ ID NO: 183,
heavy chain CDR2 comprising an amino acid sequence represented by SEQ ID NO: 238, and
heavy chain CDR3 comprising an amino acid sequence represented by SEQ ID NO: 185, and
a light chain variable region comprising
light chain CDR1 comprising an amino acid sequence represented by SEQ ID NO: 186,
light chain CDR2 comprising an amino acid sequence represented by SEQ ID NO: 239, and
light chain CDR3 comprising an amino acid sequence represented by SEQ ID NO: 188, and
a binds to human CD3 and cynomolgus monkey CD3.

**45.** The molecule according to claim 44, wherein, in the heavy chain CDR2,
a first $X_{aa}$ is selected from a group consisting of (A, E, G, H, I, L, T, V, R, and S), and
a second $X_{aa}$ is S; or
the first $X_{aa}$ is N, and
the second $X_{aa}$ is selected from a group consisting of (E, R, F, Y, L, V, I, K, and T), and
in the light chain CDR2,
$X_{aa}$ is selected from a group consisting of (Q, A, G, S, N, and D)
and
the molecule binds to human CD3 and cynomolgus monkey CD3.

**46.** The molecule according to claim 44 or 45, wherein, in the heavy chain CDR2,
the first $X_{aa}$ is selected from a group consisting of (R and S), and the second $X_{aa}$ is S, and
in the light chain CDR2,
$X_{aa}$ is selected from a group consisting of (Q, A, G, S, N, and D), and
binds to human CD3 and cynomolgus monkey CD3.

**47.** The molecule according to any one of claims 42 to 45, comprising a heavy chain variable region comprising an amino acid sequence represented by SEQ ID NO: 240 and a light chain variable region comprising an amino acid sequence represented by any one of SEQ ID NOs: 241, 242, and 243; wherein
in the amino acid sequence represented by SEQ ID NO: 240, a first $X_{aa}$ is selected from a group consisting of (A, E, G, H, I, L, T, V, R, and S), and
a second $X_{aa}$ is S; or
the first $X_{aa}$ is N, and the second $X_{aa}$ is selected from a group consisting of (E, R, F, Y, L, V, I, K, and T), and
in the amino acid sequence represented by any one of SEQ ID NOs: 241, 242, and 243,
$X_{aa}$ is selected from a group consisting of (Q, A, G, S, N, and D).

**48.** The molecule according to claim 47, wherein
the first $X_{aa}$ is selected from a group consisting of (R and S), and
the second $X_{aa}$ is S, and
in an amino acid sequence represented by any one of SEQ ID NOs: 241, 242, and 243,
$X_{aa}$ is selected from a group consisting of (Q, A, G, S, N, and D).

**49.** The molecule according to any one of claims 42 to 45, wherein the molecule comprising the antibody that binds to human GPRC5D or the antigen-binding fragment of the antibody according to any one of claims 1 to 31 and 37, and an antibody or an antigen-binding fragment of the antibody that comprises a heavy chain variable region comprising
the amino acid sequence of heavy chain CDR1 represented by SEQ ID NO: 183,
the amino acid sequence of heavy chain CDR2 represented by SEQ ID NO: 184, and
the amino acid sequence of heavy chain CDR3 represented by SEQ ID NO: 185, and
a light chain variable region comprising
the amino acid sequence of light chain CDR1 represented by SEQ ID NO: 186,
the amino acid sequence of light chain CDR2 represented by SEQ ID NO: 187, and
the amino acid sequence of light chain CDR3 represented by SEQ ID NO: 188, and binds to human CD3 and cynomolgus monkey CD3.

**50.** The molecule according to claim 49, wherein the antibody that binds to human CD3 and cynomolgus monkey CD3 or the antigen-binding fragment of the antibody is an antibody or an antigen-binding fragment of the antibody comprising a heavy chain variable region comprising the amino acid sequence represented by SEQ ID NO: 155, and a light chain variable region comprising an amino acid sequence represented by any one of SEQ ID NOs: 156, 158, and 160.

**51.** The molecule according to any one of claims 44 to 50, wherein the antigen-binding fragment of the antibody that binds to human CD3 and cynomolgus monkey CD3 is Fab, F(ab)', Fv, scFv, or sdAb.

**52.** The molecule according to any one of claims 44 to 51, wherein the antibody that binds to human CD3 and cynomolgus monkey CD3 is a humanized antibody or a human antibody comprising a human immunoglobulin constant region, or Fc or an Fc mutation.

**53.** The molecule according to any one of claims 44 to 52, wherein the antibody that binds to human CD3 and cynomolgus monkey CD3 or the antigen-binding fragment of the antibody is an antibody or an antigen-binding fragment of the antibody comprising an amino acid sequence represented by any one of SEQ ID NOs: 180, 181, and 182.

**54.** The molecule according to any one of claims 40 to 44, wherein the antibody that binds to human CD3 and cynomolgus monkey CD3 or the antigen-binding fragment of the antibody is bound with the antibody or the antigen-binding fragment of the antibody according to any one of claims 1 to 31 and 37 via a linker or without a linker.

**55.** The molecule according to any one of claims 42 to 44, wherein the molecule comprises the antibody that binds to human GPRC5D or the antigen-binding fragment of the antibody according to claims 2, 8 or 9, and an antibody that binds to human CD3 and cynomolgus monkey CD3 or an antigen-binding fragment of the antibody comprising any one heavy change variable region and light chain variable region selected from a group shown below, comprising

• a heavy chain variable region comprising an amino acid sequence represented by amino acid residues 25 to 142 of the amino acid sequence represented by SEQ ID NO: 207, and a light chain variable region comprising an amino acid sequence represented by amino acid residues 24 to 132 of the amino acid sequence represented by SEQ ID NO: 209,
• a heavy chain variable region comprising an amino acid sequence represented by amino acid residues 25 to 142 of the amino acid sequence represented by SEQ ID NO: 211, and a light chain variable region comprising an amino acid sequence represented by amino acid residues 24 to 130 of the amino acid sequence represented by SEQ ID NO: 213,
• a heavy chain variable region comprising an amino acid sequence represented by amino acid residues 2 to 119 of SEQ ID NO: 244, and a light chain variable region comprising an amino acid sequence represented by amino acid residues 135 to 243 of SEQ ID NO: 244,
• a heavy chain variable region comprising an amino acid sequence represented by amino acid residues 2 to 119 of SEQ ID NO: 245, and a light chain variable region comprising an amino acid sequence represented by amino acid residues 135 to 241 of SEQ ID NO: 245,
• a heavy chain variable region comprising an amino acid sequence represented by amino acid residues 2 to 119 of SEQ ID NO: 246, and a light chain variable region comprising an amino acid sequence represented by amino acid residues 135 to 243 of SEQ ID NO: 246,
• a heavy chain variable region comprising an amino acid sequence represented by amino acid residues 2 to 119 of SEQ ID NO: 247, and a light chain variable region comprising an amino acid sequence represented by amino acid residues 135 to 243 of SEQ ID NO: 247,
• a heavy chain variable region comprising an amino acid sequence represented by amino acid residues 2 to 119 of SEQ ID NO: 248, and a light chain variable region comprising an amino acid sequence represented by amino acid residues 135 to 243 of SEQ ID NO: 248,
• a heavy chain variable region comprising an amino acid sequence represented by amino acid residues 2 to 119 of SEQ ID NO: 249, and a light chain variable region comprising an amino acid sequence represented by amino acid residues 135 to 243 of SEQ ID NO: 249,
• a heavy chain variable region comprising an amino acid sequence represented by amino acid residues 2 to 119 of SEQ ID NO: 250, and a light chain variable region comprising an amino acid sequence represented by amino acid residues 135 to 243 of SEQ ID NO: 250,
• a heavy chain variable region comprising an amino acid sequence represented by amino acid residues 2 to 119 of SEQ ID NO: 251, and a light chain variable region comprising an amino acid sequence represented by amino acid residues 135 to 243 of SEQ ID NO: 251,
• a heavy chain variable region comprising an amino acid sequence represented by amino acid residues 2 to 119 of SEQ ID NO: 252, and a light chain variable region comprising an amino acid sequence represented by amino acid residues 135 to 243 of SEQ ID NO: 252,

• a heavy chain variable region comprising an amino acid sequence represented by amino acid residues 2 to 119 of SEQ ID NO: 253, and a light chain variable region comprising an amino acid sequence represented by amino acid residues 135 to 242 of SEQ ID NO: 253,
• a heavy chain variable region comprising an amino acid sequence represented by amino acid residues 2 to 119 of SEQ ID NO: 254, and a light chain variable region comprising an amino acid sequence represented by amino acid residues 135 to 243 of SEQ ID NO: 254, or
• a heavy chain variable region comprising an amino acid sequence represented by amino acid residues 2 to 119 of SEQ ID NO: 255, and a light chain variable region comprising an amino acid sequence represented by amino acid residues 135 to 243 of SEQ ID NO: 255, and comprises an antibody that binds to human CD3 and cynomolgus monkey CD3 or an antigen-binding fragment of the antibody.

**56.** The molecule according to claim 55, wherein the antibody that binds to human GPRC5D or the antigen-binding fragment of the antibody comprising a heavy chain variable region comprising an amino acid sequence represented by amino acid residues 20 to 142 of the amino acid sequence represented by SEQ ID NO: 76, and a light chain variable region comprising an amino acid sequence represented by amino acid residues 21 to 127 of the amino acid sequence represented by SEQ ID NO: 72, and

i) a heavy chain constant region comprising an amino acid sequence represented by amino acid residues 147 to 475 of the amino acid sequence represented by SEQ ID NO: 199, and a light chain constant region comprising an amino acid sequence represented by amino acid residues 131 to 237 of the amino acid sequence represented by SEQ ID NO: 203,
ii) a heavy chain constant region comprising an amino acid sequence represented by amino acid residues 147 to 475 of the amino acid sequence represented by SEQ ID NO: 201, and a light chain constant region comprising an amino acid sequence represented by amino acid residues 131 to 237 of the amino acid sequence represented by SEQ ID NO: 205,
iii) a heavy chain constant region comprising an amino acid sequence represented by amino acid residues 147 to 475 of the amino acid sequence represented by SEQ ID NO: 215, and a light chain constant region comprising an amino acid sequence represented by amino acid residues 131 to 237 of the amino acid sequence represented by SEQ ID NO: 217,
or
iv) a heavy chain constant region comprising an amino acid sequence represented by amino acid residues 147 to 476 of the amino acid sequence represented by SEQ ID NO: 237, and a light chain constant region comprising an amino acid sequence represented by amino acid residues 131 to 237 of the amino acid sequence represented by SEQ ID NO: 217, and
the antibody that binds to human CD3 and cynomolgus monkey CD3 or the antigen-binding fragment of the antibody further comprises an Fc mutation.

**57.** The molecule according to claim 55, wherein the antibody that binds to human GPRC5D or the antigen-binding fragment of the antibody comprises a heavy chain variable region comprising an amino acid sequence represented by amino acid residues 20 to 142 of the amino acid sequence represented by SEQ ID NO: 76, and a light chain variable region comprising an amino acid sequence represented by amino acid residues 21 to 127 of the amino acid sequence represented by SEQ ID NO: 72, and

iii) a heavy chain constant region comprising an amino acid sequence represented by amino acid residues 147 to 475 of the amino acid sequence represented by SEQ ID NO: 215, and a light chain constant region comprising an amino acid sequence represented by amino acid residues 131 to 237 of the amino acid sequence represented by SEQ ID NO: 217,
or
iv) a heavy chain constant region comprising an amino acid sequence represented by amino acid residues 147 to 476 of the amino acid sequence represented by SEQ ID NO: 237, and a light chain constant region comprising an amino acid sequence represented by amino acid residues 131 to 237 of the amino acid sequence represented by SEQ ID NO: 217,
and
the antibody that binds to human CD3 and cynomolgus monkey CD3 or the antigen-binding fragment of the antibody further comprises an Fc mutation.

**58.** The molecule according to claim 55, comprising
the antibody that binds to human GPRC5D or the antigen-binding fragment of the antibody comprising a heavy chain comprising an amino acid sequence represented by amino acid residues 24 to 475 of the amino acid sequence represented by SEQ ID NO: 215 and a light chain comprising an amino acid sequence represented by amino acid residues 24 to 237 of the amino acid sequence represented by SEQ ID NO: 217, and the antibody that binds to human CD3 and cynomolgus monkey CD3 or the antigen-binding fragment of the antibody, comprising an amino acid sequence represented by amino acid residues 24 to 266 of the amino acid sequence represented by SEQ ID NO: 219 and an Fc mutation;
the antibody that binds to human GPRC5D or the antigen-binding fragment of the antibody comprising a heavy chain comprising an amino acid sequence represented by amino acid residues 24 to 475 of the amino acid sequence represented by SEQ ID NO: 215 and a light chain comprising an amino acid sequence represented by amino acid residues 24 to 237 of the amino acid sequence represented by SEQ ID NO: 217, and the antibody that binds to human CD3 and cynomolgus monkey CD3 or the antigen-binding fragment of the antibody, comprising an amino acid sequence represented by amino acid residues 24 to 264 of the amino acid sequence represented by SEQ ID NO: 221 and an Fc mutation;
the antibody that binds to human GPRC5D or the antigen-binding fragment of the antibody comprising a heavy chain comprising an amino acid sequence represented by amino acid residues 24 to 475 of the amino acid sequence represented by SEQ ID NO: 215 and a light chain comprising an amino acid sequence represented by amino acid residues 24 to 237 of the amino acid sequence represented by SEQ ID NO: 217, and the antibody that binds to human CD3 and cynomolgus monkey CD3 or the antigen-binding fragment of the antibody, comprising an amino acid sequence represented by amino acid residues 24 to 264 of the amino acid sequence represented by SEQ ID NO: 225 and an Fc mutation;
the antibody that binds to human GPRC5D or the antigen-binding fragment of the antibody comprising a heavy chain comprising an amino acid sequence represented by amino acid residues 24 to 475 of the amino acid sequence represented by SEQ ID NO: 215 and a light chain comprising an amino acid sequence represented by amino acid residues 24 to 237 of the amino acid sequence represented by SEQ ID NO: 217, and the antibody that binds to human CD3 and cynomolgus monkey CD3 or the antigen-binding fragment of the antibody, comprising an amino acid sequence represented by amino acid residues 24 to 266 of the amino acid sequence represented by SEQ ID NO: 227 and an Fc mutation;
the antibody that binds to human GPRC5D or the antigen-binding fragment of the antibody comprising a heavy chain comprising an amino acid sequence represented by amino acid residues 24 to 475 of the amino acid sequence represented by SEQ ID NO: 215 and a light chain comprising an amino acid sequence represented by amino acid residues 24 to 237 of the amino acid sequence represented by SEQ ID NO: 217, and the antibody that binds to human CD3 and cynomolgus monkey CD3 or the antigen-binding fragment of the antibody, comprising an amino acid sequence represented by amino acid residues 24 to 266 of the amino acid sequence represented by SEQ ID NO: 229 and an Fc mutation;
the antibody that binds to human GPRC5D or the antigen-binding fragment of the antibody comprising a heavy chain comprising an amino acid sequence represented by amino acid residues 24 to 476 of the amino acid sequence represented by SEQ ID NO: 215 and a light chain comprising an amino acid sequence represented by amino acid residues 24 to 237 of the amino acid sequence represented by SEQ ID NO: 217, and the antibody that binds to human CD3 and cynomolgus monkey CD3 or the antigen-binding fragment of the antibody, comprising an amino acid sequence represented by amino acid residues 24 to 264 of the amino acid sequence represented by SEQ ID NO: 231 and an Fc mutation;
the antibody that binds to human GPRC5D or the antigen-binding fragment of the antibody comprising a heavy chain comprising an amino acid sequence represented by amino acid residues 24 to 476 of the amino acid sequence represented by SEQ ID NO: 215 and a light chain comprising an amino acid sequence represented by amino acid residues 24 to 237 of an amino acid sequence represented by SEQ ID NO: 217, and the antibody that binds to human CD3 and cynomolgus monkey CD3 or the antigen-binding fragment of the antibody, comprising an amino acid sequence represented by amino acid residues 24 to 266 of the amino acid sequence represented by SEQ ID NO: 233 and an Fc mutation; or
the antibody that binds to human GPRC5D or the antigen-binding fragment of the antibody comprising a heavy chain comprising an amino acid sequence represented by amino acid residues 24 to 476 of the amino acid sequence represented by SEQ ID NO: 237 and a light chain comprising an amino acid sequence represented by amino acid residues 24 to 237 of the amino acid sequence represented by SEQ ID NO: 217, and the antibody that binds to human CD3 and cynomolgus monkey CD3 or the antigen-binding fragment of the antibody, comprising an amino acid sequence represented by amino acid residues 24 to 266 of the amino acid sequence represented by SEQ ID NO: 235 and an Fc mutation.

**59.** The molecule according to claim 55, comprising
the antibody that binds to human GPRC5D or the antigen-binding fragment of the antibody comprising a heavy chain comprising an amino acid sequence represented by amino acid residues 24 to 475 of the amino acid sequence represented by SEQ ID NO: 215 and a light chain comprising an amino acid sequence represented by amino acid residues 24 to 237 of the amino acid sequence represented by SEQ ID NO: 217, and the antibody that binds to human CD3 and cynomolgus monkey CD3 or the antigen-binding fragment of the antibody, comprising an amino acid sequence represented by amino acid residues 24 to 264 of the amino acid sequence represented by SEQ ID NO: 225 and an Fc mutation;
the antibody that binds to human GPRC5D or the antigen-binding fragment of the antibody comprising a heavy chain comprising an amino acid sequence represented by amino acid residues 24 to 475 of the amino acid sequence represented by SEQ ID NO: 215 and a light chain comprising an amino acid sequence represented by amino acid residues 24 to 237 of the amino acid sequence represented by SEQ ID NO: 217, and
the antibody that binds to human CD3 and cynomolgus monkey CD3 or an antigen-binding fragment of the antibody comprising an amino acid sequence represented by amino acid residues 24 to 266 of the amino acid sequence represented by SEQ ID NO: 227 and an Fc mutation;
the antibody that binds to human GPRC5D or the antigen-binding fragment of the antibody comprising a heavy chain comprising an amino acid sequence represented by amino acid residues 24 to 475 of the amino acid sequence represented by SEQ ID NO: 215 and a light chain comprising an amino acid sequence represented by amino acid residues 24 to 237 of the amino acid sequence represented by SEQ ID NO: 217, and the antibody that binds to human CD3 and cynomolgus monkey CD3 or the antigen-binding fragment of the antibody, comprising an amino acid sequence represented by amino acid residues 24 to 266 of the amino acid sequence represented by SEQ ID NO: 229 and an Fc mutation;
the antibody that binds to human GPRC5D or the antigen-binding fragment of the antibody comprising a heavy chain comprising an amino acid sequence represented by amino acid residues 24 to 476 of the amino acid sequence represented by SEQ ID NO: 237 and a light chain comprising an amino acid sequence represented by amino acid residues 24 to 237 of the amino acid sequence represented by SEQ ID NO: 217, and the antibody that binds to human CD3 and cynomolgus monkey CD3 or the antigen-binding fragment of the antibody, comprising an amino acid sequence represented by amino acid residues 24 to 264 of the amino acid sequence represented by SEQ ID NO: 231 and an Fc mutation;
the antibody that binds to human GPRC5D or the antigen-binding fragment of the antibody comprising a heavy chain comprising an amino acid sequence represented by amino acid residues 24 to 476 of the amino acid sequence represented by SEQ ID NO: 237 and a light chain comprising an amino acid sequence represented by amino acid residues 24 to 237 of the amino acid sequence represented by SEQ ID NO: 217, and the antibody that binds to human CD3 and cynomolgus monkey CD3 or the antigen-binding fragment of the antibody, comprising an amino acid sequence represented by amino acid residues 24 to 266 of the amino acid sequence represented by SEQ ID NO: 233 and an Fc mutation; or
the antibody that binds to human GPRC5D or the antigen-binding fragment of the antibody comprising a heavy chain comprising an amino acid sequence represented by amino acid residues 24 to 476 of the amino acid sequence represented by SEQ ID NO: 237 and a light chain comprising an amino acid sequence represented by amino acid residues 24 to 237 of the amino acid sequence represented by SEQ ID NO: 217, and an antibody that binds to human CD3 and cynomolgus monkey CD3 or antigen-binding fragment of the antibody comprising an amino acid sequence represented by amino acid residues 24 to 266 of the amino acid sequence represented by SEQ ID NO: 235 and an Fc mutation.

**60.** The molecule according to claim 55, wherein the antibody that binds to human GPRC5D or the antigen-binding fragment of the antibody comprises a heavy chain variable region comprising an amino acid sequence represented by amino acid residues 20 to 142 of the amino acid sequence represented by SEQ ID NO: 76 and a light chain variable region comprising an amino acid sequence represented by amino acid residues 21 to 127 of the amino acid sequence represented by SEQ ID NO: 72, and comprises

i) a heavy chain constant region comprising an amino acid sequence represented by amino acid residues 147 to 475 of the amino acid sequence represented by SEQ ID NO: 199, and a light chain constant region comprising an amino acid sequence represented by amino acid residues 131 to 237 of the amino acid sequence represented by SEQ ID NO: 203,
ii) a heavy chain constant region comprising an amino acid sequence represented by amino acid residues 147 to 475 of the amino acid sequence represented by SEQ ID NO: 201, and a light chain constant region comprising an amino acid sequence represented by amino acid residues 131 to 237 of the amino acid sequence represented by SEQ ID NO: 205,
iii) a heavy chain constant region comprising an amino acid sequence represented by amino acid residues 147 to 475 of the amino acid sequence represented by SEQ ID NO: 215, and a light chain constant region comprising an amino acid sequence represented by amino acid residues 131 to 237 of the amino acid sequence represented by SEQ ID NO: 217,

or

iv) a heavy chain constant region comprising an amino acid sequence represented by amino acid residues 147 to 476 of the amino acid sequence represented by SEQ ID NO: 237, and a light chain constant region comprising an amino acid sequence represented by amino acid residues 131 to 237 of the amino acid sequence represented by SEQ ID NO: 217,

and

the antibody that binds to human CD3 and cynomolgus monkey CD3 or the antigen-binding fragment of the antibody further comprises

v) a heavy chain constant region comprising an amino acid sequence represented by amino acid residues 143 to 471 of the amino acid sequence represented by SEQ ID NO: 207, and a light chain constant region comprising an amino acid sequence represented by amino acid residues 133 to 238 of the amino acid sequence represented by SEQ ID NO: 209, or

vi) a heavy chain constant region comprising an amino acid sequence represented by amino acid residues 143 to 471 of the amino acid sequence represented by SEQ ID NO: 211, and a light chain constant region comprising an amino acid sequence represented by amino acid residues 131 to 236 of the amino acid sequence represented by SEQ ID NO: 213.

**61.** The molecule according to claim 55, wherein the antibody that binds to human GPRC5D or the antigen-binding fragment of the antibody comprises a heavy chain variable region comprising amino acid residues 20 to 142 of SEQ ID NO: 76 and a light chain variable region comprising an amino acid sequence represented by amino acid residues 21 to 127 of SEQ ID NO: 72, and

iii) a heavy chain constant region comprising an amino acid sequence represented by amino acid residues 147 to 475 of the amino acid sequence represented by SEQ ID NO: 215, and a light chain constant region comprising an amino acid sequence represented by amino acid residues 131 to 237 of the amino acid sequence represented by SEQ ID NO: 217, or

iv) a heavy chain constant region comprising an amino acid sequence represented amino acid residues 147 to 476 of the amino acid sequence represented by SEQ ID NO: 237, and a light chain constant region comprising an amino acid sequence represented amino acid residues 131 to 237 of the amino acid sequence represented by SEQ ID NO: 217, and

the antibody that binds to human CD3 and cynomolgus monkey CD3 or the antigen-binding fragment of the antibody, and further comprises

v) a heavy chain constant region comprising an amino acid sequence represented by amino acid residues 143 to 471 of the amino acid sequence represented by SEQ ID NO: 207, and a light chain constant region comprising an amino acid sequence represented by amino acid residues 133 to 238 of the amino acid sequence represented by SEQ ID NO: 209, or

vi) a heavy chain constant region comprising an amino acid sequence represented by amino acid residues 143 to 471 of the amino acid sequence represented by SEQ ID NO: 211, and a light chain constant region comprising an amino acid sequence represented by amino acid residues 131 to 236 of the amino acid sequence represented by SEQ ID NO: 213.

**62.** The molecule according to claim 55, comprising

the antibody that binds to human GPRC5D or the antigen-binding fragment of the antibody comprising a heavy chain comprising an amino acid sequence represented by amino acid residues 24 to 475 of the amino acid sequence represented by SEQ ID NO: 199 and a light chain comprising an amino acid sequence represented by amino acid residues 24 to 237 of the amino acid sequence represented by SEQ ID NO: 203, and the antibody that binds to human CD3 and cynomolgus monkey CD3 or the antigen-binding fragment of the antibody, comprising a heavy chain comprising an amino acid sequence represented by amino acid residues 25 to 471 of the amino acid sequence represented by SEQ ID NO: 207 and a light chain comprising an amino acid sequence represented by amino acid residues 24 to 238 of the amino acid sequence represented by SEQ ID NO: 209

or

the antibody that binds to human GPRC5D or the antigen-binding fragment of the antibody comprising a heavy chain comprising an amino acid sequence represented by amino acid residues 24 to 475 of the amino acid sequence represented by SEQ ID NO: 201 and a light chain comprising an amino acid sequence represented by amino acid residues 24 to 237 of the amino acid sequence represented by SEQ ID NO: 205, and the antibody that binds to human CD3 and cynomolgus monkey CD3 or the antigen-binding fragment of the antibody, comprising a heavy chain comprising an amino acid sequence represented by amino acid residues 25 to 471 of the amino acid sequence represented by SEQ ID NO: 211 and a light chain comprising an amino acid sequence represented by amino acid residues 24 to 236 of the amino acid sequence represented by SEQ ID NO: 213.

**63.** The molecule according to claim 55, wherein the antibody that binds to human GPRC5D or the antigen-binding fragment of the antibody further comprises a heavy chain variable region comprising an amino acid sequence represented by amino acid residues 20 to 142 of the amino acid sequence represented by SEQ ID NO: 76 and a light chain variable region comprising an amino acid sequence represented by amino acid residues 21 to 127 of the amino acid sequence represented by SEQ ID NO: 72, and further comprises an Fc mutation, and the antibody that binds to human CD3 and cynomolgus monkey CD3 or the antigen-binding fragment of the antibody further comprises an Fc mutation.

**64.** The molecule according to claim 55, comprising
the antibody that binds to human GPRC5D or an antigen-binding fragment of the antibody comprising an amino acid sequence represented by amino acid residues 24 to 271 of the amino acid sequence represented by SEQ ID NO: 223 and an Fc mutation, and the antibody that binds to human CD3 and cynomolgus monkey CD3 or the antigen-binding fragment of the antibody comprising an amino acid sequence represented by amino acid residues 24 to 266 of the amino acid sequence represented by SEQ ID NO: 219 and an Fc mutation, or
the antibody that binds to human GPRC5D or the antigen-binding fragment of the antibody comprising amino acid sequence represented by amino acid residues 24 to 271 of the amino acid sequence represented by SEQ ID NO: 223 and an Fc mutation, and the antibody that binds to human CD3 and cynomolgus monkey CD3 or the antigen-binding fragment of the antibody comprising an amino acid sequence represented by amino acid residues 24 to 264 of the amino acid sequence represented by SEQ ID NO: 221 and an Fc mutation.

**65.** The molecule according to claim 53, wherein the antibody that binds to human CD3 and cynomolgus monkey CD3 or the antigen-binding fragment of the antibody is bound with the antibody or the antigen-binding fragment of the antibody according to claim 19 via a linker or without a linker.

**66.** The molecule that binds to human CD3 and cynomolgus monkey CD3, and to human GPRC5D according to claim 54 or 65, wherein the molecule has an amino acid sequence represented by any one of SEQ ID NOs: 171 to 179.

**67.** A molecule comprising an amino acid sequence encoded by a nucleotide sequence contained in a polynucleotide hybridizing under stringent conditions to a complementary strand of a polynucleotide comprising a nucleotide sequence encoding an amino acid sequence contained in the antibody that binds to human CD3 and cynomolgus monkey CD3 or the antigen-binding fragment of the antibody contained in the molecule according to any one of claims 50, 53, 58, 59, 62, 64, 65, and 66, and that binds to human CD3 and cynomolgus monkey CD3, and to human GPRC5D.

**68.** A molecule comprising an amino acid sequence 90% or more identical to the amino acid sequence contained in the antibody that binds to human CD3 and cynomolgus monkey CD3 or the antigen-binding fragment of the antibody according to any one of claims 50, 53, 58, 59, 62, 64, 65, and 66, and binds to human CD3 and cynomolgus monkey CD3, and to human GPRC5D.

**69.** A molecule comprising an amino acid sequence derived by the substitution, deletion, or addition of 1 to several amino acid(s) from an amino acid sequence contained in the antibody that binds to human CD3 and cynomolgus monkey CD3 or the antigen-binding fragment of the antibody contained in the molecule according to any one of claims 50, 53, 58, 59, 62, 64, 65, and 66, and binds to human CD3 and cynomolgus monkey CD,3 and to human GPRC5D.

**70.** The molecule according to any one of claims 42 to 69, wherein the molecule binds to cynomolgus monkey GPRC5D.

**71.** The molecule according to any one of claims 43 to 70, wherein the molecule is bispecific.

**72.** The molecule according to any one of claims 42 to 71, wherein the molecule is a polypeptide.

**73.** A polynucleotide comprising a nucleotide sequence encoding the amino acid sequence of the molecule according to claim 72.

**74.** A vector comprising the polynucleotide according to claim 73.

**75.** A cell producing the polynucleotide according to claim 73 or the vector according to claim 74, or the molecule according to claim 71.

**76.** A method for producing a molecule that binds to human CD3 and cynomolgus monkey CD3, and to human GPRC5D, comprising steps of: culturing the cell according to claim 75; and recovering a molecule that binds to human CD3 and cynomolgus monkey CD3 and/or to human GPRC5D from the cultures.

**77.** A molecule that binds to human CD3 and cynomolgus monkey CD3, and to human GPRC5D, the molecule being obtained by the method according to claim 76.

**78.** The molecule according to claim 77, wherein the molecule binds to cynomolgus monkey GPRC5D.

**79.** A pharmaceutical composition for treatment and/or prevention comprising the molecule according to any one of claims 42 to 72, 77, and 78, the polynucleotide according to claim 73, or the vector according to claim 74 as an active ingredient.

**80.** The pharmaceutical composition according to claim 79, wherein the pharmaceutical composition is for the treatment and/or prevention of a cancer.

**81.** The pharmaceutical composition according to claim 80, wherein the cancer is breast cancer, endometrial cancer, ovary cancer, lung cancer, stomach cancer, prostate cancer, kidney cancer, liver cancer, pancreatic cancer, colorectal cancer, esophageal cancer, urinary bladder cancer, uterine cervix cancer, blood cancer, lymphoma, or malignant melanoma expressing a GPRC5D protein.

**82.** The pharmaceutical composition according to claim 79 or 80, wherein the cancer is multiple myeloma expressing a GPRC5D protein.

**83.** A method for treating and/or preventing a cancer, comprising administering the molecule according to any one of claims 42 to 72, 77, and 78, or the pharmaceutical composition according to any one of claims 79 to 82.

**84.** The pharmaceutical composition according to any one of claims 79 to 82, wherein the pharmaceutical composition induces cytotoxicity to cells expressing GPRC5D by the redirection of T cells to the cells.

**85.** The method according to claim 83, wherein the method induces cytotoxicity to cells expressing GPRC5D by the redirection of T cells to the cells.

**86.** A method for inducing cytotoxicity to cells expressing GPRC5D by the redirection of T cells to the cells, comprising a step of administering the molecule according to any one of claims 42 to 72, 77, and 78, or the pharmaceutical composition according to any one of claims 79 to 82.

**87.** A method for redirecting T cells to cells expressing GPRC5D, comprising a step of administering the molecule according to any one of claims 42 to 72, 77, and 78, or the pharmaceutical composition according to any one of claims 79 to 82.

[Figure 1]

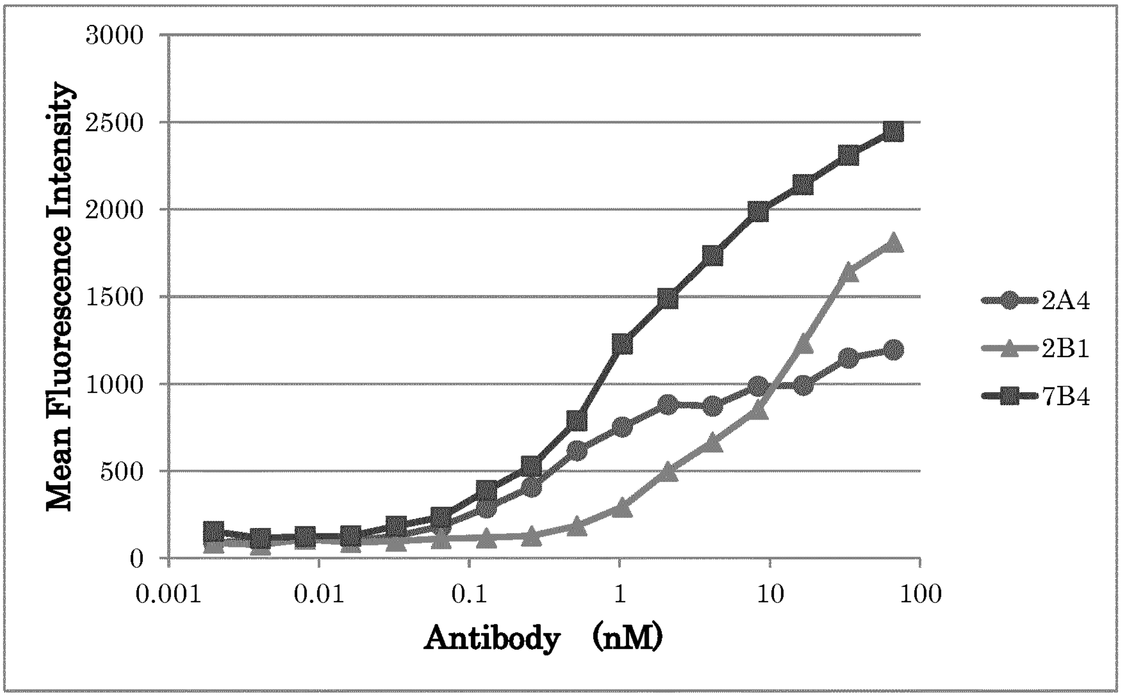


[Figure 2]

MYKDCIESTGDYFLLCDAEGPWGIILE(BIOTIN)-$NH_2$

(Sigma-Aldrich Co. LLC)

(SEQ ID NO: 1 of the Sequence Listing)

[Figure 3]

MYKDCIESTGDYFLLCDAEGPWGIILE-K(BIOTIN)-$NH_2$

(PEPTIDE INSTITUTE, INC.)

(SEQ ID NO: 2 of the Sequence Listing)

[Figure 4]

A

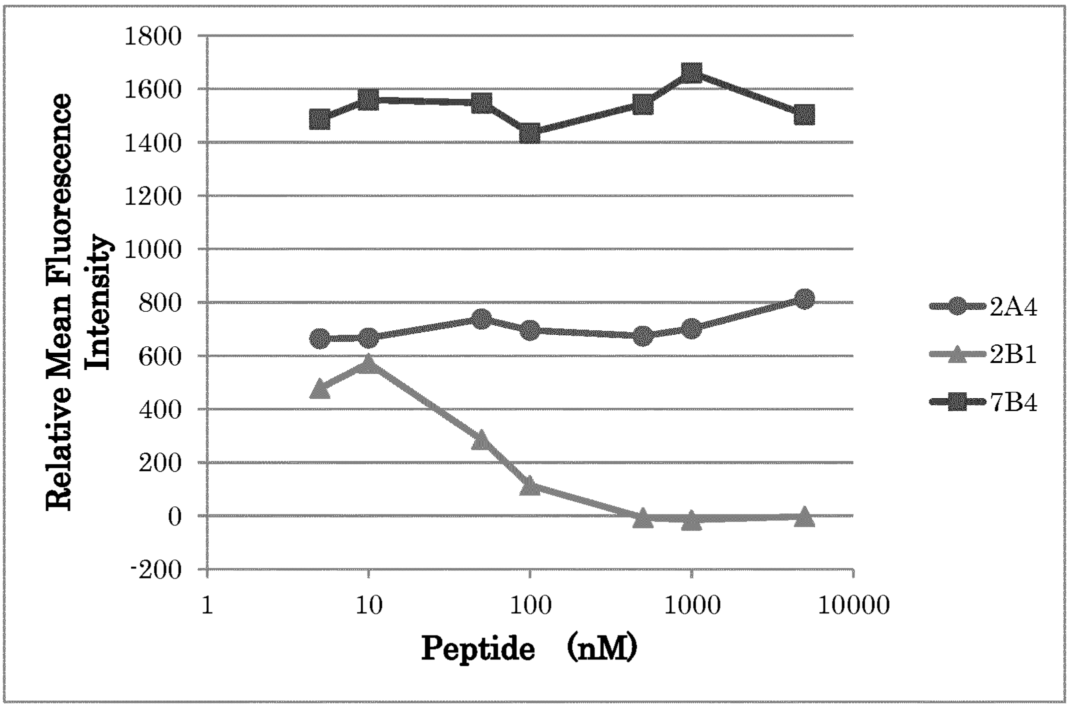
1800
1600
1400
1200
1000
800
600
400
200
0
-200
Relative Mean Fluorescence Intensity
1
10
100
1000
10000
Peptide (nM)
2A4
2B1
7B4

B

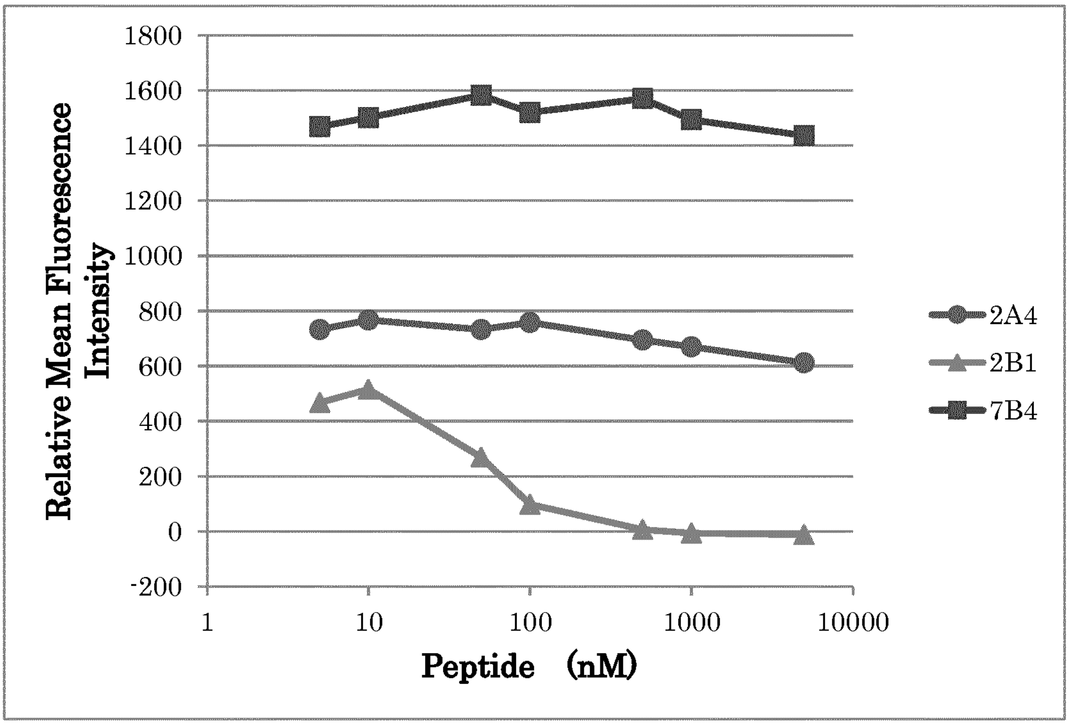
1800
1600
1400
1200
1000
800
600
400
200
0
-200
Relative Mean Fluorescence Intensity
1
10
100
1000
10000
Peptide (nM)
2A4
2B1
7B4

[Figure 5]

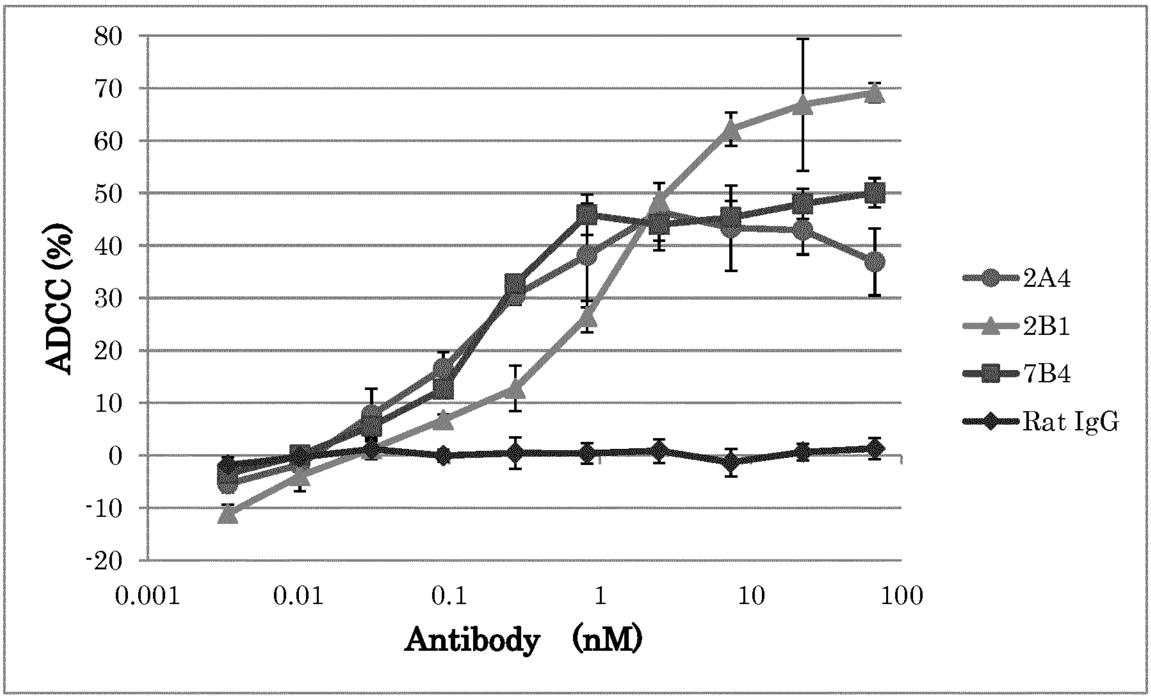
80
70
60
50
40
30
20
10
0
-10
-20
ADCC (%)
0.001
0.01
0.1
1
10
100
Antibody (nM)
2A4
2B1
7B4
Rat IgG

[Figure 6]
5'-CTCCAGAGTTCCAGGTCACGGTGACTGGC-3'

(SEQ ID NO: 3 of the Sequence Listing)

[Figure 7]
5'-TCAGTAACACTGTCCAGGACACCATCTC-3'

(SEQ ID NO: 10 of the Sequence Listing)

[Figure 8]
CAGGTCCAGTTGCAGCAATCTGGAGCTGAGCTGGCTAAACCTGGGACTTCAGTGAAGCT
GTCCTGCAAGGCTTCTGGCTATACCTTCACCAGCTACTATATTTACTGGGTAAAGCAGA
GGCCTGGACAGGGCCTTGAGTGGATCGGATATGTTTATCCTGGATATGGTGGTACTTAC
TACAGTGATAAGTTCAAGGGCAAAGCCACATTTACTGCAGACACATCCTCCAGCACAGC
CTACATGCTACTGGGCAGCCTGACACCTGAGGACTCTGCGTACTATTTCTGTGCAAGAC
GGAAGGGTATAATTCGGGGTCCGGGGTACTTTGATTACTGGGGCCAAGGAGTCATGGTC
ACAGTCTCCTCA

(SEQ ID NO: 4 of the Sequence Listing)

[Figure 9]
QVQLQQSGAELAKPGTSVKLSCKASGYTFTSYYIYWVKQRPGQGLEWIGYVYPGYGGTY
YSDKFKGKATFTADTSSSTAYMLLGSLTPEDSAYYFCARRKGIIRGPGYFDYWGQGVMV
TVSS

(SEQ ID NO: 5 of the Sequence Listing)

[Figure 10]

CAGGTTACTCTGAAAGAGTCTGGCCCTGGGATATTGCAGCCCTCCCAGACCCTCAGTCT
GACTTGCACTTTCTCTGGGTTTTCACTGAACACTTATGATATGGGTGTGGGCTGGATTC
GTCAGCCTTCAGGGAAGGGTCTGGAGTGGCTGGCAAACATTTGGTGGGATGATGATAAG
TACTACAATCCATCTCTGAGAAACCGGCTCACAATCTCCAAGGACACCTCCAACAACCA
AGCATTCCTCAAGATCACCAATGTGGACACTGCAGATACTGCCACATACTACTGTGCTC
GGATCGAAACTGTCCGGGTTTCCCGTAAGGGGTTTGCTCACTGGGGCCAAGGCACTCTG
GTCACTGTCTCTTCA

(SEQ ID NO: 6 of the Sequence Listing)

[Figure 11]

QVTLKESGPGILQPSQTLSLTCTFSGFSLNTYDMGVGWIRQPSGKGLEWLANIWWDDDK
YYNPSLRNRLTISKDTSNNQAFLKITNVDTADTATYYCARIETVRVSRKGFAHWGQGTL
VTVSS

(SEQ ID NO: 7 of the Sequence Listing)

[Figure 12]

GAGATACACCTGCAGGAGTCAGGACCTGGCCTTGTGAAACCTTCACAGTCACTCTCCCT
CACCTGTTCTGTCACTGGTTACACCATTACCAGTGGTTATGATTGGAGCTGGATCCGGA
AGTTCCCAGGAAATAAAATGGAGTGGATGGCATACATGAGCTATAGAGGTAGCACTAAC
TACAACCCCTCGCTCAAAAGTCGAATCTCCATTACAAGAGACACATCCAAGAATCAGTT
CTTCCTGCAGTTGAATTCTGTCACTACTGAGGATACAGCCACATATTACTGTGCCCTAA
CAAGGACCTATTGGTATAACTATTACTATGTTTTGGATGCCTGGGGTCAAGGAGCTTCA
GTCACTGTCTCCTCA

(SEQ ID NO: 8 of the Sequence Listing)

[Figure 13]

EIHLQESGPGLVKPSQSLSLTCSVTGYTITSGYDWSWIRKFPGNKMEWMAYMSYRGSTN
YNPSLKSRISITRDTSKNQFFLQLNSVTTEDTATYYCALTRTYWYNYYYVLDAWGQGAS
VTVSS

(SEQ ID NO: 9 of the Sequence Listing)

[Figure 14]

GACATCCAGATGACACAGTCTCCAGCTTCCCTGTCTGCATCTCTGGGAGAAACTGTCTC
CATCGAATGTCTTGCAAGTGAGGGCATTTCCAATAGTTTAGCGTGGTATCAGCAGAAGC
CAGGGAAATCTCCTCAGCTCCTGATCTATGGTGCAAGTAGCTTGCAAGACGGGGTCCCA
TCACGGTTCAGTGGCAGTGGTTCTGGCACGCAGTATTCTCTCAAGATCAGCGGCATGCA
ACCTGAAGATGAAGGGCTTTATTACTGTCAACAGGGTTACAAGTATCCTCCGACGTTCG
GTGGAGGCACCAAGCTGGAATTGAAA

(SEQ ID NO: 11 of the Sequence Listing)

[Figure 15]

DIQMTQSPASLSASLGETVSIECLASEGISNSLAWYQQKPGKSPQLLIYGASSLQDGVP
SRFSGSGSGTQYSLKISGMQPEDEGLYYCQQGYKYPPTFGGGTKLELK

(SEQ ID NO: 12 of the Sequence Listing)

[Figure 16]

GAAACTGTGATGACCCAGTCTCCCACATCCATGTCCACATCAATAGGAGAGAGGGTCAC
CCTGAACTGCAAGGCCAGTCAGAGTGTGGGTATTAATGTAGACTGGTACCAACAGACAC
CAGGGCAGTCTCCTAAACTGCTGATATATGGGGCATCCAACCGGCACACTGGGGTCCCT
GATCGCTTCACAGGCAGTGGATTTGGGAGAGATTTCACTCTCACCATCAGCAACGTGGA
GGCTGAAGACCTGGCTGTTTATTACTGTCTGCAGCATGGCTCCATTCCTCCGACGTTCG
GTGGAGGCACCAAGCTGGAATTGAAA

(SEQ ID NO: 13 of the Sequence Listing)

[Figure 17]

ETVMTQSPTSMSTSIGERVTLNCKASQSVGINVDWYQQTPGQSPKLLIYGASNRHTGVP
DRFTGSGFGRDFTLTISNVEAEDLAVYYCLQHGSIPPTFGGGTKLELK

(SEQ ID NO: 14 of the Sequence Listing)

[Figure 18]

GACATCCAGATGACCCAGTCTCCTTCATTCCTGTCTGCATCTGTGGGAGAAAGAGTCAC
TCTCAGCTGCAAAGCAAGTCAGAATATTAACAAGTACTTAGACTGGTATCAGCAAAAGC
TTGGAGAACCTCCCAAACTCCTGATATATAATACAAACAATTTGCATACGGGCATCCCA
TCAAGGTTCAGTGGCAGTGGATCTGGTACAGATTACACACTCACCATCAGCGGCCTGCA
GCCTGAAGATGTTGCCACATATTTCTGCTTGCAGCGTAATAGTTGGTACACGTTTGGAG
CTGGGACCAAGCTGGAACTGAAA

(SEQ ID NO: 15 of the Sequence Listing)

[Figure 19]

DIQMTQSPSFLSASVGERVTLSCKASQNINKYLDWYQQKLGEPPKLLIYNTNNLHTGIP
SRFSGSGSGTDYTLTISGLQPEDVATYFCLQRNSWYTFGAGTKLELK

(SEQ ID NO: 16 of the Sequence Listing)

[Figure 20]

GCCTCCGGACTCTAGAGCCACCATGGTGCTGCAGACCCAGGTGTTCATCTCCCTGCTGC
TGTGGATCTCCGGCGCGTACGGCGATATCGTGATGATTAAACGTACGGTGGCCGCCCCC
TCCGTGTTCATCTTCCCCCCCTCCGACGAGCAGCTGAAGTCCGGCACCGCCTCCGTGGT
GTGCCTGCTGAATAACTTCTACCCCAGAGAGGCCAAGGTGCAGTGGAAGGTGGACAACG
CCCTGCAGTCCGGGAACTCCCAGGAGAGCGTGACCGAGCAGGACAGCAAGGACAGCACC
TACAGCCTGAGCAGCACCCTGACCCTGAGCAAAGCCGACTACGAGAAGCACAAGGTGTA
CGCCTGCGAGGTGACCCACCAGGGCCTGAGCTCCCCCGTCACCAAGAGCTTCAACAGGG
GGGAGTGTTAGGGGCCCGTTTAAACGGGGGAGGCTA

(SEQ ID NO: 17 of the Sequence Listing)

[Figure 21]

5'-TATACCGTCGACCTCTAGCTAGAGCTTGGC-3'

(SEQ ID NO: 18 of the Sequence Listing)

[Figure 22]
5'-GCTATGGCAGGGCCTGCCGCCCCGACGTTG-3'

(SEQ ID NO: 19 of the Sequence Listing)

[Figure 23]
GCCTCCGGACTCTAGAGCCACCATGAAACACCTGTGGTTCTTCCTCCTGCTGGTGGCAG
CTCCCAGATGGGTGCTGAGCCAGGTGCAATTGTGCAGGCGGTTAGCTCAGCCTCCACCA
AGGGCCCAAGCGTCTTCCCCCTGGCACCCTCCTCCAAGAGCACCTCTGGCGGCACAGCC
GCCCTGGGCTGCCTGGTCAAGGACTACTTCCCCGAACCCGTGACCGTGAGCTGGAACTC
AGGCGCCCTGACCAGCGGCGTGCACACCTTCCCCGCTGTCCTGCAGTCCTCAGGACTCT
ACTCCCTCAGCAGCGTGGTGACCGTGCCCTCCAGCAGCTTGGGCACCCAGACCTACATC
TGCAACGTGAATCACAAGCCCAGCAACACCAAGGTGGACAAGAGAGTTGAGCCCAAATC
TTGTGACAAAACTCACACATGCCCACCCTGCCCAGCACCTGAACTCCTGGGGGGACCCT
CAGTCTTCCTCTTCCCCCCAAAACCCAAGGACACCCTCATGATCTCCCGGACCCCTGAG
GTCACATGCGTGGTGGTGGACGTGAGCCACGAAGACCCTGAGGTCAAGTTCAACTGGTA
CGTGGACGGCGTGGAGGTGCATAATGCCAAGACAAAGCCCCGGGAGGAGCAGTACAACA
GCACGTACCGGGTGGTCAGCGTCCTCACCGTCCTGCACCAGGACTGGCTGAATGGCAAG
GAGTACAAGTGCAAGGTCTCCAACAAAGCCCTCCCAGCCCCCATCGAGAAAACCATCTC
CAAAGCCAAAGGCCAGCCCCGGGAACCACAGGTGTACACCCTGCCCCCATCCCGGGAGG
AGATGACCAAGAACCAGGTCAGCCTGACCTGCCTGGTCAAAGGCTTCTATCCCAGCGAC
ATCGCCGTGGAGTGGGAGAGCAATGGCCAGCCCGAGAACAACTACAAGACCACCCCTCC
CGTGCTGGACTCCGACGGCTCCTTCTTCCTCTACAGCAAGCTCACCGTGGACAAGAGCA
GGTGGCAGCAGGGCAACGTCTTCTCATGCTCCGTGATGCATGAGGCTCTGCACAACCAC
TACACCCAGAAGAGCCTCTCCCTGTCTCCCGGCAAATGAGATATCGGGCCCGTTTAAAC
GGGGGAGGCTA

(SEQ ID NO: 20 of the Sequence Listing)

[Figure 24]

ATGGTGCTGCAGACCCAGGTGTTCATCTCCCTGCTGCTGTGGATCTCCGGCGCGTACGG
CGACATCCAGATGACACAGTCTCCAGCTTCCCTGTCTGCATCTCTGGGAGAAACTGTCT
CCATCGAATGTCTTGCAAGTGAGGGCATTTCCAATAGTTTAGCGTGGTATCAGCAGAAG
CCAGGGAAATCTCCTCAGCTCCTGATCTATGGTGCAAGTAGCTTGCAAGACGGGGTCCC
ATCACGGTTCAGTGGCAGTGGTTCTGGCACGCAGTATTCTCTCAAGATCAGCGGCATGC
AACCTGAAGATGAAGGGCTTTATTACTGTCAACAGGGTTACAAGTATCCTCCGACGTTC
GGTGGAGGCACCAAGCTGGAATTGAAACGGGCTGTGGCCGCCCCCTCCGTGTTCATCTT
CCCCCCCTCCGACGAGCAGCTGAAGTCCGGCACCGCCTCCGTGGTGTGCCTGCTGAATA
ACTTCTACCCCAGAGAGGCCAAGGTGCAGTGGAAGGTGGACAACGCCCTGCAGTCCGGG
AACTCCCAGGAGAGCGTGACCGAGCAGGACAGCAAGGACAGCACCTACAGCCTGAGCAG
CACCCTGACCCTGAGCAAAGCCGACTACGAGAAGCACAAGGTGTACGCCTGCGAGGTGA
CCCACCAGGGCCTGAGCTCCCCCGTCACCAAGAGCTTCAACAGGGGGGAGTGT

(SEQ ID NO: 21 of the Sequence Listing)

[Figure 25]

MVLQTQVFISLLLWISGAYGDIQMTQSPASLSASLGETVSIECLASEGISNSLAWYQQK
PGKSPQLLIYGASSLQDGVPSRFSGSGSGTQYSLKISGMQPEDEGLYYCQQGYKYPPTF
GGGTKLELKRAVAAPSVFIFPPSDEQLKSGTASVVCLLNNFYPREAKVQWKVDNALQSG
NSQESVTEQDSKDSTYSLSSTLTLSKADYEKHKVYACEVTHQGLSSPVTKSFNRGEC

(SEQ ID NO: 22 of the Sequence Listing)

[Figure 26]

5'-ATCTCCGGCGCGTACGGCGACATCCAGATGACACAGTCTCCAGC-3'

(SEQ ID NO: 23 of the Sequence Listing)

[Figure 27]

5'-GGAGGGGGCGGCCACAGCCCGTTTCAATTCCAGCTTGGTGCCTC-3'

(SEQ ID NO: 24 of the Sequence Listing)

[Figure 28]

ATGAAACACCTGTGGTTCTTCCTCCTGCTGGTGGCAGCTCCCAGATGGGTGCTGAGCCA
GGTCCAGTTGCAGCAATCTGGAGCTGAGCTGGCTAAACCTGGGACTTCAGTGAAGCTGT
CCTGCAAGGCTTCTGGCTATACCTTCACCAGCTACTATATTTACTGGGTAAAGCAGAGG
CCTGGACAGGGCCTTGAGTGGATCGGATATGTTTATCCTGGATATGGTGGTACTTACTA
CAGTGATAAGTTCAAGGGCAAAGCCACATTTACTGCAGACACATCCTCCAGCACAGCCT
ACATGCTACTGGGCAGCCTGACACCTGAGGACTCTGCGTACTATTTCTGTGCAAGACGG
AAGGGTATAATTCGGGGTCCGGGGTACTTTGATTACTGGGGCCAAGGAGTCATGGTCAC
AGTCAGCTCAGCCTCCACCAAGGGCCCAAGCGTCTTCCCCCTGGCACCCTCCTCCAAGA
GCACCTCTGGCGGCACAGCCGCCCTGGGCTGCCTGGTCAAGGACTACTTCCCCGAACCC
GTGACCGTGAGCTGGAACTCAGGCGCCCTGACCAGCGGCGTGCACACCTTCCCCGCTGT
CCTGCAGTCCTCAGGACTCTACTCCCTCAGCAGCGTGGTGACCGTGCCCTCCAGCAGCT
TGGGCACCCAGACCTACATCTGCAACGTGAATCACAAGCCCAGCAACACCAAGGTGGAC
AAGAGAGTTGAGCCCAAATCTTGTGACAAAACTCACACATGCCCACCCTGCCCAGCACC
TGAACTCCTGGGGGGACCCTCAGTCTTCCTCTTCCCCCCAAAACCCAAGGACACCCTCA
TGATCTCCCGGACCCCTGAGGTCACATGCGTGGTGGTGGACGTGAGCCACGAAGACCCT
GAGGTCAAGTTCAACTGGTACGTGGACGGCGTGGAGGTGCATAATGCCAAGACAAAGCC
CCGGGAGGAGCAGTACAACAGCACGTACCGGGTGGTCAGCGTCCTCACCGTCCTGCACC
AGGACTGGCTGAATGGCAAGGAGTACAAGTGCAAGGTCTCCAACAAAGCCCTCCCAGCC
CCCATCGAGAAAACCATCTCCAAAGCCAAAGGCCAGCCCCGGGAACCACAGGTGTACAC
CCTGCCCCCATCCCGGGAGGAGATGACCAAGAACCAGGTCAGCCTGACCTGCCTGGTCA
AAGGCTTCTATCCCAGCGACATCGCCGTGGAGTGGGAGAGCAATGGCCAGCCCGAGAAC
AACTACAAGACCACCCCTCCCGTGCTGGACTCCGACGGCTCCTTCTTCCTCTACAGCAA
GCTCACCGTGGACAAGAGCAGGTGGCAGCAGGGCAACGTCTTCTCATGCTCCGTGATGC
ATGAGGCTCTGCACAACCACTACACCCAGAAGAGCCTCTCCCTGTCTCCCGGCAAA

(SEQ ID NO: 25 of the Sequence Listing)

[Figure 29]

MKHLWFFLLLVAAPRWVLSQVQLQQSGAELAKPGTSVKLSCKASGYTFTSYYIYWVKQR
PGQGLEWIGYVYPGYGGTYYSDKFKGKATFTADTSSSTAYMLLGSLTPEDSAYYFCARR
KGIIRGPGYFDYWGQGVMVTVSSASTKGPSVFPLAPSSKSTSGGTAALGCLVKDYFPEP
VTVSWNSGALTSGVHTFPAVLQSSGLYSLSSVVTVPSSSLGTQTYICNVNHKPSNTKVD
KRVEPKSCDKTHTCPPCPAPELLGGPSVFLFPPKPKDTLMISRTPEVTCVVVDVSHEDP
EVKFNWYVDGVEVHNAKTKPREEQYNSTYRVVSVLTVLHQDWLNGKEYKCKVSNKALPA
PIEKTISKAKGQPREPQVYTLPPSREEMTKNQVSLTCLVKGFYPSDIAVEWESNGQPEN
NYKTTPPVLDSDGSFFLYSKLTVDKSRWQQGNVFSCSVMHEALHNHYTQKSLSLSPGK

(SEQ ID NO: 26 of the Sequence Listing)

[Figure 30]

5'-CCAGATGGGTGCTGAGCCAGGTCCAGTTGCAGCAATCTGGAGCTG-3'

(SEQ ID NO: 27 of the Sequence Listing)

[Figure 31]

5'-CTTGGTGGAGGCTGAGCTGACTGTGACCATGACTCCTTGGCCCCAG-3'

(SEQ ID NO: 28 of the Sequence Listing)

[Figure 32]

ATGGTGCTGCAGACCCAGGTGTTCATCTCCCTGCTGCTGTGGATCTCCGGCGCGTACGG
CGAAACTGTGATGACCCAGTCTCCCACATCCATGTCCACATCAATAGGAGAGAGGGTCA
CCCTGAACTGCAAGGCCAGTCAGAGTGTGGGTATTAATGTAGACTGGTACCAACAGACA
CCAGGGCAGTCTCCTAAACTGCTGATATATGGGGCATCCAACCGGCACACTGGGGTCCC
TGATCGCTTCACAGGCAGTGGATTTGGGAGAGATTTCACTCTCACCATCAGCAACGTGG
AGGCTGAAGACCTGGCTGTTTATTACTGTCTGCAGCATGGCTCCATTCCTCCGACGTTC
GGTGGAGGCACCAAGCTGGAATTGAAACGGGCTGTGGCCGCCCCCTCCGTGTTCATCTT
CCCCCCCTCCGACGAGCAGCTGAAGTCCGGCACCGCCTCCGTGGTGTGCCTGCTGAATA
ACTTCTACCCCAGAGAGGCCAAGGTGCAGTGGAAGGTGGACAACGCCCTGCAGTCCGGG
AACTCCCAGGAGAGCGTGACCGAGCAGGACAGCAAGGACAGCACCTACAGCCTGAGCAG
CACCCTGACCCTGAGCAAAGCCGACTACGAGAAGCACAAGGTGTACGCCTGCGAGGTGA
CCCACCAGGGCCTGAGCTCCCCCGTCACCAAGAGCTTCAACAGGGGGGAGTGT

(SEQ ID NO: 29 of the Sequence Listing)

[Figure 33]

MVLQTQVFISLLLWISGAYGETVMTQSPTSMSTSIGERVTLNCKASQSVGINVDWYQQT
PGQSPKLLIYGASNRHTGVPDRFTGSGFGRDFTLTISNVEAEDLAVYYCLQHGSIPPTF
GGGTKLELKRAVAAPSVFIFPPSDEQLKSGTASVVCLLNNFYPREAKVQWKVDNALQSG
NSQESVTEQDSKDSTYSLSSTLTLSKADYEKHKVYACEVTHQGLSSPVTKSFNRGEC

(SEQ ID NO: 30 of the Sequence Listing)

[Figure 34]

5'-ATCTCCGGCGCGTACGGCGAAACTGTGATGACCCAGTCTCCCAC-3'

(SEQ ID NO: 31 of the Sequence Listing)

[Figure 35]

5'-GGAGGGGGCGGCCACAGCCCGTTTCAATTCCAGCTTGGTGCCTC-3'

(SEQ ID NO: 32 of the Sequence Listing)

[Figure 36]

ATGAAACACCTGTGGTTCTTCCTCCTGCTGGTGGCAGCTCCCAGATGGGTGCTGAGCCA
GGTTACTCTGAAAGAGTCTGGCCCTGGGATATTGCAGCCCTCCCAGACCCTCAGTCTGA
CTTGCACTTTCTCTGGGTTTTCACTGAACACTTATGATATGGGTGTGGGCTGGATTCGT
CAGCCTTCAGGGAAGGGTCTGGAGTGGCTGGCAAACATTTGGTGGGATGATGATAAGTA
CTACAATCCATCTCTGAGAAACCGGCTCACAATCTCCAAGGACACCTCCAACAACCAAG
CATTCCTCAAGATCACCAATGTGGACACTGCAGATACTGCCACATACTACTGTGCTCGG
ATCGAAACTGTCCGGGTTTCCCGTAAGGGGTTTGCTCACTGGGGCCAAGGCACTCTGGT
CACTGTCAGCTCAGCCTCCACCAAGGGCCCAAGCGTCTTCCCCCTGGCACCCTCCTCCA
AGAGCACCTCTGGCGGCACAGCCGCCCTGGGCTGCCTGGTCAAGGACTACTTCCCCGAA
CCCGTGACCGTGAGCTGGAACTCAGGCGCCCTGACCAGCGGCGTGCACACCTTCCCCGC
TGTCCTGCAGTCCTCAGGACTCTACTCCCTCAGCAGCGTGGTGACCGTGCCCTCCAGCA
GCTTGGGCACCCAGACCTACATCTGCAACGTGAATCACAAGCCCAGCAACACCAAGGTG
GACAAGAGAGTTGAGCCCAAATCTTGTGACAAAACTCACACATGCCCACCCTGCCCAGC
ACCTGAACTCCTGGGGGGACCCTCAGTCTTCCTCTTCCCCCCAAAACCCAAGGACACCC
TCATGATCTCCCGGACCCCTGAGGTCACATGCGTGGTGGTGGACGTGAGCCACGAAGAC
CCTGAGGTCAAGTTCAACTGGTACGTGGACGGCGTGGAGGTGCATAATGCCAAGACAAA
GCCCCGGGAGGAGCAGTACAACAGCACGTACCGGGTGGTCAGCGTCCTCACCGTCCTGC
ACCAGGACTGGCTGAATGGCAAGGAGTACAAGTGCAAGGTCTCCAACAAAGCCCTCCCA
GCCCCCATCGAGAAAACCATCTCCAAAGCCAAAGGCCAGCCCCGGGAACCACAGGTGTA
CACCCTGCCCCCATCCCGGGAGGAGATGACCAAGAACCAGGTCAGCCTGACCTGCCTGG
TCAAAGGCTTCTATCCCAGCGACATCGCCGTGGAGTGGGAGAGCAATGGCCAGCCCGAG
AACAACTACAAGACCACCCCTCCCGTGCTGGACTCCGACGGCTCCTTCTTCCTCTACAG
CAAGCTCACCGTGGACAAGAGCAGGTGGCAGCAGGGCAACGTCTTCTCATGCTCCGTGA
TGCATGAGGCTCTGCACAACCACTACACCCAGAAGAGCCTCTCCCTGTCTCCCGGCAAA
(SEQ ID NO: 33 of the Sequence Listing)

[Figure 37]

MKHLWFFLLLVAAPRWVLSQVTLKESGPGILQPSQTLSLTCTFSGFSLNTYDMGVGWIR
QPSGKGLEWLANIWWDDDKYYNPSLRNRLTISKDTSNNQAFLKITNVDTADTATYYCAR
IETVRVSRKGFAHWGQGTLVTVSSASTKGPSVFPLAPSSKSTSGGTAALGCLVKDYFPE
PVTVSWNSGALTSGVHTFPAVLQSSGLYSLSSVVTVPSSSLGTQTYICNVNHKPSNTKV
DKRVEPKSCDKTHTCPPCPAPELLGGPSVFLFPPKPKDTLMISRTPEVTCVVVDVSHED
PEVKFNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSPGK

(SEQ ID NO: 34 of the Sequence Listing)

[Figure 38]

5'-CCAGATGGGTGCTGAGCCAGGTTACTCTGAAAGAGTCTGGCCCTG-3'

(SEQ ID NO: 35 of the Sequence Listing)

[Figure 39]

5'-CTTGGTGGAGGCTGAGCTGACAGTGACCAGAGTGCCTTGGCCCCAG-3'

(SEQ ID NO: 36 of the Sequence Listing)

[Figure 40]

ATGGTGCTGCAGACCCAGGTGTTCATCTCCCTGCTGCTGTGGATCTCCGGCGCGTACGG
CGACATCCAGATGACCCAGTCTCCTTCATTCCTGTCTGCATCTGTGGGAGAAAGAGTCA
CTCTCAGCTGCAAAGCAAGTCAGAATATTAACAAGTACTTAGACTGGTATCAGCAAAAG
CTTGGAGAACCTCCCAAACTCCTGATATATAATACAAACAATTTGCATACGGGCATCCC
ATCAAGGTTCAGTGGCAGTGGATCTGGTACAGATTACACACTCACCATCAGCGGCCTGC
AGCCTGAAGATGTTGCCACATATTTCTGCTTGCAGCGTAATAGTTGGTACACGTTTGGA
GCTGGGACCAAGCTGGAACTGAAACGGGCTGTGGCCGCCCCCTCCGTGTTCATCTTCCC
CCCCTCCGACGAGCAGCTGAAGTCCGGCACCGCCTCCGTGGTGTGCCTGCTGAATAACT
TCTACCCCAGAGAGGCCAAGGTGCAGTGGAAGGTGGACAACGCCCTGCAGTCCGGGAAC
TCCCAGGAGAGCGTGACCGAGCAGGACAGCAAGGACAGCACCTACAGCCTGAGCAGCAC
CCTGACCCTGAGCAAAGCCGACTACGAGAAGCACAAGGTGTACGCCTGCGAGGTGACCC
ACCAGGGCCTGAGCTCCCCCGTCACCAAGAGCTTCAACAGGGGGGAGTGT

(SEQ ID NO: 37 of the Sequence Listing)

[Figure 41]

MVLQTQVFISLLLWISGAYGDIQMTQSPSFLSASVGERVTLSCKASQNINKYLDWYQQK
LGEPPKLLIYNTNNLHTGIPSRFSGSGSGTDYTLTISGLQPEDVATYFCLQRNSWYTFG
AGTKLELKRAVAAPSVFIFPPSDEQLKSGTASVVCLLNNFYPREAKVQWKVDNALQSGN
SQESVTEQDSKDSTYSLSSTLTLSKADYEKHKVYACEVTHQGLSSPVTKSFNRGEC

(SEQ ID NO: 38 of the Sequence Listing)

[Figure 42]

5'-ATCTCCGGCGCGTACGGCGACATCCAGATGACCCAGTCTCCTTC-3'

(SEQ ID NO: 39 of the Sequence Listing)

[Figure 43]

5'-GGAGGGGGCGGCCACAGCCCGTTTCAGTTCCAGCTTGGTCCCAG-3'

(SEQ ID NO: 40 of the Sequence Listing)

[Figure 44]

ATGAAACACCTGTGGTTCTTCCTCCTGCTGGTGGCAGCTCCCAGATGGGTGCTGAGCGA
GATACACCTGCAGGAGTCAGGACCTGGCCTTGTGAAACCTTCACAGTCACTCTCCCTCA
CCTGTTCTGTCACTGGTTACACCATTACCAGTGGTTATGATTGGAGCTGGATCCGGAAG
TTCCCAGGAAATAAAATGGAGTGGATGGCATACATGAGCTATAGAGGTAGCACTAACTA
CAACCCCTCGCTCAAAAGTCGAATCTCCATTACAAGAGACACATCCAAGAATCAGTTCT
TCCTGCAGTTGAATTCTGTCACTACTGAGGATACAGCCACATATTACTGTGCCCTAACA
AGGACCTATTGGTATAACTATTACTATGTTTTGGATGCCTGGGGTCAAGGAGCTTCAGT
CACTGTCAGCTCAGCCTCCACCAAGGGCCCAAGCGTCTTCCCCCTGGCACCCTCCTCCA
AGAGCACCTCTGGCGGCACAGCCGCCCTGGGCTGCCTGGTCAAGGACTACTTCCCCGAA
CCCGTGACCGTGAGCTGGAACTCAGGCGCCCTGACCAGCGGCGTGCACACCTTCCCCGC
TGTCCTGCAGTCCTCAGGACTCTACTCCCTCAGCAGCGTGGTGACCGTGCCCTCCAGCA
GCTTGGGCACCCAGACCTACATCTGCAACGTGAATCACAAGCCCAGCAACACCAAGGTG
GACAAGAGAGTTGAGCCCAAATCTTGTGACAAAACTCACACATGCCCACCCTGCCCAGC
ACCTGAACTCCTGGGGGGACCCTCAGTCTTCCTCTTCCCCCCAAAACCCAAGGACACCC
TCATGATCTCCCGGACCCCTGAGGTCACATGCGTGGTGGTGGACGTGAGCCACGAAGAC
CCTGAGGTCAAGTTCAACTGGTACGTGGACGGCGTGGAGGTGCATAATGCCAAGACAAA
GCCCCGGGAGGAGCAGTACAACAGCACGTACCGGGTGGTCAGCGTCCTCACCGTCCTGC
ACCAGGACTGGCTGAATGGCAAGGAGTACAAGTGCAAGGTCTCCAACAAAGCCCTCCCA
GCCCCCATCGAGAAAACCATCTCCAAAGCCAAAGGCCAGCCCCGGGAACCACAGGTGTA
CACCCTGCCCCCATCCCGGGAGGAGATGACCAAGAACCAGGTCAGCCTGACCTGCCTGG
TCAAAGGCTTCTATCCCAGCGACATCGCCGTGGAGTGGGAGAGCAATGGCCAGCCCGAG
AACAACTACAAGACCACCCCTCCCGTGCTGGACTCCGACGGCTCCTTCTTCCTCTACAG
CAAGCTCACCGTGGACAAGAGCAGGTGGCAGCAGGGCAACGTCTTCTCATGCTCCGTGA
TGCATGAGGCTCTGCACAACCACTACACCCAGAAGAGCCTCTCCCTGTCTCCCGGCAAA

(SEQ ID NO: 41 of the Sequence Listing)

[Figure 45]

MKHLWFFLLLVAAPRWVLSEIHLQESGPGLVKPSQSLSLTCSVTGYTITSGYDWSWIRK
FPGNKMEWMAYMSYRGSTNYNPSLKSRISITRDTSKNQFFLQLNSVTTEDTATYYCALT
RTYWYNYYYVLDAWGQGASVTVSSASTKGPSVFPLAPSSKSTSGGTAALGCLVKDYFPE
PVTVSWNSGALTSGVHTFPAVLQSSGLYSLSSVVTVPSSSLGTQTYICNVNHKPSNTKV
DKRVEPKSCDKTHTCPPCPAPELLGGPSVFLFPPKPKDTLMISRTPEVTCVVVDVSHED
PEVKFNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSPGK

(SEQ ID NO: 42 of the Sequence Listing)

[Figure 46]

5'-CCAGATGGGTGCTGAGCGAGATACACCTGCAGGAGTCAGGACCTG-3'

(SEQ ID NO: 43 of the Sequence Listing)

[Figure 47]

5'-CTTGGTGGAGGCTGAGCTGACAGTGACTGAAGCTCCTTGACCCCAG-3'

(SEQ ID NO: 44 of the Sequence Listing)

[Figure 48]

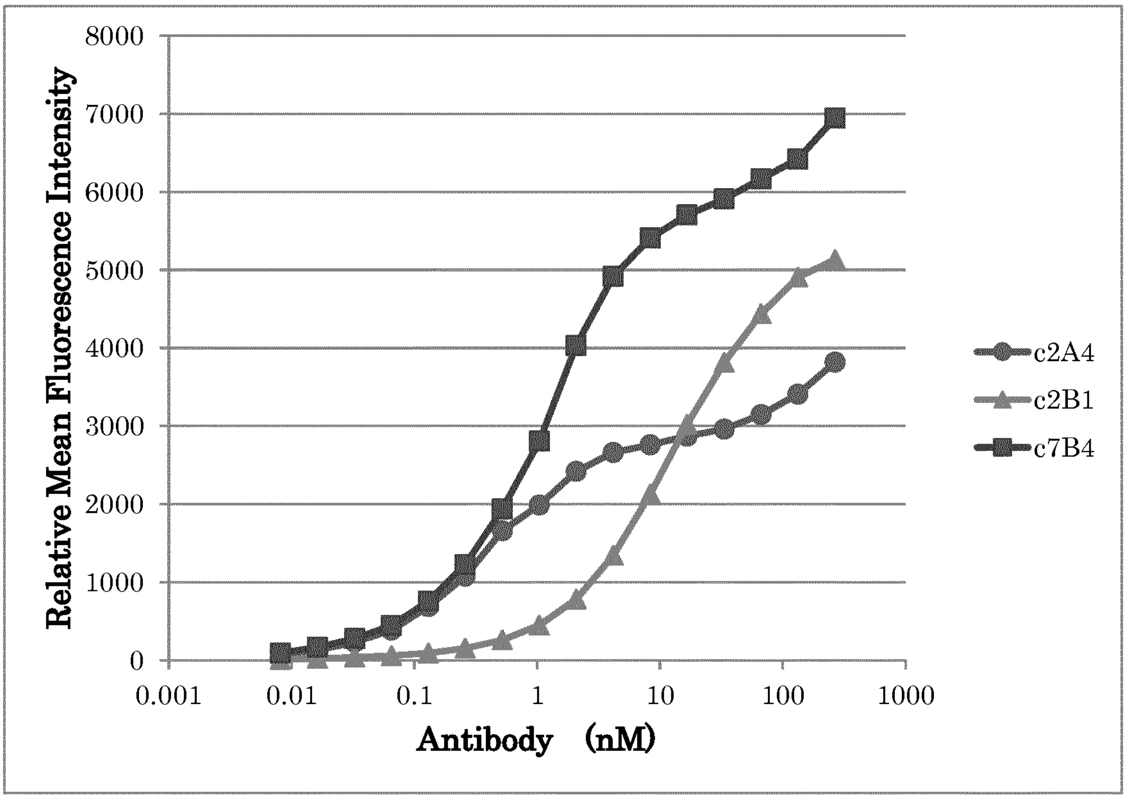

Relative Mean Fluorescence Intensity
8000
7000
6000
5000
4000
3000
2000
1000
0
0.001
0.01
0.1
1
10
100
1000
Antibody (nM)
c2A4
c2B1
c7B4

[Figure 49]

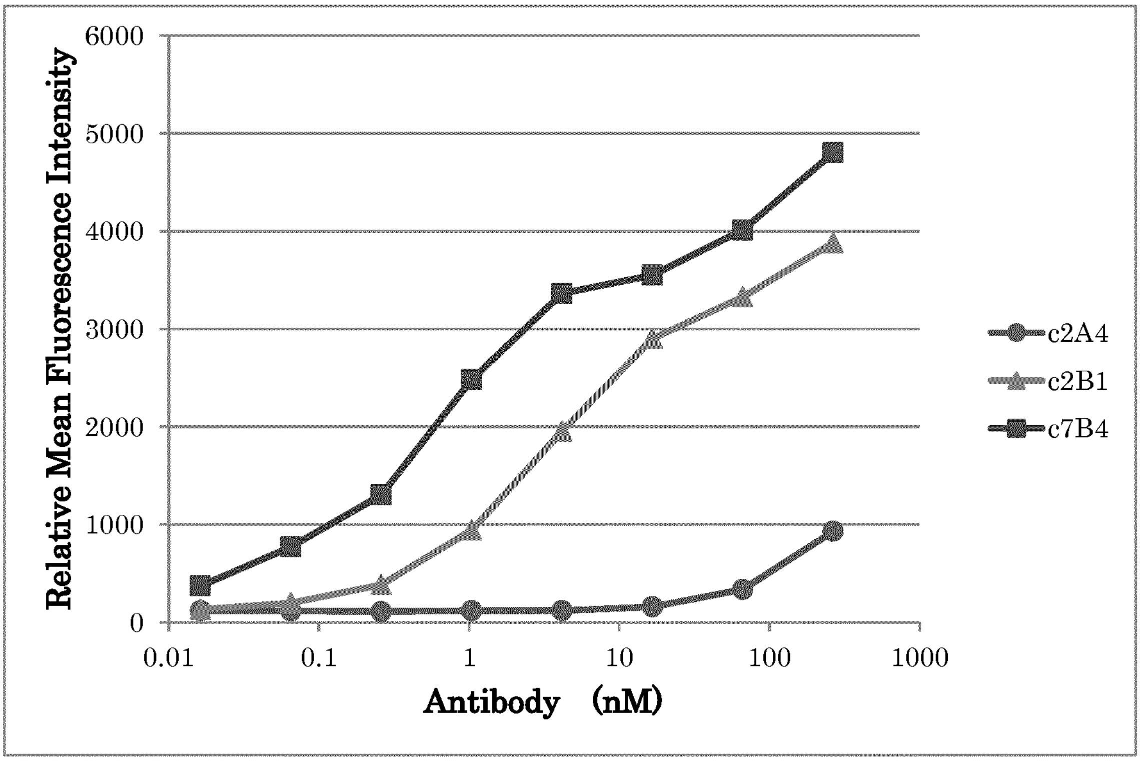


[Figure 50]

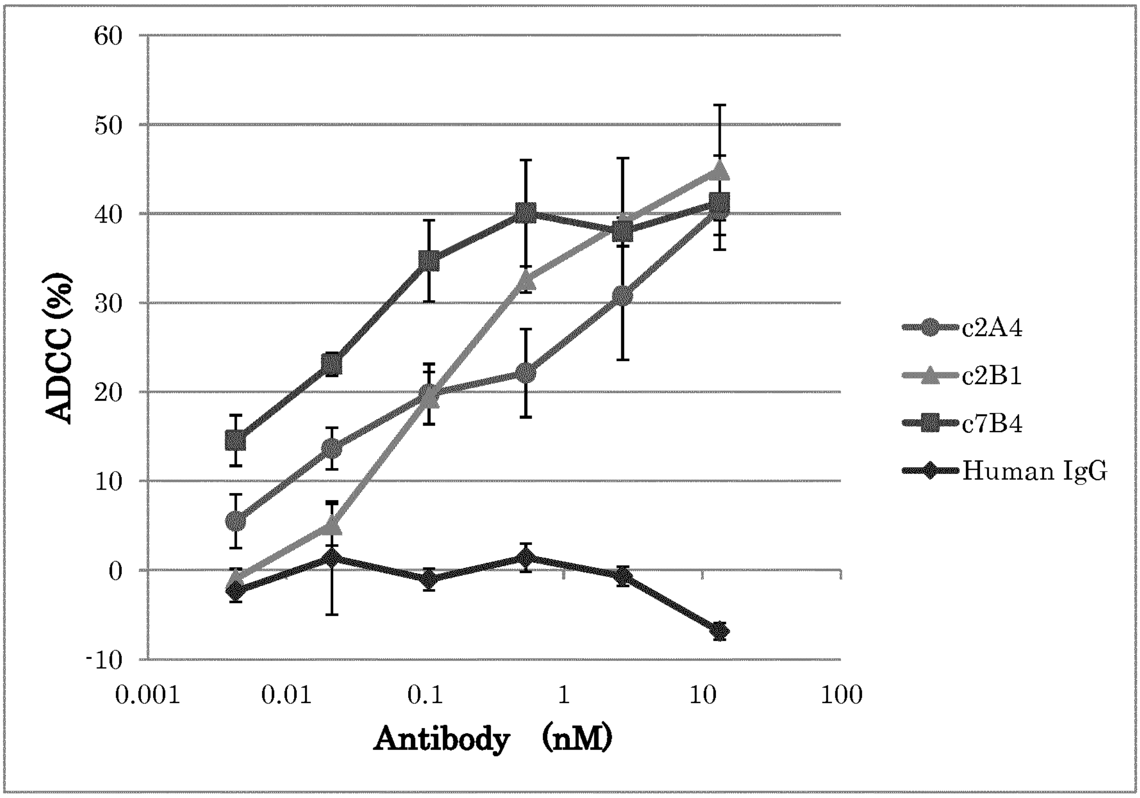

[Figure 51]

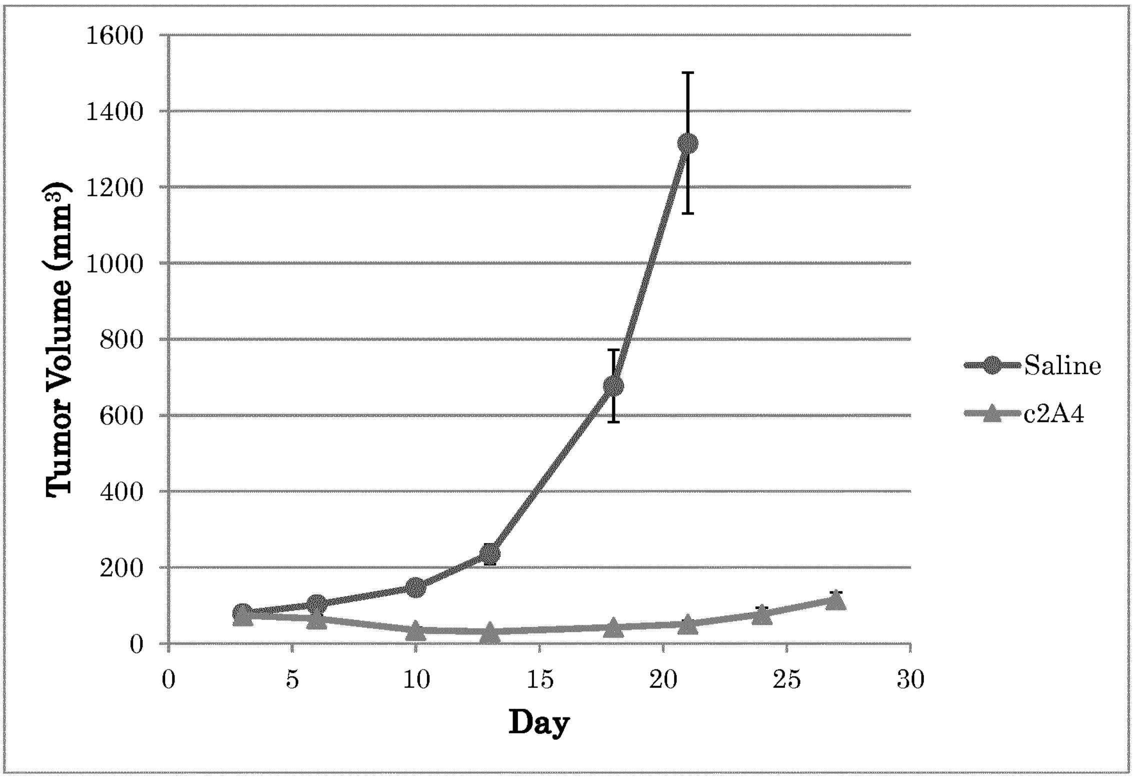


[Figure 52]

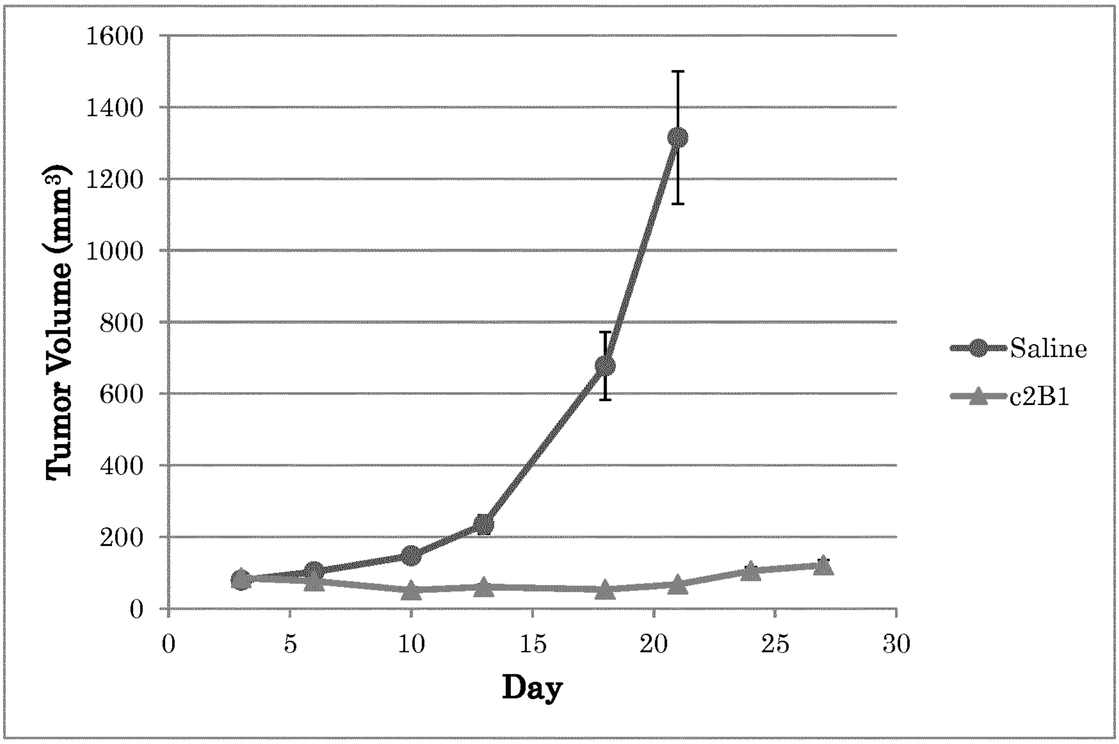

[Figure 53]

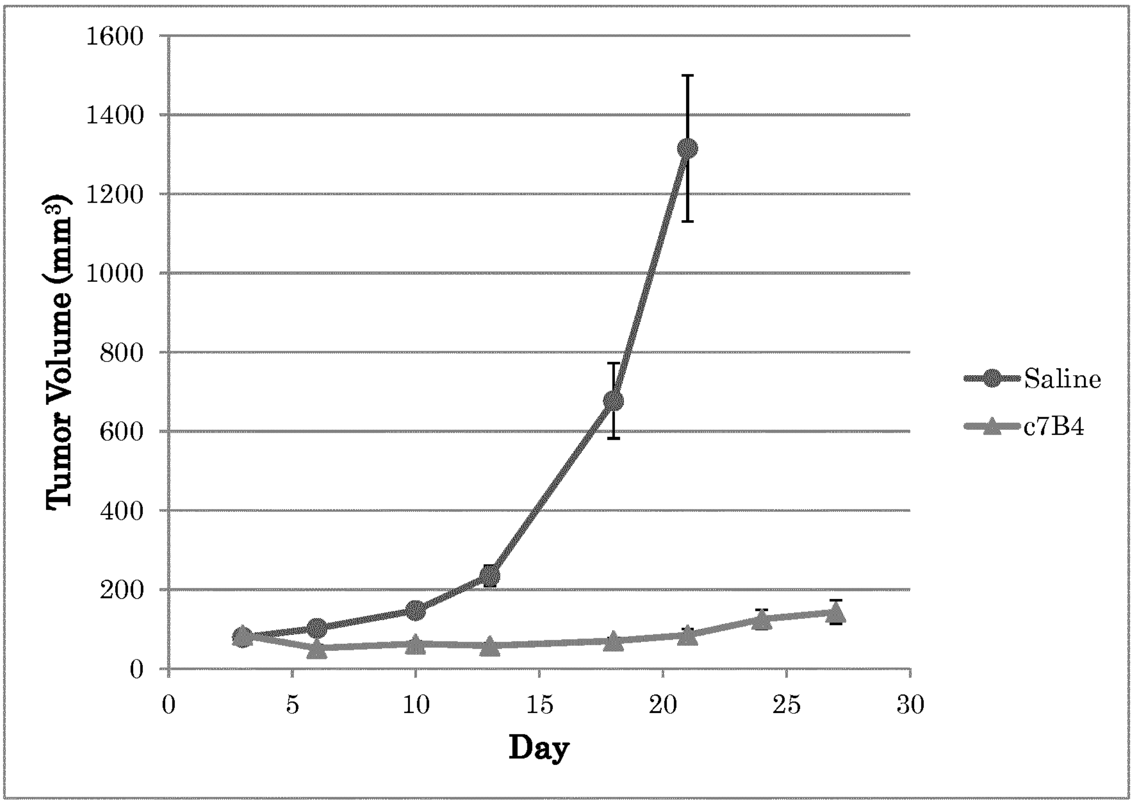


[Figure 54]
GYTFTSYY

(SEQ ID NO: 45 of the Sequence Listing)

[Figure 55]
VYPGYGGT

(SEQ ID NO: 46 of the Sequence Listing)

[Figure 56]
ARRKGIIRGPGYFDY

(SEQ ID NO: 47 of the Sequence Listing)

[Figure 57]
GFSLNTYDMG

(SEQ ID NO: 48 of the Sequence Listing)

[Figure 58]
IWWDDDK

(SEQ ID NO: 49 of the Sequence Listing)

[Figure 59]
ARIETVRVSRKGFAH

(SEQ ID NO: 50 of the Sequence Listing)

[Figure 60]
GYTITSGYD

(SEQ ID NO: 51 of the Sequence Listing)

[Figure 61]
MSYRGST

(SEQ ID NO: 52 of the Sequence Listing)

[Figure 62]
ALTRTYWYNYYYVLDA

(SEQ ID NO: 53 of the Sequence Listing)

[Figure 63]
EGISNS

(SEQ ID NO: 54 of the Sequence Listing)

[Figure 64]
GAS

(SEQ ID NO: 55 of the Sequence Listing)

[Figure 65]
QQGYKYPPT

(SEQ ID NO: 56 of the Sequence Listing)

[Figure 66]
QSVGIN

(SEQ ID NO: 57 of the Sequence Listing)

[Figure 67]
GAS

(SEQ ID NO: 58 of the Sequence Listing)

[Figure 68]
LQHGSIPPT

(SEQ ID NO: 59 of the Sequence Listing)

[Figure 69]
QNINKY

(SEQ ID NO: 60 of the Sequence Listing)

[Figure 70]
NTN

(SEQ ID NO: 61 of the Sequence Listing)

[Figure 71]
LQRNSWYT

(SEQ ID NO: 62 of the Sequence Listing)

[Figure 72]
ATGGTGCTGCAGACCCAGGTGTTCATCTCCCTGCTGCTGTGGATCTCCGGCGCGTACGG
CGATACCGTGATGACCCAGAGCCCTGACAGCCTGGCCGTGTCTCTGGGAGAGAGAGCCA
CCATCAACTGCAAGGCCAGCCAGAGCGTGGGCATCAACGTGGACTGGTATCAGCAGAAG
CCCGGCCAGCCCCCCAAGCTGCTGATCTACGGCGCCAGCAATAGACACACCGGCGTGCC
CGATAGATTCAGCGGCTCTGGCAGCGGCAGAGACTTCACCCTGACCATCAGCTCCCTGC
AGGCCGAGGATGTGGCCGTGTACTACTGTCTGCAGCACGGCAGCATCCCCCCCACATTT
GGCCAGGGCACCAAGGTGGAAATCAAGCGTACGGTGGCCGCCCCCTCCGTGTTCATCTT
CCCCCCCTCCGACGAGCAGCTGAAGTCCGGCACCGCCTCCGTGGTGTGCCTGCTGAATA
ACTTCTACCCCAGAGAGGCCAAGGTGCAGTGGAAGGTGGACAACGCCCTGCAGTCCGGG
AACTCCCAGGAGAGCGTGACCGAGCAGGACAGCAAGGACAGCACCTACAGCCTGAGCAG
CACCCTGACCCTGAGCAAAGCCGACTACGAGAAGCACAAGGTGTACGCCTGCGAGGTGA
CCCACCAGGGCCTGAGCTCCCCCGTCACCAAGAGCTTCAACAGGGGGGAGTGT

(SEQ ID NO: 63 of the Sequence Listing)

[Figure 73]

MVLQTQVFISLLLWISGAYGDTVMTQSPDSLAVSLGERATINCKASQSVGINVDWYQQK
PGQPPKLLIYGASNRHTGVPDRFSGSGSGRDFTLTISSLQAEDVAVYYCLQHGSIPPTF
GQGTKVEIKRTVAAPSVFIFPPSDEQLKSGTASVVCLLNNFYPREAKVQWKVDNALQSG
NSQESVTEQDSKDSTYSLSSTLTLSKADYEKHKVYACEVTHQGLSSPVTKSFNRGEC

(SEQ ID NO: 64 of the Sequence Listing)

[Figure 74]

ATGGTGCTGCAGACCCAGGTGTTCATCTCCCTGCTGCTGTGGATCTCCGGCGCGTACGG
CGATACCGTGATGACCCAGAGCCCTGACAGCCTGGCCGTGTCTCTGGGAGAGAGAGCCA
CCATCAACTGCAAGGCCAGCCAGAGCGTGGGCATCAACGTGGACTGGTATCAGCAGAAG
CCCGGCCAGCCCCCCAAGCTGCTGATCTACGGCGCCAGCAATAGACACACCGGCGTGCC
CGATAGATTCAGCGGCAGCGGCTTCGGCACCGACTTCACCCTGACAATCAGCTCCCTGC
AGGCCGAGGACGTGGCCGTGTACTACTGTCTGCAGCACGGCAGCATCCCCCCCACATTT
GGCCAGGGCACCAAGGTGGAAATCAAGCGTACGGTGGCCGCCCCCTCCGTGTTCATCTT
CCCCCCCTCCGACGAGCAGCTGAAGTCCGGCACCGCCTCCGTGGTGTGCCTGCTGAATA
ACTTCTACCCCAGAGAGGCCAAGGTGCAGTGGAAGGTGGACAACGCCCTGCAGTCCGGG
AACTCCCAGGAGAGCGTGACCGAGCAGGACAGCAAGGACAGCACCTACAGCCTGAGCAG
CACCCTGACCCTGAGCAAAGCCGACTACGAGAAGCACAAGGTGTACGCCTGCGAGGTGA
CCCACCAGGGCCTGAGCTCCCCCGTCACCAAGAGCTTCAACAGGGGGGAGTGT

(SEQ ID NO: 65 of the Sequence Listing)

[Figure 75]

MVLQTQVFISLLLWISGAYGDTVMTQSPDSLAVSLGERATINCKASQSVGINVDWYQQK
PGQPPKLLIYGASNRHTGVPDRFSGSGFGTDFTLTISSLQAEDVAVYYCLQHGSIPPTF
GQGTKVEIKRTVAAPSVFIFPPSDEQLKSGTASVVCLLNNFYPREAKVQWKVDNALQSG
NSQESVTEQDSKDSTYSLSSTLTLSKADYEKHKVYACEVTHQGLSSPVTKSFNRGEC

(SEQ ID NO: 66 of the Sequence Listing)

[Figure 76]

ATGGTGCTGCAGACCCAGGTGTTCATCTCCCTGCTGCTGTGGATCTCCGGCGCGTACGG
CGATACCGTGATGACCCAGAGCCCTGACAGCCTGGCCGTGTCTCTGGGAGAGAGAGCCA
CCATCAACTGCAAGGCCAGCCAGAGCGTGGGCATCAACGTGGACTGGTATCAGCAGAAG
CCCGGCCAGCCCCCCAAGCTGCTGATCTACGGCGCCAGCAATAGACACACCGGCGTGCC
CGATAGATTCAGCGGCAGCGGCTTCGGCAGAGACTTCACCCTGACCATCAGCTCCCTGC
AGGCCGAGGATGTGGCCGTGTACTACTGTCTGCAGCACGGCAGCATCCCCCCCACATTT
GGCCAGGGCACCAAGGTGGAAATCAAGCGTACGGTGGCCGCCCCCTCCGTGTTCATCTT
CCCCCCCTCCGACGAGCAGCTGAAGTCCGGCACCGCCTCCGTGGTGTGCCTGCTGAATA
ACTTCTACCCCAGAGAGGCCAAGGTGCAGTGGAAGGTGGACAACGCCCTGCAGTCCGGG
AACTCCCAGGAGAGCGTGACCGAGCAGGACAGCAAGGACAGCACCTACAGCCTGAGCAG
CACCCTGACCCTGAGCAAAGCCGACTACGAGAAGCACAAGGTGTACGCCTGCGAGGTGA
CCCACCAGGGCCTGAGCTCCCCCGTCACCAAGAGCTTCAACAGGGGGGAGTGT

(SEQ ID NO: 67 of the Sequence Listing)

[Figure 77]

MVLQTQVFISLLLWISGAYGDTVMTQSPDSLAVSLGERATINCKASQSVGINVDWYQQK
PGQPPKLLIYGASNRHTGVPDRFSGSGFGRDFTLTISSLQAEDVAVYYCLQHGSIPPTF
GQGTKVEIKRTVAAPSVFIFPPSDEQLKSGTASVVCLLNNFYPREAKVQWKVDNALQSG
NSQESVTEQDSKDSTYSLSSTLTLSKADYEKHKVYACEVTHQGLSSPVTKSFNRGEC

(SEQ ID NO: 68 of the Sequence Listing)

[Figure 78]

ATGGTGCTGCAGACCCAGGTGTTCATCTCCCTGCTGCTGTGGATCTCCGGCGCGTACGG
CGATACCGTGATGACCCAGAGCCCTGACAGCCTGGCCGTGTCTCTGGGAGAGAGAGCCA
CCATCAACTGCAAGGCCAGCCAGAGCGTGGGCATCAACGTGGACTGGTATCAGCAGAAG
CCCGGCCAGAGCCCCAAGCTGCTGATCTACGGCGCCAGCAACAGACACACCGGCGTGCC
CGATAGATTCAGCGGCAGCGGCTTCGGCAGAGACTTCACCCTGACCATCAGCTCCCTGC
AGGCCGAGGATGTGGCCGTGTACTACTGTCTGCAGCACGGCAGCATCCCCCCCACATTT
GGCCAGGGCACCAAGGTGGAAATCAAGCGTACGGTGGCCGCCCCCTCCGTGTTCATCTT
CCCCCCCTCCGACGAGCAGCTGAAGTCCGGCACCGCCTCCGTGGTGTGCCTGCTGAATA
ACTTCTACCCCAGAGAGGCCAAGGTGCAGTGGAAGGTGGACAACGCCCTGCAGTCCGGG
AACTCCCAGGAGAGCGTGACCGAGCAGGACAGCAAGGACAGCACCTACAGCCTGAGCAG
CACCCTGACCCTGAGCAAAGCCGACTACGAGAAGCACAAGGTGTACGCCTGCGAGGTGA
CCCACCAGGGCCTGAGCTCCCCCGTCACCAAGAGCTTCAACAGGGGGGAGTGT

(SEQ ID NO: 69 of the Sequence Listing)

[Figure 79]

MVLQTQVFISLLLWISGAYGDTVMTQSPDSLAVSLGERATINCKASQSVGINVDWYQQK
PGQSPKLLIYGASNRHTGVPDRFSGSGFGRDFTLTISSLQAEDVAVYYCLQHGSIPPTF
GQGTKVEIKRTVAAPSVFIFPPSDEQLKSGTASVVCLLNNFYPREAKVQWKVDNALQSG
NSQESVTEQDSKDSTYSLSSTLTLSKADYEKHKVYACEVTHQGLSSPVTKSFNRGEC

(SEQ ID NO: 70 of the Sequence Listing)

[Figure 80]

ATGGTGCTGCAGACCCAGGTGTTCATCTCCCTGCTGCTGTGGATCTCCGGCGCGTACGG
CGCCACCAGAATGACACAGAGCCCCAGCAGCTTCAGCGCCAGCACCGGCGACAGAGTGA
CCATCACATGCAAGGCCAGCCAGAGCGTGGGCATCAACGTGGACTGGTATCAGCAGAAG
CCCGGCAAGAGCCCCAAGCTGCTGATCTACGGCGCCTCCAACAGACACACCGGCGTGCC
CAGCAGATTTTCCGGCAGCGGCTTCGGCAGAGACTTCACCCTGACCATCAGCAGCCTGC
AGAGCGAGGACTTCGCCACCTACTACTGCCTGCAGCACGGCAGCATCCCCCCTACATTT
GGCGGAGGCACCAAGGTGGAAATCAAGCGTACGGTGGCCGCCCCCTCCGTGTTCATCTT
CCCCCCCTCCGACGAGCAGCTGAAGTCCGGCACCGCCTCCGTGGTGTGCCTGCTGAATA
ACTTCTACCCCAGAGAGGCCAAGGTGCAGTGGAAGGTGGACAACGCCCTGCAGTCCGGG
AACTCCCAGGAGAGCGTGACCGAGCAGGACAGCAAGGACAGCACCTACAGCCTGAGCAG
CACCCTGACCCTGAGCAAAGCCGACTACGAGAAGCACAAGGTGTACGCCTGCGAGGTGA
CCCACCAGGGCCTGAGCTCCCCCGTCACCAAGAGCTTCAACAGGGGGGAGTGT

(SEQ ID NO: 71 of the Sequence Listing)

[Figure 81]

MVLQTQVFISLLLWISGAYGATRMTQSPSSFSASTGDRVTITCKASQSVGINVDWYQQK
PGKSPKLLIYGASNRHTGVPSRFSGSGFGRDFTLTISSLQSEDFATYYCLQHGSIPPTF
GGGTKVEIKRTVAAPSVFIFPPSDEQLKSGTASVVCLLNNFYPREAKVQWKVDNALQSG
NSQESVTEQDSKDSTYSLSSTLTLSKADYEKHKVYACEVTHQGLSSPVTKSFNRGEC

(SEQ ID NO: 72 of the Sequence Listing)

[Figure 82]

ATGAAACACCTGTGGTTCTTCCTCCTGCTGGTGGCAGCTCCCAGATGGGTGCTGAGCCA
GGTGCAGCTGGTGGAATCTGGCGGCGGACTCGTGAAGCCTAGCCAGACCCTGAGCCTGA
CCTGCACCTTCAGCGGCTTCAGCCTGAACACCTACGACATGGGCGTGGGCTGGATCAGA
CAGCCTCCTGGCAAGGGCCTGGAATGGATCGGCAACATTTGGTGGGACGACGACAAGTA
CTACAACCCCAGCCTGAAGTCCAGAGTGACCATCAGCGTGGACACCAGCAAGAACCAGG
CCTCCCTGAAGCTGAGCAGCGTGACAGCCGCCGATACCGCCGTGTACTACTGCGCCAGA
ATCGAGACAGTGCGGGTGTCCAGAAAGGGCTTCGCCCACTGGGGACAGGGCACACTCGT
GACCGTCAGCTCAGCCTCCACCAAGGGCCCAAGCGTCTTCCCCCTGGCACCCTCCTCCA
AGAGCACCTCTGGCGGCACAGCCGCCCTGGGCTGCCTGGTCAAGGACTACTTCCCCGAA
CCCGTGACCGTGAGCTGGAACTCAGGCGCCCTGACCAGCGGCGTGCACACCTTCCCCGC
TGTCCTGCAGTCCTCAGGACTCTACTCCCTCAGCAGCGTGGTGACCGTGCCCTCCAGCA
GCTTGGGCACCCAGACCTACATCTGCAACGTGAATCACAAGCCCAGCAACACCAAGGTG
GACAAGAGAGTTGAGCCCAAATCTTGTGACAAAACTCACACATGCCCACCCTGCCCAGC
ACCTGAACTCCTGGGGGGACCCTCAGTCTTCCTCTTCCCCCCAAAACCCAAGGACACCC
TCATGATCTCCCGGACCCCTGAGGTCACATGCGTGGTGGTGGACGTGAGCCACGAAGAC
CCTGAGGTCAAGTTCAACTGGTACGTGGACGGCGTGGAGGTGCATAATGCCAAGACAAA
GCCCCGGGAGGAGCAGTACAACAGCACGTACCGGGTGGTCAGCGTCCTCACCGTCCTGC
ACCAGGACTGGCTGAATGGCAAGGAGTACAAGTGCAAGGTCTCCAACAAAGCCCTCCCA
GCCCCCATCGAGAAAACCATCTCCAAAGCCAAAGGCCAGCCCCGGGAACCACAGGTGTA
CACCCTGCCCCCATCCCGGGAGGAGATGACCAAGAACCAGGTCAGCCTGACCTGCCTGG
TCAAAGGCTTCTATCCCAGCGACATCGCCGTGGAGTGGGAGAGCAATGGCCAGCCCGAG
AACAACTACAAGACCACCCCTCCCGTGCTGGACTCCGACGGCTCCTTCTTCCTCTACAG
CAAGCTCACCGTGGACAAGAGCAGGTGGCAGCAGGGCAACGTCTTCTCATGCTCCGTGA
TGCATGAGGCTCTGCACAACCACTACACCCAGAAGAGCCTCTCCCTGTCTCCCGGCAAA

(SEQ ID NO: 73 of the Sequence Listing)

[Figure 83]

MKHLWFFLLLVAAPRWVLSQVQLVESGGGLVKPSQTLSLTCTFSGFSLNTYDMGVGWIR
QPPGKGLEWIGNIWWDDDKYYNPSLKSRVTISVDTSKNQASLKLSSVTAADTAVYYCAR
IETVRVSRKGFAHWGQGTLVTVSSASTKGPSVFPLAPSSKSTSGGTAALGCLVKDYFPE
PVTVSWNSGALTSGVHTFPAVLQSSGLYSLSSVVTVPSSSLGTQTYICNVNHKPSNTKV
DKRVEPKSCDKTHTCPPCPAPELLGGPSVFLFPPKPKDTLMISRTPEVTCVVVDVSHED
PEVKFNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSPGK

(SEQ ID NO: 74 of the Sequence Listing)

[Figure 84]

ATGAAACACCTGTGGTTCTTCCTCCTGCTGGTGGCAGCTCCCAGATGGGTGCTGAGCCA
GGTGCAGCTGGTGGAATCTGGCGGCGGACTCGTGAAGCCTAGCCAGACCCTGAGCCTGA
CCTGCACCTTCAGCGGCTTCAGCCTGAACACCTACGACATGGGCGTGGGCTGGATCAGA
CAGCCTCCTGGCAAGGGCCTGGAATGGATCGGCAACATTTGGTGGGACGACGACAAGTA
CTACAACCCCAGCCTGAAGTCCAGAGTGACCATCAGCAAGGACACCAGCAACAACCAGG
CCTCCCTGAAGCTGAGCAGCGTGACAGCCGCCGATACCGCCGTGTACTACTGCGCCAGA
ATCGAGACAGTGCGGGTGTCCAGAAAGGGCTTCGCCCACTGGGGACAGGGCACACTCGT
GACCGTCAGCTCAGCCTCCACCAAGGGCCCAAGCGTCTTCCCCCTGGCACCCTCCTCCA
AGAGCACCTCTGGCGGCACAGCCGCCCTGGGCTGCCTGGTCAAGGACTACTTCCCCGAA
CCCGTGACCGTGAGCTGGAACTCAGGCGCCCTGACCAGCGGCGTGCACACCTTCCCCGC
TGTCCTGCAGTCCTCAGGACTCTACTCCCTCAGCAGCGTGGTGACCGTGCCCTCCAGCA
GCTTGGGCACCCAGACCTACATCTGCAACGTGAATCACAAGCCCAGCAACACCAAGGTG
GACAAGAGAGTTGAGCCCAAATCTTGTGACAAAACTCACACATGCCCACCCTGCCCAGC
ACCTGAACTCCTGGGGGGACCCTCAGTCTTCCTCTTCCCCCCAAAACCCAAGGACACCC
TCATGATCTCCCGGACCCCTGAGGTCACATGCGTGGTGGTGGACGTGAGCCACGAAGAC
CCTGAGGTCAAGTTCAACTGGTACGTGGACGGCGTGGAGGTGCATAATGCCAAGACAAA
GCCCCGGGAGGAGCAGTACAACAGCACGTACCGGGTGGTCAGCGTCCTCACCGTCCTGC
ACCAGGACTGGCTGAATGGCAAGGAGTACAAGTGCAAGGTCTCCAACAAAGCCCTCCCA
GCCCCCATCGAGAAAACCATCTCCAAAGCCAAAGGCCAGCCCCGGGAACCACAGGTGTA
CACCCTGCCCCCATCCCGGGAGGAGATGACCAAGAACCAGGTCAGCCTGACCTGCCTGG
TCAAAGGCTTCTATCCCAGCGACATCGCCGTGGAGTGGGAGAGCAATGGCCAGCCCGAG
AACAACTACAAGACCACCCCTCCCGTGCTGGACTCCGACGGCTCCTTCTTCCTCTACAG
CAAGCTCACCGTGGACAAGAGCAGGTGGCAGCAGGGCAACGTCTTCTCATGCTCCGTGA
TGCATGAGGCTCTGCACAACCACTACACCCAGAAGAGCCTCTCCCTGTCTCCCGGCAAA

(SEQ ID NO: 75 of the Sequence Listing)

[Figure 85]

MKHLWFFLLLVAAPRWVLSQVQLVESGGGLVKPSQTLSLTCTFSGFSLNTYDMGVGWIR
QPPGKGLEWIGNIWWDDDKYYNPSLKSRVTISKDTSNNQASLKLSSVTAADTAVYYCAR
IETVRVSRKGFAHWGQGTLVTVSSASTKGPSVFPLAPSSKSTSGGTAALGCLVKDYFPE
PVTVSWNSGALTSGVHTFPAVLQSSGLYSLSSVVTVPSSSLGTQTYICNVNHKPSNTKV
DKRVEPKSCDKTHTCPPCPAPELLGGPSVFLFPPKPKDTLMISRTPEVTCVVVDVSHED
PEVKFNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSPGK

(SEQ ID NO: 76 of the Sequence Listing)

[Figure 86]

ATGAAACACCTGTGGTTCTTCCTCCTGCTGGTGGCAGCTCCCAGATGGGTGCTGAGCCA
GGTGCAGCTGGTGGAATCTGGCGGCGGACTCGTGAAGCCTAGCCAGACCCTGAGCCTGA
CCTGCACCTTCAGCGGCTTCAGCCTGAACACCTACGACATGGGCGTGGGCTGGATCAGA
CAGCCTCCTGGCAAGGGCCTGGAATGGATCGCCAACATTTGGTGGGACGACGACAAGTA
CTACAACCCCAGCCTGAAGTCCAGAGTGACCATCAGCAAGGACACCAGCAACAACCAGG
CCTCCCTGAAGCTGAGCAGCGTGACAGCCGCCGATACCGCCGTGTACTACTGCGCCAGA
ATCGAGACAGTGCGGGTGTCCAGAAAGGGCTTCGCCCACTGGGGACAGGGCACACTCGT
GACCGTCAGCTCAGCCTCCACCAAGGGCCCAAGCGTCTTCCCCCTGGCACCCTCCTCCA
AGAGCACCTCTGGCGGCACAGCCGCCCTGGGCTGCCTGGTCAAGGACTACTTCCCCGAA
CCCGTGACCGTGAGCTGGAACTCAGGCGCCCTGACCAGCGGCGTGCACACCTTCCCCGC
TGTCCTGCAGTCCTCAGGACTCTACTCCCTCAGCAGCGTGGTGACCGTGCCCTCCAGCA
GCTTGGGCACCCAGACCTACATCTGCAACGTGAATCACAAGCCCAGCAACACCAAGGTG
GACAAGAGAGTTGAGCCCAAATCTTGTGACAAAACTCACACATGCCCACCCTGCCCAGC
ACCTGAACTCCTGGGGGGACCCTCAGTCTTCCTCTTCCCCCCAAAACCCAAGGACACCC
TCATGATCTCCCGGACCCCTGAGGTCACATGCGTGGTGGTGGACGTGAGCCACGAAGAC
CCTGAGGTCAAGTTCAACTGGTACGTGGACGGCGTGGAGGTGCATAATGCCAAGACAAA
GCCCCGGGAGGAGCAGTACAACAGCACGTACCGGGTGGTCAGCGTCCTCACCGTCCTGC
ACCAGGACTGGCTGAATGGCAAGGAGTACAAGTGCAAGGTCTCCAACAAAGCCCTCCCA
GCCCCCATCGAGAAAACCATCTCCAAAGCCAAAGGCCAGCCCCGGGAACCACAGGTGTA
CACCCTGCCCCCATCCCGGGAGGAGATGACCAAGAACCAGGTCAGCCTGACCTGCCTGG
TCAAAGGCTTCTATCCCAGCGACATCGCCGTGGAGTGGGAGAGCAATGGCCAGCCCGAG
AACAACTACAAGACCACCCCTCCCGTGCTGGACTCCGACGGCTCCTTCTTCCTCTACAG
CAAGCTCACCGTGGACAAGAGCAGGTGGCAGCAGGGCAACGTCTTCTCATGCTCCGTGA
TGCATGAGGCTCTGCACAACCACTACACCCAGAAGAGCCTCTCCCTGTCTCCCGGCAAA

(SEQ ID NO: 77 of the Sequence Listing)

[Figure 87]

MKHLWFFLLLVAAPRWVLSQVQLVESGGGLVKPSQTLSLTCTFSGFSLNTYDMGVGWIR
QPPGKGLEWIANIWWDDDKYYNPSLKSRVTISKDTSNNQASLKLSSVTAADTAVYYCAR
IETVRVSRKGFAHWGQGTLVTVSSASTKGPSVFPLAPSSKSTSGGTAALGCLVKDYFPE
PVTVSWNSGALTSGVHTFPAVLQSSGLYSLSSVVTVPSSSLGTQTYICNVNHKPSNTKV
DKRVEPKSCDKTHTCPPCPAPELLGGPSVFLFPPKPKDTLMISRTPEVTCVVVDVSHED
PEVKFNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSPGK

(SEQ ID NO: 78 of the Sequence Listing)

[Figure 88]

ATGAAACACCTGTGGTTCTTCCTCCTGCTGGTGGCAGCTCCCAGATGGGTGCTGAGCCA
AGTGACCCTGAAAGAGTCCGGCCCTGCCCTCGTGAAGCCTACCCAGACCCTGACACTGA
CCTGCACCTTCAGCGGCTTCAGCCTGAACACCTACGACATGGGCGTGGGCTGGATCAGA
CAGCCTCCTGGCAAGGGCCTGGAATGGCTGGCCAACATTTGGTGGGACGACGACAAGTA
CTACAGCCCCAGCCTGAAGTCCCGGCTGACCATCACCAAGGACACCAGCAACAACCAGG
CCGTGCTGACCATGACAAACATGGACCCCGTGGACACCGCCACCTACTACTGCGCCAGA
ATCGAGACAGTGCGGGTGTCCAGAAAGGGCTTCGCCCACTGGGGACAGGGCACACTCGT
GACCGTCAGCTCAGCCTCCACCAAGGGCCCAAGCGTCTTCCCCCTGGCACCCTCCTCCA
AGAGCACCTCTGGCGGCACAGCCGCCCTGGGCTGCCTGGTCAAGGACTACTTCCCCGAA
CCCGTGACCGTGAGCTGGAACTCAGGCGCCCTGACCAGCGGCGTGCACACCTTCCCCGC
TGTCCTGCAGTCCTCAGGACTCTACTCCCTCAGCAGCGTGGTGACCGTGCCCTCCAGCA
GCTTGGGCACCCAGACCTACATCTGCAACGTGAATCACAAGCCCAGCAACACCAAGGTG
GACAAGAGAGTTGAGCCCAAATCTTGTGACAAAACTCACACATGCCCACCCTGCCCAGC
ACCTGAACTCCTGGGGGGACCCTCAGTCTTCCTCTTCCCCCCAAAACCCAAGGACACCC
TCATGATCTCCCGGACCCCTGAGGTCACATGCGTGGTGGTGGACGTGAGCCACGAAGAC
CCTGAGGTCAAGTTCAACTGGTACGTGGACGGCGTGGAGGTGCATAATGCCAAGACAAA
GCCCCGGGAGGAGCAGTACAACAGCACGTACCGGGTGGTCAGCGTCCTCACCGTCCTGC
ACCAGGACTGGCTGAATGGCAAGGAGTACAAGTGCAAGGTCTCCAACAAAGCCCTCCCA
GCCCCCATCGAGAAAACCATCTCCAAAGCCAAAGGCCAGCCCCGGGAACCACAGGTGTA
CACCCTGCCCCCATCCCGGGAGGAGATGACCAAGAACCAGGTCAGCCTGACCTGCCTGG
TCAAAGGCTTCTATCCCAGCGACATCGCCGTGGAGTGGGAGAGCAATGGCCAGCCCGAG
AACAACTACAAGACCACCCCTCCCGTGCTGGACTCCGACGGCTCCTTCTTCCTCTACAG
CAAGCTCACCGTGGACAAGAGCAGGTGGCAGCAGGGCAACGTCTTCTCATGCTCCGTGA
TGCATGAGGCTCTGCACAACCACTACACCCAGAAGAGCCTCTCCCTGTCTCCCGGCAAA

(SEQ ID NO: 79 of the Sequence Listing)

[Figure 89]

MKHLWFFLLLVAAPRWVLSQVTLKESGPALVKPTQTLTLTCTFSGFSLNTYDMGVGWIR
QPPGKGLEWLANIWWDDDKYYSPSLKSRLTITKDTSNNQAVLTMTNMDPVDTATYYCAR
IETVRVSRKGFAHWGQGTLVTVSSASTKGPSVFPLAPSSKSTSGGTAALGCLVKDYFPE
PVTVSWNSGALTSGVHTFPAVLQSSGLYSLSSVVTVPSSSLGTQTYICNVNHKPSNTKV
DKRVEPKSCDKTHTCPPCPAPELLGGPSVFLFPPKPKDTLMISRTPEVTCVVVDVSHED
PEVKFNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSPGK

(SEQ ID NO: 80 of the Sequence Listing)

[Figure 90]

ATGGTGCTGCAGACCCAGGTGTTCATCTCCCTGCTGCTGTGGATCTCCGGCGCGTACGG
CGAGATCGTGCTGACACAGAGCCCTGGCACCCTGAGCCTGTCTCCAGGCGAAAGAGCCA
CCCTGTCCTGCAAGGCCAGCCAGAACATCAACAAGTACCTGGACTGGTATCAGCAGAAG
CCCGGCCAGCCCCCCAGACTGCTGATCTACAACACCAACAACCTGCACACCGGCATCCC
CGACAGATTCAGCGGCTCTGGCAGCGGCACCGACTACACCCTGACCATCAGCAGACTGG
AACCCGAGGACTTCGCCGTGTACTACTGCCTGCAGCGGAACAGCTGGTACACCTTCGGC
CAGGGCACCAAGGTGGAAATCAAGCGTACGGTGGCCGCCCCCTCCGTGTTCATCTTCCC
CCCCTCCGACGAGCAGCTGAAGTCCGGCACCGCCTCCGTGGTGTGCCTGCTGAATAACT
TCTACCCCAGAGAGGCCAAGGTGCAGTGGAAGGTGGACAACGCCCTGCAGTCCGGGAAC
TCCCAGGAGAGCGTGACCGAGCAGGACAGCAAGGACAGCACCTACAGCCTGAGCAGCAC
CCTGACCCTGAGCAAAGCCGACTACGAGAAGCACAAGGTGTACGCCTGCGAGGTGACCC
ACCAGGGCCTGAGCTCCCCCGTCACCAAGAGCTTCAACAGGGGGGAGTGT

(SEQ ID NO: 81 of the Sequence Listing)

[Figure 91]

MVLQTQVFISLLLWISGAYGEIVLTQSPGTLSLSPGERATLSCKASQNINKYLDWYQQK
PGQPPRLLIYNTNNLHTGIPDRFSGSGSGTDYTLTISRLEPEDFAVYYCLQRNSWYTFG
QGTKVEIKRTVAAPSVFIFPPSDEQLKSGTASVVCLLNNFYPREAKVQWKVDNALQSGN
SQESVTEQDSKDSTYSLSSTLTLSKADYEKHKVYACEVTHQGLSSPVTKSFNRGEC

(SEQ ID NO: 82 of the Sequence Listing)

[Figure 92]

ATGGTGCTGCAGACCCAGGTGTTCATCTCCCTGCTGCTGTGGATCTCCGGCGCGTACGG
CGACATCCAGATGACCCAGAGCCCTAGCAGCCTGAGCCTGTCCCCTGGCGAAAGAGCCA
CCCTGAGCTGCAAGGCCAGCCAGAACATCAACAAGTACCTGGACTGGTATCAGCAGAAG
CCCGGCCAGCCCCCCAGACTGCTGATCTACAACACCAACAACCTGCACACCGGCATCCC
CGACAGATTCAGCGGCTCTGGCAGCGGCACCGACTACACCCTGACCATCAGCAGACTGG
AACCCGAGGACTTCGCCGTGTACTACTGCCTGCAGCGGAACAGCTGGTACACCTTCGGC
CAGGGCACCAAGGTGGAAATCAAGCGTACGGTGGCCGCCCCCTCCGTGTTCATCTTCCC
CCCCTCCGACGAGCAGCTGAAGTCCGGCACCGCCTCCGTGGTGTGCCTGCTGAATAACT
TCTACCCCAGAGAGGCCAAGGTGCAGTGGAAGGTGGACAACGCCCTGCAGTCCGGGAAC
TCCCAGGAGAGCGTGACCGAGCAGGACAGCAAGGACAGCACCTACAGCCTGAGCAGCAC
CCTGACCCTGAGCAAAGCCGACTACGAGAAGCACAAGGTGTACGCCTGCGAGGTGACCC
ACCAGGGCCTGAGCTCCCCCGTCACCAAGAGCTTCAACAGGGGGGAGTGT

(SEQ ID NO: 83 of the Sequence Listing)

[Figure 93]

MVLQTQVFISLLLWISGAYGDIQMTQSPSSLSLSPGERATLSCKASQNINKYLDWYQQK
PGQPPRLLIYNTNNLHTGIPDRFSGSGSGTDYTLTISRLEPEDFAVYYCLQRNSWYTFG
QGTKVEIKRTVAAPSVFIFPPSDEQLKSGTASVVCLLNNFYPREAKVQWKVDNALQSGN
SQESVTEQDSKDSTYSLSSTLTLSKADYEKHKVYACEVTHQGLSSPVTKSFNRGEC

(SEQ ID NO: 84 of the Sequence Listing)

[Figure 94]

ATGAAACACCTGTGGTTCTTCCTCCTGCTGGTGGCAGCTCCCAGATGGGTGCTGAGCCA
GGTGCAGCTGCAGGAATCTGGCCCTGGCCTCGTGAAGCCTAGCCAGACCCTGAGCCTGA
CCTGTACCGTGTCCGGCTACACCATCACCAGCGGCTACGACTGGTCCTGGATCAGACAG
CCTCCTGGCAAGGGCCTGGAATGGATCGCCTACATGAGCTACCGGGGCAGCACCAACTA
CAACCCCAGCCTGAAGTCCAGAGTGACCATCAGCCGGGACACCAGCAAGAACCAGTTCT
CCCTGAAGCTGAGCAGCGTGACAGCCGCCGATACCGCCGTGTACTACTGCGCCCTGACC
CGGACCTACTGGTACAACTACTACTACGTGCTGGACGCCTGGGGCCAGGGCACACTCGT
GACCGTCAGCTCAGCCTCCACCAAGGGCCCAAGCGTCTTCCCCCTGGCACCCTCCTCCA
AGAGCACCTCTGGCGGCACAGCCGCCCTGGGCTGCCTGGTCAAGGACTACTTCCCCGAA
CCCGTGACCGTGAGCTGGAACTCAGGCGCCCTGACCAGCGGCGTGCACACCTTCCCCGC
TGTCCTGCAGTCCTCAGGACTCTACTCCCTCAGCAGCGTGGTGACCGTGCCCTCCAGCA
GCTTGGGCACCCAGACCTACATCTGCAACGTGAATCACAAGCCCAGCAACACCAAGGTG
GACAAGAGAGTTGAGCCCAAATCTTGTGACAAAACTCACACATGCCCACCCTGCCCAGC
ACCTGAACTCCTGGGGGGACCCTCAGTCTTCCTCTTCCCCCCAAAACCCAAGGACACCC
TCATGATCTCCCGGACCCCTGAGGTCACATGCGTGGTGGTGGACGTGAGCCACGAAGAC
CCTGAGGTCAAGTTCAACTGGTACGTGGACGGCGTGGAGGTGCATAATGCCAAGACAAA
GCCCCGGGAGGAGCAGTACAACAGCACGTACCGGGTGGTCAGCGTCCTCACCGTCCTGC
ACCAGGACTGGCTGAATGGCAAGGAGTACAAGTGCAAGGTCTCCAACAAAGCCCTCCCA
GCCCCCATCGAGAAAACCATCTCCAAAGCCAAAGGCCAGCCCCGGGAACCACAGGTGTA
CACCCTGCCCCCATCCCGGGAGGAGATGACCAAGAACCAGGTCAGCCTGACCTGCCTGG
TCAAAGGCTTCTATCCCAGCGACATCGCCGTGGAGTGGGAGAGCAATGGCCAGCCCGAG
AACAACTACAAGACCACCCCTCCCGTGCTGGACTCCGACGGCTCCTTCTTCCTCTACAG
CAAGCTCACCGTGGACAAGAGCAGGTGGCAGCAGGGCAACGTCTTCTCATGCTCCGTGA
TGCATGAGGCTCTGCACAACCACTACACCCAGAAGAGCCTCTCCCTGTCTCCCGGCAAA

(SEQ ID NO: 85 of the Sequence Listing)

[Figure 95]

MKHLWFFLLLVAAPRWVLSQVQLQESGPGLVKPSQTLSLTCTVSGYTITSGYDWSWIRQ
PPGKGLEWIAYMSYRGSTNYNPSLKSRVTISRDTSKNQFSLKLSSVTAADTAVYYCALT
RTYWYNYYYVLDAWGQGTLVTVSSASTKGPSVFPLAPSSKSTSGGTAALGCLVKDYFPE
PVTVSWNSGALTSGVHTFPAVLQSSGLYSLSSVVTVPSSSLGTQTYICNVNHKPSNTKV
DKRVEPKSCDKTHTCPPCPAPELLGGPSVFLFPPKPKDTLMISRTPEVTCVVVDVSHED
PEVKFNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSPGK

(SEQ ID NO: 86 of the Sequence Listing)

[Figure 96]

ATGAAACACCTGTGGTTCTTCCTCCTGCTGGTGGCAGCTCCCAGATGGGTGCTGAGCGA
GATCCAGCTGCAGGAATCTGGCCCTGGCCTCGTGAAGCCTAGCCAGACCCTGAGCCTGA
CCTGTACCGTGTCCGGCTACACCATCACCAGCGGCTACGACTGGTCCTGGATCAGACAG
CCTCCTGGCAAGGGCCTGGAATGGATCGGCTACATGAGCTACCGGGGCAGCACCAACTA
CAACCCCAGCCTGAAGTCCAGAGTGACCATCAGCCGGGACACCAGCAAGAACCAGTTCT
CCCTGAAGCTGAGCAGCGTGACAGCCGCCGATACCGCCGTGTACTACTGCGCCCTGACC
CGGACCTACTGGTACAACTACTACTACGTGCTGGACGCCTGGGGCCAGGGCACACTCGT
GACCGTCAGCTCAGCCTCCACCAAGGGCCCAAGCGTCTTCCCCCTGGCACCCTCCTCCA
AGAGCACCTCTGGCGGCACAGCCGCCCTGGGCTGCCTGGTCAAGGACTACTTCCCCGAA
CCCGTGACCGTGAGCTGGAACTCAGGCGCCCTGACCAGCGGCGTGCACACCTTCCCCGC
TGTCCTGCAGTCCTCAGGACTCTACTCCCTCAGCAGCGTGGTGACCGTGCCCTCCAGCA
GCTTGGGCACCCAGACCTACATCTGCAACGTGAATCACAAGCCCAGCAACACCAAGGTG
GACAAGAGAGTTGAGCCCAAATCTTGTGACAAAACTCACACATGCCCACCCTGCCCAGC
ACCTGAACTCCTGGGGGGACCCTCAGTCTTCCTCTTCCCCCCAAAACCCAAGGACACCC
TCATGATCTCCCGGACCCCTGAGGTCACATGCGTGGTGGTGGACGTGAGCCACGAAGAC
CCTGAGGTCAAGTTCAACTGGTACGTGGACGGCGTGGAGGTGCATAATGCCAAGACAAA
GCCCCGGGAGGAGCAGTACAACAGCACGTACCGGGTGGTCAGCGTCCTCACCGTCCTGC
ACCAGGACTGGCTGAATGGCAAGGAGTACAAGTGCAAGGTCTCCAACAAAGCCCTCCCA
GCCCCCATCGAGAAAACCATCTCCAAAGCCAAAGGCCAGCCCCGGGAACCACAGGTGTA
CACCCTGCCCCCATCCCGGGAGGAGATGACCAAGAACCAGGTCAGCCTGACCTGCCTGG
TCAAAGGCTTCTATCCCAGCGACATCGCCGTGGAGTGGGAGAGCAATGGCCAGCCCGAG
AACAACTACAAGACCACCCCTCCCGTGCTGGACTCCGACGGCTCCTTCTTCCTCTACAG
CAAGCTCACCGTGGACAAGAGCAGGTGGCAGCAGGGCAACGTCTTCTCATGCTCCGTGA
TGCATGAGGCTCTGCACAACCACTACACCCAGAAGAGCCTCTCCCTGTCTCCCGGCAAA

(SEQ ID NO: 87 of the Sequence Listing)

[Figure 97]

MKHLWFFLLLVAAPRWVLSEIQLQESGPGLVKPSQTLSLTCTVSGYTITSGYDWSWIRQ
PPGKGLEWIGYMSYRGSTNYNPSLKSRVTISRDTSKNQFSLKLSSVTAADTAVYYCALT
RTYWYNYYYVLDAWGQGTLVTVSSASTKGPSVFPLAPSSKSTSGGTAALGCLVKDYFPE
PVTVSWNSGALTSGVHTFPAVLQSSGLYSLSSVVTVPSSSLGTQTYICNVNHKPSNTKV
DKRVEPKSCDKTHTCPPCPAPELLGGPSVFLFPPKPKDTLMISRTPEVTCVVVDVSHED
PEVKFNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSPGK

(SEQ ID NO: 88 of the Sequence Listing)

[Figure 98]

ATGAAACACCTGTGGTTCTTCCTCCTGCTGGTGGCAGCTCCCAGATGGGTGCTGAGCGA
GATCCAGCTGCAGGAATCTGGCCCTGGCCTCGTGAAGCCTAGCCAGACCCTGAGCCTGA
CCTGTACCGTGTCCGGCTACACCATCACCAGCGGCTACGACTGGTCCTGGATCAGACAG
CCTCCTGGCAAGGGCCTGGAATGGATCGCCTACATGAGCTACCGGGGCAGCACCAACTA
CAACCCCAGCCTGAAGTCCAGAGTGACCATCAGCCGGGACACCAGCAAGAACCAGTTCT
CCCTGAAGCTGAGCAGCGTGACAGCCGCCGATACCGCCGTGTACTACTGCGCCCTGACC
CGGACCTACTGGTACAACTACTACTACGTGCTGGACGCCTGGGGCCAGGGCACACTCGT
GACCGTCAGCTCAGCCTCCACCAAGGGCCCAAGCGTCTTCCCCCTGGCACCCTCCTCCA
AGAGCACCTCTGGCGGCACAGCCGCCCTGGGCTGCCTGGTCAAGGACTACTTCCCCGAA
CCCGTGACCGTGAGCTGGAACTCAGGCGCCCTGACCAGCGGCGTGCACACCTTCCCCGC
TGTCCTGCAGTCCTCAGGACTCTACTCCCTCAGCAGCGTGGTGACCGTGCCCTCCAGCA
GCTTGGGCACCCAGACCTACATCTGCAACGTGAATCACAAGCCCAGCAACACCAAGGTG
GACAAGAGAGTTGAGCCCAAATCTTGTGACAAAACTCACACATGCCCACCCTGCCCAGC
ACCTGAACTCCTGGGGGGACCCTCAGTCTTCCTCTTCCCCCCAAAACCCAAGGACACCC
TCATGATCTCCCGGACCCCTGAGGTCACATGCGTGGTGGTGGACGTGAGCCACGAAGAC
CCTGAGGTCAAGTTCAACTGGTACGTGGACGGCGTGGAGGTGCATAATGCCAAGACAAA
GCCCCGGGAGGAGCAGTACAACAGCACGTACCGGGTGGTCAGCGTCCTCACCGTCCTGC
ACCAGGACTGGCTGAATGGCAAGGAGTACAAGTGCAAGGTCTCCAACAAAGCCCTCCCA
GCCCCCATCGAGAAAACCATCTCCAAAGCCAAAGGCCAGCCCCGGGAACCACAGGTGTA
CACCCTGCCCCCATCCCGGGAGGAGATGACCAAGAACCAGGTCAGCCTGACCTGCCTGG
TCAAAGGCTTCTATCCCAGCGACATCGCCGTGGAGTGGGAGAGCAATGGCCAGCCCGAG
AACAACTACAAGACCACCCCTCCCGTGCTGGACTCCGACGGCTCCTTCTTCCTCTACAG
CAAGCTCACCGTGGACAAGAGCAGGTGGCAGCAGGGCAACGTCTTCTCATGCTCCGTGA
TGCATGAGGCTCTGCACAACCACTACACCCAGAAGAGCCTCTCCCTGTCTCCCGGCAAA

(SEQ ID NO: 89 of the Sequence Listing)

[Figure 99]

MKHLWFFLLLVAAPRWVLSEIQLQESGPGLVKPSQTLSLTCTVSGYTITSGYDWSWIRQ
PPGKGLEWIAYMSYRGSTNYNPSLKSRVTISRDTSKNQFSLKLSSVTAADTAVYYCALT
RTYWYNYYYVLDAWGQGTLVTVSSASTKGPSVFPLAPSSKSTSGGTAALGCLVKDYFPE
PVTVSWNSGALTSGVHTFPAVLQSSGLYSLSSVVTVPSSSLGTQTYICNVNHKPSNTKV
DKRVEPKSCDKTHTCPPCPAPELLGGPSVFLFPPKPKDTLMISRTPEVTCVVVDVSHED
PEVKFNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSPGK

(SEQ ID NO: 90 of the Sequence Listing)

[Figure 100]

ATGAAACACCTGTGGTTCTTCCTCCTGCTGGTGGCAGCTCCCAGATGGGTGCTGAGCGA
GATCCAGCTGCAGGAATCTGGCCCTGGCCTCGTGAAGCCTAGCCAGACCCTGAGCCTGA
CCTGTACCGTGTCCGGCTACACCATCACCAGCGGCTACGACTGGTCCTGGATCAGAAAG
CCCCCTGGCAACAAGATGGAATGGATCGCCTACATGAGCTACCGGGGCAGCACCAACTA
CAACCCCAGCCTGAAGTCCAGAGTGACCATCAGCCGGGACACCAGCAAGAACCAGTTCT
CCCTGAAGCTGAGCAGCGTGACAGCCGCCGATACCGCCGTGTACTACTGCGCCCTGACC
CGGACCTACTGGTACAACTACTACTACGTGCTGGACGCCTGGGGCCAGGGCACACTCGT
GACCGTCAGCTCAGCCTCCACCAAGGGCCCAAGCGTCTTCCCCCTGGCACCCTCCTCCA
AGAGCACCTCTGGCGGCACAGCCGCCCTGGGCTGCCTGGTCAAGGACTACTTCCCCGAA
CCCGTGACCGTGAGCTGGAACTCAGGCGCCCTGACCAGCGGCGTGCACACCTTCCCCGC
TGTCCTGCAGTCCTCAGGACTCTACTCCCTCAGCAGCGTGGTGACCGTGCCCTCCAGCA
GCTTGGGCACCCAGACCTACATCTGCAACGTGAATCACAAGCCCAGCAACACCAAGGTG
GACAAGAGAGTTGAGCCCAAATCTTGTGACAAAACTCACACATGCCCACCCTGCCCAGC
ACCTGAACTCCTGGGGGGACCCTCAGTCTTCCTCTTCCCCCCAAAACCCAAGGACACCC
TCATGATCTCCCGGACCCCTGAGGTCACATGCGTGGTGGTGGACGTGAGCCACGAAGAC
CCTGAGGTCAAGTTCAACTGGTACGTGGACGGCGTGGAGGTGCATAATGCCAAGACAAA
GCCCCGGGAGGAGCAGTACAACAGCACGTACCGGGTGGTCAGCGTCCTCACCGTCCTGC
ACCAGGACTGGCTGAATGGCAAGGAGTACAAGTGCAAGGTCTCCAACAAAGCCCTCCCA
GCCCCCATCGAGAAAACCATCTCCAAAGCCAAAGGCCAGCCCCGGGAACCACAGGTGTA
CACCCTGCCCCCATCCCGGGAGGAGATGACCAAGAACCAGGTCAGCCTGACCTGCCTGG
TCAAAGGCTTCTATCCCAGCGACATCGCCGTGGAGTGGGAGAGCAATGGCCAGCCCGAG
AACAACTACAAGACCACCCCTCCCGTGCTGGACTCCGACGGCTCCTTCTTCCTCTACAG
CAAGCTCACCGTGGACAAGAGCAGGTGGCAGCAGGGCAACGTCTTCTCATGCTCCGTGA
TGCATGAGGCTCTGCACAACCACTACACCCAGAAGAGCCTCTCCCTGTCTCCCGGCAAA

(SEQ ID NO: 91 of the Sequence Listing)

[Figure 101]

MKHLWFFLLLVAAPRWVLSEIQLQESGPGLVKPSQTLSLTCTVSGYTITSGYDWSWIRK
PPGNKMEWIAYMSYRGSTNYNPSLKSRVTISRDTSKNQFSLKLSSVTAADTAVYYCALT
RTYWYNYYYVLDAWGQGTLVTVSSASTKGPSVFPLAPSSKSTSGGTAALGCLVKDYFPE
PVTVSWNSGALTSGVHTFPAVLQSSGLYSLSSVVTVPSSSLGTQTYICNVNHKPSNTKV
DKRVEPKSCDKTHTCPPCPAPELLGGPSVFLFPPKPKDTLMISRTPEVTCVVVDVSHED
PEVKFNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSPGK

(SEQ ID NO: 92 of the Sequence Listing)

[Figure 102]

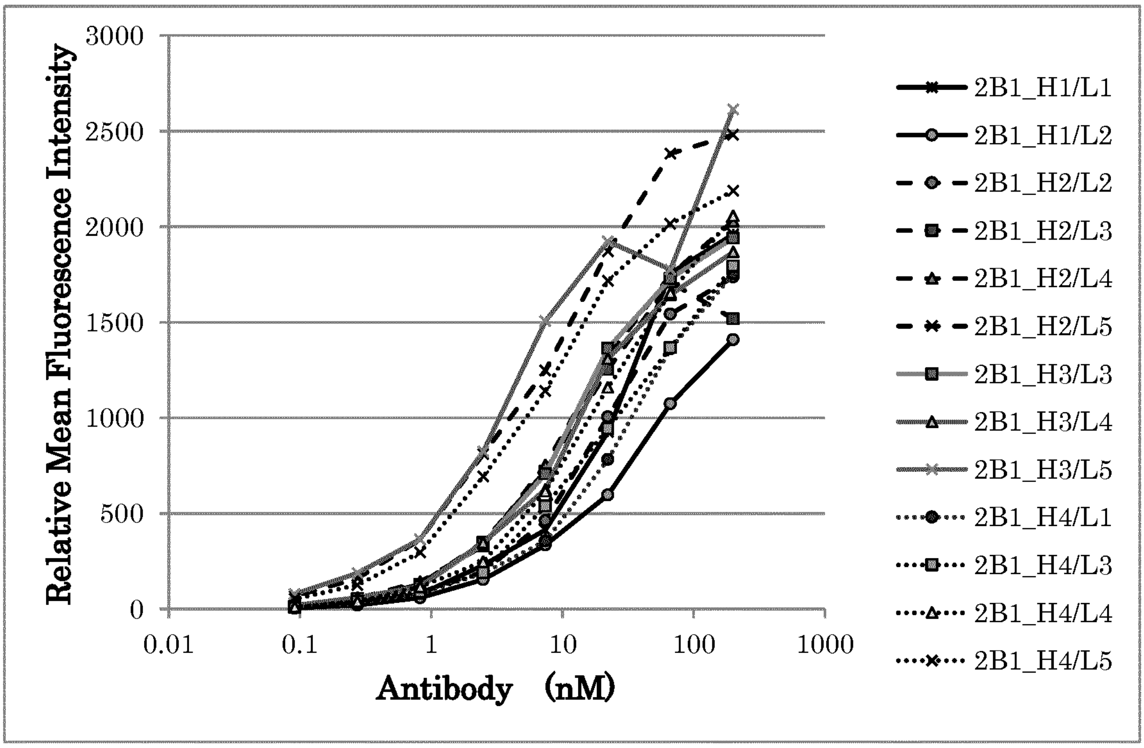


[Figure 103]

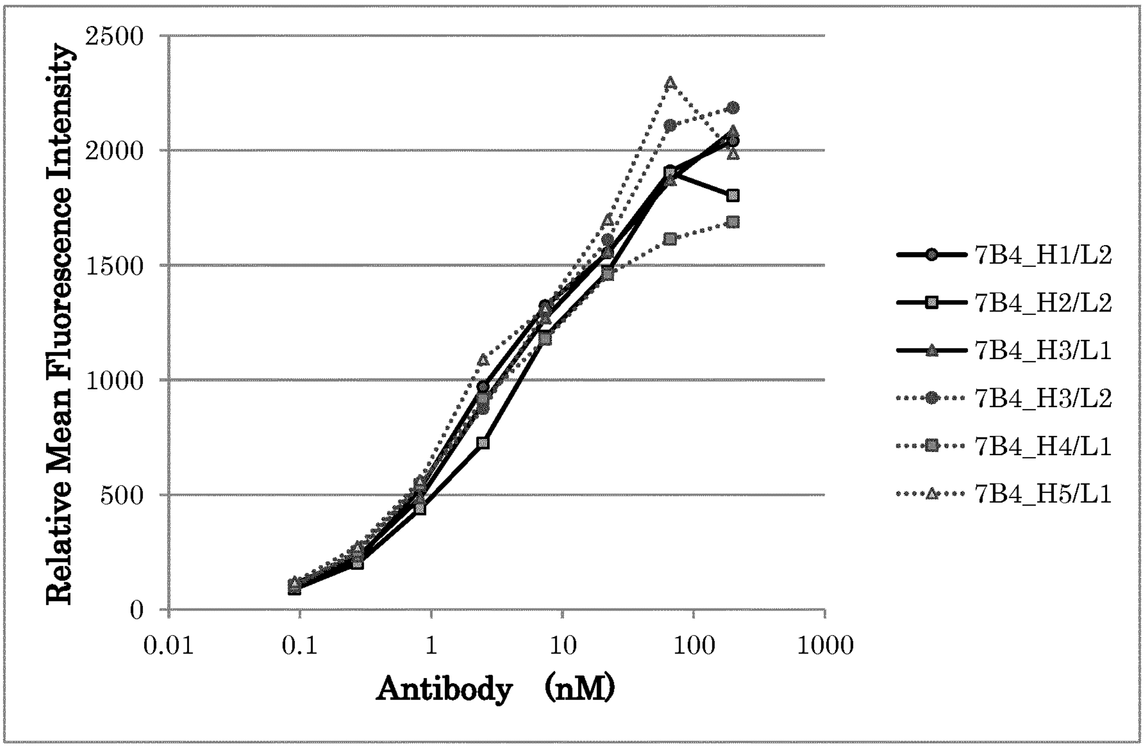

[Figure 104]

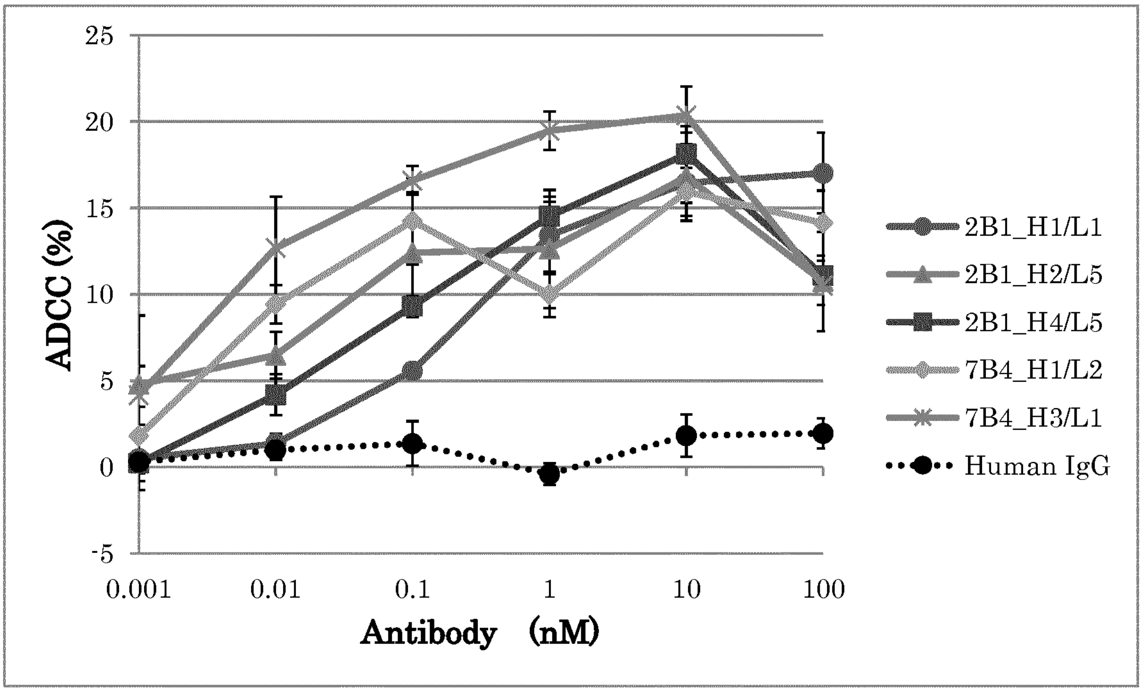
25
20
15
10
5
0
-5
ADCC (%)
0.001
0.01
0.1
1
10
100
Antibody (nM)
2B1_H1/L1
2B1_H2/L5
2B1_H4/L5
7B4_H1/L2
7B4_H3/L1
Human IgG

[Figure 105]
MYKDCIESTGDYFLPCDSEGPWGIVLEK(BIOTIN)-$NH_2$

(SEQ ID NO: 93 of the Sequence Listing)

[Figure 106]
5'-CTCTTCGCTATTACGCCAGCTGGCGA-3'
'
(SEQ ID NO: 94 of the Sequence Listing)

[Figure 107]
5'-ATAACAATTTCACACAGGAAACAGCTATGA-3'

(SEQ ID NO: 95 of the Sequence Listing)

[Figure 108]
CAGGTGCAGCTGGTGCAGTCTGGGGCTGAGGTGAAGAAGCCTGGGGCCTCAGTGAAGGT
CTCCTGCAAGGCTTCTGGATACACCTTCACCGGCTACTATATGCACTGGGTGCGACAGG
CCCCTGGACAAGGGCTTGAGTGGATGGGACGGATCAACCCTAACAGTGGTGGCACAAAC
TATGCACAGAAGTTTCAGGGCAGGGTCACCATGACCAGGGACACGTCCATCAGCACAGC
CTACATGGAGCTGAGCAGGCTGAGATCTGACGACACGGCCGTGTATTACTGTGCGAGTG
GGAAGAGTAACTACCCCTGGGGCCAGGGAACCCTGGTCACCGTCTCCTCA

(SEQ ID NO: 96 of the Sequence Listing)

[Figure 109]
QVQLVQSGAEVKKPGASVKVSCKASGYTFTGYYMHWVRQAPGQGLEWMGRINPNSGGTN
YAQKFQGRVTMTRDTSISTAYMELSRLRSDDTAVYYCASGKSNYPWGQGTLVTVSS

(SEQ ID NO: 97 of the Sequence Listing)

[Figure 110]

CAGCCTGTGCTGACTCAGCCACCCTCAGCGTCTGGGACCCCCGGGCAGAGGGTCACCAT
CTCTTGTTCTGGAAGCAACTCCAACATCGGCAGTAGAACTGTGAATTGGTACCAGCACC
TCCCAGGAACGGCCCCCAGACTCCTCATCTATGGTAATAATCAGTGGCCCTCAGGGGTC
CCTGACCGATTCTCTGGCTCTAAGTCTGGCTCCTCAGCCTCCCTGGCCATCAGTGGGCT
CCGGTCCGAGGATGAGGCTGATTATTACTGTGCAGCATGGGATGACAGCCTGAGTGGTG
TGGTATTCGGCGGAGGGACCAAGCTGACCGTCCTA

(SEQ ID NO: 98 of the Sequence Listing)

[Figure 111]

QPVLTQPPSASGTPGQRVTISCSGSNSNIGSRTVNWYQHLPGTAPRLLIYGNNQWPSGV
PDRFSGSKSGSSASLAISGLRSEDEADYYCAAWDDSLSGVVFGGGTKLTVL

(SEQ ID NO: 99 of the Sequence Listing)

[Figure 112]

CAGGTGCAGCTGGTGCAGTCTGGGGCTGAGGTGAAGAAGCCTGGGTCCTCGGTGAAGGT
CTCCTGCAAGGCTTCTGGAGGCACCTTCAGCAGGTATGCTATCAGCTGGGTGCGACAGG
CCCCTGGACAAGGGCTTGAGTGGATGGGAGGGATCATCCCTATCTTTGGAACAGCAAAC
TACGCACAGAAGTTCCAGGGCAGAGTCACAATTACCGCGGACCAATCCACGAGAACAGC
CTACATGGACCTGGGCAGACTGAGATCTGAGGACACGGCCGTGTATTATTGTGCTCTCG
AGGGATTCAGGGACTCATTAAAACGAAATCCTTTTGATATCTGGGGCCAAGGGACAATG
GTCACCGTCTCTTCA

(SEQ ID NO: 100 of the Sequence Listing)

[Figure 113]

QVQLVQSGAEVKKPGSSVKVSCKASGGTFSRYAISWVRQAPGQGLEWMGGIIPIFGTAN
YAQKFQGRVTITADQSTRTAYMDLGRLRSEDTAVYYCALEGFRDSLKRNPFDIWGQGTM
VTVSS

(SEQ ID NO: 101 of the Sequence Listing)

[Figure 114]

CAGTCTGTCGTGACGCAGCCGCCCTCAGTGTCTGCGGCCCCAGGACAGAAGGTCACCAT
CTCCTGCTCTGGAGGCACCACCAACATTGGGAGTAATTATGTATCCTGGTATCAGCAGC
TCCCAGGAACAGCCCCCAAATTCCTCATGTATGAAAATAATAAGCGACCCTCAGGCATT
CCTGACCGATTCTCTGGCTCCAAGTCTGGCACGTCAGCCACCCTGGCCATCACCGGACT
CCAGACTGGGGACGAGGCCACTTATTACTGCTCAACATGGGATAGCAGCCTGAATACTG
GGCTATTCGGCGGAGGGACCAAGCTGACCGTCCTA

(SEQ ID NO: 102 of the Sequence Listing)

[Figure 115]

QSVVTQPPSVSAAPGQKVTISCSGGTTNIGSNYVSWYQQLPGTAPKFLMYENNKRPSGI
PDRFSGSKSGTSATLAITGLQTGDEATYYCSTWDSSLNTGLFGGGTKLTVL

(SEQ ID NO: 103 of the Sequence Listing)

[Figure 116]

GAGGTGCAGCTGGTGGAGTCCGGGGGAGGCGTGGTCCAGCCTGGGAGGTCCCTGAGACT
CTCCTGTGCAGCCTCTGGATTCACCTTTAGGAACTATGCCATGAGCTGGGTCCGCCAGG
CTCCAGGGAAGGGGCTGGAGTGGGTCTCAGGTATTAGTGGTACTGGTGGTAGCACATAC
TACGCAGACTCCGTGAAGGGCCGGTTCACCATCTCCAGAGACAATTCCAAGGACACACT
ATATCTGCAATTGAACAGCCTGAGAGCCGAGGACACGGCCGTATATTACTGTGCGAAAT
CCCGTAGTATGAGTATCAACTACGGTATGGACGTCTGGGGCCAAGGGACCACGGTCACC
GTCTCCTCA

(SEQ ID NO: 104 of the Sequence Listing)

[Figure 117]

EVQLVESGGGVVQPGRSLRLSCAASGFTFRNYAMSWVRQAPGKGLEWVSGISGTGGSTY
YADSVKGRFTISRDNSKDTLYLQLNSLRAEDTAVYYCAKSRSMSINYGMDVWGQGTTVT
VSS

(SEQ ID NO: 105 of the Sequence Listing)

[Figure 118]

TCCTATGAGCTGACTCAGCCACCCTCAGTGTCCGTGTCCCCAGGACAGACAGTCAGCAT
CACCTGCTCTGGAGATAATTTGGGTGATAGATATGCCTCCTGGTATCAGCAGAAGCCAG
GCCAGTCCCCTGTGCAGGTCATCTATCAAGATACCAAGCGGCCCTCAGGGATCCCTGAG
CGATTCTCTGGCTCCAACTCTGGGAACACAGCCACTCTGACCATCAGCGGGACCCAGGC
TATGGATGAGGCTGACTATTACTGTCAGGCGTGGGACAGCAACACTGTGGTATTCGGCG
GAGGGACCAAGCTGACCGTCCTA

(SEQ ID NO: 106 of the Sequence Listing)

[Figure 119]

SYELTQPPSVSVSPGQTVSITCSGDNLGDRYASWYQQKPGQSPVQVIYQDTKRPSGIPE
RFSGSNSGNTATLTISGTQAMDEADYYCQAWDSNTVVFGGGTKLTVL

(SEQ ID NO: 107 of the Sequence Listing)

[Figure 120]

CAGGTGCAGCTGGTGCAGTCTGGGGCTGAGGTGAAGAAGCCTGGGGCCTCAGTGAAGGT
CTCCTGCAAGGCTTCTGGATACACCTTCACCGGCTACTATATGCACTGGGTGCGACAGG
CCCCTGGACAAGGGCTTGAGTGGATGGGACGGATCAACCCTAACAGTGGTGGCACAAAC
TATGCACAGAAGTTTCAGGGCAGGGTCACCATGACCAGGGACACGTCCATCAGCACAGC
CTACATGGAGCTGAGCGGGCTGAGATCTGACGACACGGCCGTTTATTACTGTGCGAGTG
GTAGTGTAAGGCATCCCTGGGGCCAGGGAACCCTGGTCACCGTCTCCTCA

(SEQ ID NO: 108 of the Sequence Listing)

[Figure 121]

QVQLVQSGAEVKKPGASVKVSCKASGYTFTGYYMHWVRQAPGQGLEWMGRINPNSGGTN
YAQKFQGRVTMTRDTSISTAYMELSGLRSDDTAVYYCASGSVRHPWGQGTLVTVSS

(SEQ ID NO: 109 of the Sequence Listing)

[Figure 122]
CAGCCTGTGCTGACTCAGCCACCCTCAGCGTCTGGGACCCCCGGGCAGAGGGTCACCAT
CTCTTGTTCTGGAAGCAGGTCCAACATCGGAGGTAATATTGTAAGTTGGTACCAGCAGT
TCCCAGGAACGGCCCCCAGACTCCTCACTTATGCTGATAATCAGCGGCCCTCAGGGGTC
CCTGACCGATTCTCTGGCTCCAAGTCTGGCACCTCAGCCTCCCTGGCCATCAGTGGCCT
CCAGTCTGAGGATGAGGCTGACTATTATTGTGCAGCATGGGATGACAGCCTGAATGGTG
TTGTGTTCGGAGGAGGCACCAAGCTGACCGTCCTA

(SEQ ID NO: 110 of the Sequence Listing)

[Figure 123]
QPVLTQPPSASGTPGQRVTISCSGSRSNIGGNIVSWYQQFPGTAPRLLTYADNQRPSGV
PDRFSGSKSGTSASLAISGLQSEDEADYYCAAWDDSLNGVVFGGGTKLTVL

(SEQ ID NO: 135 of the Sequence Listing)

[Figure 124]
GYTFTGYY

(SEQ ID NO: 111 of the Sequence Listing)

[Figure 125]
INPNSGGT

(SEQ ID NO: 112 of the Sequence Listing)

[Figure 126]
ASGKSNYP

(SEQ ID NO: 113 of the Sequence Listing)

[Figure 127]
NSNIGSRT

(SEQ ID NO: 114 of the Sequence Listing)

[Figure 128]
GNN

(SEQ ID NO: 115 of the Sequence Listing)

[Figure 129]
AAWDDSLSGVV

(SEQ ID NO: 116 of the Sequence Listing)

[Figure 130]
GGTFSRYA

(SEQ ID NO: 117 of the Sequence Listing)

[Figure 131]
IIPIFGTA

(SEQ ID NO: 118 of the Sequence Listing)

[Figure 132]
ALEGFRDSLKRNPFDI

(SEQ ID NO: 119 of the Sequence Listing)

[Figure 133]
TTNIGSNY

(SEQ ID NO: 120 of the Sequence Listing)

[Figure 134]
ENN

(SEQ ID NO: 121 of the Sequence Listing)

[Figure 135]
STWDSSLNTGL

(SEQ ID NO: 122 of the Sequence Listing)

[Figure 136]
GFTFRNYA

(SEQ ID NO: 123 of the Sequence Listing)

[Figure 137]
ISGTGGST

(SEQ ID NO: 124 of the Sequence Listing)

[Figure 138]
AKSRSMSINYGMDV

(SEQ ID NO: 125 of the Sequence Listing)

[Figure 139]
NLGDRY

(SEQ ID NO: 126 of the Sequence Listing)

[Figure 140]
QDT

(SEQ ID NO: 127 of the Sequence Listing)

[Figure 141]
QAWDSNTVV

(SEQ ID NO: 128 of the Sequence Listing)

[Figure 142]
GYTFTGYY

(SEQ ID NO: 129 of the Sequence Listing)

[Figure 143]
INPNSGGT

(SEQ ID NO: 130 of the Sequence Listing)

[Figure 144]
ASGSVRHP

(SEQ ID NO: 131 of the Sequence Listing)

[Figure 145]
RSNIGGNI

(SEQ ID NO: 132 of the Sequence Listing)

[Figure 146]
ADN

(SEQ ID NO: 133 of the Sequence Listing)

[Figure 147]
AAWDDSLNGVV

(SEQ ID NO: 134 of the Sequence Listing)

[Figure 148]

ATGAAACACCTGTGGTTCTTCCTCCTGCTGGTGGCAGCTCCCAGATGGGTGCTGAGCCA
GGTGCAGCTGGTGCAGTCTGGGGCTGAGGTGAAGAAGCCTGGGGCCTCAGTGAAGGTCT
CCTGCAAGGCTTCTGGATACACCTTCACCGGCTACTATATGCACTGGGTGCGACAGGCC
CCTGGACAAGGGCTTGAGTGGATGGGACGGATCAACCCTAACAGTGGTGGCACAAACTA
TGCACAGAAGTTTCAGGGCAGGGTCACCATGACCAGGGACACGTCCATCAGCACAGCCT
ACATGGAGCTGAGCAGGCTGAGATCTGACGACACGGCCGTGTATTACTGTGCGAGTGGG
AAGAGTAACTACCCCTGGGGCCAGGGAACCCTGGTCACCGTCAGCTCAGCCTCCACCAA
GGGCCCAAGCGTCTTCCCCCTGGCACCCTCCTCCAAGAGCACCTCTGGCGGCACAGCCG
CCCTGGGCTGCCTGGTCAAGGACTACTTCCCCGAACCCGTGACCGTGAGCTGGAACTCA
GGCGCCCTGACCAGCGGCGTGCACACCTTCCCCGCTGTCCTGCAGTCCTCAGGACTCTA
CTCCCTCAGCAGCGTGGTGACCGTGCCCTCCAGCAGCTTGGGCACCCAGACCTACATCT
GCAACGTGAATCACAAGCCCAGCAACACCAAGGTGGACAAGAGAGTTGAGCCCAAATCT
TGTGACAAAACTCACACATGCCCACCCTGCCCAGCACCTGAACTCCTGGGGGGACCCTC
AGTCTTCCTCTTCCCCCCAAAACCCAAGGACACCCTCATGATCTCCCGGACCCCTGAGG
TCACATGCGTGGTGGTGGACGTGAGCCACGAAGACCCTGAGGTCAAGTTCAACTGGTAC
GTGGACGGCGTGGAGGTGCATAATGCCAAGACAAAGCCCCGGGAGGAGCAGTACAACAG
CACGTACCGGGTGGTCAGCGTCCTCACCGTCCTGCACCAGGACTGGCTGAATGGCAAGG
AGTACAAGTGCAAGGTCTCCAACAAAGCCCTCCCAGCCCCCATCGAGAAAACCATCTCC
AAAGCCAAAGGCCAGCCCCGGGAACCACAGGTGTACACCCTGCCCCCATCCCGGGAGGA
GATGACCAAGAACCAGGTCAGCCTGACCTGCCTGGTCAAAGGCTTCTATCCCAGCGACA
TCGCCGTGGAGTGGGAGAGCAATGGCCAGCCCGAGAACAACTACAAGACCACCCCTCCC
GTGCTGGACTCCGACGGCTCCTTCTTCCTCTACAGCAAGCTCACCGTGGACAAGAGCAG
GTGGCAGCAGGGCAACGTCTTCTCATGCTCCGTGATGCATGAGGCTCTGCACAACCACT
ACACCCAGAAGAGCCTCTCCCTGTCTCCCGGCAAA

(SEQ ID NO: 136 of the Sequence Listing)

[Figure 149]

ATGGTGCTGCAGACCCAGGTGTTCATCTCCCTGCTGCTGTGGATCTCCGGCGCGTACGG
CCAGCCTGTGCTGACTCAGCCACCCTCAGCGTCTGGGACCCCCGGGCAGAGGGTCACCA
TCTCTTGTTCTGGAAGCAACTCCAACATCGGCAGTAGAACTGTGAATTGGTACCAGCAC
CTCCCAGGAACGGCCCCCAGACTCCTCATCTATGGTAATAATCAGTGGCCCTCAGGGGT
CCCTGACCGATTCTCTGGCTCTAAGTCTGGCTCCTCAGCCTCCCTGGCCATCAGTGGGC
TCCGGTCCGAGGATGAGGCTGATTATTACTGTGCAGCATGGGATGACAGCCTGAGTGGT
GTGGTATTCGGCGGAGGGACCAAGTTAACTGTGCTTGGCCAGCCTAAGGCTGCCCCTAG
CGTGACCCTGTTCCCTCCTTCCAGCGAGGAGCTTCAAGCTAACAAGGCCACCCTGGTGT
GTCTTATCTCTGACTTCTACCCTGGCGCTGTGACCGTGGCCTGGAAGGCTGACAGCTCC
CCTGTGAAGGCCGGAGTGGAGACCACCACACCTAGCAAGCAGTCTAACAACAAGTACGC
TGCCAGCTCCTACCTGAGCCTTACCCCTGAGCAGTGGAAGTCTCACAGAAGCTACTCCT
GTCAAGTGACCCACGAGGGCAGCACCGTGGAGAAGACCGTGGCTCCTACCGAGTGTTCC

(SEQ ID NO: 137 of the Sequence Listing)

[Figure 150]

ATGAAACACCTGTGGTTCTTCCTCCTGCTGGTGGCAGCTCCCAGATGGGTGCTGAGCCA
GGTGCAGCTGGTGCAGTCTGGGGCTGAGGTGAAGAAGCCTGGGTCCTCGGTGAAGGTCT
CCTGCAAGGCTTCTGGAGGCACCTTCAGCAGGTATGCTATCAGCTGGGTGCGACAGGCC
CCTGGACAAGGGCTTGAGTGGATGGGAGGGATCATCCCTATCTTTGGAACAGCAAACTA
CGCACAGAAGTTCCAGGGCAGAGTCACAATTACCGCGGACCAATCCACGAGAACAGCCT
ACATGGACCTGGGCAGACTGAGATCTGAGGACACGGCCGTGTATTATTGTGCTCTCGAG
GGATTCAGGGACTCATTAAAACGAAATCCTTTTGATATCTGGGGCCAAGGGACAATGGT
CACCGTCAGCTCAGCCTCCACCAAGGGCCCAAGCGTCTTCCCCCTGGCACCCTCCTCCA
AGAGCACCTCTGGCGGCACAGCCGCCCTGGGCTGCCTGGTCAAGGACTACTTCCCCGAA
CCCGTGACCGTGAGCTGGAACTCAGGCGCCCTGACCAGCGGCGTGCACACCTTCCCCGC
TGTCCTGCAGTCCTCAGGACTCTACTCCCTCAGCAGCGTGGTGACCGTGCCCTCCAGCA
GCTTGGGCACCCAGACCTACATCTGCAACGTGAATCACAAGCCCAGCAACACCAAGGTG
GACAAGAGAGTTGAGCCCAAATCTTGTGACAAAACTCACACATGCCCACCCTGCCCAGC
ACCTGAACTCCTGGGGGGACCCTCAGTCTTCCTCTTCCCCCCAAAACCCAAGGACACCC
TCATGATCTCCCGGACCCCTGAGGTCACATGCGTGGTGGTGGACGTGAGCCACGAAGAC
CCTGAGGTCAAGTTCAACTGGTACGTGGACGGCGTGGAGGTGCATAATGCCAAGACAAA
GCCCCGGGAGGAGCAGTACAACAGCACGTACCGGGTGGTCAGCGTCCTCACCGTCCTGC
ACCAGGACTGGCTGAATGGCAAGGAGTACAAGTGCAAGGTCTCCAACAAAGCCCTCCCA
GCCCCCATCGAGAAAACCATCTCCAAAGCCAAAGGCCAGCCCCGGGAACCACAGGTGTA
CACCCTGCCCCCATCCCGGGAGGAGATGACCAAGAACCAGGTCAGCCTGACCTGCCTGG
TCAAAGGCTTCTATCCCAGCGACATCGCCGTGGAGTGGGAGAGCAATGGCCAGCCCGAG
AACAACTACAAGACCACCCCTCCCGTGCTGGACTCCGACGGCTCCTTCTTCCTCTACAG
CAAGCTCACCGTGGACAAGAGCAGGTGGCAGCAGGGCAACGTCTTCTCATGCTCCGTGA
TGCATGAGGCTCTGCACAACCACTACACCCAGAAGAGCCTCTCCCTGTCTCCCGGCAAA

(SEQ ID NO: 138 of the Sequence Listing)

[Figure 151]

ATGGTGCTGCAGACCCAGGTGTTCATCTCCCTGCTGCTGTGGATCTCCGGCGCGTACGG
CCAGTCTGTCGTGACGCAGCCGCCCTCAGTGTCTGCGGCCCCAGGACAGAAGGTCACCA
TCTCCTGCTCTGGAGGCACCACCAACATTGGGAGTAATTATGTATCCTGGTATCAGCAG
CTCCCAGGAACAGCCCCCAAATTCCTCATGTATGAAAATAATAAGCGACCCTCAGGCAT
TCCTGACCGATTCTCTGGCTCCAAGTCTGGCACGTCAGCCACCCTGGCCATCACCGGAC
TCCAGACTGGGGACGAGGCCACTTATTACTGCTCAACATGGGATAGCAGCCTGAATACT
GGGCTATTCGGCGGAGGGACCAAGTTAACTGTGCTTGGCCAGCCTAAGGCTGCCCCTAG
CGTGACCCTGTTCCCTCCTTCCAGCGAGGAGCTTCAAGCTAACAAGGCCACCCTGGTGT
GTCTTATCTCTGACTTCTACCCTGGCGCTGTGACCGTGGCCTGGAAGGCTGACAGCTCC
CCTGTGAAGGCCGGAGTGGAGACCACCACACCTAGCAAGCAGTCTAACAACAAGTACGC
TGCCAGCTCCTACCTGAGCCTTACCCCTGAGCAGTGGAAGTCTCACAGAAGCTACTCCT
GTCAAGTGACCCACGAGGGCAGCACCGTGGAGAAGACCGTGGCTCCTACCGAGTGTTCC

(SEQ ID NO: 139 of the Sequence Listing)

[Figure 152]

ATGAAACACCTGTGGTTCTTCCTCCTGCTGGTGGCAGCTCCCAGATGGGTGCTGAGCGA
GGTGCAGCTGGTGGAGTCCGGGGGAGGCGTGGTCCAGCCTGGGAGGTCCCTGAGACTCT
CCTGTGCAGCCTCTGGATTCACCTTTAGGAACTATGCCATGAGCTGGGTCCGCCAGGCT
CCAGGGAAGGGGCTGGAGTGGGTCTCAGGTATTAGTGGTACTGGTGGTAGCACATACTA
CGCAGACTCCGTGAAGGGCCGGTTCACCATCTCCAGAGACAATTCCAAGGACACACTAT
ATCTGCAATTGAACAGCCTGAGAGCCGAGGACACGGCCGTATATTACTGTGCGAAATCC
CGTAGTATGAGTATCAACTACGGTATGGACGTCTGGGGCCAAGGGACCACGGTCACCGT
CAGCTCAGCCTCCACCAAGGGCCCAAGCGTCTTCCCCCTGGCACCCTCCTCCAAGAGCA
CCTCTGGCGGCACAGCCGCCCTGGGCTGCCTGGTCAAGGACTACTTCCCCGAACCCGTG
ACCGTGAGCTGGAACTCAGGCGCCCTGACCAGCGGCGTGCACACCTTCCCCGCTGTCCT
GCAGTCCTCAGGACTCTACTCCCTCAGCAGCGTGGTGACCGTGCCCTCCAGCAGCTTGG
GCACCCAGACCTACATCTGCAACGTGAATCACAAGCCCAGCAACACCAAGGTGGACAAG
AGAGTTGAGCCCAAATCTTGTGACAAAACTCACACATGCCCACCCTGCCCAGCACCTGA
ACTCCTGGGGGGACCCTCAGTCTTCCTCTTCCCCCCAAAACCCAAGGACACCCTCATGA
TCTCCCGGACCCCTGAGGTCACATGCGTGGTGGTGGACGTGAGCCACGAAGACCCTGAG
GTCAAGTTCAACTGGTACGTGGACGGCGTGGAGGTGCATAATGCCAAGACAAAGCCCCG
GGAGGAGCAGTACAACAGCACGTACCGGGTGGTCAGCGTCCTCACCGTCCTGCACCAGG
ACTGGCTGAATGGCAAGGAGTACAAGTGCAAGGTCTCCAACAAAGCCCTCCCAGCCCCC
ATCGAGAAAACCATCTCCAAAGCCAAAGGCCAGCCCCGGGAACCACAGGTGTACACCCT
GCCCCCATCCCGGGAGGAGATGACCAAGAACCAGGTCAGCCTGACCTGCCTGGTCAAAG
GCTTCTATCCCAGCGACATCGCCGTGGAGTGGGAGAGCAATGGCCAGCCCGAGAACAAC
TACAAGACCACCCCTCCCGTGCTGGACTCCGACGGCTCCTTCTTCCTCTACAGCAAGCT
CACCGTGGACAAGAGCAGGTGGCAGCAGGGCAACGTCTTCTCATGCTCCGTGATGCATG
AGGCTCTGCACAACCACTACACCCAGAAGAGCCTCTCCCTGTCTCCCGGCAAA

(SEQ ID NO: 140 of the Sequence Listing)

[Figure 153]

ATGGTGCTGCAGACCCAGGTGTTCATCTCCCTGCTGCTGTGGATCTCCGGCGCGTACGG
CTCCTATGAGCTGACTCAGCCACCCTCAGTGTCCGTGTCCCCAGGACAGACAGTCAGCA
TCACCTGCTCTGGAGATAATTTGGGTGATAGATATGCCTCCTGGTATCAGCAGAAGCCA
GGCCAGTCCCCTGTGCAGGTCATCTATCAAGATACCAAGCGGCCCTCAGGGATCCCTGA
GCGATTCTCTGGCTCCAACTCTGGGAACACAGCCACTCTGACCATCAGCGGGACCCAGG
CTATGGATGAGGCTGACTATTACTGTCAGGCGTGGGACAGCAACACTGTGGTATTCGGC
GGAGGGACCAAGTTAACTGTGCTTGGCCAGCCTAAGGCTGCCCCTAGCGTGACCCTGTT
CCCTCCTTCCAGCGAGGAGCTTCAAGCTAACAAGGCCACCCTGGTGTGTCTTATCTCTG
ACTTCTACCCTGGCGCTGTGACCGTGGCCTGGAAGGCTGACAGCTCCCCTGTGAAGGCC
GGAGTGGAGACCACCACACCTAGCAAGCAGTCTAACAACAAGTACGCTGCCAGCTCCTA
CCTGAGCCTTACCCCTGAGCAGTGGAAGTCTCACAGAAGCTACTCCTGTCAAGTGACCC
ACGAGGGCAGCACCGTGGAGAAGACCGTGGCTCCTACCGAGTGTTCC

(SEQ ID NO: 141 of the Sequence Listing)

[Figure 154]

ATGAAACACCTGTGGTTCTTCCTCCTGCTGGTGGCAGCTCCCAGATGGGTGCTGAGCCA
GGTGCAGCTGGTGCAGTCTGGGGCTGAGGTGAAGAAGCCTGGGGCCTCAGTGAAGGTCT
CCTGCAAGGCTTCTGGATACACCTTCACCGGCTACTATATGCACTGGGTGCGACAGGCC
CCTGGACAAGGGCTTGAGTGGATGGGACGGATCAACCCTAACAGTGGTGGCACAAACTA
TGCACAGAAGTTTCAGGGCAGGGTCACCATGACCAGGGACACGTCCATCAGCACAGCCT
ACATGGAGCTGAGCGGGCTGAGATCTGACGACACGGCCGTTTATTACTGTGCGAGTGGT
AGTGTAAGGCATCCCTGGGGCCAGGGAACCCTGGTCACCGTCAGCTCAGCCTCCACCAA
GGGCCCAAGCGTCTTCCCCCTGGCACCCTCCTCCAAGAGCACCTCTGGCGGCACAGCCG
CCCTGGGCTGCCTGGTCAAGGACTACTTCCCCGAACCCGTGACCGTGAGCTGGAACTCA
GGCGCCCTGACCAGCGGCGTGCACACCTTCCCCGCTGTCCTGCAGTCCTCAGGACTCTA
CTCCCTCAGCAGCGTGGTGACCGTGCCCTCCAGCAGCTTGGGCACCCAGACCTACATCT
GCAACGTGAATCACAAGCCCAGCAACACCAAGGTGGACAAGAGAGTTGAGCCCAAATCT
TGTGACAAAACTCACACATGCCCACCCTGCCCAGCACCTGAACTCCTGGGGGGACCCTC
AGTCTTCCTCTTCCCCCCAAAACCCAAGGACACCCTCATGATCTCCCGGACCCCTGAGG
TCACATGCGTGGTGGTGGACGTGAGCCACGAAGACCCTGAGGTCAAGTTCAACTGGTAC
GTGGACGGCGTGGAGGTGCATAATGCCAAGACAAAGCCCCGGGAGGAGCAGTACAACAG
CACGTACCGGGTGGTCAGCGTCCTCACCGTCCTGCACCAGGACTGGCTGAATGGCAAGG
AGTACAAGTGCAAGGTCTCCAACAAAGCCCTCCCAGCCCCCATCGAGAAAACCATCTCC
AAAGCCAAAGGCCAGCCCCGGGAACCACAGGTGTACACCCTGCCCCCATCCCGGGAGGA
GATGACCAAGAACCAGGTCAGCCTGACCTGCCTGGTCAAAGGCTTCTATCCCAGCGACA
TCGCCGTGGAGTGGGAGAGCAATGGCCAGCCCGAGAACAACTACAAGACCACCCCTCCC
GTGCTGGACTCCGACGGCTCCTTCTTCCTCTACAGCAAGCTCACCGTGGACAAGAGCAG
GTGGCAGCAGGGCAACGTCTTCTCATGCTCCGTGATGCATGAGGCTCTGCACAACCACT
ACACCCAGAAGAGCCTCTCCCTGTCTCCCGGCAAA

(SEQ ID NO: 142 of the Sequence Listing)

[Figure 155]

ATGGTGCTGCAGACCCAGGTGTTCATCTCCCTGCTGCTGTGGATCTCCGGCGCGTACGG
CCAGCCTGTGCTGACTCAGCCACCCTCAGCGTCTGGGACCCCCGGGCAGAGGGTCACCA
TCTCTTGTTCTGGAAGCAGGTCCAACATCGGAGGTAATATTGTAAGTTGGTACCAGCAG
TTCCCAGGAACGGCCCCCAGACTCCTCACTTATGCTGATAATCAGCGGCCCTCAGGGGT
CCCTGACCGATTCTCTGGCTCCAAGTCTGGCACCTCAGCCTCCCTGGCCATCAGTGGCC
TCCAGTCTGAGGATGAGGCTGACTATTATTGTGCAGCATGGGATGACAGCCTGAATGGT
GTTGTGTTCGGAGGAGGCACCAAGTTAACTGTGCTTGGCCAGCCTAAGGCTGCCCCTAG
CGTGACCCTGTTCCCTCCTTCCAGCGAGGAGCTTCAAGCTAACAAGGCCACCCTGGTGT
GTCTTATCTCTGACTTCTACCCTGGCGCTGTGACCGTGGCCTGGAAGGCTGACAGCTCC
CCTGTGAAGGCCGGAGTGGAGACCACCACACCTAGCAAGCAGTCTAACAACAAGTACGC
TGCCAGCTCCTACCTGAGCCTTACCCCTGAGCAGTGGAAGTCTCACAGAAGCTACTCCT
GTCAAGTGACCCACGAGGGCAGCACCGTGGAGAAGACCGTGGCTCCTACCGAGTGTTCC

(SEQ ID NO: 143 of the Sequence Listing)

[Figure 156]

MKHLWFFLLLVAAPRWVLSQVQLVQSGAEVKKPGASVKVSCKASGYTFTGYYMHWVRQA
PGQGLEWMGRINPNSGGTNYAQKFQGRVTMTRDTSISTAYMELSRLRSDDTAVYYCASG
KSNYPWGQGTLVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKRVEPKS
CDKTHTCPPCPAPELLGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIEKTIS
KAKGQPREPQVYTLPPSREEMTKNQVSLTCLVKGFYPSDIAVEWESNGQPENNYKTTPP
VLDSDGSFFLYSKLTVDKSRWQQGNVFSCSVMHEALHNHYTQKSLSLSPGK

(SEQ ID NO: 144 of the Sequence Listing)

[Figure 157]

MVLQTQVFISLLLWISGAYGQPVLTQPPSASGTPGQRVTISCSGSNSNIGSRTVNWYQH
LPGTAPRLLIYGNNQWPSGVPDRFSGSKSGSSASLAISGLRSEDEADYYCAAWDDSLSG
VVFGGGTKLTVLGQPKAAPSVTLFPPSSEELQANKATLVCLISDFYPGAVTVAWKADSS
PVKAGVETTTPSKQSNNKYAASSYLSLTPEQWKSHRSYSCQVTHEGSTVEKTVAPTECS

(SEQ ID NO: 145 of the Sequence Listing)

[Figure 158]

MKHLWFFLLLVAAPRWVLSQVQLVQSGAEVKKPGSSVKVSCKASGGTFSRYAISWVRQA
PGQGLEWMGGIIPIFGTANYAQKFQGRVTITADQSTRTAYMDLGRLRSEDTAVYYCALE
GFRDSLKRNPFDIWGQGTMVTVSSASTKGPSVFPLAPSSKSTSGGTAALGCLVKDYFPE
PVTVSWNSGALTSGVHTFPAVLQSSGLYSLSSVVTVPSSSLGTQTYICNVNHKPSNTKV
DKRVEPKSCDKTHTCPPCPAPELLGGPSVFLFPPKPKDTLMISRTPEVTCVVVDVSHED
PEVKFNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSLSLSPGK

(SEQ ID NO: 146 of the Sequence Listing)

[Figure 159]

MVLQTQVFISLLLWISGAYGQSVVTQPPSVSAAPGQKVTISCSGGTTNIGSNYVSWYQQ
LPGTAPKFLMYENNKRPSGIPDRFSGSKSGTSATLAITGLQTGDEATYYCSTWDSSLNT
GLFGGGTKLTVLGQPKAAPSVTLFPPSSEELQANKATLVCLISDFYPGAVTVAWKADSS
PVKAGVETTTPSKQSNNKYAASSYLSLTPEQWKSHRSYSCQVTHEGSTVEKTVAPTECS

(SEQ ID NO: 147 of the Sequence Listing)

[Figure 160]

MKHLWFFLLLVAAPRWVLSEVQLVESGGGVVQPGRSLRLSCAASGFTFRNYAMSWVRQA
PGKGLEWVSGISGTGGSTYYADSVKGRFTISRDNSKDTLYLQLNSLRAEDTAVYYCAKS
RSMSINYGMDVWGQGTTVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNTKVDK
RVEPKSCDKTHTCPPCPAPELLGGPSVFLFPPKPKDTLMISRTPEVTCVVVDVSHEDPE
VKFNWYVDGVEVHNAKTKPREEQYNSTYRVVSVLTVLHQDWLNGKEYKCKVSNKALPAP
IEKTISKAKGQPREPQVYTLPPSREEMTKNQVSLTCLVKGFYPSDIAVEWESNGQPENN
YKTTPPVLDSDGSFFLYSKLTVDKSRWQQGNVFSCSVMHEALHNHYTQKSLSLSPGK

(SEQ ID NO: 148 of the Sequence Listing)

[Figure 161]

MVLQTQVFISLLLWISGAYGSYELTQPPSVSVSPGQTVSITCSGDNLGDRYASWYQQKP
GQSPVQVIYQDTKRPSGIPERFSGSNSGNTATLTISGTQAMDEADYYCQAWDSNTVVFG
GGTKLTVLGQPKAAPSVTLFPPSSEELQANKATLVCLISDFYPGAVTVAWKADSSPVKA
GVETTTPSKQSNNKYAASSYLSLTPEQWKSHRSYSCQVTHEGSTVEKTVAPTECS

(SEQ ID NO: 149 of the Sequence Listing)

[Figure 162]

MKHLWFFLLLVAAPRWVLSQVQLVQSGAEVKKPGASVKVSCKASGYTFTGYYMHWVRQA
PGQGLEWMGRINPNSGGTNYAQKFQGRVTMTRDTSISTAYMELSGLRSDDTAVYYCASG
SVRHPWGQGTLVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKRVEPKS
CDKTHTCPPCPAPELLGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIEKTIS
KAKGQPREPQVYTLPPSREEMTKNQVSLTCLVKGFYPSDIAVEWESNGQPENNYKTTPP
VLDSDGSFFLYSKLTVDKSRWQQGNVFSCSVMHEALHNHYTQKSLSLSPGK

(SEQ ID NO: 150 of the Sequence Listing)

[Figure 163]

MVLQTQVFISLLLWISGAYGQPVLTQPPSASGTPGQRVTISCSGSRSNIGGNIVSWYQQ
FPGTAPRLLTYADNQRPSGVPDRFSGSKSGTSASLAISGLQSEDEADYYCAAWDDSLNG
VVFGGGTKLTVLGQPKAAPSVTLFPPSSEELQANKATLVCLISDFYPGAVTVAWKADSS
PVKAGVETTTPSKQSNNKYAASSYLSLTPEQWKSHRSYSCQVTHEGSTVEKTVAPTECS

(SEQ ID NO: 151 of the Sequence Listing)

[Figure 164]

A

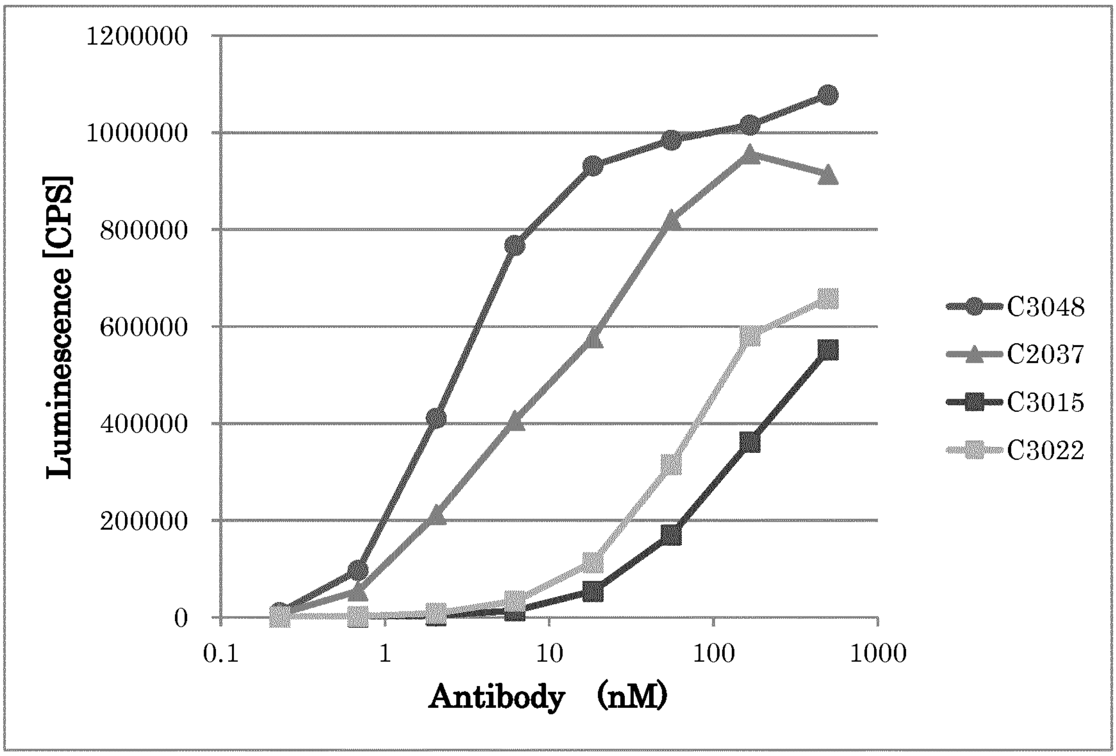


B

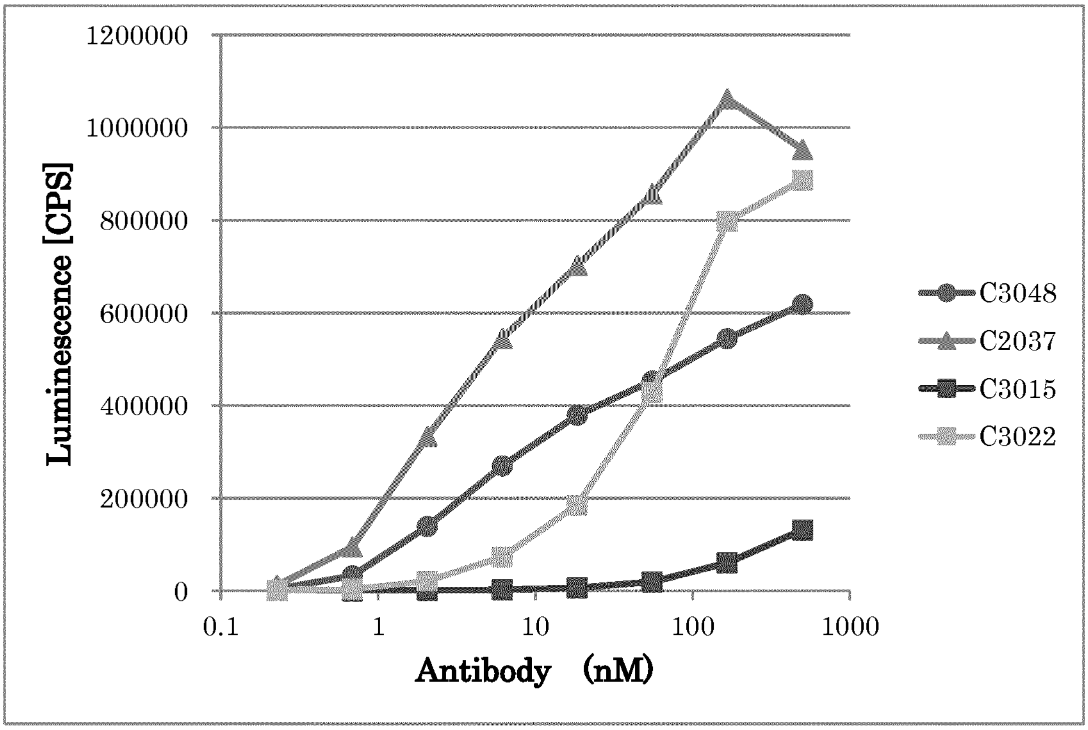

[Figure 165]

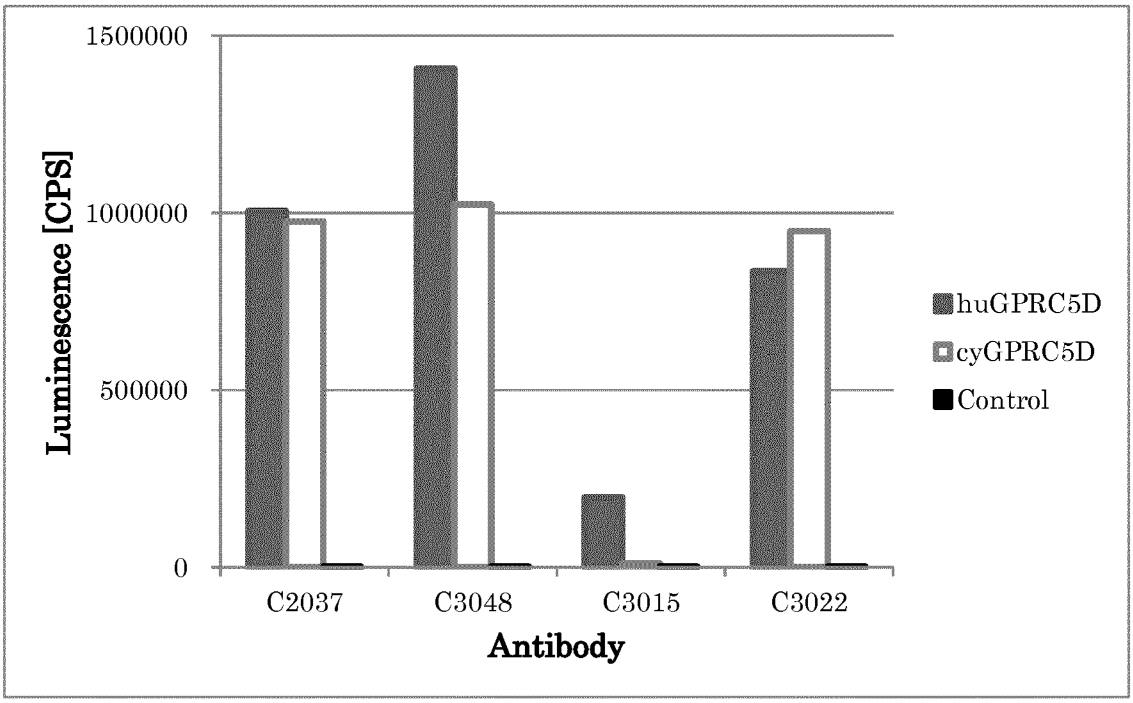


[Figure 166]

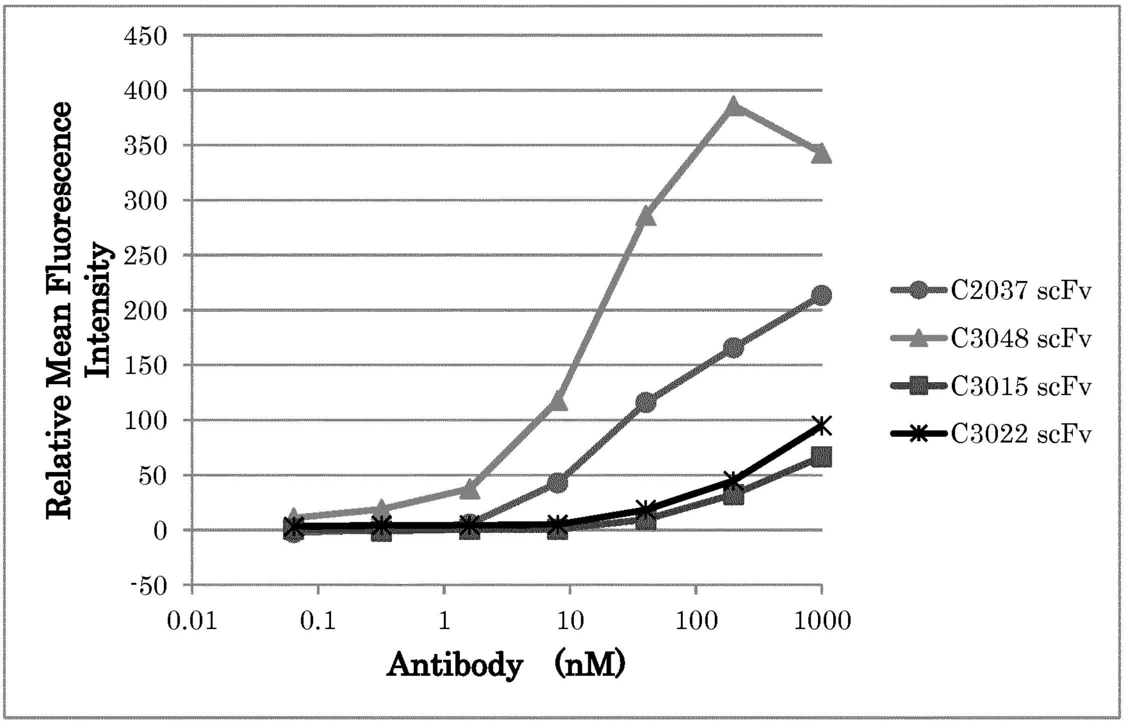

[Figure 167]

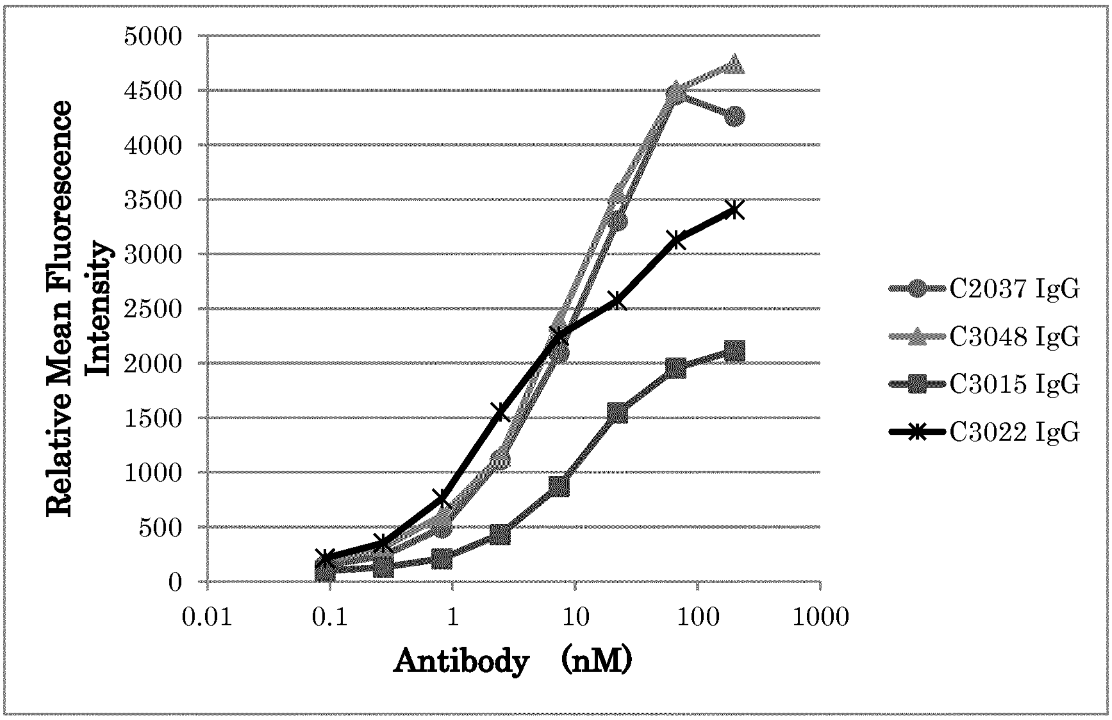


[Figure 168]

GAGGTGCAGTTGGTGGAGTCTGGGGGAGGCCTGGTGCAGCCTGGAAGGGCCCTGAAACT
CTCCTGTGTAGTCTCTGGAGTCACATTCAATTACTACGGGATGAGCTGGATCCGCCAGG
CTCCAGGGAAGGGGCTGGAGTGGGTTGCATCCATTACTAATTCTGGTGGTAGAATTTAC
TATCCAGACTCTGTGAAGGGCCGATTCACTATCTCCAGAGAAAATACACAAAAGACCCT
ATACCTACAAATGAACAGTCTGAGGTCTGAGGACACGGCCACTTATTACTGTACTCTCG
ATGGTCGCGATGGTTGGGTTGCTTACTGGGGCCAAGGCACTCTGGTCACTGTCTCTTCA

(SEQ ID NO: 152 of the Sequence Listing)

[Figure 169]

CAGTTTGTGCTTACTCAGCCAAACTCTGTGTCTACGAATCTCGGAACCACAGTCGAACT
GTCTTGCAAGCGCAACACTGGGAACATTGGAAGCAATTATGTGAACTGGTACCAGCAGC
ATGAGGGAAGATCTCCCACCACTATTATTTATAGGGATGATAAGAGACCAGATGGAGTT
TCTGACAGGTTCTCTGGGTCCATTGACAGATCTTCCAAGTCAGCCCTCCTGACAATCAA
TAATGTGCAGACTGAAGATGAAGCTGACTACTTCTGTCAGTCTTACAGTAGTGGTTTTA
TTTTCGGCGGTGGAACCAAGCTCACTGTCCTA

(SEQ ID NO: 153 of the Sequence Listing)

[Figure 170]

ATGAAACACCTGTGGTTCTTCCTCCTGCTGGTGGCAGCTCCCAGATGGGTGCTGAGCGA
GGTGCAGTTGGTGGAGTCTGGGGGAGGCCTGGTGCAGCCTGGAAGGGCCCTGAAACTCT
CCTGTGTAGTCTCTGGAGTCACATTCAATTACTACGGGATGAGCTGGATCCGCCAGGCT
CCAGGGAAGGGGCTGGAGTGGGTTGCATCCATTACTAATTCTGGTGGTAGAATTTACTA
TCCAGACTCTGTGAAGGGCCGATTCACTATCTCCAGAGAAAATACACAAAAGACCCTAT
ACCTACAAATGAACAGTCTGAGGTCTGAGGACACGGCCACTTATTACTGTACTCTCGAT
GGTCGCGATGGTTGGGTTGCTTACTGGGGCCAAGGCACTCTGGTCACTGTCTCTTCAGG
TGGAGGCGGTTCAGGCGGAGGTGGCAGCGGCGGTGGCGGGAGTCAGTTTGTGCTTACTC
AGCCAAACTCTGTGTCTACGAATCTCGGAACCACAGTCGAACTGTCTTGCAAGCGCAAC
ACTGGGAACATTGGAAGCAATTATGTGAACTGGTACCAGCAGCATGAGGGAAGATCTCC
CACCACTATTATTTATAGGGATGATAAGAGACCAGATGGAGTTTCTGACAGGTTCTCTG
GGTCCATTGACAGATCTTCCAAGTCAGCCCTCCTGACAATCAATAATGTGCAGACTGAA
GATGAAGCTGACTACTTCTGTCAGTCTTACAGTAGTGGTTTTATTTTCGGCGGTGGAAC
CAAGCTCACTGTCCTAGGCGCGTCTGCGGCCGCAGGATCCGGTGGTGATTACAAAGATG
ATGACGATAAAGGTGCAGCGGCGCATCACCATCATCACCAC

(SEQ ID NO: 154 of the Sequence Listing)

[Figure 171]

EVQLVESGGGLVQPGGSLRLSCAASGVTFNYYGMSWIRQAPGKGLEWVASITNSGGRIY
YPDSVKGRFTISRENTQKTLYLQMNSLRAEDTAVYYCTLDGRDGWVAYWGQGTLVTVSS

(SEQ ID NO: 155 of the Sequence Listing)

[Figure 172]

NFMLTQPHSVSESPGKTVTISCKRNTGNIGSNYVNWYQQHEGSSPTTIIYRDDKRPDGV
SDRFSGSIDRSSKSASLTISNLKTEDEADYFCQSYSSGFIFGGGTKLTVL

(SEQ ID NO: 156 of the Sequence Listing)

[Figure 173]

ATGAAACACCTGTGGTTCTTCCTCCTGCTGGTGGCAGCTCCCAGATGGGTGCTGAGCGG
AGAAGTGCAGCTGGTGGAATCCGGGGGGGGCCTGGTGCAGCCTGGGGGGAGCCTGAGAC
TGAGTTGTGCCGCCTCTGGGGTGACATTTAACTACTATGGCATGTCTTGGATCCGCCAG
GCACCTGGAAAGGGCCTGGAGTGGGTGGCCAGCATCACTAATTCCGGCGGGCGAATCTA
CTATCCCGACAGCGTCAAGGGCAGGTTCACAATTTCCCGCGAGAACACACAGAAAACTC
TGTACCTGCAGATGAATAGCCTGAGAGCCGAAGATACAGCTGTGTACTATTGCACTCTG
GACGGCAGGGATGGGTGGGTCGCCTATTGGGGGCAGGGAACCCTGGTGACAGTCAGCTC
CGGAGGAGGAGGATCTGGCGGAGGAGGCAGTGGGGGAGGCGGGTCAAACTTTATGCTGA
CCCAGCCCCACAGTGTGTCAGAGAGCCCTGGCAAGACTGTCACCATCTCTTGTAAAAGG
AACACCGGAAATATTGGCAGTAACTACGTGAATTGGTATCAGCAGCATGAAGGGTCTAG
TCCAACCACAATCATCTACCGGGACGATAAGAGACCCGACGGGGTGTCCGATCGATTCT
CCGGATCTATCGACCGGTCAAGCAAGAGTGCTTCACTGACCATTAGCAATCTGAAAACA
GAGGACGAAGCAGATTACTTTTGCCAGTCCTATTCCTCTGGCTTCATCTTTGGAGGCGG
GACTAAACTGACCGTGCTGGGCGCGGCCGCAGGTGCAGGTGGTGATTACAAAGATGATG
ACGATAAAGGTGCAGCGGCGCATCACCATCATCACCAC

(SEQ ID NO: 157 of the Sequence Listing)

[Figure 174]

QAVLTQPSSVSGVPGQRVTISCKRNTGNIGSNYVNWYQQLPGTAPKLLIYRDDKRPSGV
PDRFSGSKSGTSASLAITGFQAEDEADYYCQSYSSGFIFGGGTKLTVL

(SEQ ID NO: 158 of the Sequence Listing)

[Figure 175]

ATGAAACACCTGTGGTTCTTCCTCCTGCTGGTGGCAGCTCCCAGATGGGTGCTGAGCGG
AGAAGTGCAGCTGGTGGAATCCGGGGGGGGCCTGGTGCAGCCTGGGGGGAGCCTGAGAC
TGAGTTGTGCCGCCTCTGGGGTGACATTTAACTACTATGGCATGTCTTGGATCCGCCAG
GCACCTGGAAAGGGCCTGGAGTGGGTGGCCAGCATCACTAATTCCGGCGGGCGAATCTA
CTATCCCGACAGCGTCAAGGGCAGGTTCACAATTTCCCGCGAGAACACACAGAAAACTC
TGTACCTGCAGATGAATAGCCTGAGAGCCGAAGATACAGCTGTGTACTATTGCACTCTG
GACGGCAGGGATGGGTGGGTCGCCTATTGGGGGCAGGGAACCCTGGTGACAGTCAGCTC
CGGAGGAGGAGGATCTGGCGGAGGAGGCAGTGGGGGAGGCGGGTCAATGGCCCAGGCTG
TGCTCACTCAGCCGTCCTCTGTTTCTGGCGTACCTGGCCAACGGGTGACCATTAGCTGT
AAAAGGAATACCGGGAATATCGGGTCTAACTACGTGAACTGGTATCAGCAGCTTCCAGG
GACAGCTCCCAAGTTGCTGATCTATCGCGACGACAAAAGACCCTCAGGGGTCCCTGACC
GATTTAGTGGCAGCAAAAGCGGTACTTCCGCTTCCCTGGCGATAACCGGCTTTCAGGCC
GAAGATGAGGCAGACTACTATTGCCAGTCATATTCCAGCGGCTTCATCTTCGGAGGCGG
AACTAAGCTGACAGTGCTGGGCGCGGCCGCAGGTGCAGGTGGTGATTACAAAGATGATG
ACGATAAAGGTGCAGCGGCGCATCACCATCATCACCAC

(SEQ ID NO: 159 of the Sequence Listing)

[Figure 176]

NFMLTQPSSVSGVPGQRVTISCTGNTGNIGSNYVNWYQQLPGTAPKLLIYRDDKRPSGV
PDRFSGSKSGTSASLAITGFQAEDEADYYCQSYSSGFIFGGGTKLTVL

(SEQ ID NO: 160 of the Sequence Listing)

[Figure 177]

ATGAAACACCTGTGGTTCTTCCTCCTGCTGGTGGCAGCTCCCAGATGGGTGCTGAGCGG
AGAAGTGCAGCTGGTGGAATCCGGGGGGGGCCTGGTGCAGCCTGGGGGGAGCCTGAGAC
TGAGTTGTGCCGCCTCTGGGGTGACATTTAACTACTATGGCATGTCTTGGATCCGCCAG
GCACCTGGAAAGGGCCTGGAGTGGGTGGCCAGCATCACTAATTCCGGCGGGCGAATCTA
CTATCCCGACAGCGTCAAGGGCAGGTTCACAATTTCCCGCGAGAACACACAGAAAACTC
TGTACCTGCAGATGAATAGCCTGAGAGCCGAAGATACAGCTGTGTACTATTGCACTCTG
GACGGCAGGGATGGGTGGGTCGCCTATTGGGGGCAGGGAACCCTGGTGACAGTCAGCTC
CGGAGGAGGAGGATCTGGCGGAGGAGGCAGTGGGGGAGGCGGGTCAAACTTTATGCTCA
CTCAGCCGTCCTCTGTTTCTGGCGTACCTGGCCAACGGGTGACCATTAGCTGTACGGGT
AATACCGGGAATATCGGGTCTAACTACGTGAACTGGTATCAGCAGCTTCCAGGGACAGC
TCCCAAGTTGCTGATCTATCGCGACGACAAAAGACCCTCAGGGGTCCCTGACCGATTTA
GTGGCAGCAAAAGCGGTACTTCCGCTTCCCTGGCGATAACCGGCTTTCAGGCCGAAGAT
GAGGCAGACTACTATTGCCAGTCATATTCCAGCGGCTTCATCTTCGGAGGCGGAACTAA
GCTGACAGTGTTGGGTGCGGCCGCAGGTGCAGGTGGTGATTACAAAGATGATGACGATA
AAGGTGCAGCGGCGCATCACCATCATCACCAC

(SEQ ID NO: 161 of the Sequence Listing)

[Figure 178]

ATGAAACACCTGTGGTTCTTCCTCCTGCTGGTGGCAGCTCCCAGATGGGTGCTGAGCGG
ACAGGTGCAGCTGGTGCAGTCTGGCGCCGAAGTGAAGAAACCAGGCGCCAGCGTGAAGG
TGTCCTGCAAGGCCAGCGGCTACACCTTTACCGGCTACTACATGCACTGGGTGCGCCAG
GCTCCAGGCCAGGGACTGGAATGGATGGGCCGGATCAACCCCAATAGCGGCGGCACCAA
CTACGCCCAGAAATTCCAGGGCAGAGTGACCATGACCCGGGACACCAGCATCAGCACCG
CCTACATGGAACTGAGCCGGCTGAGAAGCGACGACACCGCCGTGTACTACTGCGCCAGC
GGCAAGAGCAACTACCCTTGGGGCCAGGGCACACTCGTGACCGTGTCTAGCGGAGGCGG
AGGATCTGGCGGCGGAGGAAGTGGCGGAGGGGGATCTCAGCCTGTGCTGACACAGCCTC
CTAGCGCCTCTGGCACACCTGGCCAGAGAGTGACAATCAGCTGCAGCGGCAGCAACAGC
AACATCGGCAGCCGGACCGTGAACTGGTATCAGCATCTGCCTGGCACCGCCCCCAGACT
GCTGATCTACGGCAACAACCAGTGGCCCAGCGGCGTGCCCGATAGATTCAGCGGCTCTA
AGAGCGGCAGCTCCGCCAGCCTGGCCATCTCTGGACTGAGATCCGAGGACGAGGCCGAC
TACTACTGTGCCGCCTGGGACGATAGCCTGAGCGGCGTGGTGTTTGGCGGAGGCACCAA
GCTGACAGTGCTGGGCGGAGGCGGTTCAGAAGTGCAGCTGGTGGAATCCGGGGGGGGCC
TGGTGCAGCCTGGGGGGAGCCTGAGACTGAGTTGTGCCGCCTCTGGGGTGACATTTAAC
TACTATGGCATGTCTTGGATCCGCCAGGCACCTGGAAAGGGCCTGGAGTGGGTGGCCAG
CATCACTAATTCCGGCGGGCGAATCTACTATCCCGACAGCGTCAAGGGCAGGTTCACAA
TTTCCCGCGAGAACACACAGAAAACTCTGTACCTGCAGATGAATAGCCTGAGAGCCGAA
GATACAGCTGTGTACTATTGCACTCTGGACGGCAGGGATGGGTGGGTCGCCTATTGGGG
GCAGGGAACCCTGGTGACAGTCAGCTCCGGAGGAGGAGGATCTGGCGGAGGAGGCAGTG
GGGGAGGCGGGTCAAACTTTATGCTGACCCAGCCCCACAGTGTGTCAGAGAGCCCTGGC
AAGACTGTCACCATCTCTTGTAAAAGGAACACCGGAAATATTGGCAGTAACTACGTGAA
TTGGTATCAGCAGCATGAAGGGTCTAGTCCAACCACAATCATCTACCGGGACGATAAGA
GACCCGACGGGGTGTCCGATCGATTCTCCGGATCTATCGACCGGTCAAGCAAGAGTGCT
TCACTGACCATTAGCAATCTGAAAACAGAGGACGAAGCAGATTACTTTTGCCAGTCCTA
TTCCTCTGGCTTCATCTTTGGAGGCGGGACTAAACTGACCGTGCTGGGCGCGGCCGCAG
GTGCAGGTGGTGATTACAAAGATGATGACGATAAAGGTGCAGCGGCGCATCACCATCAT
CACCAC

(SEQ ID NO: 162 of the Sequence Listing)

[Figure 179]

ATGAAACACCTGTGGTTCTTCCTCCTGCTGGTGGCAGCTCCCAGATGGGTGCTGAGCGG
ACAGGTGCAGCTGGTGCAGTCTGGCGCCGAAGTGAAGAAACCCGGCAGCAGCGTGAAGG
TGTCCTGCAAGGCTAGCGGCGGCACCTTCAGCAGATACGCCATCTCTTGGGTGCGCCAG
GCCCCTGGACAGGGCCTGGAATGGATGGGCGGCATCATCCCCATCTTCGGCACCGCCAA
CTACGCCCAGAAATTCCAGGGCAGAGTGACCATCACCGCCGACCAGAGCACCCGGACCG
CCTATATGGATCTGGGCCGGCTGAGAAGCGAGGACACCGCCGTGTACTACTGCGCCCTG
GAAGGCTTCCGGGACAGCCTGAAGAGAAACCCCTTCGACATCTGGGGCCAGGGCACAAT
GGTCACCGTGTCTAGCGGAGGCGGAGGATCTGGCGGCGGAGGAAGTGGCGGAGGGGGAT
CTCAGTCTGTCGTGACCCAGCCTCCTAGCGTGTCAGCCGCCCCAGGCCAGAAAGTGACA
ATCAGCTGTTCTGGCGGCACCACCAACATCGGCAGCAACTACGTGTCCTGGTATCAGCA
GCTGCCCGGAACCGCCCCCAAGTTCCTGATGTACGAGAACAACAAGCGGCCCAGCGGCA
TCCCCGACAGATTCAGCGGCAGCAAGAGCGGCACAAGCGCCACACTGGCCATCACCGGA
CTGCAGACAGGCGACGAGGCCACCTACTACTGCTCCACCTGGGACAGCAGCCTGAACAC
CGGCCTGTTTGGCGGAGGCACCAAGCTGACAGTGCTGGGCGGAGGCGGTTCAGAAGTGC
AGCTGGTGGAATCCGGGGGGGGCCTGGTGCAGCCTGGGGGGAGCCTGAGACTGAGTTGT
GCCGCCTCTGGGGTGACATTTAACTACTATGGCATGTCTTGGATCCGCCAGGCACCTGG
AAAGGGCCTGGAGTGGGTGGCCAGCATCACTAATTCCGGCGGGCGAATCTACTATCCCG
ACAGCGTCAAGGGCAGGTTCACAATTTCCCGCGAGAACACACAGAAAACTCTGTACCTG
CAGATGAATAGCCTGAGAGCCGAAGATACAGCTGTGTACTATTGCACTCTGGACGGCAG
GGATGGGTGGGTCGCCTATTGGGGGCAGGGAACCCTGGTGACAGTCAGCTCCGGAGGAG
GAGGATCTGGCGGAGGAGGCAGTGGGGGAGGCGGGTCAAACTTTATGCTGACCCAGCCC
CACAGTGTGTCAGAGAGCCCTGGCAAGACTGTCACCATCTCTTGTAAAAGGAACACCGG
AAATATTGGCAGTAACTACGTGAATTGGTATCAGCAGCATGAAGGGTCTAGTCCAACCA
CAATCATCTACCGGGACGATAAGAGACCCGACGGGGTGTCCGATCGATTCTCCGGATCT
ATCGACCGGTCAAGCAAGAGTGCTTCACTGACCATTAGCAATCTGAAAACAGAGGACGA
AGCAGATTACTTTTGCCAGTCCTATTCCTCTGGCTTCATCTTTGGAGGCGGGACTAAAC
TGACCGTGCTGGGCGCGGCCGCAGGTGCAGGTGGTGATTACAAAGATGATGACGATAAA
GGTGCAGCGGCGCATCACCATCATCACCAC

(SEQ ID NO: 163 of the Sequence Listing)

[Figure 180]

ATGAAACACCTGTGGTTCTTCCTCCTGCTGGTGGCAGCTCCCAGATGGGTGCTGAGCGG
ACAGGTGCAGCTGGTGCAGTCTGGCGCCGAAGTGAAGAAACCAGGCGCCAGCGTGAAGG
TGTCCTGCAAGGCCAGCGGCTACACCTTTACCGGCTACTACATGCACTGGGTGCGCCAG
GCTCCAGGCCAGGGACTGGAATGGATGGGCCGGATCAACCCCAATAGCGGCGGCACCAA
CTACGCCCAGAAATTCCAGGGCAGAGTGACCATGACCCGGGACACCAGCATCAGCACCG
CCTACATGGAACTGAGCGGCCTGAGAAGCGACGACACCGCCGTGTACTACTGTGCCTCT
GGCTCTGTGCGGCACCCTTGGGGACAGGGAACACTCGTGACCGTGTCCAGCGGTGGAGG
CGGTTCAGGCGGAGGTGGCAGCGGCGGTGGCGGGAGTCAGCCTGTGCTGACACAGCCTC
CAAGCGCCTCTGGCACACCTGGCCAGAGAGTGACAATCAGCTGCAGCGGCAGCAGAAGC
AACATCGGCGGCAACATCGTGTCCTGGTATCAGCAGTTCCCCGGCACCGCCCCTAGACT
GCTGACCTACGCCGACAACCAGAGGCCTAGCGGCGTGCCCGATAGATTCAGCGGCTCTA
AGAGCGGCACCAGCGCCAGCCTGGCCATCTCTGGCCTGCAGTCTGAGGACGAGGCCGAC
TACTATTGCGCCGCCTGGGACGACAGCCTGAACGGCGTGGTGTTTGGCGGAGGCACCAA
GCTGACAGTGCTGGGCGGAGGCGGTTCAGAAGTGCAGCTGGTGGAATCCGGGGGGGGCC
TGGTGCAGCCTGGGGGGAGCCTGAGACTGAGTTGTGCCGCCTCTGGGGTGACATTTAAC
TACTATGGCATGTCTTGGATCCGCCAGGCACCTGGAAAGGGCCTGGAGTGGGTGGCCAG
CATCACTAATTCCGGCGGGCGAATCTACTATCCCGACAGCGTCAAGGGCAGGTTCACAA
TTTCCCGCGAGAACACACAGAAAACTCTGTACCTGCAGATGAATAGCCTGAGAGCCGAA
GATACAGCTGTGTACTATTGCACTCTGGACGGCAGGGATGGGTGGGTCGCCTATTGGGG
GCAGGGAACCCTGGTGACAGTCAGCTCCGGAGGAGGAGGATCTGGCGGAGGAGGCAGTG
GGGGAGGCGGGTCAAACTTTATGCTGACCCAGCCCCACAGTGTGTCAGAGAGCCCTGGC
AAGACTGTCACCATCTCTTGTAAAAGGAACACCGGAAATATTGGCAGTAACTACGTGAA
TTGGTATCAGCAGCATGAAGGGTCTAGTCCAACCACAATCATCTACCGGGACGATAAGA
GACCCGACGGGGTGTCCGATCGATTCTCCGGATCTATCGACCGGTCAAGCAAGAGTGCT
TCACTGACCATTAGCAATCTGAAAACAGAGGACGAAGCAGATTACTTTTGCCAGTCCTA
TTCCTCTGGCTTCATCTTTGGAGGCGGGACTAAACTGACCGTGCTGGGCGCGGCCGCAG
GTGCAGGTGGTGATTACAAAGATGATGACGATAAAGGTGCAGCGGCGCATCACCATCAT
CACCAC

(SEQ ID NO: 164 of the Sequence Listing)

[Figure 181]

ATGAAACACCTGTGGTTCTTCCTCCTGCTGGTGGCAGCTCCCAGATGGGTGCTGAGCGG
ACAGGTGCAGCTGGTGCAGTCTGGCGCCGAAGTGAAGAAACCAGGCGCCAGCGTGAAGG
TGTCCTGCAAGGCCAGCGGCTACACCTTTACCGGCTACTACATGCACTGGGTGCGCCAG
GCTCCAGGCCAGGGACTGGAATGGATGGGCCGGATCAACCCCAATAGCGGCGGCACCAA
CTACGCCCAGAAATTCCAGGGCAGAGTGACCATGACCCGGGACACCAGCATCAGCACCG
CCTACATGGAACTGAGCCGGCTGAGAAGCGACGACACCGCCGTGTACTACTGCGCCAGC
GGCAAGAGCAACTACCCTTGGGGCCAGGGCACACTCGTGACCGTGTCTAGCGGAGGCGG
AGGATCTGGCGGCGGAGGAAGTGGCGGAGGGGGATCTCAGCCTGTGCTGACACAGCCTC
CTAGCGCCTCTGGCACACCTGGCCAGAGAGTGACAATCAGCTGCAGCGGCAGCAACAGC
AACATCGGCAGCCGGACCGTGAACTGGTATCAGCATCTGCCTGGCACCGCCCCCAGACT
GCTGATCTACGGCAACAACCAGTGGCCCAGCGGCGTGCCCGATAGATTCAGCGGCTCTA
AGAGCGGCAGCTCCGCCAGCCTGGCCATCTCTGGACTGAGATCCGAGGACGAGGCCGAC
TACTACTGTGCCGCCTGGGACGATAGCCTGAGCGGCGTGGTGTTTGGCGGAGGCACCAA
GCTGACAGTGCTGGGCGGAGGCGGTTCAGAAGTGCAGCTGGTGGAATCCGGGGGGGGCC
TGGTGCAGCCTGGGGGGAGCCTGAGACTGAGTTGTGCCGCCTCTGGGGTGACATTTAAC
TACTATGGCATGTCTTGGATCCGCCAGGCACCTGGAAAGGGCCTGGAGTGGGTGGCCAG
CATCACTAATTCCGGCGGGCGAATCTACTATCCCGACAGCGTCAAGGGCAGGTTCACAA
TTTCCCGCGAGAACACACAGAAAACTCTGTACCTGCAGATGAATAGCCTGAGAGCCGAA
GATACAGCTGTGTACTATTGCACTCTGGACGGCAGGGATGGGTGGGTCGCCTATTGGGG
GCAGGGAACCCTGGTGACAGTCAGCTCCGGAGGAGGAGGATCTGGCGGAGGAGGCAGTG
GGGGAGGCGGGTCAATGGCCCAGGCTGTGCTCACTCAGCCGTCCTCTGTTTCTGGCGTA
CCTGGCCAACGGGTGACCATTAGCTGTAAAAGGAATACCGGGAATATCGGGTCTAACTA
CGTGAACTGGTATCAGCAGCTTCCAGGGACAGCTCCCAAGTTGCTGATCTATCGCGACG
ACAAAAGACCCTCAGGGGTCCCTGACCGATTTAGTGGCAGCAAAAGCGGTACTTCCGCT
TCCCTGGCGATAACCGGCTTTCAGGCCGAAGATGAGGCAGACTACTATTGCCAGTCATA
TTCCAGCGGCTTCATCTTCGGAGGCGGAACTAAGCTGACAGTGTTGGGCGCGGCCGCAG
GTGCAGGTGGTGATTACAAAGATGATGACGATAAAGGTGCAGCGGCGCATCACCATCAT
CACCAC

(SEQ ID NO: 165 of the Sequence Listing)

[Figure 182]

ATGAAACACCTGTGGTTCTTCCTCCTGCTGGTGGCAGCTCCCAGATGGGTGCTGAGCGG
ACAGGTGCAGCTGGTGCAGTCTGGCGCCGAAGTGAAGAAACCCGGCAGCAGCGTGAAGG
TGTCCTGCAAGGCTAGCGGCGGCACCTTCAGCAGATACGCCATCTCTTGGGTGCGCCAG
GCCCCTGGACAGGGCCTGGAATGGATGGGCGGCATCATCCCCATCTTCGGCACCGCCAA
CTACGCCCAGAAATTCCAGGGCAGAGTGACCATCACCGCCGACCAGAGCACCCGGACCG
CCTATATGGATCTGGGCCGGCTGAGAAGCGAGGACACCGCCGTGTACTACTGCGCCCTG
GAAGGCTTCCGGGACAGCCTGAAGAGAAACCCCTTCGACATCTGGGGCCAGGGCACAAT
GGTCACCGTGTCTAGCGGAGGCGGAGGATCTGGCGGCGGAGGAAGTGGCGGAGGGGGAT
CTCAGTCTGTCGTGACCCAGCCTCCTAGCGTGTCAGCCGCCCCAGGCCAGAAAGTGACA
ATCAGCTGTTCTGGCGGCACCACCAACATCGGCAGCAACTACGTGTCCTGGTATCAGCA
GCTGCCCGGAACCGCCCCCAAGTTCCTGATGTACGAGAACAACAAGCGGCCCAGCGGCA
TCCCCGACAGATTCAGCGGCAGCAAGAGCGGCACAAGCGCCACACTGGCCATCACCGGA
CTGCAGACAGGCGACGAGGCCACCTACTACTGCTCCACCTGGGACAGCAGCCTGAACAC
CGGCCTGTTTGGCGGAGGCACCAAGCTGACAGTGCTGGGCGGAGGCGGTTCAGAAGTGC
AGCTGGTGGAATCCGGGGGGGGCCTGGTGCAGCCTGGGGGGAGCCTGAGACTGAGTTGT
GCCGCCTCTGGGGTGACATTTAACTACTATGGCATGTCTTGGATCCGCCAGGCACCTGG
AAAGGGCCTGGAGTGGGTGGCCAGCATCACTAATTCCGGCGGGCGAATCTACTATCCCG
ACAGCGTCAAGGGCAGGTTCACAATTTCCCGCGAGAACACACAGAAAACTCTGTACCTG
CAGATGAATAGCCTGAGAGCCGAAGATACAGCTGTGTACTATTGCACTCTGGACGGCAG
GGATGGGTGGGTCGCCTATTGGGGGCAGGGAACCCTGGTGACAGTCAGCTCCGGAGGAG
GAGGATCTGGCGGAGGAGGCAGTGGGGGAGGCGGGTCAATGGCCCAGGCTGTGCTCACT
CAGCCGTCCTCTGTTTCTGGCGTACCTGGCCAACGGGTGACCATTAGCTGTAAAAGGAA
TACCGGGAATATCGGGTCTAACTACGTGAACTGGTATCAGCAGCTTCCAGGGACAGCTC
CCAAGTTGCTGATCTATCGCGACGACAAAAGACCCTCAGGGGTCCCTGACCGATTTAGT
GGCAGCAAAAGCGGTACTTCCGCTTCCCTGGCGATAACCGGCTTTCAGGCCGAAGATGA
GGCAGACTACTATTGCCAGTCATATTCCAGCGGCTTCATCTTCGGAGGCGGAACTAAGC
TGACAGTGTTGGGCGCGGCCGCAGGTGCAGGTGGTGATTACAAAGATGATGACGATAAA
GGTGCAGCGGCGCATCACCATCATCACCAC

(SEQ ID NO: 166 of the Sequence Listing)

[Figure 183]

ATGAAACACCTGTGGTTCTTCCTCCTGCTGGTGGCAGCTCCCAGATGGGTGCTGAGCGG
ACAGGTGCAGCTGGTGCAGTCTGGCGCCGAAGTGAAGAAACCAGGCGCCAGCGTGAAGG
TGTCCTGCAAGGCCAGCGGCTACACCTTTACCGGCTACTACATGCACTGGGTGCGCCAG
GCTCCAGGCCAGGGACTGGAATGGATGGGCCGGATCAACCCCAATAGCGGCGGCACCAA
CTACGCCCAGAAATTCCAGGGCAGAGTGACCATGACCCGGGACACCAGCATCAGCACCG
CCTACATGGAACTGAGCGGCCTGAGAAGCGACGACACCGCCGTGTACTACTGTGCCTCT
GGCTCTGTGCGGCACCCTTGGGGACAGGGAACACTCGTGACCGTGTCCAGCGGTGGAGG
CGGTTCAGGCGGAGGTGGCAGCGGCGGTGGCGGGAGTCAGCCTGTGCTGACACAGCCTC
CAAGCGCCTCTGGCACACCTGGCCAGAGAGTGACAATCAGCTGCAGCGGCAGCAGAAGC
AACATCGGCGGCAACATCGTGTCCTGGTATCAGCAGTTCCCCGGCACCGCCCCTAGACT
GCTGACCTACGCCGACAACCAGAGGCCTAGCGGCGTGCCCGATAGATTCAGCGGCTCTA
AGAGCGGCACCAGCGCCAGCCTGGCCATCTCTGGCCTGCAGTCTGAGGACGAGGCCGAC
TACTATTGCGCCGCCTGGGACGACAGCCTGAACGGCGTGGTGTTTGGCGGAGGCACCAA
GCTGACAGTGCTGGGCGGAGGCGGTTCAGAAGTGCAGCTGGTGGAATCCGGGGGGGGCC
TGGTGCAGCCTGGGGGGAGCCTGAGACTGAGTTGTGCCGCCTCTGGGGTGACATTTAAC
TACTATGGCATGTCTTGGATCCGCCAGGCACCTGGAAAGGGCCTGGAGTGGGTGGCCAG
CATCACTAATTCCGGCGGGCGAATCTACTATCCCGACAGCGTCAAGGGCAGGTTCACAA
TTTCCCGCGAGAACACACAGAAAACTCTGTACCTGCAGATGAATAGCCTGAGAGCCGAA
GATACAGCTGTGTACTATTGCACTCTGGACGGCAGGGATGGGTGGGTCGCCTATTGGGG
GCAGGGAACCCTGGTGACAGTCAGCTCCGGAGGAGGAGGATCTGGCGGAGGAGGCAGTG
GGGGAGGCGGGTCAATGGCCCAGGCTGTGCTCACTCAGCCGTCCTCTGTTTCTGGCGTA
CCTGGCCAACGGGTGACCATTAGCTGTAAAAGGAATACCGGGAATATCGGGTCTAACTA
CGTGAACTGGTATCAGCAGCTTCCAGGGACAGCTCCCAAGTTGCTGATCTATCGCGACG
ACAAAAGACCCTCAGGGGTCCCTGACCGATTTAGTGGCAGCAAAAGCGGTACTTCCGCT
TCCCTGGCGATAACCGGCTTTCAGGCCGAAGATGAGGCAGACTACTATTGCCAGTCATA
TTCCAGCGGCTTCATCTTCGGAGGCGGAACTAAGCTGACAGTGTTGGGCGCGGCCGCAG
GTGCAGGTGGTGATTACAAAGATGATGACGATAAAGGTGCAGCGGCGCATCACCATCAT
CACCAC

(SEQ ID NO: 167 of the Sequence Listing)

[Figure 184]

ATGAAACACCTGTGGTTCTTCCTCCTGCTGGTGGCAGCTCCCAGATGGGTGCTGAGCGG
ACAGGTGCAGCTGGTGCAGTCTGGCGCCGAAGTGAAGAAACCAGGCGCCAGCGTGAAGG
TGTCCTGCAAGGCCAGCGGCTACACCTTTACCGGCTACTACATGCACTGGGTGCGCCAG
GCTCCAGGCCAGGGACTGGAATGGATGGGCCGGATCAACCCCAATAGCGGCGGCACCAA
CTACGCCCAGAAATTCCAGGGCAGAGTGACCATGACCCGGGACACCAGCATCAGCACCG
CCTACATGGAACTGAGCCGGCTGAGAAGCGACGACACCGCCGTGTACTACTGCGCCAGC
GGCAAGAGCAACTACCCTTGGGGCCAGGGCACACTCGTGACCGTGTCTAGCGGAGGCGG
AGGATCTGGCGGCGGAGGAAGTGGCGGAGGGGGATCTCAGCCTGTGCTGACACAGCCTC
CTAGCGCCTCTGGCACACCTGGCCAGAGAGTGACAATCAGCTGCAGCGGCAGCAACAGC
AACATCGGCAGCCGGACCGTGAACTGGTATCAGCATCTGCCTGGCACCGCCCCCAGACT
GCTGATCTACGGCAACAACCAGTGGCCCAGCGGCGTGCCCGATAGATTCAGCGGCTCTA
AGAGCGGCAGCTCCGCCAGCCTGGCCATCTCTGGACTGAGATCCGAGGACGAGGCCGAC
TACTACTGTGCCGCCTGGGACGATAGCCTGAGCGGCGTGGTGTTTGGCGGAGGCACCAA
GCTGACAGTGCTGGGCGGAGGCGGTTCAGAAGTGCAGCTGGTGGAATCCGGGGGGGGCC
TGGTGCAGCCTGGGGGGAGCCTGAGACTGAGTTGTGCCGCCTCTGGGGTGACATTTAAC
TACTATGGCATGTCTTGGATCCGCCAGGCACCTGGAAAGGGCCTGGAGTGGGTGGCCAG
CATCACTAATTCCGGCGGGCGAATCTACTATCCCGACAGCGTCAAGGGCAGGTTCACAA
TTTCCCGCGAGAACACACAGAAAACTCTGTACCTGCAGATGAATAGCCTGAGAGCCGAA
GATACAGCTGTGTACTATTGCACTCTGGACGGCAGGGATGGGTGGGTCGCCTATTGGGG
GCAGGGAACCCTGGTGACAGTCAGCTCCGGAGGAGGAGGATCTGGCGGAGGAGGCAGTG
GGGGAGGCGGGTCAAACTTTATGCTCACTCAGCCGTCCTCTGTTTCTGGCGTACCTGGC
CAACGGGTGACCATTAGCTGTACGGGTAATACCGGGAATATCGGGTCTAACTACGTGAA
CTGGTATCAGCAGCTTCCAGGGACAGCTCCCAAGTTGCTGATCTATCGCGACGACAAAA
GACCCTCAGGGGTCCCTGACCGATTTAGTGGCAGCAAAAGCGGTACTTCCGCTTCCCTG
GCGATAACCGGCTTTCAGGCCGAAGATGAGGCAGACTACTATTGCCAGTCATATTCCAG
CGGCTTCATCTTCGGAGGCGGAACTAAGCTGACAGTGTTGGGCGCGGCCGCAGGTGCAG
GTGGTGATTACAAAGATGATGACGATAAAGGTGCAGCGGCGCATCACCATCATCACCAC

(SEQ ID NO: 168 of the Sequence Listing)

[Figure 185]

ATGAAACACCTGTGGTTCTTCCTCCTGCTGGTGGCAGCTCCCAGATGGGTGCTGAGCGG
ACAGGTGCAGCTGGTGCAGTCTGGCGCCGAAGTGAAGAAACCCGGCAGCAGCGTGAAGG
TGTCCTGCAAGGCTAGCGGCGGCACCTTCAGCAGATACGCCATCTCTTGGGTGCGCCAG
GCCCCTGGACAGGGCCTGGAATGGATGGGCGGCATCATCCCCATCTTCGGCACCGCCAA
CTACGCCCAGAAATTCCAGGGCAGAGTGACCATCACCGCCGACCAGAGCACCCGGACCG
CCTATATGGATCTGGGCCGGCTGAGAAGCGAGGACACCGCCGTGTACTACTGCGCCCTG
GAAGGCTTCCGGGACAGCCTGAAGAGAAACCCCTTCGACATCTGGGGCCAGGGCACAAT
GGTCACCGTGTCTAGCGGAGGCGGAGGATCTGGCGGCGGAGGAAGTGGCGGAGGGGGAT
CTCAGTCTGTCGTGACCCAGCCTCCTAGCGTGTCAGCCGCCCCAGGCCAGAAAGTGACA
ATCAGCTGTTCTGGCGGCACCACCAACATCGGCAGCAACTACGTGTCCTGGTATCAGCA
GCTGCCCGGAACCGCCCCCAAGTTCCTGATGTACGAGAACAACAAGCGGCCCAGCGGCA
TCCCCGACAGATTCAGCGGCAGCAAGAGCGGCACAAGCGCCACACTGGCCATCACCGGA
CTGCAGACAGGCGACGAGGCCACCTACTACTGCTCCACCTGGGACAGCAGCCTGAACAC
CGGCCTGTTTGGCGGAGGCACCAAGCTGACAGTGCTGGGCGGAGGCGGTTCAGAAGTGC
AGCTGGTGGAATCCGGGGGGGGCCTGGTGCAGCCTGGGGGGAGCCTGAGACTGAGTTGT
GCCGCCTCTGGGGTGACATTTAACTACTATGGCATGTCTTGGATCCGCCAGGCACCTGG
AAAGGGCCTGGAGTGGGTGGCCAGCATCACTAATTCCGGCGGGCGAATCTACTATCCCG
ACAGCGTCAAGGGCAGGTTCACAATTTCCCGCGAGAACACACAGAAAACTCTGTACCTG
CAGATGAATAGCCTGAGAGCCGAAGATACAGCTGTGTACTATTGCACTCTGGACGGCAG
GGATGGGTGGGTCGCCTATTGGGGGCAGGGAACCCTGGTGACAGTCAGCTCCGGAGGAG
GAGGATCTGGCGGAGGAGGCAGTGGGGGAGGCGGGTCAAACTTTATGCTCACTCAGCCG
TCCTCTGTTTCTGGCGTACCTGGCCAACGGGTGACCATTAGCTGTACGGGTAATACCGG
GAATATCGGGTCTAACTACGTGAACTGGTATCAGCAGCTTCCAGGGACAGCTCCCAAGT
TGCTGATCTATCGCGACGACAAAAGACCCTCAGGGGTCCCTGACCGATTTAGTGGCAGC
AAAAGCGGTACTTCCGCTTCCCTGGCGATAACCGGCTTTCAGGCCGAAGATGAGGCAGA
CTACTATTGCCAGTCATATTCCAGCGGCTTCATCTTCGGAGGCGGAACTAAGCTGACAG
TGTTGGGCGCGGCCGCAGGTGCAGGTGGTGATTACAAAGATGATGACGATAAAGGTGCA
GCGGCGCATCACCATCATCACCAC

(SEQ ID NO: 169 of the Sequence Listing)

[Figure 186]

ATGAAACACCTGTGGTTCTTCCTCCTGCTGGTGGCAGCTCCCAGATGGGTGCTGAGCGG
ACAGGTGCAGCTGGTGCAGTCTGGCGCCGAAGTGAAGAAACCAGGCGCCAGCGTGAAGG
TGTCCTGCAAGGCCAGCGGCTACACCTTTACCGGCTACTACATGCACTGGGTGCGCCAG
GCTCCAGGCCAGGGACTGGAATGGATGGGCCGGATCAACCCCAATAGCGGCGGCACCAA
CTACGCCCAGAAATTCCAGGGCAGAGTGACCATGACCCGGGACACCAGCATCAGCACCG
CCTACATGGAACTGAGCGGCCTGAGAAGCGACGACACCGCCGTGTACTACTGTGCCTCT
GGCTCTGTGCGGCACCCTTGGGGACAGGGAACACTCGTGACCGTGTCCAGCGGTGGAGG
CGGTTCAGGCGGAGGTGGCAGCGGCGGTGGCGGGAGTCAGCCTGTGCTGACACAGCCTC
CAAGCGCCTCTGGCACACCTGGCCAGAGAGTGACAATCAGCTGCAGCGGCAGCAGAAGC
AACATCGGCGGCAACATCGTGTCCTGGTATCAGCAGTTCCCCGGCACCGCCCCTAGACT
GCTGACCTACGCCGACAACCAGAGGCCTAGCGGCGTGCCCGATAGATTCAGCGGCTCTA
AGAGCGGCACCAGCGCCAGCCTGGCCATCTCTGGCCTGCAGTCTGAGGACGAGGCCGAC
TACTATTGCGCCGCCTGGGACGACAGCCTGAACGGCGTGGTGTTTGGCGGAGGCACCAA
GCTGACAGTGCTGGGCGGAGGCGGTTCAGAAGTGCAGCTGGTGGAATCCGGGGGGGGCC
TGGTGCAGCCTGGGGGGAGCCTGAGACTGAGTTGTGCCGCCTCTGGGGTGACATTTAAC
TACTATGGCATGTCTTGGATCCGCCAGGCACCTGGAAAGGGCCTGGAGTGGGTGGCCAG
CATCACTAATTCCGGCGGGCGAATCTACTATCCCGACAGCGTCAAGGGCAGGTTCACAA
TTTCCCGCGAGAACACACAGAAAACTCTGTACCTGCAGATGAATAGCCTGAGAGCCGAA
GATACAGCTGTGTACTATTGCACTCTGGACGGCAGGGATGGGTGGGTCGCCTATTGGGG
GCAGGGAACCCTGGTGACAGTCAGCTCCGGAGGAGGAGGATCTGGCGGAGGAGGCAGTG
GGGGAGGCGGGTCAAACTTTATGCTCACTCAGCCGTCCTCTGTTTCTGGCGTACCTGGC
CAACGGGTGACCATTAGCTGTACGGGTAATACCGGGAATATCGGGTCTAACTACGTGAA
CTGGTATCAGCAGCTTCCAGGGACAGCTCCCAAGTTGCTGATCTATCGCGACGACAAAA
GACCCTCAGGGGTCCCTGACCGATTTAGTGGCAGCAAAAGCGGTACTTCCGCTTCCCTG
GCGATAACCGGCTTTCAGGCCGAAGATGAGGCAGACTACTATTGCCAGTCATATTCCAG
CGGCTTCATCTTCGGAGGCGGAACTAAGCTGACAGTGTTGGGCGCGGCCGCAGGTGCAG
GTGGTGATTACAAAGATGATGACGATAAAGGTGCAGCGGCGCATCACCATCATCACCAC

(SEQ ID NO: 170 of the Sequence Listing)

[Figure 187]

MKHLWFFLLLVAAPRWVLSGQVQLVQSGAEVKKPGASVKVSCKASGYTFTGYYMHWVRQ
APGQGLEWMGRINPNSGGTNYAQKFQGRVTMTRDTSISTAYMELSRLRSDDTAVYYCAS
GKSNYPWGQGTLVTVSSGGGGSGGGGSGGGGSQPVLTQPPSASGTPGQRVTISCSGSNS
NIGSRTVNWYQHLPGTAPRLLIYGNNQWPSGVPDRFSGSKSGSSASLAISGLRSEDEAD
YYCAAWDDSLSGVVFGGGTKLTVLGGGGSEVQLVESGGGLVQPGGSLRLSCAASGVTFN
YYGMSWIRQAPGKGLEWVASITNSGGRIYYPDSVKGRFTISRENTQKTLYLQMNSLRAE
DTAVYYCTLDGRDGWVAYWGQGTLVTVSSGGGGSGGGGSGGGGSNFMLTQPHSVSESPG
KTVTISCKRNTGNIGSNYVNWYQQHEGSSPTTIIYRDDKRPDGVSDRFSGSIDRSSKSA
SLTISNLKTEDEADYFCQSYSSGFIFGGGTKLTVLGAAAGAGGDYKDDDDKGAAAHHHH
HH

(SEQ ID NO: 171 of the Sequence Listing)

[Figure 188]

MKHLWFFLLLVAAPRWVLSGQVQLVQSGAEVKKPGSSVKVSCKASGGTFSRYAISWVRQ
APGQGLEWMGGIIPIFGTANYAQKFQGRVTITADQSTRTAYMDLGRLRSEDTAVYYCAL
EGFRDSLKRNPFDIWGQGTMVTVSSGGGGSGGGGSGGGGSQSVVTQPPSVSAAPGQKVT
ISCSGGTTNIGSNYVSWYQQLPGTAPKFLMYENNKRPSGIPDRFSGSKSGTSATLAITG
LQTGDEATYYCSTWDSSLNTGLFGGGTKLTVLGGGGSEVQLVESGGGLVQPGGSLRLSC
AASGVTFNYYGMSWIRQAPGKGLEWVASITNSGGRIYYPDSVKGRFTISRENTQKTLYL
QMNSLRAEDTAVYYCTLDGRDGWVAYWGQGTLVTVSSGGGGSGGGGSGGGGSNFMLTQP
HSVSESPGKTVTISCKRNTGNIGSNYVNWYQQHEGSSPTTIIYRDDKRPDGVSDRFSGS
IDRSSKSASLTISNLKTEDEADYFCQSYSSGFIFGGGTKLTVLGAAAGAGGDYKDDDDK
GAAAHHHHHH

(SEQ ID NO: 172 of the Sequence Listing)

[Figure 189]
MKHLWFFLLLVAAPRWVLSGQVQLVQSGAEVKKPGASVKVSCKASGYTFTGYYMHWVRQ
APGQGLEWMGRINPNSGGTNYAQKFQGRVTMTRDTSISTAYMELSGLRSDDTAVYYCAS
GSVRHPWGQGTLVTVSSGGGGSGGGGSGGGGSQPVLTQPPSASGTPGQRVTISCSGSRS
NIGGNIVSWYQQFPGTAPRLLTYADNQRPSGVPDRFSGSKSGTSASLAISGLQSEDEAD
YYCAAWDDSLNGVVFGGGTKLTVLGGGGSEVQLVESGGGLVQPGGSLRLSCAASGVTFN
YYGMSWIRQAPGKGLEWVASITNSGGRIYYPDSVKGRFTISRENTQKTLYLQMNSLRAE
DTAVYYCTLDGRDGWVAYWGQGTLVTVSSGGGGSGGGGSGGGGSNFMLTQPHSVSESPG
KTVTISCKRNTGNIGSNYVNWYQQHEGSSPTTIIYRDDKRPDGVSDRFSGSIDRSSKSA
SLTISNLKTEDEADYFCQSYSSGFIFGGGTKLTVLGAAAGAGGDYKDDDDKGAAAHHHH
HH

(SEQ ID NO: 173 of the Sequence Listing)

[Figure 190]
MKHLWFFLLLVAAPRWVLSGQVQLVQSGAEVKKPGASVKVSCKASGYTFTGYYMHWVRQ
APGQGLEWMGRINPNSGGTNYAQKFQGRVTMTRDTSISTAYMELSRLRSDDTAVYYCAS
GKSNYPWGQGTLVTVSSGGGGSGGGGSGGGGSQPVLTQPPSASGTPGQRVTISCSGSNS
NIGSRTVNWYQHLPGTAPRLLIYGNNQWPSGVPDRFSGSKSGSSASLAISGLRSEDEAD
YYCAAWDDSLSGVVFGGGTKLTVLGGGGSEVQLVESGGGLVQPGGSLRLSCAASGVTFN
YYGMSWIRQAPGKGLEWVASITNSGGRIYYPDSVKGRFTISRENTQKTLYLQMNSLRAE
DTAVYYCTLDGRDGWVAYWGQGTLVTVSSGGGGSGGGGSGGGGSMAQAVLTQPSSVSGV
PGQRVTISCKRNTGNIGSNYVNWYQQLPGTAPKLLIYRDDKRPSGVPDRFSGSKSGTSA
SLAITGFQAEDEADYYCQSYSSGFIFGGGTKLTVLGAAAGAGGDYKDDDDKGAAAHHHH
HH

(SEQ ID NO: 174 of the Sequence Listing)

[Figure 191]

MKHLWFFLLLVAAPRWVLSGQVQLVQSGAEVKKPGSSVKVSCKASGGTFSRYAISWVRQ
APGQGLEWMGGIIPIFGTANYAQKFQGRVTITADQSTRTAYMDLGRLRSEDTAVYYCAL
EGFRDSLKRNPFDIWGQGTMVTVSSGGGGSGGGGSGGGGSQSVVTQPPSVSAAPGQKVT
ISCSGGTTNIGSNYVSWYQQLPGTAPKFLMYENNKRPSGIPDRFSGSKSGTSATLAITG
LQTGDEATYYCSTWDSSLNTGLFGGGTKLTVLGGGGSEVQLVESGGGLVQPGGSLRLSC
AASGVTFNYYGMSWIRQAPGKGLEWVASITNSGGRIYYPDSVKGRFTISRENTQKTLYL
QMNSLRAEDTAVYYCTLDGRDGWVAYWGQGTLVTVSSGGGGSGGGGSGGGGSMAQAVLT
QPSSVSGVPGQRVTISCKRNTGNIGSNYVNWYQQLPGTAPKLLIYRDDKRPSGVPDRFS
GSKSGTSASLAITGFQAEDEADYYCQSYSSGFIFGGGTKLTVLGAAAGAGGDYKDDDDK
GAAAHHHHHH

(SEQ ID NO: 175 of the Sequence Listing)

[Figure 192]

MKHLWFFLLLVAAPRWVLSGQVQLVQSGAEVKKPGASVKVSCKASGYTFTGYYMHWVRQ
APGQGLEWMGRINPNSGGTNYAQKFQGRVTMTRDTSISTAYMELSGLRSDDTAVYYCAS
GSVRHPWGQGTLVTVSSGGGGSGGGGSGGGGSQPVLTQPPSASGTPGQRVTISCSGSRS
NIGGNIVSWYQQFPGTAPRLLTYADNQRPSGVPDRFSGSKSGTSASLAISGLQSEDEAD
YYCAAWDDSLNGVVFGGGTKLTVLGGGGSEVQLVESGGGLVQPGGSLRLSCAASGVTFN
YYGMSWIRQAPGKGLEWVASITNSGGRIYYPDSVKGRFTISRENTQKTLYLQMNSLRAE
DTAVYYCTLDGRDGWVAYWGQGTLVTVSSGGGGSGGGGSGGGGSMAQAVLTQPSSVSGV
PGQRVTISCKRNTGNIGSNYVNWYQQLPGTAPKLLIYRDDKRPSGVPDRFSGSKSGTSA
SLAITGFQAEDEADYYCQSYSSGFIFGGGTKLTVLGAAAGAGGDYKDDDDKGAAAHHHH
HH

(SEQ ID NO: 176 of the Sequence Listing)

[Figure 193]

MKHLWFFLLLVAAPRWVLSGQVQLVQSGAEVKKPGASVKVSCKASGYTFTGYYMHWVRQ
APGQGLEWMGRINPNSGGTNYAQKFQGRVTMTRDTSISTAYMELSRLRSDDTAVYYCAS
GKSNYPWGQGTLVTVSSGGGGSGGGGSGGGGSQPVLTQPPSASGTPGQRVTISCSGSNS
NIGSRTVNWYQHLPGTAPRLLIYGNNQWPSGVPDRFSGSKSGSSASLAISGLRSEDEAD
YYCAAWDDSLSGVVFGGGTKLTVLGGGGSEVQLVESGGGLVQPGGSLRLSCAASGVTFN
YYGMSWIRQAPGKGLEWVASITNSGGRIYYPDSVKGRFTISRENTQKTLYLQMNSLRAE
DTAVYYCTLDGRDGWVAYWGQGTLVTVSSGGGGSGGGGSGGGGSNFMLTQPSSVSGVPG
QRVTISCTGNTGNIGSNYVNWYQQLPGTAPKLLIYRDDKRPSGVPDRFSGSKSGTSASL
AITGFQAEDEADYYCQSYSSGFIFGGGTKLTVLGAAAGAGGDYKDDDDKGAAAHHHHHH

(SEQ ID NO: 177 of the Sequence Listing)

[Figure 194]

MKHLWFFLLLVAAPRWVLSGQVQLVQSGAEVKKPGSSVKVSCKASGGTFSRYAISWVRQ
APGQGLEWMGGIIPIFGTANYAQKFQGRVTITADQSTRTAYMDLGRLRSEDTAVYYCAL
EGFRDSLKRNPFDIWGQGTMVTVSSGGGGSGGGGSGGGGSQSVVTQPPSVSAAPGQKVT
ISCSGGTTNIGSNYVSWYQQLPGTAPKFLMYENNKRPSGIPDRFSGSKSGTSATLAITG
LQTGDEATYYCSTWDSSLNTGLFGGGTKLTVLGGGGSEVQLVESGGGLVQPGGSLRLSC
AASGVTFNYYGMSWIRQAPGKGLEWVASITNSGGRIYYPDSVKGRFTISRENTQKTLYL
QMNSLRAEDTAVYYCTLDGRDGWVAYWGQGTLVTVSSGGGGSGGGGSGGGGSNFMLTQP
SSVSGVPGQRVTISCTGNTGNIGSNYVNWYQQLPGTAPKLLIYRDDKRPSGVPDRFSGS
KSGTSASLAITGFQAEDEADYYCQSYSSGFIFGGGTKLTVLGAAAGAGGDYKDDDDKGA
AAHHHHHH

(SEQ ID NO: 178 of the Sequence Listing)

[Figure 195]

MKHLWFFLLLVAAPRWVLSGQVQLVQSGAEVKKPGASVKVSCKASGYTFTGYYMHWVRQ
APGQGLEWMGRINPNSGGTNYAQKFQGRVTMTRDTSISTAYMELSGLRSDDTAVYYCAS
GSVRHPWGQGTLVTVSSGGGGSGGGGSGGGGSQPVLTQPPSASGTPGQRVTISCSGSRS
NIGGNIVSWYQQFPGTAPRLLTYADNQRPSGVPDRFSGSKSGTSASLAISGLQSEDEAD
YYCAAWDDSLNGVVFGGGTKLTVLGGGGSEVQLVESGGGLVQPGGSLRLSCAASGVTFN
YYGMSWIRQAPGKGLEWVASITNSGGRIYYPDSVKGRFTISRENTQKTLYLQMNSLRAE
DTAVYYCTLDGRDGWVAYWGQGTLVTVSSGGGGSGGGGSGGGGSNFMLTQPSSVSGVPG
QRVTISCTGNTGNIGSNYVNWYQQLPGTAPKLLIYRDDKRPSGVPDRFSGSKSGTSASL
AITGFQAEDEADYYCQSYSSGFIFGGGTKLTVLGAAAGAGGDYKDDDDKGAAAHHHHHH

(SEQ ID NO: 179 of the Sequence Listing)

[Figure 196]

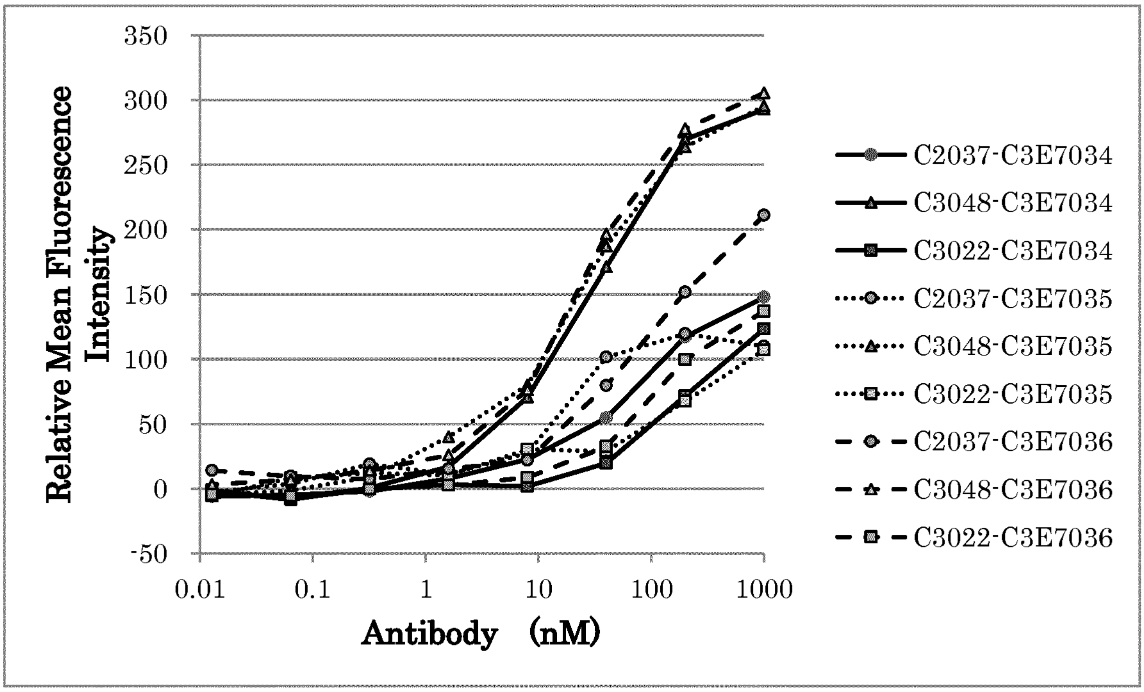

[Figure 197]

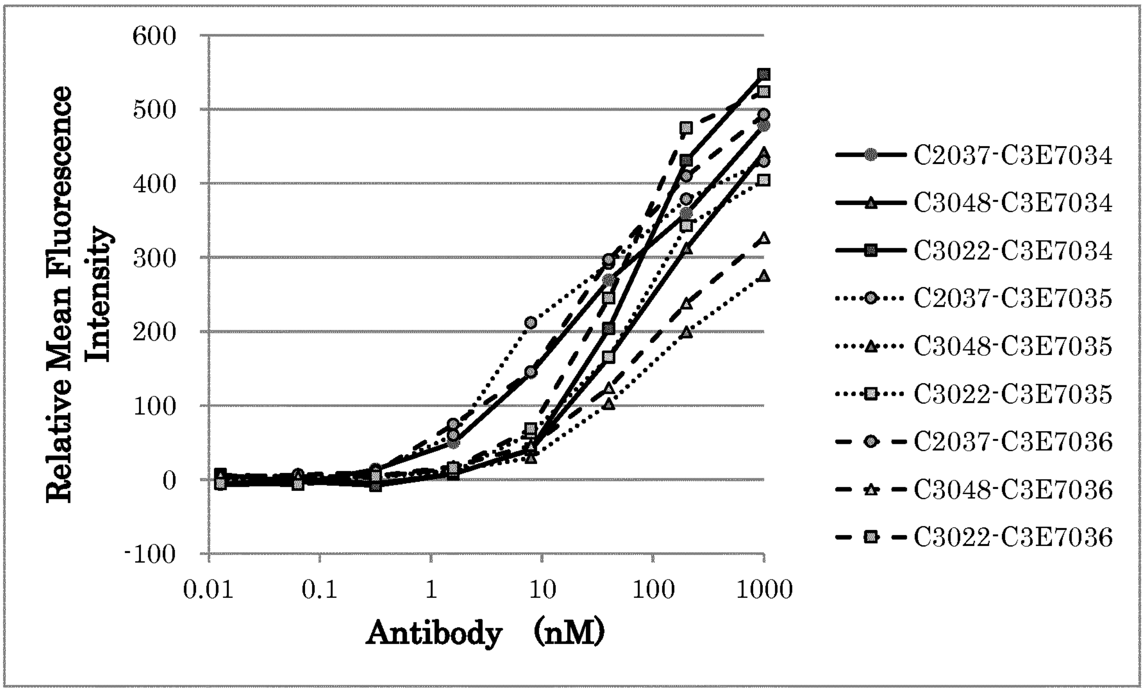


[Figure 198]

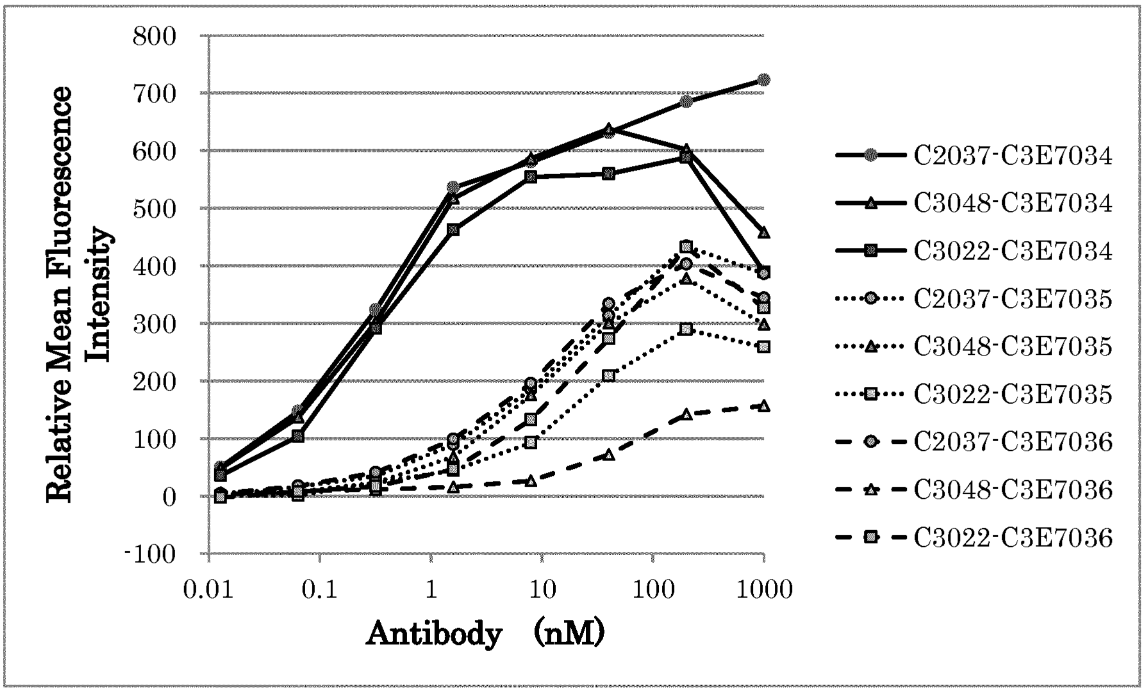

[Figure 199]

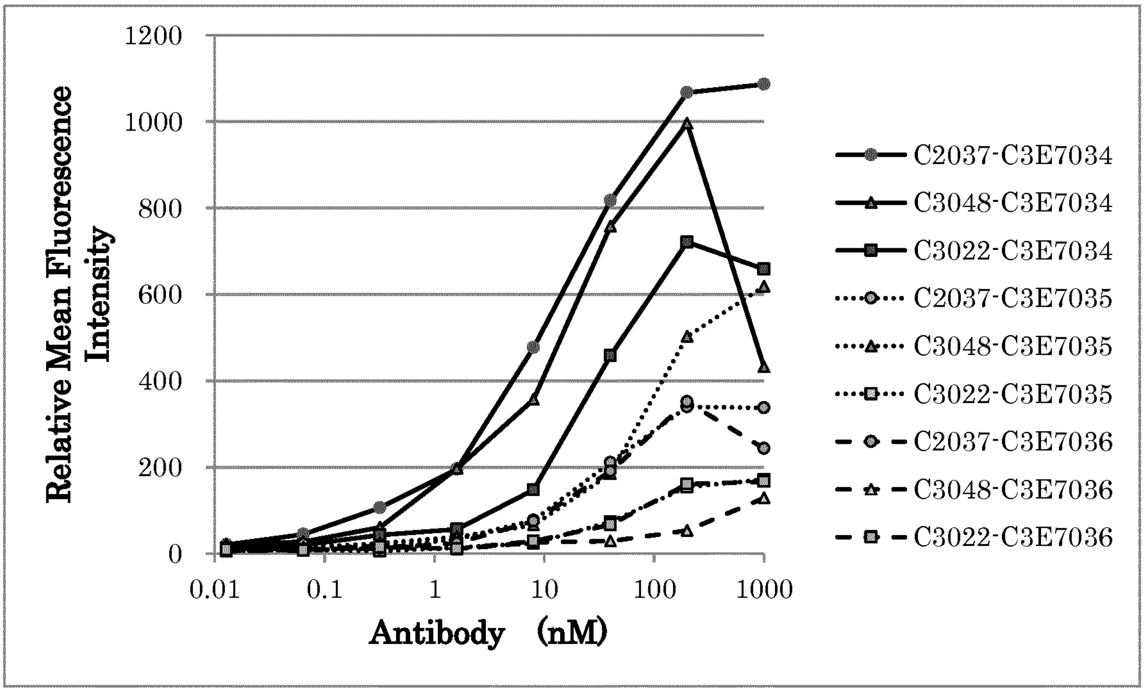


[Figure 200]

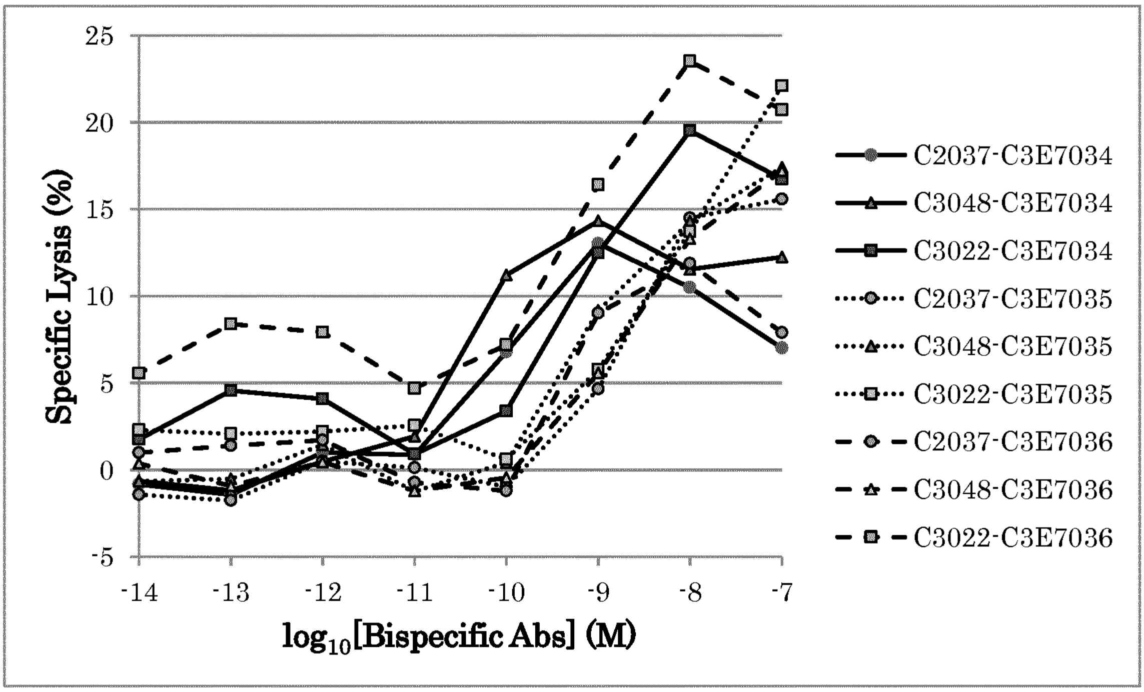

[Figure 201]

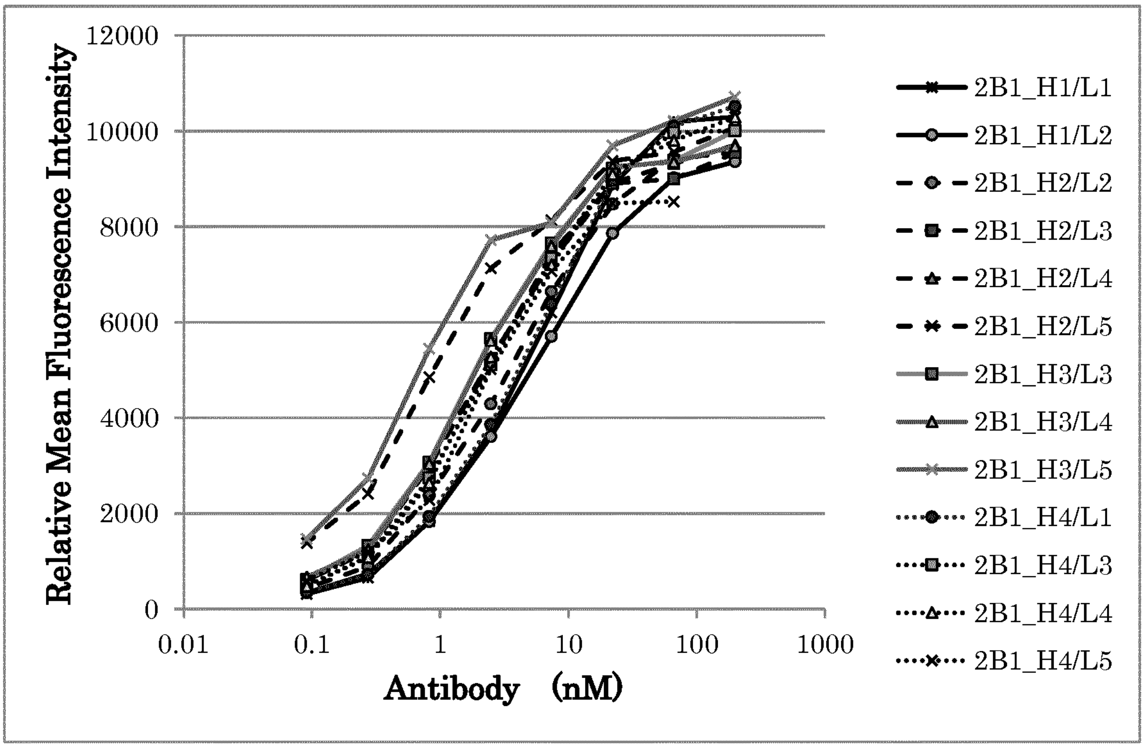


[Figure 202]

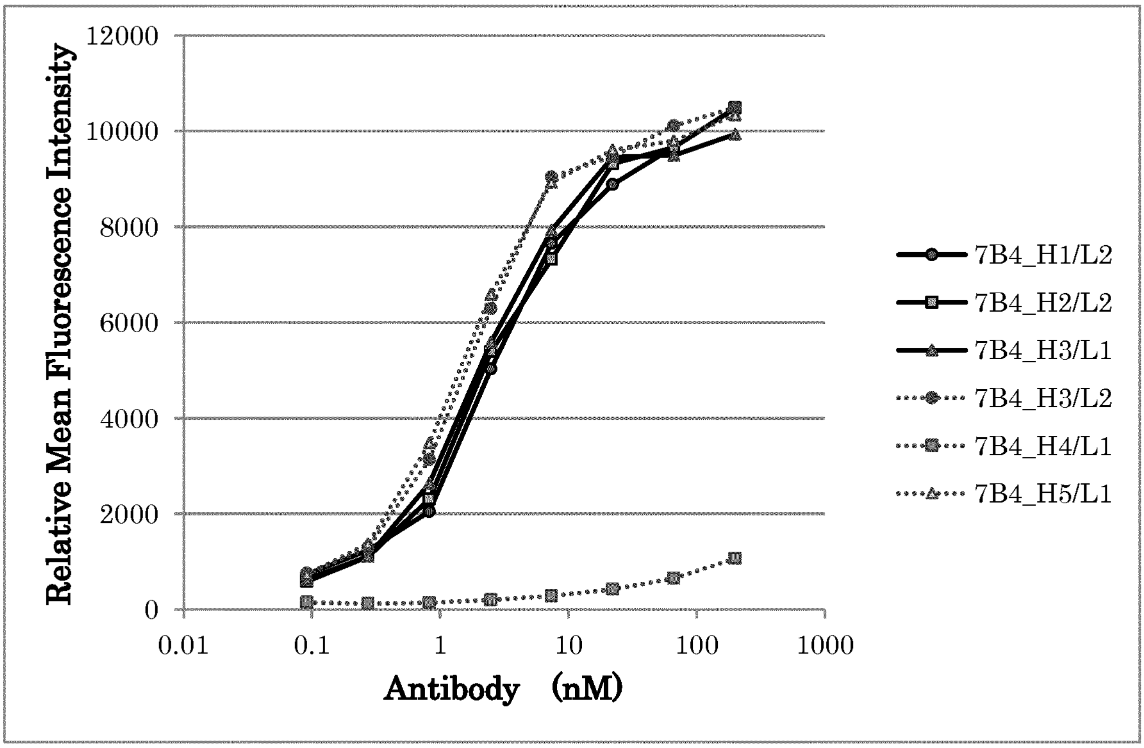

[Figure 203]
GEVQLVESGGGLVQPGGSLRLSCAASGVTFNYYGMSWIRQAPGKGLEWVASITNSGGRI
YYPDSVKGRFTISRENTQKTLYLQMNSLRAEDTAVYYCTLDGRDGWVAYWGQGTLVTVS
SGGGGSGGGGSGGGGSNFMLTQPHSVSESPGKTVTISCKRNTGNIGSNYVNWYQQHEGS
SPTTIIYRDDKRPDGVSDRFSGSIDRSSKSASLTISNLKTEDEADYFCQSYSSGFIFGG
GTKLTVLGAAAGAGGDYKDDDDKGAAAHHHHHH

(SEQ ID NO: 180 of the Sequence Listing)

[Figure 204]
GEVQLVESGGGLVQPGGSLRLSCAASGVTFNYYGMSWIRQAPGKGLEWVASITNSGGRI
YYPDSVKGRFTISRENTQKTLYLQMNSLRAEDTAVYYCTLDGRDGWVAYWGQGTLVTVS
SGGGGSGGGGSGGGGSMAQAVLTQPSSVSGVPGQRVTISCKRNTGNIGSNYVNWYQQLP
GTAPKLLIYRDDKRPSGVPDRFSGSKSGTSASLAITGFQAEDEADYYCQSYSSGFIFGG
GTKLTVLGAAAGAGGDYKDDDDKGAAAHHHHHH

(SEQ ID NO: 181 of the Sequence Listing)

[Figure 205]
GEVQLVESGGGLVQPGGSLRLSCAASGVTFNYYGMSWIRQAPGKGLEWVASITNSGGRI
YYPDSVKGRFTISRENTQKTLYLQMNSLRAEDTAVYYCTLDGRDGWVAYWGQGTLVTVS
SGGGGSGGGGSGGGGSNFMLTQPSSVSGVPGQRVTISCTGNTGNIGSNYVNWYQQLPGT
APKLLIYRDDKRPSGVPDRFSGSKSGTSASLAITGFQAEDEADYYCQSYSSGFIFGGGT
KLTVLGAAAGAGGDYKDDDDKGAAAHHHHHH

(SEQ ID NO: 182 of the Sequence Listing)

[Figure 206]
GVTFNYYG

(SEQ ID NO: 183 of the Sequence Listing)

[Figure 207]
ITNSGGRI

(SEQ ID NO: 184 of the Sequence Listing)

[Figure 208]
TLDGRDGWVAY

(SEQ ID NO: 185 of the Sequence Listing)

[Figure 209]
TGNIGSNY

(SEQ ID NO: 186 of the Sequence Listing)

[Figure 210]
RDD

(SEQ ID NO: 187 of the Sequence Listing)

[Figure 211]
QSYSSGFI

(SEQ ID NO: 188 of the Sequence Listing)

[Figure 212]
MQSGTHWRVLGLCLLSVGVWGQDGNEEMGGITQTPYKVSISGTTVILTCPQYPGSEILW
QHNDKNIGGDEDDKNIGSDEDHLSLKEFSELEQSGYYVCYPRGSKPEDANFYLYLRARV
CENCMEMDVMSVATIVIVDICITGGLLLLVYYWSKNRKAKAKPVTRGAGAGGRQRGQNK
ERPPPVPNPDYEPIRKGQRDLYSGLNQRRI

(SEQ ID NO: 189 of the Sequence Listing)

[Figure 213]

C A G G T G C A G C T G G T G C A G T C T G G G G C T G A G G T G A A

G A A G C C T G G G G C C T C A G T G A A G G T C T C C T G C A A G G

C T T C T G G A T A C A C C T T C A C C G G C T A C T A T A T G C A C

T G G G T G C G A C A G G C C C C T G G A C A A G G G C T T G A G T G

G A T G G G A C G G A T C A A C C C T A A C A G T G G T G G C A C A A

A C T A T G C A C A G A A G T T T C A G G G C A G G G T C A C C A T G

A C C A G G G A C A C G T C C A T C A G C A C A G C C T A C A T G G A

G C T G A G C G G G C T G A G A T C T G A C G A C A C G G C C G T T T

A T T A C T G T A A G A G T G G T A G T G T A A G G C C T C C C T G G

G G C C A G G G A A C C C T G G T C A C C G T C T C C T C A

(SEQ ID NO: 190 of the Sequence Listing)

[Figure 214]

Q V Q L V Q S G A E V K K P G A S V K V S C K A S G Y T F T G Y Y M H

W V R Q A P G Q G L E W M G R I N P N S G G T N Y A Q K F Q G R V T M

T R D T S I S T A Y M E L S G L R S D D T A V Y Y C K S G S V R P P W

G Q G T L V T V S S

(SEQ ID NO: 191 of the Sequence Listing)

[Figure 215]

C A G C C T G T G C T G A C T C A G C C A C C C T C A G C G T C T G G

G A C C C C C G G G C A G A G G G T C A C C A T C T C T T G T T C T G

G A A G C A G G T C C A A C A T C G G A G G T A A T A T T G T A A G T

T G G T A C C A G C A G T T C C C A G G A A C G G C C C C C A G A C T

C C T C A C T T A T C G G A A T A C T C G G C G G C C C T C A G G G G

T C C C T G A C C G A T T C T C T G G C T C C A A G T C T G G C A C C

T C A G C C T C C C T G G C C A T C A G T G G C C T C C A G T C T G A

G G A T G A G G C T G A C T A T T A T T G T G C A G C A T G G G A T G

A C A G C C T G G G G G G T G T T G T G T T C G G A G G A G G C A C C

A A G C T G A C C G T C C T A

(SEQ ID NO: 192 of the Sequence Listing)

[Figure 216]

Q P V L T Q P P S A S G T P G Q R V T I S C S G S R S N I G G N I V S

W Y Q Q F P G T A P R L L T Y R N T R R P S G V P D R F S G S K S G T

S A S L A I S G L Q S E D E A D Y Y C A A W D D S L G G V V F G G G T

K L T V L

(SEQ ID NO: 193 of the Sequence Listing)

[Figure 217]

C A G G T T C A G C T G G T T C A G T C T G G C G C C G A A G T G A A

G A A A C C T G G C A G C A G C G T G A A G G T G T C C T G C A A A G

C T T C T G G C G G C A C C C T G A G C A G A T A C G C C A T C T C T

T G G G T T C G A C A G G C C C C T G G A C A A G G C C T G G A A T G

G A T G G G A G G C A T C A T C C C C A T C T T C G G C A C C G C C A

A T T A C G C C C A G A A A T T C C A G G G C A G A G T G A C C A T C

A C C G C C G A C C A G T C T A C C A G A A C C G C C T A C A T G G A

G C T G G G C A G A C T G A G A A G C G A G G A C A C C G C C G T G T

A C T A C T G T G C T C T G G A A G G C T T C C G G G A C A G C C T G

A A G A G A A A C C C C G T G G A C A T C T G G G G C A G A G G C A C

C A T G G T T A C A G T G T C T A G C

(SEQ ID NO: 194 of the Sequence Listing)

[Figure 218]

Q V Q L V Q S G A E V K K P G S S V K V S C K A S G G T L S R Y A I S

W V R Q A P G Q G L E W M G G I I P I F G T A N Y A Q K F Q G R V T I

T A D Q S T R T A Y M E L G R L R S E D T A V Y Y C A L E G F R D S L

K R N P V D I W G R G T M V T V S S

(SEQ ID NO: 195 of the Sequence Listing)

[Figure 219]

C A A T C T G T G G T C A C A C A G C C T C C A A G C G T G T C A G C

T G C C C C A G G C C A G A A A G T G A C A A T C C C T T G T A G C G

G C G G C A C C A C C A A C A T C G G C A G C A A T T A C G T G T C C

T G G T A T C A G C A G C T G C C C G G A A C A G C C C C T A A G T T

C C T G A T G T A C G A G A A C A A C A A G C G G C C C A G C G G C A

T C C C C G A T A G A T T T T C T G G C A G C A A G A G C G G C A C C

A G C G C C A C A C T G G C T A T T A C A G G A C T G G A G A C A G A

G G A C G A G G C C A C C T A C T A C T G T A G C A C C T G G G A C A

G C A G C C T G A A C G C C G G A C T T T T T G G A G G C G G C A C A

G A G C T G A C A G T T C T T

(SEQ ID NO: 196 of the Sequence Listing)

[Figure 220]

Q S V V T Q P P S V S A A P G Q K V T I P C S G G T T N I G S N Y V S

W Y Q Q L P G T A P K F L M Y E N N K R P S G I P D R F S G S K S G T

S A T L A I T G L E T E D E A T Y Y C S T W D S S L N A G L F G G G T

E L T V L

(SEQ ID NO: 197 of the Sequence Listing)

[Figure 221]

| Name of clone | Dissociation rate constants (M) |
|---|---|
| C3022 | 9. 5x10$^{-8}$ |
| E1018 | 3. 2x10$^{-12}$ |
| C3048 | 3. 6x10$^{-10}$ |
| D1012 | 2. 2x10$^{-10}$ |

[Figure 222]

ATGAGGCCTACCTGGGCCTGGTGGCTGTTCCTGGTGCTGCTGCTGGCCCTGTG
GGCCCCCGCCCGGGGCCAGGTGCAGCTGGTGGAGTCCGGCGGCGGCCTGGTGA
AGCCCAGCCAGACCCTGTCCCTGACCTGCACATTCAGCGGCTTTTCCCTGAAC
ACATACGATATGGGAGTGGGATGGATCAGACAGCCACCTGGCAAGGGCCTGGA
GTGGATCGGCAACATCTGGTGGGACGATGACAAGTACTATAATCCCTCTCTGA
AGAGCAGAGTGACCATCTCTAAGGATACAAGCAACAATCAGGCCTCCCTGAAG
CTGAGCTCCGTGACCGCCGCCGACACAGCCGTGTACTATTGCGCCAGGATCGA
GACAGTGCGGGTGAGCCGGAAGGGATTCGCACACTGGGGACAGGGCACCCTGG
TGACAGTGTCTAGCGCTAGCACAAAGGGACCTAGCGTGTTCCCACTGGCCCCT
TCCTCTAAGTCCACCTCTGGAGGAACAGCCGCCCTGGGCTGTAGGGTGAAGGA
CTATTTCCCTGAGCCAGTGACCGTGTCCTGGAACTCTGGGGCCCTGACCAGCG
GAGTGCACACATTTCCTGCCGTGCTGCAGAGCTCCGGCCTGTACAGCCTGTCT
AGCGTGGTGACCGTGCCATCCTCTAGCCTGGGCACCCAGACATATATCTGCAA
CGTGAATCACAAGCCAAGCAATACAAAGGTCGACAAGCGGGTGGAGCCCAAGT
CCTGTGACAAGACCCACACATGCCCACCCTGTCCGGCGCCAGAGGCTGCAGGA
GGACCAAGCGTGTTCCTGTTTCCACCCAAGCCTAAAGACACACTGATGATTTC
CCGAACCCCCGAAGTCACATGCGTGGTCGTGTCTGTGAGTCACGAGGACCCTG
AAGTCAAGTTCAACTGGTACGTGGATGGCGTCGAGGTGCATAATGCCAAGACT
AAACCTAGGGAGGAACAGTACAACTCAACCTATCGCGTCGTGAGCGTCCTGAC
AGTGCTGCACCAGGATTGGCTGAACGGCAAAGAATATAAGTGCAAAGTGAGCA
ATAAGGCCCTGCCCGCTCCTATCGAGAAAACCATTTCCAAGGCTAAAGGGCAG
CCTCGCGAACCACAGGTCTACGTGCTGCCCCCTAGCCGCGACGAACTGACTAA
AAATCAGGTCTCTCTGCTGTGTCTGGTCAAAGGATTCTACCCTTCCGACATCG
CCGTGGAGTGGGAAAGTAACGGCCAGCCCGAGAACAATTACCTGACCTGGCCC
CCTGTGCTGGACTCTGATGGGAGTTTCTTTCTGTATTCAAAGCTGACAGTCGA
TAAAAGCCGGTGGCAGCAGGGCAATGTGTTCAGCTGCTCCGTCATGCACGAAG
CACTGCACAACCATTACACTCAGAAGTCCCTGTCCCTGTCACCTGGC

(SEQ ID NO: 198 of the Sequence Listing)

[Figure 223]

M R P T W A W W L F L V L L L A L W A P A R G Q V Q L V E S G G G L V

K P S Q T L S L T C T F S G F S L N T Y D M G V G W I R Q P P G K G L

E W I G N I W W D D D K Y Y N P S L K S R V T I S K D T S N N Q A S L

K L S S V T A A D T A V Y Y C A R I E T V R V S R K G F A H W G Q G T

L V T V S S A S T K G P S V F P L A P S S K S T S G G T A A L G C R V

K D Y F P E P V T V S W N S G A L T S G V H T F P A V L Q S S G L Y S

L S S V V T V P S S S L G T Q T Y I C N V N H K P S N T K V D K R V E

P K S C D K T H T C P P C P A P E A A G G P S V F L F P P K P K D T L

M I S R T P E V T C V V V S V S H E D P E V K F N W Y V D G V E V H N

A K T K P R E E Q Y N S T Y R V V S V L T V L H Q D W L N G K E Y K C

K V S N K A L P A P I E K T I S K A K G Q P R E P Q V Y V L P P S R D

E L T K N Q V S L L C L V K G F Y P S D I A V E W E S N G Q P E N N Y

L T W P P V L D S D G S F F L Y S K L T V D K S R W Q Q G N V F S C S

V M H E A L H N H Y T Q K S L S L S P G

(SEQ ID NO: 199 of the Sequence Listing)

[Figure 224]

ATGAGGCCTACCTGGGCCTGGTGGCTGTTCCTGGTGCTGCTGCTGGCCCTGTG
GGCCCCCGCCCGGGGCCAGGTGCAGCTGGTGGAGTCCGGCGGCGGCCTGGTGA
AGCCCAGCCAGACCCTGTCCCTGACCTGCACATTCAGCGGCTTTTCCCTGAAC
ACATACGATATGGGAGTGGGATGGATCAGACAGCCACCTGGCAAGGGCCTGGA
GTGGATCGGCAACATCTGGTGGGACGATGACAAGTACTATAATCCCTCTCTGA
AGAGCAGAGTGACCATCTCTAAGGATACAAGCAACAATCAGGCCTCCCTGAAG
CTGAGCTCCGTGACCGCCGCCGACACAGCCGTGTACTATTGCGCCAGGATCGA
GACAGTGCGGGTGAGCCGGAAGGGATTCGCACACTGGGGACAGGGCACCCTGG
TGACAGTGTCTAGCGCTAGCACCAAGGGACCTAGCGTGTTCCCAGAGGCCCCT
TCCTCTAAGTCCACCTCTGGAGGAACAGCCGCCCTGGGCTGTCTGGTGACCGA
CTATTTCCCTGAGCCAGTGACAGTGTCCTGGAACTCTGGGGCCCTGACCAGCG
GAGTGCACACATTTCCTGCCGTGCTGGAGAGCTCCGGCCTGTACAGCCTGTCT
AGCGTGGTGACCGTGCCATCCTCTAGCCTGGGCACCCAGACATATATCTGCAA
CGTGAATCACAAGCCAAGCAATACAAAGGTCGACAAGAGAGTGGAGCCCAAGT
CCTGTGACAAGACCCACACATGCCCACCCTGTCCGGCGCCAGAGGCTGCAGGA
GGACCAAGCGTGTTCCTGTTTCCACCCAAGCCTAAAGACACACTGATGATTTC
CCGAACCCCCGAAGTCACATGCGTGGTCGTGTCTGTGAGTCACGAGGACCCTG
AAGTCAAGTTCAACTGGTACGTGGATGGCGTCGAGGTGCATAATGCCAAGACT
AAACCTAGGGAGGAACAGTACAACTCAACCTATCGCGTCGTGAGCGTCCTGAC
AGTGCTGCACCAGGATTGGCTGAACGGCAAAGAATATAAGTGCAAAGTGAGCA
ATAAGGCCCTGCCCGCTCCTATCGAGAAAACCATTTCCAAGGCTAAAGGGCAG
CCTCGCGAACCACAGGTCTACGTGCTGCCCCCTAGCCGCGACGAACTGACTAA
AAATCAGGTCTCTCTGCTGTGTCTGGTCAAAGGATTCTACCCTTCCGACATCG
CCGTGGAGTGGGAAAGTAACGGCCAGCCCGAGAACAATTACCTGACCTGGCCC
CCTGTGCTGGACTCTGATGGGAGTTTCTTTCTGTATTCAAAGCTGACAGTCGA
TAAAAGCCGGTGGCAGCAGGGCAATGTGTTCAGCTGCTCCGTCATGCACGAAG
CACTGCACAACCATTACACTCAGAAGTCCCTGTCCCTGTCACCTGGC

(SEQ ID NO: 200 of the Sequence Listing)

[Figure 225]

M R P T W A W W L F L V L L L A L W A P A R G Q V Q L V E S G G G L V

K P S Q T L S L T C T F S G F S L N T Y D M G V G W I R Q P P G K G L

E W I G N I W W D D D K Y Y N P S L K S R V T I S K D T S N N Q A S L

K L S S V T A A D T A V Y Y C A R I E T V R V S R K G F A H W G Q G T

L V T V S S A S T K G P S V F P E A P S S K S T S G G T A A L G C L V

T D Y F P E P V T V S W N S G A L T S G V H T F P A V L E S S G L Y S

L S S V V T V P S S S L G T Q T Y I C N V N H K P S N T K V D K R V E

P K S C D K T H T C P P C P A P E A A G G P S V F L F P P K P K D T L

M I S R T P E V T C V V V S V S H E D P E V K F N W Y V D G V E V H N

A K T K P R E E Q Y N S T Y R V V S V L T V L H Q D W L N G K E Y K C

K V S N K A L P A P I E K T I S K A K G Q P R E P Q V Y V L P P S R D

E L T K N Q V S L L C L V K G F Y P S D I A V E W E S N G Q P E N N Y

L T W P P V L D S D G S F F L Y S K L T V D K S R W Q Q G N V F S C S

V M H E A L H N H Y T Q K S L S L S P G

(SEQ ID NO: 201 of the Sequence Listing)

[Figure 226]

ATGCGGCCCACCTGGGCATGGTGGCTGTTCCTGGTGCTGCTGCTGGCCCTGTG
GGCCCCCGCCCGGGGGGCCACCAGAATGACACAGTCTCCAAGCTCCTTTTCCG
CCTCTACAGGCGACAGAGTGACCATCACATGCAAGGCCAGCCAGTCCGTGGGC
ATCAACGTGGATTGGTACCAGCAGAAGCCTGGCAAGTCCCCCAAGCTGCTGAT
CTATGGGGCCAGCAATAGGCACACCGGCGTGCCTTCTCGCTTCTCTGGCAGCG
GCTTCGGCAGGGACTTTACCCTGACAATCTCTAGCCTGCAGAGCGAGGATTTC
GCCACCTACTATTGTCTGCAGCACGGCTCCATCCCCCCTACCTTTGGCGGAGG
CACAAAGGTGGAGATCAAGAGAACAGTGGCGGCGCCCAGTGTCTTCATTTTTC
CCCCTAGCGACGAAGAGCTGAAGTCTGGGACAGCCAGTGTGGACTGTTGGCTG
AACAACTTCTACCCTAGAGAGGCTAAAGTGCAGTGGAAGGTCGATAACGCACT
GCAGTCCGGAAATTCTCAGGAGAGTGTGACTGAACAGGACTCAAAAGATAGCA
CCTATTCCCTGTCAAGCACACTGACTCTGAGCAAGGCCGACTACGAGAAGCAT
AAAGTGTATGCTTGTGAAGTCACCCACCAGGGGCTGAGTTCACCAGTCACAAA
ATCATTCAACAGAGGGGAGTGC

(SEQ ID NO: 202 of the Sequence Listing)

[Figure 227]

M R P T W A W W L F L V L L L A L W A P A R G A T R M T Q S P S S F S

A S T G D R V T I T C K A S Q S V G I N V D W Y Q Q K P G K S P K L L

I Y G A S N R H T G V P S R F S G S G F G R D F T L T I S S L Q S E D

F A T Y Y C L Q H G S I P P T F G G G T K V E I K R T V A A P S V F I

F P P S D E E L K S G T A S V D C W L N N F Y P R E A K V Q W K V D N

A L Q S G N S Q E S V T E Q D S K D S T Y S L S S T L T L S K A D Y E

K H K V Y A C E V T H Q G L S S P V T K S F N R G E C

(SEQ ID NO: 203 of the Sequence Listing)

[Figure 228]

ATGCGGCCCACCTGGGCATGGTGGCTGTTCCTGGTGCTGCTGCTGGCCCTGTG
GGCCCCCGCCCGGGGGGCCACCAGAATGACACAGTCTCCAAGCTCCTTTTCCG
CCTCTACAGGCGACAGAGTGACCATCACATGCAAGGCCAGCCAGTCCGTGGGC
ATCAACGTGGATTGGTACCAGCAGAAGCCTGGCAAGTCCCCCAAGCTGCTGAT
CTATGGGGCCAGCAATAGGCACACCGGCGTGCCTTCTCGCTTCTCTGGCAGCG
GCTTCGGCAGGGACTTTACCCTGACAATCTCTAGCCTGCAGAGCGAGGATTTC
GCCACCTACTATTGTCTGCAGCACGGCTCCATCCCCCCTACCTTTGGCGGAGG
CACAAAGGTGGAGATCAAGAGAACAGTGGCGGCGCCCAGTGTCTTCATTTTTC
CCCCTAGCGACGAACAGCTGAAGTCTGGGACAGCCAGAGTGGGCTGTTGGCTG
AACAACTTCTACCCTAGAGAGGCTAAAGTGCAGTGGAAGGTCGATAACGCACT
GCAGTCCGGAAATTCTCAGGAGAGTGTGACTGAACAGGACTCAAAAGATAGCA
CCTATTCCCTGAGAAGCACACTGACTCTGAGCAAGGCCGACTACGAGAAGCAT
AAAGTGTATGCTTGTGAAGTCACCCACCAGGGGCTGAGTTCACCAGTCACAAA
ATCATTCAACAGAGGGGAGTGC

(SEQ ID NO: 204of the Sequence Listing)

[Figure 229]

MRPTWAWWLFLVLLLALWAPARGATRMTQSPSSFS
ASTGDRVTITCKASQSVGINVDWYQQKPGKSPKLL
IYGASNRHTGVPSRFSGSGFGRDFTLTISSLQSED
FATYYCLQHGSIPPTFGGGTKVEIKRTVAAPSVFI
FPPSDEQLKSGTARVGCWLNNFYPREAKVQWKVDN
ALQSGNSQESVTEQDSKDSTYSLRSTLTLSKADYE
KHKVYACEVTHQGLSSPVTKSFNRGEC

(SEQ ID NO: 205 of the Sequence Listing)

[Figure 230]

ATGCGCCCCACCTGGGCATGGTGGCTGTTCCTGGTGCTGCTGCTGGCCCTGTG
GGCCCCTGCCCGGGGCGGCGAGGTGCAGCTGGTGGAGAGCGGCGGCGGCCTGG
TGCAGCCCGGCGGCTCTCTGCGGCTGAGCTGCGCCGCCTCCGGCGTGACATTC
AACTACTATGGCATGTCCTGGATCAGACAGGCCCCAGGCAAGGGCCTGGAGTG
GGTGGCCTCTATCACCAATAGCGGCGGCAGGATCTACTATCCCGACTCCGTGA
AGGGCCGGTTTACAATCTCTAGAGAGAACACCCAGAAGACACTGTACCTGCAG
ATGAACAGCCTGCGGGCCGAGGATACCGCCGTGTACTATTGTACACTGGACGG
CAGAGACGGATGGGTGGCATATTGGGGACAGGGCACCCTGGTGACAGTGAGCT
CCGCTAGCACTAAAGGCCCAAGCGTGTTTCCTCTGGCTCCAAGCTCAAAAAGC
ACTTCCGGGGGGACCGCATGGCTGGGATGTGATGTGACCGACTACTTCCCCGA
GCCTGTGACCGTGTCCTGGAACTCTGGGGCCCTGACCAGCGGAGTGCACACAT
TTCCAGCCGTGCTGCAGAGCTCCGGCCTGTATAGCCTGTCTAGCGTGGTGACA
GTGCCCTCCAGTAGCCTGGGCACTCAGACTTATATCTGTAATGTCAACCACAA
ACCATCAAACACCAAAGTCGACAAGAAAGTGGAGCCCAAGAGCTGTGATAAAA
CTCATACCTGCCCACCTTGTCCGGCGCCAGAGGCTGCAGGAGGACCAAGCGTG
TTCCTGTTTCCACCCAAGCCTAAAGACACACTGATGATTTCCCGAACCCCCGA
AGTCACATGCGTGGTCGTGTCTGTGAGTCACGAGGACCCTGAAGTCAAGTTCA
ACTGGTACGTGGATGGCGTCGAGGTGCATAATGCCAAGACTAAACCTAGGGAG
GAACAGTACAACTCAACCTATCGCGTCGTGAGCGTCCTGACAGTGCTGCACCA
GGATTGGCTGAACGGCAAAGAATATAAGTGCAAAGTGAGCAATAAGGCCCTGC
CCGCTCCTATCGAGAAAACCATTTCCAAGGCTAAAGGGCAGCCTCGCGAACCA
CAGGTCTACGTCTACCCCCCATCAAGAGATGAACTGACAAAAAATCAGGTCTC
TCTGACATGCCTGGTCAAAGGATTCTACCCTTCCGACATCGCCGTGGAGTGGG
AAAGTAACGGCCAGCCCGAGAACAATTACAAGACCACACCCCCTGTCCTGGAC
TCTGATGGGAGTTTCGCTCTGGTGTCAAAGCTGACCGTCGATAAAAGCCGGTG
GCAGCAGGGCAATGTGTTTAGCTGCTCCGTCATGCACGAAGCCCTGCACAATC
ACTACACACAGAAGTCCCTGAGCCTGAGCCCTGGC

(SEQ ID NO: 206 of the Sequence Listing)

[Figure 231]

M R P T W A W W L F L V L L L A L W A P A R G G E V Q L V E S G G G L

V Q P G G S L R L S C A A S G V T F N Y Y G M S W I R Q A P G K G L E

W V A S I T N S G G R I Y Y P D S V K G R F T I S R E N T Q K T L Y L

Q M N S L R A E D T A V Y Y C T L D G R D G W V A Y W G Q G T L V T V

S S A S T K G P S V F P L A P S S K S T S G G T A W L G C D V T D Y F

P E P V T V S W N S G A L T S G V H T F P A V L Q S S G L Y S L S S V

V T V P S S S L G T Q T Y I C N V N H K P S N T K V D K K V E P K S C

D K T H T C P P C P A P E A A G G P S V F L F P P K P K D T L M I S R

T P E V T C V V V S V S H E D P E V K F N W Y V D G V E V H N A K T K

P R E E Q Y N S T Y R V V S V L T V L H Q D W L N G K E Y K C K V S N

K A L P A P I E K T I S K A K G Q P R E P Q V Y V Y P P S R D E L T K

N Q V S L T C L V K G F Y P S D I A V E W E S N G Q P E N N Y K T T P

P V L D S D G S F A L V S K L T V D K S R W Q Q G N V F S C S V M H E

A L H N H Y T Q K S L S L S P G

(SEQ ID NO: 207 of the Sequence Listing)

[Figure 232]

ATGCGGCCTACCTGGGCCTGGTGGCTGTTCCTGGTGCTGCTGCTGGCCCTGTG
GGCCCCAGCCAGGGGCAACTTTATGCTGACCCAGCCCCACAGCGTGTCCGAGT
CTCCTGGCAAGACCGTGACAATCTCCTGCAAGCGGAACACAGGCAATATCGGC
TCTAACTACGTGAATTGGTATCAGCAGCACGAGGGCAGCTCCCCAACCACAAT
CATCTACCGGGACGATAAGCGGCCCGACGGCGTGTCTGATAGGTTCAGCGGCT
CCATCGACCGCTCTAGCAAGTCTGCCAGCCTGACCATCAGCAATCTGAAGACA
GAGGACGAGGCCGATTACTTTTGTCAGTCCTATTCCTCTGGCTTCATCTTTGG
AGGAGGAACCAAGCTGACAGTGCTGGGCCAGCCCAAGGCGGCGCCCAGCGTGA
CTCTGTTTCCACCCAGCTCCGAGCAGCTGCAGGCCAATAAGGCTAGACTGGTC
TGTCTGATTTCCGACTTCTACCCCGGGGCTGTGACAGTCGCATGGAAGGCCGA
TTCTAGTCCTGTGAAAGCAGGAGTCGAGACCACAACTCCATCAAAGCAGAGCA
ACAACAAGTACGCAGCCTCAAGCTATCTGTCTCTGACACCTGAACAGTGGAAA
AGCCACCGGTCTTATAGTTGTCAGGTGACTCACGAGGGCTCAACAGTGGAAAA
GACAGTCGCACCCGCAGAATGCTCA

(SEQ ID NO: 208 of the Sequence Listing)

[Figure 233]

MRPTWAWWLFLVLLLALWAPARGNFMLTQPHSVSESPGKTVTISCKRNTGNIG
SNYVNWYQQHEGSSPTTIIYRDDKRPDGVSDRFSGSIDRSSKSASLTISNLKT
EDEADYFCQSYSSGFIFGGGTKLTVLGQPKAAPSVTLFPPSSEQLQANKARLV
CLISDFYPGAVTVAWKADSSPVKAGVETTTPSKQSNNKYAASSYLSLTPEQWK
SHRSYSCQVTHEGSTVEKTVAPAECS

(SEQ ID NO: 209 of the Sequence Listing)

[Figure 234]

ATGCGCCCCACCTGGGCATGGTGGCTGTTCCTGGTGCTGCTGCTGGCCCTGTG
GGCCCCTGCCCGGGGCGGCGAGGTGCAGCTGGTGGAGAGCGGCGGCGGCCTGG
TGCAGCCCGGCGGCTCTCTGCGGCTGAGCTGCGCCGCCTCCGGCGTGACATTC
AACTACTATGGCATGTCCTGGATCAGACAGGCCCCAGGCAAGGGCCTGGAGTG
GGTGGCCTCTATCACCAATAGCGGCGGCAGGATCTACTATCCCGACTCCGTGA
AGGGCCGGTTTACAATCTCTAGAGAGAACACCCAGAAGACACTGTACCTGCAG
ATGAACAGCCTGCGGGCCGAGGATACCGCCGTGTACTATTGTACACTGGACGG
CAGAGACGGATGGGTGGCATATTGGGGACAGGGCACCCTGGTGACAGTGAGCT
CCGCTAGCACTAAAGGACCAAGCGTGTTTCCACTGGCTCCATCATCCAAAAGC
ACAAGCGGCGGGACTGCCTGGCTGGGCTGTAAGGTGAAGGACTACTTCCCCGA
GCCTGTGACCGTGTCCTGGAACTCTGGGGCCCTGACCAGCGGAGTGCACACAT
TTCCAGCCGTGCTGCAGAGCTCCGGCCTGTATAGCCTGTCTAGCGTGGTGACA
GTGCCCTCCTCAAGCCTGGGCACTCAGACCTATATTTGTAATGTCAACCATAA
ACCTTCCAACACTAAAGTCGACAAGAAAGTGGAGCCCAAGAGCTGTGATAAAA
CTCATACCTGCCCACCTTGTCCGGCGCCAGAGGCTGCAGGAGGACCAAGCGTG
TTCCTGTTTCCACCCAAGCCTAAAGACACACTGATGATTTCCCGAACCCCCGA
AGTCACATGCGTGGTCGTGTCTGTGAGTCACGAGGACCCTGAAGTCAAGTTCA
ACTGGTACGTGGATGGCGTCGAGGTGCATAATGCCAAGACTAAACCTAGGGAG
GAACAGTACAACTCAACCTATCGCGTCGTGAGCGTCCTGACAGTGCTGCACCA
GGATTGGCTGAACGGCAAAGAATATAAGTGCAAAGTGAGCAATAAGGCCCTGC
CCGCTCCTATCGAGAAAACCATTTCCAAGGCTAAAGGGCAGCCTCGCGAACCA
CAGGTCTACGTCTACCCCCCATCAAGAGATGAACTGACAAAAAATCAGGTCTC
TCTGACATGCCTGGTCAAAGGATTCTACCCTTCCGACATCGCCGTGGAGTGGG
AAAGTAACGGCCAGCCCGAGAACAATTACAAGACCACACCCCCTGTCCTGGAC
TCTGATGGGAGTTTCGCTCTGGTGTCAAAGCTGACCGTCGATAAAAGCCGGTG
GCAGCAGGGCAATGTGTTTAGCTGCTCCGTCATGCACGAAGCCCTGCACAATC
ACTACACACAGAAGTCCCTGAGCCTGAGCCCTGGC

(SEQ ID NO: 210 of the Sequence Listing)

[Figure 235]

M R P T W A W W L F L V L L L A L W A P A R G G E V Q L V E S G G G L

V Q P G G S L R L S C A A S G V T F N Y Y G M S W I R Q A P G K G L E

W V A S I T N S G G R I Y Y P D S V K G R F T I S R E N T Q K T L Y L

Q M N S L R A E D T A V Y Y C T L D G R D G W V A Y W G Q G T L V T V

S S A S T K G P S V F P L A P S S K S T S G G T A W L G C K V K D Y F

P E P V T V S W N S G A L T S G V H T F P A V L Q S S G L Y S L S S V

V T V P S S S L G T Q T Y I C N V N H K P S N T K V D K K V E P K S C

D K T H T C P P C P A P E A A G G P S V F L F P P K P K D T L M I S R

T P E V T C V V V S V S H E D P E V K F N W Y V D G V E V H N A K T K

P R E E Q Y N S T Y R V V S V L T V L H Q D W L N G K E Y K C K V S N

K A L P A P I E K T I S K A K G Q P R E P Q V Y V Y P P S R D E L T K

N Q V S L T C L V K G F Y P S D I A V E W E S N G Q P E N N Y K T T P

P V L D S D G S F A L V S K L T V D K S R W Q Q G N V F S C S V M H E

A L H N H Y T Q K S L S L S P G

(SEQ ID NO: 211 of the Sequence Listing)

[Figure 236]

ATGCGGCCCACCTGGGCATGGTGGCTGTTCCTGGTGCTGCTGCTGGCCCTGTG
GGCCCCTGCCAGGGGCAACTTTATGCTGACCCAGCCTAGCTCCGTGAGCGGAG
TGCCAGGACAGAGGGTGACAATCTCCTGCACCGGCAACACAGGCAATATCGGC
TCTAACTACGTGAATTGGTATCAGCAGCTGCCCGGCACAGCCCCTAAGCTGCT
GATCTACCGGGACGATAAGCGGCCCAGCGGAGTGCCAGACAGGTTCTCCGGCT
CTAAGAGCGGCACCTCCGCCTCTCTGGCCATCACAGGCTTTCAGGCCGAGGAC
GAGGCCGATTACTATTGTCAGTCCTATTCTAGCGGCTTCATCTTTGGAGGAGG
AACCAAGCTGACAGTGCTGGGCCAGCCCAAGGCGGCGCCCAGTGTCACACTGT
TTCCCCCTAGCTCCGAGGAACTGCAGGCTAACACCGCAACACTGGACTGTCTG
ATCAGCGACTTCTACCCTGGAGCTGTGACTGTCGCCTGGAAGGCTGATTCTAG
TCCAGTGAAAGCAGGCGTCGAGACCACAACTCCCTCTAAGCAGAGTAACAACA
AGTACGCAGCCTCAAGCTATCTGTCACTGACCCCAGAACAGTGGAAGAGCCAC
CGGAGCTATTCCTGCCAGGTCACTCACGAAGGCTCCACTGTCGAGAAAACCGT
CGCTCCCACCGAATGTTCA

(SEQ ID NO: 212 of the Sequence Listing)

[Figure 237]

M R P T W A W W L F L V L L L A L W A P A R G N F M L T Q P S S V S G

V P G Q R V T I S C T G N T G N I G S N Y V N W Y Q Q L P G T A P K L

L I Y R D D K R P S G V P D R F S G S K S G T S A S L A I T G F Q A E

D E A D Y Y C Q S Y S S G F I F G G G T K L T V L G Q P K A A P S V T

L F P P S S E E L Q A N T A T L D C L I S D F Y P G A V T V A W K A D

S S P V K A G V E T T T P S K Q S N N K Y A A S S Y L S L T P E Q W K

S H R S Y S C Q V T H E G S T V E K T V A P T E C S

(SEQ ID NO: 213 of the Sequence Listing)

[Figure 238]

ATGAGGCCTACCTGGGCCTGGTGGCTGTTCCTGGTGCTGCTGCTGGCCCTGTG
GGCCCCCGCCCGGGGCCAGGTGCAGCTGGTGGAGTCCGGCGGCGGCCTGGTGA
AGCCCAGCCAGACCCTGTCCCTGACCTGCACATTCAGCGGCTTTTCCCTGAAC
ACATACGATATGGGAGTGGGATGGATCAGACAGCCACCTGGCAAGGGCCTGGA
GTGGATCGGCAACATCTGGTGGGACGATGACAAGTACTATAATCCCTCTCTGA
AGAGCAGAGTGACCATCTCTAAGGATACAAGCAACAATCAGGCCTCCCTGAAG
CTGAGCTCCGTGACCGCCGCCGACACAGCCGTGTACTATTGCGCCAGGATCGA
GACAGTGCGGGTGAGCCGGAAGGGATTCGCACACTGGGGACAGGGCACCCTGG
TGACAGTGTCTAGCGCTAGCACAAAGGGACCTAGCGTGTTCCCACTGGCCCCT
TCCTCTAAGTCCACCTCTGGAGGAACAGCCGCCCTGGGCTGTCTGGTGAAGGA
CTATTTCCCTGAGCCAGTGACCGTGTCCTGGAACTCTGGGGCCCTGACCAGCG
GAGTGCACACATTTCCTGCCGTGCTGCAGAGCTCCGGCCTGTACAGCCTGTCT
AGCGTGGTGACCGTGCCATCCTCTAGCCTGGGCACCCAGACATATATCTGCAA
CGTGAATCACAAGCCAAGCAATACAAAGGTCGACAAGAGAGTGGAGCCCAAGT
CCTGTGACAAGACCCACACATGCCCACCCTGTCCGGCGCCAGAGGCTGCAGGA
GGACCAAGCGTGTTCCTGTTTCCACCCAAGCCTAAAGACACACTGATGATTTC
CCGAACCCCCGAAGTCACATGCGTGGTCGTGTCTGTGAGTCACGAGGACCCTG
AAGTCAAGTTCAACTGGTACGTGGATGGCGTCGAGGTGCATAATGCCAAGACT
AAACCTAGGGAGGAACAGTACAACTCAACCTATCGCGTCGTGAGCGTCCTGAC
AGTGCTGCACCAGGATTGGCTGAACGGCAAAGAATATAAGTGCAAAGTGAGCA
ATAAGGCCCTGCCCGCTCCTATCGAGAAAACCATTTCCAAGGCTAAAGGGCAG
CCTCGCGAACCACAGGTCTACGTGCTGCCCCCTAGCCGCGACGAACTGACTAA
AAATCAGGTCTCTCTGCTGTGTCTGGTCAAAGGATTCTACCCTTCCGACATCG
CCGTGGAGTGGGAAAGTAACGGCCAGCCCGAGAACAATTACCTGACCTGGCCC
CCTGTGCTGGACTCTGATGGGAGTTTCTTTCTGTATTCAAAGCTGACAGTCGA
TAAAAGCCGGTGGCAGCAGGGCAATGTGTTCAGCTGCTCCGTCATGCACGAAG
CACTGCACAACCATTACACTCAGAAGTCCCTGTCCCTGTCACCTGGC

(SEQ ID NO: 214 of the Sequence Listing)

[Figure 239]

M R P T W A W W L F L V L L L A L W A P A R G Q V Q L V E S G G G L V

K P S Q T L S L T C T F S G F S L N T Y D M G V G W I R Q P P G K G L

E W I G N I W W D D D K Y Y N P S L K S R V T I S K D T S N N Q A S L

K L S S V T A A D T A V Y Y C A R I E T V R V S R K G F A H W G Q G T

L V T V S S A S T K G P S V F P L A P S S K S T S G G T A A L G C L V

K D Y F P E P V T V S W N S G A L T S G V H T F P A V L Q S S G L Y S

L S S V V T V P S S S L G T Q T Y I C N V N H K P S N T K V D K R V E

P K S C D K T H T C P P C P A P E A A G G P S V F L F P P K P K D T L

M I S R T P E V T C V V V S V S H E D P E V K F N W Y V D G V E V H N

A K T K P R E E Q Y N S T Y R V V S V L T V L H Q D W L N G K E Y K C

K V S N K A L P A P I E K T I S K A K G Q P R E P Q V Y V L P P S R D

E L T K N Q V S L L C L V K G F Y P S D I A V E W E S N G Q P E N N Y

L T W P P V L D S D G S F F L Y S K L T V D K S R W Q Q G N V F S C S

V M H E A L H N H Y T Q K S L S L S P G

(SEQ ID NO: 215 of the Sequence Listing)

[Figure 240]

ATGCGGCCCACCTGGGCATGGTGGCTGTTCCTGGTGCTGCTGCTGGCCCTGTG
GGCCCCCGCCCGGGGGGCCACCAGAATGACACAGTCTCCAAGCTCCTTTTCCG
CCTCTACAGGCGACAGAGTGACCATCACATGCAAGGCCAGCCAGTCCGTGGGC
ATCAACGTGGATTGGTACCAGCAGAAGCCTGGCAAGTCCCCCAAGCTGCTGAT
CTATGGGGCCAGCAATAGGCACACCGGCGTGCCTTCTCGCTTCTCTGGCAGCG
GCTTCGGCAGGGACTTTACCCTGACAATCTCTAGCCTGCAGAGCGAGGATTTC
GCCACCTACTATTGTCTGCAGCACGGCTCCATCCCCCCTACCTTTGGCGGAGG
CACAAAGGTGGAGATCAAGAGAACAGTGGCGGCGCCCAGTGTCTTCATTTTTC
CCCCTAGCGACGAACAGCTGAAGTCTGGGACAGCCAGTGTGGTCTGTCTGCTG
AACAACTTCTACCCTAGAGAGGCTAAAGTGCAGTGGAAGGTCGATAACGCACT
GCAGTCCGGAAATTCTCAGGAGAGTGTGACTGAACAGGACTCAAAAGATAGCA
CCTATTCCCTGTCAAGCACACTGACTCTGAGCAAGGCCGACTACGAGAAGCAT
AAAGTGTATGCTTGTGAAGTCACCCACCAGGGGCTGAGTTCACCAGTCACAAA
ATCATTCAACAGAGGGGAGTGC

(SEQ ID NO: 216 of the Sequence Listing)

[Figure 241]

MRPTWAWWLFLVLLLALWAPARGATRMTQSPSSFSASTGDRVTITCKASQSVG
INVDWYQQKPGKSPKLLIYGASNRHTGVPSRFSGSGFGRDFTLTISSLQSEDF
ATYYCLQHGSIPPTFGGGTKVEIKRTVAAPSVFIFPPSDEQLKSGTASVVCLL
NNFYPREAKVQWKVDNALQSGNSQESVTEQDSKDSTYSLSSTLTLSKADYEKH
KVYACEVTHQGLSSPVTKSFNRGEC

(SEQ ID NO: 217 of the Sequence Listing)

[Figure 242]

ATGCGCCCTACCTGGGCCTGGTGGCTGTTCCTGGTGCTGCTGCTGGCCCTGTG
GGCCCCAGCCCGGGGCGGCGAGGTGCAGCTGGTGGAGTCTGGCGGCGGCCTGG
TGCAGCCCGGCGGCAGCCTGCGGCTGTCCTGCGCCGCCTCTGGCGTGACCTTC
AACTACTATGGCATGAGCTGGATCAGACAGGCCCCCGGCAAGGGCCTGGAGTG
GGTGGCCAGCATCACCAATTCCGGCGGCAGGATCTACTATCCTGACTCTGTGA
AGGGCAGGTTTACAATCAGCCGCGAGAACACCCAGAAGACACTGTACCTGCAG
ATGAACAGCCTGCGGGCCGAGGATACCGCCGTGTACTATTGCACACTGGACGG
CAGAGACGGATGGGTGGCATATTGGGGACAGGGCACCCTGGTGACAGTGAGCT
CCGGCGGCGGCGGCTCTGGAGGAGGAGGCAGCGGCGGAGGAGGCTCCAACTTT
ATGCTGACCCAGCCCCACTCTGTGAGCGAGTCCCCTGGCAAGACCGTGACAAT
CTCCTGTAAGAGAAACACAGGCAATATCGGCTCTAACTACGTGAATTGGTATC
AGCAGCACGAGGGCTCTAGCCCAACCACAATCATCTACCGGGACGATAAGCGG
CCCGACGGCGTGAGCGATCGGTTCTCTGGCAGCATCGACAGATCCTCTAAGTC
CGCCTCTCTGACCATCAGCAATCTGAAGACAGAGGACGAGGCCGATTACTTTT
GTCAGTCCTATAGCTCCGGCTTCATCTTTGGCGGCGGCACCAAGCTGACAGTG
CTGGCGGCCGAGCCTAAAAGTAGCGATAAAACCCATACCTGCCCCCCCTGCCC
GGCGCCAGAGGCTGCAGGAGGACCAAGCGTGTTCCTGTTTCCACCCAAGCCTA
AAGACACACTGATGATTTCCCGAACCCCCGAAGTCACATGCGTGGTCGTGTCT
GTGAGTCACGAGGACCCTGAAGTCAAGTTCAACTGGTACGTGGATGGCGTCGA
GGTGCATAATGCCAAGACTAAACCTAGGGAGGAACAGTACAACTCAACCTATC
GCGTCGTGAGCGTCCTGACAGTGCTGCACCAGGATTGGCTGAACGGCAAAGAA
TATAAGTGCAAAGTGAGCAATAAGGCCCTGCCCGCTCCTATCGAGAAAACCAT
TTCCAAGGCTAAAGGGCAGCCTCGCGAACCACAGGTCTACGTCTACCCCCCAT
CAAGAGATGAACTGACAAAAAATCAGGTCTCTCTGACATGCCTGGTCAAAGGA
TTCTACCCTTCCGACATCGCCGTGGAGTGGGAAAGTAACGGCCAGCCCGAGAA
CAATTACAAGACCACACCCCCTGTCCTGGACTCTGATGGGAGTTTCGCTCTGG
TGTCAAAGCTGACCGTCGATAAAAGCCGGTGGCAGCAGGGCAATGTGTTTAGC
TGCTCCGTCATGCACGAAGCCCTGCACAATCACTACACACAGAAGTCCCTGAG
CCTGAGCCCTGGC

(SEQ ID NO: 218 of the Sequence Listing)

[Figure 243]

M R P T W A W W L F L V L L L A L W A P A R G G E V Q L V E S G G G L

V Q P G G S L R L S C A A S G V T F N Y Y G M S W I R Q A P G K G L E

W V A S I T N S G G R I Y Y P D S V K G R F T I S R E N T Q K T L Y L

Q M N S L R A E D T A V Y Y C T L D G R D G W V A Y W G Q G T L V T V

S S G G G G S G G G G S G G G G S N F M L T Q P H S V S E S P G K T V

T I S C K R N T G N I G S N Y V N W Y Q Q H E G S S P T T I I Y R D D

K R P D G V S D R F S G S I D R S S K S A S L T I S N L K T E D E A D

Y F C Q S Y S S G F I F G G G T K L T V L A A E P K S S D K T H T C P

P C P A P E A A G G P S V F L F P P K P K D T L M I S R T P E V T C V

V V S V S H E D P E V K F N W Y V D G V E V H N A K T K P R E E Q Y N

S T Y R V V S V L T V L H Q D W L N G K E Y K C K V S N K A L P A P I

E K T I S K A K G Q P R E P Q V Y V Y P P S R D E L T K N Q V S L T C

L V K G F Y P S D I A V E W E S N G Q P E N N Y K T T P P V L D S D G

S F A L V S K L T V D K S R W Q Q G N V F S C S V M H E A L H N H Y T

Q K S L S L S P G

(SEQ ID NO: 219 of the Sequence Listing)

[Figure 244]

ATGCGGCCCACCTGGGCATGGTGGCTGTTCCTGGTGCTGCTGCTGGCCCTGTG
GGCCCCTGCCAGGGGCGGCGAGGTGCAGCTGGTGGAGAGCGGCGGCGGCCTGG
TGCAGCCTGGAGGCTCTCTGAGGCTGAGCTGCGCAGCCTCCGGCGTGACATTC
AACTACTATGGCATGTCCTGGATCAGACAGGCCCCAGGCAAGGGCCTGGAGTG
GGTGGCCTCTATCACCAATAGCGGCGGCAGAATCTACTATCCCGACTCCGTGA
AGGGCAGGTTTACAATCAGCCGGGAGAACACCCAGAAGACACTGTACCTGCAG
ATGAATAGCCTGCGGGCCGAGGATACCGCCGTGTACTATTGCACACTGGACGG
CAGAGATGGATGGGTGGCATATTGGGGACAGGGCACCCTGGTGACAGTGAGCT
CCGGAGGAGGAGGCTCCGGCGGCGGAGGCTCTGGCGGCGGCGGCAGCAACTTT
ATGCTGACCCAGCCTTCTAGCGTGAGCGGAGTGCCAGGACAGAGGGTGACAAT
CTCCTGTACCGGCAACACAGGCAATATCGGCTCTAACTACGTGAATTGGTATC
AGCAGCTGCCAGGAACCGCCCCTAAGCTGCTGATCTACCGGGACGATAAGCGG
CCCTCTGGAGTGCCAGACCGGTTCAGCGGCTCCAAGTCTGGCACCAGCGCCTC
CCTGGCCATCACAGGATTTCAGGCCGAGGACGAGGCCGATTACTATTGTCAGT
CCTATTCCTCTGGCTTCATCTTTGGCGGCGGCACCAAGCTGACAGTGCTGGCG
GCCGAGCCTAAAAGTAGCGATAAAACCCATACCTGCCCCCCCTGCCCGGCGCC
AGAGGCTGCAGGAGGACCAAGCGTGTTCCTGTTTCCACCCAAGCCTAAAGACA
CACTGATGATTTCCCGAACCCCCGAAGTCACATGCGTGGTCGTGTCTGTGAGT
CACGAGGACCCTGAAGTCAAGTTCAACTGGTACGTGGATGGCGTCGAGGTGCA
TAATGCCAAGACTAAACCTAGGGAGGAACAGTACAACTCAACCTATCGCGTCG
TGAGCGTCCTGACAGTGCTGCACCAGGATTGGCTGAACGGCAAAGAATATAAG
TGCAAAGTGAGCAATAAGGCCCTGCCCGCTCCTATCGAGAAAACCATTTCCAA
GGCTAAAGGGCAGCCTCGCGAACCACAGGTCTACGTCTACCCCCCATCAAGAG
ATGAACTGACAAAAAATCAGGTCTCTCTGACATGCCTGGTCAAAGGATTCTAC
CCTTCCGACATCGCCGTGGAGTGGGAAAGTAACGGCCAGCCCGAGAACAATTA
CAAGACCACACCCCCTGTCCTGGACTCTGATGGGAGTTTCGCTCTGGTGTCAA
AGCTGACCGTCGATAAAAGCCGGTGGCAGCAGGGCAATGTGTTTAGCTGCTCC
GTCATGCACGAAGCCCTGCACAATCACTACACACAGAAGTCCCTGAGCCTGAG
CCCTGGC

(SEQ ID NO: 220 of the Sequence Listing)

[Figure 245]

M R P T W A W W L F L V L L L A L W A P A R G G E V Q L V E S G G G L

V Q P G G S L R L S C A A S G V T F N Y Y G M S W I R Q A P G K G L E

W V A S I T N S G G R I Y Y P D S V K G R F T I S R E N T Q K T L Y L

Q M N S L R A E D T A V Y Y C T L D G R D G W V A Y W G Q G T L V T V

S S G G G G S G G G G S G G G G S N F M L T Q P S S V S G V P G Q R V

T I S C T G N T G N I G S N Y V N W Y Q Q L P G T A P K L L I Y R D D

K R P S G V P D R F S G S K S G T S A S L A I T G F Q A E D E A D Y Y

C Q S Y S S G F I F G G G T K L T V L A A E P K S S D K T H T C P P C

P A P E A A G G P S V F L F P P K P K D T L M I S R T P E V T C V V V

S V S H E D P E V K F N W Y V D G V E V H N A K T K P R E E Q Y N S T

Y R V V S V L T V L H Q D W L N G K E Y K C K V S N K A L P A P I E K

T I S K A K G Q P R E P Q V Y V Y P P S R D E L T K N Q V S L T C L V

K G F Y P S D I A V E W E S N G Q P E N N Y K T T P P V L D S D G S F

A L V S K L T V D K S R W Q Q G N V F S C S V M H E A L H N H Y T Q K

S L S L S P G

(SEQ ID NO: 221 of the Sequence Listing)

[Figure 246]

A T G C G G C C T A C C T G G G C C T G G T G G C T G T T C C T G G T G C T G C T G C T G G C C C T G T G
G G C C C C C G C C C G G G G G G C C A C C C G C A T G A C A C A G T C C C C T A G C T C C T T T T C T G
C C A G C A C A G G C G A T A G A G T G A C C A T C A C A T G C A A G G C C T C C C A G T C T G T G G G C
A T C A A C G T G G A C T G G T A C C A G C A G A A G C C T G G C A A G A G C C C A A A G C T G C T G A T
C T A T G G G G C C A G C A A T A G G C A C A C C G G A G T G C C A T C T A G A T T C A G C G G C T C C G
G C T T C G G C A G A G A C T T C A C C C T G A C A A T C T C T A G C C T G C A G A G C G A G G A C T T C
G C C A C A T A C T A T T G C C T G C A G C A C G G C T C C A T C C C C C C T A C C T T T G G C G G A G G
C A C A A A G G T G G A G A T C A A G G G A G G A G G A G G C T C C G G C G G A G G A G G C T C T G G C G
G C G G C G G C A G C G G C G G C G G C C A G G T G C A G C T G G T G G A G T C C G G A G G A G G C C T G
G T G A A G C C A A G C C A G A C C C T G T C C C T G A C C T G T A C A T T C T C T G G C T T T A G C C T
G A A C A C A T A C G A T A T G G G A G T G G G A T G G A T C A G G C A G C C A C C A G G C A A G G G C C
T G G A G T G G A T C G G C A A C A T C T G G T G G G A C G A T G A C A A G T A C T A T A A T C C C T C C
C T G A A G T C T C G C G T G A C C A T C T C T A A G G A T A C A A G C A A C A A T C A G G C C T C T C T
G A A G C T G T C C T C T G T G A C C G C C G C C G A C A C A G C C G T G T A C T A T T G T G C A A G G A
T C G A G A C A G T G C G G G T G A G C C G G A A G G G A T T T G C A C A C T G G G G A C A G G G C A C C
C T G G T G A C A G T G A G C T C T G C G G C C G A G C C T A A A A G T A G C G A T A A A A C C C A T A C
C T G C C C C C C C T G C C C G G C G C C A G A G G C T G C A G G A G G A C C A A G C G T G T T C C T G T
T T C C A C C C A A G C C T A A A G A C A C A C T G A T G A T T T C C C G A A C C C C C G A A G T C A C A
T G C G T G G T C G T G T C T G T G A G T C A C G A G G A C C C T G A A G T C A A G T T C A A C T G G T A
C G T G G A T G G C G T C G A G G T G C A T A A T G C C A A G A C T A A A C C T A G G G A G G A A C A G T
A C A A C T C A A C C T A T C G C G T C G T G A G C G T C C T G A C A G T G C T G C A C C A G G A T T G G
C T G A A C G G C A A A G A A T A T A A G T G C A A A G T G A G C A A T A A G G C C C T G C C C G C T C C
T A T C G A G A A A A C C A T T T C C A A G G C T A A A G G G C A G C C T C G C G A A C C A C A G G T C T
A C G T G C T G C C C C C T A G C C G C G A C G A A C T G A C T A A A A A T C A G G T C T C T C T G C T G
T G T C T G G T C A A A G G A T T C T A C C C T T C C G A C A T C G C C G T G G A G T G G G A A A G T A A
C G G C C A G C C C G A G A A C A A T T A C C T G A C C T G G C C C C C T G T G C T G G A C T C T G A T G
G G A G T T T C T T T C T G T A T T C A A A G C T G A C A G T C G A T A A A A G C C G G T G G C A G C A G
G G C A A T G T G T T C A G C T G C T C C G T C A T G C A C G A A G C A C T G C A C A A C C A T T A C A C
T C A G A A G T C C C T G T C C C T G T C A C C T G G C

(SEQ ID NO: 222 of the Sequence Listing)

[Figure 247]

M R P T W A W W L F L V L L L A L W A P A R G A T R M T Q S P S S F S

A S T G D R V T I T C K A S Q S V G I N V D W Y Q Q K P G K S P K L L

I Y G A S N R H T G V P S R F S G S G F G R D F T L T I S S L Q S E D

F A T Y Y C L Q H G S I P P T F G G G T K V E I K G G G G S G G G G S

G G G G S G G G Q V Q L V E S G G G L V K P S Q T L S L T C T F S G F

S L N T Y D M G V G W I R Q P P G K G L E W I G N I W W D D D K Y Y N

P S L K S R V T I S K D T S N N Q A S L K L S S V T A A D T A V Y Y C

A R I E T V R V S R K G F A H W G Q G T L V T V S S A A E P K S S D K

T H T C P P C P A P E A A G G P S V F L F P P K P K D T L M I S R T P

E V T C V V V S V S H E D P E V K F N W Y V D G V E V H N A K T K P R

E E Q Y N S T Y R V V S V L T V L H Q D W L N G K E Y K C K V S N K A

L P A P I E K T I S K A K G Q P R E P Q V Y V L P P S R D E L T K N Q

V S L L C L V K G F Y P S D I A V E W E S N G Q P E N N Y L T W P P V

L D S D G S F F L Y S K L T V D K S R W Q Q G N V F S C S V M H E A L

H N H Y T Q K S L S L S P G

(SEQ ID NO: 223 of the Sequence Listing)

[Figure 248]

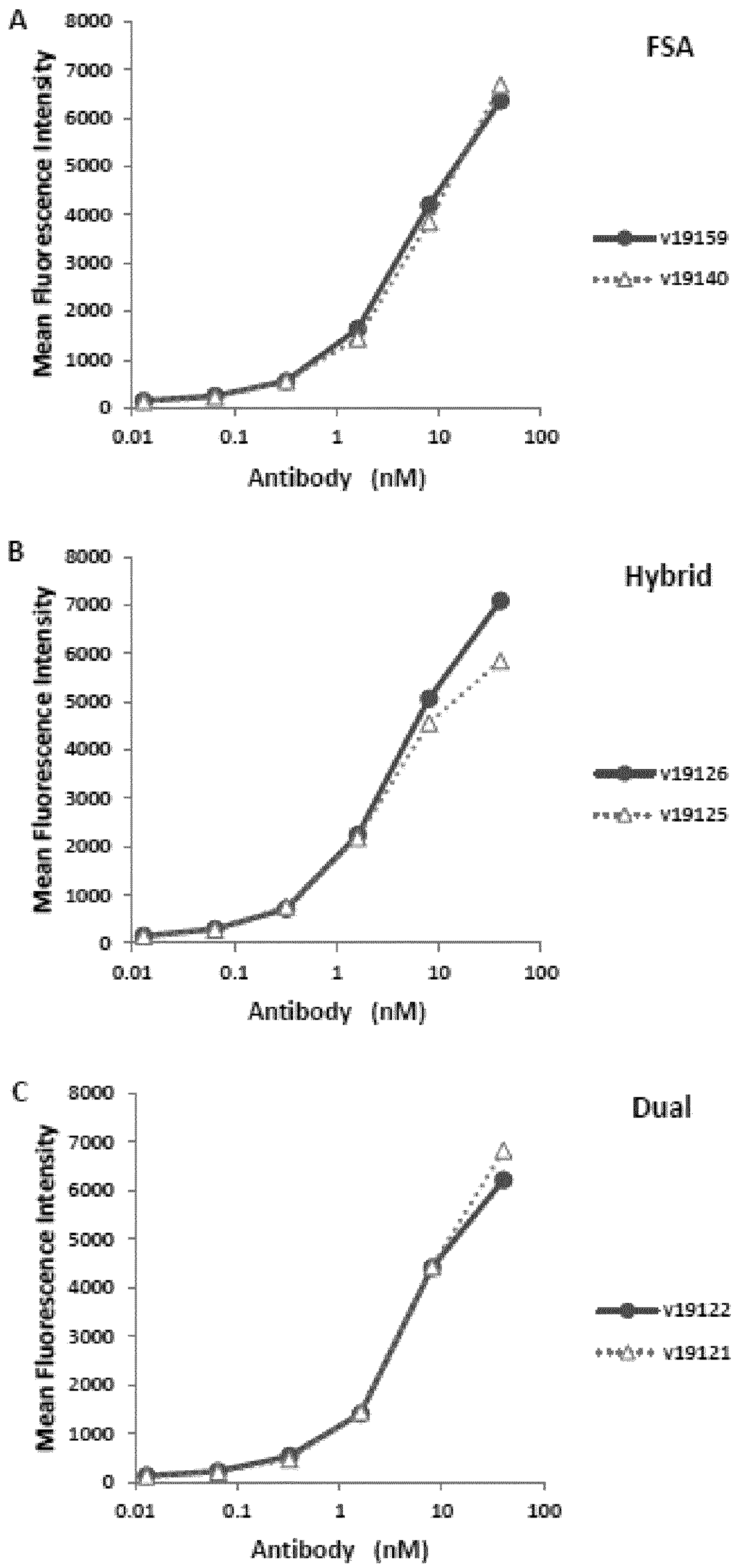
A
FSA
Mean Fluorescence Intensity
8000
7000
6000
5000
4000
3000
2000
1000
0
0.01
0.1
1
10
100
Antibody (nM)
v19159
v19140
B
Hybrid
Mean Fluorescence Intensity
Antibody (nM)
v19126
v19125
C
Dual
Mean Fluorescence Intensity
Antibody (nM)
v19122
v19121

[Figure 249]

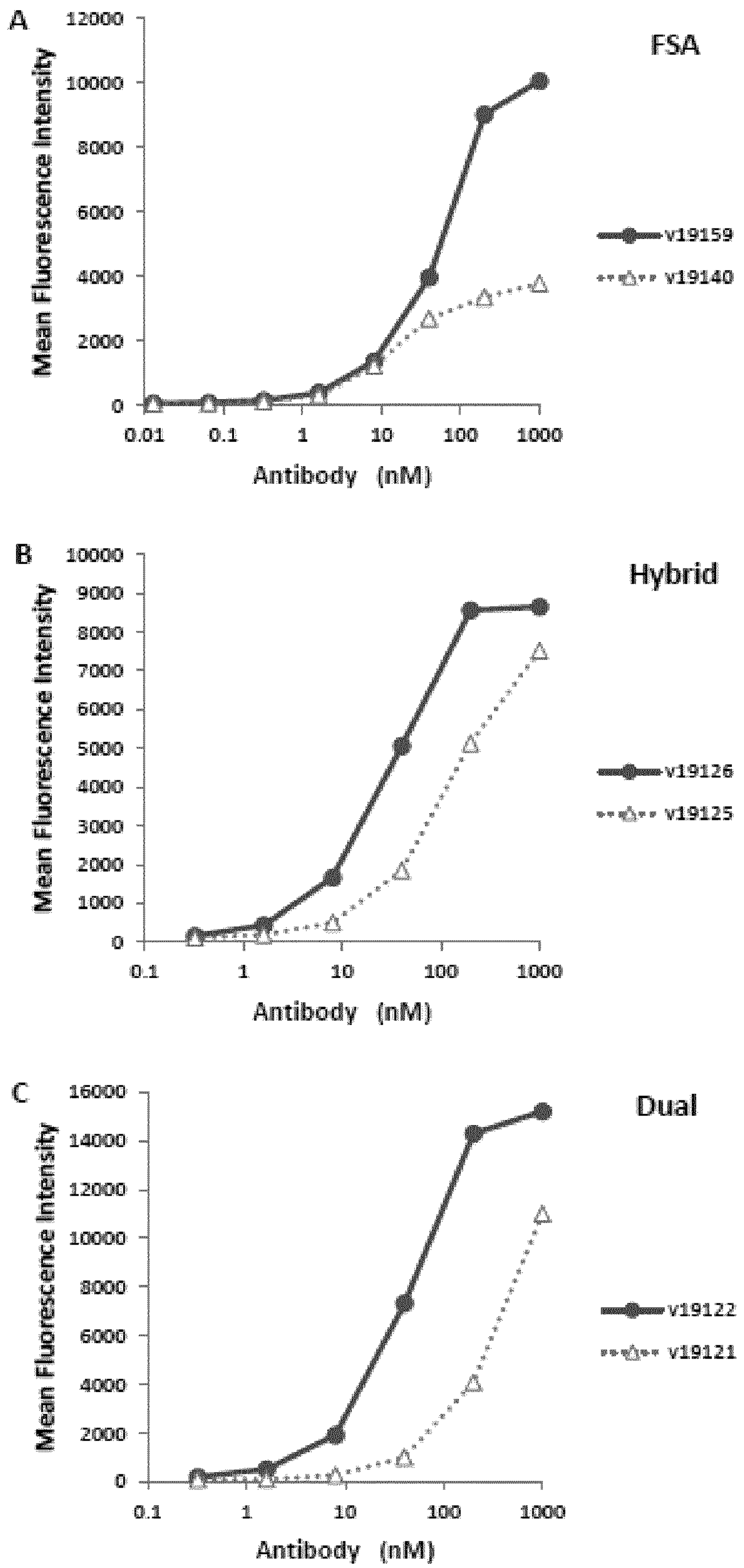
A
FSA
Mean Fluorescence Intensity
12000
10000
8000
6000
4000
2000
0
0.01
0.1
1
10
100
1000
Antibody (nM)
v19159
v19140
B
Hybrid
Mean Fluorescence Intensity
10000
9000
8000
7000
6000
5000
4000
3000
2000
1000
0
0.1
1
10
100
1000
Antibody (nM)
v19126
v19125
C
Dual
Mean Fluorescence Intensity
16000
14000
12000
10000
8000
6000
4000
2000
0
0.1
1
10
100
1000
Antibody (nM)
v19122
v19121

[Figure 250]

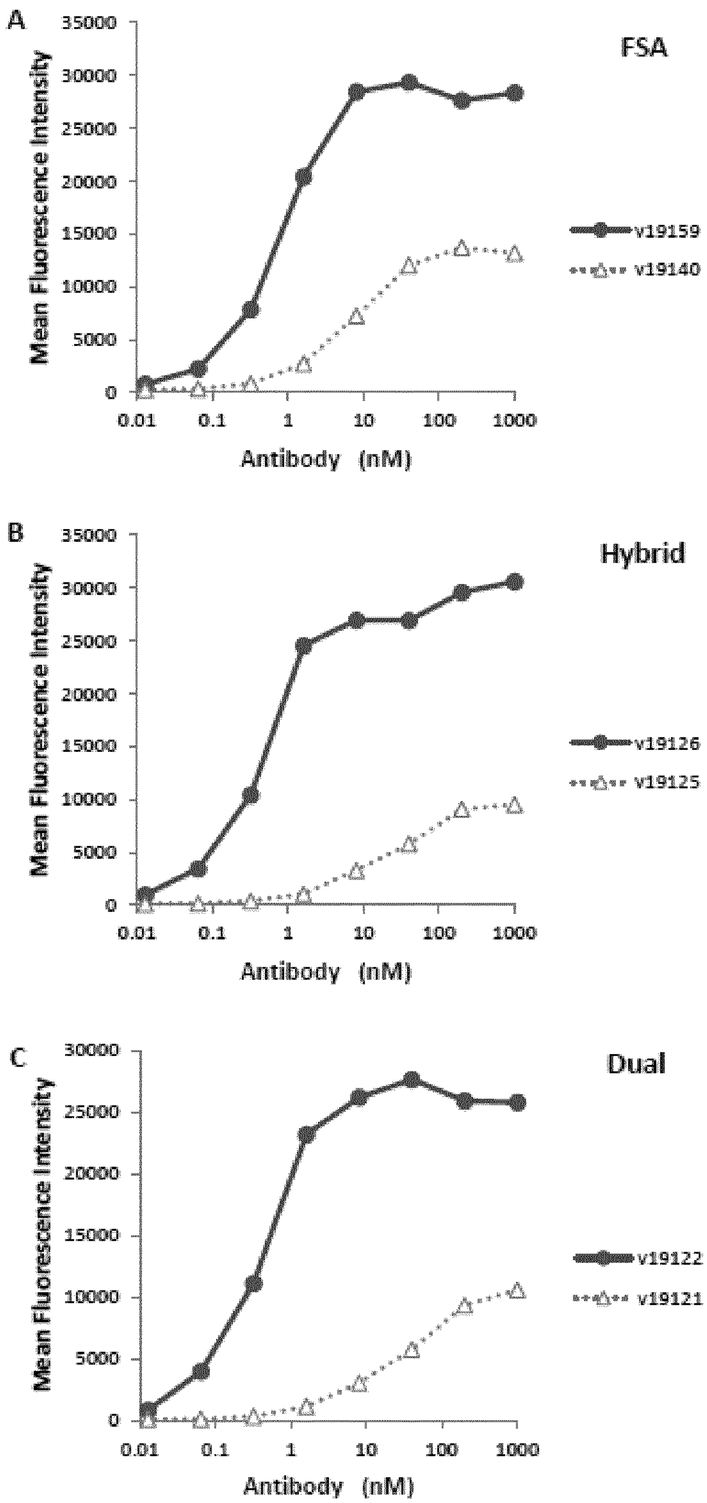
A
FSA
Mean Fluorescence Intensity
35000
30000
25000
20000
15000
10000
5000
0
0.01
0.1
1
10
100
1000
Antibody (nM)
v19159
v19140
B
Hybrid
Mean Fluorescence Intensity
35000
30000
25000
20000
15000
10000
5000
0
0.01
0.1
1
10
100
1000
Antibody (nM)
v19126
v19125
C
Dual
Mean Fluorescence Intensity
30000
25000
20000
15000
10000
5000
0
0.01
0.1
1
10
100
1000
Antibody (nM)
v19122
v19121

[Figure 251]

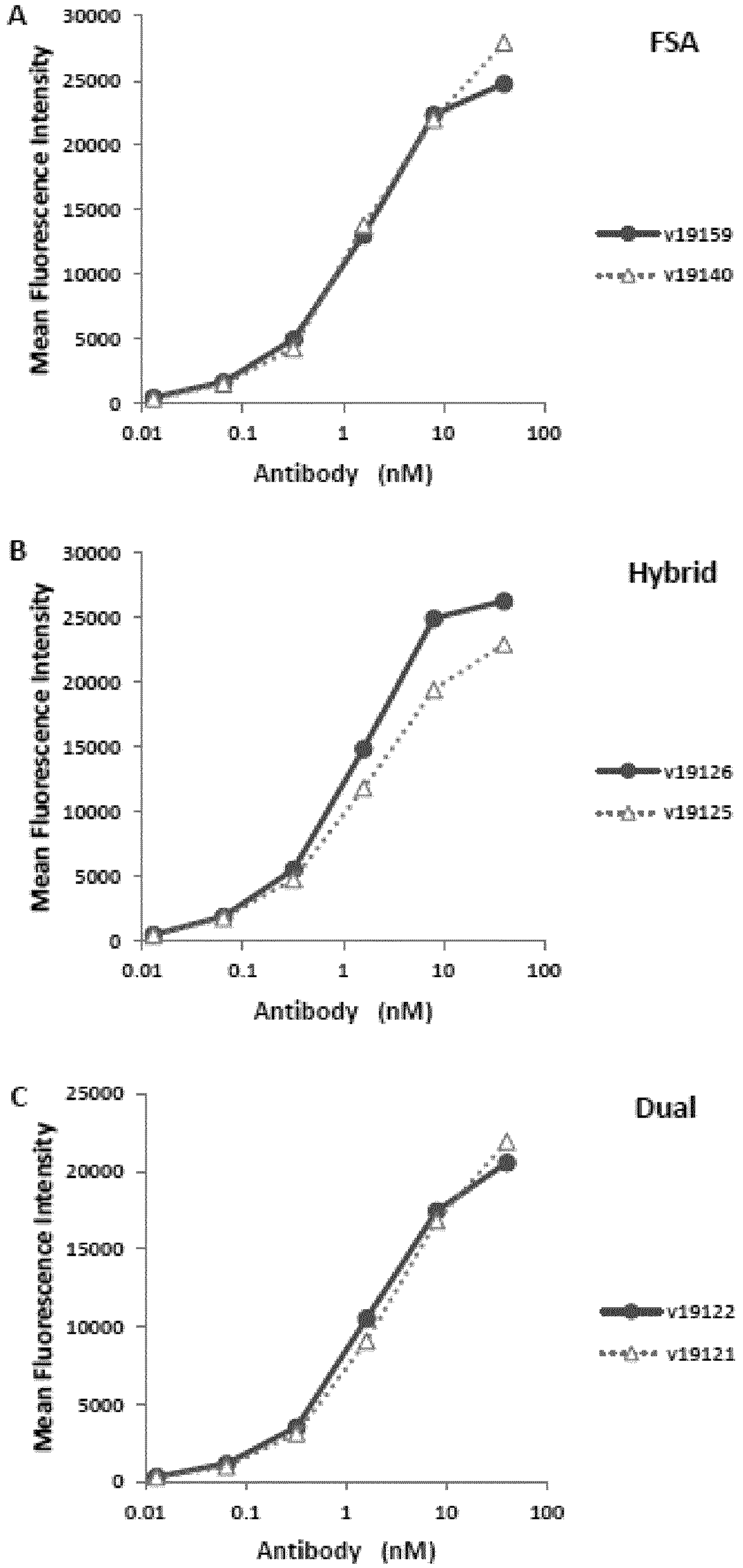
A
FSA
Mean Fluorescence Intensity
30000
25000
20000
15000
10000
5000
0
0.01
0.1
1
10
100
Antibody (nM)
v19159
v19140
B
Hybrid
Mean Fluorescence Intensity
30000
25000
20000
15000
10000
5000
0
0.01
0.1
1
10
100
Antibody (nM)
v19126
v19125
C
Dual
Mean Fluorescence Intensity
25000
20000
15000
10000
5000
0
0.01
0.1
1
10
100
Antibody (nM)
v19122
v19121

[Figure 252]

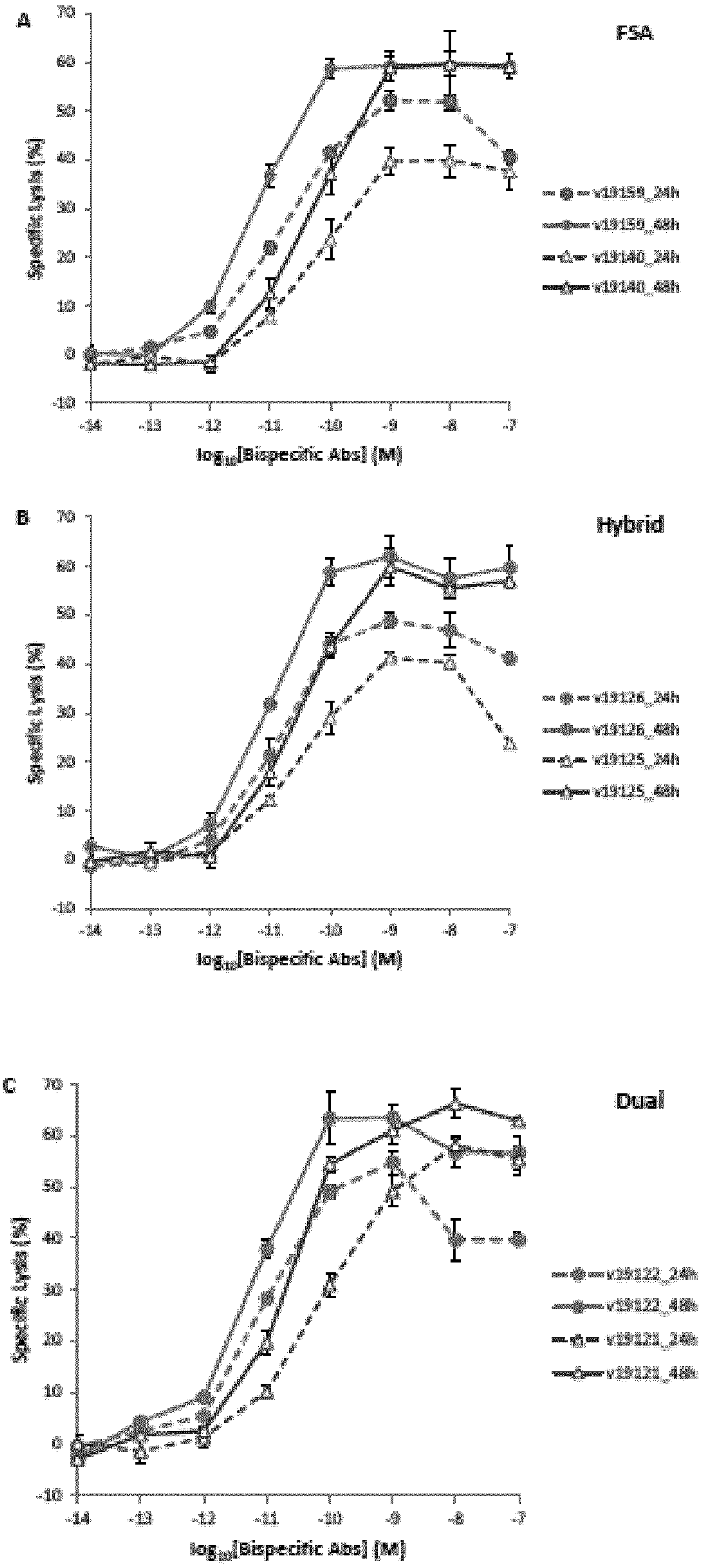
A
FSA
Specific Lysis (%)
log10[Bispecific Abs] (M)
v19159_24h
v19159_48h
v19140_24h
v19140_48h
B
Hybrid
Specific Lysis (%)
log10[Bispecific Abs] (M)
v19126_24h
v19126_48h
v19125_24h
v19125_48h
C
Dual
Specific Lysis (%)
log10[Bispecific Abs] (M)
v19122_24h
v19122_48h
v19121_24h
v19121_48h

[Figure 253]

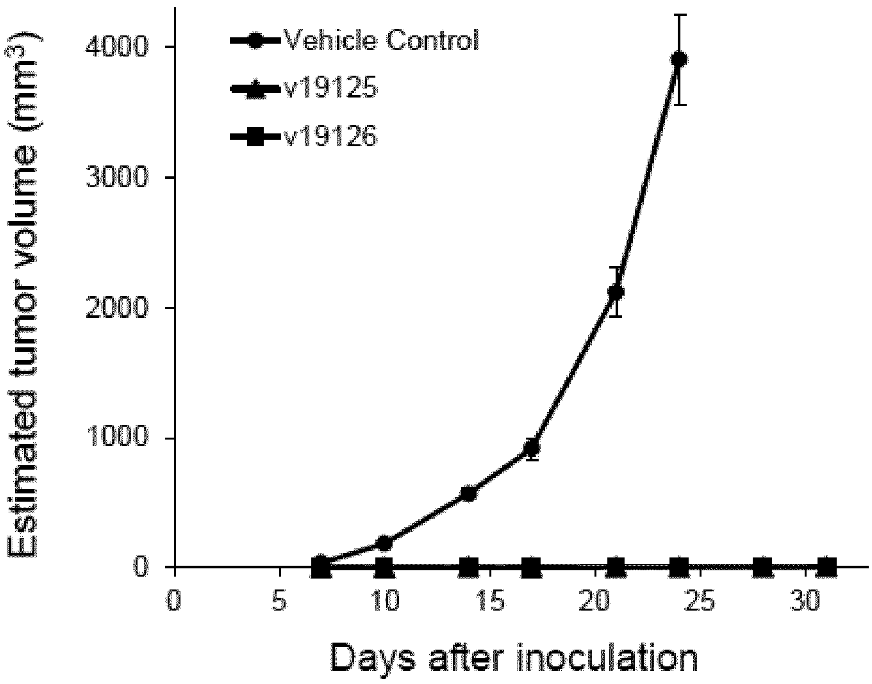
Vehicle Control
v19125
v19126
Estimated tumor volume (mm3)
4000
3000
2000
1000
0
0
5
10
15
20
25
30
Days after inoculation

[Figure 254]

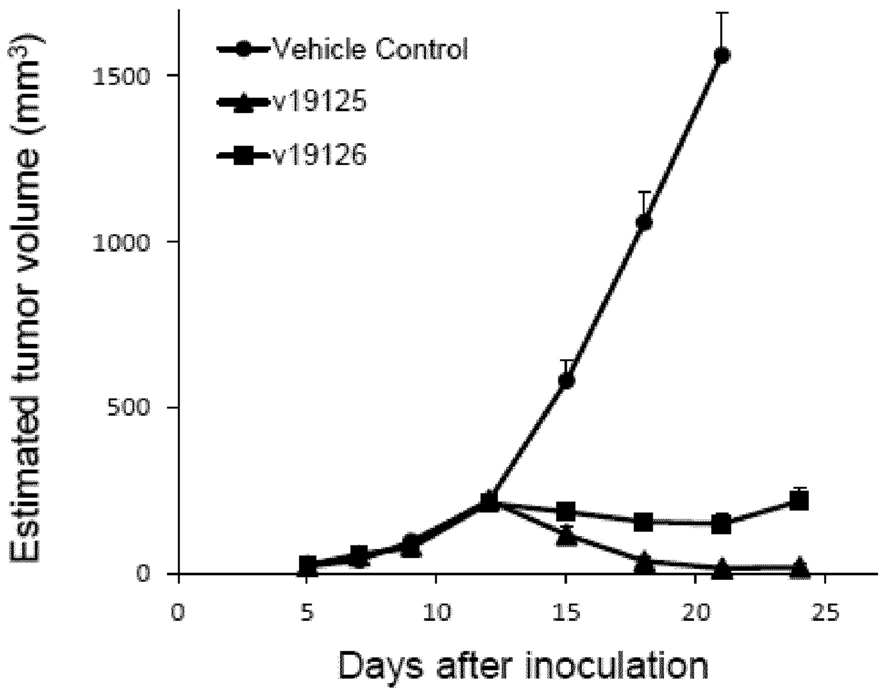
Vehicle Control
v19125
v19126
Estimated tumor volume (mm3)
1500
1000
500
0
0
5
10
15
20
25
Days after inoculation

[Figure 255]

ATGCGGCCCACCTGGGCATGGTGGCTGTTCCTGGTGCTGCTGCTGGC
CCTGTGGGCCCCTGCCAGGGGCGGAGAAGTGCAGCTGGTGGAATCCG
GGGGGGGCCTGGTGCAGCCTGGGGGGAGCCTGAGACTGAGTTGTGCC
GCCTCTGGGGTGACATTTAACTACTATGGCATGTCTTGGATCCGCCA
GGCACCTGGAAAGGGCCTGGAGTGGGTGGCCAGCATCACTAGGTCCG
GCGGGCGAATCTACTATCCCGACAGCGTCAAGGGCAGGTTCACAATT
TCCCGCGAGAACACACAGAAAACTCTGTACCTGCAGATGAATAGCCT
GAGAGCCGAAGATACAGCTGTGTACTATTGCACTCTGGACGGCAGGG
ATGGGTGGGTCGCCTATTGGGGGCAGGGAACCCTGGTGACAGTCAGC
TCCGGAGGAGGAGGATCTGGCGGAGGAGGCAGTGGGGGAGGCGGGTC
AAACTTTATGCTCACTCAGCCGTCCTCTGTTTCTGGCGTACCTGGCC
AACGGGTGACCATTAGCTGTACGGGTAATACCGGGAATATCGGGTCT
AACTACGTGAACTGGTATCAGCAGCTTCCAGGGACAGCTCCCAAGTT
GCTGATCTATCGCGACGACAAAAGACCCTCAGGGGTCCCTGACCGAT
TTAGTGGCAGCAAAAGCGGTACTTCCGCTTCCCTGGCGATAACCGGC
TTTCAGGCCGAAGATGAGGCAGACTACTATTGCCAGTCATATTCCAG
CGGCTTCATCTTCGGAGGCGGAACTAAGCTGACAGTGTTGGCGGCCG
AGCCTAAAAGTAGCGATAAAACCCATACCTGCCCCCCCTGCCCGGCG
CCAGAGGCTGCAGGAGGACCAAGCGTGTTCCTGTTTCCACCCAAGCC
TAAAGACACACTGATGATTTCCCGAACCCCCGAAGTCACATGCGTGG
TCGTGTCTGTGAGTCACGAGGACCCTGAAGTCAAGTTCAACTGGTAC
GTGGATGGCGTCGAGGTGCATAATGCCAAGACTAAACCTAGGGAGGA
ACAGTACAACTCAACCTATCGCGTCGTGAGCGTCCTGACAGTGCTGC
ACCAGGATTGGCTGAACGGCAAAGAATATAAGTGCAAAGTGAGCAAT
AAGGCCCTGCCCGCTCCTATCGAGAAAACCATTTCCAAGGCTAAAGG
GCAGCCTCGCGAACCACAGGTCTACGTCTACCCCCCATCAAGAGATG
AACTGACAAAAAATCAGGTCTCTCTGACATGCCTGGTCAAAGGATTC
TACCCTTCCGACATCGCCGTGGAGTGGGAAAGTAACGGCCAGCCCGA
GAACAATTACAAGACCACACCCCCTGTCCTGGACTCTGATGGGAGTT
TCGCTCTGGTGTCAAAGCTGACCGTCGATAAAAGCCGGTGGCAGCAG
GGCAATGTGTTTAGCTGCTCCGTCATGCACGAAGCCCTGCACAATCA
CTACACACAGAAGTCCCTGAGCCTGAGCCCTGGC

(SEQ ID NO: 224 of the Sequence Listing)

[Figure 256]

M R P T W A W W L F L V L L L A L W A P A R G G E V Q L V E S G G G L

V Q P G G S L R L S C A A S G V T F N Y Y G M S W I R Q A P G K G L E

W V A S I T R S G G R I Y Y P D S V K G R F T I S R E N T Q K T L Y L

Q M N S L R A E D T A V Y Y C T L D G R D G W V A Y W G Q G T L V T V

S S G G G G S G G G G S G G G G S N F M L T Q P S S V S G V P G Q R V

T I S C T G N T G N I G S N Y V N W Y Q Q L P G T A P K L L I Y R D D

K R P S G V P D R F S G S K S G T S A S L A I T G F Q A E D E A D Y Y

C Q S Y S S G F I F G G G T K L T V L A A E P K S S D K T H T C P P C

P A P E A A G G P S V F L F P P K P K D T L M I S R T P E V T C V V V

S V S H E D P E V K F N W Y V D G V E V H N A K T K P R E E Q Y N S T

Y R V V S V L T V L H Q D W L N G K E Y K C K V S N K A L P A P I E K

T I S K A K G Q P R E P Q V Y V Y P P S R D E L T K N Q V S L T C L V

K G F Y P S D I A V E W E S N G Q P E N N Y K T T P P V L D S D G S F

A L V S K L T V D K S R W Q Q G N V F S C S V M H E A L H N H Y T Q K

S L S L S P G

(SEQ ID NO: 225 of the Sequence Listing)

[Figure 257]

ATGCGCCCTACCTGGGCCTGGTGGCTGTTCCTGGTGCTGCTGCTGGCCCTGTG
GGCCCCAGCCCGGGGCGGAGAAGTGCAGCTGGTGGAATCCGGGGGGGGCCTGG
TGCAGCCTGGGGGGAGCCTGAGACTGAGTTGTGCCGCCTCTGGGGTGACATTT
AACTACTATGGCATGTCTTGGATCCGCCAGGCACCTGGAAAGGGCCTGGAGTG
GGTGGCCAGCATCACTAGGTCCGGCGGGCGAATCTACTATCCCGACAGCGTCA
AGGGCAGGTTCACAATTTCCCGCGAGAACACACAGAAAACTCTGTACCTGCAG
ATGAATAGCCTGAGAGCCGAAGATACAGCTGTGTACTATTGCACTCTGGACGG
CAGGGATGGGTGGGTCGCCTATTGGGGGCAGGGAACCCTGGTGACAGTCAGCT
CCGGAGGAGGAGGATCTGGCGGAGGAGGCAGTGGGGGAGGCGGGTCAAACTTT
ATGCTGACCCAGCCCCACAGTGTGTCAGAGAGCCCTGGCAAGACTGTCACCAT
CTCTTGTAAAAGGAACACCGGAAATATTGGCAGTAACTACGTGAATTGGTATC
AGCAGCATGAAGGGTCTAGTCCAACCACAATCATCTACCGGAACGATAAGAGA
CCCGACGGGGTGTCCGATCGATTCTCCGGATCTATCGACCGGTCAAGCAAGAG
TGCTTCACTGACCATTAGCAATCTGAAAACAGAGGACGAAGCAGATTACTTTT
GCCAGTCCTATTCCTCTGGCTTCATCTTTGGAGGCGGGACTAAACTGACCGTG
CTGGCGGCCGAGCCTAAAAGTAGCGATAAAACCCATACCTGCCCCCCCTGCCC
GGCGCCAGAGGCTGCAGGAGGACCAAGCGTGTTCCTGTTTCCACCCAAGCCTA
AAGACACACTGATGATTTCCCGAACCCCCGAAGTCACATGCGTGGTCGTGTCT
GTGAGTCACGAGGACCCTGAAGTCAAGTTCAACTGGTACGTGGATGGCGTCGA
GGTGCATAATGCCAAGACTAAACCTAGGGAGGAACAGTACAACTCAACCTATC
GCGTCGTGAGCGTCCTGACAGTGCTGCACCAGGATTGGCTGAACGGCAAAGAA
TATAAGTGCAAAGTGAGCAATAAGGCCCTGCCCGCTCCTATCGAGAAAACCAT
TTCCAAGGCTAAAGGGCAGCCTCGCGAACCACAGGTCTACGTCTACCCCCCAT
CAAGAGATGAACTGACAAAAAATCAGGTCTCTCTGACATGCCTGGTCAAAGGA
TTCTACCCTTCCGACATCGCCGTGGAGTGGGAAAGTAACGGCCAGCCCGAGAA
CAATTACAAGACCACACCCCCTGTCCTGGACTCTGATGGGAGTTTCGCTCTGG
TGTCAAAGCTGACCGTCGATAAAAGCCGGTGGCAGCAGGGCAATGTGTTTAGC
TGCTCCGTCATGCACGAAGCCCTGCACAATCACTACACACAGAAGTCCCTGAG
CCTGAGCCCTGGC

(SEQ ID NO: 226 of the Sequence Listing)

[Figure 258]

M R P T W A W W L F L V L L L A L W A P A R G G E V Q L V E S G G G L

V Q P G G S L R L S C A A S G V T F N Y Y G M S W I R Q A P G K G L E

W V A S I T R S G G R I Y Y P D S V K G R F T I S R E N T Q K T L Y L

Q M N S L R A E D T A V Y Y C T L D G R D G W V A Y W G Q G T L V T V

S S G G G G S G G G G S G G G G S N F M L T Q P H S V S E S P G K T V

T I S C K R N T G N I G S N Y V N W Y Q Q H E G S S P T T I I Y R N D

K R P D G V S D R F S G S I D R S S K S A S L T I S N L K T E D E A D

Y F C Q S Y S S G F I F G G G T K L T V L A A E P K S S D K T H T C P

P C P A P E A A G G P S V F L F P P K P K D T L M I S R T P E V T C V

V V S V S H E D P E V K F N W Y V D G V E V H N A K T K P R E E Q Y N

S T Y R V V S V L T V L H Q D W L N G K E Y K C K V S N K A L P A P I

E K T I S K A K G Q P R E P Q V Y V Y P P S R D E L T K N Q V S L T C

L V K G F Y P S D I A V E W E S N G Q P E N N Y K T T P P V L D S D G

S F A L V S K L T V D K S R W Q Q G N V F S C S V M H E A L H N H Y T

Q K S L S L S P G

(SEQ ID NO: 227 of the Sequence Listing)

[Figure 259]

ATGCGCCCTACCTGGGCCTGGTGGCTGTTCCTGGTGCTGCTGCTGGCCCTGTG
GGCCCCAGCCCGGGGCGGAGAAGTGCAGCTGGTGGAATCCGGGGGGGGCCTGG
TGCAGCCTGGGGGGAGCCTGAGACTGAGTTGTGCCGCCTCTGGGGTGACATTT
AACTACTATGGCATGTCTTGGATCCGCCAGGCACCTGGAAAGGGCCTGGAGTG
GGTGGCCAGCATCACTTCTTCCGGCGGGCGAATCTACTATCCCGACAGCGTCA
AGGGCAGGTTCACAATTTCCCGCGAGAACACACAGAAAACTCTGTACCTGCAG
ATGAATAGCCTGAGAGCCGAAGATACAGCTGTGTACTATTGCACTCTGGACGG
CAGGGATGGGTGGGTCGCCTATTGGGGGCAGGGAACCCTGGTGACAGTCAGCT
CCGGAGGAGGAGGATCTGGCGGAGGAGGCAGTGGGGGAGGCGGGTCAAACTTT
ATGCTGACCCAGCCCCACAGTGTGTCAGAGAGCCCTGGCAAGACTGTCACCAT
CTCTTGTAAAAGGAACACCGGAAATATTGGCAGTAACTACGTGAATTGGTATC
AGCAGCATGAAGGGTCTAGTCCAACCACAATCATCTACCGGAACGATAAGAGA
CCCGACGGGGTGTCCGATCGATTCTCCGGATCTATCGACCGGTCAAGCAAGAG
TGCTTCACTGACCATTAGCAATCTGAAAACAGAGGACGAAGCAGATTACTTTT
GCCAGTCCTATTCCTCTGGCTTCATCTTTGGAGGCGGGACTAAACTGACCGTG
CTGGCGGCCGAGCCTAAAAGTAGCGATAAAACCCATACCTGCCCCCCCTGCCC
GGCGCCAGAGGCTGCAGGAGGACCAAGCGTGTTCCTGTTTCCACCCAAGCCTA
AAGACACACTGATGATTTCCCGAACCCCCGAAGTCACATGCGTGGTCGTGTCT
GTGAGTCACGAGGACCCTGAAGTCAAGTTCAACTGGTACGTGGATGGCGTCGA
GGTGCATAATGCCAAGACTAAACCTAGGGAGGAACAGTACAACTCAACCTATC
GCGTCGTGAGCGTCCTGACAGTGCTGCACCAGGATTGGCTGAACGGCAAAGAA
TATAAGTGCAAAGTGAGCAATAAGGCCCTGCCCGCTCCTATCGAGAAAACCAT
TTCCAAGGCTAAAGGGCAGCCTCGCGAACCACAGGTCTACGTCTACCCCCCAT
CAAGAGATGAACTGACAAAAAATCAGGTCTCTCTGACATGCCTGGTCAAAGGA
TTCTACCCTTCCGACATCGCCGTGGAGTGGGAAAGTAACGGCCAGCCCGAGAA
CAATTACAAGACCACACCCCCTGTCCTGGACTCTGATGGGAGTTTCGCTCTGG
TGTCAAAGCTGACCGTCGATAAAAGCCGGTGGCAGCAGGGCAATGTGTTTAGC
TGCTCCGTCATGCACGAAGCCCTGCACAATCACTACACACAGAAGTCCCTGAG
CCTGAGCCCTGGC

(SEQ ID NO: 228 of the Sequence Listing)

[Figure 260]

M R P T W A W W L F L V L L L A L W A P A R G G E V Q L V E S G G G L

V Q P G G S L R L S C A A S G V T F N Y Y G M S W I R Q A P G K G L E

W V A S I T S S G G R I Y Y P D S V K G R F T I S R E N T Q K T L Y L

Q M N S L R A E D T A V Y Y C T L D G R D G W V A Y W G Q G T L V T V

S S G G G G S G G G G S G G G G S N F M L T Q P H S V S E S P G K T V

T I S C K R N T G N I G S N Y V N W Y Q Q H E G S S P T T I I Y R N D

K R P D G V S D R F S G S I D R S S K S A S L T I S N L K T E D E A D

Y F C Q S Y S S G F I F G G G T K L T V L A A E P K S S D K T H T C P

P C P A P E A A G G P S V F L F P P K P K D T L M I S R T P E V T C V

V V S V S H E D P E V K F N W Y V D G V E V H N A K T K P R E E Q Y N

S T Y R V V S V L T V L H Q D W L N G K E Y K C K V S N K A L P A P I

E K T I S K A K G Q P R E P Q V Y V Y P P S R D E L T K N Q V S L T C

L V K G F Y P S D I A V E W E S N G Q P E N N Y K T T P P V L D S D G

S F A L V S K L T V D K S R W Q Q G N V F S C S V M H E A L H N H Y T

Q K S L S L S P G

(SEQ ID NO: 229 of the Sequence Listing)

[Figure 261]

A T G C G G C C C A C C T G G G C A T G G T G G C T G T T C C T G G T G C T G C T G C T G G C C C T G T G
G G C C C C T G C C A G G G G C G G A G A A G T G C A G C T G G T G G A A T C C G G G G G G G C C T G G
T G C A G C C T G G G G G G A G C C T G A G A C T G A G T T G T G C C G C C T C T G G G G T G A C A T T T
A A C T A C T A T G G C A T G T C T T G G A T C C G C C A G G C A C C T G G A A A G G G C C T G G A G T G
G G T G G C C A G C A T C A C T A G G T C C G G C G G G C G A A T C T A C T A T C C C G A C A G C G T C A
A G G G C A G G T T C A C A A T T T C C C G C G A G A A C A C A C A G A A A A C T C T G T A C C T G C A G
A T G A A T A G C C T G A G A G C C G A A G A T A C A G C T G T G T A C T A T T G C A C T C T G G A C G G
C A G G G A T G G G T G G G T C G C C T A T T G G G G G C A G G G A A C C C T G G T G A C A G T C A G C T
C C G G A G G A G G A G G A T C T G G C G G A G G A G G C A G T G G G G G A G G C G G G T C A A A C T T T
A T G C T C A C T C A G C C G T C C T C T G T T T C T G G C G T A C C T G G C C A A C G G G T G A C C A T
T A G C T G T A C G G G T A A T A C C G G G A A T A T C G G G T C T A A C T A C G T G A A C T G G T A T C
A G C A G C T T C C A G G G A C A G C T C C C A A G T T G C T G A T C T A T C G C G A C G A C A A A A G A
C C C T C A G G G G T C C C T G A C C G A T T T A G T G G C A G C A A A A G C G G T A C T T C C G C T T C
C C T G G C G A T A A C C G G C T T T C A G G C C G A A G A T G A G G C A G A C T A C T A T T G C C A G T
C A T A T T C C A G C G G C T T C A T C T T C G G A G G C G G A A C T A A G C T G A C A G T G T T G G C G
G C C G A G C C T A A A A G T A G C G A T A A A A C C C A T A C C T G C C C C C C C T G C C C G G C G C C
A G A G G C T G C A G G A G G A C C A A G C G T G T T C C T G T T T C C A C C C A A G C C T A A A G A C A
C A C T G A T G A T T T C C C G A A C C C C C G A A G T C A C A T G C G T G G T C G T G T C T G T G A G T
C A C G A G G A C C C T G A A G T C A A G T T C A A C T G G T A C G T G G A T G G C G T C G A G G T G C A
T A A T G C C A A G A C T A A A C C T A G G G A G G A A C A G T A C A A C T C A A C C T A T C G C G T C G
T G A G C G T C C T G A C A G T G C T G C A C C A G G A T T G G C T G A A C G G C A A A G A A T A T A A G
T G C A A A G T G A G C A A T A A G G C C C T G C C C G C T C C T A T C G A G A A A A C C A T T T C C A A
G G C T A A A G G G C A G C C T C G C G A A C C A C A G G T C T A C G T C T A C C C C C C A T C A A G A G
A T G A A C T G A C A A A A A A T C A G G T C T C T C T G A C A T G C C T G G T C A A A G G A T T C T A C
C C T T C C G A C A T C G C C G T G G A G T G G G A A A G T A A C G G C C A G C C C G A G A A C A A T T A
C A A G A C C A C A C C C C C T G T C C T G G A C T C T G A T G G G A G T T T C G C T C T G G T G T C A A
A G C T G A C C G T C G A T A A A A G C C G G T G G C A G C A G G G C A A T G T G T T T A G C T G C T C C
G T C A T G C A C G A A G C C C T G C A C A A T C A C T A C A C A C A G A A G T C C C T G A G C C T G A G
C C C T G G C A A G

(SEQ ID NO: 230 of the Sequence Listing)

[Figure 262]

M R P T W A W W L F L V L L L A L W A P A R G G E V Q L V E S G G G L

V Q P G G S L R L S C A A S G V T F N Y Y G M S W I R Q A P G K G L E

W V A S I T R S G G R I Y Y P D S V K G R F T I S R E N T Q K T L Y L

Q M N S L R A E D T A V Y Y C T L D G R D G W V A Y W G Q G T L V T V

S S G G G G S G G G G S G G G G S N F M L T Q P S S V S G V P G Q R V

T I S C T G N T G N I G S N Y V N W Y Q Q L P G T A P K L L I Y R D D

K R P S G V P D R F S G S K S G T S A S L A I T G F Q A E D E A D Y Y

C Q S Y S S G F I F G G G T K L T V L A A E P K S S D K T H T C P P C

P A P E A A G G P S V F L F P P K P K D T L M I S R T P E V T C V V V

S V S H E D P E V K F N W Y V D G V E V H N A K T K P R E E Q Y N S T

Y R V V S V L T V L H Q D W L N G K E Y K C K V S N K A L P A P I E K

T I S K A K G Q P R E P Q V Y V Y P P S R D E L T K N Q V S L T C L V

K G F Y P S D I A V E W E S N G Q P E N N Y K T T P P V L D S D G S F

A L V S K L T V D K S R W Q Q G N V F S C S V M H E A L H N H Y T Q K

S L S L S P G K

(SEQ ID NO: 231 of the Sequence Listing)

[Figure 263]

ATGCGCCCTACCTGGGCCTGGTGGCTGTTCCTGGTGCTGCTGCTGGCCCTGTG
GGCCCCAGCCCGGGGCGGAGAAGTGCAGCTGGTGGAATCCGGGGGGGCCTGG
TGCAGCCTGGGGGGAGCCTGAGACTGAGTTGTGCCGCCTCTGGGGTGACATTT
AACTACTATGGCATGTCTTGGATCCGCCAGGCACCTGGAAAGGGCCTGGAGTG
GGTGGCCAGCATCACTAGGTCCGGCGGGCGAATCTACTATCCCGACAGCGTCA
AGGGCAGGTTCACAATTTCCCGCGAGAACACACAGAAAACTCTGTACCTGCAG
ATGAATAGCCTGAGAGCCGAAGATACAGCTGTGTACTATTGCACTCTGGACGG
CAGGGATGGGTGGGTCGCCTATTGGGGGCAGGGAACCCTGGTGACAGTCAGCT
CCGGAGGAGGAGGATCTGGCGGAGGAGGCAGTGGGGGAGGCGGGTCAAACTTT
ATGCTGACCCAGCCCCACAGTGTGTCAGAGAGCCCTGGCAAGACTGTCACCAT
CTCTTGTAAAAGGAACACCGGAAATATTGGCAGTAACTACGTGAATTGGTATC
AGCAGCATGAAGGGTCTAGTCCAACCACAATCATCTACCGGAACGATAAGAGA
CCCGACGGGGTGTCCGATCGATTCTCCGGATCTATCGACCGGTCAAGCAAGAG
TGCTTCACTGACCATTAGCAATCTGAAAACAGAGGACGAAGCAGATTACTTTT
GCCAGTCCTATTCCTCTGGCTTCATCTTTGGAGGCGGGACTAAACTGACCGTG
CTGGCGGCCGAGCCTAAAAGTAGCGATAAAACCCATACCTGCCCCCCCTGCCC
GGCGCCAGAGGCTGCAGGAGGACCAAGCGTGTTCCTGTTTCCACCCAAGCCTA
AAGACACACTGATGATTTCCCGAACCCCCGAAGTCACATGCGTGGTCGTGTCT
GTGAGTCACGAGGACCCTGAAGTCAAGTTCAACTGGTACGTGGATGGCGTCGA
GGTGCATAATGCCAAGACTAAACCTAGGGAGGAACAGTACAACTCAACCTATC
GCGTCGTGAGCGTCCTGACAGTGCTGCACCAGGATTGGCTGAACGGCAAAGAA
TATAAGTGCAAAGTGAGCAATAAGGCCCTGCCCGCTCCTATCGAGAAAACCAT
TTCCAAGGCTAAAGGGCAGCCTCGCGAACCACAGGTCTACGTCTACCCCCCAT
CAAGAGATGAACTGACAAAAAATCAGGTCTCTCTGACATGCCTGGTCAAAGGA
TTCTACCCTTCCGACATCGCCGTGGAGTGGGAAAGTAACGGCCAGCCCGAGAA
CAATTACAAGACCACACCCCCTGTCCTGGACTCTGATGGGAGTTTCGCTCTGG
TGTCAAAGCTGACCGTCGATAAAAGCCGGTGGCAGCAGGGCAATGTGTTTAGC
TGCTCCGTCATGCACGAAGCCCTGCACAATCACTACACACAGAAGTCCCTGAG
CCTGAGCCCTGGCAAG

(SEQ ID NO: 232 of the Sequence Listing)

[Figure 264]

M R P T W A W W L F L V L L L A L W A P A R G G E V Q L V E S G G G L

V Q P G G S L R L S C A A S G V T F N Y Y G M S W I R Q A P G K G L E

W V A S I T R S G G R I Y Y P D S V K G R F T I S R E N T Q K T L Y L

Q M N S L R A E D T A V Y Y C T L D G R D G W V A Y W G Q G T L V T V

S S G G G G S G G G G S G G G G S N F M L T Q P H S V S E S P G K T V

T I S C K R N T G N I G S N Y V N W Y Q Q H E G S S P T T I I Y R N D

K R P D G V S D R F S G S I D R S S K S A S L T I S N L K T E D E A D

Y F C Q S Y S S G F I F G G G T K L T V L A A E P K S S D K T H T C P

P C P A P E A A G G P S V F L F P P K P K D T L M I S R T P E V T C V

V V S V S H E D P E V K F N W Y V D G V E V H N A K T K P R E E Q Y N

S T Y R V V S V L T V L H Q D W L N G K E Y K C K V S N K A L P A P I

E K T I S K A K G Q P R E P Q V Y V Y P P S R D E L T K N Q V S L T C

L V K G F Y P S D I A V E W E S N G Q P E N N Y K T T P P V L D S D G

S F A L V S K L T V D K S R W Q Q G N V F S C S V M H E A L H N H Y T

Q K S L S L S P G K

(SEQ ID NO: 233 of the Sequence Listing)

[Figure 265]

ATGCGCCCTACCTGGGCCTGGTGGCTGTTCCTGGTGCTGCTGCTGGCCCTGTG
GGCCCCAGCCCGGGGCGGAGAAGTGCAGCTGGTGGAATCCGGGGGGGGCCTGG
TGCAGCCTGGGGGGAGCCTGAGACTGAGTTGTGCCGCCTCTGGGGTGACATTT
AACTACTATGGCATGTCTTGGATCCGCCAGGCACCTGGAAAGGGCCTGGAGTG
GGTGGCCAGCATCACTTCTTCCGGCGGGCGAATCTACTATCCCGACAGCGTCA
AGGGCAGGTTCACAATTTCCCGCGAGAACACACAGAAAACTCTGTACCTGCAG
ATGAATAGCCTGAGAGCCGAAGATACAGCTGTGTACTATTGCACTCTGGACGG
CAGGGATGGGTGGGTCGCCTATTGGGGGCAGGGAACCCTGGTGACAGTCAGCT
CCGGAGGAGGAGGATCTGGCGGAGGAGGCAGTGGGGGAGGCGGGTCAAACTTT
ATGCTGACCCAGCCCCACAGTGTGTCAGAGAGCCCTGGCAAGACTGTCACCAT
CTCTTGTAAAAGGAACACCGGAAATATTGGCAGTAACTACGTGAATTGGTATC
AGCAGCATGAAGGGTCTAGTCCAACCACAATCATCTACCGGAACGATAAGAGA
CCCGACGGGGTGTCCGATCGATTCTCCGGATCTATCGACCGGTCAAGCAAGAG
TGCTTCACTGACCATTAGCAATCTGAAAACAGAGGACGAAGCAGATTACTTTT
GCCAGTCCTATTCCTCTGGCTTCATCTTTGGAGGCGGGACTAAACTGACCGTG
CTGGCGGCCGAGCCTAAAAGTAGCGATAAAACCCATACCTGCCCCCCCTGCCC
GGCGCCAGAGGCTGCAGGAGGACCAAGCGTGTTCCTGTTTCCACCCAAGCCTA
AAGACACACTGATGATTTCCCGAACCCCCGAAGTCACATGCGTGGTCGTGTCT
GTGAGTCACGAGGACCCTGAAGTCAAGTTCAACTGGTACGTGGATGGCGTCGA
GGTGCATAATGCCAAGACTAAACCTAGGGAGGAACAGTACAACTCAACCTATC
GCGTCGTGAGCGTCCTGACAGTGCTGCACCAGGATTGGCTGAACGGCAAAGAA
TATAAGTGCAAAGTGAGCAATAAGGCCCTGCCCGCTCCTATCGAGAAAACCAT
TTCCAAGGCTAAAGGGCAGCCTCGCGAACCACAGGTCTACGTCTACCCCCCAT
CAAGAGATGAACTGACAAAAAATCAGGTCTCTCTGACATGCCTGGTCAAAGGA
TTCTACCCTTCCGACATCGCCGTGGAGTGGGAAAGTAACGGCCAGCCCGAGAA
CAATTACAAGACCACACCCCCTGTCCTGGACTCTGATGGGAGTTTCGCTCTGG
TGTCAAAGCTGACCGTCGATAAAAGCCGGTGGCAGCAGGGCAATGTGTTTAGC
TGCTCCGTCATGCACGAAGCCCTGCACAATCACTACACACAGAAGTCCCTGAG
CCTGAGCCCTGGCAAG

(SEQ ID NO: 234 of the Sequence Listing)

[Figure 266]

M R P T W A W W L F L V L L L A L W A P A R G G E V Q L V E S G G G L

V Q P G G S L R L S C A A S G V T F N Y Y G M S W I R Q A P G K G L E

W V A S I T S S G G R I Y Y P D S V K G R F T I S R E N T Q K T L Y L

Q M N S L R A E D T A V Y Y C T L D G R D G W V A Y W G Q G T L V T V

S S G G G G S G G G G S G G G G S N F M L T Q P H S V S E S P G K T V

T I S C K R N T G N I G S N Y V N W Y Q Q H E G S S P T T I I Y R N D

K R P D G V S D R F S G S I D R S S K S A S L T I S N L K T E D E A D

Y F C Q S Y S S G F I F G G G T K L T V L A A E P K S S D K T H T C P

P C P A P E A A G G P S V F L F P P K P K D T L M I S R T P E V T C V

V V S V S H E D P E V K F N W Y V D G V E V H N A K T K P R E E Q Y N

S T Y R V V S V L T V L H Q D W L N G K E Y K C K V S N K A L P A P I

E K T I S K A K G Q P R E P Q V Y V Y P P S R D E L T K N Q V S L T C

L V K G F Y P S D I A V E W E S N G Q P E N N Y K T T P P V L D S D G

S F A L V S K L T V D K S R W Q Q G N V F S C S V M H E A L H N H Y T

Q K S L S L S P G K

(SEQ ID NO: 235 of the Sequence Listing)

[Figure 267]

A T G A G G C C T A C C T G G G C C T G G T G G C T G T T C C T G G T G C T G C T G C T G G C C C T G T G
G G C C C C C G C C C G G G G C C A G G T G C A G C T G G T G G A G T C C G G C G G C G G C C T G G T G A
A G C C C A G C C A G A C C C T G T C C C T G A C C T G C A C A T T C A G C G G C T T T T C C C T G A A C
A C A T A C G A T A T G G G A G T G G G A T G G A T C A G A C A G C C A C C T G G C A A G G G C C T G G A
G T G G A T C G G C A A C A T C T G G T G G G A C G A T G A C A A G T A C T A T A A T C C C T C T C T G A
A G A G C A G A G T G A C C A T C T C T A A G G A T A C A A G C A A C A A T C A G G C C T C C C T G A A G
C T G A G C T C C G T G A C C G C C G C C G A C A C A G C C G T G T A C T A T T G C G C C A G G A T C G A
G A C A G T G C G G G T G A G C C G G A A G G G A T T C G C A C A C T G G G G A C A G G G C A C C C T G G
T G A C A G T G T C T A G C G C T A G C A C A A A G G G A C C T A G C G T G T T C C C A C T G G C C C C T
T C C T C T A A G T C C A C C T C T G G A G G A A C A G C C G C C C T G G G C T G T C T G G T G A A G G A
C T A T T T C C C T G A G C C A G T G A C C G T G T C C T G G A A C T C T G G G G C C C T G A C C A G C G
G A G T G C A C A C A T T T C C T G C C G T G C T G C A G A G C T C C G G C C T G T A C A G C C T G T C T
A G C G T G G T G A C C G T G C C A T C C T C T A G C C T G G G C A C C C A G A C A T A T A T C T G C A A
C G T G A A T C A C A A G C C A A G C A A T A C A A A G G T C G A C A A G A G A G T G G A G C C C A A G T
C C T G T G A C A A G A C C C A C A C A T G C C C A C C C T G T C C G G C G C C A G A G G C T G C A G G A
G G A C C A A G C G T G T T C C T G T T T C C A C C C A A G C C T A A A G A C A C A C T G A T G A T T T C
C C G A A C C C C C G A A G T C A C A T G C G T G G T C G T G T C T G T G A G T C A C G A G G A C C C T G
A A G T C A A G T T C A A C T G G T A C G T G G A T G G C G T C G A G G T G C A T A A T G C C A A G A C T
A A A C C T A G G G A G G A A C A G T A C A A C T C A A C C T A T C G C G T C G T G A G C G T C C T G A C
A G T G C T G C A C C A G G A T T G G C T G A A C G G C A A A G A A T A T A A G T G C A A A G T G A G C A
A T A A G G C C C T G C C C G C T C C T A T C G A G A A A A C C A T T T C C A A G G C T A A A G G G C A G
C C T C G C G A A C C A C A G G T C T A C G T G C T G C C C C C T A G C C G C G A C G A A C T G A C T A A
A A A T C A G G T C T C T C T G C T G T G T C T G G T C A A A G G A T T C T A C C C T T C C G A C A T C G
C C G T G G A G T G G G A A A G T A A C G G C C A G C C C G A G A A C A A T T A C C T G A C C T G G C C C
C C T G T G C T G G A C T C T G A T G G G A G T T T C T T T C T G T A T T C A A A G C T G A C A G T C G A
T A A A A G C C G G T G G C A G C A G G G C A A T G T G T T C A G C T G C T C C G T C A T G C A C G A A G
C A C T G C A C A A C C A T T A C A C T C A G A A G T C C C T G T C C C T G T C A C C T G G C A A G

(SEQ ID NO: 236 of the Sequence Listing)

[Figure 268]

M R P T W A W W L F L V L L L A L W A P A R G Q V Q L V E S G G G L V

K P S Q T L S L T C T F S G F S L N T Y D M G V G W I R Q P P G K G L

E W I G N I W W D D D K Y Y N P S L K S R V T I S K D T S N N Q A S L

K L S S V T A A D T A V Y Y C A R I E T V R V S R K G F A H W G Q G T

L V T V S S A S T K G P S V F P L A P S S K S T S G G T A A L G C L V

K D Y F P E P V T V S W N S G A L T S G V H T F P A V L Q S S G L Y S

L S S V V T V P S S S L G T Q T Y I C N V N H K P S N T K V D K R V E

P K S C D K T H T C P P C P A P E A A G G P S V F L F P P K P K D T L

M I S R T P E V T C V V V S V S H E D P E V K F N W Y V D G V E V H N

A K T K P R E E Q Y N S T Y R V V S V L T V L H Q D W L N G K E Y K C

K V S N K A L P A P I E K T I S K A K G Q P R E P Q V Y V L P P S R D

E L T K N Q V S L L C L V K G F Y P S D I A V E W E S N G Q P E N N Y

L T W P P V L D S D G S F F L Y S K L T V D K S R W Q Q G N V F S C S

V M H E A L H N H Y T Q K S L S L S P G K

(SEQ ID NO: 237 of the Sequence Listing)

[Figure 269]

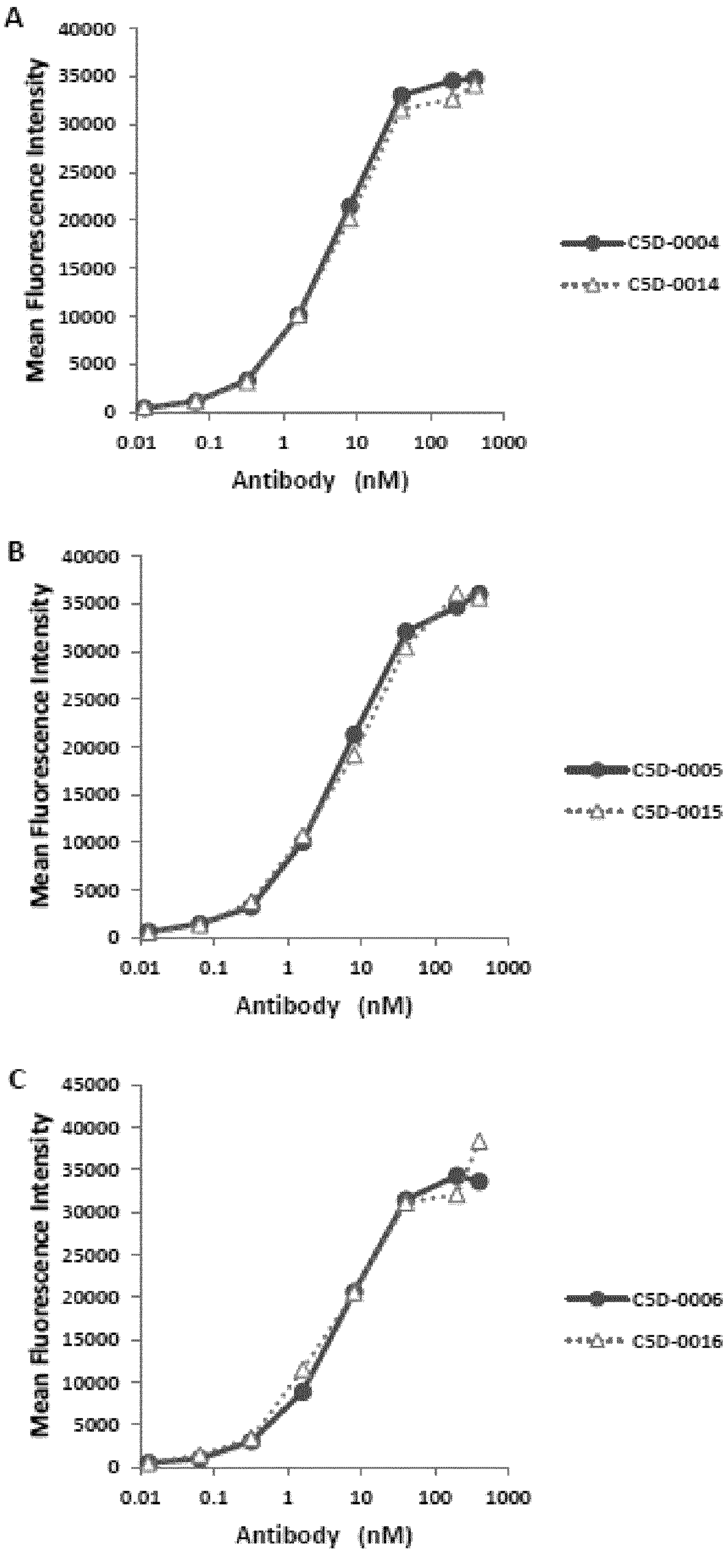
A
Mean Fluorescence Intensity
40000
35000
30000
25000
20000
15000
10000
5000
0
0.01
0.1
1
10
100
1000
Antibody (nM)
C5D-0004
C5D-0014
B
Mean Fluorescence Intensity
40000
35000
30000
25000
20000
15000
10000
5000
0
0.01
0.1
1
10
100
1000
Antibody (nM)
C5D-0005
C5D-0015
C
Mean Fluorescence Intensity
45000
40000
35000
30000
25000
20000
15000
10000
5000
0
0.01
0.1
1
10
100
1000
Antibody (nM)
C5D-0006
C5D-0016

[Figure 270]

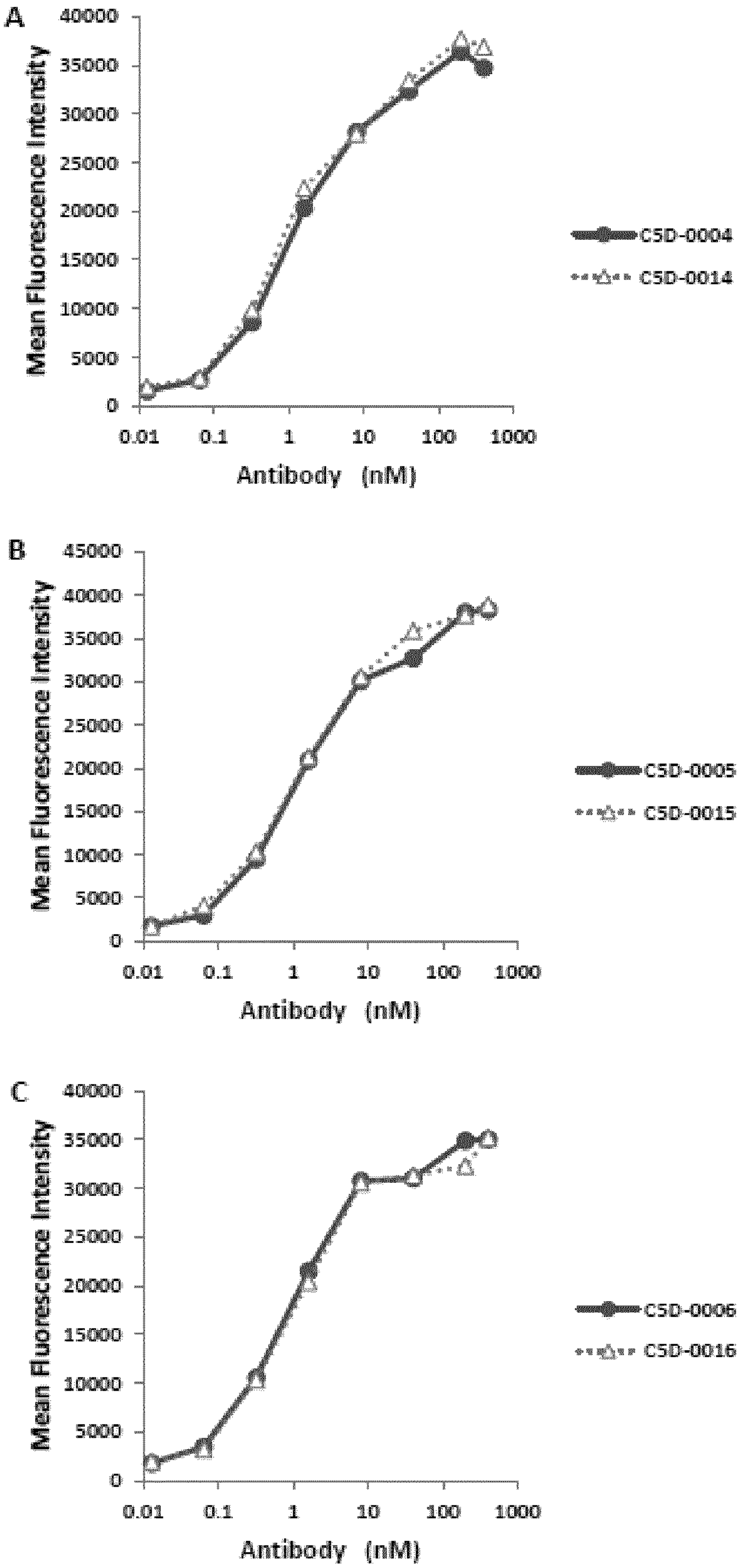
A
Mean Fluorescence Intensity
40000
35000
30000
25000
20000
15000
10000
5000
0
0.01
0.1
1
10
100
1000
Antibody (nM)
C5D-0004
C5D-0014
B
Mean Fluorescence Intensity
45000
40000
35000
30000
25000
20000
15000
10000
5000
0
0.01
0.1
1
10
100
1000
Antibody (nM)
C5D-0005
C5D-0015
C
Mean Fluorescence Intensity
40000
35000
30000
25000
20000
15000
10000
5000
0
0.01
0.1
1
10
100
1000
Antibody (nM)
C5D-0006
C5D-0016

[Figure 271]

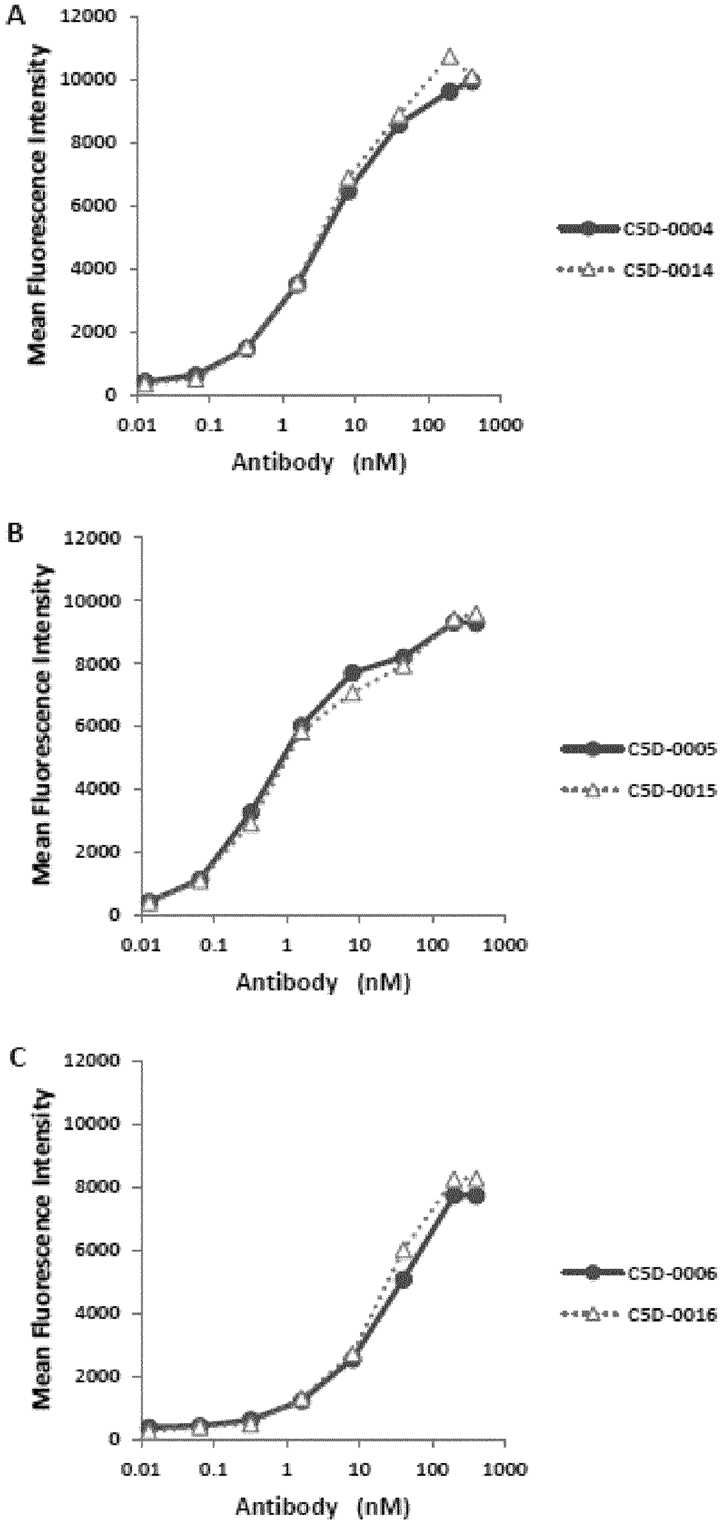
A
Mean Fluorescence Intensity
12000
10000
8000
6000
4000
2000
0
0.01
0.1
1
10
100
1000
Antibody (nM)
C5D-0004
C5D-0014
B
Mean Fluorescence Intensity
12000
10000
8000
6000
4000
2000
0
0.01
0.1
1
10
100
1000
Antibody (nM)
C5D-0005
C5D-0015
C
Mean Fluorescence Intensity
12000
10000
8000
6000
4000
2000
0
0.01
0.1
1
10
100
1000
Antibody (nM)
C5D-0006
C5D-0016

[Figure 272]

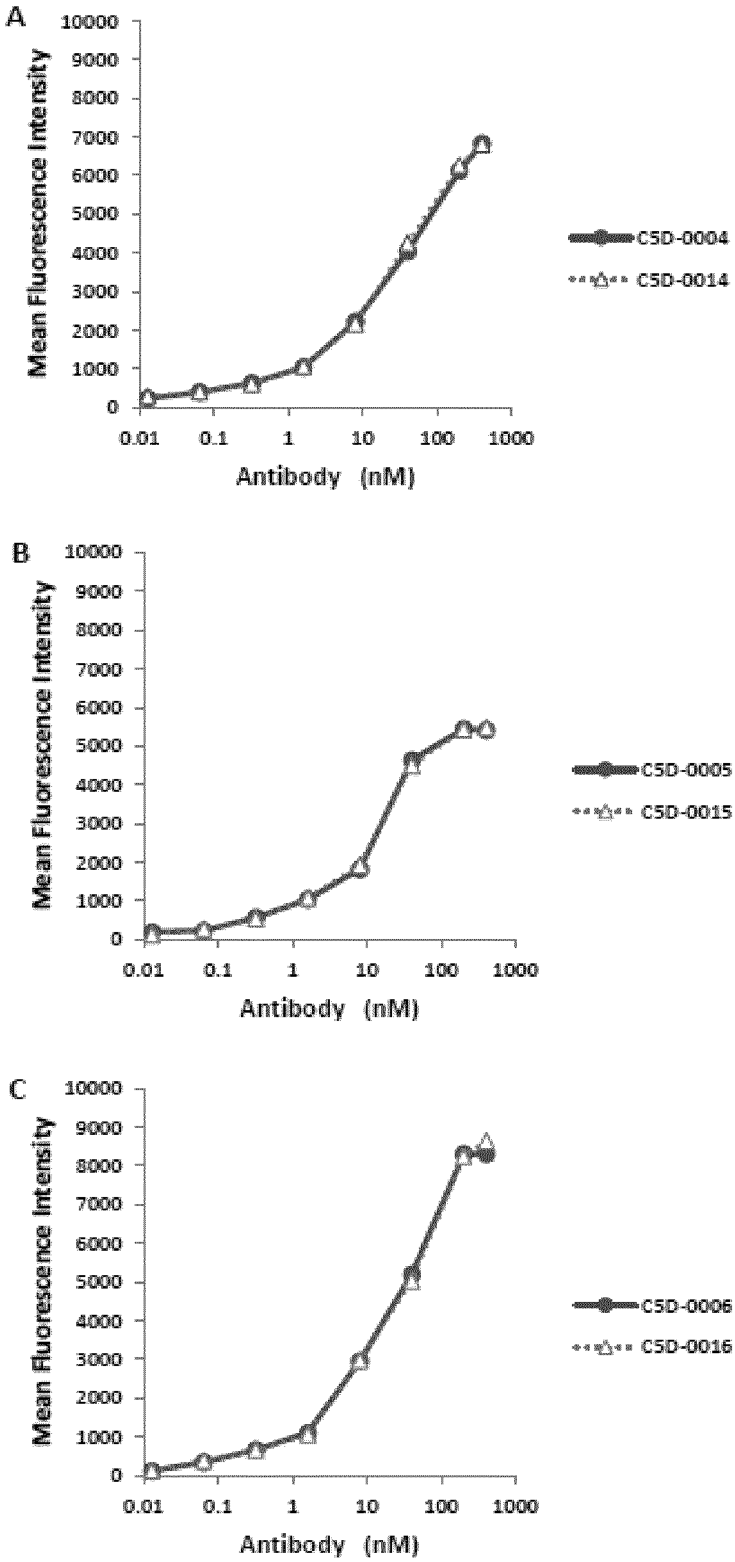
A
Mean Fluorescence Intensity
Antibody (nM)
C5D-0004
C5D-0014
B
Mean Fluorescence Intensity
Antibody (nM)
C5D-0005
C5D-0015
C
Mean Fluorescence Intensity
Antibody (nM)
C5D-0006
C5D-0016

[Figure 273-1]

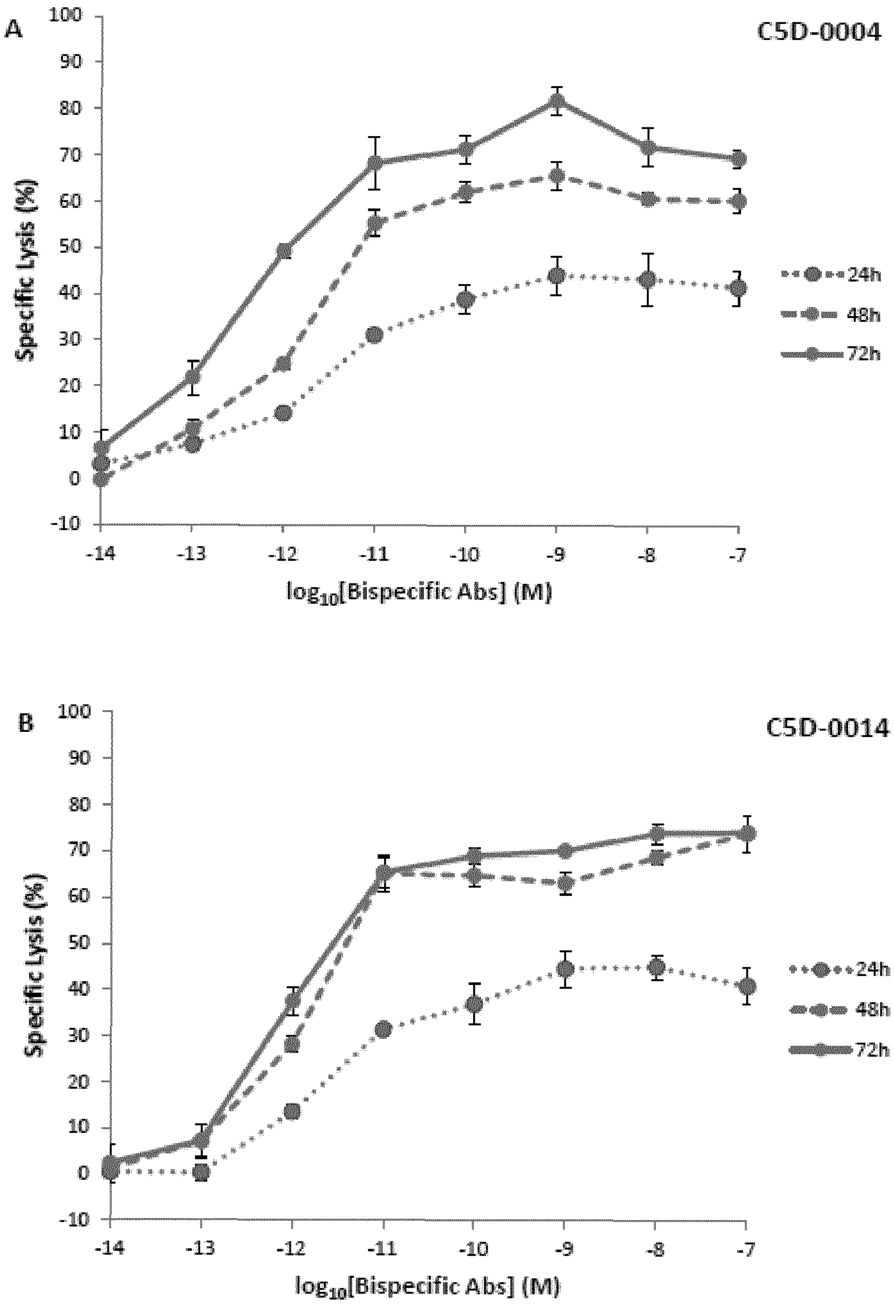
A
C5D-0004
Specific Lysis (%)
100
90
80
70
60
50
40
30
20
10
0
-10
-14
-13
-12
-11
-10
-9
-8
-7
$log_{10}$[Bispecific Abs] (M)
24h
48h
72h
B
C5D-0014
Specific Lysis (%)
$log_{10}$[Bispecific Abs] (M)
24h
48h
72h

[Figure 273-2]

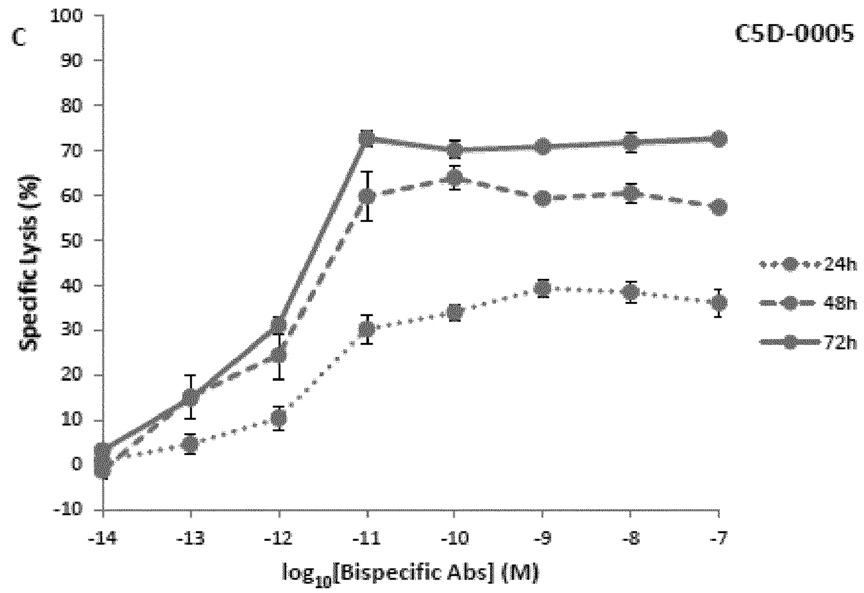
C
C5D-0005
Specific Lysis (%)
log10[Bispecific Abs] (M)
24h
48h
72h

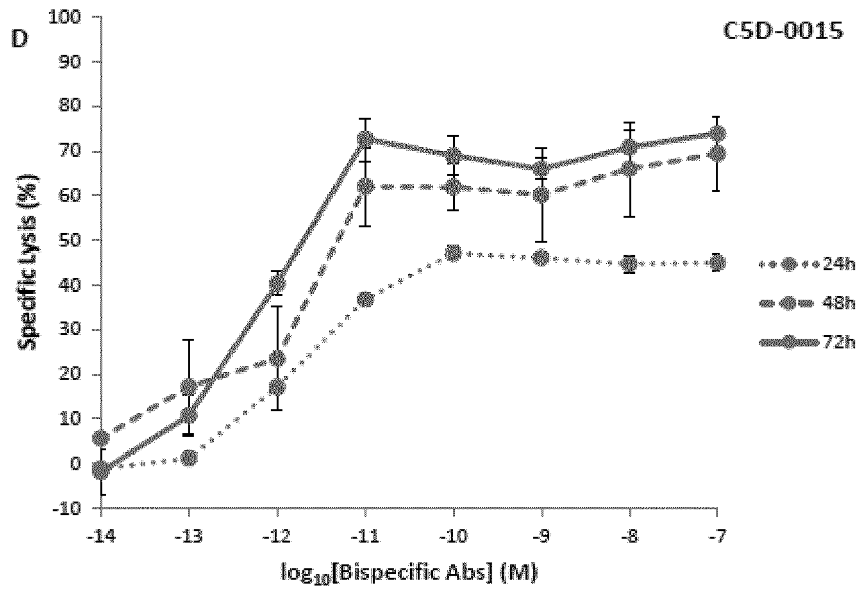
D
C5D-0015
Specific Lysis (%)
log10[Bispecific Abs] (M)
24h
48h
72h

[Figure 273-3]

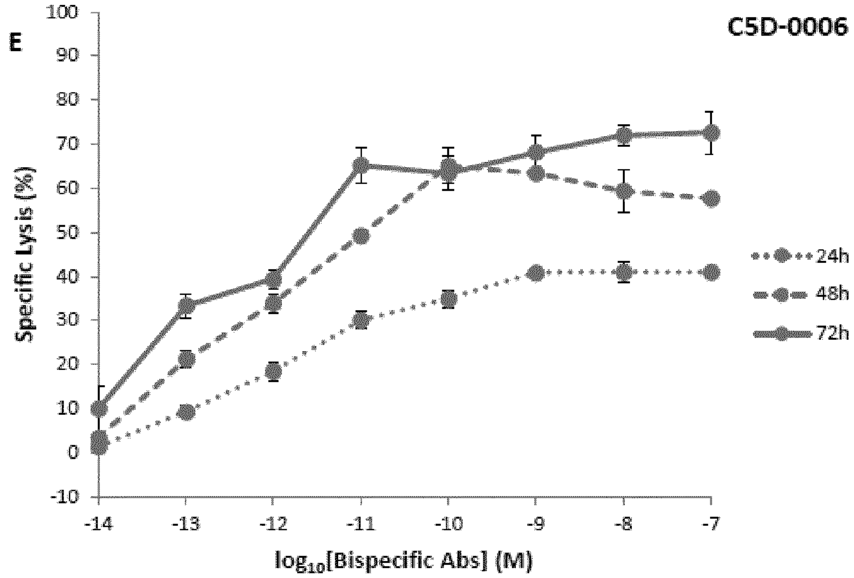
E
C5D-0006
Specific Lysis (%)
log10[Bispecific Abs] (M)
24h
48h
72h
100
90
80
70
60
50
40
30
20
10
0
-10
-14
-13
-12
-11
-10
-9
-8
-7

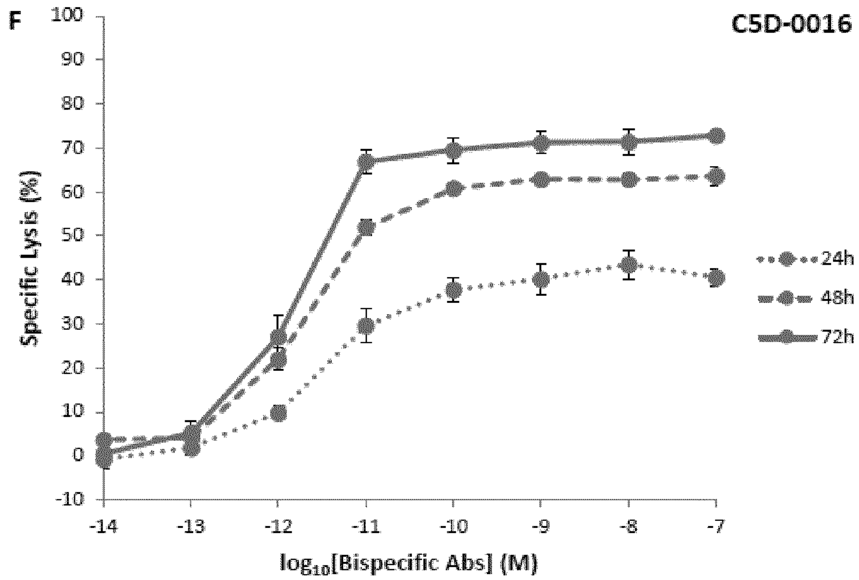
F
C5D-0016
Specific Lysis (%)
log10[Bispecific Abs] (M)
24h
48h
72h
100
90
80
70
60
50
40
30
20
10
0
-10
-14
-13
-12
-11
-10
-9
-8
-7

[Figure 274]

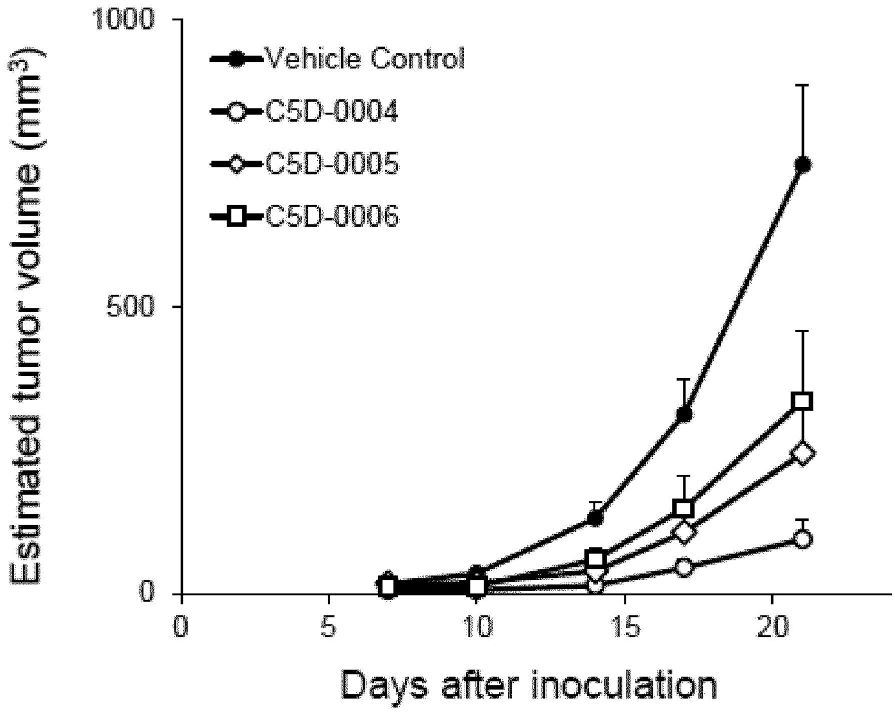
Estimated tumor volume ($mm^3$)
1000
500
0
Vehicle Control
C5D-0004
C5D-0005
C5D-0006
0
5
10
15
20
Days after inoculation

[Figure 275]

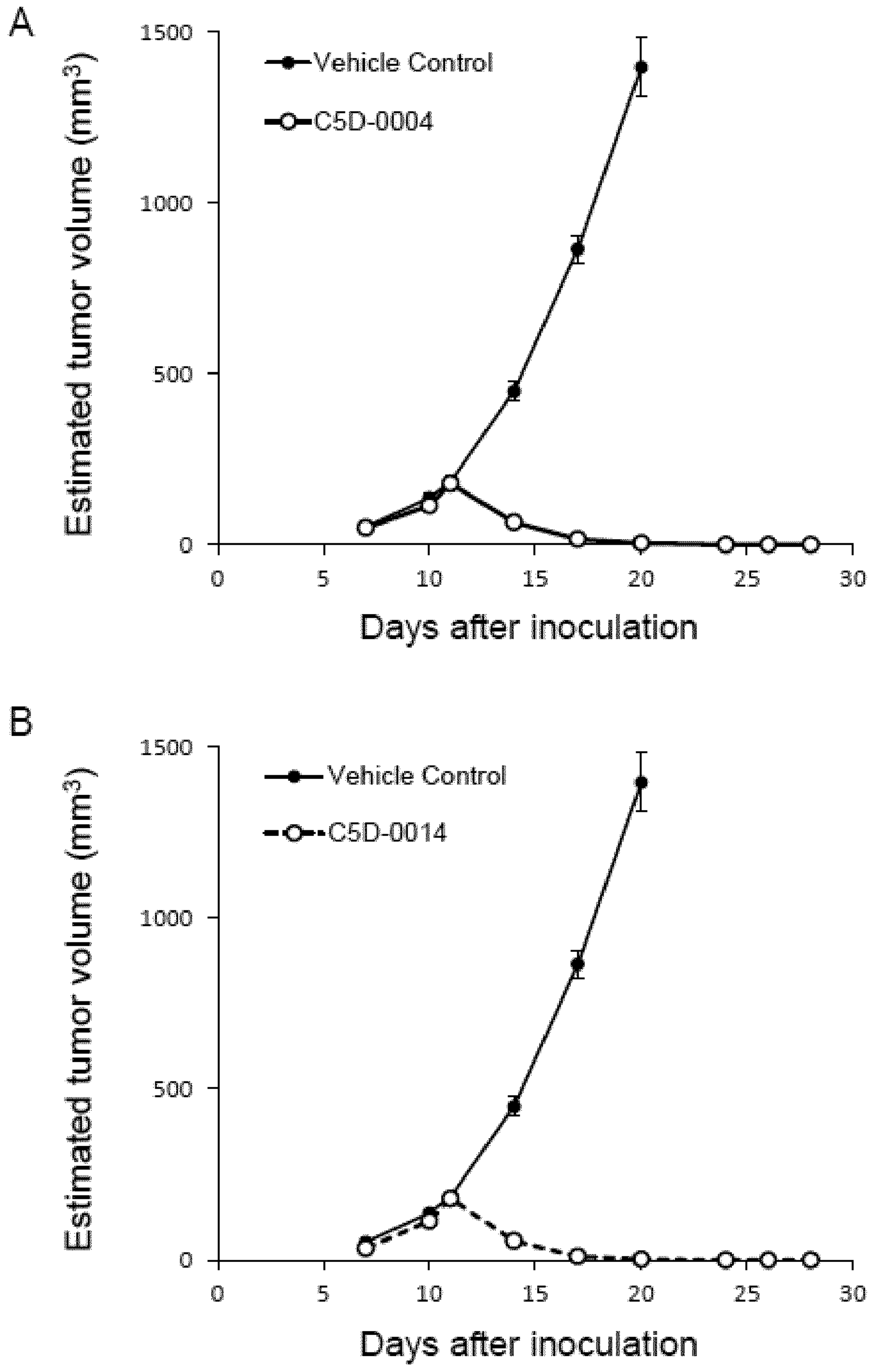
A
1500
1000
500
0
Estimated tumor volume (mm3)
Vehicle Control
C5D-0004
0
5
10
15
20
25
30
Days after inoculation
B
1500
1000
500
0
Estimated tumor volume (mm3)
Vehicle Control
C5D-0014
0
5
10
15
20
25
30
Days after inoculation

[Figure 276]

I T Xaa Xaa G G R I

(Xaa is an arbitrary natural amino acid residue)

(SEQ ID NO: 238 of the Sequence Listing)

[Figure 277]

R Xaa D

(Xaa is an arbitrary natural amino acid residue)

(SEQ ID NO: 239 of the Sequence Listing)

[Figure 278]

E V Q L V E S G G G L V Q P G G S L R L S C A A S G V T F N Y Y G M S

W I R Q A P G K G L E W V A S I T Xaa Xaa G G R I Y Y P D S V K G R F

T I S R E N T Q K T L Y L Q M N S L R A E D T A V Y Y C T L D G R D G

W V A Y W G Q G T L V T V S S

(Xaa is an arbitrary natural amino acid residue)

(SEQ ID NO: 240 of the Sequence Listing)

[Figure 279]

N F M L T Q P H S V S E S P G K T V T I S C K R N T G N I G S N Y V N

W Y Q Q H E G S S P T T I I Y R Xaa D K R P D G V S D R F S G S I D R

S S K S A S L T I S N L K T E D E A D Y F C Q S Y S S G F I F G G G T

K L T V L

(Xaa is an arbitrary natural amino acid residue)

(SEQ ID NO: 241of the Sequence Listing)

[Figure 280]

Q A V L T Q P S S V S G V P G Q R V T I S C K R N T G N I G S N Y V N

W Y Q Q L P G T A P K L L I Y R Xaa D K R P S G V P D R F S G S K S G

T S A S L A I T G F Q A E D E A D Y Y C Q S Y S S G F I F G G G T K L

T V L

(Xaa is an arbitrary natural amino acid residue)

(SEQ ID NO: 242 of the Sequence Listing)

[Figure 281]

N F M L T Q P S S V S G V P G Q R V T I S C T G N T G N I G S N Y V N

W Y Q Q L P G T A P K L L I Y R Xaa D K R P S G V P D R F S G S K S G

T S A S L A I T G F Q A E D E A D Y Y C Q S Y S S G F I F G G G T K L

T V L

(Xaa is an arbitrary natural amino acid residue)

(SEQ ID NO: 243 of the Sequence Listing)

[Figure 282]

GEVQLVESGGGLVQPGGSLRLSCAASGVTFNYYGMSWIRQAPGKGLEWVASITRSGGRI
YYPDSVKGRFTISRENTQKTLYLQMNSLRAEDTAVYYCTLDGRDGWVAYWGQGTLVTVS
SGGGGSGGGGSGGGGSNFMLTQPHSVSESPGKTVTISCKRNTGNIGSNYVNWYQQHEGS
SPTTIIYRDDKRPDGVSDRFSGSIDRSSKSASLTISNLKTEDEADYFCQSYSSGFIFGG
GTKLTVLGAAAGAGGDYKDDDDKGAAAHHHHHH
(SEQ ID NO: 244 of the Sequence Listing)

[Figure 283]
GEVQLVESGGGLVQPGGSLRLSCAASGVTFNYYGMSWIRQAPGKGLEWVASITRSGGRI
YYPDSVKGRFTISRENTQKTLYLQMNSLRAEDTAVYYCTLDGRDGWVAYWGQGTLVTVS
SGGGGSGGGGSGGGGSNFMLTQPSSVSGVPGQRVTISCTGNTGNIGSNYVNWYQQLPGT
APKLLIYRDDKRPSGVPDRFSGSKSGTSASLAITGFQAEDEADYYCQSYSSGFIFGGGT
KLTVLGAAAGAGGDYKDDDDKGAAAHHHHHH
(SEQ ID NO: 245 of the Sequence Listing)

[Figure 284]

GEVQLVESGGGLVQPGGSLRLSCAASGVTFNYYGMSWIRQAPGKGLEWVASITRSGGRI
YYPDSVKGRFTISRENTQKTLYLQMNSLRAEDTAVYYCTLDGRDGWVAYWGQGTLVTVS
SGGGGSGGGGSGGGGSNFMLTQPHSVSESPGKTVTISCKRNTGNIGSNYVNWYQQHEGS
SPTTIIYRGDKRPDGVSDRFSGSIDRSSKSASLTISNLKTEDEADYFCQSYSSGFIFGG
GTKLTVLGAAAGAGGDYKDDDDKGAAAHHHHHH

(SEQ ID NO: 246 of the Sequence Listing)

[Figure 285]

GEVQLVESGGGLVQPGGSLRLSCAASGVTFNYYGMSWIRQAPGKGLEWVASITRSGGRI
YYPDSVKGRFTISRENTQKTLYLQMNSLRAEDTAVYYCTLDGRDGWVAYWGQGTLVTVS
SGGGGSGGGGSGGGGSNFMLTQPHSVSESPGKTVTISCKRNTGNIGSNYVNWYQQHEGS
SPTTIIYRQDKRPDGVSDRFSGSIDRSSKSASLTISNLKTEDEADYFCQSYSSGFIFGG
GTKLTVLGAAAGAGGDYKDDDDKGAAAHHHHHH

(SEQ ID NO: 247 of the Sequence Listing)

[Figure 286]

GEVQLVESGGGLVQPGGSLRLSCAASGVTFNYYGMSWIRQAPGKGLEWVASITRSGGRI
YYPDSVKGRFTISRENTQKTLYLQMNSLRAEDTAVYYCTLDGRDGWVAYWGQGTLVTVS
SGGGGSGGGGSGGGGSNFMLTQPHSVSESPGKTVTISCKRNTGNIGSNYVNWYQQHEGS
SPTTIIYRNDKRPDGVSDRFSGSIDRSSKSASLTISNLKTEDEADYFCQSYSSGFIFGG
GTKLTVLGAAAGAGGDYKDDDDKGAAAHHHHHH

(SEQ ID NO: 248 of the Sequence Listing)

[Figure 287]

GEVQLVESGGGLVQPGGSLRLSCAASGVTFNYYGMSWIRQAPGKGLEWVASITRSGGRI
YYPDSVKGRFTISRENTQKTLYLQMNSLRAEDTAVYYCTLDGRDGWVAYWGQGTLVTVS
SGGGGSGGGGSGGGGSNFMLTQPHSVSESPGKTVTISCKRNTGNIGSNYVNWYQQHEGS
SPTTIIYRSDKRPDGVSDRFSGSIDRSSKSASLTISNLKTEDEADYFCQSYSSGFIFGG
GTKLTVLGAAAGAGGDYKDDDDKGAAAHHHHHH

(SEQ ID NO: 249 of the Sequence Listing)

[Figure 288]

GEVQLVESGGGLVQPGGSLRLSCAASGVTFNYYGMSWIRQAPGKGLEWVASITRSGGRI
YYPDSVKGRFTISRENTQKTLYLQMNSLRAEDTAVYYCTLDGRDGWVAYWGQGTLVTVS
SGGGGSGGGGSGGGGSNFMLTQPHSVSESPGKTVTISCKRNTGNIGSNYVNWYQQHEGS
SPTTIIYRADKRPDGVSDRFSGSIDRSSKSASLTISNLKTEDEADYFCQSYSSGFIFGG
GTKLTVLGAAAGAGGDYKDDDDKGAAAHHHHHH

(SEQ ID NO: 250 of the Sequence Listing)

[Figure 289]

(SEQ ID NO: 251 of the Sequence Listing)

[Figure 290]

GEVQLVESGGGLVQPGGSLRLSCAASGVTFNYYGMSWIRQAPGKGLEWVASITSSGGRI
YYPDSVKGRFTISRENTQKTLYLQMNSLRAEDTAVYYCTLDGRDGWVAYWGQGTLVTVS
SGGGGSGGGGSGGGGSNFMLTQPHSVSESPGKTVTISCKRNTGNIGSNYVNWYQQHEGS
SPTTIIYRGDKRPDGVSDRFSGSIDRSSKSASLTISNLKTEDEADYFCQSYSSGFIFGG
GTKLTVLGAAAGAGGDYKDDDDKGAAAHHHHHH

(SEQ ID NO: 252 of the Sequence Listing)

[Figure 291]

GEVQLVESGGGLVQPGGSLRLSCAASGVTFNYYGMSWIRQAPGKGLEWVASITSSGGRI
YYPDSVKGRFTISRENTQKTLYLQMNSLRAEDTAVYYCTLDGRDGWVAYWGQGTLVTVS
SGGGGSGGGGSGGGGSNFMLTQPHSVSESPGKTVTISCKRNTGNIGSNYVNWYQQHEGS
SPTTIIYRNDKRPDGVSDRFSGSIDRSSKSASLTISNLKTEDEADYFCQSYSSGFIFGG
GTKLTVLGAAAGAGGDYKDDDDKGAAAHHHHHH

(SEQ ID NO: 253 of the Sequence Listing)

[Figure 292]

GEVQLVESGGGLVQPGGSLRLSCAASGVTFNYYGMSWIRQAPGKGLEWVASITSSGGRI
YYPDSVKGRFTISRENTQKTLYLQMNSLRAEDTAVYYCTLDGRDGWVAYWGQGTLVTVS
SGGGGSGGGGSGGGGSNFMLTQPHSVSESPGKTVTISCKRNTGNIGSNYVNWYQQHEGS
SPTTIIYRSDKRPDGVSDRFSGSIDRSSKSASLTISNLKTEDEADYFCQSYSSGFIFGG
GTKLTVLGAAAGAGGDYKDDDDKGAAAHHHHHH
(SEQ ID NO: 254 of the Sequence Listing)

[Figure 293]

GEVQLVESGGGLVQPGGSLRLSCAASGVTFNYYGMSWIRQAPGKGLEWVASITSSGGRI
YYPDSVKGRFTISRENTQKTLYLQMNSLRAEDTAVYYCTLDGRDGWVAYWGQGTLVTVS
SGGGGSGGGGSGGGGSNFMLTQPHSVSESPGKTVTISCKRNTGNIGSNYVNWYQQHEGS
SPTTIIYRADKRPDGVSDRFSGSIDRSSKSASLTISNLKTEDEADYFCQSYSSGFIFGG
GTKLTVLGAAAGAGGDYKDDDDKGAAAHHHHHH
(SEQ ID NO: 255 of the Sequence Listing)

**INTERNATIONAL SEARCH REPORT**

International application No.
PCT/JP2018/003888

A. CLASSIFICATION OF SUBJECT MATTER
Int.Cl. C12N15/09(2006.01)i, A61K39/395(2006.01)i, A61P35/00(2006.01)i, A61P35/02(2006.01)i, C07K16/28(2006.01)i, C07K16/46(2006.01)i, C12N1/15(2006.01)i, C12N1/19(2006.01)i, C12N1/21(2006.01)i, C12N5/10(2006.01)i, C12P21/08(2006.01)i

According to International Patent Classification (IPC) or to both national classification and IPC

B. FIELDS SEARCHED
Minimum documentation searched (classification system followed by classification symbols)
Int.Cl. C12N15/00-15/90, C07K1/00-19/00, A61K39/00-39/44, A61P1/00-43/00, C12N1/00-7/08, C12P21/08

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
JSTPlus/JMEDPlus/JST7580 (JDreamIII), CAplus/REGISTRY/MEDLINE/EMBASE/BIOSIS/WPIDS (STN), UniProt/GeneSeq

C. DOCUMENTS CONSIDERED TO BE RELEVANT

| Category* | Citation of document, with indication, where appropriate, of the relevant passages | Relevant to claim No. |
|---|---|---|
| X<br>A | WO 2016/090329 A2 (MEMORIAL SLOAN-KETTERING CANCER CENTER) 09 June 2016, claims, page 185, line 22 to page 186, line 5, examples, table 36 & EP 3227324 A2 & CN 107206077 A & KR 10-2017-0108945 A | 13-27, 32-54, 67-87<br>1-12, 28-31, 55-66 |
| P, X | WO 2018/017786 A2 (JANSSEN PHARMACEUTICA NV) 25 January 2018, claims, page 11, lines 1-6, examples & US 2018/0037651 A1 | 28-49, 51-64, 66-87 |
| A | WO 2016/090312 A1 (MEMORIAL SLOAN-KETTERING CANCER CENTER) 09 June 2016 & EP 3227339 A1 & KR 10-2017-0109537 A & CN 107428829 A | 1-87 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 19 April 2018 (19.04.2018) | 01 May 2018 (01.05.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2016090329 A
- WO 2013063702 A
- WO 9627011 A
- WO 2009089004 A
- WO 2014110601 A
- WO 2010151792 A
- WO 1990007861 A
- WO 2013147153 A
- WO 995432 A
- WO 0061739 A
- WO 0231140 A
- WO 0948072 A
- WO 9007861 A
- US 6972323 B
- WO 9201047 A
- WO 9220791 A
- WO 9306213 A
- WO 9311236 A
- WO 9319172 A
- WO 9501438 A
- WO 9515388 A
- US 4946778 A
- US 5260203 A
- US 5091513 A
- US 5455030 A
- WO 2004061104 A
- US 7129330 B
- US 6214388 B
- WO 2014190441 A
- WO 2009137911 A


### Non-patent literature cited in the description


- **H BRAUNER-OSBORNE et al.** *Biochim Biophys Acta,* April 2001, vol. 1518 (3), 237-248
- **S INOUE et al.** *Journal of Investigative Dermatology,* March 2004, vol. 122 (3), 565-573
- **J ATAMANIUK et al.** *European Journal of Clinical Investigation,* May 2012, vol. 42 (9), 953-960
- **Y COHEN et al.** *Hematology,* November 2013, vol. 18 (6), 348-351
- *Developmental and Comparative Immunology,* 2003, vol. 27, 55-77
- **SAIKI, R.K. et al.** *Science,* 1988, vol. 239, 487-489
- *Nature,* 1986, vol. 321, 522-525
- **LEE, H-S et al.** *Molecular Immunology,* 1999, vol. 36, 61-71
- **SHIRRMANN, T. et al.** *mAbs,* 2010, vol. 2 (1), 1-4
- **MUYLDEMANS S. et al.** *Protein Eng.,* 1994, vol. 7 (9), 1129-35
- **HAMERS-CASTERMAN C. et al.** *Nature,* 1993, vol. 363 (6428), 446-448
- *Journal of Chromatography A,* 1995, vol. 705, 129-134
- *Analytical Biochemistry,* 2007, vol. 360, 75-83
- *Methods Mol Biol.,* 2013, vol. 1045, 1-27
- *Nature Biotechnology,* 2005, vol. 23, 1137-1146
- *Protein Eng Des Sel.,* 2012, (2), 81-8
- **ARS KJER-NIELSEN et al.** *PNAS,* 2004
- **KELLY L ARNETT et al.** *PNAS,* 2004
- **KOHLER ; MILSTEIN.** *Nature,* 1975, vol. 256, 495-497
- Monoclonal Antibodies. Plenum Press, 1980, 365-367
- **TOMIZUKA, K. et al.** *Nature Genetics,* 1997, vol. 16, 133-143
- **KUROIWA, Y.** *Nuc. Acids Res.,* 1998, vol. 26, 3447-3448
- **YOSHIDA, H.** Animal Cell Technology: Basic and Applied Aspects. Kluwer Academic Publishers, 1999, vol. 10, 69-73
- **TOMIZUKA, K.** *Proc. Natl. Acad. Sci. USA,* 2000, vol. 97, 722-727
- *Annu. Rev. Immunol,* 1994, vol. 12, 433-455
- *J Biol Chem,* 1999, vol. 274 (26), 18218-30
- *Methods Mol Biol,* 2002, vol. 178, 59-71
- *J Immunol Methods,* 1997, vol. 201 (1), 35-55
- **HUSTON, J.S. et al.** *PNAS,* 1988, vol. 85, 5879-5883
- **SUBEDI GP et al.** *J Vis Exp.,* 2015, 106
- **GLUZMAN, Y.** *Cell,* 1981, vol. 23, 175-182
- **CHODHFR ; URLAUB, G. ; CHASIN, L.A.** *PNAS,* 1980, vol. 77, 4126-4220
- Molecular Cloning. Cold Spring Harbor Laboratory Press, 1989
- *Methods in Enzymology,* 1991, vol. 203, 121-153
- *Nuc. Acid Res.,* 2007, vol. 35, D301-D303

- *Proc. Natl. Acad. Sci. USA,* 1989, vol. 86, 10029-10033
- **KABAT et al.** Sequences of Proteins of Immunological Interest. Public Health Service National Institutes, 1991
- **QUEEN et al.** *Proc. Natl. Acad. Sci. USA,* 1989, vol. 86, 10029-10033
- **ZACCOLO et al.** *J. Mol. Biol.,* 1996, vol. 255, 589-603
- **RAYMOND C. et al.** Methods. National Research Council Canada, 2011, vol. 55, 44-51

SEQUENCE LISTING

<110> DAIICHI SANKYO COMPANY, LIMITED

<120> ANTI-GPRC5D ANTIBODY AND MOLECULE COMPRISING THE ANTIBODY

<130> FP-1804

<150> JP2017/020220
<151> 2017-02-07

<160> 255

<170> PatentIn version 3.5

<210> 1
<211> 27
<212> PRT
<213> Homo sapiens

<220>
<221> MISC_FEATURE
<222> (27)..(27)
<223> (27) Xaa is biotinylated Glutamic Acid

<400> 1

```
Met Tyr Lys Asp Cys Ile Glu Ser Thr Gly Asp Tyr Phe Leu Leu Cys
1               5                   10                  15


Asp Ala Glu Gly Pro Trp Gly Ile Ile Leu Xaa
            20                  25
```

<210> 2
<211> 28
<212> PRT
<213> Homo sapiens

<220>
<221> misc_feature
<222> (28)..(28)
<223> (28) Xaa is biotinylated Lysine

<400> 2

```
Met Tyr Lys Asp Cys Ile Glu Ser Thr Gly Asp Tyr Phe Leu Leu Cys
1               5                   10                  15


Asp Ala Glu Gly Pro Trp Gly Ile Ile Leu Glu Xaa
            20                  25
```

<210> 3
<211> 29
<212> DNA
<213> Artificial Sequence

<220>

<223>  primer for amplifying cDNA encording 2A4 VH by PCR

<400>  3
ctccagagtt ccaggtcacg gtgactggc                                  29

<210>  4
<211>  366
<212>  DNA
<213>  Rattus norvegicus

<400>  4
caggtccagt tgcagcaatc tggagctgag ctggctaaac ctgggacttc agtgaagctg      60

tcctgcaagg cttctggcta taccttcacc agctactata tttactgggt aaagcagagg     120

cctggacagg gccttgagtg gatcggatat gtttatcctg gatatggtgg tacttactac     180

agtgataagt tcaagggcaa agccacattt actgcagaca catcctccag cacagcctac     240

atgctactgg gcagcctgac acctgaggac tctgcgtact atttctgtgc aagacggaag     300

ggtataattc ggggtccggg gtactttgat tactggggcc aaggagtcat ggtcacagtc     360

tcctca                                                            366

<210>  5
<211>  122
<212>  PRT
<213>  Rattus norvegicus

<400>  5

Gln Val Gln Leu Gln Gln Ser Gly Ala Glu Leu Ala Lys Pro Gly Thr
1               5                   10                  15

Ser Val Lys Leu Ser Cys Lys Ala Ser Gly Tyr Thr Phe Thr Ser Tyr
            20                  25                  30

Tyr Ile Tyr Trp Val Lys Gln Arg Pro Gly Gln Gly Leu Glu Trp Ile
        35                  40                  45

Gly Tyr Val Tyr Pro Gly Tyr Gly Gly Thr Tyr Tyr Ser Asp Lys Phe
    50                  55                  60

Lys Gly Lys Ala Thr Phe Thr Ala Asp Thr Ser Ser Ser Thr Ala Tyr
65                  70                  75                  80

Met Leu Leu Gly Ser Leu Thr Pro Glu Asp Ser Ala Tyr Tyr Phe Cys
                85                  90                  95

Ala Arg Arg Lys Gly Ile Ile Arg Gly Pro Gly Tyr Phe Asp Tyr Trp
            100                 105                 110

Gly Gln Gly Val Met Val Thr Val Ser Ser

115 120

<210> 6
<211> 369
<212> DNA
<213> Rattus norvegicus

<400> 6
caggttactc tgaaagagtc tggccctggg atattgcagc cctcccagac cctcagtctg 60

acttgcactt tctctgggtt ttcactgaac acttatgata tgggtgtggg ctggattcgt 120

cagccttcag ggaagggtct ggagtggctg gcaaacattt ggtgggatga tgataagtac 180

tacaatccat ctctgagaaa ccggctcaca atctccaagg acacctccaa caaccaagca 240

ttcctcaaga tcaccaatgt ggacactgca gatactgcca catactactg tgctcggatc 300

gaaactgtcc gggtttcccg taaggggttt gctcactggg gccaaggcac tctggtcact 360

gtctcttca 369

<210> 7
<211> 123
<212> PRT
<213> Rattus norvegicus

<400> 7

Gln Val Thr Leu Lys Glu Ser Gly Pro Gly Ile Leu Gln Pro Ser Gln
1 5 10 15

Thr Leu Ser Leu Thr Cys Thr Phe Ser Gly Phe Ser Leu Asn Thr Tyr
20 25 30

Asp Met Gly Val Gly Trp Ile Arg Gln Pro Ser Gly Lys Gly Leu Glu
35 40 45

Trp Leu Ala Asn Ile Trp Trp Asp Asp Asp Lys Tyr Tyr Asn Pro Ser
50 55 60

Leu Arg Asn Arg Leu Thr Ile Ser Lys Asp Thr Ser Asn Asn Gln Ala
65 70 75 80

Phe Leu Lys Ile Thr Asn Val Asp Thr Ala Asp Thr Ala Thr Tyr Tyr
85 90 95

Cys Ala Arg Ile Glu Thr Val Arg Val Ser Arg Lys Gly Phe Ala His
100 105 110

Trp Gly Gln Gly Thr Leu Val Thr Val Ser Ser
115 120

<210> 8
<211> 369
<212> DNA
<213> Rattus norvegicus

<400> 8
gagatacacc tgcaggagtc aggacctggc cttgtgaaac cttcacagtc actctccctc 60

acctgttctg tcactggtta caccattacc agtggttatg attggagctg gatccggaag 120

ttcccaggaa ataaaatgga gtggatggca tacatgagct atagaggtag cactaactac 180

aacccctcgc tcaaaagtcg aatctccatt acaagagaca catccaagaa tcagttcttc 240

ctgcagttga attctgtcac tactgaggat acagccacat attactgtgc cctaacaagg 300

acctattggt ataactatta ctatgttttg gatgcctggg gtcaaggagc ttcagtcact 360

gtctcctca 369

<210> 9
<211> 123
<212> PRT
<213> Rattus norvegicus

<400> 9

Glu Ile His Leu Gln Glu Ser Gly Pro Gly Leu Val Lys Pro Ser Gln
1 5 10 15

Ser Leu Ser Leu Thr Cys Ser Val Thr Gly Tyr Thr Ile Thr Ser Gly
20 25 30

Tyr Asp Trp Ser Trp Ile Arg Lys Phe Pro Gly Asn Lys Met Glu Trp
35 40 45

Met Ala Tyr Met Ser Tyr Arg Gly Ser Thr Asn Tyr Asn Pro Ser Leu
50 55 60

Lys Ser Arg Ile Ser Ile Thr Arg Asp Thr Ser Lys Asn Gln Phe Phe
65 70 75 80

Leu Gln Leu Asn Ser Val Thr Thr Glu Asp Thr Ala Thr Tyr Tyr Cys
85 90 95

Ala Leu Thr Arg Thr Tyr Trp Tyr Asn Tyr Tyr Tyr Val Leu Asp Ala
100 105 110

Trp Gly Gln Gly Ala Ser Val Thr Val Ser Ser
115 120

<210> 10
<211> 28
<212> DNA

<213> Artificial Sequence

<220>
<223> primer for amplifying cDNA of 2A4_VH by PCR

<400> 10
tcagtaacac tgtccaggac accatctc 28

<210> 11
<211> 321
<212> DNA
<213> Rattus norvegicus

<400> 11
gacatccaga tgacacagtc tccagcttcc ctgtctgcat ctctgggaga aactgtctcc 60

atcgaatgtc ttgcaagtga gggcatttcc aatagtttag cgtggtatca gcagaagcca 120

gggaaatctc ctcagctcct gatctatggt gcaagtagct tgcaagacgg ggtcccatca 180

cggttcagtg gcagtggttc tggcacgcag tattctctca agatcagcgg catgcaacct 240

gaagatgaag ggctttatta ctgtcaacag ggttacaagt atcctccgac gttcggtgga 300

ggcaccaagc tggaattgaa a 321

<210> 12
<211> 107
<212> PRT
<213> Rattus norvegicus

<400> 12

Asp Ile Gln Met Thr Gln Ser Pro Ala Ser Leu Ser Ala Ser Leu Gly
1               5                   10                  15

Glu Thr Val Ser Ile Glu Cys Leu Ala Ser Glu Gly Ile Ser Asn Ser
            20                  25                  30

Leu Ala Trp Tyr Gln Gln Lys Pro Gly Lys Ser Pro Gln Leu Leu Ile
        35                  40                  45

Tyr Gly Ala Ser Ser Leu Gln Asp Gly Val Pro Ser Arg Phe Ser Gly
    50                  55                  60

Ser Gly Ser Gly Thr Gln Tyr Ser Leu Lys Ile Ser Gly Met Gln Pro
65                  70                  75                  80

Glu Asp Glu Gly Leu Tyr Tyr Cys Gln Gln Gly Tyr Lys Tyr Pro Pro
                85                  90                  95

Thr Phe Gly Gly Gly Thr Lys Leu Glu Leu Lys
            100                 105

<210> 13
<211> 321
<212> DNA
<213> Rattus norvegicus

<400> 13
gaaactgtga tgacccagtc tcccacatcc atgtccacat caataggaga gagggtcacc 60

ctgaactgca aggccagtca gagtgtgggt attaatgtag actggtacca acagacacca 120

gggcagtctc ctaaactgct gatatatggg gcatccaacc ggcacactgg ggtccctgat 180

cgcttcacag gcagtggatt tgggagagat ttcactctca ccatcagcaa cgtggaggct 240

gaagacctgg ctgtttatta ctgtctgcag catggctcca ttcctccgac gttcggtgga 300

ggcaccaagc tggaattgaa a 321

<210> 14
<211> 107
<212> PRT
<213> Rattus norvegicus

<400> 14

Glu Thr Val Met Thr Gln Ser Pro Thr Ser Met Ser Thr Ser Ile Gly
1 5 10 15

Glu Arg Val Thr Leu Asn Cys Lys Ala Ser Gln Ser Val Gly Ile Asn
20 25 30

Val Asp Trp Tyr Gln Gln Thr Pro Gly Gln Ser Pro Lys Leu Leu Ile
35 40 45

Tyr Gly Ala Ser Asn Arg His Thr Gly Val Pro Asp Arg Phe Thr Gly
50 55 60

Ser Gly Phe Gly Arg Asp Phe Thr Leu Thr Ile Ser Asn Val Glu Ala
65 70 75 80

Glu Asp Leu Ala Val Tyr Tyr Cys Leu Gln His Gly Ser Ile Pro Pro
85 90 95

Thr Phe Gly Gly Gly Thr Lys Leu Glu Leu Lys
100 105

<210> 15
<211> 318
<212> DNA
<213> Rattus norvegicus

<400> 15
gacatccaga tgacccagtc tccttcattc ctgtctgcat ctgtgggaga aagagtcact 60

ctcagctgca aagcaagtca gaatattaac aagtacttag actggtatca gcaaaagctt 120

ggagaacctc ccaaactcct gatatataat acaaacaatt tgcatacggg catcccatca 180

aggttcagtg gcagtggatc tggtacagat tacacactca ccatcagcgg cctgcagcct 240

gaagatgttg ccacatattt ctgcttgcag cgtaatagtt ggtacacgtt tggagctggg 300

accaagctgg aactgaaa 318

<210> 16
<211> 106
<212> PRT
<213> Rattus norvegicus

<400> 16

Asp Ile Gln Met Thr Gln Ser Pro Ser Phe Leu Ser Ala Ser Val Gly
1 5 10 15

Glu Arg Val Thr Leu Ser Cys Lys Ala Ser Gln Asn Ile Asn Lys Tyr
20 25 30

Leu Asp Trp Tyr Gln Gln Lys Leu Gly Glu Pro Pro Lys Leu Leu Ile
35 40 45

Tyr Asn Thr Asn Asn Leu His Thr Gly Ile Pro Ser Arg Phe Ser Gly
50 55 60

Ser Gly Ser Gly Thr Asp Tyr Thr Leu Thr Ile Ser Gly Leu Gln Pro
65 70 75 80

Glu Asp Val Ala Thr Tyr Phe Cys Leu Gln Arg Asn Ser Trp Tyr Thr
85 90 95

Phe Gly Ala Gly Thr Lys Leu Glu Leu Lys
100 105

<210> 17
<211> 449
<212> DNA
<213> Artificial Sequence

<220>
<223> DNA fragment comprising DNA sequence encording amino acid sequence of human k-chain of secretion signal sequence and human k-chain of constant region

<400> 17
gcctccggac tctagagcca ccatggtgct gcagacccag gtgttcatct ccctgctgct 60

gtggatctcc ggcgcgtacg gcgatatcgt gatgattaaa cgtacggtgg ccgccccctc 120

cgtgttcatc ttcccccct ccgacgagca gctgaagtcc ggcaccgcct ccgtggtgtg 180

cctgctgaat aacttctacc ccagagaggc caaggtgcag tggaaggtgg acaacgccct 240

gcagtccggg aactcccagg agagcgtgac cgagcaggac agcaaggaca gcacctacag 300

cctgagcagc accctgaccc tgagcaaagc cgactacgag aagcacaagg tgtacgcctg 360

cgaggtgacc caccagggcc tgagctcccc cgtcaccaag agcttcaaca gggggagtg 420

ttaggggccc gtttaaacgg gggaggcta 449

<210> 18
<211> 30
<212> DNA
<213> Artificial Sequence

<220>
<223> primer F for expression vector of VL

<400> 18
tataccgtcg acctctagct agagcttggc 30

<210> 19
<211> 30
<212> DNA
<213> Artificial Sequence

<220>
<223> primer R for expression vector of VL

<400> 19
gctatggcag ggcctgccgc cccgacgttg 30

<210> 20
<211> 1132
<212> DNA
<213> Artificial Sequence

<220>
<223> DNA fragment comprising DNA sequence encording amino acid sequence of human heavy chain of signal sequence and human IgG1 constant region

<400> 20
gcctccggac tctagagcca ccatgaaaca cctgtggttc ttcctcctgc tggtggcagc 60

tcccagatgg gtgctgagcc aggtgcaatt gtgcaggcgg ttagctcagc ctccaccaag 120

ggcccaagcg tcttcccct ggcaccctcc tccaagagca cctctggcgg cacagccgcc 180

ctgggctgcc tggtcaagga ctacttcccc gaacccgtga ccgtgagctg gaactcaggc 240

gccctgacca gcggcgtgca caccttcccc gctgtcctgc agtcctcagg actctactcc 300

ctcagcagcg tggtgaccgt gccctccagc agcttgggca cccagaccta catctgcaac 360

gtgaatcaca agcccagcaa caccaaggtg gacaagagag ttgagcccaa atcttgtgac 420

aaaactcaca catgcccacc ctgcccagca cctgaactcc tggggggacc ctcagtcttc 480

ctcttccccc caaaacccaa ggacaccctc atgatctccc ggacccctga ggtcacatgc 540

gtggtggtgg acgtgagcca cgaagaccct gaggtcaagt tcaactggta cgtggacggc 600

gtggaggtgc ataatgccaa gacaaagccc cgggaggagc agtacaacag cacgtaccgg 660

gtggtcagcg tcctcaccgt cctgcaccag gactggctga atggcaagga gtacaagtgc 720

aaggtctcca acaaagccct cccagccccc atcgagaaaa ccatctccaa agccaaaggc 780

cagccccggg aaccacaggt gtacaccctg cccccatccc gggaggagat gaccaagaac 840

caggtcagcc tgacctgcct ggtcaaaggc ttctatccca gcgacatcgc cgtggagtgg 900

gagagcaatg gccagcccga gaacaactac aagaccaccc ctcccgtgct ggactccgac 960

ggctccttct tcctctacag caagctcacc gtggacaaga gcaggtggca gcagggcaac 1020

gtcttctcat gctccgtgat gcatgaggct ctgcacaacc actacaccca gaagagcctc 1080

tccctgtctc ccggcaaatg agatatcggg cccgtttaaa cgggggaggc ta 1132

<210> 21
<211> 702
<212> DNA
<213> Artificial Sequence

<220>
<223> nucleotide sequence of c2A4 L

<400> 21
atggtgctgc agacccaggt gttcatctcc ctgctgctgt ggatctccgg cgcgtacggc 60

gacatccaga tgacacagtc tccagcttcc ctgtctgcat ctctgggaga aactgtctcc 120

atcgaatgtc ttgcaagtga gggcatttcc aatagtttag cgtggtatca gcagaagcca 180

gggaaatctc ctcagctcct gatctatggt gcaagtagct tgcaagacgg ggtcccatca 240

cggttcagtg gcagtggttc tggcacgcag tattctctca agatcagcgg catgcaacct 300

gaagatgaag ggctttatta ctgtcaacag ggttacaagt atcctccgac gttcggtgga 360

ggcaccaagc tggaattgaa acgggctgtg gccgccccct ccgtgttcat cttccccccc 420

tccgacgagc agctgaagtc cggcaccgcc tccgtggtgt gcctgctgaa taacttctac 480

cccagagagg ccaaggtgca gtggaaggtg gacaacgccc tgcagtccgg gaactcccag 540

gagagcgtga ccgagcagga cagcaaggac agcacctaca gcctgagcag caccctgacc 600

ctgagcaaag ccgactacga gaagcacaag gtgtacgcct gcgaggtgac ccaccagggc 660

ctgagctccc ccgtcaccaa gagcttcaac aggggggagt gt 702

<210> 22
<211> 234
<212> PRT
<213> Artificial Sequence

<220>
<223> amino acid sequence of c2A4 L

<400> 22

```
Met Val Leu Gln Thr Gln Val Phe Ile Ser Leu Leu Leu Trp Ile Ser
1               5                   10                  15

Gly Ala Tyr Gly Asp Ile Gln Met Thr Gln Ser Pro Ala Ser Leu Ser
            20                  25                  30

Ala Ser Leu Gly Glu Thr Val Ser Ile Glu Cys Leu Ala Ser Glu Gly
        35                  40                  45

Ile Ser Asn Ser Leu Ala Trp Tyr Gln Gln Lys Pro Gly Lys Ser Pro
    50                  55                  60

Gln Leu Leu Ile Tyr Gly Ala Ser Ser Leu Gln Asp Gly Val Pro Ser
65                  70                  75                  80

Arg Phe Ser Gly Ser Gly Ser Gly Thr Gln Tyr Ser Leu Lys Ile Ser
                85                  90                  95

Gly Met Gln Pro Glu Asp Glu Gly Leu Tyr Tyr Cys Gln Gln Gly Tyr
            100                 105                 110

Lys Tyr Pro Pro Thr Phe Gly Gly Gly Thr Lys Leu Glu Leu Lys Arg
        115                 120                 125

Ala Val Ala Ala Pro Ser Val Phe Ile Phe Pro Pro Ser Asp Glu Gln
    130                 135                 140

Leu Lys Ser Gly Thr Ala Ser Val Val Cys Leu Leu Asn Asn Phe Tyr
145                 150                 155                 160

Pro Arg Glu Ala Lys Val Gln Trp Lys Val Asp Asn Ala Leu Gln Ser
                165                 170                 175

Gly Asn Ser Gln Glu Ser Val Thr Glu Gln Asp Ser Lys Asp Ser Thr
            180                 185                 190

Tyr Ser Leu Ser Ser Thr Leu Thr Leu Ser Lys Ala Asp Tyr Glu Lys
        195                 200                 205

His Lys Val Tyr Ala Cys Glu Val Thr His Gln Gly Leu Ser Ser Pro
    210                 215                 220

Val Thr Lys Ser Phe Asn Arg Gly Glu Cys
225                 230
```

<210> 23

<211> 44
<212> DNA
<213> Artificial Sequence

<220>
<223> primer set F for c2A4 L

<400> 23
atctccggcg cgtacggcga catccagatg acacagtctc cagc 44

<210> 24
<211> 44
<212> DNA
<213> Artificial Sequence

<220>
<223> primer set R for c2A4 L

<400> 24
ggaggggcg gccacagccc gtttcaattc cagcttggtg cctc 44

<210> 25
<211> 1413
<212> DNA
<213> Artificial Sequence

<220>
<223> nucleotide sequence of c2A4 H

<400> 25
atgaaacacc tgtggttctt cctcctgctg gtggcagctc ccagatgggt gctgagccag 60

gtccagttgc agcaatctgg agctgagctg gctaaacctg ggacttcagt gaagctgtcc 120

tgcaaggctt ctggctatac cttcaccagc tactatattt actgggtaaa gcagaggcct 180

ggacagggcc ttgagtggat cggatatgtt tatcctggat atggtggtac ttactacagt 240

gataagttca agggcaaagc cacatttact gcagacacat cctccagcac agcctacatg 300

ctactgggca gcctgacacc tgaggactct gcgtactatt tctgtgcaag acggaagggt 360

ataattcggg gtccggggta ctttgattac tggggccaag gagtcatggt cacagtcagc 420

tcagcctcca ccaagggccc aagcgtcttc cccctggcac cctcctccaa gagcacctct 480

ggcggcacag ccgccctggg ctgcctggtc aaggactact tccccgaacc cgtgaccgtg 540

agctggaact caggcgccct gaccagcggc gtgcacacct tccccgctgt cctgcagtcc 600

tcaggactct actccctcag cagcgtggtg accgtgccct ccagcagctt gggcacccag 660

acctacatct gcaacgtgaa tcacaagccc agcaacacca aggtggacaa gagagttgag 720

cccaaatctt gtgacaaaac tcacacatgc ccaccctgcc cagcacctga actcctgggg 780

ggaccctcag tcttcctctt ccccccaaaa cccaaggaca ccctcatgat ctcccggacc 840

cctgaggtca catgcgtggt ggtggacgtg agccacgaag accctgaggt caagttcaac 900

tggtacgtgg acggcgtgga ggtgcataat gccaagacaa agccccggga ggagcagtac 960

aacagcacgt accgggtggt cagcgtcctc accgtcctgc accaggactg gctgaatggc 1020

aaggagtaca agtgcaaggt ctccaacaaa gccctcccag cccccatcga gaaaaccatc 1080

tccaaagcca aaggccagcc ccgggaacca caggtgtaca ccctgccccc atcccgggag 1140

gagatgacca agaaccaggt cagcctgacc tgcctggtca aaggcttcta tcccagcgac 1200

atcgccgtgg agtgggagag caatggccag cccgagaaca actacaagac cacccctccc 1260

gtgctggact ccgacggctc cttcttcctc tacagcaagc tcaccgtgga caagagcagg 1320

tggcagcagg gcaacgtctt ctcatgctcc gtgatgcatg aggctctgca caaccactac 1380

acccagaaga gcctctccct gtctcccggc aaa 1413

<210> 26
<211> 471
<212> PRT
<213> Artificial Sequence

<220>
<223> amino acid sequence of c2A4 H

<400> 26

Met Lys His Leu Trp Phe Phe Leu Leu Leu Val Ala Ala Pro Arg Trp
1 5 10 15

Val Leu Ser Gln Val Gln Leu Gln Gln Ser Gly Ala Glu Leu Ala Lys
20 25 30

Pro Gly Thr Ser Val Lys Leu Ser Cys Lys Ala Ser Gly Tyr Thr Phe
35 40 45

Thr Ser Tyr Tyr Ile Tyr Trp Val Lys Gln Arg Pro Gly Gln Gly Leu
50 55 60

Glu Trp Ile Gly Tyr Val Tyr Pro Gly Tyr Gly Gly Thr Tyr Tyr Ser
65 70 75 80

Asp Lys Phe Lys Gly Lys Ala Thr Phe Thr Ala Asp Thr Ser Ser Ser
85 90 95

Thr Ala Tyr Met Leu Leu Gly Ser Leu Thr Pro Glu Asp Ser Ala Tyr
100 105 110

Tyr Phe Cys Ala Arg Arg Lys Gly Ile Ile Arg Gly Pro Gly Tyr Phe
115 120 125

Asp Tyr Trp Gly Gln Gly Val Met Val Thr Val Ser Ser Ala Ser Thr
130 135 140

Lys Gly Pro Ser Val Phe Pro Leu Ala Pro Ser Ser Lys Ser Thr Ser
145 150 155 160

Gly Gly Thr Ala Ala Leu Gly Cys Leu Val Lys Asp Tyr Phe Pro Glu
165 170 175

Pro Val Thr Val Ser Trp Asn Ser Gly Ala Leu Thr Ser Gly Val His
180 185 190

Thr Phe Pro Ala Val Leu Gln Ser Ser Gly Leu Tyr Ser Leu Ser Ser
195 200 205

Val Val Thr Val Pro Ser Ser Ser Leu Gly Thr Gln Thr Tyr Ile Cys
210 215 220

Asn Val Asn His Lys Pro Ser Asn Thr Lys Val Asp Lys Arg Val Glu
225 230 235 240

Pro Lys Ser Cys Asp Lys Thr His Thr Cys Pro Pro Cys Pro Ala Pro
245 250 255

Glu Leu Leu Gly Gly Pro Ser Val Phe Leu Phe Pro Pro Lys Pro Lys
260 265 270

Asp Thr Leu Met Ile Ser Arg Thr Pro Glu Val Thr Cys Val Val Val
275 280 285

Asp Val Ser His Glu Asp Pro Glu Val Lys Phe Asn Trp Tyr Val Asp
290 295 300

Gly Val Glu Val His Asn Ala Lys Thr Lys Pro Arg Glu Glu Gln Tyr
305 310 315 320

Asn Ser Thr Tyr Arg Val Val Ser Val Leu Thr Val Leu His Gln Asp
325 330 335

Trp Leu Asn Gly Lys Glu Tyr Lys Cys Lys Val Ser Asn Lys Ala Leu
340 345 350

Pro Ala Pro Ile Glu Lys Thr Ile Ser Lys Ala Lys Gly Gln Pro Arg
355 360 365

Glu Pro Gln Val Tyr Thr Leu Pro Pro Ser Arg Glu Glu Met Thr Lys
370 375 380

Asn Gln Val Ser Leu Thr Cys Leu Val Lys Gly Phe Tyr Pro Ser Asp
385 390 395 400

```
Ile Ala Val Glu Trp Glu Ser Asn Gly Gln Pro Glu Asn Asn Tyr Lys
                405                 410                 415

Thr Thr Pro Pro Val Leu Asp Ser Asp Gly Ser Phe Phe Leu Tyr Ser
            420                 425                 430

Lys Leu Thr Val Asp Lys Ser Arg Trp Gln Gln Gly Asn Val Phe Ser
        435                 440                 445

Cys Ser Val Met His Glu Ala Leu His Asn His Tyr Thr Gln Lys Ser
    450                 455                 460

Leu Ser Leu Ser Pro Gly Lys
465                 470


<210>  27
<211>  45
<212>  DNA
<213>  Artificial Sequence

<220>
<223>  primer set F for c2A4 H

<400>  27
ccagatgggt gctgagccag gtccagttgc agcaatctgg agctg                       45


<210>  28
<211>  46
<212>  DNA
<213>  Artificial Sequence

<220>
<223>  primer set R for c2A4 H

<400>  28
cttggtggag gctgagctga ctgtgaccat gactccttgg ccccag                      46


<210>  29
<211>  702
<212>  DNA
<213>  Artificial Sequence

<220>
<223>  nucleotide sequence of c2B1 L

<400>  29
atggtgctgc agacccaggt gttcatctcc ctgctgctgt ggatctccgg cgcgtacggc      60

gaaactgtga tgacccagtc tcccacatcc atgtccacat caataggaga gagggtcacc     120

ctgaactgca aggccagtca gagtgtgggt attaatgtag actggtacca acagacacca     180

gggcagtctc ctaaactgct gatatatggg gcatccaacc ggcacactgg ggtccctgat     240
```

cgcttcacag gcagtggatt tgggagagat ttcactctca ccatcagcaa cgtggaggct 300

gaagacctgg ctgtttatta ctgtctgcag catggctcca ttcctccgac gttcggtgga 360

ggcaccaagc tggaattgaa acgggctgtg gccgcccct ccgtgttcat cttccccccc 420

tccgacgagc agctgaagtc cggcaccgcc tccgtggtgt gcctgctgaa taacttctac 480

cccagagagg ccaaggtgca gtggaaggtg gacaacgccc tgcagtccgg gaactcccag 540

gagagcgtga ccgagcagga cagcaaggac agcacctaca gcctgagcag caccctgacc 600

ctgagcaaag ccgactacga gaagcacaag gtgtacgcct gcgaggtgac ccaccagggc 660

ctgagctccc ccgtcaccaa gagcttcaac agggggagt gt 702

<210> 30
<211> 234
<212> PRT
<213> Artificial Sequence

<220>
<223> amino acid sequence of c2B1 L

<400> 30

Met Val Leu Gln Thr Gln Val Phe Ile Ser Leu Leu Leu Trp Ile Ser
1 5 10 15

Gly Ala Tyr Gly Glu Thr Val Met Thr Gln Ser Pro Thr Ser Met Ser
20 25 30

Thr Ser Ile Gly Glu Arg Val Thr Leu Asn Cys Lys Ala Ser Gln Ser
35 40 45

Val Gly Ile Asn Val Asp Trp Tyr Gln Gln Thr Pro Gly Gln Ser Pro
50 55 60

Lys Leu Leu Ile Tyr Gly Ala Ser Asn Arg His Thr Gly Val Pro Asp
65 70 75 80

Arg Phe Thr Gly Ser Gly Phe Gly Arg Asp Phe Thr Leu Thr Ile Ser
85 90 95

Asn Val Glu Ala Glu Asp Leu Ala Val Tyr Tyr Cys Leu Gln His Gly
100 105 110

Ser Ile Pro Pro Thr Phe Gly Gly Gly Thr Lys Leu Glu Leu Lys Arg
115 120 125

Ala Val Ala Ala Pro Ser Val Phe Ile Phe Pro Pro Ser Asp Glu Gln
130 135 140

Leu Lys Ser Gly Thr Ala Ser Val Val Cys Leu Leu Asn Asn Phe Tyr
145 150 155 160

Pro Arg Glu Ala Lys Val Gln Trp Lys Val Asp Asn Ala Leu Gln Ser
165 170 175

Gly Asn Ser Gln Glu Ser Val Thr Glu Gln Asp Ser Lys Asp Ser Thr
180 185 190

Tyr Ser Leu Ser Ser Thr Leu Thr Leu Ser Lys Ala Asp Tyr Glu Lys
195 200 205

His Lys Val Tyr Ala Cys Glu Val Thr His Gln Gly Leu Ser Ser Pro
210 215 220

Val Thr Lys Ser Phe Asn Arg Gly Glu Cys
225 230

<210> 31
<211> 44
<212> DNA
<213> Artificial Sequence

<220>
<223> primer set F for c2B1 L

<400> 31
atctccggcg cgtacggcga aactgtgatg acccagtctc ccac 44

<210> 32
<211> 44
<212> DNA
<213> Artificial Sequence

<220>
<223> primer set R for c2B1 L

<400> 32
ggagggggcg gccacagccc gtttcaattc cagcttggtg cctc 44

<210> 33
<211> 1416
<212> DNA
<213> Artificial Sequence

<220>
<223> nucleotide sequence of c2B1 H

<400> 33
atgaaacacc tgtggttctt cctcctgctg gtggcagctc ccagatgggt gctgagccag 60

gttactctga aagagtctgg ccctgggata ttgcagccct cccagaccct cagtctgact 120

tgcactttct ctgggttttc actgaacact tatgatatgg gtgtgggctg gattcgtcag 180

cccttcaggga agggtctgga gtggctggca aacatttggt gggatgatga taagtactac 240

aatccatctc tgagaaaccg gctcacaatc tccaaggaca cctccaacaa ccaagcattc 300

ctcaagatca ccaatgtgga cactgcagat actgccacat actactgtgc tcggatcgaa 360

actgtccggg tttcccgtaa ggggtttgct cactggggcc aaggcactct ggtcactgtc 420

agctcagcct ccaccaaggg cccaagcgtc ttccccctgg caccctcctc caagagcacc 480

tctggcggca cagccgccct gggctgcctg gtcaaggact acttccccga acccgtgacc 540

gtgagctgga actcaggcgc cctgaccagc ggcgtgcaca ccttccccgc tgtcctgcag 600

tcctcaggac tctactccct cagcagcgtg gtgaccgtgc cctccagcag cttgggcacc 660

cagacctaca tctgcaacgt gaatcacaag cccagcaaca ccaaggtgga caagagagtt 720

gagcccaaat cttgtgacaa aactcacaca tgcccaccct gcccagcacc tgaactcctg 780

gggggaccct cagtcttcct cttcccccca aaacccaagg acaccctcat gatctcccgg 840

acccctgagg tcacatgcgt ggtggtggac gtgagccacg aagaccctga ggtcaagttc 900

aactggtacg tggacggcgt ggaggtgcat aatgccaaga caaagccccg ggaggagcag 960

tacaacagca cgtaccgggt ggtcagcgtc ctcaccgtcc tgcaccagga ctggctgaat 1020

ggcaaggagt acaagtgcaa ggtctccaac aaagccctcc cagcccccat cgagaaaacc 1080

atctccaaag ccaaaggcca gccccgggaa ccacaggtgt acaccctgcc cccatcccgg 1140

gaggagatga ccaagaacca ggtcagcctg acctgcctgg tcaaaggctt ctatcccagc 1200

gacatcgccg tggagtggga gagcaatggc cagcccgaga acaactacaa gaccacccct 1260

cccgtgctgg actccgacgg ctccttcttc ctctacagca agctcaccgt ggacaagagc 1320

aggtggcagc agggcaacgt cttctcatgc tccgtgatgc atgaggctct gcacaaccac 1380

tacacccaga agagcctctc cctgtctccc ggcaaa 1416

<210> 34
<211> 472
<212> PRT
<213> Artificial Sequence

<220>
<223> amino acid sequence of c2B1 H

<400> 34

Met Lys His Leu Trp Phe Phe Leu Leu Leu Val Ala Ala Pro Arg Trp
1 5 10 15

Val Leu Ser Gln Val Thr Leu Lys Glu Ser Gly Pro Gly Ile Leu Gln
20 25 30

Pro Ser Gln Thr Leu Ser Leu Thr Cys Thr Phe Ser Gly Phe Ser Leu
35 40 45

Asn Thr Tyr Asp Met Gly Val Gly Trp Ile Arg Gln Pro Ser Gly Lys
50 55 60

Gly Leu Glu Trp Leu Ala Asn Ile Trp Trp Asp Asp Asp Lys Tyr Tyr
65 70 75 80

Asn Pro Ser Leu Arg Asn Arg Leu Thr Ile Ser Lys Asp Thr Ser Asn
85 90 95

Asn Gln Ala Phe Leu Lys Ile Thr Asn Val Asp Thr Ala Asp Thr Ala
100 105 110

Thr Tyr Tyr Cys Ala Arg Ile Glu Thr Val Arg Val Ser Arg Lys Gly
115 120 125

Phe Ala His Trp Gly Gln Gly Thr Leu Val Thr Val Ser Ser Ala Ser
130 135 140

Thr Lys Gly Pro Ser Val Phe Pro Leu Ala Pro Ser Ser Lys Ser Thr
145 150 155 160

Ser Gly Gly Thr Ala Ala Leu Gly Cys Leu Val Lys Asp Tyr Phe Pro
165 170 175

Glu Pro Val Thr Val Ser Trp Asn Ser Gly Ala Leu Thr Ser Gly Val
180 185 190

His Thr Phe Pro Ala Val Leu Gln Ser Ser Gly Leu Tyr Ser Leu Ser
195 200 205

Ser Val Val Thr Val Pro Ser Ser Ser Leu Gly Thr Gln Thr Tyr Ile
210 215 220

Cys Asn Val Asn His Lys Pro Ser Asn Thr Lys Val Asp Lys Arg Val
225 230 235 240

Glu Pro Lys Ser Cys Asp Lys Thr His Thr Cys Pro Pro Cys Pro Ala
245 250 255

Pro Glu Leu Leu Gly Gly Pro Ser Val Phe Leu Phe Pro Pro Lys Pro
260 265 270

Lys Asp Thr Leu Met Ile Ser Arg Thr Pro Glu Val Thr Cys Val Val
275 280 285

Val Asp Val Ser His Glu Asp Pro Glu Val Lys Phe Asn Trp Tyr Val

290 295 300

Asp Gly Val Glu Val His Asn Ala Lys Thr Lys Pro Arg Glu Glu Gln
305 310 315 320

Tyr Asn Ser Thr Tyr Arg Val Val Ser Val Leu Thr Val Leu His Gln
325 330 335

Asp Trp Leu Asn Gly Lys Glu Tyr Lys Cys Lys Val Ser Asn Lys Ala
340 345 350

Leu Pro Ala Pro Ile Glu Lys Thr Ile Ser Lys Ala Lys Gly Gln Pro
355 360 365

Arg Glu Pro Gln Val Tyr Thr Leu Pro Pro Ser Arg Glu Glu Met Thr
370 375 380

Lys Asn Gln Val Ser Leu Thr Cys Leu Val Lys Gly Phe Tyr Pro Ser
385 390 395 400

Asp Ile Ala Val Glu Trp Glu Ser Asn Gly Gln Pro Glu Asn Asn Tyr
405 410 415

Lys Thr Thr Pro Pro Val Leu Asp Ser Asp Gly Ser Phe Phe Leu Tyr
420 425 430

Ser Lys Leu Thr Val Asp Lys Ser Arg Trp Gln Gln Gly Asn Val Phe
435 440 445

Ser Cys Ser Val Met His Glu Ala Leu His Asn His Tyr Thr Gln Lys
450 455 460

Ser Leu Ser Leu Ser Pro Gly Lys
465 470

<210> 35
<211> 45
<212> DNA
<213> Artificial Sequence

<220>
<223> primer set F for c2B1 H

<400> 35
ccagatgggt gctgagccag gttactctga aagagtctgg ccctg 45

<210> 36
<211> 46
<212> DNA
<213> Artificial Sequence

<220>
<223> primer set R for c2B1 H

<400> 36
cttggtggag gctgagctga cagtgaccag agtgccttgg ccccag 46

<210> 37
<211> 699
<212> DNA
<213> Artificial Sequence

<220>
<223> nucleotide sequence of c7B4 L

<400> 37
atggtgctgc agacccaggt gttcatctcc ctgctgctgt ggatctccgg cgcgtacggc 60

gacatccaga tgacccagtc tccttcattc ctgtctgcat ctgtgggaga aagagtcact 120

ctcagctgca aagcaagtca gaatattaac aagtacttag actggtatca gcaaaagctt 180

ggagaacctc ccaaactcct gatatataat acaaacaatt tgcatacggg catcccatca 240

aggttcagtg gcagtggatc tggtacagat tacacactca ccatcagcgg cctgcagcct 300

gaagatgttg ccacatattt ctgcttgcag cgtaatagtt ggtacacgtt tggagctggg 360

accaagctgg aactgaaacg ggctgtggcc gccccctccg tgttcatctt cccccctcc 420

gacgagcagc tgaagtccgg caccgcctcc gtggtgtgcc tgctgaataa cttctacccc 480

agagaggcca aggtgcagtg gaaggtggac aacgccctgc agtccgggaa ctcccaggag 540

agcgtgaccg agcaggacag caaggacagc acctacagcc tgagcagcac cctgaccctg 600

agcaaagccg actacgagaa gcacaaggtg tacgcctgcg aggtgaccca ccagggcctg 660

agctcccccg tcaccaagag cttcaacagg ggggagtgt 699

<210> 38
<211> 233
<212> PRT
<213> Artificial Sequence

<220>
<223> amino acid sequence of c7B4 L

<400> 38

Met Val Leu Gln Thr Gln Val Phe Ile Ser Leu Leu Leu Trp Ile Ser
1 5 10 15

Gly Ala Tyr Gly Asp Ile Gln Met Thr Gln Ser Pro Ser Phe Leu Ser
20 25 30

Ala Ser Val Gly Glu Arg Val Thr Leu Ser Cys Lys Ala Ser Gln Asn
35 40 45

Ile Asn Lys Tyr Leu Asp Trp Tyr Gln Gln Lys Leu Gly Glu Pro Pro
    50                  55                  60

Lys Leu Leu Ile Tyr Asn Thr Asn Asn Leu His Thr Gly Ile Pro Ser
65                  70                  75                  80

Arg Phe Ser Gly Ser Gly Ser Gly Thr Asp Tyr Thr Leu Thr Ile Ser
                85                  90                  95

Gly Leu Gln Pro Glu Asp Val Ala Thr Tyr Phe Cys Leu Gln Arg Asn
            100                 105                 110

Ser Trp Tyr Thr Phe Gly Ala Gly Thr Lys Leu Glu Leu Lys Arg Ala
        115                 120                 125

Val Ala Ala Pro Ser Val Phe Ile Phe Pro Pro Ser Asp Glu Gln Leu
    130                 135                 140

Lys Ser Gly Thr Ala Ser Val Val Cys Leu Leu Asn Asn Phe Tyr Pro
145                 150                 155                 160

Arg Glu Ala Lys Val Gln Trp Lys Val Asp Asn Ala Leu Gln Ser Gly
                165                 170                 175

Asn Ser Gln Glu Ser Val Thr Glu Gln Asp Ser Lys Asp Ser Thr Tyr
            180                 185                 190

Ser Leu Ser Ser Thr Leu Thr Leu Ser Lys Ala Asp Tyr Glu Lys His
        195                 200                 205

Lys Val Tyr Ala Cys Glu Val Thr His Gln Gly Leu Ser Ser Pro Val
    210                 215                 220

Thr Lys Ser Phe Asn Arg Gly Glu Cys
225                 230

<210> 39
<211> 44
<212> DNA
<213> Artificial Sequence

<220>
<223> primer set F for c7B4 L

<400> 39
atctccggcg cgtacggcga catccagatg acccagtctc cttc 44

<210> 40
<211> 44

<212> DNA
<213> Artificial Sequence

<220>
<223> primer set R for c7B4 L

<400> 40
ggaggggcg gccacagccc gtttcagttc cagcttggtc ccag 44

<210> 41
<211> 1416
<212> DNA
<213> Artificial Sequence

<220>
<223> nucleotide sequence of c7B4 H

<400> 41
atgaaacacc tgtggttctt cctcctgctg gtggcagctc ccagatgggt gctgagcgag 60

atacacctgc aggagtcagg acctggcctt gtgaaacctt cacagtcact ctccctcacc 120

tgttctgtca ctggttacac cattaccagt ggttatgatt ggagctggat ccggaagttc 180

ccaggaaata aaatggagtg gatggcatac atgagctata gaggtagcac taactacaac 240

ccctcgctca aaagtcgaat ctccattaca agagacacat ccaagaatca gttcttcctg 300

cagttgaatt ctgtcactac tgaggataca gccacatatt actgtgccct aacaaggacc 360

tattggtata actattacta tgttttggat gcctggggtc aaggagcttc agtcactgtc 420

agctcagcct ccaccaaggg cccaagcgtc ttcccctgg caccctcctc caagagcacc 480

tctggcggca cagccgccct gggctgcctg gtcaaggact acttccccga acccgtgacc 540

gtgagctgga actcaggcgc cctgaccagc ggcgtgcaca ccttccccgc tgtcctgcag 600

tcctcaggac tctactccct cagcagcgtg gtgaccgtgc cctccagcag cttgggcacc 660

cagacctaca tctgcaacgt gaatcacaag cccagcaaca ccaaggtgga caagagagtt 720

gagcccaaat cttgtgacaa aactcacaca tgcccaccct gcccagcacc tgaactcctg 780

gggggaccct cagtcttcct cttcccccca aaacccaagg acaccctcat gatctcccgg 840

acccctgagg tcacatgcgt ggtggtggac gtgagccacg aagaccctga ggtcaagttc 900

aactggtacg tggacggcgt ggaggtgcat aatgccaaga caaagccccg ggaggagcag 960

tacaacagca cgtaccgggt ggtcagcgtc ctcaccgtcc tgcaccagga ctggctgaat 1020

ggcaaggagt acaagtgcaa ggtctccaac aaagccctcc cagcccccat cgagaaaacc 1080

atctccaaag ccaaaggcca gccccgggaa ccacaggtgt acaccctgcc cccatcccgg 1140

gaggagatga ccaagaacca ggtcagcctg acctgcctgg tcaaaggctt ctatcccagc 1200

gacatcgccg tggagtggga gagcaatggc cagcccgaga acaactacaa gaccaccccct 1260

cccgtgctgg actccgacgg ctccttcttc ctctacagca agctcaccgt ggacaagagc 1320

aggtggcagc agggcaacgt cttctcatgc tccgtgatgc atgaggctct gcacaaccac 1380

tacacccaga agagcctctc cctgtctccc ggcaaa 1416

<210> 42
<211> 472
<212> PRT
<213> Artificial Sequence

<220>
<223> amino acid sequence of c7B4 H

<400> 42

Met Lys His Leu Trp Phe Phe Leu Leu Leu Val Ala Ala Pro Arg Trp
1 5 10 15

Val Leu Ser Glu Ile His Leu Gln Glu Ser Gly Pro Gly Leu Val Lys
20 25 30

Pro Ser Gln Ser Leu Ser Leu Thr Cys Ser Val Thr Gly Tyr Thr Ile
35 40 45

Thr Ser Gly Tyr Asp Trp Ser Trp Ile Arg Lys Phe Pro Gly Asn Lys
50 55 60

Met Glu Trp Met Ala Tyr Met Ser Tyr Arg Gly Ser Thr Asn Tyr Asn
65 70 75 80

Pro Ser Leu Lys Ser Arg Ile Ser Ile Thr Arg Asp Thr Ser Lys Asn
85 90 95

Gln Phe Phe Leu Gln Leu Asn Ser Val Thr Thr Glu Asp Thr Ala Thr
100 105 110

Tyr Tyr Cys Ala Leu Thr Arg Thr Tyr Trp Tyr Asn Tyr Tyr Tyr Val
115 120 125

Leu Asp Ala Trp Gly Gln Gly Ala Ser Val Thr Val Ser Ser Ala Ser
130 135 140

Thr Lys Gly Pro Ser Val Phe Pro Leu Ala Pro Ser Ser Lys Ser Thr
145 150 155 160

Ser Gly Gly Thr Ala Ala Leu Gly Cys Leu Val Lys Asp Tyr Phe Pro
165 170 175

Glu Pro Val Thr Val Ser Trp Asn Ser Gly Ala Leu Thr Ser Gly Val
180 185 190

```
His Thr Phe Pro Ala Val Leu Gln Ser Ser Gly Leu Tyr Ser Leu Ser
        195                 200                 205

Ser Val Val Thr Val Pro Ser Ser Ser Leu Gly Thr Gln Thr Tyr Ile
    210                 215                 220

Cys Asn Val Asn His Lys Pro Ser Asn Thr Lys Val Asp Lys Arg Val
225                 230                 235                 240

Glu Pro Lys Ser Cys Asp Lys Thr His Thr Cys Pro Pro Cys Pro Ala
                245                 250                 255

Pro Glu Leu Leu Gly Gly Pro Ser Val Phe Leu Phe Pro Pro Lys Pro
            260                 265                 270

Lys Asp Thr Leu Met Ile Ser Arg Thr Pro Glu Val Thr Cys Val Val
        275                 280                 285

Val Asp Val Ser His Glu Asp Pro Glu Val Lys Phe Asn Trp Tyr Val
    290                 295                 300

Asp Gly Val Glu Val His Asn Ala Lys Thr Lys Pro Arg Glu Glu Gln
305                 310                 315                 320

Tyr Asn Ser Thr Tyr Arg Val Val Ser Val Leu Thr Val Leu His Gln
                325                 330                 335

Asp Trp Leu Asn Gly Lys Glu Tyr Lys Cys Lys Val Ser Asn Lys Ala
            340                 345                 350

Leu Pro Ala Pro Ile Glu Lys Thr Ile Ser Lys Ala Lys Gly Gln Pro
        355                 360                 365

Arg Glu Pro Gln Val Tyr Thr Leu Pro Pro Ser Arg Glu Glu Met Thr
    370                 375                 380

Lys Asn Gln Val Ser Leu Thr Cys Leu Val Lys Gly Phe Tyr Pro Ser
385                 390                 395                 400

Asp Ile Ala Val Glu Trp Glu Ser Asn Gly Gln Pro Glu Asn Asn Tyr
                405                 410                 415

Lys Thr Thr Pro Pro Val Leu Asp Ser Asp Gly Ser Phe Phe Leu Tyr
            420                 425                 430

Ser Lys Leu Thr Val Asp Lys Ser Arg Trp Gln Gln Gly Asn Val Phe
        435                 440                 445
```

Ser Cys Ser Val Met His Glu Ala Leu His Asn His Tyr Thr Gln Lys
450 455 460

Ser Leu Ser Leu Ser Pro Gly Lys
465 470

<210> 43
<211> 45
<212> DNA
<213> Artificial Sequence

<220>
<223> primer set F for c7B4 H

<400> 43
ccagatgggt gctgagcgag atacacctgc aggagtcagg acctg 45

<210> 44
<211> 46
<212> DNA
<213> Artificial Sequence

<220>
<223> primer set R for c7B4 H

<400> 44
cttggtggag gctgagctga cagtgactga agctccttga ccccag 46

<210> 45
<211> 8
<212> PRT
<213> Artificial Sequence

<220>
<223> amino acid sequence of 2A4 H CDR1

<400> 45

Gly Tyr Thr Phe Thr Ser Tyr Tyr
1 5

<210> 46
<211> 8
<212> PRT
<213> Artificial Sequence

<220>
<223> amino acid sequence of 2A4 H CDR2

<400> 46

Val Tyr Pro Gly Tyr Gly Gly Thr
1 5

<210> 47
<211> 15

<212> PRT
<213> Artificial Sequence

<220>
<223> amino acid sequence of 2A4 H CDR3

<400> 47

Ala Arg Arg Lys Gly Ile Ile Arg Gly Pro Gly Tyr Phe Asp Tyr
1 5 10 15

<210> 48
<211> 10
<212> PRT
<213> Artificial Sequence

<220>
<223> amino acid sequence of 2B1 H CDR1

<400> 48

Gly Phe Ser Leu Asn Thr Tyr Asp Met Gly
1 5 10

<210> 49
<211> 7
<212> PRT
<213> Artificial Sequence

<220>
<223> amino acid sequence of 2B1 H CDR2

<400> 49

Ile Trp Trp Asp Asp Asp Lys
1 5

<210> 50
<211> 15
<212> PRT
<213> Artificial Sequence

<220>
<223> amino acid sequence of 2B1 H CDR3

<400> 50

Ala Arg Ile Glu Thr Val Arg Val Ser Arg Lys Gly Phe Ala His
1 5 10 15

<210> 51
<211> 9
<212> PRT
<213> Artificial Sequence

<220>
<223> amino acid sequence of 7B4 H CDR1

<400> 51

Gly Tyr Thr Ile Thr Ser Gly Tyr Asp
1               5

<210>  52
<211>  7
<212>  PRT
<213>  Artificial Sequence

<220>
<223>  amino acid sequence of 7B4 H CDR2

<400>  52

Met Ser Tyr Arg Gly Ser Thr
1               5

<210>  53
<211>  16
<212>  PRT
<213>  Artificial Sequence

<220>
<223>  amino acid sequence of 7B4 H CDR3

<400>  53

Ala Leu Thr Arg Thr Tyr Trp Tyr Asn Tyr Tyr Tyr Val Leu Asp Ala
1               5                   10                  15

<210>  54
<211>  6
<212>  PRT
<213>  Artificial Sequence

<220>
<223>  amino acid sequence of 2A4 L CDR1

<400>  54

Glu Gly Ile Ser Asn Ser
1               5

<210>  55
<211>  3
<212>  PRT
<213>  Artificial Sequence

<220>
<223>  amino acid sequence of 2A4 L CDR2

<400>  55

Gly Ala Ser
1

<210>  56
<211>  9

<212> PRT
<213> Artificial Sequence

<220>
<223> amino acid sequence of 2A4 L CDR3

<400> 56

Gln Gln Gly Tyr Lys Tyr Pro Pro Thr
1 5

<210> 57
<211> 6
<212> PRT
<213> Artificial Sequence

<220>
<223> amino acid sequence of 2B1 L CDR1

<400> 57

Gln Ser Val Gly Ile Asn
1 5

<210> 58
<211> 3
<212> PRT
<213> Artificial Sequence

<220>
<223> amino acid sequence of 2B1 L CDR2

<400> 58

Gly Ala Ser
1

<210> 59
<211> 9
<212> PRT
<213> Artificial Sequence

<220>
<223> amino acid sequence of 2B1 L CDR3

<400> 59

Leu Gln His Gly Ser Ile Pro Pro Thr
1 5

<210> 60
<211> 6
<212> PRT
<213> Artificial Sequence

<220>
<223> amino acid sequence of 7B4 L CDR1

<400> 60

Gln Asn Ile Asn Lys Tyr
1               5

<210> 61
<211> 3
<212> PRT
<213> Artificial Sequence

<220>
<223> amino acid sequence of 7B4 L CDR2

<400> 61

Asn Thr Asn
1

<210> 62
<211> 8
<212> PRT
<213> Artificial Sequence

<220>
<223> amino acid sequence of 7B4 L CDR3

<400> 62

Leu Gln Arg Asn Ser Trp Tyr Thr
1               5

<210> 63
<211> 702
<212> DNA
<213> Artificial Sequence

<220>
<223> nucleotide sequence of h2B1_L1

<400> 63
atggtgctgc agacccaggt gttcatctcc ctgctgctgt ggatctccgg cgcgtacggc 60

gataccgtga tgacccagag ccctgacagc ctggccgtgt ctctgggaga gagagccacc 120

atcaactgca aggccagcca gagcgtgggc atcaacgtgg actggtatca gcagaagccc 180

ggccagcccc ccaagctgct gatctacggc gccagcaata gacacaccgg cgtgcccgat 240

agattcagcg gctctggcag cggcagagac ttcaccctga ccatcagctc cctgcaggcc 300

gaggatgtgg ccgtgtacta ctgtctgcag cacggcagca tccccccac atttggccag 360

ggcaccaagg tggaaatcaa gcgtacggtg gccgccccct ccgtgttcat cttccccccc 420

tccgacgagc agctgaagtc cggcaccgcc tccgtggtgt gcctgctgaa taacttctac 480

cccagagagg ccaaggtgca gtggaaggtg gacaacgccc tgcagtccgg gaactcccag 540

gagagcgtga ccgagcagga cagcaaggac agcacctaca gcctgagcag caccctgacc 600

ctgagcaaag ccgactacga gaagcacaag gtgtacgcct gcgaggtgac ccaccagggc 660

ctgagctccc ccgtcaccaa gagcttcaac aggggggagt gt 702

<210> 64
<211> 234
<212> PRT
<213> Artificial Sequence

<220>
<223> amino acid sequence of h2B1_L1

<400> 64

Met Val Leu Gln Thr Gln Val Phe Ile Ser Leu Leu Leu Trp Ile Ser
1 5 10 15

Gly Ala Tyr Gly Asp Thr Val Met Thr Gln Ser Pro Asp Ser Leu Ala
20 25 30

Val Ser Leu Gly Glu Arg Ala Thr Ile Asn Cys Lys Ala Ser Gln Ser
35 40 45

Val Gly Ile Asn Val Asp Trp Tyr Gln Gln Lys Pro Gly Gln Pro Pro
50 55 60

Lys Leu Leu Ile Tyr Gly Ala Ser Asn Arg His Thr Gly Val Pro Asp
65 70 75 80

Arg Phe Ser Gly Ser Gly Ser Gly Arg Asp Phe Thr Leu Thr Ile Ser
85 90 95

Ser Leu Gln Ala Glu Asp Val Ala Val Tyr Tyr Cys Leu Gln His Gly
100 105 110

Ser Ile Pro Pro Thr Phe Gly Gln Gly Thr Lys Val Glu Ile Lys Arg
115 120 125

Thr Val Ala Ala Pro Ser Val Phe Ile Phe Pro Pro Ser Asp Glu Gln
130 135 140

Leu Lys Ser Gly Thr Ala Ser Val Val Cys Leu Leu Asn Asn Phe Tyr
145 150 155 160

Pro Arg Glu Ala Lys Val Gln Trp Lys Val Asp Asn Ala Leu Gln Ser
165 170 175

Gly Asn Ser Gln Glu Ser Val Thr Glu Gln Asp Ser Lys Asp Ser Thr
180 185 190

Tyr Ser Leu Ser Ser Thr Leu Thr Leu Ser Lys Ala Asp Tyr Glu Lys

195 200 205

His Lys Val Tyr Ala Cys Glu Val Thr His Gln Gly Leu Ser Ser Pro
210 215 220

Val Thr Lys Ser Phe Asn Arg Gly Glu Cys
225 230

<210> 65
<211> 702
<212> DNA
<213> Artificial Sequence

<220>
<223> nucleotide sequence of h2B1_L2

<400> 65
atggtgctgc agacccaggt gttcatctcc ctgctgctgt ggatctccgg cgcgtacggc 60

gataccgtga tgacccagag ccctgacagc ctggccgtgt ctctgggaga gagagccacc 120

atcaactgca aggccagcca gagcgtgggc atcaacgtgg actggtatca gcagaagccc 180

ggccagcccc ccaagctgct gatctacggc gccagcaata gacacaccgg cgtgcccgat 240

agattcagcg gcagcggctt cggcaccgac ttcaccctga caatcagctc cctgcaggcc 300

gaggacgtgg ccgtgtacta ctgtctgcag cacggcagca tcccccccac atttggccag 360

ggcaccaagg tggaaatcaa gcgtacggtg gccgcccccct ccgtgttcat cttccccccc 420

tccgacgagc agctgaagtc cggcaccgcc tccgtggtgt gcctgctgaa taacttctac 480

cccagagagg ccaaggtgca gtggaaggtg gacaacgccc tgcagtccgg gaactcccag 540

gagagcgtga ccgagcagga cagcaaggac agcacctaca gcctgagcag caccctgacc 600

ctgagcaaag ccgactacga gaagcacaag gtgtacgcct gcgaggtgac ccaccagggc 660

ctgagctccc ccgtcaccaa gagcttcaac aggggggagt gt 702

<210> 66
<211> 234
<212> PRT
<213> Artificial Sequence

<220>
<223> amino acid sequence of h2B1_L2

<400> 66

Met Val Leu Gln Thr Gln Val Phe Ile Ser Leu Leu Leu Trp Ile Ser
1 5 10 15

Gly Ala Tyr Gly Asp Thr Val Met Thr Gln Ser Pro Asp Ser Leu Ala
20 25 30

```
Val Ser Leu Gly Glu Arg Ala Thr Ile Asn Cys Lys Ala Ser Gln Ser
        35                  40                  45

Val Gly Ile Asn Val Asp Trp Tyr Gln Gln Lys Pro Gly Gln Pro Pro
    50                  55                  60

Lys Leu Leu Ile Tyr Gly Ala Ser Asn Arg His Thr Gly Val Pro Asp
65                  70                  75                  80

Arg Phe Ser Gly Ser Gly Phe Gly Thr Asp Phe Thr Leu Thr Ile Ser
                85                  90                  95

Ser Leu Gln Ala Glu Asp Val Ala Val Tyr Tyr Cys Leu Gln His Gly
            100                 105                 110

Ser Ile Pro Pro Thr Phe Gly Gln Gly Thr Lys Val Glu Ile Lys Arg
        115                 120                 125

Thr Val Ala Ala Pro Ser Val Phe Ile Phe Pro Pro Ser Asp Glu Gln
    130                 135                 140

Leu Lys Ser Gly Thr Ala Ser Val Val Cys Leu Leu Asn Asn Phe Tyr
145                 150                 155                 160

Pro Arg Glu Ala Lys Val Gln Trp Lys Val Asp Asn Ala Leu Gln Ser
                165                 170                 175

Gly Asn Ser Gln Glu Ser Val Thr Glu Gln Asp Ser Lys Asp Ser Thr
            180                 185                 190

Tyr Ser Leu Ser Ser Thr Leu Thr Leu Ser Lys Ala Asp Tyr Glu Lys
        195                 200                 205

His Lys Val Tyr Ala Cys Glu Val Thr His Gln Gly Leu Ser Ser Pro
    210                 215                 220

Val Thr Lys Ser Phe Asn Arg Gly Glu Cys
225                 230


<210>  67
<211>  702
<212>  DNA
<213>  Artificial Sequence

<220>
<223>  nucleotide sequence of h2B1_L3

<400>  67
atggtgctgc agacccaggt gttcatctcc ctgctgctgt ggatctccgg cgcgtacggc       60
```

gataccgtga tgacccagag ccctgacagc ctggccgtgt ctctgggaga gagagccacc 120

atcaactgca aggccagcca gagcgtgggc atcaacgtgg actggtatca gcagaagccc 180

ggccagcccc ccaagctgct gatctacggc gccagcaata gacacaccgg cgtgccccgat 240

agattcagcg gcagcggctt cggcagagac ttcaccctga ccatcagctc cctgcaggcc 300

gaggatgtgg ccgtgtacta ctgtctgcag cacggcagca tcccccccac atttggccag 360

ggcaccaagg tggaaatcaa gcgtacggtg gccgccccct ccgtgttcat cttccccccc 420

tccgacgagc agctgaagtc cggcaccgcc tccgtggtgt gcctgctgaa taacttctac 480

cccagagagg ccaaggtgca gtggaaggtg gacaacgccc tgcagtccgg gaactcccag 540

gagagcgtga ccgagcagga cagcaaggac agcacctaca gcctgagcag caccctgacc 600

ctgagcaaag ccgactacga gaagcacaag gtgtacgcct gcgaggtgac ccaccagggc 660

ctgagctccc ccgtcaccaa gagcttcaac aggggggagt gt 702

<210> 68
<211> 234
<212> PRT
<213> Artificial Sequence

<220>
<223> amino acid sequence of h2B1_L3

<400> 68

Met Val Leu Gln Thr Gln Val Phe Ile Ser Leu Leu Leu Trp Ile Ser
1 5 10 15

Gly Ala Tyr Gly Asp Thr Val Met Thr Gln Ser Pro Asp Ser Leu Ala
20 25 30

Val Ser Leu Gly Glu Arg Ala Thr Ile Asn Cys Lys Ala Ser Gln Ser
35 40 45

Val Gly Ile Asn Val Asp Trp Tyr Gln Gln Lys Pro Gly Gln Pro Pro
50 55 60

Lys Leu Leu Ile Tyr Gly Ala Ser Asn Arg His Thr Gly Val Pro Asp
65 70 75 80

Arg Phe Ser Gly Ser Gly Phe Gly Arg Asp Phe Thr Leu Thr Ile Ser
85 90 95

Ser Leu Gln Ala Glu Asp Val Ala Val Tyr Tyr Cys Leu Gln His Gly
100 105 110

Ser Ile Pro Pro Thr Phe Gly Gln Gly Thr Lys Val Glu Ile Lys Arg
115 120 125

Thr Val Ala Ala Pro Ser Val Phe Ile Phe Pro Pro Ser Asp Glu Gln
130 135 140

Leu Lys Ser Gly Thr Ala Ser Val Val Cys Leu Leu Asn Asn Phe Tyr
145 150 155 160

Pro Arg Glu Ala Lys Val Gln Trp Lys Val Asp Asn Ala Leu Gln Ser
165 170 175

Gly Asn Ser Gln Glu Ser Val Thr Glu Gln Asp Ser Lys Asp Ser Thr
180 185 190

Tyr Ser Leu Ser Ser Thr Leu Thr Leu Ser Lys Ala Asp Tyr Glu Lys
195 200 205

His Lys Val Tyr Ala Cys Glu Val Thr His Gln Gly Leu Ser Ser Pro
210 215 220

Val Thr Lys Ser Phe Asn Arg Gly Glu Cys
225 230

<210> 69
<211> 702
<212> DNA
<213> Artificial Sequence

<220>
<223> nucleotide sequence of h2B1_L4

<400> 69
atggtgctgc agacccaggt gttcatctcc ctgctgctgt ggatctccgg cgcgtacggc 60

gataccgtga tgacccagag ccctgacagc ctggccgtgt ctctgggaga gagagccacc 120

atcaactgca aggccagcca gagcgtgggc atcaacgtgg actggtatca gcagaagccc 180

ggccagagcc ccaagctgct gatctacggc gccagcaaca gacacaccgg cgtgcccgat 240

agattcagcg gcagcggctt cggcagagac ttcaccctga ccatcagctc cctgcaggcc 300

gaggatgtgg ccgtgtacta ctgtctgcag cacggcagca tcccccccac atttggccag 360

ggcaccaagg tggaaatcaa gcgtacggtg gccgccccct ccgtgttcat cttccccccc 420

tccgacgagc agctgaagtc cggcaccgcc tccgtggtgt gcctgctgaa taacttctac 480

cccagagagg ccaaggtgca gtggaaggtg gacaacgccc tgcagtccgg gaactcccag 540

gagagcgtga ccgagcagga cagcaaggac agcacctaca gcctgagcag caccctgacc 600

ctgagcaaag ccgactacga gaagcacaag gtgtacgcct gcgaggtgac ccaccagggc 660

ctgagctccc ccgtcaccaa gagcttcaac aggggggagt gt 702

<210> 70
<211> 234
<212> PRT
<213> Artificial Sequence

<220>
<223> amino acid sequence of h2B1_L4

<400> 70

Met Val Leu Gln Thr Gln Val Phe Ile Ser Leu Leu Leu Trp Ile Ser
1               5                   10                  15

Gly Ala Tyr Gly Asp Thr Val Met Thr Gln Ser Pro Asp Ser Leu Ala
            20                  25                  30

Val Ser Leu Gly Glu Arg Ala Thr Ile Asn Cys Lys Ala Ser Gln Ser
        35                  40                  45

Val Gly Ile Asn Val Asp Trp Tyr Gln Gln Lys Pro Gly Gln Ser Pro
    50                  55                  60

Lys Leu Leu Ile Tyr Gly Ala Ser Asn Arg His Thr Gly Val Pro Asp
65                  70                  75                  80

Arg Phe Ser Gly Ser Gly Phe Gly Arg Asp Phe Thr Leu Thr Ile Ser
                85                  90                  95

Ser Leu Gln Ala Glu Asp Val Ala Val Tyr Tyr Cys Leu Gln His Gly
            100                 105                 110

Ser Ile Pro Pro Thr Phe Gly Gln Gly Thr Lys Val Glu Ile Lys Arg
        115                 120                 125

Thr Val Ala Ala Pro Ser Val Phe Ile Phe Pro Pro Ser Asp Glu Gln
    130                 135                 140

Leu Lys Ser Gly Thr Ala Ser Val Val Cys Leu Leu Asn Asn Phe Tyr
145                 150                 155                 160

Pro Arg Glu Ala Lys Val Gln Trp Lys Val Asp Asn Ala Leu Gln Ser
                165                 170                 175

Gly Asn Ser Gln Glu Ser Val Thr Glu Gln Asp Ser Lys Asp Ser Thr
            180                 185                 190

Tyr Ser Leu Ser Ser Thr Leu Thr Leu Ser Lys Ala Asp Tyr Glu Lys
        195                 200                 205

His Lys Val Tyr Ala Cys Glu Val Thr His Gln Gly Leu Ser Ser Pro
    210                 215                 220

Val Thr Lys Ser Phe Asn Arg Gly Glu Cys
225                 230

<210> 71
<211> 702
<212> DNA
<213> Artificial Sequence

<220>
<223> nucleotide sequence of h2B1_L5

<400> 71
atggtgctgc agacccaggt gttcatctcc ctgctgctgt ggatctccgg cgcgtacggc 60

gccaccagaa tgacacagag ccccagcagc ttcagcgcca gcaccggcga cagagtgacc 120

atcacatgca aggccagcca gagcgtgggc atcaacgtgg actggtatca gcagaagccc 180

ggcaagagcc ccaagctgct gatctacggc gcctccaaca gacacaccgg cgtgcccagc 240

agattttccg gcagcggctt cggcagagac ttcaccctga ccatcagcag cctgcagagc 300

gaggacttcg ccacctacta ctgcctgcag cacggcagca tcccccctac atttggcgga 360

ggcaccaagg tggaaatcaa gcgtacggtg gccgcccccct ccgtgttcat cttccccccc 420

tccgacgagc agctgaagtc cggcaccgcc tccgtggtgt gcctgctgaa taacttctac 480

cccagagagg ccaaggtgca gtggaaggtg gacaacgccc tgcagtccgg gaactcccag 540

gagagcgtga ccgagcagga cagcaaggac agcacctaca gcctgagcag caccctgacc 600

ctgagcaaag ccgactacga gaagcacaag gtgtacgcct gcgaggtgac ccaccagggc 660

ctgagctccc ccgtcaccaa gagcttcaac agggggggagt gt 702

<210> 72
<211> 234
<212> PRT
<213> Artificial Sequence

<220>
<223> amino acid sequence of h2B1_L5

<400> 72

Met Val Leu Gln Thr Gln Val Phe Ile Ser Leu Leu Leu Trp Ile Ser
1               5                   10                  15

Gly Ala Tyr Gly Ala Thr Arg Met Thr Gln Ser Pro Ser Ser Phe Ser
            20                  25                  30

Ala Ser Thr Gly Asp Arg Val Thr Ile Thr Cys Lys Ala Ser Gln Ser
        35                  40                  45

Val Gly Ile Asn Val Asp Trp Tyr Gln Gln Lys Pro Gly Lys Ser Pro
    50                  55                  60

Lys Leu Leu Ile Tyr Gly Ala Ser Asn Arg His Thr Gly Val Pro Ser
65                  70                  75                  80

Arg Phe Ser Gly Ser Gly Phe Gly Arg Asp Phe Thr Leu Thr Ile Ser
                85                  90                  95

Ser Leu Gln Ser Glu Asp Phe Ala Thr Tyr Tyr Cys Leu Gln His Gly
            100                 105                 110

Ser Ile Pro Pro Thr Phe Gly Gly Gly Thr Lys Val Glu Ile Lys Arg
        115                 120                 125

Thr Val Ala Ala Pro Ser Val Phe Ile Phe Pro Pro Ser Asp Glu Gln
    130                 135                 140

Leu Lys Ser Gly Thr Ala Ser Val Val Cys Leu Leu Asn Asn Phe Tyr
145                 150                 155                 160

Pro Arg Glu Ala Lys Val Gln Trp Lys Val Asp Asn Ala Leu Gln Ser
                165                 170                 175

Gly Asn Ser Gln Glu Ser Val Thr Glu Gln Asp Ser Lys Asp Ser Thr
            180                 185                 190

Tyr Ser Leu Ser Ser Thr Leu Thr Leu Ser Lys Ala Asp Tyr Glu Lys
        195                 200                 205

His Lys Val Tyr Ala Cys Glu Val Thr His Gln Gly Leu Ser Ser Pro
    210                 215                 220

Val Thr Lys Ser Phe Asn Arg Gly Glu Cys
225                 230

<210> 73
<211> 1416
<212> DNA
<213> Artificial Sequence

<220>
<223> nucleotide sequence of h2B1_H1

<400> 73
atgaaacacc tgtggttctt cctcctgctg gtggcagctc ccagatgggt gctgagccag 60

gtgcagctgg tggaatctgg cggcggactc gtgaagccta gccagaccct gagcctgacc 120

tgcaccttca gcggcttcag cctgaacacc tacgacatgg gcgtgggctg gatcagacag 180

```
cctcctggca agggcctgga atggatcggc aacatttggt gggacgacga caagtactac      240
aaccccagcc tgaagtccag agtgaccatc agcgtggaca ccagcaagaa ccaggcctcc      300
ctgaagctga gcagcgtgac agccgccgat accgccgtgt actactgcgc cagaatcgag      360
acagtgcggg tgtccagaaa gggcttcgcc cactggggac agggcacact cgtgaccgtc      420
agctcagcct ccaccaaggg cccaagcgtc ttccccctgg caccctcctc caagagcacc      480
tctggcggca cagccgccct gggctgcctg gtcaaggact acttccccga acccgtgacc      540
gtgagctgga actcaggcgc cctgaccagc ggcgtgcaca ccttccccgc tgtcctgcag      600
tcctcaggac tctactccct cagcagcgtg gtgaccgtgc cctccagcag cttgggcacc      660
cagacctaca tctgcaacgt gaatcacaag cccagcaaca ccaaggtgga caagagagtt      720
gagcccaaat cttgtgacaa aactcacaca tgcccaccct gcccagcacc tgaactcctg      780
gggggaccct cagtcttcct cttcccccca aaacccaagg acaccctcat gatctcccgg      840
acccctgagg tcacatgcgt ggtggtggac gtgagccacg aagaccctga ggtcaagttc      900
aactggtacg tggacggcgt ggaggtgcat aatgccaaga caaagccccg ggaggagcag      960
tacaacagca cgtaccgggt ggtcagcgtc ctcaccgtcc tgcaccagga ctggctgaat     1020
ggcaaggagt acaagtgcaa ggtctccaac aaagccctcc cagcccccat cgagaaaacc     1080
atctccaaag ccaaaggcca gccccgggaa ccacaggtgt acaccctgcc cccatcccgg     1140
gaggagatga ccaagaacca ggtcagcctg acctgcctgg tcaaaggctt ctatcccagc     1200
gacatcgccg tggagtggga gagcaatggc cagcccgaga acaactacaa gaccacccct     1260
cccgtgctgg actccgacgg ctccttcttc ctctacagca agctcaccgt ggacaagagc     1320
aggtggcagc agggcaacgt cttctcatgc tccgtgatgc atgaggctct gcacaaccac     1380
tacacccaga agagcctctc cctgtctccc ggcaaa                               1416
```

<210> 74
<211> 472
<212> PRT
<213> Artificial Sequence

<220>
<223> amino acid sequence of h2B1_H1

<400> 74

```
Met Lys His Leu Trp Phe Phe Leu Leu Leu Val Ala Ala Pro Arg Trp
1               5                   10                  15

Val Leu Ser Gln Val Gln Leu Val Glu Ser Gly Gly Gly Leu Val Lys
            20                  25                  30

Pro Ser Gln Thr Leu Ser Leu Thr Cys Thr Phe Ser Gly Phe Ser Leu
```

35 40 45

Asn Thr Tyr Asp Met Gly Val Gly Trp Ile Arg Gln Pro Pro Gly Lys
50 55 60

Gly Leu Glu Trp Ile Gly Asn Ile Trp Trp Asp Asp Asp Lys Tyr Tyr
65 70 75 80

Asn Pro Ser Leu Lys Ser Arg Val Thr Ile Ser Val Asp Thr Ser Lys
85 90 95

Asn Gln Ala Ser Leu Lys Leu Ser Ser Val Thr Ala Ala Asp Thr Ala
100 105 110

Val Tyr Tyr Cys Ala Arg Ile Glu Thr Val Arg Val Ser Arg Lys Gly
115 120 125

Phe Ala His Trp Gly Gln Gly Thr Leu Val Thr Val Ser Ser Ala Ser
130 135 140

Thr Lys Gly Pro Ser Val Phe Pro Leu Ala Pro Ser Ser Lys Ser Thr
145 150 155 160

Ser Gly Gly Thr Ala Ala Leu Gly Cys Leu Val Lys Asp Tyr Phe Pro
165 170 175

Glu Pro Val Thr Val Ser Trp Asn Ser Gly Ala Leu Thr Ser Gly Val
180 185 190

His Thr Phe Pro Ala Val Leu Gln Ser Ser Gly Leu Tyr Ser Leu Ser
195 200 205

Ser Val Val Thr Val Pro Ser Ser Ser Leu Gly Thr Gln Thr Tyr Ile
210 215 220

Cys Asn Val Asn His Lys Pro Ser Asn Thr Lys Val Asp Lys Arg Val
225 230 235 240

Glu Pro Lys Ser Cys Asp Lys Thr His Thr Cys Pro Pro Cys Pro Ala
245 250 255

Pro Glu Leu Leu Gly Gly Pro Ser Val Phe Leu Phe Pro Pro Lys Pro
260 265 270

Lys Asp Thr Leu Met Ile Ser Arg Thr Pro Glu Val Thr Cys Val Val
275 280 285

Val Asp Val Ser His Glu Asp Pro Glu Val Lys Phe Asn Trp Tyr Val
    290                 295                 300

Asp Gly Val Glu Val His Asn Ala Lys Thr Lys Pro Arg Glu Glu Gln
305                 310                 315                 320

Tyr Asn Ser Thr Tyr Arg Val Val Ser Val Leu Thr Val Leu His Gln
                325                 330                 335

Asp Trp Leu Asn Gly Lys Glu Tyr Lys Cys Lys Val Ser Asn Lys Ala
            340                 345                 350

Leu Pro Ala Pro Ile Glu Lys Thr Ile Ser Lys Ala Lys Gly Gln Pro
        355                 360                 365

Arg Glu Pro Gln Val Tyr Thr Leu Pro Pro Ser Arg Glu Glu Met Thr
    370                 375                 380

Lys Asn Gln Val Ser Leu Thr Cys Leu Val Lys Gly Phe Tyr Pro Ser
385                 390                 395                 400

Asp Ile Ala Val Glu Trp Glu Ser Asn Gly Gln Pro Glu Asn Asn Tyr
                405                 410                 415

Lys Thr Thr Pro Pro Val Leu Asp Ser Asp Gly Ser Phe Phe Leu Tyr
            420                 425                 430

Ser Lys Leu Thr Val Asp Lys Ser Arg Trp Gln Gln Gly Asn Val Phe
        435                 440                 445

Ser Cys Ser Val Met His Glu Ala Leu His Asn His Tyr Thr Gln Lys
    450                 455                 460

Ser Leu Ser Leu Ser Pro Gly Lys
465                 470

<210> 75
<211> 1416
<212> DNA
<213> Artificial Sequence

<220>
<223> nucleotide sequence of h2B1_H2

<400> 75
atgaaacacc tgtggttctt cctcctgctg gtggcagctc ccagatgggt gctgagccag 60

gtgcagctgg tggaatctgg cggcggactc gtgaagccta gccagaccct gagcctgacc 120

tgcaccttca gcggcttcag cctgaacacc tacgacatgg gcgtgggctg gatcagacag 180

cctcctggca agggcctgga atggatcggc aacatttggt gggacgacga caagtactac 240

aaccccagcc tgaagtccag agtgaccatc agcaaggaca ccagcaacaa ccaggcctcc 300

ctgaagctga gcagcgtgac agccgccgat accgccgtgt actactgcgc cagaatcgag 360

acagtgcggg tgtccagaaa gggcttcgcc cactggggac agggcacact cgtgaccgtc 420

agctcagcct ccaccaaggg cccaagcgtc ttcccctgg caccctcctc caagagcacc 480

tctggcggca cagccgccct gggctgcctg gtcaaggact acttccccga acccgtgacc 540

gtgagctgga actcaggcgc cctgaccagc ggcgtgcaca ccttccccgc tgtcctgcag 600

tcctcaggac tctactccct cagcagcgtg gtgaccgtgc cctccagcag cttgggcacc 660

cagacctaca tctgcaacgt gaatcacaag cccagcaaca ccaaggtgga caagagagtt 720

gagcccaaat cttgtgacaa aactcacaca tgcccaccct gcccagcacc tgaactcctg 780

gggggaccct cagtcttcct cttcccccca aaacccaagg acaccctcat gatctcccgg 840

acccctgagg tcacatgcgt ggtggtggac gtgagccacg aagaccctga ggtcaagttc 900

aactggtacg tggacggcgt ggaggtgcat aatgccaaga caaagccccg ggaggagcag 960

tacaacagca cgtaccgggt ggtcagcgtc ctcaccgtcc tgcaccagga ctggctgaat 1020

ggcaaggagt acaagtgcaa ggtctccaac aaagccctcc cagcccccat cgagaaaacc 1080

atctccaaag ccaaaggcca gccccgggaa ccacaggtgt acaccctgcc cccatcccgg 1140

gaggagatga ccaagaacca ggtcagcctg acctgcctgg tcaaaggctt ctatcccagc 1200

gacatcgccg tggagtggga gagcaatggc cagcccgaga acaactacaa gaccacccct 1260

cccgtgctgg actccgacgg ctccttcttc ctctacagca agctcaccgt ggacaagagc 1320

aggtggcagc agggcaacgt cttctcatgc tccgtgatgc atgaggctct gcacaaccac 1380

tacacccaga agagcctctc cctgtctccc ggcaaa 1416

<210> 76
<211> 472
<212> PRT
<213> Artificial Sequence

<220>
<223> amino acid sequence of h2B1_H2

<400> 76

Met Lys His Leu Trp Phe Phe Leu Leu Leu Val Ala Ala Pro Arg Trp
1 5 10 15

Val Leu Ser Gln Val Gln Leu Val Glu Ser Gly Gly Gly Leu Val Lys
20 25 30

Pro Ser Gln Thr Leu Ser Leu Thr Cys Thr Phe Ser Gly Phe Ser Leu
35 40 45

```
Asn Thr Tyr Asp Met Gly Val Gly Trp Ile Arg Gln Pro Pro Gly Lys
    50                  55                  60

Gly Leu Glu Trp Ile Gly Asn Ile Trp Trp Asp Asp Asp Lys Tyr Tyr
65                  70                  75                  80

Asn Pro Ser Leu Lys Ser Arg Val Thr Ile Ser Lys Asp Thr Ser Asn
                85                  90                  95

Asn Gln Ala Ser Leu Lys Leu Ser Ser Val Thr Ala Ala Asp Thr Ala
            100                 105                 110

Val Tyr Tyr Cys Ala Arg Ile Glu Thr Val Arg Val Ser Arg Lys Gly
        115                 120                 125

Phe Ala His Trp Gly Gln Gly Thr Leu Val Thr Val Ser Ser Ala Ser
    130                 135                 140

Thr Lys Gly Pro Ser Val Phe Pro Leu Ala Pro Ser Ser Lys Ser Thr
145                 150                 155                 160

Ser Gly Gly Thr Ala Ala Leu Gly Cys Leu Val Lys Asp Tyr Phe Pro
                165                 170                 175

Glu Pro Val Thr Val Ser Trp Asn Ser Gly Ala Leu Thr Ser Gly Val
            180                 185                 190

His Thr Phe Pro Ala Val Leu Gln Ser Ser Gly Leu Tyr Ser Leu Ser
        195                 200                 205

Ser Val Val Thr Val Pro Ser Ser Ser Leu Gly Thr Gln Thr Tyr Ile
    210                 215                 220

Cys Asn Val Asn His Lys Pro Ser Asn Thr Lys Val Asp Lys Arg Val
225                 230                 235                 240

Glu Pro Lys Ser Cys Asp Lys Thr His Thr Cys Pro Pro Cys Pro Ala
                245                 250                 255

Pro Glu Leu Leu Gly Gly Pro Ser Val Phe Leu Phe Pro Pro Lys Pro
            260                 265                 270

Lys Asp Thr Leu Met Ile Ser Arg Thr Pro Glu Val Thr Cys Val Val
        275                 280                 285

Val Asp Val Ser His Glu Asp Pro Glu Val Lys Phe Asn Trp Tyr Val
```

290 295 300

Asp Gly Val Glu Val His Asn Ala Lys Thr Lys Pro Arg Glu Glu Gln
305 310 315 320

Tyr Asn Ser Thr Tyr Arg Val Val Ser Val Leu Thr Val Leu His Gln
325 330 335

Asp Trp Leu Asn Gly Lys Glu Tyr Lys Cys Lys Val Ser Asn Lys Ala
340 345 350

Leu Pro Ala Pro Ile Glu Lys Thr Ile Ser Lys Ala Lys Gly Gln Pro
355 360 365

Arg Glu Pro Gln Val Tyr Thr Leu Pro Pro Ser Arg Glu Glu Met Thr
370 375 380

Lys Asn Gln Val Ser Leu Thr Cys Leu Val Lys Gly Phe Tyr Pro Ser
385 390 395 400

Asp Ile Ala Val Glu Trp Glu Ser Asn Gly Gln Pro Glu Asn Asn Tyr
405 410 415

Lys Thr Thr Pro Pro Val Leu Asp Ser Asp Gly Ser Phe Phe Leu Tyr
420 425 430

Ser Lys Leu Thr Val Asp Lys Ser Arg Trp Gln Gln Gly Asn Val Phe
435 440 445

Ser Cys Ser Val Met His Glu Ala Leu His Asn His Tyr Thr Gln Lys
450 455 460

Ser Leu Ser Leu Ser Pro Gly Lys
465 470

<210> 77
<211> 1416
<212> DNA
<213> Artificial Sequence

<220>
<223> nucleotide sequence of h2B1_H3

<400> 77
atgaaacacc tgtggttctt cctcctgctg gtggcagctc ccagatgggt gctgagccag 60

gtgcagctgg tggaatctgg cggcggactc gtgaagccta gccagaccct gagcctgacc 120

tgcaccttca gcggcttcag cctgaacacc tacgacatgg gcgtgggctg gatcagacag 180

cctcctggca agggcctgga atggatcgcc aacatttggt gggacgacga caagtactac 240

aaccccagcc tgaagtccag agtgaccatc agcaaggaca ccagcaacaa ccaggcctcc 300

ctgaagctga gcagcgtgac agccgccgat accgccgtgt actactgcgc cagaatcgag 360

acagtgcggg tgtccagaaa gggcttcgcc cactggggac agggcacact cgtgaccgtc 420

agctcagcct ccaccaaggg cccaagcgtc ttcccctgg caccctcctc caagagcacc 480

tctggcggca cagccgccct gggctgcctg gtcaaggact acttccccga acccgtgacc 540

gtgagctgga actcaggcgc cctgaccagc ggcgtgcaca ccttccccgc tgtcctgcag 600

tcctcaggac tctactccct cagcagcgtg gtgaccgtgc cctccagcag cttgggcacc 660

cagacctaca tctgcaacgt gaatcacaag cccagcaaca ccaaggtgga caagagagtt 720

gagcccaaat cttgtgacaa aactcacaca tgcccaccct gcccagcacc tgaactcctg 780

gggggaccct cagtcttcct cttcccccca aaacccaagg acaccctcat gatctcccgg 840

acccctgagg tcacatgcgt ggtggtggac gtgagccacg aagaccctga ggtcaagttc 900

aactggtacg tggacggcgt ggaggtgcat aatgccaaga caaagccccg ggaggagcag 960

tacaacagca cgtaccgggt ggtcagcgtc ctcaccgtcc tgcaccagga ctggctgaat 1020

ggcaaggagt acaagtgcaa ggtctccaac aaagccctcc cagcccccat cgagaaaacc 1080

atctccaaag ccaaaggcca gccccgggaa ccacaggtgt acaccctgcc cccatcccgg 1140

gaggagatga ccaagaacca ggtcagcctg acctgcctgg tcaaaggctt ctatcccagc 1200

gacatcgccg tggagtggga gagcaatggc cagcccgaga acaactacaa gaccacccct 1260

cccgtgctgg actccgacgg ctccttcttc ctctacagca agctcaccgt ggacaagagc 1320

aggtggcagc agggcaacgt cttctcatgc tccgtgatgc atgaggctct gcacaaccac 1380

tacacccaga agagcctctc cctgtctccc ggcaaa 1416

<210> 78
<211> 472
<212> PRT
<213> Artificial Sequence

<220>
<223> amino acid sequence of h2B1_H3

<400> 78

Met Lys His Leu Trp Phe Phe Leu Leu Leu Val Ala Ala Pro Arg Trp
1 5 10 15

Val Leu Ser Gln Val Gln Leu Val Glu Ser Gly Gly Gly Leu Val Lys
20 25 30

Pro Ser Gln Thr Leu Ser Leu Thr Cys Thr Phe Ser Gly Phe Ser Leu
35 40 45

```
Asn Thr Tyr Asp Met Gly Val Gly Trp Ile Arg Gln Pro Pro Gly Lys
    50                  55                  60

Gly Leu Glu Trp Ile Ala Asn Ile Trp Trp Asp Asp Asp Lys Tyr Tyr
65                  70                  75                  80

Asn Pro Ser Leu Lys Ser Arg Val Thr Ile Ser Lys Asp Thr Ser Asn
                85                  90                  95

Asn Gln Ala Ser Leu Lys Leu Ser Ser Val Thr Ala Ala Asp Thr Ala
            100                 105                 110

Val Tyr Tyr Cys Ala Arg Ile Glu Thr Val Arg Val Ser Arg Lys Gly
        115                 120                 125

Phe Ala His Trp Gly Gln Gly Thr Leu Val Thr Val Ser Ser Ala Ser
    130                 135                 140

Thr Lys Gly Pro Ser Val Phe Pro Leu Ala Pro Ser Ser Lys Ser Thr
145                 150                 155                 160

Ser Gly Gly Thr Ala Ala Leu Gly Cys Leu Val Lys Asp Tyr Phe Pro
                165                 170                 175

Glu Pro Val Thr Val Ser Trp Asn Ser Gly Ala Leu Thr Ser Gly Val
            180                 185                 190

His Thr Phe Pro Ala Val Leu Gln Ser Ser Gly Leu Tyr Ser Leu Ser
        195                 200                 205

Ser Val Val Thr Val Pro Ser Ser Ser Leu Gly Thr Gln Thr Tyr Ile
    210                 215                 220

Cys Asn Val Asn His Lys Pro Ser Asn Thr Lys Val Asp Lys Arg Val
225                 230                 235                 240

Glu Pro Lys Ser Cys Asp Lys Thr His Thr Cys Pro Pro Cys Pro Ala
                245                 250                 255

Pro Glu Leu Leu Gly Gly Pro Ser Val Phe Leu Phe Pro Pro Lys Pro
            260                 265                 270

Lys Asp Thr Leu Met Ile Ser Arg Thr Pro Glu Val Thr Cys Val Val
        275                 280                 285

Val Asp Val Ser His Glu Asp Pro Glu Val Lys Phe Asn Trp Tyr Val
    290                 295                 300
```

Asp Gly Val Glu Val His Asn Ala Lys Thr Lys Pro Arg Glu Glu Gln
305 310 315 320

Tyr Asn Ser Thr Tyr Arg Val Val Ser Val Leu Thr Val Leu His Gln
325 330 335

Asp Trp Leu Asn Gly Lys Glu Tyr Lys Cys Lys Val Ser Asn Lys Ala
340 345 350

Leu Pro Ala Pro Ile Glu Lys Thr Ile Ser Lys Ala Lys Gly Gln Pro
355 360 365

Arg Glu Pro Gln Val Tyr Thr Leu Pro Pro Ser Arg Glu Glu Met Thr
370 375 380

Lys Asn Gln Val Ser Leu Thr Cys Leu Val Lys Gly Phe Tyr Pro Ser
385 390 395 400

Asp Ile Ala Val Glu Trp Glu Ser Asn Gly Gln Pro Glu Asn Asn Tyr
405 410 415

Lys Thr Thr Pro Pro Val Leu Asp Ser Asp Gly Ser Phe Phe Leu Tyr
420 425 430

Ser Lys Leu Thr Val Asp Lys Ser Arg Trp Gln Gln Gly Asn Val Phe
435 440 445

Ser Cys Ser Val Met His Glu Ala Leu His Asn His Tyr Thr Gln Lys
450 455 460

Ser Leu Ser Leu Ser Pro Gly Lys
465 470

<210> 79
<211> 1416
<212> DNA
<213> Artificial Sequence

<220>
<223> nucleotide sequence of h2B1_H4

<400> 79
atgaaacacc tgtggttctt cctcctgctg gtggcagctc ccagatgggt gctgagccaa 60

gtgaccctga aagagtccgg ccctgccctc gtgaagccta cccagaccct gacactgacc 120

tgcaccttca gcggcttcag cctgaacacc tacgacatgg gcgtgggctg gatcagacag 180

cctcctggca agggcctgga atggctggcc aacatttggt gggacgacga caagtactac 240

agccccagcc tgaagtcccg gctgaccatc accaaggaca ccagcaacaa ccaggccgtg 300
ctgaccatga caaacatgga ccccgtggac accgccacct actactgcgc cagaatcgag 360
acagtgcggg tgtccagaaa gggcttcgcc cactggggac agggcacact cgtgaccgtc 420
agctcagcct ccaccaaggg cccaagcgtc ttcccctgg caccctcctc caagagcacc 480
tctggcggca cagccgccct gggctgcctg gtcaaggact acttccccga acccgtgacc 540
gtgagctgga actcaggcgc cctgaccagc ggcgtgcaca ccttccccgc tgtcctgcag 600
tcctcaggac tctactccct cagcagcgtg gtgaccgtgc cctccagcag cttgggcacc 660
cagacctaca tctgcaacgt gaatcacaag cccagcaaca ccaaggtgga caagagagtt 720
gagcccaaat cttgtgacaa aactcacaca tgcccaccct gcccagcacc tgaactcctg 780
gggggaccct cagtcttcct cttcccccca aaacccaagg acaccctcat gatctcccgg 840
acccctgagg tcacatgcgt ggtggtggac gtgagccacg aagaccctga ggtcaagttc 900
aactggtacg tggacggcgt ggaggtgcat aatgccaaga caaagccccg ggaggagcag 960
tacaacagca cgtaccgggt ggtcagcgtc ctcaccgtcc tgcaccagga ctggctgaat 1020
ggcaaggagt acaagtgcaa ggtctccaac aaagccctcc cagcccccat cgagaaaacc 1080
atctccaaag ccaaaggcca gccccgggaa ccacaggtgt acaccctgcc cccatcccgg 1140
gaggagatga ccaagaacca ggtcagcctg acctgcctgg tcaaaggctt ctatcccagc 1200
gacatcgccg tggagtggga gagcaatggc cagcccgaga acaactacaa gaccacccct 1260
cccgtgctgg actccgacgg ctccttcttc ctctacagca agctcaccgt ggacaagagc 1320
aggtggcagc agggcaacgt cttctcatgc tccgtgatgc atgaggctct gcacaaccac 1380
tacacccaga agagcctctc cctgtctccc ggcaaa 1416

<210> 80
<211> 472
<212> PRT
<213> Artificial Sequence

<220>
<223> amino acid sequence of h2B1_H4

<400> 80

Met Lys His Leu Trp Phe Phe Leu Leu Leu Val Ala Ala Pro Arg Trp
1 5 10 15

Val Leu Ser Gln Val Thr Leu Lys Glu Ser Gly Pro Ala Leu Val Lys
20 25 30

Pro Thr Gln Thr Leu Thr Leu Thr Cys Thr Phe Ser Gly Phe Ser Leu
35 40 45

Asn Thr Tyr Asp Met Gly Val Gly Trp Ile Arg Gln Pro Pro Gly Lys
    50                  55                  60

Gly Leu Glu Trp Leu Ala Asn Ile Trp Trp Asp Asp Asp Lys Tyr Tyr
65                  70                  75                  80

Ser Pro Ser Leu Lys Ser Arg Leu Thr Ile Thr Lys Asp Thr Ser Asn
                85                  90                  95

Asn Gln Ala Val Leu Thr Met Thr Asn Met Asp Pro Val Asp Thr Ala
            100                 105                 110

Thr Tyr Tyr Cys Ala Arg Ile Glu Thr Val Arg Val Ser Arg Lys Gly
        115                 120                 125

Phe Ala His Trp Gly Gln Gly Thr Leu Val Thr Val Ser Ser Ala Ser
    130                 135                 140

Thr Lys Gly Pro Ser Val Phe Pro Leu Ala Pro Ser Ser Lys Ser Thr
145                 150                 155                 160

Ser Gly Gly Thr Ala Ala Leu Gly Cys Leu Val Lys Asp Tyr Phe Pro
                165                 170                 175

Glu Pro Val Thr Val Ser Trp Asn Ser Gly Ala Leu Thr Ser Gly Val
            180                 185                 190

His Thr Phe Pro Ala Val Leu Gln Ser Ser Gly Leu Tyr Ser Leu Ser
        195                 200                 205

Ser Val Val Thr Val Pro Ser Ser Ser Leu Gly Thr Gln Thr Tyr Ile
    210                 215                 220

Cys Asn Val Asn His Lys Pro Ser Asn Thr Lys Val Asp Lys Arg Val
225                 230                 235                 240

Glu Pro Lys Ser Cys Asp Lys Thr His Thr Cys Pro Pro Cys Pro Ala
                245                 250                 255

Pro Glu Leu Leu Gly Gly Pro Ser Val Phe Leu Phe Pro Pro Lys Pro
            260                 265                 270

Lys Asp Thr Leu Met Ile Ser Arg Thr Pro Glu Val Thr Cys Val Val
        275                 280                 285

Val Asp Val Ser His Glu Asp Pro Glu Val Lys Phe Asn Trp Tyr Val
    290                 295                 300

Asp Gly Val Glu Val His Asn Ala Lys Thr Lys Pro Arg Glu Glu Gln
305 310 315 320

Tyr Asn Ser Thr Tyr Arg Val Val Ser Val Leu Thr Val Leu His Gln
325 330 335

Asp Trp Leu Asn Gly Lys Glu Tyr Lys Cys Lys Val Ser Asn Lys Ala
340 345 350

Leu Pro Ala Pro Ile Glu Lys Thr Ile Ser Lys Ala Lys Gly Gln Pro
355 360 365

Arg Glu Pro Gln Val Tyr Thr Leu Pro Pro Ser Arg Glu Glu Met Thr
370 375 380

Lys Asn Gln Val Ser Leu Thr Cys Leu Val Lys Gly Phe Tyr Pro Ser
385 390 395 400

Asp Ile Ala Val Glu Trp Glu Ser Asn Gly Gln Pro Glu Asn Asn Tyr
405 410 415

Lys Thr Thr Pro Pro Val Leu Asp Ser Asp Gly Ser Phe Phe Leu Tyr
420 425 430

Ser Lys Leu Thr Val Asp Lys Ser Arg Trp Gln Gln Gly Asn Val Phe
435 440 445

Ser Cys Ser Val Met His Glu Ala Leu His Asn His Tyr Thr Gln Lys
450 455 460

Ser Leu Ser Leu Ser Pro Gly Lys
465 470

<210> 81
<211> 699
<212> DNA
<213> Artificial Sequence

<220>
<223> nucleotide sequence of h7B4_L1

<400> 81
atggtgctgc agacccaggt gttcatctcc ctgctgctgt ggatctccgg cgcgtacggc 60

gagatcgtgc tgacacagag ccctggcacc ctgagcctgt ctccaggcga aagagccacc 120

ctgtcctgca aggccagcca gaacatcaac aagtacctgg actggtatca gcagaagccc 180

ggccagcccc ccagactgct gatctacaac accaacaacc tgcacaccgg catccccgac 240

agattcagcg gctctggcag cggcaccgac tacaccctga ccatcagcag actggaaccc 300

```
gaggacttcg ccgtgtacta ctgcctgcag cggaacagct ggtacacctt cggccagggc      360

accaaggtgg aaatcaagcg tacggtggcc gcccccctccg tgttcatctt cccccccctcc      420

gacgagcagc tgaagtccgg caccgcctcc gtggtgtgcc tgctgaataa cttctacccc      480

agagaggcca aggtgcagtg gaaggtggac aacgccctgc agtccgggaa ctcccaggag      540

agcgtgaccg agcaggacag caaggacagc acctacagcc tgagcagcac cctgaccctg      600

agcaaagccg actacgagaa gcacaaggtg tacgcctgcg aggtgaccca ccagggcctg      660

agctcccccg tcaccaagag cttcaacagg ggggagtgt                              699
```

<210> 82
<211> 233
<212> PRT
<213> Artificial Sequence

<220>
<223> amino acid sequence of h7B4_L1

<400> 82

```
Met Val Leu Gln Thr Gln Val Phe Ile Ser Leu Leu Leu Trp Ile Ser
1               5                   10                  15

Gly Ala Tyr Gly Glu Ile Val Leu Thr Gln Ser Pro Gly Thr Leu Ser
            20                  25                  30

Leu Ser Pro Gly Glu Arg Ala Thr Leu Ser Cys Lys Ala Ser Gln Asn
        35                  40                  45

Ile Asn Lys Tyr Leu Asp Trp Tyr Gln Gln Lys Pro Gly Gln Pro Pro
    50                  55                  60

Arg Leu Leu Ile Tyr Asn Thr Asn Asn Leu His Thr Gly Ile Pro Asp
65                  70                  75                  80

Arg Phe Ser Gly Ser Gly Ser Gly Thr Asp Tyr Thr Leu Thr Ile Ser
                85                  90                  95

Arg Leu Glu Pro Glu Asp Phe Ala Val Tyr Tyr Cys Leu Gln Arg Asn
            100                 105                 110

Ser Trp Tyr Thr Phe Gly Gln Gly Thr Lys Val Glu Ile Lys Arg Thr
        115                 120                 125

Val Ala Ala Pro Ser Val Phe Ile Phe Pro Pro Ser Asp Glu Gln Leu
    130                 135                 140

Lys Ser Gly Thr Ala Ser Val Val Cys Leu Leu Asn Asn Phe Tyr Pro
```

145 150 155 160

Arg Glu Ala Lys Val Gln Trp Lys Val Asp Asn Ala Leu Gln Ser Gly
165 170 175

Asn Ser Gln Glu Ser Val Thr Glu Gln Asp Ser Lys Asp Ser Thr Tyr
180 185 190

Ser Leu Ser Ser Thr Leu Thr Leu Ser Lys Ala Asp Tyr Glu Lys His
195 200 205

Lys Val Tyr Ala Cys Glu Val Thr His Gln Gly Leu Ser Ser Pro Val
210 215 220

Thr Lys Ser Phe Asn Arg Gly Glu Cys
225 230

<210> 83
<211> 699
<212> DNA
<213> Artificial Sequence

<220>
<223> nucleotide sequence of h7B4_L2

<400> 83
atggtgctgc agacccaggt gttcatctcc ctgctgctgt ggatctccgg cgcgtacggc 60

gacatccaga tgacccagag ccctagcagc ctgagcctgt cccctggcga aagagccacc 120

ctgagctgca aggccagcca gaacatcaac aagtacctgg actggtatca gcagaagccc 180

ggccagcccc ccagactgct gatctacaac accaacaacc tgcacaccgg catccccgac 240

agattcagcg gctctggcag cggcaccgac tacaccctga ccatcagcag actggaaccc 300

gaggacttcg ccgtgtacta ctgcctgcag cggaacagct ggtacacctt cggccagggc 360

accaaggtgg aaatcaagcg tacggtggcc gccccctccg tgttcatctt cccccctcc 420

gacgagcagc tgaagtccgg caccgcctcc gtggtgtgcc tgctgaataa cttctacccc 480

agagaggcca aggtgcagtg gaaggtggac aacgccctgc agtccgggaa ctcccaggag 540

agcgtgaccg agcaggacag caaggacagc acctacagcc tgagcagcac cctgaccctg 600

agcaaagccg actacgagaa gcacaaggtg tacgcctgcg aggtgaccca ccagggcctg 660

agctcccccg tcaccaagag cttcaacagg ggggagtgt 699

<210> 84
<211> 233
<212> PRT
<213> Artificial Sequence

<220>

<223>  amino acid sequence of h7B4_L2

<400>  84

```
Met Val Leu Gln Thr Gln Val Phe Ile Ser Leu Leu Leu Trp Ile Ser
1               5                   10                  15

Gly Ala Tyr Gly Asp Ile Gln Met Thr Gln Ser Pro Ser Ser Leu Ser
            20                  25                  30

Leu Ser Pro Gly Glu Arg Ala Thr Leu Ser Cys Lys Ala Ser Gln Asn
        35                  40                  45

Ile Asn Lys Tyr Leu Asp Trp Tyr Gln Gln Lys Pro Gly Gln Pro Pro
    50                  55                  60

Arg Leu Leu Ile Tyr Asn Thr Asn Asn Leu His Thr Gly Ile Pro Asp
65                  70                  75                  80

Arg Phe Ser Gly Ser Gly Ser Gly Thr Asp Tyr Thr Leu Thr Ile Ser
                85                  90                  95

Arg Leu Glu Pro Glu Asp Phe Ala Val Tyr Tyr Cys Leu Gln Arg Asn
            100                 105                 110

Ser Trp Tyr Thr Phe Gly Gln Gly Thr Lys Val Glu Ile Lys Arg Thr
        115                 120                 125

Val Ala Ala Pro Ser Val Phe Ile Phe Pro Pro Ser Asp Glu Gln Leu
    130                 135                 140

Lys Ser Gly Thr Ala Ser Val Val Cys Leu Leu Asn Asn Phe Tyr Pro
145                 150                 155                 160

Arg Glu Ala Lys Val Gln Trp Lys Val Asp Asn Ala Leu Gln Ser Gly
                165                 170                 175

Asn Ser Gln Glu Ser Val Thr Glu Gln Asp Ser Lys Asp Ser Thr Tyr
            180                 185                 190

Ser Leu Ser Ser Thr Leu Thr Leu Ser Lys Ala Asp Tyr Glu Lys His
        195                 200                 205

Lys Val Tyr Ala Cys Glu Val Thr His Gln Gly Leu Ser Ser Pro Val
    210                 215                 220

Thr Lys Ser Phe Asn Arg Gly Glu Cys
225                 230
```

<210> 85
<211> 1416
<212> DNA
<213> Artificial Sequence

<220>
<223> nucleotide sequence of h7B4_H1

<400> 85
atgaaacacc tgtggttctt cctcctgctg gtggcagctc ccagatgggt gctgagccag 60

gtgcagctgc aggaatctgg ccctggcctc gtgaagccta gccagaccct gagcctgacc 120

tgtaccgtgt ccggctacac catcaccagc ggctacgact ggtcctggat cagacagcct 180

cctggcaagg gcctggaatg gatcgcctac atgagctacc ggggcagcac caactacaac 240

cccagcctga agtccagagt gaccatcagc cgggacacca gcaagaacca gttctccctg 300

aagctgagca gcgtgacagc cgccgatacc gccgtgtact actgcgccct gacccggacc 360

tactggtaca actactacta cgtgctggac gcctggggcc agggcacact cgtgaccgtc 420

agctcagcct ccaccaaggg cccaagcgtc ttcccccctgg caccctcctc caagagcacc 480

tctggcggca cagccgccct gggctgcctg gtcaaggact acttccccga acccgtgacc 540

gtgagctgga actcaggcgc cctgaccagc ggcgtgcaca ccttccccgc tgtcctgcag 600

tcctcaggac tctactccct cagcagcgtg gtgaccgtgc cctccagcag cttgggcacc 660

cagacctaca tctgcaacgt gaatcacaag cccagcaaca ccaaggtgga caagagagtt 720

gagcccaaat cttgtgacaa aactcacaca tgcccaccct gcccagcacc tgaactcctg 780

gggggaccct cagtcttcct cttcccccca aaacccaagg acaccctcat gatctcccgg 840

acccctgagg tcacatgcgt ggtggtggac gtgagccacg aagaccctga ggtcaagttc 900

aactggtacg tggacggcgt ggaggtgcat aatgccaaga caaagccccg ggaggagcag 960

tacaacagca cgtaccgggt ggtcagcgtc ctcaccgtcc tgcaccagga ctggctgaat 1020

ggcaaggagt acaagtgcaa ggtctccaac aaagccctcc cagcccccat cgagaaaacc 1080

atctccaaag ccaaaggcca gccccgggaa ccacaggtgt acaccctgcc cccatcccgg 1140

gaggagatga ccaagaacca ggtcagcctg acctgcctgg tcaaaggctt ctatcccagc 1200

gacatcgccg tggagtggga gagcaatggc cagcccgaga acaactacaa gaccacccct 1260

cccgtgctgg actccgacgg ctccttcttc ctctacagca agctcaccgt ggacaagagc 1320

aggtggcagc agggcaacgt cttctcatgc tccgtgatgc atgaggctct gcacaaccac 1380

tacacccaga agagcctctc cctgtctccc ggcaaa 1416

<210> 86
<211> 472
<212> PRT
<213> Artificial Sequence

<220>
<223> amino acid sequence of h7B4_H1

<400> 86

```
Met Lys His Leu Trp Phe Phe Leu Leu Leu Val Ala Ala Pro Arg Trp
1               5                   10                  15

Val Leu Ser Gln Val Gln Leu Gln Glu Ser Gly Pro Gly Leu Val Lys
            20                  25                  30

Pro Ser Gln Thr Leu Ser Leu Thr Cys Thr Val Ser Gly Tyr Thr Ile
        35                  40                  45

Thr Ser Gly Tyr Asp Trp Ser Trp Ile Arg Gln Pro Pro Gly Lys Gly
    50                  55                  60

Leu Glu Trp Ile Ala Tyr Met Ser Tyr Arg Gly Ser Thr Asn Tyr Asn
65                  70                  75                  80

Pro Ser Leu Lys Ser Arg Val Thr Ile Ser Arg Asp Thr Ser Lys Asn
                85                  90                  95

Gln Phe Ser Leu Lys Leu Ser Ser Val Thr Ala Ala Asp Thr Ala Val
            100                 105                 110

Tyr Tyr Cys Ala Leu Thr Arg Thr Tyr Trp Tyr Asn Tyr Tyr Tyr Val
        115                 120                 125

Leu Asp Ala Trp Gly Gln Gly Thr Leu Val Thr Val Ser Ser Ala Ser
    130                 135                 140

Thr Lys Gly Pro Ser Val Phe Pro Leu Ala Pro Ser Ser Lys Ser Thr
145                 150                 155                 160

Ser Gly Gly Thr Ala Ala Leu Gly Cys Leu Val Lys Asp Tyr Phe Pro
                165                 170                 175

Glu Pro Val Thr Val Ser Trp Asn Ser Gly Ala Leu Thr Ser Gly Val
            180                 185                 190

His Thr Phe Pro Ala Val Leu Gln Ser Ser Gly Leu Tyr Ser Leu Ser
        195                 200                 205

Ser Val Val Thr Val Pro Ser Ser Ser Leu Gly Thr Gln Thr Tyr Ile
    210                 215                 220

Cys Asn Val Asn His Lys Pro Ser Asn Thr Lys Val Asp Lys Arg Val
```

225 230 235 240

Glu Pro Lys Ser Cys Asp Lys Thr His Thr Cys Pro Pro Cys Pro Ala
245 250 255

Pro Glu Leu Leu Gly Gly Pro Ser Val Phe Leu Phe Pro Pro Lys Pro
260 265 270

Lys Asp Thr Leu Met Ile Ser Arg Thr Pro Glu Val Thr Cys Val Val
275 280 285

Val Asp Val Ser His Glu Asp Pro Glu Val Lys Phe Asn Trp Tyr Val
290 295 300

Asp Gly Val Glu Val His Asn Ala Lys Thr Lys Pro Arg Glu Glu Gln
305 310 315 320

Tyr Asn Ser Thr Tyr Arg Val Val Ser Val Leu Thr Val Leu His Gln
325 330 335

Asp Trp Leu Asn Gly Lys Glu Tyr Lys Cys Lys Val Ser Asn Lys Ala
340 345 350

Leu Pro Ala Pro Ile Glu Lys Thr Ile Ser Lys Ala Lys Gly Gln Pro
355 360 365

Arg Glu Pro Gln Val Tyr Thr Leu Pro Pro Ser Arg Glu Glu Met Thr
370 375 380

Lys Asn Gln Val Ser Leu Thr Cys Leu Val Lys Gly Phe Tyr Pro Ser
385 390 395 400

Asp Ile Ala Val Glu Trp Glu Ser Asn Gly Gln Pro Glu Asn Asn Tyr
405 410 415

Lys Thr Thr Pro Pro Val Leu Asp Ser Asp Gly Ser Phe Phe Leu Tyr
420 425 430

Ser Lys Leu Thr Val Asp Lys Ser Arg Trp Gln Gln Gly Asn Val Phe
435 440 445

Ser Cys Ser Val Met His Glu Ala Leu His Asn His Tyr Thr Gln Lys
450 455 460

Ser Leu Ser Leu Ser Pro Gly Lys
465 470

<210> 87
<211> 1416
<212> DNA
<213> Artificial Sequence

<220>
<223> nucleotide sequence of h7B4_H2

<400> 87
atgaaacacc tgtggttctt cctcctgctg gtggcagctc ccagatgggt gctgagcgag 60

atccagctgc aggaatctgg ccctggcctc gtgaagccta gccagaccct gagcctgacc 120

tgtaccgtgt ccggctacac catcaccagc ggctacgact ggtcctggat cagacagcct 180

cctggcaagg gcctggaatg gatcggctac atgagctacc ggggcagcac caactacaac 240

cccagcctga agtccagagt gaccatcagc cgggacacca gcaagaacca gttctccctg 300

aagctgagca gcgtgacagc cgccgatacc gccgtgtact actgcgccct gacccggacc 360

tactggtaca actactacta cgtgctggac gcctggggcc agggcacact cgtgaccgtc 420

agctcagcct ccaccaaggg cccaagcgtc ttccccctgg caccctcctc caagagcacc 480

tctggcggca cagccgccct gggctgcctg gtcaaggact acttccccga acccgtgacc 540

gtgagctgga actcaggcgc cctgaccagc ggcgtgcaca ccttccccgc tgtcctgcag 600

tcctcaggac tctactccct cagcagcgtg gtgaccgtgc cctccagcag cttgggcacc 660

cagacctaca tctgcaacgt gaatcacaag cccagcaaca ccaaggtgga caagagagtt 720

gagcccaaat cttgtgacaa aactcacaca tgcccaccct gcccagcacc tgaactcctg 780

gggggaccct cagtcttcct cttcccccca aaacccaagg acaccctcat gatctcccgg 840

acccctgagg tcacatgcgt ggtggtggac gtgagccacg aagaccctga ggtcaagttc 900

aactggtacg tggacggcgt ggaggtgcat aatgccaaga caaagccccg ggaggagcag 960

tacaacagca cgtaccgggt ggtcagcgtc ctcaccgtcc tgcaccagga ctggctgaat 1020

ggcaaggagt acaagtgcaa ggtctccaac aaagccctcc cagcccccat cgagaaaacc 1080

atctccaaag ccaaaggcca gccccgggaa ccacaggtgt acaccctgcc cccatcccgg 1140

gaggagatga ccaagaacca ggtcagcctg acctgcctgg tcaaaggctt ctatcccagc 1200

gacatcgccg tggagtggga gagcaatggc cagcccgaga acaactacaa gaccacccct 1260

cccgtgctgg actccgacgg ctccttcttc ctctacagca agctcaccgt ggacaagagc 1320

aggtggcagc agggcaacgt cttctcatgc tccgtgatgc atgaggctct gcacaaccac 1380

tacacccaga agagcctctc cctgtctccc ggcaaa 1416

<210> 88
<211> 472
<212> PRT
<213> Artificial Sequence

<220>
<223> amino acid sequence of h7B4_H2

<400> 88

Met Lys His Leu Trp Phe Phe Leu Leu Leu Val Ala Ala Pro Arg Trp
1 5 10 15

Val Leu Ser Glu Ile Gln Leu Gln Glu Ser Gly Pro Gly Leu Val Lys
20 25 30

Pro Ser Gln Thr Leu Ser Leu Thr Cys Thr Val Ser Gly Tyr Thr Ile
35 40 45

Thr Ser Gly Tyr Asp Trp Ser Trp Ile Arg Gln Pro Pro Gly Lys Gly
50 55 60

Leu Glu Trp Ile Gly Tyr Met Ser Tyr Arg Gly Ser Thr Asn Tyr Asn
65 70 75 80

Pro Ser Leu Lys Ser Arg Val Thr Ile Ser Arg Asp Thr Ser Lys Asn
85 90 95

Gln Phe Ser Leu Lys Leu Ser Ser Val Thr Ala Ala Asp Thr Ala Val
100 105 110

Tyr Tyr Cys Ala Leu Thr Arg Thr Tyr Trp Tyr Asn Tyr Tyr Tyr Val
115 120 125

Leu Asp Ala Trp Gly Gln Gly Thr Leu Val Thr Val Ser Ser Ala Ser
130 135 140

Thr Lys Gly Pro Ser Val Phe Pro Leu Ala Pro Ser Ser Lys Ser Thr
145 150 155 160

Ser Gly Gly Thr Ala Ala Leu Gly Cys Leu Val Lys Asp Tyr Phe Pro
165 170 175

Glu Pro Val Thr Val Ser Trp Asn Ser Gly Ala Leu Thr Ser Gly Val
180 185 190

His Thr Phe Pro Ala Val Leu Gln Ser Ser Gly Leu Tyr Ser Leu Ser
195 200 205

Ser Val Val Thr Val Pro Ser Ser Ser Leu Gly Thr Gln Thr Tyr Ile
210 215 220

Cys Asn Val Asn His Lys Pro Ser Asn Thr Lys Val Asp Lys Arg Val
225 230 235 240

```
Glu Pro Lys Ser Cys Asp Lys Thr His Thr Cys Pro Pro Cys Pro Ala
                245                 250                 255

Pro Glu Leu Leu Gly Gly Pro Ser Val Phe Leu Phe Pro Pro Lys Pro
            260                 265                 270

Lys Asp Thr Leu Met Ile Ser Arg Thr Pro Glu Val Thr Cys Val Val
        275                 280                 285

Val Asp Val Ser His Glu Asp Pro Glu Val Lys Phe Asn Trp Tyr Val
    290                 295                 300

Asp Gly Val Glu Val His Asn Ala Lys Thr Lys Pro Arg Glu Glu Gln
305                 310                 315                 320

Tyr Asn Ser Thr Tyr Arg Val Val Ser Val Leu Thr Val Leu His Gln
                325                 330                 335

Asp Trp Leu Asn Gly Lys Glu Tyr Lys Cys Lys Val Ser Asn Lys Ala
            340                 345                 350

Leu Pro Ala Pro Ile Glu Lys Thr Ile Ser Lys Ala Lys Gly Gln Pro
        355                 360                 365

Arg Glu Pro Gln Val Tyr Thr Leu Pro Pro Ser Arg Glu Glu Met Thr
    370                 375                 380

Lys Asn Gln Val Ser Leu Thr Cys Leu Val Lys Gly Phe Tyr Pro Ser
385                 390                 395                 400

Asp Ile Ala Val Glu Trp Glu Ser Asn Gly Gln Pro Glu Asn Asn Tyr
                405                 410                 415

Lys Thr Thr Pro Pro Val Leu Asp Ser Asp Gly Ser Phe Phe Leu Tyr
            420                 425                 430

Ser Lys Leu Thr Val Asp Lys Ser Arg Trp Gln Gln Gly Asn Val Phe
        435                 440                 445

Ser Cys Ser Val Met His Glu Ala Leu His Asn His Tyr Thr Gln Lys
    450                 455                 460

Ser Leu Ser Leu Ser Pro Gly Lys
465                 470
```

<210> 89

<211> 1416
<212> DNA
<213> Artificial Sequence

<220>
<223> nucleotide sequence of h7B4_H3

<400> 89
atgaaacacc tgtggttctt cctcctgctg gtggcagctc ccagatgggt gctgagcgag 60

atccagctgc aggaatctgg ccctggcctc gtgaagccta gccagaccct gagcctgacc 120

tgtaccgtgt ccggctacac catcaccagc ggctacgact ggtcctggat cagacagcct 180

cctggcaagg gcctggaatg gatcgcctac atgagctacc ggggcagcac caactacaac 240

cccagcctga agtccagagt gaccatcagc cgggacacca gcaagaacca gttctccctg 300

aagctgagca gcgtgacagc cgccgatacc gccgtgtact actgcgccct gacccggacc 360

tactggtaca actactacta cgtgctggac gcctggggcc agggcacact cgtgaccgtc 420

agctcagcct ccaccaaggg cccaagcgtc ttccccctgg caccctcctc caagagcacc 480

tctggcggca cagccgccct gggctgcctg gtcaaggact acttccccga acccgtgacc 540

gtgagctgga actcaggcgc cctgaccagc ggcgtgcaca ccttccccgc tgtcctgcag 600

tcctcaggac tctactccct cagcagcgtg gtgaccgtgc cctccagcag cttgggcacc 660

cagacctaca tctgcaacgt gaatcacaag cccagcaaca ccaaggtgga caagagagtt 720

gagcccaaat cttgtgacaa aactcacaca tgcccaccct gcccagcacc tgaactcctg 780

gggggaccct cagtcttcct cttcccccca aaacccaagg acaccctcat gatctcccgg 840

acccctgagg tcacatgcgt ggtggtggac gtgagccacg aagaccctga ggtcaagttc 900

aactggtacg tggacggcgt ggaggtgcat aatgccaaga caaagccccg ggaggagcag 960

tacaacagca cgtaccgggt ggtcagcgtc ctcaccgtcc tgcaccagga ctggctgaat 1020

ggcaaggagt acaagtgcaa ggtctccaac aaagccctcc cagcccccat cgagaaaacc 1080

atctccaaag ccaaaggcca gccccgggaa ccacaggtgt acaccctgcc cccatcccgg 1140

gaggagatga ccaagaacca ggtcagcctg acctgcctgg tcaaaggctt ctatcccagc 1200

gacatcgccg tggagtggga gagcaatggc cagcccgaga acaactacaa gaccacccct 1260

cccgtgctgg actccgacgg ctccttcttc ctctacagca agctcaccgt ggacaagagc 1320

aggtggcagc agggcaacgt cttctcatgc tccgtgatgc atgaggctct gcacaaccac 1380

tacacccaga agagcctctc cctgtctccc ggcaaa 1416

<210> 90
<211> 472
<212> PRT
<213> Artificial Sequence

<220>

<223> amino acid sequence of h7B4_H3

<400> 90

Met Lys His Leu Trp Phe Phe Leu Leu Leu Val Ala Ala Pro Arg Trp
1               5                   10                  15

Val Leu Ser Glu Ile Gln Leu Gln Glu Ser Gly Pro Gly Leu Val Lys
            20                  25                  30

Pro Ser Gln Thr Leu Ser Leu Thr Cys Thr Val Ser Gly Tyr Thr Ile
        35                  40                  45

Thr Ser Gly Tyr Asp Trp Ser Trp Ile Arg Gln Pro Pro Gly Lys Gly
    50                  55                  60

Leu Glu Trp Ile Ala Tyr Met Ser Tyr Arg Gly Ser Thr Asn Tyr Asn
65                  70                  75                  80

Pro Ser Leu Lys Ser Arg Val Thr Ile Ser Arg Asp Thr Ser Lys Asn
                85                  90                  95

Gln Phe Ser Leu Lys Leu Ser Ser Val Thr Ala Ala Asp Thr Ala Val
            100                 105                 110

Tyr Tyr Cys Ala Leu Thr Arg Thr Tyr Trp Tyr Asn Tyr Tyr Tyr Val
        115                 120                 125

Leu Asp Ala Trp Gly Gln Gly Thr Leu Val Thr Val Ser Ser Ala Ser
    130                 135                 140

Thr Lys Gly Pro Ser Val Phe Pro Leu Ala Pro Ser Ser Lys Ser Thr
145                 150                 155                 160

Ser Gly Gly Thr Ala Ala Leu Gly Cys Leu Val Lys Asp Tyr Phe Pro
                165                 170                 175

Glu Pro Val Thr Val Ser Trp Asn Ser Gly Ala Leu Thr Ser Gly Val
            180                 185                 190

His Thr Phe Pro Ala Val Leu Gln Ser Ser Gly Leu Tyr Ser Leu Ser
        195                 200                 205

Ser Val Val Thr Val Pro Ser Ser Ser Leu Gly Thr Gln Thr Tyr Ile
    210                 215                 220

Cys Asn Val Asn His Lys Pro Ser Asn Thr Lys Val Asp Lys Arg Val
225                 230                 235                 240

```
Glu Pro Lys Ser Cys Asp Lys Thr His Thr Cys Pro Pro Cys Pro Ala
                245                 250                 255

Pro Glu Leu Leu Gly Gly Pro Ser Val Phe Leu Phe Pro Pro Lys Pro
            260                 265                 270

Lys Asp Thr Leu Met Ile Ser Arg Thr Pro Glu Val Thr Cys Val Val
        275                 280                 285

Val Asp Val Ser His Glu Asp Pro Glu Val Lys Phe Asn Trp Tyr Val
    290                 295                 300

Asp Gly Val Glu Val His Asn Ala Lys Thr Lys Pro Arg Glu Glu Gln
305                 310                 315                 320

Tyr Asn Ser Thr Tyr Arg Val Val Ser Val Leu Thr Val Leu His Gln
                325                 330                 335

Asp Trp Leu Asn Gly Lys Glu Tyr Lys Cys Lys Val Ser Asn Lys Ala
            340                 345                 350

Leu Pro Ala Pro Ile Glu Lys Thr Ile Ser Lys Ala Lys Gly Gln Pro
        355                 360                 365

Arg Glu Pro Gln Val Tyr Thr Leu Pro Pro Ser Arg Glu Glu Met Thr
    370                 375                 380

Lys Asn Gln Val Ser Leu Thr Cys Leu Val Lys Gly Phe Tyr Pro Ser
385                 390                 395                 400

Asp Ile Ala Val Glu Trp Glu Ser Asn Gly Gln Pro Glu Asn Asn Tyr
                405                 410                 415

Lys Thr Thr Pro Pro Val Leu Asp Ser Asp Gly Ser Phe Phe Leu Tyr
            420                 425                 430

Ser Lys Leu Thr Val Asp Lys Ser Arg Trp Gln Gln Gly Asn Val Phe
        435                 440                 445

Ser Cys Ser Val Met His Glu Ala Leu His Asn His Tyr Thr Gln Lys
    450                 455                 460

Ser Leu Ser Leu Ser Pro Gly Lys
465                 470
```

<210>  91
<211>  1416

<212> DNA
<213> Artificial Sequence

<220>
<223> nucleotide sequence of h7B4_H5

<400> 91
atgaaacacc tgtggttctt cctcctgctg gtggcagctc ccagatgggt gctgagcgag 60

atccagctgc aggaatctgg ccctggcctc gtgaagccta gccagaccct gagcctgacc 120

tgtaccgtgt ccggctacac catcaccagc ggctacgact ggtcctggat cagaaagccc 180

cctggcaaca agatggaatg gatcgcctac atgagctacc ggggcagcac caactacaac 240

cccagcctga agtccagagt gaccatcagc cgggacacca gcaagaacca gttctccctg 300

aagctgagca gcgtgacagc cgccgatacc gccgtgtact actgcgccct gacccggacc 360

tactggtaca actactacta cgtgctggac gcctggggcc agggcacact cgtgaccgtc 420

agctcagcct ccaccaaggg cccaagcgtc ttccccctgg caccctcctc caagagcacc 480

tctggcggca cagccgccct gggctgcctg gtcaaggact acttccccga acccgtgacc 540

gtgagctgga actcaggcgc cctgaccagc ggcgtgcaca ccttccccgc tgtcctgcag 600

tcctcaggac tctactccct cagcagcgtg gtgaccgtgc cctccagcag cttgggcacc 660

cagacctaca tctgcaacgt gaatcacaag cccagcaaca ccaaggtgga caagagagtt 720

gagcccaaat cttgtgacaa aactcacaca tgcccaccct gcccagcacc tgaactcctg 780

gggggaccct cagtcttcct cttcccccca aaacccaagg acaccctcat gatctcccgg 840

acccctgagg tcacatgcgt ggtggtggac gtgagccacg aagaccctga ggtcaagttc 900

aactggtacg tggacggcgt ggaggtgcat aatgccaaga caaagccccg ggaggagcag 960

tacaacagca cgtaccgggt ggtcagcgtc ctcaccgtcc tgcaccagga ctggctgaat 1020

ggcaaggagt acaagtgcaa ggtctccaac aaagccctcc cagcccccat cgagaaaacc 1080

atctccaaag ccaaaggcca gccccgggaa ccacaggtgt acaccctgcc cccatcccgg 1140

gaggagatga ccaagaacca ggtcagcctg acctgcctgg tcaaaggctt ctatcccagc 1200

gacatcgccg tggagtggga gagcaatggc cagcccgaga acaactacaa gaccacccct 1260

cccgtgctgg actccgacgg ctccttcttc ctctacagca agctcaccgt ggacaagagc 1320

aggtggcagc agggcaacgt cttctcatgc tccgtgatgc atgaggctct gcacaaccac 1380

tacacccaga agagcctctc cctgtctccc ggcaaa 1416

<210> 92
<211> 472
<212> PRT
<213> Artificial Sequence

<220>
<223> amino acid sequence of h7B4_H5

<400> 92

```
Met Lys His Leu Trp Phe Phe Leu Leu Leu Val Ala Ala Pro Arg Trp
1               5                   10                  15

Val Leu Ser Glu Ile Gln Leu Gln Glu Ser Gly Pro Gly Leu Val Lys
            20                  25                  30

Pro Ser Gln Thr Leu Ser Leu Thr Cys Thr Val Ser Gly Tyr Thr Ile
        35                  40                  45

Thr Ser Gly Tyr Asp Trp Ser Trp Ile Arg Lys Pro Pro Gly Asn Lys
    50                  55                  60

Met Glu Trp Ile Ala Tyr Met Ser Tyr Arg Gly Ser Thr Asn Tyr Asn
65                  70                  75                  80

Pro Ser Leu Lys Ser Arg Val Thr Ile Ser Arg Asp Thr Ser Lys Asn
                85                  90                  95

Gln Phe Ser Leu Lys Leu Ser Ser Val Thr Ala Ala Asp Thr Ala Val
            100                 105                 110

Tyr Tyr Cys Ala Leu Thr Arg Thr Tyr Trp Tyr Asn Tyr Tyr Tyr Val
        115                 120                 125

Leu Asp Ala Trp Gly Gln Gly Thr Leu Val Thr Val Ser Ser Ala Ser
    130                 135                 140

Thr Lys Gly Pro Ser Val Phe Pro Leu Ala Pro Ser Ser Lys Ser Thr
145                 150                 155                 160

Ser Gly Gly Thr Ala Ala Leu Gly Cys Leu Val Lys Asp Tyr Phe Pro
                165                 170                 175

Glu Pro Val Thr Val Ser Trp Asn Ser Gly Ala Leu Thr Ser Gly Val
            180                 185                 190

His Thr Phe Pro Ala Val Leu Gln Ser Ser Gly Leu Tyr Ser Leu Ser
        195                 200                 205

Ser Val Val Thr Val Pro Ser Ser Ser Leu Gly Thr Gln Thr Tyr Ile
    210                 215                 220

Cys Asn Val Asn His Lys Pro Ser Asn Thr Lys Val Asp Lys Arg Val
225                 230                 235                 240
```

```
Glu Pro Lys Ser Cys Asp Lys Thr His Thr Cys Pro Pro Cys Pro Ala
                245                 250                 255

Pro Glu Leu Leu Gly Gly Pro Ser Val Phe Leu Phe Pro Pro Lys Pro
            260                 265                 270

Lys Asp Thr Leu Met Ile Ser Arg Thr Pro Glu Val Thr Cys Val Val
        275                 280                 285

Val Asp Val Ser His Glu Asp Pro Glu Val Lys Phe Asn Trp Tyr Val
    290                 295                 300

Asp Gly Val Glu Val His Asn Ala Lys Thr Lys Pro Arg Glu Glu Gln
305                 310                 315                 320

Tyr Asn Ser Thr Tyr Arg Val Val Ser Val Leu Thr Val Leu His Gln
                325                 330                 335

Asp Trp Leu Asn Gly Lys Glu Tyr Lys Cys Lys Val Ser Asn Lys Ala
            340                 345                 350

Leu Pro Ala Pro Ile Glu Lys Thr Ile Ser Lys Ala Lys Gly Gln Pro
        355                 360                 365

Arg Glu Pro Gln Val Tyr Thr Leu Pro Pro Ser Arg Glu Glu Met Thr
    370                 375                 380

Lys Asn Gln Val Ser Leu Thr Cys Leu Val Lys Gly Phe Tyr Pro Ser
385                 390                 395                 400

Asp Ile Ala Val Glu Trp Glu Ser Asn Gly Gln Pro Glu Asn Asn Tyr
                405                 410                 415

Lys Thr Thr Pro Pro Val Leu Asp Ser Asp Gly Ser Phe Phe Leu Tyr
            420                 425                 430

Ser Lys Leu Thr Val Asp Lys Ser Arg Trp Gln Gln Gly Asn Val Phe
        435                 440                 445

Ser Cys Ser Val Met His Glu Ala Leu His Asn His Tyr Thr Gln Lys
    450                 455                 460

Ser Leu Ser Leu Ser Pro Gly Lys
465                 470
```

<210> 93
<211> 28
<212> PRT

<213> Artificial Sequence

<220>
<223> polypeptide encording N-terminal sequence of cynomologous GPRC5D

<220>
<221> misc_feature
<222> (28)..(28)
<223> 28 X is biotinylated Lysine

<400> 93

Met Tyr Lys Asp Cys Ile Glu Ser Thr Gly Asp Tyr Phe Leu Pro Cys
1 5 10 15

Asp Ser Glu Gly Pro Trp Gly Ile Val Leu Glu Xaa
20 25

<210> 94
<211> 26
<212> DNA
<213> Artificial Sequence

<220>
<223> primer A used for sequence analysis of scFv

<400> 94
ctcttcgcta ttacgccagc tggcga 26

<210> 95
<211> 30
<212> DNA
<213> Artificial Sequence

<220>
<223> primer B used for sewquence analysis of scFv

<400> 95
ataacaattt cacacaggaa acagctatga 30

<210> 96
<211> 345
<212> DNA
<213> Artificial Sequence

<220>
<223> nucleotide sequence of C2037 VH

<400> 96
caggtgcagc tggtgcagtc tggggctgag gtgaagaagc ctggggcctc agtgaaggtc 60

tcctgcaagg cttctggata caccttcacc ggctactata tgcactgggt gcgacaggcc 120

cctggacaag ggcttgagtg gatgggacgg atcaacccta acagtggtgg cacaaactat 180

gcacagaagt ttcagggcag ggtcaccatg accagggaca cgtccatcag cacagcctac 240

atggagctga gcaggctgag atctgacgac acggccgtgt attactgtgc gagtgggaag 300

agtaactacc cctggggcca gggaaccctg gtcaccgtct cctca 345

<210> 97
<211> 115
<212> PRT
<213> Artificial Sequence

<220>
<223> amino acid sequence of C2037 VH

<400> 97

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Gly Tyr
20 25 30

Tyr Met His Trp Val Arg Gln Ala Pro Gly Gln Gly Leu Glu Trp Met
35 40 45

Gly Arg Ile Asn Pro Asn Ser Gly Gly Thr Asn Tyr Ala Gln Lys Phe
50 55 60

Gln Gly Arg Val Thr Met Thr Arg Asp Thr Ser Ile Ser Thr Ala Tyr
65 70 75 80

Met Glu Leu Ser Arg Leu Arg Ser Asp Asp Thr Ala Val Tyr Tyr Cys
85 90 95

Ala Ser Gly Lys Ser Asn Tyr Pro Trp Gly Gln Gly Thr Leu Val Thr
100 105 110

Val Ser Ser
115

<210> 98
<211> 330
<212> DNA
<213> Artificial Sequence

<220>
<223> nucleotide sequence of C2037 VL

<400> 98
cagcctgtgc tgactcagcc accctcagcg tctgggaccc ccgggcagag ggtcaccatc 60

tcttgttctg gaagcaactc caacatcggc agtagaactg tgaattggta ccagcacctc 120

ccaggaacgg cccccagact cctcatctat ggtaataatc agtggccctc aggggtccct 180

gaccgattct ctggctctaa gtctggctcc tcagcctccc tggccatcag tgggctccgg 240

tccgaggatg aggctgatta ttactgtgca gcatgggatg acagcctgag tggtgtggta 300

ttcggcggag ggaccaagct gaccgtccta 330

<210> 99
<211> 110
<212> PRT
<213> Artificial Sequence

<220>
<223> amino acid sequence of C2037 VL

<400> 99

Gln Pro Val Leu Thr Gln Pro Pro Ser Ala Ser Gly Thr Pro Gly Gln
1 5 10 15

Arg Val Thr Ile Ser Cys Ser Gly Ser Asn Ser Asn Ile Gly Ser Arg
20 25 30

Thr Val Asn Trp Tyr Gln His Leu Pro Gly Thr Ala Pro Arg Leu Leu
35 40 45

Ile Tyr Gly Asn Asn Gln Trp Pro Ser Gly Val Pro Asp Arg Phe Ser
50 55 60

Gly Ser Lys Ser Gly Ser Ser Ala Ser Leu Ala Ile Ser Gly Leu Arg
65 70 75 80

Ser Glu Asp Glu Ala Asp Tyr Tyr Cys Ala Ala Trp Asp Asp Ser Leu
85 90 95

Ser Gly Val Val Phe Gly Gly Gly Thr Lys Leu Thr Val Leu
100 105 110

<210> 100
<211> 369
<212> DNA
<213> Artificial Sequence

<220>
<223> nucleotide sequence of C3048 VH

<400> 100
caggtgcagc tggtgcagtc tggggctgag gtgaagaagc ctgggtcctc ggtgaaggtc 60

tcctgcaagg cttctggagg caccttcagc aggtatgcta tcagctgggt gcgacaggcc 120

cctggacaag ggcttgagtg gatgggaggg atcatcccta tctttggaac agcaaactac 180

gcacagaagt tccagggcag agtcacaatt accgcggacc aatccacgag aacagcctac 240

atggacctgg gcagactgag atctgaggac acggccgtgt attattgtgc tctcgaggga 300

ttcagggact cattaaaacg aaatcctttt gatatctggg gccaagggac aatggtcacc 360

gtctcttca 369

<210> 101
<211> 123
<212> PRT
<213> Artificial Sequence

<220>
<223> amino acid sequence of C3048 VH

<400> 101

Gln Val Gln Leu Val Gln Ser Gly Ala Glu Val Lys Lys Pro Gly Ser
1 5 10 15

Ser Val Lys Val Ser Cys Lys Ala Ser Gly Gly Thr Phe Ser Arg Tyr
20 25 30

Ala Ile Ser Trp Val Arg Gln Ala Pro Gly Gln Gly Leu Glu Trp Met
35 40 45

Gly Gly Ile Ile Pro Ile Phe Gly Thr Ala Asn Tyr Ala Gln Lys Phe
50 55 60

Gln Gly Arg Val Thr Ile Thr Ala Asp Gln Ser Thr Arg Thr Ala Tyr
65 70 75 80

Met Asp Leu Gly Arg Leu Arg Ser Glu Asp Thr Ala Val Tyr Tyr Cys
85 90 95

Ala Leu Glu Gly Phe Arg Asp Ser Leu Lys Arg Asn Pro Phe Asp Ile
100 105 110

Trp Gly Gln Gly Thr Met Val Thr Val Ser Ser
115 120

<210> 102
<211> 330
<212> DNA
<213> Artificial Sequence

<220>
<223> nucleotide sequence of C3048 VL

<400> 102
cagtctgtcg tgacgcagcc gccctcagtg tctgcggccc caggacagaa ggtcaccatc 60

tcctgctctg gaggcaccac caacattggg agtaattatg tatcctggta tcagcagctc 120

ccaggaacag cccccaaatt cctcatgtat gaaaataata agcgaccctc aggcattcct 180

gaccgattct ctggctccaa gtctggcacg tcagccaccc tggccatcac cggactccag 240

actggggacg aggccactta ttactgctca acatgggata gcagcctgaa tactgggcta 300

ttcggcggag ggaccaagct gaccgtccta 330

<210> 103
<211> 110
<212> PRT
<213> Artificial Sequence

<220>
<223> amino acid sequence of C3048 VL

<400> 103

Gln Ser Val Val Thr Gln Pro Pro Ser Val Ser Ala Ala Pro Gly Gln
1 5 10 15

Lys Val Thr Ile Ser Cys Ser Gly Gly Thr Thr Asn Ile Gly Ser Asn
20 25 30

Tyr Val Ser Trp Tyr Gln Gln Leu Pro Gly Thr Ala Pro Lys Phe Leu
35 40 45

Met Tyr Glu Asn Asn Lys Arg Pro Ser Gly Ile Pro Asp Arg Phe Ser
50 55 60

Gly Ser Lys Ser Gly Thr Ser Ala Thr Leu Ala Ile Thr Gly Leu Gln
65 70 75 80

Thr Gly Asp Glu Ala Thr Tyr Tyr Cys Ser Thr Trp Asp Ser Ser Leu
85 90 95

Asn Thr Gly Leu Phe Gly Gly Gly Thr Lys Leu Thr Val Leu
100 105 110

<210> 104
<211> 363
<212> DNA
<213> Artificial Sequence

<220>
<223> nucleotide sequence of C3015 VH

<400> 104
gaggtgcagc tggtggagtc cgggggaggc gtggtccagc ctgggaggtc cctgagactc 60

tcctgtgcag cctctggatt cacctttagg aactatgcca tgagctgggt ccgccaggct 120

ccagggaagg ggctggagtg ggtctcaggt attagtggta ctggtggtag cacatactac 180

gcagactccg tgaagggccg gttcaccatc tccagagaca attccaagga cacactatat 240

ctgcaattga acagcctgag agccgaggac acggccgtat attactgtgc gaaatcccgt 300

agtatgagta tcaactacgg tatggacgtc tggggccaag ggaccacggt caccgtctcc 360

tca 363

<210> 105
<211> 121
<212> PRT
<213> Artificial Sequence

<220>
<223> amino acid sequence of C3015 VH

<400> 105

Glu Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Arg Asn Tyr
            20                  25                  30

Ala Met Ser Trp Val Arg Gln Ala Pro Gly Lys Gly Leu Glu Trp Val
        35                  40                  45

Ser Gly Ile Ser Gly Thr Gly Gly Ser Thr Tyr Tyr Ala Asp Ser Val
    50                  55                  60

Lys Gly Arg Phe Thr Ile Ser Arg Asp Asn Ser Lys Asp Thr Leu Tyr
65                  70                  75                  80

Leu Gln Leu Asn Ser Leu Arg Ala Glu Asp Thr Ala Val Tyr Tyr Cys
                85                  90                  95

Ala Lys Ser Arg Ser Met Ser Ile Asn Tyr Gly Met Asp Val Trp Gly
            100                 105                 110

Gln Gly Thr Thr Val Thr Val Ser Ser
        115                 120

<210> 106
<211> 318
<212> DNA
<213> Artificial Sequence

<220>
<223> nucleotide sequence of C3015 VL

<400> 106
tcctatgagc tgactcagcc accctcagtg tccgtgtccc caggacagac agtcagcatc 60

acctgctctg gagataattt gggtgataga tatgcctcct ggtatcagca gaagccaggc 120

cagtcccctg tgcaggtcat ctatcaagat accaagcggc cctcagggat ccctgagcga 180

ttctctggct ccaactctgg gaacacagcc actctgacca tcagcgggac ccaggctatg 240

gatgaggctg actattactg tcaggcgtgg gacagcaaca ctgtggtatt cggcggaggg 300

accaagctga ccgtccta 318

<210> 107
<211> 106
<212> PRT
<213> Artificial Sequence

<220>
<223> amino acid sequence of C3015 VL

<400> 107

Ser Tyr Glu Leu Thr Gln Pro Pro Ser Val Ser Val Ser Pro Gly Gln
1 5 10 15

Thr Val Ser Ile Thr Cys Ser Gly Asp Asn Leu Gly Asp Arg Tyr Ala
20 25 30

Ser Trp Tyr Gln Gln Lys Pro Gly Gln Ser Pro Val Gln Val Ile Tyr
35 40 45

Gln Asp Thr Lys Arg Pro Ser Gly Ile Pro Glu Arg Phe Ser Gly Ser
50 55 60

Asn Ser Gly Asn Thr Ala Thr Leu Thr Ile Ser Gly Thr Gln Ala Met
65 70 75 80

Asp Glu Ala Asp Tyr Tyr Cys Gln Ala Trp Asp Ser Asn Thr Val Val
85 90 95

Phe Gly Gly Gly Thr Lys Leu Thr Val Leu
100 105

<210> 108
<211> 345
<212> DNA
<213> Artificial Sequence

<220>
<223> nucleotide sequence of C3022 VH

<400> 108
caggtgcagc tggtgcagtc tggggctgag gtgaagaagc ctggggcctc agtgaaggtc 60

tcctgcaagg cttctggata caccttcacc ggctactata tgcactgggt gcgacaggcc 120

cctggacaag ggcttgagtg gatgggacgg atcaacccta acagtggtgg cacaaactat 180

gcacagaagt ttcagggcag ggtcaccatg accagggaca cgtccatcag cacagcctac 240

atggagctga gcgggctgag atctgacgac acggccgttt attactgtgc gagtggtagt 300

gtaaggcatc cctggggcca gggaaccctg gtcaccgtct cctca 345

<210> 109
<211> 115
<212> PRT
<213> Artificial Sequence

<220>
<223> amino acid sequence of C3022 VH

<400> 109

```
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Tyr Met His Trp Val Arg Gln Ala Pro Gly Gln Gly Leu Glu Trp Met
        35                  40                  45

Gly Arg Ile Asn Pro Asn Ser Gly Gly Thr Asn Tyr Ala Gln Lys Phe
    50                  55                  60

Gln Gly Arg Val Thr Met Thr Arg Asp Thr Ser Ile Ser Thr Ala Tyr
65                  70                  75                  80

Met Glu Leu Ser Gly Leu Arg Ser Asp Asp Thr Ala Val Tyr Tyr Cys
                85                  90                  95

Ala Ser Gly Ser Val Arg His Pro Trp Gly Gln Gly Thr Leu Val Thr
            100                 105                 110

Val Ser Ser
        115
```

<210> 110
<211> 330
<212> DNA
<213> Artificial Sequence

<220>
<223> nucleotide sequence of C3022 VL

<400> 110

```
cagcctgtgc tgactcagcc accctcagcg tctgggaccc ccgggcagag ggtcaccatc      60

tcttgttctg gaagcaggtc caacatcgga ggtaatattg taagttggta ccagcagttc     120

ccaggaacgg cccccagact cctcacttat gctgataatc agcggccctc aggggtccct     180

gaccgattct ctggctccaa gtctggcacc tcagcctccc tggccatcag tggcctccag     240

tctgaggatg aggctgacta ttattgtgca gcatgggatg acagcctgaa tggtgttgtg     300
```

ttcggaggag gcaccaagct gaccgtccta 330

<210> 111
<211> 8
<212> PRT
<213> Artificial Sequence

<220>
<223> amino acid sequence of C2037 H CDR1

<400> 111

Gly Tyr Thr Phe Thr Gly Tyr Tyr
1 5

<210> 112
<211> 8
<212> PRT
<213> Artificial Sequence

<220>
<223> amino acid sequence of C2037 H CDR2

<400> 112

Ile Asn Pro Asn Ser Gly Gly Thr
1 5

<210> 113
<211> 8
<212> PRT
<213> Artificial Sequence

<220>
<223> amino acid sequence of C2037 H CDR3

<400> 113

Ala Ser Gly Lys Ser Asn Tyr Pro
1 5

<210> 114
<211> 8
<212> PRT
<213> Artificial Sequence

<220>
<223> amino acid sequence of C2037 L CDR1

<400> 114

Asn Ser Asn Ile Gly Ser Arg Thr
1 5

<210> 115
<211> 3
<212> PRT
<213> Artificial Sequence

<220>
<223> amino acid sequence of C2037 L CDR2

<400> 115

Gly Asn Asn
1

<210> 116
<211> 11
<212> PRT
<213> Artificial Sequence

<220>
<223> amino acid sequence of C2037 L CDR3

<400> 116

Ala Ala Trp Asp Asp Ser Leu Ser Gly Val Val
1 5 10

<210> 117
<211> 8
<212> PRT
<213> Artificial Sequence

<220>
<223> amino acid sequence of C3048 H CDR1

<400> 117

Gly Gly Thr Phe Ser Arg Tyr Ala
1 5

<210> 118
<211> 8
<212> PRT
<213> Artificial Sequence

<220>
<223> amino acid sequence of C3048 H CDR2

<400> 118

Ile Ile Pro Ile Phe Gly Thr Ala
1 5

<210> 119
<211> 16
<212> PRT
<213> Artificial Sequence

<220>
<223> amino acid sequence of C3048 H CDR3

<400> 119

Ala Leu Glu Gly Phe Arg Asp Ser Leu Lys Arg Asn Pro Phe Asp Ile

1 5 10 15

<210> 120
<211> 8
<212> PRT
<213> Artificial Sequence

<220>
<223> amino acid sequence of C3048 L CDR1

<400> 120

Thr Thr Asn Ile Gly Ser Asn Tyr
1 5

<210> 121
<211> 3
<212> PRT
<213> Artificial Sequence

<220>
<223> amino acid sequence of C3048 L CDR2

<400> 121

Glu Asn Asn
1

<210> 122
<211> 11
<212> PRT
<213> Artificial Sequence

<220>
<223> amino acid sequence of C3048 L CDR3

<400> 122

Ser Thr Trp Asp Ser Ser Leu Asn Thr Gly Leu
1 5 10

<210> 123
<211> 8
<212> PRT
<213> Artificial Sequence

<220>
<223> amino acid sequence of C3015 H CDR1

<400> 123

Gly Phe Thr Phe Arg Asn Tyr Ala
1 5

<210> 124
<211> 8
<212> PRT
<213> Artificial Sequence

<220>
<223>  amino acid sequence of C3015 H CDR2

<400>  124

Ile Ser Gly Thr Gly Gly Ser Thr
1               5

<210>  125
<211>  14
<212>  PRT
<213>  Artificial Sequence

<220>
<223>  amino acid sequence of C3015 H CDR3

<400>  125

Ala Lys Ser Arg Ser Met Ser Ile Asn Tyr Gly Met Asp Val
1               5                   10

<210>  126
<211>  6
<212>  PRT
<213>  Artificial Sequence

<220>
<223>  amino acid sequence of C3015 L CDR1

<400>  126

Asn Leu Gly Asp Arg Tyr
1               5

<210>  127
<211>  3
<212>  PRT
<213>  Artificial Sequence

<220>
<223>  amino acid sequence of C3015 L CDR2

<400>  127

Gln Asp Thr
1

<210>  128
<211>  9
<212>  PRT
<213>  Artificial Sequence

<220>
<223>  amino acid sequence of C3015 L CDR3

<400>  128

Gln Ala Trp Asp Ser Asn Thr Val Val

1 5

<210> 129
<211> 8
<212> PRT
<213> Artificial Sequence

<220>
<223> amino acid sequence of C3022 H CDR1

<400> 129

Gly Tyr Thr Phe Thr Gly Tyr Tyr
1 5

<210> 130
<211> 8
<212> PRT
<213> Artificial Sequence

<220>
<223> amino acid sequence of C3022 H CDR2

<400> 130

Ile Asn Pro Asn Ser Gly Gly Thr
1 5

<210> 131
<211> 8
<212> PRT
<213> Artificial Sequence

<220>
<223> amino acid sequence of C3022 H CDR3

<400> 131

Ala Ser Gly Ser Val Arg His Pro
1 5

<210> 132
<211> 8
<212> PRT
<213> Artificial Sequence

<220>
<223> amino acid sequence of C3022 L CDR1

<400> 132

Arg Ser Asn Ile Gly Gly Asn Ile
1 5

<210> 133
<211> 3
<212> PRT
<213> Artificial Sequence

<220>
<223> amino acid sequence of C3022 L CDR2

<400> 133

Ala Asp Asn
1

<210> 134
<211> 11
<212> PRT
<213> Artificial Sequence

<220>
<223> amino acid sequence of C3022 L CDR3

<400> 134

```
Ala Ala Trp Asp Asp Ser Leu Asn Gly Val Val
1               5                   10
```

<210> 135
<211> 110
<212> PRT
<213> Artificial Sequence

<220>
<223> amino acid sequence of C3022 VL

<400> 135

```
Gln Pro Val Leu Thr Gln Pro Pro Ser Ala Ser Gly Thr Pro Gly Gln
1               5                   10                  15

Arg Val Thr Ile Ser Cys Ser Gly Ser Arg Ser Asn Ile Gly Gly Asn
            20                  25                  30

Ile Val Ser Trp Tyr Gln Gln Phe Pro Gly Thr Ala Pro Arg Leu Leu
        35                  40                  45

Thr Tyr Ala Asp Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Tyr Cys Ala Ala Trp Asp Asp Ser Leu
                85                  90                  95

Asn Gly Val Val Phe Gly Gly Gly Thr Lys Leu Thr Val Leu
            100                 105                 110
```

<210> 136

<211> 1392
<212> DNA
<213> Artificial Sequence

<220>
<223> nucleotide sequence of C2037 H in IgG form

<400> 136
atgaaacacc tgtggttctt cctcctgctg gtggcagctc ccagatgggt gctgagccag 60

gtgcagctgg tgcagtctgg ggctgaggtg aagaagcctg gggcctcagt gaaggtctcc 120

tgcaaggctt ctggatacac cttcaccggc tactatatgc actgggtgcg acaggcccct 180

ggacaagggc ttgagtggat gggacggatc aaccctaaca gtggtggcac aaactatgca 240

cagaagtttc agggcagggt caccatgacc agggacacgt ccatcagcac agcctacatg 300

gagctgagca ggctgagatc tgacgacacg gccgtgtatt actgtgcgag tgggaagagt 360

aactacccct ggggccaggg aaccctggtc accgtcagct cagcctccac caagggccca 420

agcgtcttcc ccctggcacc ctcctccaag agcacctctg gcggcacagc cgccctgggc 480

tgcctggtca aggactactt ccccgaaccc gtgaccgtga gctggaactc aggcgccctg 540

accagcggcg tgcacacctt ccccgctgtc ctgcagtcct caggactcta ctccctcagc 600

agcgtggtga ccgtgccctc cagcagcttg ggcacccaga cctacatctg caacgtgaat 660

cacaagccca gcaacaccaa ggtggacaag agagttgagc ccaaatcttg tgacaaaact 720

cacacatgcc caccctgccc agcacctgaa ctcctggggg gaccctcagt cttcctcttc 780

cccccaaaac ccaaggacac cctcatgatc tcccggaccc ctgaggtcac atgcgtggtg 840

gtggacgtga gccacgaaga ccctgaggtc aagttcaact ggtacgtgga cggcgtggag 900

gtgcataatg ccaagacaaa gccccgggag gagcagtaca acagcacgta ccgggtggtc 960

agcgtcctca ccgtcctgca ccaggactgg ctgaatggca aggagtacaa gtgcaaggtc 1020

tccaacaaag ccctcccagc ccccatcgag aaaaccatct ccaaagccaa aggccagccc 1080

cgggaaccac aggtgtacac cctgccccca tcccgggagg agatgaccaa gaaccaggtc 1140

agcctgacct gcctggtcaa aggcttctat cccagcgaca tcgccgtgga gtgggagagc 1200

aatggccagc ccgagaacaa ctacaagacc acccctcccg tgctggactc cgacggctcc 1260

ttcttcctct acagcaagct caccgtggac aagagcaggt ggcagcaggg caacgtcttc 1320

tcatgctccg tgatgcatga ggctctgcac aaccactaca cccagaagag cctctccctg 1380

tctcccggca aa 1392

<210> 137
<211> 708
<212> DNA
<213> Artificial Sequence

<220>

<223> nucleotide sequence of C2037 L in IgG form

<400> 137
atggtgctgc agacccaggt gttcatctcc ctgctgctgt ggatctccgg cgcgtacggc 60

cagcctgtgc tgactcagcc accctcagcg tctgggaccc ccgggcagag ggtcaccatc 120

tcttgttctg gaagcaactc caacatcggc agtagaactg tgaattggta ccagcacctc 180

ccaggaacgg cccccagact cctcatctat ggtaataatc agtggccctc aggggtccct 240

gaccgattct ctggctctaa gtctggctcc tcagcctccc tggccatcag tgggctccgg 300

tccgaggatg aggctgatta ttactgtgca gcatgggatg acagcctgag tggtgtggta 360

ttcggcggag ggaccaagtt aactgtgctt ggccagccta aggctgcccc tagcgtgacc 420

ctgttccctc cttccagcga ggagcttcaa gctaacaagg ccaccctggt gtgtcttatc 480

tctgacttct accctggcgc tgtgaccgtg gcctggaagg ctgacagctc ccctgtgaag 540

gccggagtgg agaccaccac acctagcaag cagtctaaca acaagtacgc tgccagctcc 600

tacctgagcc ttacccctga gcagtggaag tctcacagaa gctactcctg tcaagtgacc 660

cacgagggca gcaccgtgga gaagaccgtg gctcctaccg agtgttcc 708

<210> 138
<211> 1416
<212> DNA
<213> Artificial Sequence

<220>
<223> nucleotide sequence of C3048 H in IgG form

<400> 138
atgaaacacc tgtggttctt cctcctgctg gtggcagctc ccagatgggt gctgagccag 60

gtgcagctgg tgcagtctgg ggctgaggtg aagaagcctg ggtcctcggt gaaggtctcc 120

tgcaaggctt ctggaggcac cttcagcagg tatgctatca gctgggtgcg acaggcccct 180

ggacaagggc ttgagtggat gggagggatc atccctatct ttggaacagc aaactacgca 240

cagaagttcc agggcagagt cacaattacc gcggaccaat ccacgagaac agcctacatg 300

gacctgggca gactgagatc tgaggacacg gccgtgtatt attgtgctct cgagggattc 360

agggactcat taaaacgaaa tccttttgat atctggggcc aagggacaat ggtcaccgtc 420

agctcagcct ccaccaaggg cccaagcgtc ttccccctgg caccctcctc caagagcacc 480

tctggcggca cagccgccct gggctgcctg gtcaaggact acttccccga acccgtgacc 540

gtgagctgga actcaggcgc cctgaccagc ggcgtgcaca ccttccccgc tgtcctgcag 600

tcctcaggac tctactccct cagcagcgtg gtgaccgtgc cctccagcag cttgggcacc 660

cagacctaca tctgcaacgt gaatcacaag cccagcaaca ccaaggtgga caagagagtt 720

gagcccaaat cttgtgacaa aactcacaca tgcccaccct gcccagcacc tgaactcctg 780

gggggaccct cagtcttcct cttcccccca aaacccaagg acaccctcat gatctcccgg 840

acccctgagg tcacatgcgt ggtggtggac gtgagccacg aagaccctga ggtcaagttc 900

aactggtacg tggacggcgt ggaggtgcat aatgccaaga caaagccccg ggaggagcag 960

tacaacagca cgtaccgggt ggtcagcgtc ctcaccgtcc tgcaccagga ctggctgaat 1020

ggcaaggagt acaagtgcaa ggtctccaac aaagccctcc cagcccccat cgagaaaacc 1080

atctccaaag ccaaaggcca gccccgggaa ccacaggtgt acaccctgcc cccatcccgg 1140

gaggagatga ccaagaacca ggtcagcctg acctgcctgg tcaaaggctt ctatcccagc 1200

gacatcgccg tggagtggga gagcaatggc cagcccgaga acaactacaa gaccacccct 1260

cccgtgctgg actccgacgg ctccttcttc ctctacagca agctcaccgt ggacaagagc 1320

aggtggcagc agggcaacgt cttctcatgc tccgtgatgc atgaggctct gcacaaccac 1380

tacacccaga agagcctctc cctgtctccc ggcaaa 1416

<210> 139
<211> 708
<212> DNA
<213> Artificial Sequence

<220>
<223> nucleotide sequence of C3048 L in IgG form

<400> 139
atggtgctgc agacccaggt gttcatctcc ctgctgctgt ggatctccgg cgcgtacggc 60

cagtctgtcg tgacgcagcc gccctcagtg tctgcggccc caggacagaa ggtcaccatc 120

tcctgctctg gaggcaccac caacattggg agtaattatg tatcctggta tcagcagctc 180

ccaggaacag cccccaaatt cctcatgtat gaaaataata agcgaccctc aggcattcct 240

gaccgattct ctggctccaa gtctggcacg tcagccaccc tggccatcac cggactccag 300

actggggacg aggccactta ttactgctca acatgggata gcagcctgaa tactgggcta 360

ttcggcggag ggaccaagtt aactgtgctt ggccagccta aggctgcccc tagcgtgacc 420

ctgttccctc cttccagcga ggagcttcaa gctaacaagg ccaccctggt gtgtcttatc 480

tctgacttct accctggcgc tgtgaccgtg gcctggaagg ctgacagctc ccctgtgaag 540

gccggagtgg agaccaccac acctagcaag cagtctaaca acaagtacgc tgccagctcc 600

tacctgagcc ttacccctga gcagtggaag tctcacagaa gctactcctg tcaagtgacc 660

cacgagggca gcaccgtgga gaagaccgtg gctcctaccg agtgttcc 708

<210> 140
<211> 1410
<212> DNA
<213> Artificial Sequence

<220>

<223>  nucleotide sequence of C3015 H in IgG form

<400>  140
atgaaacacc tgtggttctt cctcctgctg gtggcagctc ccagatgggt gctgagcgag        60

gtgcagctgg tggagtccgg gggaggcgtg gtccagcctg ggaggtccct gagactctcc       120

tgtgcagcct ctggattcac ctttaggaac tatgccatga gctgggtccg ccaggctcca       180

gggaaggggc tggagtgggt ctcaggtatt agtggtactg gtggtagcac atactacgca       240

gactccgtga agggccggtt caccatctcc agagacaatt ccaaggacac actatatctg       300

caattgaaca gcctgagagc cgaggacacg gccgtatatt actgtgcgaa atcccgtagt       360

atgagtatca actacggtat ggacgtctgg ggccaaggga ccacggtcac cgtcagctca       420

gcctccacca agggcccaag cgtcttcccc ctggcaccct cctccaagag cacctctggc       480

ggcacagccg ccctgggctg cctggtcaag gactacttcc ccgaacccgt gaccgtgagc       540

tggaactcag gcgccctgac cagcggcgtg cacaccttcc ccgctgtcct gcagtcctca       600

ggactctact ccctcagcag cgtggtgacc gtgccctcca gcagcttggg cacccagacc       660

tacatctgca acgtgaatca caagcccagc aacaccaagg tggacaagag agttgagccc       720

aaatcttgtg acaaaactca cacatgccca ccctgcccag cacctgaact cctgggggga       780

ccctcagtct tcctcttccc cccaaaaccc aaggacaccc tcatgatctc ccggacccct       840

gaggtcacat gcgtggtggt ggacgtgagc cacgaagacc ctgaggtcaa gttcaactgg       900

tacgtggacg gcgtggaggt gcataatgcc aagacaaagc cccgggagga gcagtacaac       960

agcacgtacc gggtggtcag cgtcctcacc gtcctgcacc aggactggct gaatggcaag      1020

gagtacaagt gcaaggtctc caacaaagcc ctcccagccc ccatcgagaa aaccatctcc      1080

aaagccaaag gccagccccg ggaaccacag gtgtacaccc tgcccccatc ccgggaggag      1140

atgaccaaga accaggtcag cctgacctgc ctggtcaaag gcttctatcc cagcgacatc      1200

gccgtggagt gggagagcaa tggccagccc gagaacaact acaagaccac ccctcccgtg      1260

ctggactccg acggctcctt cttcctctac agcaagctca ccgtggacaa gagcaggtgg      1320

cagcagggca acgtcttctc atgctccgtg atgcatgagg ctctgcacaa ccactacacc      1380

cagaagagcc tctccctgtc tcccggcaaa                                       1410

<210>  141
<211>  696
<212>  DNA
<213>  Artificial Sequence

<220>
<223>  nucleotide sequence of C3015 L in IgG form

<400>  141
atggtgctgc agacccaggt gttcatctcc ctgctgctgt ggatctccgg cgcgtacggc        60

tcctatgagc tgactcagcc accctcagtg tccgtgtccc caggacagac agtcagcatc 120

acctgctctg gagataattt gggtgataga tatgcctcct ggtatcagca gaagccaggc 180

cagtcccctg tgcaggtcat ctatcaagat accaagcggc cctcagggat ccctgagcga 240

ttctctggct ccaactctgg gaacacagcc actctgacca tcagcgggac ccaggctatg 300

gatgaggctg actattactg tcaggcgtgg gacagcaaca ctgtggtatt cggcggaggg 360

accaagttaa ctgtgcttgg ccagcctaag gctgccccta gcgtgaccct gttccctcct 420

tccagcgagg agcttcaagc taacaaggcc accctggtgt gtcttatctc tgacttctac 480

cctggcgctg tgaccgtggc ctggaaggct gacagctccc ctgtgaaggc cggagtggag 540

accaccacac ctagcaagca gtctaacaac aagtacgctg ccagctccta cctgagcctt 600

acccctgagc agtggaagtc tcacagaagc tactcctgtc aagtgaccca cgagggcagc 660

accgtggaga agaccgtggc tcctaccgag tgttcc 696

<210> 142
<211> 1392
<212> DNA
<213> Artificial Sequence

<220>
<223> nucleotide sequence of C3022 H in IgG form

<400> 142
atgaaacacc tgtggttctt cctcctgctg gtggcagctc ccagatgggt gctgagccag 60

gtgcagctgg tgcagtctgg ggctgaggtg aagaagcctg gggcctcagt gaaggtctcc 120

tgcaaggctt ctggatacac cttcaccggc tactatatgc actgggtgcg acaggcccct 180

ggacaagggc ttgagtggat gggacggatc aaccctaaca gtggtggcac aaactatgca 240

cagaagtttc agggcagggt caccatgacc agggacacgt ccatcagcac agcctacatg 300

gagctgagcg ggctgagatc tgacgacacg gccgtttatt actgtgcgag tggtagtgta 360

aggcatccct ggggccaggg aaccctggtc accgtcagct cagcctccac caagggccca 420

agcgtcttcc ccctggcacc ctcctccaag agcacctctg gcggcacagc cgccctgggc 480

tgcctggtca aggactactt ccccgaaccc gtgaccgtga gctggaactc aggcgccctg 540

accagcggcg tgcacacctt ccccgctgtc ctgcagtcct caggactcta ctccctcagc 600

agcgtggtga ccgtgccctc cagcagcttg ggcacccaga cctacatctg caacgtgaat 660

cacaagccca gcaacaccaa ggtggacaag agagttgagc ccaaatcttg tgacaaaact 720

cacacatgcc caccctgccc agcacctgaa ctcctggggg gaccctcagt cttcctcttc 780

cccccaaaac ccaaggacac cctcatgatc tcccggaccc ctgaggtcac atgcgtggtg 840

gtggacgtga gccacgaaga ccctgaggtc aagttcaact ggtacgtgga cggcgtggag 900

gtgcataatg ccaagacaaa gccccgggag gagcagtaca acagcacgta ccgggtggtc 960

agcgtcctca ccgtcctgca ccaggactgg ctgaatggca aggagtacaa gtgcaaggtc 1020

tccaacaaag ccctcccagc ccccatcgag aaaaccatct ccaaagccaa aggccagccc 1080

cgggaaccac aggtgtacac cctgccccca tcccgggagg agatgaccaa gaaccaggtc 1140

agcctgacct gcctggtcaa aggcttctat cccagcgaca tcgccgtgga gtgggagagc 1200

aatggccagc ccgagaacaa ctacaagacc acgcctcccg tgctggactc cgacggctcc 1260

ttcttcctct acagcaagct caccgtggac aagagcaggt ggcagcaggg caacgtcttc 1320

tcatgctccg tgatgcatga ggctctgcac aaccactaca cccagaagag cctctccctg 1380

tctcccggca aa 1392

<210> 143
<211> 708
<212> DNA
<213> Artificial Sequence

<220>
<223> nucleotide sequence of C3022 L in IgG form

<400> 143
atggtgctgc agacccaggt gttcatctcc ctgctgctgt ggatctccgg cgcgtacggc 60

cagcctgtgc tgactcagcc accctcagcg tctgggaccc ccgggcagag ggtcaccatc 120

tcttgttctg gaagcaggtc caacatcgga ggtaatattg taagttggta ccagcagttc 180

ccaggaacgg cccccagact cctcacttat gctgataatc agcggccctc aggggtccct 240

gaccgattct ctggctccaa gtctggcacc tcagcctccc tggccatcag tggcctccag 300

tctgaggatg aggctgacta ttattgtgca gcatgggatg acagcctgaa tggtgttgtg 360

ttcggaggag gcaccaagtt aactgtgctt ggccagccta aggctgcccc tagcgtgacc 420

ctgttccctc cttccagcga ggagcttcaa gctaacaagg ccaccctggt gtgtcttatc 480

tctgacttct accctggcgc tgtgaccgtg gcctggaagg ctgacagctc ccctgtgaag 540

gccggagtgg agaccaccac acctagcaag cagtctaaca acaagtacgc tgccagctcc 600

tacctgagcc ttacccctga gcagtggaag tctcacagaa gctactcctg tcaagtgacc 660

cacgagggca gcaccgtgga gaagaccgtg gctcctaccg agtgttcc 708

<210> 144
<211> 464
<212> PRT
<213> Artificial Sequence

<220>
<223> amino acid sequence of C2037 H in IgG form

<400> 144

Met Lys His Leu Trp Phe Phe Leu Leu Leu Val Ala Ala Pro Arg Trp

1 5 10 15

Val Leu Ser Gln Val Gln Leu Val Gln Ser Gly Ala Glu Val Lys Lys
20 25 30

Pro Gly Ala Ser Val Lys Val Ser Cys Lys Ala Ser Gly Tyr Thr Phe
35 40 45

Thr Gly Tyr Tyr Met His Trp Val Arg Gln Ala Pro Gly Gln Gly Leu
50 55 60

Glu Trp Met Gly Arg Ile Asn Pro Asn Ser Gly Gly Thr Asn Tyr Ala
65 70 75 80

Gln Lys Phe Gln Gly Arg Val Thr Met Thr Arg Asp Thr Ser Ile Ser
85 90 95

Thr Ala Tyr Met Glu Leu Ser Arg Leu Arg Ser Asp Asp Thr Ala Val
100 105 110

Tyr Tyr Cys Ala Ser Gly Lys Ser Asn Tyr Pro Trp Gly Gln Gly Thr
115 120 125

Leu Val Thr Val Ser Ser Ala Ser Thr Lys Gly Pro Ser Val Phe Pro
130 135 140

Leu Ala Pro Ser Ser Lys Ser Thr Ser Gly Gly Thr Ala Ala Leu Gly
145 150 155 160

Cys Leu Val Lys Asp Tyr Phe Pro Glu Pro Val Thr Val Ser Trp Asn
165 170 175

Ser Gly Ala Leu Thr Ser Gly Val His Thr Phe Pro Ala Val Leu Gln
180 185 190

Ser Ser Gly Leu Tyr Ser Leu Ser Ser Val Val Thr Val Pro Ser Ser
195 200 205

Ser Leu Gly Thr Gln Thr Tyr Ile Cys Asn Val Asn His Lys Pro Ser
210 215 220

Asn Thr Lys Val Asp Lys Arg Val Glu Pro Lys Ser Cys Asp Lys Thr
225 230 235 240

His Thr Cys Pro Pro Cys Pro Ala Pro Glu Leu Leu Gly Gly Pro Ser
245 250 255

Val Phe Leu Phe Pro Pro Lys Pro Lys Asp Thr Leu Met Ile Ser Arg
260 265 270

Thr Pro Glu Val Thr Cys Val Val Val Asp Val Ser His Glu Asp Pro
275 280 285

Glu Val Lys Phe Asn Trp Tyr Val Asp Gly Val Glu Val His Asn Ala
290 295 300

Lys Thr Lys Pro Arg Glu Glu Gln Tyr Asn Ser Thr Tyr Arg Val Val
305 310 315 320

Ser Val Leu Thr Val Leu His Gln Asp Trp Leu Asn Gly Lys Glu Tyr
325 330 335

Lys Cys Lys Val Ser Asn Lys Ala Leu Pro Ala Pro Ile Glu Lys Thr
340 345 350

Ile Ser Lys Ala Lys Gly Gln Pro Arg Glu Pro Gln Val Tyr Thr Leu
355 360 365

Pro Pro Ser Arg Glu Glu Met Thr Lys Asn Gln Val Ser Leu Thr Cys
370 375 380

Leu Val Lys Gly Phe Tyr Pro Ser Asp Ile Ala Val Glu Trp Glu Ser
385 390 395 400

Asn Gly Gln Pro Glu Asn Asn Tyr Lys Thr Thr Pro Pro Val Leu Asp
405 410 415

Ser Asp Gly Ser Phe Phe Leu Tyr Ser Lys Leu Thr Val Asp Lys Ser
420 425 430

Arg Trp Gln Gln Gly Asn Val Phe Ser Cys Ser Val Met His Glu Ala
435 440 445

Leu His Asn His Tyr Thr Gln Lys Ser Leu Ser Leu Ser Pro Gly Lys
450 455 460

<210> 145
<211> 236
<212> PRT
<213> Artificial Sequence

<220>
<223> amino acid sequence of C2037 L in IgG form

<400> 145

Met Val Leu Gln Thr Gln Val Phe Ile Ser Leu Leu Leu Trp Ile Ser

```
1               5                   10                  15

Gly Ala Tyr Gly Gln Pro Val Leu Thr Gln Pro Pro Ser Ala Ser Gly
            20                  25                  30

Thr Pro Gly Gln Arg Val Thr Ile Ser Cys Ser Gly Ser Asn Ser Asn
        35                  40                  45

Ile Gly Ser Arg Thr Val Asn Trp Tyr Gln His Leu Pro Gly Thr Ala
    50                  55                  60

Pro Arg Leu Leu Ile Tyr Gly Asn Asn Gln Trp Pro Ser Gly Val Pro
65                  70                  75                  80

Asp Arg Phe Ser Gly Ser Lys Ser Gly Ser Ser Ala Ser Leu Ala Ile
                85                  90                  95

Ser Gly Leu Arg Ser Glu Asp Glu Ala Asp Tyr Tyr Cys Ala Ala Trp
            100                 105                 110

Asp Asp Ser Leu Ser Gly Val Val Phe Gly Gly Gly Thr Lys Leu Thr
        115                 120                 125

Val Leu Gly Gln Pro Lys Ala Ala Pro Ser Val Thr Leu Phe Pro Pro
    130                 135                 140

Ser Ser Glu Glu Leu Gln Ala Asn Lys Ala Thr Leu Val Cys Leu Ile
145                 150                 155                 160

Ser Asp Phe Tyr Pro Gly Ala Val Thr Val Ala Trp Lys Ala Asp Ser
                165                 170                 175

Ser Pro Val Lys Ala Gly Val Glu Thr Thr Thr Pro Ser Lys Gln Ser
            180                 185                 190

Asn Asn Lys Tyr Ala Ala Ser Ser Tyr Leu Ser Leu Thr Pro Glu Gln
        195                 200                 205

Trp Lys Ser His Arg Ser Tyr Ser Cys Gln Val Thr His Glu Gly Ser
    210                 215                 220

Thr Val Glu Lys Thr Val Ala Pro Thr Glu Cys Ser
225                 230                 235
```

<210>  146
<211>  472
<212>  PRT
<213>  Artificial Sequence

<220>
<223>  amino acid sequence of C3048 H in IgG form

<400>  146

Met Lys His Leu Trp Phe Phe Leu Leu Leu Val Ala Ala Pro Arg Trp
1               5                   10                  15

Val Leu Ser Gln Val Gln Leu Val Gln Ser Gly Ala Glu Val Lys Lys
            20                  25                  30

Pro Gly Ser Ser Val Lys Val Ser Cys Lys Ala Ser Gly Gly Thr Phe
        35                  40                  45

Ser Arg Tyr Ala Ile Ser Trp Val Arg Gln Ala Pro Gly Gln Gly Leu
    50                  55                  60

Glu Trp Met Gly Gly Ile Ile Pro Ile Phe Gly Thr Ala Asn Tyr Ala
65                  70                  75                  80

Gln Lys Phe Gln Gly Arg Val Thr Ile Thr Ala Asp Gln Ser Thr Arg
                85                  90                  95

Thr Ala Tyr Met Asp Leu Gly Arg Leu Arg Ser Glu Asp Thr Ala Val
            100                 105                 110

Tyr Tyr Cys Ala Leu Glu Gly Phe Arg Asp Ser Leu Lys Arg Asn Pro
        115                 120                 125

Phe Asp Ile Trp Gly Gln Gly Thr Met Val Thr Val Ser Ser Ala Ser
    130                 135                 140

Thr Lys Gly Pro Ser Val Phe Pro Leu Ala Pro Ser Ser Lys Ser Thr
145                 150                 155                 160

Ser Gly Gly Thr Ala Ala Leu Gly Cys Leu Val Lys Asp Tyr Phe Pro
                165                 170                 175

Glu Pro Val Thr Val Ser Trp Asn Ser Gly Ala Leu Thr Ser Gly Val
            180                 185                 190

His Thr Phe Pro Ala Val Leu Gln Ser Ser Gly Leu Tyr Ser Leu Ser
        195                 200                 205

Ser Val Val Thr Val Pro Ser Ser Ser Leu Gly Thr Gln Thr Tyr Ile
    210                 215                 220

Cys Asn Val Asn His Lys Pro Ser Asn Thr Lys Val Asp Lys Arg Val

```
225                 230                 235                 240

Glu Pro Lys Ser Cys Asp Lys Thr His Thr Cys Pro Pro Cys Pro Ala
                245                 250                 255

Pro Glu Leu Leu Gly Gly Pro Ser Val Phe Leu Phe Pro Pro Lys Pro
            260                 265                 270

Lys Asp Thr Leu Met Ile Ser Arg Thr Pro Glu Val Thr Cys Val Val
        275                 280                 285

Val Asp Val Ser His Glu Asp Pro Glu Val Lys Phe Asn Trp Tyr Val
    290                 295                 300

Asp Gly Val Glu Val His Asn Ala Lys Thr Lys Pro Arg Glu Glu Gln
305                 310                 315                 320

Tyr Asn Ser Thr Tyr Arg Val Val Ser Val Leu Thr Val Leu His Gln
                325                 330                 335

Asp Trp Leu Asn Gly Lys Glu Tyr Lys Cys Lys Val Ser Asn Lys Ala
            340                 345                 350

Leu Pro Ala Pro Ile Glu Lys Thr Ile Ser Lys Ala Lys Gly Gln Pro
        355                 360                 365

Arg Glu Pro Gln Val Tyr Thr Leu Pro Pro Ser Arg Glu Glu Met Thr
    370                 375                 380

Lys Asn Gln Val Ser Leu Thr Cys Leu Val Lys Gly Phe Tyr Pro Ser
385                 390                 395                 400

Asp Ile Ala Val Glu Trp Glu Ser Asn Gly Gln Pro Glu Asn Asn Tyr
                405                 410                 415

Lys Thr Thr Pro Pro Val Leu Asp Ser Asp Gly Ser Phe Phe Leu Tyr
            420                 425                 430

Ser Lys Leu Thr Val Asp Lys Ser Arg Trp Gln Gln Gly Asn Val Phe
        435                 440                 445

Ser Cys Ser Val Met His Glu Ala Leu His Asn His Tyr Thr Gln Lys
    450                 455                 460

Ser Leu Ser Leu Ser Pro Gly Lys
465                 470
```

<210>  147
<211>  236
<212>  PRT
<213>  Artificial Sequence

<220>
<223>  amino acid sequence of C3048 L in IgG form

<400>  147

```
Met Val Leu Gln Thr Gln Val Phe Ile Ser Leu Leu Leu Trp Ile Ser
1               5                   10                  15

Gly Ala Tyr Gly Gln Ser Val Val Thr Gln Pro Pro Ser Val Ser Ala
            20                  25                  30

Ala Pro Gly Gln Lys Val Thr Ile Ser Cys Ser Gly Gly Thr Thr Asn
        35                  40                  45

Ile Gly Ser Asn Tyr Val Ser Trp Tyr Gln Gln Leu Pro Gly Thr Ala
    50                  55                  60

Pro Lys Phe Leu Met Tyr Glu Asn Asn Lys Arg Pro Ser Gly Ile Pro
65                  70                  75                  80

Asp Arg Phe Ser Gly Ser Lys Ser Gly Thr Ser Ala Thr Leu Ala Ile
                85                  90                  95

Thr Gly Leu Gln Thr Gly Asp Glu Ala Thr Tyr Tyr Cys Ser Thr Trp
            100                 105                 110

Asp Ser Ser Leu Asn Thr Gly Leu Phe Gly Gly Gly Thr Lys Leu Thr
        115                 120                 125

Val Leu Gly Gln Pro Lys Ala Ala Pro Ser Val Thr Leu Phe Pro Pro
    130                 135                 140

Ser Ser Glu Glu Leu Gln Ala Asn Lys Ala Thr Leu Val Cys Leu Ile
145                 150                 155                 160

Ser Asp Phe Tyr Pro Gly Ala Val Thr Val Ala Trp Lys Ala Asp Ser
                165                 170                 175

Ser Pro Val Lys Ala Gly Val Glu Thr Thr Thr Pro Ser Lys Gln Ser
            180                 185                 190

Asn Asn Lys Tyr Ala Ala Ser Ser Tyr Leu Ser Leu Thr Pro Glu Gln
        195                 200                 205

Trp Lys Ser His Arg Ser Tyr Ser Cys Gln Val Thr His Glu Gly Ser
```

210 215 220

Thr Val Glu Lys Thr Val Ala Pro Thr Glu Cys Ser
225 230 235

<210> 148
<211> 470
<212> PRT
<213> Artificial Sequence

<220>
<223> amino acid sequence of C3015 H in IgG form

<400> 148

Met Lys His Leu Trp Phe Phe Leu Leu Leu Val Ala Ala Pro Arg Trp
1 5 10 15

Val Leu Ser Glu Val Gln Leu Val Glu Ser Gly Gly Gly Val Val Gln
20 25 30

Pro Gly Arg Ser Leu Arg Leu Ser Cys Ala Ala Ser Gly Phe Thr Phe
35 40 45

Arg Asn Tyr Ala Met Ser Trp Val Arg Gln Ala Pro Gly Lys Gly Leu
50 55 60

Glu Trp Val Ser Gly Ile Ser Gly Thr Gly Gly Ser Thr Tyr Tyr Ala
65 70 75 80

Asp Ser Val Lys Gly Arg Phe Thr Ile Ser Arg Asp Asn Ser Lys Asp
85 90 95

Thr Leu Tyr Leu Gln Leu Asn Ser Leu Arg Ala Glu Asp Thr Ala Val
100 105 110

Tyr Tyr Cys Ala Lys Ser Arg Ser Met Ser Ile Asn Tyr Gly Met Asp
115 120 125

Val Trp Gly Gln Gly Thr Thr Val Thr Val Ser Ser Ala Ser Thr Lys
130 135 140

Gly Pro Ser Val Phe Pro Leu Ala Pro Ser Ser Lys Ser Thr Ser Gly
145 150 155 160

Gly Thr Ala Ala Leu Gly Cys Leu Val Lys Asp Tyr Phe Pro Glu Pro
165 170 175

Val Thr Val Ser Trp Asn Ser Gly Ala Leu Thr Ser Gly Val His Thr
180 185 190

```
Phe Pro Ala Val Leu Gln Ser Ser Gly Leu Tyr Ser Leu Ser Ser Val
        195                 200                 205

Val Thr Val Pro Ser Ser Ser Leu Gly Thr Gln Thr Tyr Ile Cys Asn
    210                 215                 220

Val Asn His Lys Pro Ser Asn Thr Lys Val Asp Lys Arg Val Glu Pro
225                 230                 235                 240

Lys Ser Cys Asp Lys Thr His Thr Cys Pro Pro Cys Pro Ala Pro Glu
                245                 250                 255

Leu Leu Gly Gly Pro Ser Val Phe Leu Phe Pro Pro Lys Pro Lys Asp
            260                 265                 270

Thr Leu Met Ile Ser Arg Thr Pro Glu Val Thr Cys Val Val Val Asp
        275                 280                 285

Val Ser His Glu Asp Pro Glu Val Lys Phe Asn Trp Tyr Val Asp Gly
    290                 295                 300

Val Glu Val His Asn Ala Lys Thr Lys Pro Arg Glu Glu Gln Tyr Asn
305                 310                 315                 320

Ser Thr Tyr Arg Val Val Ser Val Leu Thr Val Leu His Gln Asp Trp
                325                 330                 335

Leu Asn Gly Lys Glu Tyr Lys Cys Lys Val Ser Asn Lys Ala Leu Pro
            340                 345                 350

Ala Pro Ile Glu Lys Thr Ile Ser Lys Ala Lys Gly Gln Pro Arg Glu
        355                 360                 365

Pro Gln Val Tyr Thr Leu Pro Pro Ser Arg Glu Glu Met Thr Lys Asn
    370                 375                 380

Gln Val Ser Leu Thr Cys Leu Val Lys Gly Phe Tyr Pro Ser Asp Ile
385                 390                 395                 400

Ala Val Glu Trp Glu Ser Asn Gly Gln Pro Glu Asn Asn Tyr Lys Thr
                405                 410                 415

Thr Pro Pro Val Leu Asp Ser Asp Gly Ser Phe Phe Leu Tyr Ser Lys
            420                 425                 430

Leu Thr Val Asp Lys Ser Arg Trp Gln Gln Gly Asn Val Phe Ser Cys
```

```
        435                 440                 445

Ser Val Met His Glu Ala Leu His Asn His Tyr Thr Gln Lys Ser Leu
    450                 455                 460

Ser Leu Ser Pro Gly Lys
465                 470
```

<210>  149
<211>  232
<212>  PRT
<213>  Artificial Sequence

<220>
<223>  amino acid sequence of C3015 L in IgG form

<400>  149

```
Met Val Leu Gln Thr Gln Val Phe Ile Ser Leu Leu Leu Trp Ile Ser
1               5                   10                  15

Gly Ala Tyr Gly Ser Tyr Glu Leu Thr Gln Pro Pro Ser Val Ser Val
            20                  25                  30

Ser Pro Gly Gln Thr Val Ser Ile Thr Cys Ser Gly Asp Asn Leu Gly
        35                  40                  45

Asp Arg Tyr Ala Ser Trp Tyr Gln Gln Lys Pro Gly Gln Ser Pro Val
    50                  55                  60

Gln Val Ile Tyr Gln Asp Thr Lys Arg Pro Ser Gly Ile Pro Glu Arg
65                  70                  75                  80

Phe Ser Gly Ser Asn Ser Gly Asn Thr Ala Thr Leu Thr Ile Ser Gly
                85                  90                  95

Thr Gln Ala Met Asp Glu Ala Asp Tyr Tyr Cys Gln Ala Trp Asp Ser
            100                 105                 110

Asn Thr Val Val Phe Gly Gly Gly Thr Lys Leu Thr Val Leu Gly Gln
        115                 120                 125

Pro Lys Ala Ala Pro Ser Val Thr Leu Phe Pro Pro Ser Ser Glu Glu
    130                 135                 140

Leu Gln Ala Asn Lys Ala Thr Leu Val Cys Leu Ile Ser Asp Phe Tyr
145                 150                 155                 160

Pro Gly Ala Val Thr Val Ala Trp Lys Ala Asp Ser Ser Pro Val Lys
                165                 170                 175
```

Ala Gly Val Glu Thr Thr Thr Pro Ser Lys Gln Ser Asn Asn Lys Tyr
180 185 190

Ala Ala Ser Ser Tyr Leu Ser Leu Thr Pro Glu Gln Trp Lys Ser His
195 200 205

Arg Ser Tyr Ser Cys Gln Val Thr His Glu Gly Ser Thr Val Glu Lys
210 215 220

Thr Val Ala Pro Thr Glu Cys Ser
225 230

<210> 150
<211> 464
<212> PRT
<213> Artificial Sequence

<220>
<223> amino acid sequence of C3022 H in IgG form

<400> 150

Met Lys His Leu Trp Phe Phe Leu Leu Leu Val Ala Ala Pro Arg Trp
1 5 10 15

Val Leu Ser Gln Val Gln Leu Val Gln Ser Gly Ala Glu Val Lys Lys
20 25 30

Pro Gly Ala Ser Val Lys Val Ser Cys Lys Ala Ser Gly Tyr Thr Phe
35 40 45

Thr Gly Tyr Tyr Met His Trp Val Arg Gln Ala Pro Gly Gln Gly Leu
50 55 60

Glu Trp Met Gly Arg Ile Asn Pro Asn Ser Gly Gly Thr Asn Tyr Ala
65 70 75 80

Gln Lys Phe Gln Gly Arg Val Thr Met Thr Arg Asp Thr Ser Ile Ser
85 90 95

Thr Ala Tyr Met Glu Leu Ser Gly Leu Arg Ser Asp Asp Thr Ala Val
100 105 110

Tyr Tyr Cys Ala Ser Gly Ser Val Arg His Pro Trp Gly Gln Gly Thr
115 120 125

Leu Val Thr Val Ser Ser Ala Ser Thr Lys Gly Pro Ser Val Phe Pro
130 135 140

```
Leu Ala Pro Ser Ser Lys Ser Thr Ser Gly Gly Thr Ala Ala Leu Gly
145                 150                 155                 160

Cys Leu Val Lys Asp Tyr Phe Pro Glu Pro Val Thr Val Ser Trp Asn
                165                 170                 175

Ser Gly Ala Leu Thr Ser Gly Val His Thr Phe Pro Ala Val Leu Gln
            180                 185                 190

Ser Ser Gly Leu Tyr Ser Leu Ser Ser Val Val Thr Val Pro Ser Ser
        195                 200                 205

Ser Leu Gly Thr Gln Thr Tyr Ile Cys Asn Val Asn His Lys Pro Ser
    210                 215                 220

Asn Thr Lys Val Asp Lys Arg Val Glu Pro Lys Ser Cys Asp Lys Thr
225                 230                 235                 240

His Thr Cys Pro Pro Cys Pro Ala Pro Glu Leu Leu Gly Gly Pro Ser
                245                 250                 255

Val Phe Leu Phe Pro Pro Lys Pro Lys Asp Thr Leu Met Ile Ser Arg
            260                 265                 270

Thr Pro Glu Val Thr Cys Val Val Val Asp Val Ser His Glu Asp Pro
        275                 280                 285

Glu Val Lys Phe Asn Trp Tyr Val Asp Gly Val Glu Val His Asn Ala
    290                 295                 300

Lys Thr Lys Pro Arg Glu Glu Gln Tyr Asn Ser Thr Tyr Arg Val Val
305                 310                 315                 320

Ser Val Leu Thr Val Leu His Gln Asp Trp Leu Asn Gly Lys Glu Tyr
                325                 330                 335

Lys Cys Lys Val Ser Asn Lys Ala Leu Pro Ala Pro Ile Glu Lys Thr
            340                 345                 350

Ile Ser Lys Ala Lys Gly Gln Pro Arg Glu Pro Gln Val Tyr Thr Leu
        355                 360                 365

Pro Pro Ser Arg Glu Glu Met Thr Lys Asn Gln Val Ser Leu Thr Cys
    370                 375                 380

Leu Val Lys Gly Phe Tyr Pro Ser Asp Ile Ala Val Glu Trp Glu Ser
385                 390                 395                 400
```

Asn Gly Gln Pro Glu Asn Asn Tyr Lys Thr Thr Pro Pro Val Leu Asp
405 410 415

Ser Asp Gly Ser Phe Phe Leu Tyr Ser Lys Leu Thr Val Asp Lys Ser
420 425 430

Arg Trp Gln Gln Gly Asn Val Phe Ser Cys Ser Val Met His Glu Ala
435 440 445

Leu His Asn His Tyr Thr Gln Lys Ser Leu Ser Leu Ser Pro Gly Lys
450 455 460

<210> 151
<211> 236
<212> PRT
<213> Artificial Sequence

<220>
<223> amino acid sequence of C3022 L in IgG form

<400> 151

Met Val Leu Gln Thr Gln Val Phe Ile Ser Leu Leu Leu Trp Ile Ser
1 5 10 15

Gly Ala Tyr Gly Gln Pro Val Leu Thr Gln Pro Pro Ser Ala Ser Gly
20 25 30

Thr Pro Gly Gln Arg Val Thr Ile Ser Cys Ser Gly Ser Arg Ser Asn
35 40 45

Ile Gly Gly Asn Ile Val Ser Trp Tyr Gln Gln Phe Pro Gly Thr Ala
50 55 60

Pro Arg Leu Leu Thr Tyr Ala Asp Asn Gln Arg Pro Ser Gly Val Pro
65 70 75 80

Asp Arg Phe Ser Gly Ser Lys Ser Gly Thr Ser Ala Ser Leu Ala Ile
85 90 95

Ser Gly Leu Gln Ser Glu Asp Glu Ala Asp Tyr Tyr Cys Ala Ala Trp
100 105 110

Asp Asp Ser Leu Asn Gly Val Val Phe Gly Gly Gly Thr Lys Leu Thr
115 120 125

Val Leu Gly Gln Pro Lys Ala Ala Pro Ser Val Thr Leu Phe Pro Pro
130 135 140

Ser Ser Glu Glu Leu Gln Ala Asn Lys Ala Thr Leu Val Cys Leu Ile
145 150 155 160

Ser Asp Phe Tyr Pro Gly Ala Val Thr Val Ala Trp Lys Ala Asp Ser
165 170 175

Ser Pro Val Lys Ala Gly Val Glu Thr Thr Thr Pro Ser Lys Gln Ser
180 185 190

Asn Asn Lys Tyr Ala Ala Ser Ser Tyr Leu Ser Leu Thr Pro Glu Gln
195 200 205

Trp Lys Ser His Arg Ser Tyr Ser Cys Gln Val Thr His Glu Gly Ser
210 215 220

Thr Val Glu Lys Thr Val Ala Pro Thr Glu Cys Ser
225 230 235

<210> 152
<211> 354
<212> DNA
<213> Rattus norvegicus

<400> 152
gaggtgcagt tggtggagtc tgggggaggc ctggtgcagc ctggaagggc cctgaaactc 60

tcctgtgtag tctctggagt cacattcaat tactacggga tgagctggat ccgccaggct 120

ccagggaagg ggctggagtg ggttgcatcc attactaatt ctggtggtag aatttactat 180

ccagactctg tgaagggccg attcactatc tccagagaaa atacacaaaa gaccctatac 240

ctacaaatga acagtctgag gtctgaggac acggccactt attactgtac tctcgatggt 300

cgcgatggtt gggttgctta ctggggccaa ggcactctgg tcactgtctc ttca 354

<210> 153
<211> 327
<212> DNA
<213> Rattus norvegicus

<400> 153
cagtttgtgc ttactcagcc aaactctgtg tctacgaatc tcggaaccac agtcgaactg 60

tcttgcaagc gcaacactgg gaacattgga agcaattatg tgaactggta ccagcagcat 120

gagggaagat ctcccaccac tattatttat agggatgata agagaccaga tggagtttct 180

gacaggttct ctgggtccat tgacagatct tccaagtcag ccctcctgac aatcaataat 240

gtgcagactg aagatgaagc tgactacttc tgtcagtctt acagtagtgg ttttattttc 300

ggcggtggaa ccaagctcac tgtccta 327

<210> 154
<211> 867
<212> DNA
<213> Artificial Sequence

<220>
<223> nucleotide sequence of C3E-7000

<400> 154
atgaaacacc tgtggttctt cctcctgctg gtggcagctc ccagatgggt gctgagcgag 60

gtgcagttgg tggagtctgg gggaggcctg gtgcagcctg gaagggccct gaaactctcc 120

tgtgtagtct ctggagtcac attcaattac tacgggatga gctggatccg ccaggctcca 180

gggaaggggc tggagtgggt tgcatccatt actaattctg gtggtagaat ttactatcca 240

gactctgtga agggccgatt cactatctcc agagaaaata cacaaaagac cctataccta 300

caaatgaaca gtctgaggtc tgaggacacg gccacttatt actgtactct cgatggtcgc 360

gatggttggg ttgcttactg gggccaaggc actctggtca ctgtctcttc aggtggaggc 420

ggttcaggcg gaggtggcag cggcggtggc gggagtcagt ttgtgcttac tcagccaaac 480

tctgtgtcta cgaatctcgg aaccacagtc gaactgtctt gcaagcgcaa cactgggaac 540

attggaagca attatgtgaa ctggtaccag cagcatgagg gaagatctcc caccactatt 600

atttataggg atgataagag accagatgga gtttctgaca ggttctctgg gtccattgac 660

agatcttcca agtcagccct cctgacaatc aataatgtgc agactgaaga tgaagctgac 720

tacttctgtc agtcttacag tagtggtttt attttcggcg gtggaaccaa gctcactgtc 780

ctaggcgcgt ctgcggccgc aggatccggt ggtgattaca aagatgatga cgataaaggt 840

gcagcggcgc atcaccatca tcaccac 867

<210> 155
<211> 118
<212> PRT
<213> Artificial Sequence

<220>
<223> amino acid sequence of C3E-7034_VH

<400> 155

Glu Val Gln Leu Val Glu Ser Gly Gly Gly Leu Val Gln Pro Gly Gly
1 5 10 15

Ser Leu Arg Leu Ser Cys Ala Ala Ser Gly Val Thr Phe Asn Tyr Tyr
20 25 30

Gly Met Ser Trp Ile Arg Gln Ala Pro Gly Lys Gly Leu Glu Trp Val
35 40 45

Ala Ser Ile Thr Asn Ser Gly Gly Arg Ile Tyr Tyr Pro Asp Ser Val

50 55 60

Lys Gly Arg Phe Thr Ile Ser Arg Glu Asn Thr Gln Lys Thr Leu Tyr
65 70 75 80

Leu Gln Met Asn Ser Leu Arg Ala Glu Asp Thr Ala Val Tyr Tyr Cys
85 90 95

Thr Leu Asp Gly Arg Asp Gly Trp Val Ala Tyr Trp Gly Gln Gly Thr
100 105 110

Leu Val Thr Val Ser Ser
115

<210> 156
<211> 109
<212> PRT
<213> Artificial Sequence

<220>
<223> amino acid sequence of C3E-7034_VL

<400> 156

Asn Phe Met Leu Thr Gln Pro His Ser Val Ser Glu Ser Pro Gly Lys
1 5 10 15

Thr Val Thr Ile Ser Cys Lys Arg Asn Thr Gly Asn Ile Gly Ser Asn
20 25 30

Tyr Val Asn Trp Tyr Gln Gln His Glu Gly Ser Ser Pro Thr Thr Ile
35 40 45

Ile Tyr Arg Asp Asp Lys Arg Pro Asp Gly Val Ser Asp Arg Phe Ser
50 55 60

Gly Ser Ile Asp Arg Ser Ser Lys Ser Ala Ser Leu Thr Ile Ser Asn
65 70 75 80

Leu Lys Thr Glu Asp Glu Ala Asp Tyr Phe Cys Gln Ser Tyr Ser Ser
85 90 95

Gly Phe Ile Phe Gly Gly Gly Thr Lys Leu Thr Val Leu
100 105

<210> 157
<211> 864
<212> DNA
<213> Artificial Sequence

<220>

<223> nucleotide sequence of C3E-7034

<400> 157
atgaaacacc tgtggttctt cctcctgctg gtggcagctc ccagatgggt gctgagcgga 60

gaagtgcagc tggtggaatc cgggggggc ctggtgcagc ctgggggag cctgagactg 120

agttgtgccg cctctggggt gacatttaac tactatggca tgtcttggat ccgccaggca 180

cctggaaagg gcctggagtg ggtggccagc atcactaatt ccggcgggcg aatctactat 240

cccgacagcg tcaagggcag gttcacaatt tcccgcgaga acacacagaa aactctgtac 300

ctgcagatga atagcctgag agccgaagat acagctgtgt actattgcac tctggacggc 360

agggatgggt gggtcgccta ttgggggcag ggaaccctgg tgacagtcag ctccggagga 420

ggaggatctg gcggaggagg cagtggggga ggcgggtcaa actttatgct gacccagccc 480

cacagtgtgt cagagagccc tggcaagact gtcaccatct cttgtaaaag gaacaccgga 540

aatattggca gtaactacgt gaattggtat cagcagcatg aagggtctag tccaaccaca 600

atcatctacc gggacgataa gagacccgac ggggtgtccg atcgattctc cggatctatc 660

gaccggtcaa gcaagagtgc ttcactgacc attagcaatc tgaaaacaga ggacgaagca 720

gattactttt gccagtccta ttcctctggc ttcatctttg gaggcgggac taaactgacc 780

gtgctgggcg cggccgcagg tgcaggtggt gattacaaag atgatgacga taaaggtgca 840

gcggcgcatc accatcatca ccac 864

<210> 158
<211> 107
<212> PRT
<213> Artificial Sequence

<220>
<223> amino acid sequence of C3E-7035_VL

<400> 158

Gln Ala Val Leu Thr Gln Pro Ser Ser Val Ser Gly Val Pro Gly Gln
1 5 10 15

Arg Val Thr Ile Ser Cys Lys Arg Asn Thr Gly Asn Ile Gly Ser Asn
20 25 30

Tyr Val Asn Trp Tyr Gln Gln Leu Pro Gly Thr Ala Pro Lys Leu Leu
35 40 45

Ile Tyr Arg Asp Asp Lys Arg Pro Ser Gly Val Pro Asp Arg Phe Ser
50 55 60

Gly Ser Lys Ser Gly Thr Ser Ala Ser Leu Ala Ile Thr Gly Phe Gln
65 70 75 80

```
Ala Glu Asp Glu Ala Asp Tyr Tyr Cys Gln Ser Tyr Ser Ser Gly Phe
                85                  90                  95

Ile Phe Gly Gly Gly Thr Lys Leu Thr Val Leu
            100                 105
```

<210> 159
<211> 864
<212> DNA
<213> Artificial Sequence

<220>
<223> nucleotide sequence of C3E-7035

<400> 159

```
atgaaacacc tgtggttctt cctcctgctg gtggcagctc ccagatgggt gctgagcgga       60

gaagtgcagc tggtggaatc cggggggggc ctggtgcagc ctggggggag cctgagactg      120

agttgtgccg cctctggggt gacatttaac tactatggca tgtcttggat ccgccaggca      180

cctggaaagg gcctggagtg ggtggccagc atcactaatt ccggcgggcg aatctactat      240

cccgacagcg tcaagggcag gttcacaatt tcccgcgaga acacacagaa aactctgtac      300

ctgcagatga atagcctgag agccgaagat acagctgtgt actattgcac tctggacggc      360

agggatgggt gggtcgccta ttgggggcag ggaaccctgg tgacagtcag ctccggagga      420

ggaggatctg gcggaggagg cagtggggga ggcgggtcaa tggcccaggc tgtgctcact      480

cagccgtcct ctgtttctgg cgtacctggc caacgggtga ccattagctg taaaaggaat      540

accgggaata tcgggtctaa ctacgtgaac tggtatcagc agcttccagg gacagctccc      600

aagttgctga tctatcgcga cgacaaaaga ccctcagggg tccctgaccg atttagtggc      660

agcaaaagcg gtacttccgc ttccctggcg ataaccggct ttcaggccga agatgaggca      720

gactactatt gccagtcata ttccagcggc ttcatcttcg gaggcggaac taagctgaca      780

gtgctgggcg cggccgcagg tgcaggtggt gattacaaag atgatgacga taaaggtgca      840

gcggcgcatc accatcatca ccac                                             864
```

<210> 160
<211> 107
<212> PRT
<213> Artificial Sequence

<220>
<223> amino acid sequence of C3E-7036_VL

<400> 160

```
Asn Phe Met Leu Thr Gln Pro Ser Ser Val Ser Gly Val Pro Gly Gln
1               5                   10                  15
```

```
Arg Val Thr Ile Ser Cys Thr Gly Asn Thr Gly Asn Ile Gly Ser Asn
            20                  25                  30

Tyr Val Asn Trp Tyr Gln Gln Leu Pro Gly Thr Ala Pro Lys Leu Leu
        35                  40                  45

Ile Tyr Arg Asp Asp Lys Arg Pro Ser Gly Val Pro Asp Arg Phe Ser
    50                  55                  60

Gly Ser Lys Ser Gly Thr Ser Ala Ser Leu Ala Ile Thr Gly Phe Gln
65                  70                  75                  80

Ala Glu Asp Glu Ala Asp Tyr Tyr Cys Gln Ser Tyr Ser Ser Gly Phe
                85                  90                  95

Ile Phe Gly Gly Gly Thr Lys Leu Thr Val Leu
            100                 105


<210>  161
<211>  858
<212>  DNA
<213>  Artificial Sequence

<220>
<223>  nucleotide sequence of C3E-7036

<400>  161
atgaaacacc tgtggttctt cctcctgctg gtggcagctc ccagatgggt gctgagcgga      60

gaagtgcagc tggtggaatc cggggggggc ctggtgcagc ctggggggag cctgagactg     120

agttgtgccg cctctggggt gacatttaac tactatggca tgtcttggat ccgccaggca     180

cctggaaagg gcctggagtg ggtggccagc atcactaatt ccggcgggcg aatctactat     240

cccgacagcg tcaagggcag gttcacaatt tcccgcgaga acacacagaa aactctgtac     300

ctgcagatga atagcctgag agccgaagat acagctgtgt actattgcac tctggacggc     360

agggatgggt gggtcgccta ttgggggcag ggaaccctgg tgacagtcag ctccggagga     420

ggaggatctg gcggaggagg cagtggggga ggcgggtcaa actttatgct cactcagccg     480

tcctctgttt ctggcgtacc tggccaacgg gtgaccatta gctgtacggg taataccggg     540

aatatcgggt ctaactacgt gaactggtat cagcagcttc cagggacagc tcccaagttg     600

ctgatctatc gcgacgacaa aagaccctca ggggtccctg accgatttag tggcagcaaa     660

agcggtactt ccgcttccct ggcgataacc ggctttcagg ccgaagatga ggcagactac     720

tattgccagt catattccag cggcttcatc ttcggaggcg gaactaagct gacagtgttg     780

ggtgcggccg caggtgcagg tggtgattac aaagatgatg acgataaagg tgcagcggcg     840

catcaccatc atcaccac                                                   858
```

<210> 162
<211> 1599
<212> DNA
<213> Artificial Sequence

<220>
<223> nucleotide_sequence_of_C2037-C3E-7034

<400> 162
atgaaacacc tgtggttctt cctcctgctg gtggcagctc ccagatgggt gctgagcgga 60

caggtgcagc tggtgcagtc tggcgccgaa gtgaagaaac caggcgccag cgtgaaggtg 120

tcctgcaagg ccagcggcta cacctttacc ggctactaca tgcactgggt gcgccaggct 180

ccaggccagg gactggaatg gatgggccgg atcaacccca atagcggcgg caccaactac 240

gcccagaaat tccagggcag agtgaccatg acccgggaca ccagcatcag caccgcctac 300

atggaactga gccggctgag aagcgacgac accgccgtgt actactgcgc cagcggcaag 360

agcaactacc cttggggcca gggcacactc gtgaccgtgt ctagcggagg cggaggatct 420

ggcggcggag gaagtggcgg agggggatct cagcctgtgc tgacacagcc tcctagcgcc 480

tctggcacac ctggccagag agtgacaatc agctgcagcg gcagcaacag caacatcggc 540

agccggaccg tgaactggta tcagcatctg cctggcaccg cccccagact gctgatctac 600

ggcaacaacc agtggcccag cggcgtgccc gatagattca gcggctctaa gagcggcagc 660

tccgccagcc tggccatctc tggactgaga tccgaggacg aggccgacta ctactgtgcc 720

gcctgggacg atagcctgag cggcgtggtg tttggcggag gcaccaagct gacagtgctg 780

ggcggaggcg gttcagaagt gcagctggtg gaatccgggg ggggcctggt gcagcctggg 840

gggagcctga gactgagttg tgccgcctct ggggtgacat ttaactacta tggcatgtct 900

tggatccgcc aggcacctgg aaagggcctg gagtgggtgg ccagcatcac taattccggc 960

gggcgaatct actatcccga cagcgtcaag ggcaggttca caatttcccg cgagaacaca 1020

cagaaaactc tgtacctgca gatgaatagc ctgagagccg aagatacagc tgtgtactat 1080

tgcactctgg acggcaggga tgggtgggtc gcctattggg ggcagggaac cctggtgaca 1140

gtcagctccg gaggaggagg atctggcgga ggaggcagtg gggaggcgg gtcaaacttt 1200

atgctgaccc agccccacag tgtgtcagag agccctggca agactgtcac catctcttgt 1260

aaaaggaaca ccggaaatat tggcagtaac tacgtgaatt ggtatcagca gcatgaaggg 1320

tctagtccaa ccacaatcat ctaccgggac gataagagac ccgacggggt gtccgatcga 1380

ttctccggat ctatcgaccg gtcaagcaag agtgcttcac tgaccattag caatctgaaa 1440

acagaggacg aagcagatta cttttgccag tcctattcct ctggcttcat ctttggaggc 1500

gggactaaac tgaccgtgct gggcgcggcc gcaggtgcag gtggtgatta caaagatgat 1560

gacgataaag gtgcagcggc gcatcaccat catcaccac 1599

<210> 163
<211> 1623
<212> DNA
<213> Artificial Sequence

<220>
<223> nucleotide sequence of C3048-C3E-7034

<400> 163
atgaaacacc tgtggttctt cctcctgctg gtggcagctc ccagatgggt gctgagcgga 60

caggtgcagc tggtgcagtc tggcgccgaa gtgaagaaac ccggcagcag cgtgaaggtg 120

tcctgcaagg ctagcggcgg caccttcagc agatacgcca tctcttgggt gcgccaggcc 180

cctggacagg gcctggaatg gatgggcggc atcatcccca tcttcggcac cgccaactac 240

gcccagaaat tccagggcag agtgaccatc accgccgacc agagcacccg gaccgcctat 300

atggatctgg gccggctgag aagcgaggac accgccgtgt actactgcgc cctggaaggc 360

ttccgggaca gcctgaagag aaacccctc gacatctggg gccagggcac aatggtcacc 420

gtgtctagcg gaggcggagg atctggcggc ggaggaagtg gcggaggggg atctcagtct 480

gtcgtgaccc agcctcctag cgtgtcagcc gccccaggcc agaaagtgac aatcagctgt 540

tctggcggca ccaccaacat cggcagcaac tacgtgtcct ggtatcagca gctgcccgga 600

accgccccca agttcctgat gtacgagaac aacaagcggc ccagcggcat ccccgacaga 660

ttcagcggca gcaagagcgg cacaagcgcc acactggcca tcaccggact gcagacaggc 720

gacgaggcca cctactactg ctccacctgg gacagcagcc tgaacaccgg cctgtttggc 780

ggaggcacca agctgacagt gctgggcgga ggcggttcag aagtgcagct ggtggaatcc 840

gggggggcc tggtgcagcc tgggggagc ctgagactga gttgtgccgc ctctggggtg 900

acatttaact actatggcat gtcttggatc cgccaggcac ctggaaaggg cctggagtgg 960

gtggccagca tcactaattc cggcgggcga atctactatc ccgacagcgt caagggcagg 1020

ttcacaattt cccgcgagaa cacacagaaa actctgtacc tgcagatgaa tagcctgaga 1080

gccgaagata cagctgtgta ctattgcact ctggacggca gggatgggtg ggtcgcctat 1140

tgggggcagg gaaccctggt gacagtcagc tccggaggag gaggatctgg cggaggaggc 1200

agtgggggag gcgggtcaaa ctttatgctg acccagcccc acagtgtgtc agagagccct 1260

ggcaagactg tcaccatctc ttgtaaaagg aacaccggaa atattggcag taactacgtg 1320

aattggtatc agcagcatga agggtctagt ccaaccacaa tcatctaccg ggacgataag 1380

agacccgacg gggtgtccga tcgattctcc ggatctatcg accggtcaag caagagtgct 1440

tcactgacca ttagcaatct gaaaacagag gacgaagcag attacttttg ccagtcctat 1500

tcctctggct tcatctttgg aggcgggact aaactgaccg tgctgggcgc ggccgcaggt 1560

gcaggtggtg attacaaaga tgatgacgat aaaggtgcag cggcgcatca ccatcatcac 1620

cac 1623

<210> 164
<211> 1599
<212> DNA
<213> Artificial Sequence

<220>
<223> nucleotide sequence of C3022-C3E-7034

<400> 164
atgaaacacc tgtggttctt cctcctgctg gtggcagctc ccagatgggt gctgagcgga 60

caggtgcagc tggtgcagtc tggcgccgaa gtgaagaaac caggcgccag cgtgaaggtg 120

tcctgcaagg ccagcggcta cacctttacc ggctactaca tgcactgggt gcgccaggct 180

ccaggccagg gactggaatg gatgggccgg atcaacccca atagcggcgg caccaactac 240

gcccagaaat tccagggcag agtgaccatg acccgggaca ccagcatcag caccgcctac 300

atggaactga gcggcctgag aagcgacgac accgccgtgt actactgtgc ctctggctct 360

gtgcggcacc cttggggaca gggaacactc gtgaccgtgt ccagcggtgg aggcggttca 420

ggcggaggtg gcagcggcgg tggcgggagt cagcctgtgc tgacacagcc tccaagcgcc 480

tctggcacac ctggccagag agtgacaatc agctgcagcg gcagcagaag caacatcggc 540

ggcaacatcg tgtcctggta tcagcagttc cccggcaccg cccctagact gctgacctac 600

gccgacaacc agaggcctag cggcgtgccc gatagattca gcggctctaa gagcggcacc 660

agcgccagcc tggccatctc tggcctgcag tctgaggacg aggccgacta ctattgcgcc 720

gcctgggacg acagcctgaa cggcgtggtg tttggcggag gcaccaagct gacagtgctg 780

ggcggaggcg gttcagaagt gcagctggtg gaatccgggg ggggcctggt gcagcctggg 840

gggagcctga gactgagttg tgccgcctct ggggtgacat ttaactacta tggcatgtct 900

tggatccgcc aggcacctgg aaagggcctg gagtgggtgg ccagcatcac taattccggc 960

gggcgaatct actatcccga cagcgtcaag ggcaggttca caatttcccg cgagaacaca 1020

cagaaaactc tgtacctgca gatgaatagc ctgagagccg aagatacagc tgtgtactat 1080

tgcactctgg acggcaggga tgggtgggtc gcctattggg ggcagggaac cctggtgaca 1140

gtcagctccg gaggaggagg atctggcgga ggaggcagtg gggaggcgg gtcaaacttt 1200

atgctgaccc agccccacag tgtgtcagag agccctggca agactgtcac catctcttgt 1260

aaaaggaaca ccggaaatat tggcagtaac tacgtgaatt ggtatcagca gcatgaaggg 1320

tctagtccaa ccacaatcat ctaccgggac gataagagac ccgacggggt gtccgatcga 1380

ttctccggat ctatcgaccg gtcaagcaag agtgcttcac tgaccattag caatctgaaa 1440

acagaggacg aagcagatta cttttgccag tcctattcct ctggcttcat ctttggaggc 1500

gggactaaac tgaccgtgct gggcgcggcc gcaggtgcag gtggtgatta caaagatgat 1560

gacgataaag gtgcagcggc gcatcaccat catcaccac 1599

<210> 165
<211> 1599
<212> DNA
<213> Artificial Sequence

<220>
<223> nucleotide sequence of C2037-C3E-7035

<400> 165
atgaaacacc tgtggttctt cctcctgctg gtggcagctc ccagatgggt gctgagcgga 60

caggtgcagc tggtgcagtc tggcgccgaa gtgaagaaac caggcgccag cgtgaaggtg 120

tcctgcaagg ccagcggcta cacctttacc ggctactaca tgcactgggt gcgccaggct 180

ccaggccagg gactggaatg gatgggccgg atcaacccca atagcggcgg caccaactac 240

gcccagaaat tccagggcag agtgaccatg acccgggaca ccagcatcag caccgcctac 300

atggaactga gccggctgag aagcgacgac accgccgtgt actactgcgc cagcggcaag 360

agcaactacc cttggggcca gggcacactc gtgaccgtgt ctagcggagg cggaggatct 420

ggcggcggag gaagtggcgg agggggatct cagcctgtgc tgacacagcc tcctagcgcc 480

tctggcacac ctggccagag agtgacaatc agctgcagcg gcagcaacag caacatcggc 540

agccggaccg tgaactggta tcagcatctg cctggcaccg cccccagact gctgatctac 600

ggcaacaacc agtggcccag cggcgtgccc gatagattca gcggctctaa gagcggcagc 660

tccgccagcc tggccatctc tggactgaga tccgaggacg aggccgacta ctactgtgcc 720

gcctgggacg atagcctgag cggcgtggtg tttggcggag gcaccaagct gacagtgctg 780

ggcggaggcg gttcagaagt gcagctggtg gaatccgggg ggggcctggt gcagcctggg 840

gggagcctga gactgagttg tgccgcctct ggggtgacat ttaactacta tggcatgtct 900

tggatccgcc aggcacctgg aaagggcctg gagtgggtgg ccagcatcac taattccggc 960

gggcgaatct actatcccga cagcgtcaag ggcaggttca caatttcccg cgagaacaca 1020

cagaaaactc tgtacctgca gatgaatagc ctgagagccg aagatacagc tgtgtactat 1080

tgcactctgg acggcaggga tgggtgggtc gcctattggg ggcagggaac cctggtgaca 1140

gtcagctccg gaggaggagg atctggcgga ggaggcagtg ggggaggcgg gtcaatggcc 1200

caggctgtgc tcactcagcc gtcctctgtt tctggcgtac ctggccaacg ggtgaccatt 1260

agctgtaaaa ggaataccgg gaatatcggg tctaactacg tgaactggta tcagcagctt 1320

ccagggacag ctcccaagtt gctgatctat cgcgacgaca aaagaccctc aggggtccct 1380

gaccgattta gtggcagcaa aagcggtact tccgcttccc tggcgataac cggctttcag 1440

gccgaagatg aggcagacta ctattgccag tcatattcca gcggcttcat cttcggaggc 1500

ggaactaagc tgacagtgtt gggcgcggcc gcaggtgcag gtggtgatta caaagatgat 1560

gacgataaag gtgcagcggc gcatcaccat catcaccac 1599

<210> 166
<211> 1623
<212> DNA
<213> Artificial Sequence

<220>
<223> nucleotide sequence of C3048-C3E-7035

<400> 166
atgaaacacc tgtggttctt cctcctgctg gtggcagctc ccagatgggt gctgagcgga 60

caggtgcagc tggtgcagtc tggcgccgaa gtgaagaaac ccggcagcag cgtgaaggtg 120

tcctgcaagg ctagcggcgg caccttcagc agatacgcca tctcttgggt gcgccaggcc 180

cctggacagg gcctggaatg gatgggcggc atcatcccca tcttcggcac cgccaactac 240

gcccagaaat tccagggcag agtgaccatc accgccgacc agagcacccg gaccgcctat 300

atggatctgg gccggctgag aagcgaggac accgccgtgt actactgcgc cctggaaggc 360

ttccgggaca gcctgaagag aaaccccttc gacatctggg gccagggcac aatggtcacc 420

gtgtctagcg gaggcggagg atctggcggc ggaggaagtg gcggaggggg atctcagtct 480

gtcgtgaccc agcctcctag cgtgtcagcc gccccaggcc agaaagtgac aatcagctgt 540

tctggcggca ccaccaacat cggcagcaac tacgtgtcct ggtatcagca gctgcccgga 600

accgccccca agttcctgat gtacgagaac aacaagcggc ccagcggcat ccccgacaga 660

ttcagcggca gcaagagcgg cacaagcgcc acactggcca tcaccggact gcagacaggc 720

gacgaggcca cctactactg ctccacctgg gacagcagcc tgaacaccgg cctgtttggc 780

ggaggcacca agctgacagt gctgggcgga ggcggttcag aagtgcagct ggtggaatcc 840

gggggggggcc tggtgcagcc tgggggggagc ctgagactga gttgtgccgc ctctggggtg 900

acatttaact actatggcat gtcttggatc cgccaggcac ctggaaaggg cctggagtgg 960

gtggccagca tcactaattc cggcgggcga atctactatc ccgacagcgt caagggcagg 1020

ttcacaattt cccgcgagaa cacacagaaa actctgtacc tgcagatgaa tagcctgaga 1080

gccgaagata cagctgtgta ctattgcact ctggacggca gggatgggtg ggtcgcctat 1140

tgggggcagg gaaccctggt gacagtcagc tccggaggag gaggatctgg cggaggaggc 1200

agtgggggag gcgggtcaat ggcccaggct gtgctcactc agccgtcctc tgtttctggc 1260

gtacctggcc aacgggtgac cattagctgt aaaaggaata ccgggaatat cgggtctaac 1320

tacgtgaact ggtatcagca gcttccaggg acagctccca agttgctgat ctatcgcgac 1380

gacaaaagac cctcaggggt ccctgaccga tttagtggca gcaaaagcgg tacttccgct 1440

tccctggcga taaccggctt tcaggccgaa gatgaggcag actactattg ccagtcatat 1500

tccagcggct tcatcttcgg aggcggaact aagctgacag tgttgggcgc ggccgcaggt 1560

gcaggtggtg attacaaaga tgatgacgat aaaggtgcag cggcgcatca ccatcatcac 1620

cac 1623

<210> 167
<211> 1599
<212> DNA
<213> Artificial Sequence

<220>
<223> nucleotide sequence of C3022-C3E-7035

<400> 167
atgaaacacc tgtggttctt cctcctgctg gtggcagctc ccagatgggt gctgagcgga 60

caggtgcagc tggtgcagtc tggcgccgaa gtgaagaaac caggcgccag cgtgaaggtg 120

tcctgcaagg ccagcggcta cacctttacc ggctactaca tgcactgggt gcgccaggct 180

ccaggccagg gactggaatg gatgggccgg atcaacccca atagcggcgg caccaactac 240

gcccagaaat tccagggcag agtgaccatg acccgggaca ccagcatcag caccgcctac 300

atggaactga gcggcctgag aagcgacgac accgccgtgt actactgtgc ctctggctct 360

gtgcggcacc cttggggaca gggaacactc gtgaccgtgt ccagcggtgg aggcggttca 420

ggcggaggtg gcagcggcgg tggcgggagt cagcctgtgc tgacacagcc tccaagcgcc 480

tctggcacac ctggccagag agtgacaatc agctgcagcg gcagcagaag caacatcggc 540

ggcaacatcg tgtcctggta tcagcagttc cccggcaccg cccctagact gctgacctac 600

gccgacaacc agaggcctag cggcgtgccc gatagattca gcggctctaa gagcggcacc 660

agcgccagcc tggccatctc tggcctgcag tctgaggacg aggccgacta ctattgcgcc 720

gcctgggacg acagcctgaa cggcgtggtg tttggcggag gcaccaagct gacagtgctg 780

ggcggaggcg gttcagaagt gcagctggtg gaatccgggg ggggcctggt gcagcctggg 840

gggagcctga gactgagttg tgccgcctct ggggtgacat ttaactacta tggcatgtct 900

tggatccgcc aggcacctgg aaagggcctg gagtgggtgg ccagcatcac taattccggc 960

gggcgaatct actatcccga cagcgtcaag ggcaggttca caatttcccg cgagaacaca 1020

cagaaaactc tgtacctgca gatgaatagc ctgagagccg aagatacagc tgtgtactat 1080

tgcactctgg acggcaggga tgggtgggtc gcctattggg ggcagggaac cctggtgaca 1140

gtcagctccg gaggaggagg atctggcgga ggaggcagtg gggaggcgg gtcaatggcc 1200

caggctgtgc tcactcagcc gtcctctgtt tctggcgtac ctggccaacg ggtgaccatt 1260

agctgtaaaa ggaataccgg gaatatcggg tctaactacg tgaactggta tcagcagctt 1320

ccagggacag ctcccaagtt gctgatctat cgcgacgaca aaagaccctc aggggtccct 1380

gaccgattta gtggcagcaa aagcggtact tccgcttccc tggcgataac cggctttcag 1440

gccgaagatg aggcagacta ctattgccag tcatattcca gcggcttcat cttcggaggc 1500

ggaactaagc tgacagtgtt gggcgcggcc gcaggtgcag gtggtgatta caaagatgat 1560

gacgataaag gtgcagcggc gcatcaccat catcaccac 1599

<210> 168
<211> 1593
<212> DNA
<213> Artificial Sequence

<220>
<223> nucleotide sequence of C2037-C3E-7036

<400> 168
atgaaacacc tgtggttctt cctcctgctg gtggcagctc ccagatgggt gctgagcgga 60

caggtgcagc tggtgcagtc tggcgccgaa gtgaagaaac caggcgccag cgtgaaggtg 120

tcctgcaagg ccagcggcta cacctttacc ggctactaca tgcactgggt gcgccaggct 180

ccaggccagg gactggaatg gatgggccgg atcaacccca atagcggcgg caccaactac 240

gcccagaaat tccagggcag agtgaccatg acccgggaca ccagcatcag caccgcctac 300

atggaactga gccggctgag aagcgacgac accgccgtgt actactgcgc cagcggcaag 360

agcaactacc cttggggcca gggcacactc gtgaccgtgt ctagcggagg cggaggatct 420

ggcggcggag gaagtggcgg agggggatct cagcctgtgc tgacacagcc tcctagcgcc 480

tctggcacac ctggccagag agtgacaatc agctgcagcg gcagcaacag caacatcggc 540

agccggaccg tgaactggta tcagcatctg cctggcaccg cccccagact gctgatctac 600

ggcaacaacc agtggcccag cggcgtgccc gatagattca gcggctctaa gagcggcagc 660

tccgccagcc tggccatctc tggactgaga tccgaggacg aggccgacta ctactgtgcc 720

gcctgggacg atagcctgag cggcgtggtg tttggcggag gcaccaagct gacagtgctg 780

ggcggaggcg gttcagaagt gcagctggtg gaatccgggg ggggcctggt gcagcctggg 840

gggagcctga gactgagttg tgccgcctct ggggtgacat ttaactacta tggcatgtct 900

tggatccgcc aggcacctgg aaagggcctg gagtgggtgg ccagcatcac taattccggc 960

gggcgaatct actatcccga cagcgtcaag ggcaggttca caatttcccg cgagaacaca 1020

cagaaaactc tgtacctgca gatgaatagc ctgagagccg aagatacagc tgtgtactat 1080

tgcactctgg acggcaggga tgggtgggtc gcctattggg ggcagggaac cctggtgaca 1140

gtcagctccg gaggaggagg atctggcgga ggaggcagtg ggggaggcgg gtcaaacttt 1200

atgctcactc agccgtcctc tgtttctggc gtacctggcc aacgggtgac cattagctgt 1260

acgggtaata ccgggaatat cgggtctaac tacgtgaact ggtatcagca gcttccaggg 1320

acagctccca agttgctgat ctatcgcgac gacaaaagac cctcaggggt ccctgaccga 1380

tttagtggca gcaaaagcgg tacttccgct tccctggcga taaccggctt tcaggccgaa 1440

gatgaggcag actactattg ccagtcatat tccagcggct tcatcttcgg aggcggaact 1500

aagctgacag tgttgggcgc ggccgcaggt gcaggtggtg attacaaaga tgatgacgat 1560

aaaggtgcag cggcgcatca ccatcatcac cac 1593

<210> 169
<211> 1617
<212> DNA
<213> Artificial Sequence

<220>
<223> nucleotide sequence of C3048-C3E-7036

<400> 169
atgaaacacc tgtggttctt cctcctgctg gtggcagctc ccagatgggt gctgagcgga 60

caggtgcagc tggtgcagtc tggcgccgaa gtgaagaaac ccggcagcag cgtgaaggtg 120

tcctgcaagg ctagcggcgg caccttcagc agatacgcca tctcttgggt gcgccaggcc 180

cctggacagg gcctggaatg gatgggcggc atcatcccca tcttcggcac cgccaactac 240

gcccagaaat tccagggcag agtgaccatc accgccgacc agagcacccg gaccgcctat 300

atggatctgg gccggctgag aagcgaggac accgccgtgt actactgcgc cctggaaggc 360

ttccgggaca gcctgaagag aaaccccttc gacatctggg gccagggcac aatggtcacc 420

gtgtctagcg gaggcggagg atctggcggc ggaggaagtg gcggaggggg atctcagtct 480

gtcgtgaccc agcctcctag cgtgtcagcc gccccaggcc agaaagtgac aatcagctgt 540

tctggcggca ccaccaacat cggcagcaac tacgtgtcct ggtatcagca gctgcccgga 600

accgccccca agttcctgat gtacgagaac aacaagcggc ccagcggcat ccccgacaga 660

ttcagcggca gcaagagcgg cacaagcgcc acactggcca tcaccggact gcagacaggc 720

gacgaggcca cctactactg ctccacctgg gacagcagcc tgaacaccgg cctgtttggc 780

ggaggcacca agctgacagt gctgggcgga ggcggttcag aagtgcagct ggtggaatcc 840

ggggggggcc tggtgcagcc tgggggggagc ctgagactga gttgtgccgc ctctggggtg 900

acatttaact actatggcat gtcttggatc cgccaggcac ctggaaaggg cctggagtgg 960

gtggccagca tcactaattc cggcgggcga atctactatc ccgacagcgt caagggcagg 1020

ttcacaattt cccgcgagaa cacacagaaa actctgtacc tgcagatgaa tagcctgaga 1080

gccgaagata cagctgtgta ctattgcact ctggacggca gggatgggtg ggtcgcctat 1140

tgggggcagg gaaccctggt gacagtcagc tccggaggag gaggatctgg cggaggaggc 1200

agtgggggag gcgggtcaaa ctttatgctc actcagccgt cctctgtttc tggcgtacct 1260

ggccaacggg tgaccattag ctgtacgggt aataccggga atatcgggtc taactacgtg 1320

aactggtatc agcagcttcc agggacagct cccaagttgc tgatctatcg cgacgacaaa 1380

agaccctcag ggtccctga ccgatttagt ggcagcaaaa gcggtacttc cgcttccctg 1440

gcgataaccg gctttcaggc cgaagatgag gcagactact attgccagtc atattccagc 1500

ggcttcatct tcggaggcgg aactaagctg acagtgttgg gcgcggccgc aggtgcaggt 1560

ggtgattaca aagatgatga cgataaaggt gcagcggcgc atcaccatca tcaccac 1617

<210> 170
<211> 1593
<212> DNA
<213> Artificial Sequence

<220>
<223> nucleotide sequence of C3022-C3E-7036

<400> 170
atgaaacacc tgtggttctt cctcctgctg gtggcagctc ccagatgggt gctgagcgga 60

caggtgcagc tggtgcagtc tggcgccgaa gtgaagaaac caggcgccag cgtgaaggtg 120

tcctgcaagg ccagcggcta cacctttacc ggctactaca tgcactgggt gcgccaggct 180

ccaggccagg gactggaatg gatgggccgg atcaacccca atagcggcgg caccaactac 240

gcccagaaat tccagggcag agtgaccatg acccgggaca ccagcatcag caccgcctac 300

atggaactga gcggcctgag aagcgacgac accgccgtgt actactgtgc ctctggctct 360

gtgcggcacc cttggggaca gggaacactc gtgaccgtgt ccagcggtgg aggcggttca 420

ggcggaggtg gcagcggcgg tggcgggagt cagcctgtgc tgacacagcc tccaagcgcc 480

tctggcacac ctggccagag agtgacaatc agctgcagcg gcagcagaag caacatcggc 540

ggcaacatcg tgtcctggta tcagcagttc cccggcaccg cccctagact gctgacctac 600

gccgacaacc agaggcctag cggcgtgccc gatagattca gcggctctaa gagcggcacc 660

agcgccagcc tggccatctc tggcctgcag tctgaggacg aggccgacta ctattgcgcc 720

gcctgggacg acagcctgaa cggcgtggtg tttggcggag gcaccaagct gacagtgctg 780

ggcggaggcg gttcagaagt gcagctggtg gaatccgggg ggggcctggt gcagcctggg 840

gggagcctga gactgagttg tgccgcctct ggggtgacat ttaactacta tggcatgtct 900

tggatccgcc aggcacctgg aaagggcctg gagtgggtgg ccagcatcac taattccggc 960

gggcgaatct actatcccga cagcgtcaag ggcaggttca caatttcccg cgagaacaca 1020

cagaaaactc tgtacctgca gatgaatagc ctgagagccg aagatacagc tgtgtactat 1080

tgcactctgg acggcaggga tgggtgggtc gcctattggg ggcagggaac cctggtgaca 1140

gtcagctccg gaggaggagg atctggcgga ggaggcagtg gggaggcgg gtcaaacttt 1200

atgctcactc agccgtcctc tgtttctggc gtacctggcc aacgggtgac cattagctgt 1260

acgggtaata ccgggaatat cgggtctaac tacgtgaact ggtatcagca gcttccaggg 1320

acagctccca agttgctgat ctatcgcgac gacaaaagac cctcaggggt ccctgaccga 1380

tttagtggca gcaaaagcgg tacttccgct tccctggcga taaccggctt tcaggccgaa 1440

gatgaggcag actactattg ccagtcatat tccagcggct tcatcttcgg aggcggaact 1500

aagctgacag tgttgggcgc ggccgcaggt gcaggtggtg attacaaaga tgatgacgat 1560

aaaggtgcag cggcgcatca ccatcatcac cac 1593

<210> 171
<211> 533
<212> PRT
<213> Artificial Sequence

<220>
<223> amino acid sequence of C2037-C3E-7034

<400> 171

Met Lys His Leu Trp Phe Phe Leu Leu Leu Val Ala Ala Pro Arg Trp
1 5 10 15

Val Leu Ser Gly Gln Val Gln Leu Val Gln Ser Gly Ala Glu Val Lys
20 25 30

Lys Pro Gly Ala Ser Val Lys Val Ser Cys Lys Ala Ser Gly Tyr Thr
35 40 45

Phe Thr Gly Tyr Tyr Met His Trp Val Arg Gln Ala Pro Gly Gln Gly
50 55 60

Leu Glu Trp Met Gly Arg Ile Asn Pro Asn Ser Gly Gly Thr Asn Tyr
65 70 75 80

Ala Gln Lys Phe Gln Gly Arg Val Thr Met Thr Arg Asp Thr Ser Ile
85 90 95

Ser Thr Ala Tyr Met Glu Leu Ser Arg Leu Arg Ser Asp Asp Thr Ala
100 105 110

Val Tyr Tyr Cys Ala Ser Gly Lys Ser Asn Tyr Pro Trp Gly Gln Gly
115 120 125

Thr Leu Val Thr Val Ser Ser Gly Gly Gly Gly Ser Gly Gly Gly Gly
130 135 140

Ser Gly Gly Gly Gly Ser Gln Pro Val Leu Thr Gln Pro Pro Ser Ala
145 150 155 160

```
Ser Gly Thr Pro Gly Gln Arg Val Thr Ile Ser Cys Ser Gly Ser Asn
                165                 170                 175

Ser Asn Ile Gly Ser Arg Thr Val Asn Trp Tyr Gln His Leu Pro Gly
            180                 185                 190

Thr Ala Pro Arg Leu Leu Ile Tyr Gly Asn Asn Gln Trp Pro Ser Gly
        195                 200                 205

Val Pro Asp Arg Phe Ser Gly Ser Lys Ser Gly Ser Ser Ala Ser Leu
    210                 215                 220

Ala Ile Ser Gly Leu Arg Ser Glu Asp Glu Ala Asp Tyr Tyr Cys Ala
225                 230                 235                 240

Ala Trp Asp Asp Ser Leu Ser Gly Val Val Phe Gly Gly Gly Thr Lys
                245                 250                 255

Leu Thr Val Leu Gly Gly Gly Gly Ser Glu Val Gln Leu Val Glu Ser
            260                 265                 270

Gly Gly Gly Leu Val Gln Pro Gly Gly Ser Leu Arg Leu Ser Cys Ala
        275                 280                 285

Ala Ser Gly Val Thr Phe Asn Tyr Tyr Gly Met Ser Trp Ile Arg Gln
    290                 295                 300

Ala Pro Gly Lys Gly Leu Glu Trp Val Ala Ser Ile Thr Asn Ser Gly
305                 310                 315                 320

Gly Arg Ile Tyr Tyr Pro Asp Ser Val Lys Gly Arg Phe Thr Ile Ser
                325                 330                 335

Arg Glu Asn Thr Gln Lys Thr Leu Tyr Leu Gln Met Asn Ser Leu Arg
            340                 345                 350

Ala Glu Asp Thr Ala Val Tyr Tyr Cys Thr Leu Asp Gly Arg Asp Gly
        355                 360                 365

Trp Val Ala Tyr Trp Gly Gln Gly Thr Leu Val Thr Val Ser Ser Gly
    370                 375                 380

Gly Gly Gly Ser Gly Gly Gly Gly Ser Gly Gly Gly Gly Ser Asn Phe
385                 390                 395                 400

Met Leu Thr Gln Pro His Ser Val Ser Glu Ser Pro Gly Lys Thr Val
                405                 410                 415
```

Thr Ile Ser Cys Lys Arg Asn Thr Gly Asn Ile Gly Ser Asn Tyr Val
420 425 430

Asn Trp Tyr Gln Gln His Glu Gly Ser Ser Pro Thr Thr Ile Ile Tyr
435 440 445

Arg Asp Asp Lys Arg Pro Asp Gly Val Ser Asp Arg Phe Ser Gly Ser
450 455 460

Ile Asp Arg Ser Ser Lys Ser Ala Ser Leu Thr Ile Ser Asn Leu Lys
465 470 475 480

Thr Glu Asp Glu Ala Asp Tyr Phe Cys Gln Ser Tyr Ser Ser Gly Phe
485 490 495

Ile Phe Gly Gly Gly Thr Lys Leu Thr Val Leu Gly Ala Ala Ala Gly
500 505 510

Ala Gly Gly Asp Tyr Lys Asp Asp Asp Asp Lys Gly Ala Ala Ala His
515 520 525

His His His His His
530

<210> 172
<211> 541
<212> PRT
<213> Artificial Sequence

<220>
<223> amino acid sequence of C3048-C3E-7034

<400> 172

Met Lys His Leu Trp Phe Phe Leu Leu Leu Val Ala Ala Pro Arg Trp
1 5 10 15

Val Leu Ser Gly Gln Val Gln Leu Val Gln Ser Gly Ala Glu Val Lys
20 25 30

Lys Pro Gly Ser Ser Val Lys Val Ser Cys Lys Ala Ser Gly Gly Thr
35 40 45

Phe Ser Arg Tyr Ala Ile Ser Trp Val Arg Gln Ala Pro Gly Gln Gly
50 55 60

Leu Glu Trp Met Gly Gly Ile Ile Pro Ile Phe Gly Thr Ala Asn Tyr
65 70 75 80

```
Ala Gln Lys Phe Gln Gly Arg Val Thr Ile Thr Ala Asp Gln Ser Thr
                85                  90                  95

Arg Thr Ala Tyr Met Asp Leu Gly Arg Leu Arg Ser Glu Asp Thr Ala
            100                 105                 110

Val Tyr Tyr Cys Ala Leu Glu Gly Phe Arg Asp Ser Leu Lys Arg Asn
        115                 120                 125

Pro Phe Asp Ile Trp Gly Gln Gly Thr Met Val Thr Val Ser Ser Gly
    130                 135                 140

Gly Gly Gly Ser Gly Gly Gly Gly Ser Gly Gly Gly Gly Ser Gln Ser
145                 150                 155                 160

Val Val Thr Gln Pro Pro Ser Val Ser Ala Ala Pro Gly Gln Lys Val
                165                 170                 175

Thr Ile Ser Cys Ser Gly Gly Thr Thr Asn Ile Gly Ser Asn Tyr Val
            180                 185                 190

Ser Trp Tyr Gln Gln Leu Pro Gly Thr Ala Pro Lys Phe Leu Met Tyr
        195                 200                 205

Glu Asn Asn Lys Arg Pro Ser Gly Ile Pro Asp Arg Phe Ser Gly Ser
    210                 215                 220

Lys Ser Gly Thr Ser Ala Thr Leu Ala Ile Thr Gly Leu Gln Thr Gly
225                 230                 235                 240

Asp Glu Ala Thr Tyr Tyr Cys Ser Thr Trp Asp Ser Ser Leu Asn Thr
                245                 250                 255

Gly Leu Phe Gly Gly Gly Thr Lys Leu Thr Val Leu Gly Gly Gly Gly
            260                 265                 270

Ser Glu Val Gln Leu Val Glu Ser Gly Gly Gly Leu Val Gln Pro Gly
        275                 280                 285

Gly Ser Leu Arg Leu Ser Cys Ala Ala Ser Gly Val Thr Phe Asn Tyr
    290                 295                 300

Tyr Gly Met Ser Trp Ile Arg Gln Ala Pro Gly Lys Gly Leu Glu Trp
305                 310                 315                 320

Val Ala Ser Ile Thr Asn Ser Gly Gly Arg Ile Tyr Tyr Pro Asp Ser
                325                 330                 335
```

Val Lys Gly Arg Phe Thr Ile Ser Arg Glu Asn Thr Gln Lys Thr Leu
340 345 350

Tyr Leu Gln Met Asn Ser Leu Arg Ala Glu Asp Thr Ala Val Tyr Tyr
355 360 365

Cys Thr Leu Asp Gly Arg Asp Gly Trp Val Ala Tyr Trp Gly Gln Gly
370 375 380

Thr Leu Val Thr Val Ser Ser Gly Gly Gly Gly Ser Gly Gly Gly Gly
385 390 395 400

Ser Gly Gly Gly Gly Ser Asn Phe Met Leu Thr Gln Pro His Ser Val
405 410 415

Ser Glu Ser Pro Gly Lys Thr Val Thr Ile Ser Cys Lys Arg Asn Thr
420 425 430

Gly Asn Ile Gly Ser Asn Tyr Val Asn Trp Tyr Gln Gln His Glu Gly
435 440 445

Ser Ser Pro Thr Thr Ile Ile Tyr Arg Asp Asp Lys Arg Pro Asp Gly
450 455 460

Val Ser Asp Arg Phe Ser Gly Ser Ile Asp Arg Ser Ser Lys Ser Ala
465 470 475 480

Ser Leu Thr Ile Ser Asn Leu Lys Thr Glu Asp Glu Ala Asp Tyr Phe
485 490 495

Cys Gln Ser Tyr Ser Ser Gly Phe Ile Phe Gly Gly Gly Thr Lys Leu
500 505 510

Thr Val Leu Gly Ala Ala Ala Gly Ala Gly Gly Asp Tyr Lys Asp Asp
515 520 525

Asp Asp Lys Gly Ala Ala Ala His His His His His His
530 535 540

<210> 173
<211> 533
<212> PRT
<213> Artificial Sequence

<220>
<223> amino acid sequence of C3022-C3E-7034

<400> 173

Met Lys His Leu Trp Phe Phe Leu Leu Leu Val Ala Ala Pro Arg Trp
1 5 10 15

Val Leu Ser Gly Gln Val Gln Leu Val Gln Ser Gly Ala Glu Val Lys
20 25 30

Lys Pro Gly Ala Ser Val Lys Val Ser Cys Lys Ala Ser Gly Tyr Thr
35 40 45

Phe Thr Gly Tyr Tyr Met His Trp Val Arg Gln Ala Pro Gly Gln Gly
50 55 60

Leu Glu Trp Met Gly Arg Ile Asn Pro Asn Ser Gly Gly Thr Asn Tyr
65 70 75 80

Ala Gln Lys Phe Gln Gly Arg Val Thr Met Thr Arg Asp Thr Ser Ile
85 90 95

Ser Thr Ala Tyr Met Glu Leu Ser Gly Leu Arg Ser Asp Asp Thr Ala
100 105 110

Val Tyr Tyr Cys Ala Ser Gly Ser Val Arg His Pro Trp Gly Gln Gly
115 120 125

Thr Leu Val Thr Val Ser Ser Gly Gly Gly Gly Ser Gly Gly Gly Gly
130 135 140

Ser Gly Gly Gly Gly Ser Gln Pro Val Leu Thr Gln Pro Pro Ser Ala
145 150 155 160

Ser Gly Thr Pro Gly Gln Arg Val Thr Ile Ser Cys Ser Gly Ser Arg
165 170 175

Ser Asn Ile Gly Gly Asn Ile Val Ser Trp Tyr Gln Gln Phe Pro Gly
180 185 190

Thr Ala Pro Arg Leu Leu Thr Tyr Ala Asp Asn Gln Arg Pro Ser Gly
195 200 205

Val Pro Asp Arg Phe Ser Gly Ser Lys Ser Gly Thr Ser Ala Ser Leu
210 215 220

Ala Ile Ser Gly Leu Gln Ser Glu Asp Glu Ala Asp Tyr Tyr Cys Ala
225 230 235 240

Ala Trp Asp Asp Ser Leu Asn Gly Val Val Phe Gly Gly Gly Thr Lys
245 250 255

```
Leu Thr Val Leu Gly Gly Gly Gly Ser Glu Val Gln Leu Val Glu Ser
            260                 265                 270

Gly Gly Gly Leu Val Gln Pro Gly Gly Ser Leu Arg Leu Ser Cys Ala
        275                 280                 285

Ala Ser Gly Val Thr Phe Asn Tyr Tyr Gly Met Ser Trp Ile Arg Gln
    290                 295                 300

Ala Pro Gly Lys Gly Leu Glu Trp Val Ala Ser Ile Thr Asn Ser Gly
305                 310                 315                 320

Gly Arg Ile Tyr Tyr Pro Asp Ser Val Lys Gly Arg Phe Thr Ile Ser
                325                 330                 335

Arg Glu Asn Thr Gln Lys Thr Leu Tyr Leu Gln Met Asn Ser Leu Arg
            340                 345                 350

Ala Glu Asp Thr Ala Val Tyr Tyr Cys Thr Leu Asp Gly Arg Asp Gly
        355                 360                 365

Trp Val Ala Tyr Trp Gly Gln Gly Thr Leu Val Thr Val Ser Ser Gly
    370                 375                 380

Gly Gly Gly Ser Gly Gly Gly Gly Ser Gly Gly Gly Gly Ser Asn Phe
385                 390                 395                 400

Met Leu Thr Gln Pro His Ser Val Ser Glu Ser Pro Gly Lys Thr Val
                405                 410                 415

Thr Ile Ser Cys Lys Arg Asn Thr Gly Asn Ile Gly Ser Asn Tyr Val
            420                 425                 430

Asn Trp Tyr Gln Gln His Glu Gly Ser Ser Pro Thr Thr Ile Ile Tyr
        435                 440                 445

Arg Asp Asp Lys Arg Pro Asp Gly Val Ser Asp Arg Phe Ser Gly Ser
    450                 455                 460

Ile Asp Arg Ser Ser Lys Ser Ala Ser Leu Thr Ile Ser Asn Leu Lys
465                 470                 475                 480

Thr Glu Asp Glu Ala Asp Tyr Phe Cys Gln Ser Tyr Ser Ser Gly Phe
                485                 490                 495

Ile Phe Gly Gly Gly Thr Lys Leu Thr Val Leu Gly Ala Ala Ala Gly
```

500 505 510

Ala Gly Gly Asp Tyr Lys Asp Asp Asp Asp Lys Gly Ala Ala Ala His
515 520 525

His His His His His
530

<210> 174
<211> 533
<212> PRT
<213> Artificial Sequence

<220>
<223> amino acid sequence of C2037-C3E-7035

<400> 174

Met Lys His Leu Trp Phe Phe Leu Leu Leu Val Ala Ala Pro Arg Trp
1 5 10 15

Val Leu Ser Gly Gln Val Gln Leu Val Gln Ser Gly Ala Glu Val Lys
20 25 30

Lys Pro Gly Ala Ser Val Lys Val Ser Cys Lys Ala Ser Gly Tyr Thr
35 40 45

Phe Thr Gly Tyr Tyr Met His Trp Val Arg Gln Ala Pro Gly Gln Gly
50 55 60

Leu Glu Trp Met Gly Arg Ile Asn Pro Asn Ser Gly Gly Thr Asn Tyr
65 70 75 80

Ala Gln Lys Phe Gln Gly Arg Val Thr Met Thr Arg Asp Thr Ser Ile
85 90 95

Ser Thr Ala Tyr Met Glu Leu Ser Arg Leu Arg Ser Asp Asp Thr Ala
100 105 110

Val Tyr Tyr Cys Ala Ser Gly Lys Ser Asn Tyr Pro Trp Gly Gln Gly
115 120 125

Thr Leu Val Thr Val Ser Ser Gly Gly Gly Gly Ser Gly Gly Gly Gly
130 135 140

Ser Gly Gly Gly Gly Ser Gln Pro Val Leu Thr Gln Pro Pro Ser Ala
145 150 155 160

Ser Gly Thr Pro Gly Gln Arg Val Thr Ile Ser Cys Ser Gly Ser Asn
165 170 175

```
Ser Asn Ile Gly Ser Arg Thr Val Asn Trp Tyr Gln His Leu Pro Gly
            180                 185                 190

Thr Ala Pro Arg Leu Leu Ile Tyr Gly Asn Asn Gln Trp Pro Ser Gly
        195                 200                 205

Val Pro Asp Arg Phe Ser Gly Ser Lys Ser Gly Ser Ser Ala Ser Leu
    210                 215                 220

Ala Ile Ser Gly Leu Arg Ser Glu Asp Glu Ala Asp Tyr Tyr Cys Ala
225                 230                 235                 240

Ala Trp Asp Asp Ser Leu Ser Gly Val Val Phe Gly Gly Gly Thr Lys
                245                 250                 255

Leu Thr Val Leu Gly Gly Gly Gly Ser Glu Val Gln Leu Val Glu Ser
            260                 265                 270

Gly Gly Gly Leu Val Gln Pro Gly Gly Ser Leu Arg Leu Ser Cys Ala
        275                 280                 285

Ala Ser Gly Val Thr Phe Asn Tyr Tyr Gly Met Ser Trp Ile Arg Gln
    290                 295                 300

Ala Pro Gly Lys Gly Leu Glu Trp Val Ala Ser Ile Thr Asn Ser Gly
305                 310                 315                 320

Gly Arg Ile Tyr Tyr Pro Asp Ser Val Lys Gly Arg Phe Thr Ile Ser
                325                 330                 335

Arg Glu Asn Thr Gln Lys Thr Leu Tyr Leu Gln Met Asn Ser Leu Arg
            340                 345                 350

Ala Glu Asp Thr Ala Val Tyr Tyr Cys Thr Leu Asp Gly Arg Asp Gly
        355                 360                 365

Trp Val Ala Tyr Trp Gly Gln Gly Thr Leu Val Thr Val Ser Ser Gly
    370                 375                 380

Gly Gly Gly Ser Gly Gly Gly Gly Ser Gly Gly Gly Gly Ser Met Ala
385                 390                 395                 400

Gln Ala Val Leu Thr Gln Pro Ser Ser Val Ser Gly Val Pro Gly Gln
                405                 410                 415

Arg Val Thr Ile Ser Cys Lys Arg Asn Thr Gly Asn Ile Gly Ser Asn
```

420 425 430

Tyr Val Asn Trp Tyr Gln Gln Leu Pro Gly Thr Ala Pro Lys Leu Leu
435 440 445

Ile Tyr Arg Asp Asp Lys Arg Pro Ser Gly Val Pro Asp Arg Phe Ser
450 455 460

Gly Ser Lys Ser Gly Thr Ser Ala Ser Leu Ala Ile Thr Gly Phe Gln
465 470 475 480

Ala Glu Asp Glu Ala Asp Tyr Tyr Cys Gln Ser Tyr Ser Ser Gly Phe
485 490 495

Ile Phe Gly Gly Gly Thr Lys Leu Thr Val Leu Gly Ala Ala Ala Gly
500 505 510

Ala Gly Gly Asp Tyr Lys Asp Asp Asp Asp Lys Gly Ala Ala Ala His
515 520 525

His His His His His
530

<210> 175
<211> 541
<212> PRT
<213> Artificial Sequence

<220>
<223> amino acid sequence of C3048-C3E-7035

<400> 175

Met Lys His Leu Trp Phe Phe Leu Leu Leu Val Ala Ala Pro Arg Trp
1 5 10 15

Val Leu Ser Gly Gln Val Gln Leu Val Gln Ser Gly Ala Glu Val Lys
20 25 30

Lys Pro Gly Ser Ser Val Lys Val Ser Cys Lys Ala Ser Gly Gly Thr
35 40 45

Phe Ser Arg Tyr Ala Ile Ser Trp Val Arg Gln Ala Pro Gly Gln Gly
50 55 60

Leu Glu Trp Met Gly Gly Ile Ile Pro Ile Phe Gly Thr Ala Asn Tyr
65 70 75 80

Ala Gln Lys Phe Gln Gly Arg Val Thr Ile Thr Ala Asp Gln Ser Thr
85 90 95

```
Arg Thr Ala Tyr Met Asp Leu Gly Arg Leu Arg Ser Glu Asp Thr Ala
            100                 105                 110

Val Tyr Tyr Cys Ala Leu Glu Gly Phe Arg Asp Ser Leu Lys Arg Asn
        115                 120                 125

Pro Phe Asp Ile Trp Gly Gln Gly Thr Met Val Thr Val Ser Ser Gly
    130                 135                 140

Gly Gly Gly Ser Gly Gly Gly Gly Ser Gly Gly Gly Gly Ser Gln Ser
145                 150                 155                 160

Val Val Thr Gln Pro Pro Ser Val Ser Ala Ala Pro Gly Gln Lys Val
                165                 170                 175

Thr Ile Ser Cys Ser Gly Gly Thr Thr Asn Ile Gly Ser Asn Tyr Val
            180                 185                 190

Ser Trp Tyr Gln Gln Leu Pro Gly Thr Ala Pro Lys Phe Leu Met Tyr
        195                 200                 205

Glu Asn Asn Lys Arg Pro Ser Gly Ile Pro Asp Arg Phe Ser Gly Ser
    210                 215                 220

Lys Ser Gly Thr Ser Ala Thr Leu Ala Ile Thr Gly Leu Gln Thr Gly
225                 230                 235                 240

Asp Glu Ala Thr Tyr Tyr Cys Ser Thr Trp Asp Ser Ser Leu Asn Thr
                245                 250                 255

Gly Leu Phe Gly Gly Gly Thr Lys Leu Thr Val Leu Gly Gly Gly Gly
            260                 265                 270

Ser Glu Val Gln Leu Val Glu Ser Gly Gly Gly Leu Val Gln Pro Gly
        275                 280                 285

Gly Ser Leu Arg Leu Ser Cys Ala Ala Ser Gly Val Thr Phe Asn Tyr
    290                 295                 300

Tyr Gly Met Ser Trp Ile Arg Gln Ala Pro Gly Lys Gly Leu Glu Trp
305                 310                 315                 320

Val Ala Ser Ile Thr Asn Ser Gly Gly Arg Ile Tyr Tyr Pro Asp Ser
                325                 330                 335

Val Lys Gly Arg Phe Thr Ile Ser Arg Glu Asn Thr Gln Lys Thr Leu
```

340 345 350

Tyr Leu Gln Met Asn Ser Leu Arg Ala Glu Asp Thr Ala Val Tyr Tyr
355 360 365

Cys Thr Leu Asp Gly Arg Asp Gly Trp Val Ala Tyr Trp Gly Gln Gly
370 375 380

Thr Leu Val Thr Val Ser Ser Gly Gly Gly Gly Ser Gly Gly Gly Gly
385 390 395 400

Ser Gly Gly Gly Gly Ser Met Ala Gln Ala Val Leu Thr Gln Pro Ser
405 410 415

Ser Val Ser Gly Val Pro Gly Gln Arg Val Thr Ile Ser Cys Lys Arg
420 425 430

Asn Thr Gly Asn Ile Gly Ser Asn Tyr Val Asn Trp Tyr Gln Gln Leu
435 440 445

Pro Gly Thr Ala Pro Lys Leu Leu Ile Tyr Arg Asp Asp Lys Arg Pro
450 455 460

Ser Gly Val Pro Asp Arg Phe Ser Gly Ser Lys Ser Gly Thr Ser Ala
465 470 475 480

Ser Leu Ala Ile Thr Gly Phe Gln Ala Glu Asp Glu Ala Asp Tyr Tyr
485 490 495

Cys Gln Ser Tyr Ser Ser Gly Phe Ile Phe Gly Gly Gly Thr Lys Leu
500 505 510

Thr Val Leu Gly Ala Ala Ala Gly Ala Gly Gly Asp Tyr Lys Asp Asp
515 520 525

Asp Asp Lys Gly Ala Ala Ala His His His His His His
530 535 540

<210> 176
<211> 533
<212> PRT
<213> Artificial Sequence

<220>
<223> amino acid sequence of C3022-C3E-7035

<400> 176

Met Lys His Leu Trp Phe Phe Leu Leu Leu Val Ala Ala Pro Arg Trp
1 5 10 15

Val Leu Ser Gly Gln Val Gln Leu Val Gln Ser Gly Ala Glu Val Lys
20 25 30

Lys Pro Gly Ala Ser Val Lys Val Ser Cys Lys Ala Ser Gly Tyr Thr
35 40 45

Phe Thr Gly Tyr Tyr Met His Trp Val Arg Gln Ala Pro Gly Gln Gly
50 55 60

Leu Glu Trp Met Gly Arg Ile Asn Pro Asn Ser Gly Gly Thr Asn Tyr
65 70 75 80

Ala Gln Lys Phe Gln Gly Arg Val Thr Met Thr Arg Asp Thr Ser Ile
85 90 95

Ser Thr Ala Tyr Met Glu Leu Ser Gly Leu Arg Ser Asp Asp Thr Ala
100 105 110

Val Tyr Tyr Cys Ala Ser Gly Ser Val Arg His Pro Trp Gly Gln Gly
115 120 125

Thr Leu Val Thr Val Ser Ser Gly Gly Gly Gly Ser Gly Gly Gly Gly
130 135 140

Ser Gly Gly Gly Gly Ser Gln Pro Val Leu Thr Gln Pro Pro Ser Ala
145 150 155 160

Ser Gly Thr Pro Gly Gln Arg Val Thr Ile Ser Cys Ser Gly Ser Arg
165 170 175

Ser Asn Ile Gly Gly Asn Ile Val Ser Trp Tyr Gln Gln Phe Pro Gly
180 185 190

Thr Ala Pro Arg Leu Leu Thr Tyr Ala Asp Asn Gln Arg Pro Ser Gly
195 200 205

Val Pro Asp Arg Phe Ser Gly Ser Lys Ser Gly Thr Ser Ala Ser Leu
210 215 220

Ala Ile Ser Gly Leu Gln Ser Glu Asp Glu Ala Asp Tyr Tyr Cys Ala
225 230 235 240

Ala Trp Asp Asp Ser Leu Asn Gly Val Val Phe Gly Gly Gly Thr Lys
245 250 255

Leu Thr Val Leu Gly Gly Gly Gly Ser Glu Val Gln Leu Val Glu Ser

260 265 270

Gly Gly Gly Leu Val Gln Pro Gly Gly Ser Leu Arg Leu Ser Cys Ala
275 280 285

Ala Ser Gly Val Thr Phe Asn Tyr Tyr Gly Met Ser Trp Ile Arg Gln
290 295 300

Ala Pro Gly Lys Gly Leu Glu Trp Val Ala Ser Ile Thr Asn Ser Gly
305 310 315 320

Gly Arg Ile Tyr Tyr Pro Asp Ser Val Lys Gly Arg Phe Thr Ile Ser
325 330 335

Arg Glu Asn Thr Gln Lys Thr Leu Tyr Leu Gln Met Asn Ser Leu Arg
340 345 350

Ala Glu Asp Thr Ala Val Tyr Tyr Cys Thr Leu Asp Gly Arg Asp Gly
355 360 365

Trp Val Ala Tyr Trp Gly Gln Gly Thr Leu Val Thr Val Ser Ser Gly
370 375 380

Gly Gly Gly Ser Gly Gly Gly Gly Ser Gly Gly Gly Gly Ser Met Ala
385 390 395 400

Gln Ala Val Leu Thr Gln Pro Ser Ser Val Ser Gly Val Pro Gly Gln
405 410 415

Arg Val Thr Ile Ser Cys Lys Arg Asn Thr Gly Asn Ile Gly Ser Asn
420 425 430

Tyr Val Asn Trp Tyr Gln Gln Leu Pro Gly Thr Ala Pro Lys Leu Leu
435 440 445

Ile Tyr Arg Asp Asp Lys Arg Pro Ser Gly Val Pro Asp Arg Phe Ser
450 455 460

Gly Ser Lys Ser Gly Thr Ser Ala Ser Leu Ala Ile Thr Gly Phe Gln
465 470 475 480

Ala Glu Asp Glu Ala Asp Tyr Tyr Cys Gln Ser Tyr Ser Ser Gly Phe
485 490 495

Ile Phe Gly Gly Gly Thr Lys Leu Thr Val Leu Gly Ala Ala Ala Gly
500 505 510

Ala Gly Gly Asp Tyr Lys Asp Asp Asp Asp Lys Gly Ala Ala Ala His
515 520 525

His His His His His
530

<210> 177
<211> 531
<212> PRT
<213> Artificial Sequence

<220>
<223> amino acid sequence of C2037-C3E-7036

<400> 177

Met Lys His Leu Trp Phe Phe Leu Leu Leu Val Ala Ala Pro Arg Trp
1 5 10 15

Val Leu Ser Gly Gln Val Gln Leu Val Gln Ser Gly Ala Glu Val Lys
20 25 30

Lys Pro Gly Ala Ser Val Lys Val Ser Cys Lys Ala Ser Gly Tyr Thr
35 40 45

Phe Thr Gly Tyr Tyr Met His Trp Val Arg Gln Ala Pro Gly Gln Gly
50 55 60

Leu Glu Trp Met Gly Arg Ile Asn Pro Asn Ser Gly Gly Thr Asn Tyr
65 70 75 80

Ala Gln Lys Phe Gln Gly Arg Val Thr Met Thr Arg Asp Thr Ser Ile
85 90 95

Ser Thr Ala Tyr Met Glu Leu Ser Arg Leu Arg Ser Asp Asp Thr Ala
100 105 110

Val Tyr Tyr Cys Ala Ser Gly Lys Ser Asn Tyr Pro Trp Gly Gln Gly
115 120 125

Thr Leu Val Thr Val Ser Ser Gly Gly Gly Gly Ser Gly Gly Gly Gly
130 135 140

Ser Gly Gly Gly Gly Ser Gln Pro Val Leu Thr Gln Pro Pro Ser Ala
145 150 155 160

Ser Gly Thr Pro Gly Gln Arg Val Thr Ile Ser Cys Ser Gly Ser Asn
165 170 175

Ser Asn Ile Gly Ser Arg Thr Val Asn Trp Tyr Gln His Leu Pro Gly

180 185 190

Thr Ala Pro Arg Leu Leu Ile Tyr Gly Asn Asn Gln Trp Pro Ser Gly
195 200 205

Val Pro Asp Arg Phe Ser Gly Ser Lys Ser Gly Ser Ser Ala Ser Leu
210 215 220

Ala Ile Ser Gly Leu Arg Ser Glu Asp Glu Ala Asp Tyr Tyr Cys Ala
225 230 235 240

Ala Trp Asp Asp Ser Leu Ser Gly Val Val Phe Gly Gly Gly Thr Lys
245 250 255

Leu Thr Val Leu Gly Gly Gly Gly Ser Glu Val Gln Leu Val Glu Ser
260 265 270

Gly Gly Gly Leu Val Gln Pro Gly Gly Ser Leu Arg Leu Ser Cys Ala
275 280 285

Ala Ser Gly Val Thr Phe Asn Tyr Tyr Gly Met Ser Trp Ile Arg Gln
290 295 300

Ala Pro Gly Lys Gly Leu Glu Trp Val Ala Ser Ile Thr Asn Ser Gly
305 310 315 320

Gly Arg Ile Tyr Tyr Pro Asp Ser Val Lys Gly Arg Phe Thr Ile Ser
325 330 335

Arg Glu Asn Thr Gln Lys Thr Leu Tyr Leu Gln Met Asn Ser Leu Arg
340 345 350

Ala Glu Asp Thr Ala Val Tyr Tyr Cys Thr Leu Asp Gly Arg Asp Gly
355 360 365

Trp Val Ala Tyr Trp Gly Gln Gly Thr Leu Val Thr Val Ser Ser Gly
370 375 380

Gly Gly Gly Ser Gly Gly Gly Gly Ser Gly Gly Gly Gly Ser Asn Phe
385 390 395 400

Met Leu Thr Gln Pro Ser Ser Val Ser Gly Val Pro Gly Gln Arg Val
405 410 415

Thr Ile Ser Cys Thr Gly Asn Thr Gly Asn Ile Gly Ser Asn Tyr Val
420 425 430

Asn Trp Tyr Gln Gln Leu Pro Gly Thr Ala Pro Lys Leu Leu Ile Tyr
435 440 445

Arg Asp Asp Lys Arg Pro Ser Gly Val Pro Asp Arg Phe Ser Gly Ser
450 455 460

Lys Ser Gly Thr Ser Ala Ser Leu Ala Ile Thr Gly Phe Gln Ala Glu
465 470 475 480

Asp Glu Ala Asp Tyr Tyr Cys Gln Ser Tyr Ser Ser Gly Phe Ile Phe
485 490 495

Gly Gly Gly Thr Lys Leu Thr Val Leu Gly Ala Ala Ala Gly Ala Gly
500 505 510

Gly Asp Tyr Lys Asp Asp Asp Asp Lys Gly Ala Ala Ala His His His
515 520 525

His His His
530

<210> 178
<211> 539
<212> PRT
<213> Artificial Sequence

<220>
<223> amino acid sequence of C3048-C3E-7036

<400> 178

Met Lys His Leu Trp Phe Phe Leu Leu Leu Val Ala Ala Pro Arg Trp
1 5 10 15

Val Leu Ser Gly Gln Val Gln Leu Val Gln Ser Gly Ala Glu Val Lys
20 25 30

Lys Pro Gly Ser Ser Val Lys Val Ser Cys Lys Ala Ser Gly Gly Thr
35 40 45

Phe Ser Arg Tyr Ala Ile Ser Trp Val Arg Gln Ala Pro Gly Gln Gly
50 55 60

Leu Glu Trp Met Gly Gly Ile Ile Pro Ile Phe Gly Thr Ala Asn Tyr
65 70 75 80

Ala Gln Lys Phe Gln Gly Arg Val Thr Ile Thr Ala Asp Gln Ser Thr
85 90 95

Arg Thr Ala Tyr Met Asp Leu Gly Arg Leu Arg Ser Glu Asp Thr Ala

```
            100                 105                 110

Val Tyr Tyr Cys Ala Leu Glu Gly Phe Arg Asp Ser Leu Lys Arg Asn
        115                 120                 125

Pro Phe Asp Ile Trp Gly Gln Gly Thr Met Val Thr Val Ser Ser Gly
    130                 135                 140

Gly Gly Gly Ser Gly Gly Gly Gly Ser Gly Gly Gly Gly Ser Gln Ser
145                 150                 155                 160

Val Val Thr Gln Pro Pro Ser Val Ser Ala Ala Pro Gly Gln Lys Val
                165                 170                 175

Thr Ile Ser Cys Ser Gly Gly Thr Thr Asn Ile Gly Ser Asn Tyr Val
            180                 185                 190

Ser Trp Tyr Gln Gln Leu Pro Gly Thr Ala Pro Lys Phe Leu Met Tyr
        195                 200                 205

Glu Asn Asn Lys Arg Pro Ser Gly Ile Pro Asp Arg Phe Ser Gly Ser
    210                 215                 220

Lys Ser Gly Thr Ser Ala Thr Leu Ala Ile Thr Gly Leu Gln Thr Gly
225                 230                 235                 240

Asp Glu Ala Thr Tyr Tyr Cys Ser Thr Trp Asp Ser Ser Leu Asn Thr
                245                 250                 255

Gly Leu Phe Gly Gly Gly Thr Lys Leu Thr Val Leu Gly Gly Gly Gly
            260                 265                 270

Ser Glu Val Gln Leu Val Glu Ser Gly Gly Gly Leu Val Gln Pro Gly
        275                 280                 285

Gly Ser Leu Arg Leu Ser Cys Ala Ala Ser Gly Val Thr Phe Asn Tyr
    290                 295                 300

Tyr Gly Met Ser Trp Ile Arg Gln Ala Pro Gly Lys Gly Leu Glu Trp
305                 310                 315                 320

Val Ala Ser Ile Thr Asn Ser Gly Gly Arg Ile Tyr Tyr Pro Asp Ser
                325                 330                 335

Val Lys Gly Arg Phe Thr Ile Ser Arg Glu Asn Thr Gln Lys Thr Leu
            340                 345                 350
```

Tyr Leu Gln Met Asn Ser Leu Arg Ala Glu Asp Thr Ala Val Tyr Tyr
355 360 365

Cys Thr Leu Asp Gly Arg Asp Gly Trp Val Ala Tyr Trp Gly Gln Gly
370 375 380

Thr Leu Val Thr Val Ser Ser Gly Gly Gly Gly Ser Gly Gly Gly Gly
385 390 395 400

Ser Gly Gly Gly Gly Ser Asn Phe Met Leu Thr Gln Pro Ser Ser Val
405 410 415

Ser Gly Val Pro Gly Gln Arg Val Thr Ile Ser Cys Thr Gly Asn Thr
420 425 430

Gly Asn Ile Gly Ser Asn Tyr Val Asn Trp Tyr Gln Gln Leu Pro Gly
435 440 445

Thr Ala Pro Lys Leu Leu Ile Tyr Arg Asp Asp Lys Arg Pro Ser Gly
450 455 460

Val Pro Asp Arg Phe Ser Gly Ser Lys Ser Gly Thr Ser Ala Ser Leu
465 470 475 480

Ala Ile Thr Gly Phe Gln Ala Glu Asp Glu Ala Asp Tyr Tyr Cys Gln
485 490 495

Ser Tyr Ser Ser Gly Phe Ile Phe Gly Gly Gly Thr Lys Leu Thr Val
500 505 510

Leu Gly Ala Ala Ala Gly Ala Gly Gly Asp Tyr Lys Asp Asp Asp Asp
515 520 525

Lys Gly Ala Ala Ala His His His His His His
530 535

<210> 179
<211> 531
<212> PRT
<213> Artificial Sequence

<220>
<223> amino acid sequence of C3022-C3E-7036

<400> 179

Met Lys His Leu Trp Phe Phe Leu Leu Leu Val Ala Ala Pro Arg Trp
1 5 10 15

Val Leu Ser Gly Gln Val Gln Leu Val Gln Ser Gly Ala Glu Val Lys

20 25 30

Lys Pro Gly Ala Ser Val Lys Val Ser Cys Lys Ala Ser Gly Tyr Thr
35 40 45

Phe Thr Gly Tyr Tyr Met His Trp Val Arg Gln Ala Pro Gly Gln Gly
50 55 60

Leu Glu Trp Met Gly Arg Ile Asn Pro Asn Ser Gly Gly Thr Asn Tyr
65 70 75 80

Ala Gln Lys Phe Gln Gly Arg Val Thr Met Thr Arg Asp Thr Ser Ile
85 90 95

Ser Thr Ala Tyr Met Glu Leu Ser Gly Leu Arg Ser Asp Asp Thr Ala
100 105 110

Val Tyr Tyr Cys Ala Ser Gly Ser Val Arg His Pro Trp Gly Gln Gly
115 120 125

Thr Leu Val Thr Val Ser Ser Gly Gly Gly Gly Ser Gly Gly Gly Gly
130 135 140

Ser Gly Gly Gly Gly Ser Gln Pro Val Leu Thr Gln Pro Pro Ser Ala
145 150 155 160

Ser Gly Thr Pro Gly Gln Arg Val Thr Ile Ser Cys Ser Gly Ser Arg
165 170 175

Ser Asn Ile Gly Gly Asn Ile Val Ser Trp Tyr Gln Gln Phe Pro Gly
180 185 190

Thr Ala Pro Arg Leu Leu Thr Tyr Ala Asp Asn Gln Arg Pro Ser Gly
195 200 205

Val Pro Asp Arg Phe Ser Gly Ser Lys Ser Gly Thr Ser Ala Ser Leu
210 215 220

Ala Ile Ser Gly Leu Gln Ser Glu Asp Glu Ala Asp Tyr Tyr Cys Ala
225 230 235 240

Ala Trp Asp Asp Ser Leu Asn Gly Val Val Phe Gly Gly Gly Thr Lys
245 250 255

Leu Thr Val Leu Gly Gly Gly Gly Ser Glu Val Gln Leu Val Glu Ser
260 265 270

Gly Gly Gly Leu Val Gln Pro Gly Gly Ser Leu Arg Leu Ser Cys Ala
275 280 285

Ala Ser Gly Val Thr Phe Asn Tyr Tyr Gly Met Ser Trp Ile Arg Gln
290 295 300

Ala Pro Gly Lys Gly Leu Glu Trp Val Ala Ser Ile Thr Asn Ser Gly
305 310 315 320

Gly Arg Ile Tyr Tyr Pro Asp Ser Val Lys Gly Arg Phe Thr Ile Ser
325 330 335

Arg Glu Asn Thr Gln Lys Thr Leu Tyr Leu Gln Met Asn Ser Leu Arg
340 345 350

Ala Glu Asp Thr Ala Val Tyr Tyr Cys Thr Leu Asp Gly Arg Asp Gly
355 360 365

Trp Val Ala Tyr Trp Gly Gln Gly Thr Leu Val Thr Val Ser Ser Gly
370 375 380

Gly Gly Gly Ser Gly Gly Gly Gly Ser Gly Gly Gly Gly Ser Asn Phe
385 390 395 400

Met Leu Thr Gln Pro Ser Ser Val Ser Gly Val Pro Gly Gln Arg Val
405 410 415

Thr Ile Ser Cys Thr Gly Asn Thr Gly Asn Ile Gly Ser Asn Tyr Val
420 425 430

Asn Trp Tyr Gln Gln Leu Pro Gly Thr Ala Pro Lys Leu Leu Ile Tyr
435 440 445

Arg Asp Asp Lys Arg Pro Ser Gly Val Pro Asp Arg Phe Ser Gly Ser
450 455 460

Lys Ser Gly Thr Ser Ala Ser Leu Ala Ile Thr Gly Phe Gln Ala Glu
465 470 475 480

Asp Glu Ala Asp Tyr Tyr Cys Gln Ser Tyr Ser Ser Gly Phe Ile Phe
485 490 495

Gly Gly Gly Thr Lys Leu Thr Val Leu Gly Ala Ala Ala Gly Ala Gly
500 505 510

Gly Asp Tyr Lys Asp Asp Asp Asp Lys Gly Ala Ala Ala His His His
515 520 525

```
His His His
    530


<210>  180
<211>  269
<212>  PRT
<213>  Artificial Sequence

<220>
<223>  amino acid sequence of C3E-7034

<400>  180

Gly Glu Val Gln Leu Val Glu Ser Gly Gly Gly Leu Val Gln Pro Gly
1               5                   10                  15


Gly Ser Leu Arg Leu Ser Cys Ala Ala Ser Gly Val Thr Phe Asn Tyr
            20                  25                  30


Tyr Gly Met Ser Trp Ile Arg Gln Ala Pro Gly Lys Gly Leu Glu Trp
        35                  40                  45


Val Ala Ser Ile Thr Asn Ser Gly Gly Arg Ile Tyr Tyr Pro Asp Ser
    50                  55                  60


Val Lys Gly Arg Phe Thr Ile Ser Arg Glu Asn Thr Gln Lys Thr Leu
65                  70                  75                  80


Tyr Leu Gln Met Asn Ser Leu Arg Ala Glu Asp Thr Ala Val Tyr Tyr
                85                  90                  95


Cys Thr Leu Asp Gly Arg Asp Gly Trp Val Ala Tyr Trp Gly Gln Gly
            100                 105                 110


Thr Leu Val Thr Val Ser Ser Gly Gly Gly Gly Ser Gly Gly Gly Gly
        115                 120                 125


Ser Gly Gly Gly Gly Ser Asn Phe Met Leu Thr Gln Pro His Ser Val
    130                 135                 140


Ser Glu Ser Pro Gly Lys Thr Val Thr Ile Ser Cys Lys Arg Asn Thr
145                 150                 155                 160


Gly Asn Ile Gly Ser Asn Tyr Val Asn Trp Tyr Gln Gln His Glu Gly
                165                 170                 175


Ser Ser Pro Thr Thr Ile Ile Tyr Arg Asp Asp Lys Arg Pro Asp Gly
            180                 185                 190
```

Val Ser Asp Arg Phe Ser Gly Ser Ile Asp Arg Ser Ser Lys Ser Ala
195 200 205

Ser Leu Thr Ile Ser Asn Leu Lys Thr Glu Asp Glu Ala Asp Tyr Phe
210 215 220

Cys Gln Ser Tyr Ser Ser Gly Phe Ile Phe Gly Gly Gly Thr Lys Leu
225 230 235 240

Thr Val Leu Gly Ala Ala Ala Gly Ala Gly Gly Asp Tyr Lys Asp Asp
245 250 255

Asp Asp Lys Gly Ala Ala Ala His His His His His His
260 265

<210> 181
<211> 269
<212> PRT
<213> Artificial Sequence

<220>
<223> amino acid sequence of C3E-7035

<400> 181

Gly Glu Val Gln Leu Val Glu Ser Gly Gly Gly Leu Val Gln Pro Gly
1 5 10 15

Gly Ser Leu Arg Leu Ser Cys Ala Ala Ser Gly Val Thr Phe Asn Tyr
20 25 30

Tyr Gly Met Ser Trp Ile Arg Gln Ala Pro Gly Lys Gly Leu Glu Trp
35 40 45

Val Ala Ser Ile Thr Asn Ser Gly Gly Arg Ile Tyr Tyr Pro Asp Ser
50 55 60

Val Lys Gly Arg Phe Thr Ile Ser Arg Glu Asn Thr Gln Lys Thr Leu
65 70 75 80

Tyr Leu Gln Met Asn Ser Leu Arg Ala Glu Asp Thr Ala Val Tyr Tyr
85 90 95

Cys Thr Leu Asp Gly Arg Asp Gly Trp Val Ala Tyr Trp Gly Gln Gly
100 105 110

Thr Leu Val Thr Val Ser Ser Gly Gly Gly Gly Ser Gly Gly Gly Gly
115 120 125

Ser Gly Gly Gly Gly Ser Met Ala Gln Ala Val Leu Thr Gln Pro Ser

```
    130                 135                 140

Ser Val Ser Gly Val Pro Gly Gln Arg Val Thr Ile Ser Cys Lys Arg
145                 150                 155                 160

Asn Thr Gly Asn Ile Gly Ser Asn Tyr Val Asn Trp Tyr Gln Gln Leu
                165                 170                 175

Pro Gly Thr Ala Pro Lys Leu Leu Ile Tyr Arg Asp Asp Lys Arg Pro
            180                 185                 190

Ser Gly Val Pro Asp Arg Phe Ser Gly Ser Lys Ser Gly Thr Ser Ala
        195                 200                 205

Ser Leu Ala Ile Thr Gly Phe Gln Ala Glu Asp Glu Ala Asp Tyr Tyr
    210                 215                 220

Cys Gln Ser Tyr Ser Ser Gly Phe Ile Phe Gly Gly Gly Thr Lys Leu
225                 230                 235                 240

Thr Val Leu Gly Ala Ala Ala Gly Ala Gly Gly Asp Tyr Lys Asp Asp
                245                 250                 255

Asp Asp Lys Gly Ala Ala Ala His His His His His His
            260                 265


<210>  182
<211>  267
<212>  PRT
<213>  Artificial Sequence

<220>
<223>  amino acid sequence of C3E-7036

<400>  182

Gly Glu Val Gln Leu Val Glu Ser Gly Gly Gly Leu Val Gln Pro Gly
1               5                   10                  15

Gly Ser Leu Arg Leu Ser Cys Ala Ala Ser Gly Val Thr Phe Asn Tyr
            20                  25                  30

Tyr Gly Met Ser Trp Ile Arg Gln Ala Pro Gly Lys Gly Leu Glu Trp
        35                  40                  45

Val Ala Ser Ile Thr Asn Ser Gly Gly Arg Ile Tyr Tyr Pro Asp Ser
    50                  55                  60

Val Lys Gly Arg Phe Thr Ile Ser Arg Glu Asn Thr Gln Lys Thr Leu
65                  70                  75                  80
```

Tyr Leu Gln Met Asn Ser Leu Arg Ala Glu Asp Thr Ala Val Tyr Tyr
85 90 95

Cys Thr Leu Asp Gly Arg Asp Gly Trp Val Ala Tyr Trp Gly Gln Gly
100 105 110

Thr Leu Val Thr Val Ser Ser Gly Gly Gly Gly Ser Gly Gly Gly Gly
115 120 125

Ser Gly Gly Gly Gly Ser Asn Phe Met Leu Thr Gln Pro Ser Ser Val
130 135 140

Ser Gly Val Pro Gly Gln Arg Val Thr Ile Ser Cys Thr Gly Asn Thr
145 150 155 160

Gly Asn Ile Gly Ser Asn Tyr Val Asn Trp Tyr Gln Gln Leu Pro Gly
165 170 175

Thr Ala Pro Lys Leu Leu Ile Tyr Arg Asp Asp Lys Arg Pro Ser Gly
180 185 190

Val Pro Asp Arg Phe Ser Gly Ser Lys Ser Gly Thr Ser Ala Ser Leu
195 200 205

Ala Ile Thr Gly Phe Gln Ala Glu Asp Glu Ala Asp Tyr Tyr Cys Gln
210 215 220

Ser Tyr Ser Ser Gly Phe Ile Phe Gly Gly Gly Thr Lys Leu Thr Val
225 230 235 240

Leu Gly Ala Ala Ala Gly Ala Gly Gly Asp Tyr Lys Asp Asp Asp Asp
245 250 255

Lys Gly Ala Ala Ala His His His His His His
260 265

<210> 183
<211> 8
<212> PRT
<213> Artificial Sequence

<220>
<223> amino acid sequence of C3E-7000_H_CDR1

<400> 183

Gly Val Thr Phe Asn Tyr Tyr Gly
1 5

<210> 184
<211> 8
<212> PRT
<213> Artificial Sequence

<220>
<223> amino acid sequence of C3E-7000_H_CDR2

<400> 184

Ile Thr Asn Ser Gly Gly Arg Ile
1               5

<210> 185
<211> 11
<212> PRT
<213> Artificial Sequence

<220>
<223> amino acid sequence of C3E-7000_H_CDR3

<400> 185

Thr Leu Asp Gly Arg Asp Gly Trp Val Ala Tyr
1               5                   10

<210> 186
<211> 8
<212> PRT
<213> Artificial Sequence

<220>
<223> amino acid sequence of C3E-7000_L_CDR1

<400> 186

Thr Gly Asn Ile Gly Ser Asn Tyr
1               5

<210> 187
<211> 3
<212> PRT
<213> Artificial Sequence

<220>
<223> amino acid sequence of C3E-7000_L_CDR2

<400> 187

Arg Asp Asp
1

<210> 188
<211> 8
<212> PRT
<213> Artificial Sequence

<220>

<223> amino acid sequence of C3E-7000_L_CDR3

<400> 188

```
Gln Ser Tyr Ser Ser Gly Phe Ile
1               5
```

<210> 189
<211> 207
<212> PRT
<213> Homo sapiens

<400> 189

```
Met Gln Ser Gly Thr His Trp Arg Val Leu Gly Leu Cys Leu Leu Ser
1               5                   10                  15

Val Gly Val Trp Gly Gln Asp Gly Asn Glu Glu Met Gly Gly Ile Thr
            20                  25                  30

Gln Thr Pro Tyr Lys Val Ser Ile Ser Gly Thr Thr Val Ile Leu Thr
        35                  40                  45

Cys Pro Gln Tyr Pro Gly Ser Glu Ile Leu Trp Gln His Asn Asp Lys
    50                  55                  60

Asn Ile Gly Gly Asp Glu Asp Asp Lys Asn Ile Gly Ser Asp Glu Asp
65                  70                  75                  80

His Leu Ser Leu Lys Glu Phe Ser Glu Leu Glu Gln Ser Gly Tyr Tyr
                85                  90                  95

Val Cys Tyr Pro Arg Gly Ser Lys Pro Glu Asp Ala Asn Phe Tyr Leu
            100                 105                 110

Tyr Leu Arg Ala Arg Val Cys Glu Asn Cys Met Glu Met Asp Val Met
        115                 120                 125

Ser Val Ala Thr Ile Val Ile Val Asp Ile Cys Ile Thr Gly Gly Leu
    130                 135                 140

Leu Leu Leu Val Tyr Tyr Trp Ser Lys Asn Arg Lys Ala Lys Ala Lys
145                 150                 155                 160

Pro Val Thr Arg Gly Ala Gly Ala Gly Gly Arg Gln Arg Gly Gln Asn
                165                 170                 175

Lys Glu Arg Pro Pro Pro Val Pro Asn Pro Asp Tyr Glu Pro Ile Arg
            180                 185                 190
```

```
Lys Gly Gln Arg Asp Leu Tyr Ser Gly Leu Asn Gln Arg Arg Ile
        195                 200                 205


<210>  190
<211>  345
<212>  DNA
<213>  Artificial Sequence

<220>
<223>  nucleotide sequence of E1018 VH

<400>  190
caggtgcagc tggtgcagtc tggggctgag gtgaagaagc ctggggcctc agtgaaggtc      60

tcctgcaagg cttctggata caccttcacc ggctactata tgcactgggt gcgacaggcc     120

cctggacaag ggcttgagtg gatgggacgg atcaacccta acagtggtgg cacaaactat     180

gcacagaagt ttcagggcag ggtcaccatg accagggaca cgtccatcag cacagcctac     240

atggagctga gcgggctgag atctgacgac acggccgttt attactgtaa gagtggtagt     300

gtaaggcctc cctggggcca gggaaccctg gtcaccgtct cctca                     345


<210>  191
<211>  115
<212>  PRT
<213>  Artificial Sequence

<220>
<223>  amino acid sequence of E1018 VH

<400>  191

Gln Val Gln Leu Val Gln Ser Gly Ala Glu Val Lys Lys Pro Gly Ala
1               5                   10                  15


Ser Val Lys Val Ser Cys Lys Ala Ser Gly Tyr Thr Phe Thr Gly Tyr
            20                  25                  30


Tyr Met His Trp Val Arg Gln Ala Pro Gly Gln Gly Leu Glu Trp Met
        35                  40                  45


Gly Arg Ile Asn Pro Asn Ser Gly Gly Thr Asn Tyr Ala Gln Lys Phe
    50                  55                  60


Gln Gly Arg Val Thr Met Thr Arg Asp Thr Ser Ile Ser Thr Ala Tyr
65                  70                  75                  80


Met Glu Leu Ser Gly Leu Arg Ser Asp Asp Thr Ala Val Tyr Tyr Cys
                85                  90                  95


Lys Ser Gly Ser Val Arg Pro Pro Trp Gly Gln Gly Thr Leu Val Thr
            100                 105                 110
```

Val Ser Ser
        115

<210>  192
<211>  330
<212>  DNA
<213>  Artificial Sequence

<220>
<223>  nucleotide sequence of E1018 VL

<400>  192
cagcctgtgc tgactcagcc accctcagcg tctgggaccc ccgggcagag ggtcaccatc        60

tcttgttctg gaagcaggtc caacatcgga ggtaatattg taagttggta ccagcagttc       120

ccaggaacgg cccccagact cctcacttat cggaatactc ggcggccctc aggggtccct       180

gaccgattct ctggctccaa gtctggcacc tcagcctccc tggccatcag tggcctccag       240

tctgaggatg aggctgacta ttattgtgca gcatgggatg acagcctggg gggtgttgtg       300

ttcggaggag gcaccaagct gaccgtccta                                        330

<210>  193
<211>  110
<212>  PRT
<213>  Artificial Sequence

<220>
<223>  amino acid sequence of E1018 VL

<400>  193

Gln Pro Val Leu Thr Gln Pro Pro Ser Ala Ser Gly Thr Pro Gly Gln
1               5                   10                  15

Arg Val Thr Ile Ser Cys Ser Gly Ser Arg Ser Asn Ile Gly Gly Asn
            20                  25                  30

Ile Val Ser Trp Tyr Gln Gln Phe Pro Gly Thr Ala Pro Arg Leu Leu
        35                  40                  45

Thr Tyr Arg Asn Thr Arg Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Tyr Cys Ala Ala Trp Asp Asp Ser Leu
                85                  90                  95

Gly Gly Val Val Phe Gly Gly Gly Thr Lys Leu Thr Val Leu
            100                 105                 110

<210> 194
<211> 369
<212> DNA
<213> Artificial Sequence

<220>
<223> nucleotide sequence of D1012 VH

<400> 194
caggttcagc tggttcagtc tggcgccgaa gtgaagaaac ctggcagcag cgtgaaggtg 60

tcctgcaaag cttctggcgg caccctgagc agatacgcca tctcttgggt tcgacaggcc 120

cctggacaag gcctggaatg gatgggaggc atcatcccca tcttcggcac cgccaattac 180

gcccagaaat tccagggcag agtgaccatc accgccgacc agtctaccag aaccgcctac 240

atggagctgg gcagactgag aagcgaggac accgccgtgt actactgtgc tctggaaggc 300

ttccgggaca gcctgaagag aaaccccgtg gacatctggg gcagaggcac catggttaca 360

gtgtctagc 369

<210> 195
<211> 123
<212> PRT
<213> Artificial Sequence

<220>
<223> amino acid sequence of D1012 VH

<400> 195

Gln Val Gln Leu Val Gln Ser Gly Ala Glu Val Lys Lys Pro Gly Ser
1 5 10 15

Ser Val Lys Val Ser Cys Lys Ala Ser Gly Gly Thr Leu Ser Arg Tyr
20 25 30

Ala Ile Ser Trp Val Arg Gln Ala Pro Gly Gln Gly Leu Glu Trp Met
35 40 45

Gly Gly Ile Ile Pro Ile Phe Gly Thr Ala Asn Tyr Ala Gln Lys Phe
50 55 60

Gln Gly Arg Val Thr Ile Thr Ala Asp Gln Ser Thr Arg Thr Ala Tyr
65 70 75 80

Met Glu Leu Gly Arg Leu Arg Ser Glu Asp Thr Ala Val Tyr Tyr Cys
85 90 95

Ala Leu Glu Gly Phe Arg Asp Ser Leu Lys Arg Asn Pro Val Asp Ile
100 105 110

```
Trp Gly Arg Gly Thr Met Val Thr Val Ser Ser
        115                 120


<210>  196
<211>  330
<212>  DNA
<213>  Artificial Sequence

<220>
<223>  nucleotide sequence of D1012 VL

<400>  196
caatctgtgg tcacacagcc tccaagcgtg tcagctgccc caggccagaa agtgacaatc      60

ccttgtagcg gcggcaccac caacatcggc agcaattacg tgtcctggta tcagcagctg     120

cccggaacag cccctaagtt cctgatgtac gagaacaaca agcggcccag cggcatcccc     180

gatagatttt ctggcagcaa gagcggcacc agcgccacac tggctattac aggactggag     240

acagaggacg aggccaccta ctactgtagc acctgggaca gcagcctgaa cgccggactt     300

tttggaggcg gcacagagct gacagttctt                                      330


<210>  197
<211>  110
<212>  PRT
<213>  Artificial Sequence

<220>
<223>  amino acid sequence of D1012 VL

<400>  197

Gln Ser Val Val Thr Gln Pro Pro Ser Val Ser Ala Ala Pro Gly Gln
1               5                   10                  15


Lys Val Thr Ile Pro Cys Ser Gly Gly Thr Thr Asn Ile Gly Ser Asn
            20                  25                  30


Tyr Val Ser Trp Tyr Gln Gln Leu Pro Gly Thr Ala Pro Lys Phe Leu
        35                  40                  45


Met Tyr Glu Asn Asn Lys Arg Pro Ser Gly Ile Pro Asp Arg Phe Ser
    50                  55                  60


Gly Ser Lys Ser Gly Thr Ser Ala Thr Leu Ala Ile Thr Gly Leu Glu
65                  70                  75                  80


Thr Glu Asp Glu Ala Thr Tyr Tyr Cys Ser Thr Trp Asp Ser Ser Leu
                85                  90                  95


Asn Ala Gly Leu Phe Gly Gly Gly Thr Glu Leu Thr Val Leu
            100                 105                 110
```

<210> 198
<211> 1425
<212> DNA
<213> Artificial Sequence

<220>
<223> nucleotide sequence of h2B1 Fab HC_1

<400> 198
atgaggccta cctgggcctg gtggctgttc ctggtgctgc tgctggccct gtgggccccc 60

gcccggggcc aggtgcagct ggtggagtcc ggcggcggcc tggtgaagcc cagccagacc 120

ctgtccctga cctgcacatt cagcggcttt tccctgaaca catacgatat gggagtggga 180

tggatcagac agccacctgg caagggcctg gagtggatcg gcaacatctg gtgggacgat 240

gacaagtact ataatccctc tctgaagagc agagtgacca tctctaagga tacaagcaac 300

aatcaggcct ccctgaagct gagctccgtg accgccgccg acacagccgt gtactattgc 360

gccaggatcg agacagtgcg ggtgagccgg aagggattcg cacactgggg acagggcacc 420

ctggtgacag tgtctagcgc tagcacaaag ggacctagcg tgttcccact ggccccttcc 480

tctaagtcca cctctggagg aacagccgcc ctgggctgta gggtgaagga ctatttccct 540

gagccagtga ccgtgtcctg gaactctggg gccctgacca gcggagtgca cacatttcct 600

gccgtgctgc agagctccgg cctgtacagc ctgtctagcg tggtgaccgt gccatcctct 660

agcctgggca cccagacata tatctgcaac gtgaatcaca agccaagcaa tacaaaggtc 720

gacaagcggg tggagcccaa gtcctgtgac aagacccaca catgcccacc ctgtccggcg 780

ccagaggctg caggaggacc aagcgtgttc ctgtttccac ccaagcctaa agacacactg 840

atgatttccc gaacccccga agtcacatgc gtggtcgtgt ctgtgagtca cgaggaccct 900

gaagtcaagt tcaactggta cgtggatggc gtcgaggtgc ataatgccaa gactaaacct 960

agggaggaac agtacaactc aacctatcgc gtcgtgagcg tcctgacagt gctgcaccag 1020

gattggctga acggcaaaga atataagtgc aaagtgagca ataaggccct gcccgctcct 1080

atcgagaaaa ccatttccaa ggctaaaggg cagcctcgcg aaccacaggt ctacgtgctg 1140

ccccctagcc gcgacgaact gactaaaaat caggtctctc tgctgtgtct ggtcaaagga 1200

ttctaccctt ccgacatcgc cgtggagtgg gaaagtaacg gccagcccga gaacaattac 1260

ctgacctggc cccctgtgct ggactctgat gggagtttct ttctgtattc aaagctgaca 1320

gtcgataaaa gccggtggca gcagggcaat gtgttcagct gctccgtcat gcacgaagca 1380

ctgcacaacc attacactca gaagtccctg tccctgtcac ctggc 1425

<210> 199
<211> 475
<212> PRT

<213>  Artificial Sequence

<220>
<223>  amino acid sequence of H2B1 Fab HC_1

<400>  199

```
Met Arg Pro Thr Trp Ala Trp Trp Leu Phe Leu Val Leu Leu Leu Ala
1               5                   10                  15

Leu Trp Ala Pro Ala Arg Gly Gln Val Gln Leu Val Glu Ser Gly Gly
            20                  25                  30

Gly Leu Val Lys Pro Ser Gln Thr Leu Ser Leu Thr Cys Thr Phe Ser
        35                  40                  45

Gly Phe Ser Leu Asn Thr Tyr Asp Met Gly Val Gly Trp Ile Arg Gln
    50                  55                  60

Pro Pro Gly Lys Gly Leu Glu Trp Ile Gly Asn Ile Trp Trp Asp Asp
65                  70                  75                  80

Asp Lys Tyr Tyr Asn Pro Ser Leu Lys Ser Arg Val Thr Ile Ser Lys
                85                  90                  95

Asp Thr Ser Asn Asn Gln Ala Ser Leu Lys Leu Ser Ser Val Thr Ala
            100                 105                 110

Ala Asp Thr Ala Val Tyr Tyr Cys Ala Arg Ile Glu Thr Val Arg Val
        115                 120                 125

Ser Arg Lys Gly Phe Ala His Trp Gly Gln Gly Thr Leu Val Thr Val
    130                 135                 140

Ser Ser Ala Ser Thr Lys Gly Pro Ser Val Phe Pro Leu Ala Pro Ser
145                 150                 155                 160

Ser Lys Ser Thr Ser Gly Gly Thr Ala Ala Leu Gly Cys Arg Val Lys
                165                 170                 175

Asp Tyr Phe Pro Glu Pro Val Thr Val Ser Trp Asn Ser Gly Ala Leu
            180                 185                 190

Thr Ser Gly Val His Thr Phe Pro Ala Val Leu Gln Ser Ser Gly Leu
        195                 200                 205

Tyr Ser Leu Ser Ser Val Val Thr Val Pro Ser Ser Ser Leu Gly Thr
    210                 215                 220
```

Gln Thr Tyr Ile Cys Asn Val Asn His Lys Pro Ser Asn Thr Lys Val
225 230 235 240

Asp Lys Arg Val Glu Pro Lys Ser Cys Asp Lys Thr His Thr Cys Pro
245 250 255

Pro Cys Pro Ala Pro Glu Ala Ala Gly Gly Pro Ser Val Phe Leu Phe
260 265 270

Pro Pro Lys Pro Lys Asp Thr Leu Met Ile Ser Arg Thr Pro Glu Val
275 280 285

Thr Cys Val Val Val Ser Val Ser His Glu Asp Pro Glu Val Lys Phe
290 295 300

Asn Trp Tyr Val Asp Gly Val Glu Val His Asn Ala Lys Thr Lys Pro
305 310 315 320

Arg Glu Glu Gln Tyr Asn Ser Thr Tyr Arg Val Val Ser Val Leu Thr
325 330 335

Val Leu His Gln Asp Trp Leu Asn Gly Lys Glu Tyr Lys Cys Lys Val
340 345 350

Ser Asn Lys Ala Leu Pro Ala Pro Ile Glu Lys Thr Ile Ser Lys Ala
355 360 365

Lys Gly Gln Pro Arg Glu Pro Gln Val Tyr Val Leu Pro Pro Ser Arg
370 375 380

Asp Glu Leu Thr Lys Asn Gln Val Ser Leu Leu Cys Leu Val Lys Gly
385 390 395 400

Phe Tyr Pro Ser Asp Ile Ala Val Glu Trp Glu Ser Asn Gly Gln Pro
405 410 415

Glu Asn Asn Tyr Leu Thr Trp Pro Pro Val Leu Asp Ser Asp Gly Ser
420 425 430

Phe Phe Leu Tyr Ser Lys Leu Thr Val Asp Lys Ser Arg Trp Gln Gln
435 440 445

Gly Asn Val Phe Ser Cys Ser Val Met His Glu Ala Leu His Asn His
450 455 460

Tyr Thr Gln Lys Ser Leu Ser Leu Ser Pro Gly
465 470 475

<210> 200
<211> 1425
<212> DNA
<213> Artificial Sequence

<220>
<223> nucleotide_sequence_of_h2B1_Fab_HC_2

<400> 200
atgaggccta cctgggcctg gtggctgttc ctggtgctgc tgctggccct gtgggccccc 60

gcccggggcc aggtgcagct ggtggagtcc ggcggcggcc tggtgaagcc cagccagacc 120

ctgtccctga cctgcacatt cagcggcttt tccctgaaca catacgatat gggagtggga 180

tggatcagac agccacctgg caagggcctg gagtggatcg gcaacatctg gtgggacgat 240

gacaagtact ataatccctc tctgaagagc agagtgacca tctctaagga tacaagcaac 300

aatcaggcct ccctgaagct gagctccgtg accgccgccg acacagccgt gtactattgc 360

gccaggatcg agacagtgcg ggtgagccgg aagggattcg cacactgggg acagggcacc 420

ctggtgacag tgtctagcgc tagcaccaag ggacctagcg tgttcccaga ggccccttcc 480

tctaagtcca cctctggagg aacagccgcc ctgggctgtc tggtgaccga ctatttccct 540

gagccagtga cagtgtcctg gaactctggg gccctgacca gcggagtgca cacatttcct 600

gccgtgctgg agagctccgg cctgtacagc ctgtctagcg tggtgaccgt gccatcctct 660

agcctgggca cccagacata tatctgcaac gtgaatcaca agccaagcaa tacaaaggtc 720

gacaagagag tggagcccaa gtcctgtgac aagacccaca catgcccacc ctgtccggcg 780

ccagaggctg caggaggacc aagcgtgttc ctgtttccac ccaagcctaa agacacactg 840

atgatttccc gaacccccga agtcacatgc gtggtcgtgt ctgtgagtca cgaggaccct 900

gaagtcaagt tcaactggta cgtggatggc gtcgaggtgc ataatgccaa gactaaacct 960

agggaggaac agtacaactc aacctatcgc gtcgtgagcg tcctgacagt gctgcaccag 1020

gattggctga acggcaaaga atataagtgc aaagtgagca ataaggccct gcccgctcct 1080

atcgagaaaa ccatttccaa ggctaaaggg cagcctcgcg aaccacaggt ctacgtgctg 1140

ccccctagcc gcgacgaact gactaaaaat caggtctctc tgctgtgtct ggtcaaagga 1200

ttctaccctt ccgacatcgc cgtggagtgg gaaagtaacg gccagcccga gaacaattac 1260

ctgacctggc cccctgtgct ggactctgat gggagtttct ttctgtattc aaagctgaca 1320

gtcgataaaa gccggtggca gcagggcaat gtgttcagct gctccgtcat gcacgaagca 1380

ctgcacaacc attacactca gaagtccctg tccctgtcac ctggc 1425

<210> 201
<211> 475
<212> PRT
<213> Artificial Sequence

<220>
<223> amino acid sequence of h2B1 Fab HC_2

<400> 201

Met Arg Pro Thr Trp Ala Trp Trp Leu Phe Leu Val Leu Leu Leu Ala
1 5 10 15

Leu Trp Ala Pro Ala Arg Gly Gln Val Gln Leu Val Glu Ser Gly Gly
20 25 30

Gly Leu Val Lys Pro Ser Gln Thr Leu Ser Leu Thr Cys Thr Phe Ser
35 40 45

Gly Phe Ser Leu Asn Thr Tyr Asp Met Gly Val Gly Trp Ile Arg Gln
50 55 60

Pro Pro Gly Lys Gly Leu Glu Trp Ile Gly Asn Ile Trp Trp Asp Asp
65 70 75 80

Asp Lys Tyr Tyr Asn Pro Ser Leu Lys Ser Arg Val Thr Ile Ser Lys
85 90 95

Asp Thr Ser Asn Asn Gln Ala Ser Leu Lys Leu Ser Ser Val Thr Ala
100 105 110

Ala Asp Thr Ala Val Tyr Tyr Cys Ala Arg Ile Glu Thr Val Arg Val
115 120 125

Ser Arg Lys Gly Phe Ala His Trp Gly Gln Gly Thr Leu Val Thr Val
130 135 140

Ser Ser Ala Ser Thr Lys Gly Pro Ser Val Phe Pro Glu Ala Pro Ser
145 150 155 160

Ser Lys Ser Thr Ser Gly Gly Thr Ala Ala Leu Gly Cys Leu Val Thr
165 170 175

Asp Tyr Phe Pro Glu Pro Val Thr Val Ser Trp Asn Ser Gly Ala Leu
180 185 190

Thr Ser Gly Val His Thr Phe Pro Ala Val Leu Glu Ser Ser Gly Leu
195 200 205

Tyr Ser Leu Ser Ser Val Val Thr Val Pro Ser Ser Ser Leu Gly Thr
210 215 220

Gln Thr Tyr Ile Cys Asn Val Asn His Lys Pro Ser Asn Thr Lys Val

225 230 235 240

Asp Lys Arg Val Glu Pro Lys Ser Cys Asp Lys Thr His Thr Cys Pro
245 250 255

Pro Cys Pro Ala Pro Glu Ala Ala Gly Gly Pro Ser Val Phe Leu Phe
260 265 270

Pro Pro Lys Pro Lys Asp Thr Leu Met Ile Ser Arg Thr Pro Glu Val
275 280 285

Thr Cys Val Val Val Ser Val Ser His Glu Asp Pro Glu Val Lys Phe
290 295 300

Asn Trp Tyr Val Asp Gly Val Glu Val His Asn Ala Lys Thr Lys Pro
305 310 315 320

Arg Glu Glu Gln Tyr Asn Ser Thr Tyr Arg Val Val Ser Val Leu Thr
325 330 335

Val Leu His Gln Asp Trp Leu Asn Gly Lys Glu Tyr Lys Cys Lys Val
340 345 350

Ser Asn Lys Ala Leu Pro Ala Pro Ile Glu Lys Thr Ile Ser Lys Ala
355 360 365

Lys Gly Gln Pro Arg Glu Pro Gln Val Tyr Val Leu Pro Pro Ser Arg
370 375 380

Asp Glu Leu Thr Lys Asn Gln Val Ser Leu Leu Cys Leu Val Lys Gly
385 390 395 400

Phe Tyr Pro Ser Asp Ile Ala Val Glu Trp Glu Ser Asn Gly Gln Pro
405 410 415

Glu Asn Asn Tyr Leu Thr Trp Pro Pro Val Leu Asp Ser Asp Gly Ser
420 425 430

Phe Phe Leu Tyr Ser Lys Leu Thr Val Asp Lys Ser Arg Trp Gln Gln
435 440 445

Gly Asn Val Phe Ser Cys Ser Val Met His Glu Ala Leu His Asn His
450 455 460

Tyr Thr Gln Lys Ser Leu Ser Leu Ser Pro Gly
465 470 475

<210> 202
<211> 711
<212> DNA
<213> Artificial Sequence

<220>
<223> nucleotide sequence of h2B1 Fab LC_1

<400> 202
atgcggccca cctgggcatg gtggctgttc ctggtgctgc tgctggccct gtgggccccc 60

gcccgggggg ccaccagaat gacacagtct ccaagctcct tttccgcctc tacaggcgac 120

agagtgacca tcacatgcaa ggccagccag tccgtgggca tcaacgtgga ttggtaccag 180

cagaagcctg gcaagtcccc caagctgctg atctatgggg ccagcaatag gcacaccggc 240

gtgccttctc gcttctctgg cagcggcttc ggcagggact ttaccctgac aatctctagc 300

ctgcagagcg aggatttcgc cacctactat tgtctgcagc acggctccat ccccccctacc 360

tttggcggag gcacaaaggt ggagatcaag agaacagtgg cggcgcccag tgtcttcatt 420

tttcccccta gcgacgaaga gctgaagtct gggacagcca gtgtggactg ttggctgaac 480

aacttctacc ctagagaggc taaagtgcag tggaaggtcg ataacgcact gcagtccgga 540

aattctcagg agagtgtgac tgaacaggac tcaaaagata gcacctattc cctgtcaagc 600

acactgactc tgagcaaggc cgactacgag aagcataaag tgtatgcttg tgaagtcacc 660

caccaggggc tgagttcacc agtcacaaaa tcattcaaca gaggggagtg c 711

<210> 203
<211> 237
<212> PRT
<213> Artificial Sequence

<220>
<223> amino acid sequence of h2B1 Fab LC_1

<400> 203

Met Arg Pro Thr Trp Ala Trp Trp Leu Phe Leu Val Leu Leu Leu Ala
1               5                   10                  15

Leu Trp Ala Pro Ala Arg Gly Ala Thr Arg Met Thr Gln Ser Pro Ser
            20                  25                  30

Ser Phe Ser Ala Ser Thr Gly Asp Arg Val Thr Ile Thr Cys Lys Ala
        35                  40                  45

Ser Gln Ser Val Gly Ile Asn Val Asp Trp Tyr Gln Gln Lys Pro Gly
    50                  55                  60

Lys Ser Pro Lys Leu Leu Ile Tyr Gly Ala Ser Asn Arg His Thr Gly
65                  70                  75                  80

Val Pro Ser Arg Phe Ser Gly Ser Gly Phe Gly Arg Asp Phe Thr Leu
85 90 95

Thr Ile Ser Ser Leu Gln Ser Glu Asp Phe Ala Thr Tyr Tyr Cys Leu
100 105 110

Gln His Gly Ser Ile Pro Pro Thr Phe Gly Gly Gly Thr Lys Val Glu
115 120 125

Ile Lys Arg Thr Val Ala Ala Pro Ser Val Phe Ile Phe Pro Pro Ser
130 135 140

Asp Glu Glu Leu Lys Ser Gly Thr Ala Ser Val Asp Cys Trp Leu Asn
145 150 155 160

Asn Phe Tyr Pro Arg Glu Ala Lys Val Gln Trp Lys Val Asp Asn Ala
165 170 175

Leu Gln Ser Gly Asn Ser Gln Glu Ser Val Thr Glu Gln Asp Ser Lys
180 185 190

Asp Ser Thr Tyr Ser Leu Ser Ser Thr Leu Thr Leu Ser Lys Ala Asp
195 200 205

Tyr Glu Lys His Lys Val Tyr Ala Cys Glu Val Thr His Gln Gly Leu
210 215 220

Ser Ser Pro Val Thr Lys Ser Phe Asn Arg Gly Glu Cys
225 230 235

<210> 204
<211> 711
<212> DNA
<213> Artificial Sequence

<220>
<223> nucleotide sequence of h2B1 Fab LC_2

<400> 204
atgcggccca cctgggcatg gtggctgttc ctggtgctgc tgctggccct gtgggccccc 60

gcccgggggg ccaccagaat gacacagtct ccaagctcct tttccgcctc tacaggcgac 120

agagtgacca tcacatgcaa ggccagccag tccgtgggca tcaacgtgga ttggtaccag 180

cagaagcctg gcaagtcccc caagctgctg atctatgggg ccagcaatag gcacaccggc 240

gtgccttctc gcttctctgg cagcggcttc ggcagggact ttaccctgac aatctctagc 300

ctgcagagcg aggatttcgc cacctactat tgtctgcagc acggctccat cccccctacc 360

tttggcggag gcacaaaggt ggagatcaag agaacagtgg cggcgcccag tgtcttcatt 420

tttcccccta gcgacgaaca gctgaagtct gggacagcca gagtgggctg ttggctgaac 480

aacttctacc ctagagaggc taaagtgcag tggaaggtcg ataacgcact gcagtccgga 540

aattctcagg agagtgtgac tgaacaggac tcaaaagata gcacctattc cctgagaagc 600

acactgactc tgagcaaggc cgactacgag aagcataaag tgtatgcttg tgaagtcacc 660

caccaggggc tgagttcacc agtcacaaaa tcattcaaca gaggggagtg c 711

<210> 205
<211> 237
<212> PRT
<213> Artificial Sequence

<220>
<223> amino acid sequence of h2B1 Fab LC_2

<400> 205

Met Arg Pro Thr Trp Ala Trp Trp Leu Phe Leu Val Leu Leu Leu Ala
1               5                   10                  15

Leu Trp Ala Pro Ala Arg Gly Ala Thr Arg Met Thr Gln Ser Pro Ser
            20                  25                  30

Ser Phe Ser Ala Ser Thr Gly Asp Arg Val Thr Ile Thr Cys Lys Ala
        35                  40                  45

Ser Gln Ser Val Gly Ile Asn Val Asp Trp Tyr Gln Gln Lys Pro Gly
    50                  55                  60

Lys Ser Pro Lys Leu Leu Ile Tyr Gly Ala Ser Asn Arg His Thr Gly
65                  70                  75                  80

Val Pro Ser Arg Phe Ser Gly Ser Gly Phe Gly Arg Asp Phe Thr Leu
                85                  90                  95

Thr Ile Ser Ser Leu Gln Ser Glu Asp Phe Ala Thr Tyr Tyr Cys Leu
            100                 105                 110

Gln His Gly Ser Ile Pro Pro Thr Phe Gly Gly Gly Thr Lys Val Glu
        115                 120                 125

Ile Lys Arg Thr Val Ala Ala Pro Ser Val Phe Ile Phe Pro Pro Ser
    130                 135                 140

Asp Glu Gln Leu Lys Ser Gly Thr Ala Arg Val Gly Cys Trp Leu Asn
145                 150                 155                 160

Asn Phe Tyr Pro Arg Glu Ala Lys Val Gln Trp Lys Val Asp Asn Ala

165 170 175

Leu Gln Ser Gly Asn Ser Gln Glu Ser Val Thr Glu Gln Asp Ser Lys
180 185 190

Asp Ser Thr Tyr Ser Leu Arg Ser Thr Leu Thr Leu Ser Lys Ala Asp
195 200 205

Tyr Glu Lys His Lys Val Tyr Ala Cys Glu Val Thr His Gln Gly Leu
210 215 220

Ser Ser Pro Val Thr Lys Ser Phe Asn Arg Gly Glu Cys
225 230 235

<210> 206
<211> 1413
<212> DNA
<213> Artificial Sequence

<220>
<223> nucleotide sequence of C3E-7034 Fab HC

<400> 206
atgcgcccca cctgggcatg gtggctgttc ctggtgctgc tgctggccct gtgggcccct 60

gcccggggcg gcgaggtgca gctggtggag agcggcggcg gcctggtgca gcccggcggc 120

tctctgcggc tgagctgcgc cgcctccggc gtgacattca actactatgg catgtcctgg 180

atcagacagg ccccaggcaa gggcctggag tgggtggcct ctatcaccaa tagcggcggc 240

aggatctact atcccgactc cgtgaagggc cggtttacaa tctctagaga gaacacccag 300

aagacactgt acctgcagat gaacagcctg cgggccgagg ataccgccgt gtactattgt 360

acactggacg gcagagacgg atgggtggca tattggggac agggcaccct ggtgacagtg 420

agctccgcta gcactaaagg cccaagcgtg tttcctctgg ctccaagctc aaaaagcact 480

tccgggggga ccgcatggct gggatgtgat gtgaccgact acttccccga gcctgtgacc 540

gtgtcctgga actctggggc cctgaccagc ggagtgcaca catttccagc cgtgctgcag 600

agctccggcc tgtatagcct gtctagcgtg gtgacagtgc cctccagtag cctgggcact 660

cagacttata tctgtaatgt caaccacaaa ccatcaaaca ccaaagtcga caagaaagtg 720

gagcccaaga gctgtgataa aactcatacc tgcccacctt gtccggcgcc agaggctgca 780

ggaggaccaa gcgtgttcct gtttccaccc aagcctaaag acacactgat gatttcccga 840

acccccgaag tcacatgcgt ggtcgtgtct gtgagtcacg aggaccctga agtcaagttc 900

aactggtacg tggatggcgt cgaggtgcat aatgccaaga ctaaacctag ggaggaacag 960

tacaactcaa cctatcgcgt cgtgagcgtc ctgacagtgc tgcaccagga ttggctgaac 1020

ggcaaagaat ataagtgcaa agtgagcaat aaggccctgc ccgctcctat cgagaaaacc 1080

atttccaagg ctaaagggca gcctcgcgaa ccacaggtct acgtctaccc cccatcaaga 1140

gatgaactga caaaaaatca ggtctctctg acatgcctgg tcaaaggatt ctacccttcc 1200

gacatcgccg tggagtggga aagtaacggc cagcccgaga acaattacaa gaccacaccc 1260

cctgtcctgg actctgatgg gagtttcgct ctggtgtcaa agctgaccgt cgataaaagc 1320

cggtggcagc agggcaatgt gtttagctgc tccgtcatgc acgaagccct gcacaatcac 1380

tacacacaga agtccctgag cctgagccct ggc 1413

<210> 207
<211> 471
<212> PRT
<213> Artificial Sequence

<220>
<223> amino acid sequence of C3E-7034 Fab HC

<400> 207

Met Arg Pro Thr Trp Ala Trp Trp Leu Phe Leu Val Leu Leu Leu Ala
1 5 10 15

Leu Trp Ala Pro Ala Arg Gly Gly Glu Val Gln Leu Val Glu Ser Gly
20 25 30

Gly Gly Leu Val Gln Pro Gly Gly Ser Leu Arg Leu Ser Cys Ala Ala
35 40 45

Ser Gly Val Thr Phe Asn Tyr Tyr Gly Met Ser Trp Ile Arg Gln Ala
50 55 60

Pro Gly Lys Gly Leu Glu Trp Val Ala Ser Ile Thr Asn Ser Gly Gly
65 70 75 80

Arg Ile Tyr Tyr Pro Asp Ser Val Lys Gly Arg Phe Thr Ile Ser Arg
85 90 95

Glu Asn Thr Gln Lys Thr Leu Tyr Leu Gln Met Asn Ser Leu Arg Ala
100 105 110

Glu Asp Thr Ala Val Tyr Tyr Cys Thr Leu Asp Gly Arg Asp Gly Trp
115 120 125

Val Ala Tyr Trp Gly Gln Gly Thr Leu Val Thr Val Ser Ser Ala Ser
130 135 140

Thr Lys Gly Pro Ser Val Phe Pro Leu Ala Pro Ser Ser Lys Ser Thr
145 150 155 160

```
Ser Gly Gly Thr Ala Trp Leu Gly Cys Asp Val Thr Asp Tyr Phe Pro
                165                 170                 175

Glu Pro Val Thr Val Ser Trp Asn Ser Gly Ala Leu Thr Ser Gly Val
            180                 185                 190

His Thr Phe Pro Ala Val Leu Gln Ser Ser Gly Leu Tyr Ser Leu Ser
        195                 200                 205

Ser Val Val Thr Val Pro Ser Ser Ser Leu Gly Thr Gln Thr Tyr Ile
    210                 215                 220

Cys Asn Val Asn His Lys Pro Ser Asn Thr Lys Val Asp Lys Lys Val
225                 230                 235                 240

Glu Pro Lys Ser Cys Asp Lys Thr His Thr Cys Pro Pro Cys Pro Ala
                245                 250                 255

Pro Glu Ala Ala Gly Gly Pro Ser Val Phe Leu Phe Pro Pro Lys Pro
            260                 265                 270

Lys Asp Thr Leu Met Ile Ser Arg Thr Pro Glu Val Thr Cys Val Val
        275                 280                 285

Val Ser Val Ser His Glu Asp Pro Glu Val Lys Phe Asn Trp Tyr Val
    290                 295                 300

Asp Gly Val Glu Val His Asn Ala Lys Thr Lys Pro Arg Glu Glu Gln
305                 310                 315                 320

Tyr Asn Ser Thr Tyr Arg Val Val Ser Val Leu Thr Val Leu His Gln
                325                 330                 335

Asp Trp Leu Asn Gly Lys Glu Tyr Lys Cys Lys Val Ser Asn Lys Ala
            340                 345                 350

Leu Pro Ala Pro Ile Glu Lys Thr Ile Ser Lys Ala Lys Gly Gln Pro
        355                 360                 365

Arg Glu Pro Gln Val Tyr Val Tyr Pro Pro Ser Arg Asp Glu Leu Thr
    370                 375                 380

Lys Asn Gln Val Ser Leu Thr Cys Leu Val Lys Gly Phe Tyr Pro Ser
385                 390                 395                 400

Asp Ile Ala Val Glu Trp Glu Ser Asn Gly Gln Pro Glu Asn Asn Tyr
                405                 410                 415
```

Lys Thr Thr Pro Pro Val Leu Asp Ser Asp Gly Ser Phe Ala Leu Val
420 425 430

Ser Lys Leu Thr Val Asp Lys Ser Arg Trp Gln Gln Gly Asn Val Phe
435 440 445

Ser Cys Ser Val Met His Glu Ala Leu His Asn His Tyr Thr Gln Lys
450 455 460

Ser Leu Ser Leu Ser Pro Gly
465 470

<210> 208
<211> 714
<212> DNA
<213> Artificial Sequence

<220>
<223> nucleotide sequence of C3E-7034 Fab LC

<400> 208
atgcggccta cctgggcctg gtggctgttc ctggtgctgc tgctggccct gtgggcccca 60

gccaggggca actttatgct gacccagccc cacagcgtgt ccgagtctcc tggcaagacc 120

gtgacaatct cctgcaagcg gaacacaggc aatatcggct ctaactacgt gaattggtat 180

cagcagcacg agggcagctc ccccaaccaca atcatctacc gggacgataa gcggcccgac 240

ggcgtgtctg ataggttcag cggctccatc gaccgctcta gcaagtctgc cagcctgacc 300

atcagcaatc tgaagacaga ggacgaggcc gattactttt gtcagtccta ttcctctggc 360

ttcatctttg gaggaggaac caagctgaca gtgctgggcc agcccaaggc ggcgcccagc 420

gtgactctgt ttccacccag ctccgagcag ctgcaggcca ataaggctag actggtctgt 480

ctgatttccg acttctaccc cggggctgtg acagtcgcat ggaaggccga ttctagtcct 540

gtgaaagcag gagtcgagac cacaactcca tcaaagcaga gcaacaacaa gtacgcagcc 600

tcaagctatc tgtctctgac acctgaacag tggaaaagcc accggtctta tagttgtcag 660

gtgactcacg agggctcaac agtggaaaag acagtcgcac ccgcagaatg ctca 714

<210> 209
<211> 238
<212> PRT
<213> Artificial Sequence

<220>
<223> amino acid sequence of C3E-7034 Fab LC

<400> 209

Met Arg Pro Thr Trp Ala Trp Trp Leu Phe Leu Val Leu Leu Leu Ala

```
1               5                   10                  15

Leu Trp Ala Pro Ala Arg Gly Asn Phe Met Leu Thr Gln Pro His Ser
            20                  25                  30

Val Ser Glu Ser Pro Gly Lys Thr Val Thr Ile Ser Cys Lys Arg Asn
        35                  40                  45

Thr Gly Asn Ile Gly Ser Asn Tyr Val Asn Trp Tyr Gln Gln His Glu
    50                  55                  60

Gly Ser Ser Pro Thr Thr Ile Ile Tyr Arg Asp Asp Lys Arg Pro Asp
65                  70                  75                  80

Gly Val Ser Asp Arg Phe Ser Gly Ser Ile Asp Arg Ser Ser Lys Ser
                85                  90                  95

Ala Ser Leu Thr Ile Ser Asn Leu Lys Thr Glu Asp Glu Ala Asp Tyr
            100                 105                 110

Phe Cys Gln Ser Tyr Ser Ser Gly Phe Ile Phe Gly Gly Gly Thr Lys
        115                 120                 125

Leu Thr Val Leu Gly Gln Pro Lys Ala Ala Pro Ser Val Thr Leu Phe
    130                 135                 140

Pro Pro Ser Ser Glu Gln Leu Gln Ala Asn Lys Ala Arg Leu Val Cys
145                 150                 155                 160

Leu Ile Ser Asp Phe Tyr Pro Gly Ala Val Thr Val Ala Trp Lys Ala
                165                 170                 175

Asp Ser Ser Pro Val Lys Ala Gly Val Glu Thr Thr Thr Pro Ser Lys
            180                 185                 190

Gln Ser Asn Asn Lys Tyr Ala Ala Ser Ser Tyr Leu Ser Leu Thr Pro
        195                 200                 205

Glu Gln Trp Lys Ser His Arg Ser Tyr Ser Cys Gln Val Thr His Glu
    210                 215                 220

Gly Ser Thr Val Glu Lys Thr Val Ala Pro Ala Glu Cys Ser
225                 230                 235
```

<210>  210
<211>  1413
<212>  DNA
<213>  Artificial Sequence

<220>
<223> nucleotide sequence of C3E-7036 Fab HC

<400> 210
atgcgcccca cctgggcatg gtggctgttc ctggtgctgc tgctggccct gtgggcccct 60

gcccggggcg gcgaggtgca gctggtggag agcggcggcg gcctggtgca gcccggcggc 120

tctctgcggc tgagctgcgc cgcctccggc gtgacattca actactatgg catgtcctgg 180

atcagacagg ccccaggcaa gggcctggag tgggtggcct ctatcaccaa tagcggcggc 240

aggatctact atcccgactc cgtgaagggc cggtttacaa tctctagaga gaacacccag 300

aagacactgt acctgcagat gaacagcctg cgggccgagg ataccgccgt gtactattgt 360

acactggacg gcagagacgg atgggtggca tattggggac agggcaccct ggtgacagtg 420

agctccgcta gcactaaagg accaagcgtg tttccactgg ctccatcatc caaaagcaca 480

agcggcggga ctgcctggct gggctgtaag gtgaaggact acttccccga gcctgtgacc 540

gtgtcctgga actctggggc cctgaccagc ggagtgcaca catttccagc cgtgctgcag 600

agctccggcc tgtatagcct gtctagcgtg gtgacagtgc cctcctcaag cctgggcact 660

cagacctata tttgtaatgt caaccataaa ccttccaaca ctaaagtcga caagaaagtg 720

gagcccaaga gctgtgataa aactcatacc tgcccacctt gtccggcgcc agaggctgca 780

ggaggaccaa gcgtgttcct gtttccaccc aagcctaaag acacactgat gatttcccga 840

acccccgaag tcacatgcgt ggtcgtgtct gtgagtcacg aggaccctga agtcaagttc 900

aactggtacg tggatggcgt cgaggtgcat aatgccaaga ctaaacctag ggaggaacag 960

tacaactcaa cctatcgcgt cgtgagcgtc ctgacagtgc tgcaccagga ttggctgaac 1020

ggcaaagaat ataagtgcaa agtgagcaat aaggccctgc ccgctcctat cgagaaaacc 1080

atttccaagg ctaaagggca gcctcgcgaa ccacaggtct acgtctaccc cccatcaaga 1140

gatgaactga caaaaaatca ggtctctctg acatgcctgg tcaaaggatt ctacccttcc 1200

gacatcgccg tggagtggga aagtaacggc cagcccgaga acaattacaa gaccacaccc 1260

cctgtcctgg actctgatgg gagtttcgct ctggtgtcaa agctgaccgt cgataaaagc 1320

cggtggcagc agggcaatgt gtttagctgc tccgtcatgc acgaagccct gcacaatcac 1380

tacacacaga agtccctgag cctgagccct ggc 1413

<210> 211
<211> 471
<212> PRT
<213> Artificial Sequence

<220>
<223> amino acid sequence of C3E-7036 Fab HC

<400> 211

```
Met Arg Pro Thr Trp Ala Trp Trp Leu Phe Leu Val Leu Leu Leu Ala
1               5                   10                  15

Leu Trp Ala Pro Ala Arg Gly Gly Glu Val Gln Leu Val Glu Ser Gly
            20                  25                  30

Gly Gly Leu Val Gln Pro Gly Gly Ser Leu Arg Leu Ser Cys Ala Ala
        35                  40                  45

Ser Gly Val Thr Phe Asn Tyr Tyr Gly Met Ser Trp Ile Arg Gln Ala
    50                  55                  60

Pro Gly Lys Gly Leu Glu Trp Val Ala Ser Ile Thr Asn Ser Gly Gly
65                  70                  75                  80

Arg Ile Tyr Tyr Pro Asp Ser Val Lys Gly Arg Phe Thr Ile Ser Arg
                85                  90                  95

Glu Asn Thr Gln Lys Thr Leu Tyr Leu Gln Met Asn Ser Leu Arg Ala
            100                 105                 110

Glu Asp Thr Ala Val Tyr Tyr Cys Thr Leu Asp Gly Arg Asp Gly Trp
        115                 120                 125

Val Ala Tyr Trp Gly Gln Gly Thr Leu Val Thr Val Ser Ser Ala Ser
    130                 135                 140

Thr Lys Gly Pro Ser Val Phe Pro Leu Ala Pro Ser Ser Lys Ser Thr
145                 150                 155                 160

Ser Gly Gly Thr Ala Trp Leu Gly Cys Lys Val Lys Asp Tyr Phe Pro
                165                 170                 175

Glu Pro Val Thr Val Ser Trp Asn Ser Gly Ala Leu Thr Ser Gly Val
            180                 185                 190

His Thr Phe Pro Ala Val Leu Gln Ser Ser Gly Leu Tyr Ser Leu Ser
        195                 200                 205

Ser Val Val Thr Val Pro Ser Ser Ser Leu Gly Thr Gln Thr Tyr Ile
    210                 215                 220

Cys Asn Val Asn His Lys Pro Ser Asn Thr Lys Val Asp Lys Lys Val
225                 230                 235                 240

Glu Pro Lys Ser Cys Asp Lys Thr His Thr Cys Pro Pro Cys Pro Ala
                245                 250                 255
```

```
Pro Glu Ala Ala Gly Gly Pro Ser Val Phe Leu Phe Pro Pro Lys Pro
            260                 265                 270

Lys Asp Thr Leu Met Ile Ser Arg Thr Pro Glu Val Thr Cys Val Val
        275                 280                 285

Val Ser Val Ser His Glu Asp Pro Glu Val Lys Phe Asn Trp Tyr Val
    290                 295                 300

Asp Gly Val Glu Val His Asn Ala Lys Thr Lys Pro Arg Glu Glu Gln
305                 310                 315                 320

Tyr Asn Ser Thr Tyr Arg Val Val Ser Val Leu Thr Val Leu His Gln
                325                 330                 335

Asp Trp Leu Asn Gly Lys Glu Tyr Lys Cys Lys Val Ser Asn Lys Ala
            340                 345                 350

Leu Pro Ala Pro Ile Glu Lys Thr Ile Ser Lys Ala Lys Gly Gln Pro
        355                 360                 365

Arg Glu Pro Gln Val Tyr Val Tyr Pro Pro Ser Arg Asp Glu Leu Thr
    370                 375                 380

Lys Asn Gln Val Ser Leu Thr Cys Leu Val Lys Gly Phe Tyr Pro Ser
385                 390                 395                 400

Asp Ile Ala Val Glu Trp Glu Ser Asn Gly Gln Pro Glu Asn Asn Tyr
                405                 410                 415

Lys Thr Thr Pro Pro Val Leu Asp Ser Asp Gly Ser Phe Ala Leu Val
            420                 425                 430

Ser Lys Leu Thr Val Asp Lys Ser Arg Trp Gln Gln Gly Asn Val Phe
        435                 440                 445

Ser Cys Ser Val Met His Glu Ala Leu His Asn His Tyr Thr Gln Lys
    450                 455                 460

Ser Leu Ser Leu Ser Pro Gly
465                 470
```

<210> 212
<211> 708
<212> DNA
<213> Artificial Sequence

<220>
<223> nucleotide sequence of C3E-7036 Fab LC

<400> 212
atgcggccca cctgggcatg gtggctgttc ctggtgctgc tgctggccct gtgggcccct 60

gccaggggca actttatgct gacccagcct agctccgtga gcggagtgcc aggacagagg 120

gtgacaatct cctgcaccgg caacacaggc aatatcggct ctaactacgt gaattggtat 180

cagcagctgc ccggcacagc ccctaagctg ctgatctacc gggacgataa gcggcccagc 240

ggagtgccag acaggttctc cggctctaag agcggcacct ccgcctctct ggccatcaca 300

ggctttcagg ccgaggacga ggccgattac tattgtcagt cctattctag cggcttcatc 360

tttggaggag gaaccaagct gacagtgctg ggccagccca aggcggcgcc cagtgtcaca 420

ctgtttcccc ctagctccga ggaactgcag gctaacaccg caacactgga ctgtctgatc 480

agcgacttct accctggagc tgtgactgtc gcctggaagg ctgattctag tccagtgaaa 540

gcaggcgtcg agaccacaac tccctctaag cagagtaaca acaagtacgc agcctcaagc 600

tatctgtcac tgaccccaga acagtggaag agccaccgga gctattcctg ccaggtcact 660

cacgaaggct ccactgtcga gaaaaccgtc gctcccaccg aatgttca 708

<210> 213
<211> 236
<212> PRT
<213> Artificial Sequence

<220>
<223> amino acid sequence of C3E-7036 Fab LC

<400> 213

Met Arg Pro Thr Trp Ala Trp Trp Leu Phe Leu Val Leu Leu Leu Ala
1 5 10 15

Leu Trp Ala Pro Ala Arg Gly Asn Phe Met Leu Thr Gln Pro Ser Ser
20 25 30

Val Ser Gly Val Pro Gly Gln Arg Val Thr Ile Ser Cys Thr Gly Asn
35 40 45

Thr Gly Asn Ile Gly Ser Asn Tyr Val Asn Trp Tyr Gln Gln Leu Pro
50 55 60

Gly Thr Ala Pro Lys Leu Leu Ile Tyr Arg Asp Asp Lys Arg Pro Ser
65 70 75 80

Gly Val Pro Asp Arg Phe Ser Gly Ser Lys Ser Gly Thr Ser Ala Ser
85 90 95

Leu Ala Ile Thr Gly Phe Gln Ala Glu Asp Glu Ala Asp Tyr Tyr Cys
100 105 110

Gln Ser Tyr Ser Ser Gly Phe Ile Phe Gly Gly Gly Thr Lys Leu Thr
115 120 125

Val Leu Gly Gln Pro Lys Ala Ala Pro Ser Val Thr Leu Phe Pro Pro
130 135 140

Ser Ser Glu Glu Leu Gln Ala Asn Thr Ala Thr Leu Asp Cys Leu Ile
145 150 155 160

Ser Asp Phe Tyr Pro Gly Ala Val Thr Val Ala Trp Lys Ala Asp Ser
165 170 175

Ser Pro Val Lys Ala Gly Val Glu Thr Thr Thr Pro Ser Lys Gln Ser
180 185 190

Asn Asn Lys Tyr Ala Ala Ser Ser Tyr Leu Ser Leu Thr Pro Glu Gln
195 200 205

Trp Lys Ser His Arg Ser Tyr Ser Cys Gln Val Thr His Glu Gly Ser
210 215 220

Thr Val Glu Lys Thr Val Ala Pro Thr Glu Cys Ser
225 230 235

<210> 214
<211> 1425
<212> DNA
<213> Artificial Sequence

<220>
<223> nucleotide sequence of h2B1 Fab HC_3

<400> 214
atgaggccta cctgggcctg gtggctgttc ctggtgctgc tgctggccct gtgggccccc 60

gcccggggcc aggtgcagct ggtggagtcc ggcggcggcc tggtgaagcc cagccagacc 120

ctgtccctga cctgcacatt cagcggcttt tccctgaaca catacgatat gggagtggga 180

tggatcagac agccacctgg caagggcctg gagtggatcg gcaacatctg gtgggacgat 240

gacaagtact ataatccctc tctgaagagc agagtgacca tctctaagga tacaagcaac 300

aatcaggcct ccctgaagct gagctccgtg accgccgccg acacagccgt gtactattgc 360

gccaggatcg agacagtgcg ggtgagccgg aagggattcg cacactgggg acagggcacc 420

ctggtgacag tgtctagcgc tagcacaaag ggacctagcg tgttcccact ggccccttcc 480

tctaagtcca cctctggagg aacagccgcc ctgggctgtc tggtgaagga ctatttccct 540

gagccagtga ccgtgtcctg gaactctggg gccctgacca gcggagtgca cacatttcct 600

gccgtgctgc agagctccgg cctgtacagc ctgtctagcg tggtgaccgt gccatcctct 660

agcctgggca cccagacata tatctgcaac gtgaatcaca agccaagcaa tacaaaggtc 720

gacaagagag tggagcccaa gtcctgtgac aagacccaca catgcccacc ctgtccggcg 780

ccagaggctg caggaggacc aagcgtgttc ctgtttccac ccaagcctaa agacacactg 840

atgatttccc gaacccccga agtcacatgc gtggtcgtgt ctgtgagtca cgaggaccct 900

gaagtcaagt tcaactggta cgtggatggc gtcgaggtgc ataatgccaa gactaaacct 960

agggaggaac agtacaactc aacctatcgc gtcgtgagcg tcctgacagt gctgcaccag 1020

gattggctga acggcaaaga atataagtgc aaagtgagca ataaggccct gcccgctcct 1080

atcgagaaaa ccatttccaa ggctaaaggg cagcctcgcg aaccacaggt ctacgtgctg 1140

ccccctagcc gcgacgaact gactaaaaat caggtctctc tgctgtgtct ggtcaaagga 1200

ttctaccctt ccgacatcgc cgtggagtgg gaaagtaacg gccagcccga gaacaattac 1260

ctgacctggc cccctgtgct ggactctgat gggagtttct ttctgtattc aaagctgaca 1320

gtcgataaaa gccggtggca gcagggcaat gtgttcagct gctccgtcat gcacgaagca 1380

ctgcacaacc attacactca gaagtccctg tccctgtcac ctggc 1425

<210> 215
<211> 475
<212> PRT
<213> Artificial Sequence

<220>
<223> amino acid sequence of h2B1 Fab HC_3

<400> 215

Met Arg Pro Thr Trp Ala Trp Trp Leu Phe Leu Val Leu Leu Leu Ala
1 5 10 15

Leu Trp Ala Pro Ala Arg Gly Gln Val Gln Leu Val Glu Ser Gly Gly
20 25 30

Gly Leu Val Lys Pro Ser Gln Thr Leu Ser Leu Thr Cys Thr Phe Ser
35 40 45

Gly Phe Ser Leu Asn Thr Tyr Asp Met Gly Val Gly Trp Ile Arg Gln
50 55 60

Pro Pro Gly Lys Gly Leu Glu Trp Ile Gly Asn Ile Trp Trp Asp Asp
65 70 75 80

Asp Lys Tyr Tyr Asn Pro Ser Leu Lys Ser Arg Val Thr Ile Ser Lys
85 90 95

```
Asp Thr Ser Asn Asn Gln Ala Ser Leu Lys Leu Ser Ser Val Thr Ala
            100                 105                 110

Ala Asp Thr Ala Val Tyr Tyr Cys Ala Arg Ile Glu Thr Val Arg Val
        115                 120                 125

Ser Arg Lys Gly Phe Ala His Trp Gly Gln Gly Thr Leu Val Thr Val
    130                 135                 140

Ser Ser Ala Ser Thr Lys Gly Pro Ser Val Phe Pro Leu Ala Pro Ser
145                 150                 155                 160

Ser Lys Ser Thr Ser Gly Gly Thr Ala Ala Leu Gly Cys Leu Val Lys
                165                 170                 175

Asp Tyr Phe Pro Glu Pro Val Thr Val Ser Trp Asn Ser Gly Ala Leu
            180                 185                 190

Thr Ser Gly Val His Thr Phe Pro Ala Val Leu Gln Ser Ser Gly Leu
        195                 200                 205

Tyr Ser Leu Ser Ser Val Val Thr Val Pro Ser Ser Ser Leu Gly Thr
    210                 215                 220

Gln Thr Tyr Ile Cys Asn Val Asn His Lys Pro Ser Asn Thr Lys Val
225                 230                 235                 240

Asp Lys Arg Val Glu Pro Lys Ser Cys Asp Lys Thr His Thr Cys Pro
                245                 250                 255

Pro Cys Pro Ala Pro Glu Ala Ala Gly Gly Pro Ser Val Phe Leu Phe
            260                 265                 270

Pro Pro Lys Pro Lys Asp Thr Leu Met Ile Ser Arg Thr Pro Glu Val
        275                 280                 285

Thr Cys Val Val Val Ser Val Ser His Glu Asp Pro Glu Val Lys Phe
    290                 295                 300

Asn Trp Tyr Val Asp Gly Val Glu Val His Asn Ala Lys Thr Lys Pro
305                 310                 315                 320

Arg Glu Glu Gln Tyr Asn Ser Thr Tyr Arg Val Val Ser Val Leu Thr
                325                 330                 335

Val Leu His Gln Asp Trp Leu Asn Gly Lys Glu Tyr Lys Cys Lys Val
```

340 345 350

Ser Asn Lys Ala Leu Pro Ala Pro Ile Glu Lys Thr Ile Ser Lys Ala
355 360 365

Lys Gly Gln Pro Arg Glu Pro Gln Val Tyr Val Leu Pro Pro Ser Arg
370 375 380

Asp Glu Leu Thr Lys Asn Gln Val Ser Leu Leu Cys Leu Val Lys Gly
385 390 395 400

Phe Tyr Pro Ser Asp Ile Ala Val Glu Trp Glu Ser Asn Gly Gln Pro
405 410 415

Glu Asn Asn Tyr Leu Thr Trp Pro Pro Val Leu Asp Ser Asp Gly Ser
420 425 430

Phe Phe Leu Tyr Ser Lys Leu Thr Val Asp Lys Ser Arg Trp Gln Gln
435 440 445

Gly Asn Val Phe Ser Cys Ser Val Met His Glu Ala Leu His Asn His
450 455 460

Tyr Thr Gln Lys Ser Leu Ser Leu Ser Pro Gly
465 470 475

<210> 216
<211> 711
<212> DNA
<213> Artificial Sequence

<220>
<223> nucleotide_sequence_of_h2B1_Fab_LC_3

<400> 216
atgcggccca cctgggcatg gtggctgttc ctggtgctgc tgctggccct gtgggccccc 60

gcccgggggg ccaccagaat gacacagtct ccaagctcct tttccgcctc tacaggcgac 120

agagtgacca tcacatgcaa ggccagccag tccgtgggca tcaacgtgga ttggtaccag 180

cagaagcctg gcaagtcccc caagctgctg atctatgggg ccagcaatag gcacaccggc 240

gtgccttctc gcttctctgg cagcggcttc ggcagggact ttaccctgac aatctctagc 300

ctgcagagcg aggatttcgc cacctactat tgtctgcagc acggctccat ccccccctacc 360

tttggcggag gcacaaaggt ggagatcaag agaacagtgg cggcgcccag tgtcttcatt 420

tttcccccta gcgacgaaca gctgaagtct gggacagcca gtgtggtctg tctgctgaac 480

aacttctacc ctagagaggc taaagtgcag tggaaggtcg ataacgcact gcagtccgga 540

aattctcagg agagtgtgac tgaacaggac tcaaaagata gcacctattc cctgtcaagc 600

acactgactc tgagcaaggc cgactacgag aagcataaag tgtatgcttg tgaagtcacc 660

caccaggggc tgagttcacc agtcacaaaa tcattcaaca gaggggagtg c 711

<210> 217
<211> 237
<212> PRT
<213> Artificial Sequence

<220>
<223> amino acid sequence of h2B1 Fab LC_3

<400> 217

Met Arg Pro Thr Trp Ala Trp Trp Leu Phe Leu Val Leu Leu Leu Ala
1 5 10 15

Leu Trp Ala Pro Ala Arg Gly Ala Thr Arg Met Thr Gln Ser Pro Ser
20 25 30

Ser Phe Ser Ala Ser Thr Gly Asp Arg Val Thr Ile Thr Cys Lys Ala
35 40 45

Ser Gln Ser Val Gly Ile Asn Val Asp Trp Tyr Gln Gln Lys Pro Gly
50 55 60

Lys Ser Pro Lys Leu Leu Ile Tyr Gly Ala Ser Asn Arg His Thr Gly
65 70 75 80

Val Pro Ser Arg Phe Ser Gly Ser Gly Phe Gly Arg Asp Phe Thr Leu
85 90 95

Thr Ile Ser Ser Leu Gln Ser Glu Asp Phe Ala Thr Tyr Tyr Cys Leu
100 105 110

Gln His Gly Ser Ile Pro Pro Thr Phe Gly Gly Gly Thr Lys Val Glu
115 120 125

Ile Lys Arg Thr Val Ala Ala Pro Ser Val Phe Ile Phe Pro Pro Ser
130 135 140

Asp Glu Gln Leu Lys Ser Gly Thr Ala Ser Val Val Cys Leu Leu Asn
145 150 155 160

Asn Phe Tyr Pro Arg Glu Ala Lys Val Gln Trp Lys Val Asp Asn Ala
165 170 175

Leu Gln Ser Gly Asn Ser Gln Glu Ser Val Thr Glu Gln Asp Ser Lys
180 185 190

Asp Ser Thr Tyr Ser Leu Ser Ser Thr Leu Thr Leu Ser Lys Ala Asp
        195                 200                 205

Tyr Glu Lys His Lys Val Tyr Ala Cys Glu Val Thr His Gln Gly Leu
    210                 215                 220

Ser Ser Pro Val Thr Lys Ser Phe Asn Arg Gly Glu Cys
225                 230                 235

<210> 218
<211> 1497
<212> DNA
<213> Artificial Sequence

<220>
<223> nucleotide sequence of C3E-7034 scFv Fc

<400> 218
atgcgcccta cctgggcctg gtggctgttc ctggtgctgc tgctggccct gtgggcccca 60

gcccggggcg gcgaggtgca gctggtggag tctggcggcg gcctggtgca gcccggcggc 120

agcctgcggc tgtcctgcgc cgcctctggc gtgaccttca actactatgg catgagctgg 180

atcagacagg cccccggcaa gggcctggag tgggtggcca gcatcaccaa ttccggcggc 240

aggatctact atcctgactc tgtgaagggc aggtttacaa tcagccgcga gaacacccag 300

aagacactgt acctgcagat gaacagcctg cgggccgagg ataccgccgt gtactattgc 360

acactggacg gcagagacgg atgggtggca tattggggac agggcaccct ggtgacagtg 420

agctccggcg gcggcggctc tggaggagga ggcagcggcg gaggaggctc caactttatg 480

ctgacccagc cccactctgt gagcgagtcc cctggcaaga ccgtgacaat ctcctgtaag 540

agaaacacag gcaatatcgg ctctaactac gtgaattggt atcagcagca cgagggctct 600

agcccaacca caatcatcta ccgggacgat aagcggcccg acggcgtgag cgatcggttc 660

tctggcagca tcgacagatc ctctaagtcc gcctctctga ccatcagcaa tctgaagaca 720

gaggacgagg ccgattactt ttgtcagtcc tatagctccg gcttcatctt tggcggcggc 780

accaagctga cagtgctggc ggccgagcct aaaagtagcg ataaaaccca tacctgcccc 840

ccctgcccgg cgccagaggc tgcaggagga ccaagcgtgt tcctgtttcc acccaagcct 900

aaagacacac tgatgatttc ccgaaccccc gaagtcacat gcgtggtcgt gtctgtgagt 960

cacgaggacc ctgaagtcaa gttcaactgg tacgtggatg gcgtcgaggt gcataatgcc 1020

aagactaaac ctagggagga acagtacaac tcaacctatc gcgtcgtgag cgtcctgaca 1080

gtgctgcacc aggattggct gaacggcaaa gaatataagt gcaaagtgag caataaggcc 1140

ctgcccgctc ctatcgagaa aaccatttcc aaggctaaag ggcagcctcg cgaaccacag 1200

gtctacgtct accccccatc aagagatgaa ctgacaaaaa atcaggtctc tctgacatgc 1260

ctggtcaaag gattctaccc ttccgacatc gccgtggagt gggaaagtaa cggccagccc 1320

gagaacaatt acaagaccac accccctgtc ctggactctg atgggagttt cgctctggtg 1380

tcaaagctga ccgtcgataa aagccggtgg cagcagggca atgtgtttag ctgctccgtc 1440

atgcacgaag ccctgcacaa tcactacaca cagaagtccc tgagcctgag ccctggc 1497

<210> 219
<211> 499
<212> PRT
<213> Artificial Sequence

<220>
<223> amino acid sequence of C3E-7034 scFv Fc

<400> 219

Met Arg Pro Thr Trp Ala Trp Trp Leu Phe Leu Val Leu Leu Leu Ala
1 5 10 15

Leu Trp Ala Pro Ala Arg Gly Gly Glu Val Gln Leu Val Glu Ser Gly
20 25 30

Gly Gly Leu Val Gln Pro Gly Gly Ser Leu Arg Leu Ser Cys Ala Ala
35 40 45

Ser Gly Val Thr Phe Asn Tyr Tyr Gly Met Ser Trp Ile Arg Gln Ala
50 55 60

Pro Gly Lys Gly Leu Glu Trp Val Ala Ser Ile Thr Asn Ser Gly Gly
65 70 75 80

Arg Ile Tyr Tyr Pro Asp Ser Val Lys Gly Arg Phe Thr Ile Ser Arg
85 90 95

Glu Asn Thr Gln Lys Thr Leu Tyr Leu Gln Met Asn Ser Leu Arg Ala
100 105 110

Glu Asp Thr Ala Val Tyr Tyr Cys Thr Leu Asp Gly Arg Asp Gly Trp
115 120 125

Val Ala Tyr Trp Gly Gln Gly Thr Leu Val Thr Val Ser Ser Gly Gly
130 135 140

Gly Gly Ser Gly Gly Gly Gly Ser Gly Gly Gly Gly Ser Asn Phe Met
145 150 155 160

Leu Thr Gln Pro His Ser Val Ser Glu Ser Pro Gly Lys Thr Val Thr
165 170 175

```
Ile Ser Cys Lys Arg Asn Thr Gly Asn Ile Gly Ser Asn Tyr Val Asn
            180                 185                 190

Trp Tyr Gln Gln His Glu Gly Ser Ser Pro Thr Thr Ile Ile Tyr Arg
        195                 200                 205

Asp Asp Lys Arg Pro Asp Gly Val Ser Asp Arg Phe Ser Gly Ser Ile
    210                 215                 220

Asp Arg Ser Ser Lys Ser Ala Ser Leu Thr Ile Ser Asn Leu Lys Thr
225                 230                 235                 240

Glu Asp Glu Ala Asp Tyr Phe Cys Gln Ser Tyr Ser Ser Gly Phe Ile
                245                 250                 255

Phe Gly Gly Gly Thr Lys Leu Thr Val Leu Ala Ala Glu Pro Lys Ser
            260                 265                 270

Ser Asp Lys Thr His Thr Cys Pro Pro Cys Pro Ala Pro Glu Ala Ala
        275                 280                 285

Gly Gly Pro Ser Val Phe Leu Phe Pro Pro Lys Pro Lys Asp Thr Leu
    290                 295                 300

Met Ile Ser Arg Thr Pro Glu Val Thr Cys Val Val Val Ser Val Ser
305                 310                 315                 320

His Glu Asp Pro Glu Val Lys Phe Asn Trp Tyr Val Asp Gly Val Glu
                325                 330                 335

Val His Asn Ala Lys Thr Lys Pro Arg Glu Glu Gln Tyr Asn Ser Thr
            340                 345                 350

Tyr Arg Val Val Ser Val Leu Thr Val Leu His Gln Asp Trp Leu Asn
        355                 360                 365

Gly Lys Glu Tyr Lys Cys Lys Val Ser Asn Lys Ala Leu Pro Ala Pro
    370                 375                 380

Ile Glu Lys Thr Ile Ser Lys Ala Lys Gly Gln Pro Arg Glu Pro Gln
385                 390                 395                 400

Val Tyr Val Tyr Pro Pro Ser Arg Asp Glu Leu Thr Lys Asn Gln Val
                405                 410                 415

Ser Leu Thr Cys Leu Val Lys Gly Phe Tyr Pro Ser Asp Ile Ala Val
            420                 425                 430
```

Glu Trp Glu Ser Asn Gly Gln Pro Glu Asn Asn Tyr Lys Thr Thr Pro
435 440 445

Pro Val Leu Asp Ser Asp Gly Ser Phe Ala Leu Val Ser Lys Leu Thr
450 455 460

Val Asp Lys Ser Arg Trp Gln Gln Gly Asn Val Phe Ser Cys Ser Val
465 470 475 480

Met His Glu Ala Leu His Asn His Tyr Thr Gln Lys Ser Leu Ser Leu
485 490 495

Ser Pro Gly

<210> 220
<211> 1491
<212> DNA
<213> Artificial Sequence

<220>
<223> nucleotide sequence of C3E-7036 scFv Fc

<400> 220
atgcggccca cctgggcatg gtggctgttc ctggtgctgc tgctggccct gtgggcccct 60

gccaggggcg gcgaggtgca gctggtggag agcggcggcg gcctggtgca gcctggaggc 120

tctctgaggc tgagctgcgc agcctccggc gtgacattca actactatgg catgtcctgg 180

atcagacagg ccccaggcaa gggcctggag tgggtggcct ctatcaccaa tagcggcggc 240

agaatctact atcccgactc cgtgaagggc aggtttacaa tcagccggga gaacacccag 300

aagacactgt acctgcagat gaatagcctg cgggccgagg ataccgccgt gtactattgc 360

acactggacg gcagagatgg atgggtggca tattggggac agggcaccct ggtgacagtg 420

agctccggag gaggaggctc cggcggcgga ggctctggcg gcggcggcag caactttatg 480

ctgacccagc cttctagcgt gagcggagtg ccaggacaga gggtgacaat ctcctgtacc 540

ggcaacacag gcaatatcgg ctctaactac gtgaattggt atcagcagct gccaggaacc 600

gcccctaagc tgctgatcta ccgggacgat aagcggccct ctggagtgcc agaccggttc 660

agcggctcca agtctggcac cagcgcctcc ctggccatca caggatttca ggccgaggac 720

gaggccgatt actattgtca gtcctattcc tctggcttca tctttggcgg cggcaccaag 780

ctgacagtgc tggcggccga gcctaaaagt agcgataaaa cccatacctg cccccccctgc 840

ccggcgccag aggctgcagg aggaccaagc gtgttcctgt ttccacccaa gcctaaagac 900

acactgatga tttcccgaac ccccgaagtc acatgcgtgg tcgtgtctgt gagtcacgag 960

gaccctgaag tcaagttcaa ctggtacgtg gatggcgtcg aggtgcataa tgccaagact 1020

aaacctaggg aggaacagta caactcaacc tatcgcgtcg tgagcgtcct gacagtgctg 1080

caccaggatt ggctgaacgg caaagaatat aagtgcaaag tgagcaataa ggccctgccc 1140

gctcctatcg agaaaaccat ttccaaggct aaagggcagc ctcgcgaacc acaggtctac 1200

gtctaccccc catcaagaga tgaactgaca aaaaatcagg tctctctgac atgcctggtc 1260

aaaggattct acccttccga catcgccgtg gagtgggaaa gtaacggcca gcccgagaac 1320

aattacaaga ccacaccccc tgtcctggac tctgatggga gtttcgctct ggtgtcaaag 1380

ctgaccgtcg ataaaagccg gtggcagcag ggcaatgtgt ttagctgctc cgtcatgcac 1440

gaagccctgc acaatcacta cacacagaag tccctgagcc tgagccctgg c 1491

<210> 221
<211> 497
<212> PRT
<213> Artificial Sequence

<220>
<223> amino acid sequence of C3E-7036 scFv Fc

<400> 221

Met Arg Pro Thr Trp Ala Trp Trp Leu Phe Leu Val Leu Leu Leu Ala
1 5 10 15

Leu Trp Ala Pro Ala Arg Gly Gly Glu Val Gln Leu Val Glu Ser Gly
20 25 30

Gly Gly Leu Val Gln Pro Gly Gly Ser Leu Arg Leu Ser Cys Ala Ala
35 40 45

Ser Gly Val Thr Phe Asn Tyr Tyr Gly Met Ser Trp Ile Arg Gln Ala
50 55 60

Pro Gly Lys Gly Leu Glu Trp Val Ala Ser Ile Thr Asn Ser Gly Gly
65 70 75 80

Arg Ile Tyr Tyr Pro Asp Ser Val Lys Gly Arg Phe Thr Ile Ser Arg
85 90 95

Glu Asn Thr Gln Lys Thr Leu Tyr Leu Gln Met Asn Ser Leu Arg Ala
100 105 110

Glu Asp Thr Ala Val Tyr Tyr Cys Thr Leu Asp Gly Arg Asp Gly Trp
115 120 125

Val Ala Tyr Trp Gly Gln Gly Thr Leu Val Thr Val Ser Ser Gly Gly
130 135 140

```
Gly Gly Ser Gly Gly Gly Gly Ser Gly Gly Gly Gly Ser Asn Phe Met
145                 150                 155                 160

Leu Thr Gln Pro Ser Ser Val Ser Gly Val Pro Gly Gln Arg Val Thr
                165                 170                 175

Ile Ser Cys Thr Gly Asn Thr Gly Asn Ile Gly Ser Asn Tyr Val Asn
            180                 185                 190

Trp Tyr Gln Gln Leu Pro Gly Thr Ala Pro Lys Leu Leu Ile Tyr Arg
        195                 200                 205

Asp Asp Lys Arg Pro Ser Gly Val Pro Asp Arg Phe Ser Gly Ser Lys
    210                 215                 220

Ser Gly Thr Ser Ala Ser Leu Ala Ile Thr Gly Phe Gln Ala Glu Asp
225                 230                 235                 240

Glu Ala Asp Tyr Tyr Cys Gln Ser Tyr Ser Ser Gly Phe Ile Phe Gly
                245                 250                 255

Gly Gly Thr Lys Leu Thr Val Leu Ala Ala Glu Pro Lys Ser Ser Asp
            260                 265                 270

Lys Thr His Thr Cys Pro Pro Cys Pro Ala Pro Glu Ala Ala Gly Gly
        275                 280                 285

Pro Ser Val Phe Leu Phe Pro Pro Lys Pro Lys Asp Thr Leu Met Ile
    290                 295                 300

Ser Arg Thr Pro Glu Val Thr Cys Val Val Val Ser Val Ser His Glu
305                 310                 315                 320

Asp Pro Glu Val Lys Phe Asn Trp Tyr Val Asp Gly Val Glu Val His
                325                 330                 335

Asn Ala Lys Thr Lys Pro Arg Glu Glu Gln Tyr Asn Ser Thr Tyr Arg
            340                 345                 350

Val Val Ser Val Leu Thr Val Leu His Gln Asp Trp Leu Asn Gly Lys
        355                 360                 365

Glu Tyr Lys Cys Lys Val Ser Asn Lys Ala Leu Pro Ala Pro Ile Glu
    370                 375                 380

Lys Thr Ile Ser Lys Ala Lys Gly Gln Pro Arg Glu Pro Gln Val Tyr
```

385 390 395 400

Val Tyr Pro Pro Ser Arg Asp Glu Leu Thr Lys Asn Gln Val Ser Leu
405 410 415

Thr Cys Leu Val Lys Gly Phe Tyr Pro Ser Asp Ile Ala Val Glu Trp
420 425 430

Glu Ser Asn Gly Gln Pro Glu Asn Asn Tyr Lys Thr Thr Pro Pro Val
435 440 445

Leu Asp Ser Asp Gly Ser Phe Ala Leu Val Ser Lys Leu Thr Val Asp
450 455 460

Lys Ser Arg Trp Gln Gln Gly Asn Val Phe Ser Cys Ser Val Met His
465 470 475 480

Glu Ala Leu His Asn His Tyr Thr Gln Lys Ser Leu Ser Leu Ser Pro
485 490 495

Gly

<210> 222
<211> 1512
<212> DNA
<213> Artificial Sequence

<220>
<223> nucleotide sequence of h2B1 scFv Fc

<400> 222
atgcggccta cctgggcctg gtggctgttc ctggtgctgc tgctggccct gtgggccccc 60

gcccgggggg ccacccgcat gacacagtcc cctagctcct tttctgccag cacaggcgat 120

agagtgacca tcacatgcaa ggcctcccag tctgtgggca tcaacgtgga ctggtaccag 180

cagaagcctg gcaagagccc aaagctgctg atctatgggg ccagcaatag gcacaccgga 240

gtgccatcta gattcagcgg ctccggcttc ggcagagact tcaccctgac aatctctagc 300

ctgcagagcg aggacttcgc cacatactat tgcctgcagc acggctccat ccccccctacc 360

tttggcggag gcacaaaggt ggagatcaag ggaggaggag gctccggcgg aggaggctct 420

ggcggcggcg gcagcggcgg cggccaggtg cagctggtgg agtccggagg aggcctggtg 480

aagccaagcc agaccctgtc cctgacctgt acattctctg gctttagcct gaacacatac 540

gatatgggag tgggatggat caggcagcca ccaggcaagg gcctggagtg gatcggcaac 600

atctggtggg acgatgacaa gtactataat ccctccctga agtctcgcgt gaccatctct 660

aaggatacaa gcaacaatca ggcctctctg aagctgtcct ctgtgaccgc cgccgacaca 720

gccgtgtact attgtgcaag gatcgagaca gtgcgggtga gccggaaggg atttgcacac 780

tggggacagg gcaccctggt gacagtgagc tctgcggccg agcctaaaag tagcgataaa 840

acccatacct gcccccctg cccggcgcca gaggctgcag gaggaccaag cgtgttcctg 900

tttccaccca agcctaaaga cacactgatg atttcccgaa cccccgaagt cacatgcgtg 960

gtcgtgtctg tgagtcacga ggaccctgaa gtcaagttca actggtacgt ggatggcgtc 1020

gaggtgcata atgccaagac taaacctagg gaggaacagt acaactcaac ctatcgcgtc 1080

gtgagcgtcc tgacagtgct gcaccaggat tggctgaacg gcaaagaata taagtgcaaa 1140

gtgagcaata aggccctgcc cgctcctatc gagaaaacca tttccaaggc taaagggcag 1200

cctcgcgaac cacaggtcta cgtgctgccc cctagccgcg acgaactgac taaaaatcag 1260

gtctctctgc tgtgtctggt caaaggattc tacccttccg acatcgccgt ggagtgggaa 1320

agtaacggcc agcccgagaa caattacctg acctggcccc ctgtgctgga ctctgatggg 1380

agtttctttc tgtattcaaa gctgacagtc gataaaagcc ggtggcagca gggcaatgtg 1440

ttcagctgct ccgtcatgca cgaagcactg cacaaccatt acactcagaa gtccctgtcc 1500

ctgtcacctg gc 1512

<210> 223
<211> 504
<212> PRT
<213> Artificial Sequence

<220>
<223> amino_acid_sequence_of_h2B1_scFv_Fc

<400> 223

```
Met Arg Pro Thr Trp Ala Trp Trp Leu Phe Leu Val Leu Leu Leu Ala
1               5                   10                  15

Leu Trp Ala Pro Ala Arg Gly Ala Thr Arg Met Thr Gln Ser Pro Ser
            20                  25                  30

Ser Phe Ser Ala Ser Thr Gly Asp Arg Val Thr Ile Thr Cys Lys Ala
        35                  40                  45

Ser Gln Ser Val Gly Ile Asn Val Asp Trp Tyr Gln Gln Lys Pro Gly
    50                  55                  60

Lys Ser Pro Lys Leu Leu Ile Tyr Gly Ala Ser Asn Arg His Thr Gly
65                  70                  75                  80

Val Pro Ser Arg Phe Ser Gly Ser Gly Phe Gly Arg Asp Phe Thr Leu
                85                  90                  95
```

Thr Ile Ser Ser Leu Gln Ser Glu Asp Phe Ala Thr Tyr Tyr Cys Leu
100 105 110

Gln His Gly Ser Ile Pro Pro Thr Phe Gly Gly Gly Thr Lys Val Glu
115 120 125

Ile Lys Gly Gly Gly Gly Ser Gly Gly Gly Gly Ser Gly Gly Gly Gly
130 135 140

Ser Gly Gly Gly Gln Val Gln Leu Val Glu Ser Gly Gly Gly Leu Val
145 150 155 160

Lys Pro Ser Gln Thr Leu Ser Leu Thr Cys Thr Phe Ser Gly Phe Ser
165 170 175

Leu Asn Thr Tyr Asp Met Gly Val Gly Trp Ile Arg Gln Pro Pro Gly
180 185 190

Lys Gly Leu Glu Trp Ile Gly Asn Ile Trp Trp Asp Asp Asp Lys Tyr
195 200 205

Tyr Asn Pro Ser Leu Lys Ser Arg Val Thr Ile Ser Lys Asp Thr Ser
210 215 220

Asn Asn Gln Ala Ser Leu Lys Leu Ser Ser Val Thr Ala Ala Asp Thr
225 230 235 240

Ala Val Tyr Tyr Cys Ala Arg Ile Glu Thr Val Arg Val Ser Arg Lys
245 250 255

Gly Phe Ala His Trp Gly Gln Gly Thr Leu Val Thr Val Ser Ser Ala
260 265 270

Ala Glu Pro Lys Ser Ser Asp Lys Thr His Thr Cys Pro Pro Cys Pro
275 280 285

Ala Pro Glu Ala Ala Gly Gly Pro Ser Val Phe Leu Phe Pro Pro Lys
290 295 300

Pro Lys Asp Thr Leu Met Ile Ser Arg Thr Pro Glu Val Thr Cys Val
305 310 315 320

Val Val Ser Val Ser His Glu Asp Pro Glu Val Lys Phe Asn Trp Tyr
325 330 335

Val Asp Gly Val Glu Val His Asn Ala Lys Thr Lys Pro Arg Glu Glu
340 345 350

Gln Tyr Asn Ser Thr Tyr Arg Val Val Ser Val Leu Thr Val Leu His
355 360 365

Gln Asp Trp Leu Asn Gly Lys Glu Tyr Lys Cys Lys Val Ser Asn Lys
370 375 380

Ala Leu Pro Ala Pro Ile Glu Lys Thr Ile Ser Lys Ala Lys Gly Gln
385 390 395 400

Pro Arg Glu Pro Gln Val Tyr Val Leu Pro Pro Ser Arg Asp Glu Leu
405 410 415

Thr Lys Asn Gln Val Ser Leu Leu Cys Leu Val Lys Gly Phe Tyr Pro
420 425 430

Ser Asp Ile Ala Val Glu Trp Glu Ser Asn Gly Gln Pro Glu Asn Asn
435 440 445

Tyr Leu Thr Trp Pro Pro Val Leu Asp Ser Asp Gly Ser Phe Phe Leu
450 455 460

Tyr Ser Lys Leu Thr Val Asp Lys Ser Arg Trp Gln Gln Gly Asn Val
465 470 475 480

Phe Ser Cys Ser Val Met His Glu Ala Leu His Asn His Tyr Thr Gln
485 490 495

Lys Ser Leu Ser Leu Ser Pro Gly
500

<210> 224
<211> 1491
<212> DNA
<213> Artificial Sequence

<220>
<223> nucleotide sequence of C3E-8015

<400> 224
atgcggccca cctgggcatg gtggctgttc ctggtgctgc tgctggccct gtgggcccct 60

gccaggggcg gagaagtgca gctggtggaa tccgggggg gcctggtgca gcctgggggg 120

agcctgagac tgagttgtgc cgcctctggg gtgacattta actactatgg catgtcttgg 180

atccgccagg cacctggaaa gggcctggag tgggtggcca gcatcactag gtccggcggg 240

cgaatctact atcccgacag cgtcaagggc aggttcacaa tttcccgcga gaacacacag 300

aaaactctgt acctgcagat gaatagcctg agagccgaag atacagctgt gtactattgc 360

```
actctggacg gcagggatgg gtgggtcgcc tattgggggc agggaaccct ggtgacagtc      420
agctccggag gaggaggatc tggcggagga ggcagtgggg gaggcgggtc aaactttatg      480
ctcactcagc cgtcctctgt ttctggcgta cctggccaac gggtgaccat tagctgtacg      540
ggtaataccg ggaatatcgg gtctaactac gtgaactggt atcagcagct tccagggaca      600
gctcccaagt tgctgatcta tcgcgacgac aaaagaccct caggggtccc tgaccgattt      660
agtggcagca aaagcggtac ttccgcttcc ctggcgataa ccggctttca ggccgaagat      720
gaggcagact actattgcca gtcatattcc agcggcttca tcttcggagg cggaactaag      780
ctgacagtgt tggcggccga gcctaaaagt agcgataaaa cccatacctg cccccccctgc     840
ccggcgccag aggctgcagg aggaccaagc gtgttcctgt ttccacccaa gcctaaagac      900
acactgatga tttcccgaac ccccgaagtc acatgcgtgg tcgtgtctgt gagtcacgag      960
gaccctgaag tcaagttcaa ctggtacgtg gatggcgtcg aggtgcataa tgccaagact     1020
aaacctaggg aggaacagta caactcaacc tatcgcgtcg tgagcgtcct gacagtgctg     1080
caccaggatt ggctgaacgg caaagaatat aagtgcaaag tgagcaataa ggccctgccc     1140
gctcctatcg agaaaaccat ttccaaggct aaagggcagc ctcgcgaacc acaggtctac     1200
gtctaccccc catcaagaga tgaactgaca aaaaatcagg tctctctgac atgcctggtc     1260
aaaggattct acccttccga catcgccgtg gagtgggaaa gtaacggcca gcccgagaac     1320
aattacaaga ccacaccccc tgtcctggac tctgatggga gtttcgctct ggtgtcaaag     1380
ctgaccgtcg ataaaagccg gtggcagcag ggcaatgtgt ttagctgctc cgtcatgcac     1440
gaagccctgc acaatcacta cacacagaag tccctgagcc tgagccctgg c              1491
```

<210> 225
<211> 497
<212> PRT
<213> Artificial Sequence

<220>
<223> amino acid sequence of C3E-8015

<400> 225

```
Met Arg Pro Thr Trp Ala Trp Trp Leu Phe Leu Val Leu Leu Leu Ala
1               5                   10                  15

Leu Trp Ala Pro Ala Arg Gly Gly Glu Val Gln Leu Val Glu Ser Gly
            20                  25                  30

Gly Gly Leu Val Gln Pro Gly Gly Ser Leu Arg Leu Ser Cys Ala Ala
        35                  40                  45

Ser Gly Val Thr Phe Asn Tyr Tyr Gly Met Ser Trp Ile Arg Gln Ala
    50                  55                  60
```

```
Pro Gly Lys Gly Leu Glu Trp Val Ala Ser Ile Thr Arg Ser Gly Gly
65                  70                  75                  80

Arg Ile Tyr Tyr Pro Asp Ser Val Lys Gly Arg Phe Thr Ile Ser Arg
                85                  90                  95

Glu Asn Thr Gln Lys Thr Leu Tyr Leu Gln Met Asn Ser Leu Arg Ala
            100                 105                 110

Glu Asp Thr Ala Val Tyr Tyr Cys Thr Leu Asp Gly Arg Asp Gly Trp
        115                 120                 125

Val Ala Tyr Trp Gly Gln Gly Thr Leu Val Thr Val Ser Ser Gly Gly
    130                 135                 140

Gly Gly Ser Gly Gly Gly Gly Ser Gly Gly Gly Gly Ser Asn Phe Met
145                 150                 155                 160

Leu Thr Gln Pro Ser Ser Val Ser Gly Val Pro Gly Gln Arg Val Thr
                165                 170                 175

Ile Ser Cys Thr Gly Asn Thr Gly Asn Ile Gly Ser Asn Tyr Val Asn
            180                 185                 190

Trp Tyr Gln Gln Leu Pro Gly Thr Ala Pro Lys Leu Leu Ile Tyr Arg
        195                 200                 205

Asp Asp Lys Arg Pro Ser Gly Val Pro Asp Arg Phe Ser Gly Ser Lys
    210                 215                 220

Ser Gly Thr Ser Ala Ser Leu Ala Ile Thr Gly Phe Gln Ala Glu Asp
225                 230                 235                 240

Glu Ala Asp Tyr Tyr Cys Gln Ser Tyr Ser Ser Gly Phe Ile Phe Gly
                245                 250                 255

Gly Gly Thr Lys Leu Thr Val Leu Ala Ala Glu Pro Lys Ser Ser Asp
            260                 265                 270

Lys Thr His Thr Cys Pro Pro Cys Pro Ala Pro Glu Ala Ala Gly Gly
        275                 280                 285

Pro Ser Val Phe Leu Phe Pro Pro Lys Pro Lys Asp Thr Leu Met Ile
    290                 295                 300

Ser Arg Thr Pro Glu Val Thr Cys Val Val Val Ser Val Ser His Glu
```

305 310 315 320

Asp Pro Glu Val Lys Phe Asn Trp Tyr Val Asp Gly Val Glu Val His
325 330 335

Asn Ala Lys Thr Lys Pro Arg Glu Glu Gln Tyr Asn Ser Thr Tyr Arg
340 345 350

Val Val Ser Val Leu Thr Val Leu His Gln Asp Trp Leu Asn Gly Lys
355 360 365

Glu Tyr Lys Cys Lys Val Ser Asn Lys Ala Leu Pro Ala Pro Ile Glu
370 375 380

Lys Thr Ile Ser Lys Ala Lys Gly Gln Pro Arg Glu Pro Gln Val Tyr
385 390 395 400

Val Tyr Pro Pro Ser Arg Asp Glu Leu Thr Lys Asn Gln Val Ser Leu
405 410 415

Thr Cys Leu Val Lys Gly Phe Tyr Pro Ser Asp Ile Ala Val Glu Trp
420 425 430

Glu Ser Asn Gly Gln Pro Glu Asn Asn Tyr Lys Thr Thr Pro Pro Val
435 440 445

Leu Asp Ser Asp Gly Ser Phe Ala Leu Val Ser Lys Leu Thr Val Asp
450 455 460

Lys Ser Arg Trp Gln Gln Gly Asn Val Phe Ser Cys Ser Val Met His
465 470 475 480

Glu Ala Leu His Asn His Tyr Thr Gln Lys Ser Leu Ser Leu Ser Pro
485 490 495

Gly

<210> 226
<211> 1497
<212> DNA
<213> Artificial Sequence

<220>
<223> nucleotide sequence of C3E-8017

<400> 226
atgcgcccta cctgggcctg gtggctgttc ctggtgctgc tgctggccct gtgggcccca 60

gcccggggcg gagaagtgca gctggtggaa tccgggggg gcctggtgca gcctgggggg 120

agcctgagac tgagttgtgc cgcctctggg gtgacattta actactatgg catgtcttgg 180

atccgccagg cacctggaaa gggcctggag tgggtggcca gcatcactag gtccggcggg 240

cgaatctact atcccgacag cgtcaagggc aggttcacaa tttcccgcga gaacacacag 300

aaaactctgt acctgcagat gaatagcctg agagccgaag atacagctgt gtactattgc 360

actctggacg gcagggatgg gtgggtcgcc tattgggggc agggaaccct ggtgacagtc 420

agctccggag gaggaggatc tggcggagga ggcagtgggg gaggcgggtc aaactttatg 480

ctgacccagc cccacagtgt gtcagagagc cctggcaaga ctgtcaccat ctcttgtaaa 540

aggaacaccg gaaatattgg cagtaactac gtgaattggt atcagcagca tgaagggtct 600

agtccaacca caatcatcta ccggaacgat aagagacccg acggggtgtc cgatcgattc 660

tccggatcta tcgaccggtc aagcaagagt gcttcactga ccattagcaa tctgaaaaca 720

gaggacgaag cagattactt ttgccagtcc tattcctctg gcttcatctt tggaggcggg 780

actaaactga ccgtgctggc ggccgagcct aaaagtagcg ataaaaccca tacctgcccc 840

ccctgcccgg cgccagaggc tgcaggagga ccaagcgtgt tcctgtttcc acccaagcct 900

aaagacacac tgatgatttc ccgaaccccc gaagtcacat gcgtggtcgt gtctgtgagt 960

cacgaggacc ctgaagtcaa gttcaactgg tacgtggatg gcgtcgaggt gcataatgcc 1020

aagactaaac ctagggagga acagtacaac tcaacctatc gcgtcgtgag cgtcctgaca 1080

gtgctgcacc aggattggct gaacggcaaa gaatataagt gcaaagtgag caataaggcc 1140

ctgcccgctc ctatcgagaa aaccatttcc aaggctaaag ggcagcctcg cgaaccacag 1200

gtctacgtct accccccatc aagagatgaa ctgacaaaaa atcaggtctc tctgacatgc 1260

ctggtcaaag gattctaccc ttccgacatc gccgtggagt gggaaagtaa cggccagccc 1320

gagaacaatt acaagaccac accccctgtc ctggactctg atgggagttt cgctctggtg 1380

tcaaagctga ccgtcgataa aagccggtgg cagcagggca atgtgtttag ctgctccgtc 1440

atgcacgaag ccctgcacaa tcactacaca cagaagtccc tgagcctgag ccctggc 1497

<210> 227
<211> 499
<212> PRT
<213> Artificial Sequence

<220>
<223> amino acid sequence of C3E-8017

<400> 227

Met Arg Pro Thr Trp Ala Trp Trp Leu Phe Leu Val Leu Leu Leu Ala
1 5 10 15

Leu Trp Ala Pro Ala Arg Gly Gly Glu Val Gln Leu Val Glu Ser Gly

20 25 30

Gly Gly Leu Val Gln Pro Gly Gly Ser Leu Arg Leu Ser Cys Ala Ala
35 40 45

Ser Gly Val Thr Phe Asn Tyr Tyr Gly Met Ser Trp Ile Arg Gln Ala
50 55 60

Pro Gly Lys Gly Leu Glu Trp Val Ala Ser Ile Thr Arg Ser Gly Gly
65 70 75 80

Arg Ile Tyr Tyr Pro Asp Ser Val Lys Gly Arg Phe Thr Ile Ser Arg
85 90 95

Glu Asn Thr Gln Lys Thr Leu Tyr Leu Gln Met Asn Ser Leu Arg Ala
100 105 110

Glu Asp Thr Ala Val Tyr Tyr Cys Thr Leu Asp Gly Arg Asp Gly Trp
115 120 125

Val Ala Tyr Trp Gly Gln Gly Thr Leu Val Thr Val Ser Ser Gly Gly
130 135 140

Gly Gly Ser Gly Gly Gly Gly Ser Gly Gly Gly Gly Ser Asn Phe Met
145 150 155 160

Leu Thr Gln Pro His Ser Val Ser Glu Ser Pro Gly Lys Thr Val Thr
165 170 175

Ile Ser Cys Lys Arg Asn Thr Gly Asn Ile Gly Ser Asn Tyr Val Asn
180 185 190

Trp Tyr Gln Gln His Glu Gly Ser Ser Pro Thr Thr Ile Ile Tyr Arg
195 200 205

Asn Asp Lys Arg Pro Asp Gly Val Ser Asp Arg Phe Ser Gly Ser Ile
210 215 220

Asp Arg Ser Ser Lys Ser Ala Ser Leu Thr Ile Ser Asn Leu Lys Thr
225 230 235 240

Glu Asp Glu Ala Asp Tyr Phe Cys Gln Ser Tyr Ser Ser Gly Phe Ile
245 250 255

Phe Gly Gly Gly Thr Lys Leu Thr Val Leu Ala Ala Glu Pro Lys Ser
260 265 270

Ser Asp Lys Thr His Thr Cys Pro Pro Cys Pro Ala Pro Glu Ala Ala
        275                 280                 285

Gly Gly Pro Ser Val Phe Leu Phe Pro Pro Lys Pro Lys Asp Thr Leu
    290                 295                 300

Met Ile Ser Arg Thr Pro Glu Val Thr Cys Val Val Val Ser Val Ser
305                 310                 315                 320

His Glu Asp Pro Glu Val Lys Phe Asn Trp Tyr Val Asp Gly Val Glu
                325                 330                 335

Val His Asn Ala Lys Thr Lys Pro Arg Glu Glu Gln Tyr Asn Ser Thr
            340                 345                 350

Tyr Arg Val Val Ser Val Leu Thr Val Leu His Gln Asp Trp Leu Asn
        355                 360                 365

Gly Lys Glu Tyr Lys Cys Lys Val Ser Asn Lys Ala Leu Pro Ala Pro
    370                 375                 380

Ile Glu Lys Thr Ile Ser Lys Ala Lys Gly Gln Pro Arg Glu Pro Gln
385                 390                 395                 400

Val Tyr Val Tyr Pro Pro Ser Arg Asp Glu Leu Thr Lys Asn Gln Val
                405                 410                 415

Ser Leu Thr Cys Leu Val Lys Gly Phe Tyr Pro Ser Asp Ile Ala Val
            420                 425                 430

Glu Trp Glu Ser Asn Gly Gln Pro Glu Asn Asn Tyr Lys Thr Thr Pro
        435                 440                 445

Pro Val Leu Asp Ser Asp Gly Ser Phe Ala Leu Val Ser Lys Leu Thr
    450                 455                 460

Val Asp Lys Ser Arg Trp Gln Gln Gly Asn Val Phe Ser Cys Ser Val
465                 470                 475                 480

Met His Glu Ala Leu His Asn His Tyr Thr Gln Lys Ser Leu Ser Leu
                485                 490                 495

Ser Pro Gly

<210>  228
<211>  1497
<212>  DNA

<213> Artificial Sequence

<220>
<223> nucleotide sequence of C3E-8018

<400> 228
atgcgcccta cctgggcctg gtggctgttc ctggtgctgc tgctggccct gtgggcccca 60

gcccggggcg gagaagtgca gctggtggaa tccgggggg gcctggtgca gcctgggggg 120

agcctgagac tgagttgtgc cgcctctggg gtgacattta actactatgg catgtcttgg 180

atccgccagg cacctggaaa gggcctggag tgggtggcca gcatcacttc ttccggcggg 240

cgaatctact atcccgacag cgtcaagggc aggttcacaa tttcccgcga gaacacacag 300

aaaactctgt acctgcagat gaatagcctg agagccgaag atacagctgt gtactattgc 360

actctggacg gcagggatgg gtgggtcgcc tattgggggc agggaaccct ggtgacagtc 420

agctccggag gaggaggatc tggcggagga ggcagtgggg gaggcgggtc aaactttatg 480

ctgacccagc cccacagtgt gtcagagagc cctggcaaga ctgtcaccat ctcttgtaaa 540

aggaacaccg gaaatattgg cagtaactac gtgaattggt atcagcagca tgaagggtct 600

agtccaacca caatcatcta ccggaacgat aagagacccg acggggtgtc cgatcgattc 660

tccggatcta tcgaccggtc aagcaagagt gcttcactga ccattagcaa tctgaaaaca 720

gaggacgaag cagattactt ttgccagtcc tattcctctg gcttcatctt tggaggcggg 780

actaaactga ccgtgctggc ggccgagcct aaaagtagcg ataaaaccca tacctgcccc 840

ccctgcccgg cgccagaggc tgcaggagga ccaagcgtgt tcctgtttcc acccaagcct 900

aaagacacac tgatgatttc ccgaaccccc gaagtcacat gcgtggtcgt gtctgtgagt 960

cacgaggacc ctgaagtcaa gttcaactgg tacgtggatg gcgtcgaggt gcataatgcc 1020

aagactaaac ctagggagga acagtacaac tcaacctatc gcgtcgtgag cgtcctgaca 1080

gtgctgcacc aggattggct gaacggcaaa gaatataagt gcaaagtgag caataaggcc 1140

ctgcccgctc ctatcgagaa aaccatttcc aaggctaaag ggcagcctcg cgaaccacag 1200

gtctacgtct accccccatc aagagatgaa ctgacaaaaa atcaggtctc tctgacatgc 1260

ctggtcaaag gattctaccc ttccgacatc gccgtggagt gggaaagtaa cggccagccc 1320

gagaacaatt acaagaccac accccctgtc ctggactctg atgggagttt cgctctggtg 1380

tcaaagctga ccgtcgataa aagccggtgg cagcagggca atgtgtttag ctgctccgtc 1440

atgcacgaag ccctgcacaa tcactacaca cagaagtccc tgagcctgag ccctggc 1497

<210> 229
<211> 499
<212> PRT
<213> Artificial Sequence

<220>

<223>  amino acid sequence of C3E-8018

<400>  229

Met Arg Pro Thr Trp Ala Trp Trp Leu Phe Leu Val Leu Leu Leu Ala
1               5                   10                  15

Leu Trp Ala Pro Ala Arg Gly Gly Glu Val Gln Leu Val Glu Ser Gly
            20                  25                  30

Gly Gly Leu Val Gln Pro Gly Gly Ser Leu Arg Leu Ser Cys Ala Ala
        35                  40                  45

Ser Gly Val Thr Phe Asn Tyr Tyr Gly Met Ser Trp Ile Arg Gln Ala
    50                  55                  60

Pro Gly Lys Gly Leu Glu Trp Val Ala Ser Ile Thr Ser Ser Gly Gly
65                  70                  75                  80

Arg Ile Tyr Tyr Pro Asp Ser Val Lys Gly Arg Phe Thr Ile Ser Arg
                85                  90                  95

Glu Asn Thr Gln Lys Thr Leu Tyr Leu Gln Met Asn Ser Leu Arg Ala
            100                 105                 110

Glu Asp Thr Ala Val Tyr Tyr Cys Thr Leu Asp Gly Arg Asp Gly Trp
        115                 120                 125

Val Ala Tyr Trp Gly Gln Gly Thr Leu Val Thr Val Ser Ser Gly Gly
    130                 135                 140

Gly Gly Ser Gly Gly Gly Gly Ser Gly Gly Gly Gly Ser Asn Phe Met
145                 150                 155                 160

Leu Thr Gln Pro His Ser Val Ser Glu Ser Pro Gly Lys Thr Val Thr
                165                 170                 175

Ile Ser Cys Lys Arg Asn Thr Gly Asn Ile Gly Ser Asn Tyr Val Asn
            180                 185                 190

Trp Tyr Gln Gln His Glu Gly Ser Ser Pro Thr Thr Ile Ile Tyr Arg
        195                 200                 205

Asn Asp Lys Arg Pro Asp Gly Val Ser Asp Arg Phe Ser Gly Ser Ile
    210                 215                 220

Asp Arg Ser Ser Lys Ser Ala Ser Leu Thr Ile Ser Asn Leu Lys Thr
225                 230                 235                 240

Glu Asp Glu Ala Asp Tyr Phe Cys Gln Ser Tyr Ser Ser Gly Phe Ile
245 250 255

Phe Gly Gly Gly Thr Lys Leu Thr Val Leu Ala Ala Glu Pro Lys Ser
260 265 270

Ser Asp Lys Thr His Thr Cys Pro Pro Cys Pro Ala Pro Glu Ala Ala
275 280 285

Gly Gly Pro Ser Val Phe Leu Phe Pro Pro Lys Pro Lys Asp Thr Leu
290 295 300

Met Ile Ser Arg Thr Pro Glu Val Thr Cys Val Val Val Ser Val Ser
305 310 315 320

His Glu Asp Pro Glu Val Lys Phe Asn Trp Tyr Val Asp Gly Val Glu
325 330 335

Val His Asn Ala Lys Thr Lys Pro Arg Glu Glu Gln Tyr Asn Ser Thr
340 345 350

Tyr Arg Val Val Ser Val Leu Thr Val Leu His Gln Asp Trp Leu Asn
355 360 365

Gly Lys Glu Tyr Lys Cys Lys Val Ser Asn Lys Ala Leu Pro Ala Pro
370 375 380

Ile Glu Lys Thr Ile Ser Lys Ala Lys Gly Gln Pro Arg Glu Pro Gln
385 390 395 400

Val Tyr Val Tyr Pro Pro Ser Arg Asp Glu Leu Thr Lys Asn Gln Val
405 410 415

Ser Leu Thr Cys Leu Val Lys Gly Phe Tyr Pro Ser Asp Ile Ala Val
420 425 430

Glu Trp Glu Ser Asn Gly Gln Pro Glu Asn Asn Tyr Lys Thr Thr Pro
435 440 445

Pro Val Leu Asp Ser Asp Gly Ser Phe Ala Leu Val Ser Lys Leu Thr
450 455 460

Val Asp Lys Ser Arg Trp Gln Gln Gly Asn Val Phe Ser Cys Ser Val
465 470 475 480

Met His Glu Ala Leu His Asn His Tyr Thr Gln Lys Ser Leu Ser Leu
485 490 495

Ser Pro Gly

<210> 230
<211> 1494
<212> DNA
<213> Artificial Sequence

<220>
<223> nucleotide sequence of C3E-8025

<400> 230
atgcggccca cctgggcatg gtggctgttc ctggtgctgc tgctggccct gtgggcccct 60
gccaggggcg gagaagtgca gctggtggaa tccggggggg gcctggtgca gcctgggggg 120
agcctgagac tgagttgtgc cgcctctggg gtgacattta actactatgg catgtcttgg 180
atccgccagg cacctggaaa gggcctggag tgggtggcca gcatcactag gtccggcggg 240
cgaatctact atcccgacag cgtcaagggc aggttcacaa tttcccgcga gaacacacag 300
aaaactctgt acctgcagat gaatagcctg agagccgaag atacagctgt gtactattgc 360
actctggacg gcagggatgg gtgggtcgcc tattgggggc agggaaccct ggtgacagtc 420
agctccggag gaggaggatc tggcggagga ggcagtgggg gaggcgggtc aaactttatg 480
ctcactcagc cgtcctctgt ttctggcgta cctggccaac gggtgaccat tagctgtacg 540
ggtaataccg ggaatatcgg gtctaactac gtgaactggt atcagcagct tccagggaca 600
gctcccaagt tgctgatcta tcgcgacgac aaaagaccct caggggtccc tgaccgattt 660
agtggcagca aaagcggtac ttccgcttcc ctggcgataa ccggctttca ggccgaagat 720
gaggcagact actattgcca gtcatattcc agcggcttca tcttcggagg cggaactaag 780
ctgacagtgt tggcggccga gcctaaaagt agcgataaaa cccatacctg cccccctgc 840
ccggcgccag aggctgcagg aggaccaagc gtgttcctgt ttccacccaa gcctaaagac 900
acactgatga tttcccgaac cccgaagtc acatgcgtgg tcgtgtctgt gagtcacgag 960
gaccctgaag tcaagttcaa ctggtacgtg gatggcgtcg aggtgcataa tgccaagact 1020
aaacctaggg aggaacagta caactcaacc tatcgcgtcg tgagcgtcct gacagtgctg 1080
caccaggatt ggctgaacgg caaagaatat aagtgcaaag tgagcaataa ggccctgccc 1140
gctcctatcg agaaaaccat ttccaaggct aaagggcagc ctcgcgaacc acaggtctac 1200
gtctaccccc catcaagaga tgaactgaca aaaaatcagg tctctctgac atgcctggtc 1260
aaaggattct acccttccga catcgccgtg gagtgggaaa gtaacggcca gcccgagaac 1320
aattacaaga ccacaccccc tgtcctggac tctgatggga gtttcgctct ggtgtcaaag 1380
ctgaccgtcg ataaaagccg gtggcagcag ggcaatgtgt ttagctgctc cgtcatgcac 1440

gaagccctgc acaatcacta cacacagaag tccctgagcc tgagccctgg caag 1494

<210> 231
<211> 498
<212> PRT
<213> Artificial Sequence

<220>
<223> amino acid sequence of C3E-8025

<400> 231

```
Met Arg Pro Thr Trp Ala Trp Trp Leu Phe Leu Val Leu Leu Leu Ala
1               5                   10                  15

Leu Trp Ala Pro Ala Arg Gly Gly Glu Val Gln Leu Val Glu Ser Gly
            20                  25                  30

Gly Gly Leu Val Gln Pro Gly Gly Ser Leu Arg Leu Ser Cys Ala Ala
        35                  40                  45

Ser Gly Val Thr Phe Asn Tyr Tyr Gly Met Ser Trp Ile Arg Gln Ala
    50                  55                  60

Pro Gly Lys Gly Leu Glu Trp Val Ala Ser Ile Thr Arg Ser Gly Gly
65                  70                  75                  80

Arg Ile Tyr Tyr Pro Asp Ser Val Lys Gly Arg Phe Thr Ile Ser Arg
                85                  90                  95

Glu Asn Thr Gln Lys Thr Leu Tyr Leu Gln Met Asn Ser Leu Arg Ala
            100                 105                 110

Glu Asp Thr Ala Val Tyr Tyr Cys Thr Leu Asp Gly Arg Asp Gly Trp
        115                 120                 125

Val Ala Tyr Trp Gly Gln Gly Thr Leu Val Thr Val Ser Ser Gly Gly
    130                 135                 140

Gly Gly Ser Gly Gly Gly Gly Ser Gly Gly Gly Gly Ser Asn Phe Met
145                 150                 155                 160

Leu Thr Gln Pro Ser Ser Val Ser Gly Val Pro Gly Gln Arg Val Thr
                165                 170                 175

Ile Ser Cys Thr Gly Asn Thr Gly Asn Ile Gly Ser Asn Tyr Val Asn
            180                 185                 190

Trp Tyr Gln Gln Leu Pro Gly Thr Ala Pro Lys Leu Leu Ile Tyr Arg
        195                 200                 205
```

Asp Asp Lys Arg Pro Ser Gly Val Pro Asp Arg Phe Ser Gly Ser Lys
    210                 215                 220

Ser Gly Thr Ser Ala Ser Leu Ala Ile Thr Gly Phe Gln Ala Glu Asp
225                 230                 235                 240

Glu Ala Asp Tyr Tyr Cys Gln Ser Tyr Ser Ser Gly Phe Ile Phe Gly
                245                 250                 255

Gly Gly Thr Lys Leu Thr Val Leu Ala Ala Glu Pro Lys Ser Ser Asp
            260                 265                 270

Lys Thr His Thr Cys Pro Pro Cys Pro Ala Pro Glu Ala Ala Gly Gly
        275                 280                 285

Pro Ser Val Phe Leu Phe Pro Pro Lys Pro Lys Asp Thr Leu Met Ile
    290                 295                 300

Ser Arg Thr Pro Glu Val Thr Cys Val Val Val Ser Val Ser His Glu
305                 310                 315                 320

Asp Pro Glu Val Lys Phe Asn Trp Tyr Val Asp Gly Val Glu Val His
                325                 330                 335

Asn Ala Lys Thr Lys Pro Arg Glu Glu Gln Tyr Asn Ser Thr Tyr Arg
            340                 345                 350

Val Val Ser Val Leu Thr Val Leu His Gln Asp Trp Leu Asn Gly Lys
        355                 360                 365

Glu Tyr Lys Cys Lys Val Ser Asn Lys Ala Leu Pro Ala Pro Ile Glu
    370                 375                 380

Lys Thr Ile Ser Lys Ala Lys Gly Gln Pro Arg Glu Pro Gln Val Tyr
385                 390                 395                 400

Val Tyr Pro Pro Ser Arg Asp Glu Leu Thr Lys Asn Gln Val Ser Leu
                405                 410                 415

Thr Cys Leu Val Lys Gly Phe Tyr Pro Ser Asp Ile Ala Val Glu Trp
            420                 425                 430

Glu Ser Asn Gly Gln Pro Glu Asn Asn Tyr Lys Thr Thr Pro Pro Val
        435                 440                 445

Leu Asp Ser Asp Gly Ser Phe Ala Leu Val Ser Lys Leu Thr Val Asp

450 455 460

Lys Ser Arg Trp Gln Gln Gly Asn Val Phe Ser Cys Ser Val Met His
465 470 475 480

Glu Ala Leu His Asn His Tyr Thr Gln Lys Ser Leu Ser Leu Ser Pro
485 490 495

Gly Lys

<210> 232
<211> 1500
<212> DNA
<213> Artificial Sequence

<220>
<223> nucleotide sequence of C3E-8027

<400> 232
atgcgcccta cctgggcctg gtggctgttc ctggtgctgc tgctggccct gtgggcccca 60

gcccggggcg gagaagtgca gctggtggaa tccggggggg gcctggtgca gcctgggggg 120

agcctgagac tgagttgtgc cgcctctggg gtgacattta actactatgg catgtcttgg 180

atccgccagg cacctggaaa gggcctggag tgggtggcca gcatcactag gtccggcggg 240

cgaatctact atcccgacag cgtcaagggc aggttcacaa tttcccgcga gaacacacag 300

aaaactctgt acctgcagat gaatagcctg agagccgaag atacagctgt gtactattgc 360

actctggacg gcagggatgg gtgggtcgcc tattgggggc agggaaccct ggtgacagtc 420

agctccggag gaggaggatc tggcggagga ggcagtgggg gaggcgggtc aaactttatg 480

ctgacccagc cccacagtgt gtcagagagc cctggcaaga ctgtcaccat ctcttgtaaa 540

aggaacaccg gaaatattgg cagtaactac gtgaattggt atcagcagca tgaagggtct 600

agtccaacca caatcatcta ccggaacgat aagagacccg acggggtgtc cgatcgattc 660

tccggatcta tcgaccggtc aagcaagagt gcttcactga ccattagcaa tctgaaaaca 720

gaggacgaag cagattactt ttgccagtcc tattcctctg gcttcatctt tggaggcggg 780

actaaactga ccgtgctggc ggccgagcct aaaagtagcg ataaaaccca tacctgcccc 840

ccctgcccgg cgccagaggc tgcaggagga ccaagcgtgt tcctgtttcc acccaagcct 900

aaagacacac tgatgatttc ccgaaccccc gaagtcacat gcgtggtcgt gtctgtgagt 960

cacgaggacc ctgaagtcaa gttcaactgg tacgtggatg gcgtcgaggt gcataatgcc 1020

aagactaaac ctagggagga acagtacaac tcaacctatc gcgtcgtgag cgtcctgaca 1080

gtgctgcacc aggattggct gaacggcaaa gaatataagt gcaaagtgag caataaggcc 1140

ctgcccgctc ctatcgagaa aaccatttcc aaggctaaag ggcagcctcg cgaaccacag 1200

gtctacgtct acccccccatc aagagatgaa ctgacaaaaa atcaggtctc tctgacatgc 1260

ctggtcaaag gattctaccc ttccgacatc gccgtggagt gggaaagtaa cggccagccc 1320

gagaacaatt acaagaccac accccctgtc ctggactctg atgggagttt cgctctggtg 1380

tcaaagctga ccgtcgataa aagccggtgg cagcagggca atgtgtttag ctgctccgtc 1440

atgcacgaag ccctgcacaa tcactacaca cagaagtccc tgagcctgag ccctggcaag 1500

<210> 233
<211> 500
<212> PRT
<213> Artificial Sequence

<220>
<223> amino acid sequence of C3E-8027

<400> 233

Met Arg Pro Thr Trp Ala Trp Trp Leu Phe Leu Val Leu Leu Leu Ala
1               5                   10                  15

Leu Trp Ala Pro Ala Arg Gly Gly Glu Val Gln Leu Val Glu Ser Gly
            20                  25                  30

Gly Gly Leu Val Gln Pro Gly Gly Ser Leu Arg Leu Ser Cys Ala Ala
        35                  40                  45

Ser Gly Val Thr Phe Asn Tyr Tyr Gly Met Ser Trp Ile Arg Gln Ala
    50                  55                  60

Pro Gly Lys Gly Leu Glu Trp Val Ala Ser Ile Thr Arg Ser Gly Gly
65                  70                  75                  80

Arg Ile Tyr Tyr Pro Asp Ser Val Lys Gly Arg Phe Thr Ile Ser Arg
                85                  90                  95

Glu Asn Thr Gln Lys Thr Leu Tyr Leu Gln Met Asn Ser Leu Arg Ala
            100                 105                 110

Glu Asp Thr Ala Val Tyr Tyr Cys Thr Leu Asp Gly Arg Asp Gly Trp
        115                 120                 125

Val Ala Tyr Trp Gly Gln Gly Thr Leu Val Thr Val Ser Ser Gly Gly
    130                 135                 140

Gly Gly Ser Gly Gly Gly Gly Ser Gly Gly Gly Gly Ser Asn Phe Met
145                 150                 155                 160

Leu Thr Gln Pro His Ser Val Ser Glu Ser Pro Gly Lys Thr Val Thr

165 170 175

Ile Ser Cys Lys Arg Asn Thr Gly Asn Ile Gly Ser Asn Tyr Val Asn
180 185 190

Trp Tyr Gln Gln His Glu Gly Ser Ser Pro Thr Thr Ile Ile Tyr Arg
195 200 205

Asn Asp Lys Arg Pro Asp Gly Val Ser Asp Arg Phe Ser Gly Ser Ile
210 215 220

Asp Arg Ser Ser Lys Ser Ala Ser Leu Thr Ile Ser Asn Leu Lys Thr
225 230 235 240

Glu Asp Glu Ala Asp Tyr Phe Cys Gln Ser Tyr Ser Ser Gly Phe Ile
245 250 255

Phe Gly Gly Gly Thr Lys Leu Thr Val Leu Ala Ala Glu Pro Lys Ser
260 265 270

Ser Asp Lys Thr His Thr Cys Pro Pro Cys Pro Ala Pro Glu Ala Ala
275 280 285

Gly Gly Pro Ser Val Phe Leu Phe Pro Pro Lys Pro Lys Asp Thr Leu
290 295 300

Met Ile Ser Arg Thr Pro Glu Val Thr Cys Val Val Val Ser Val Ser
305 310 315 320

His Glu Asp Pro Glu Val Lys Phe Asn Trp Tyr Val Asp Gly Val Glu
325 330 335

Val His Asn Ala Lys Thr Lys Pro Arg Glu Glu Gln Tyr Asn Ser Thr
340 345 350

Tyr Arg Val Val Ser Val Leu Thr Val Leu His Gln Asp Trp Leu Asn
355 360 365

Gly Lys Glu Tyr Lys Cys Lys Val Ser Asn Lys Ala Leu Pro Ala Pro
370 375 380

Ile Glu Lys Thr Ile Ser Lys Ala Lys Gly Gln Pro Arg Glu Pro Gln
385 390 395 400

Val Tyr Val Tyr Pro Pro Ser Arg Asp Glu Leu Thr Lys Asn Gln Val
405 410 415

Ser Leu Thr Cys Leu Val Lys Gly Phe Tyr Pro Ser Asp Ile Ala Val
420 425 430

Glu Trp Glu Ser Asn Gly Gln Pro Glu Asn Asn Tyr Lys Thr Thr Pro
435 440 445

Pro Val Leu Asp Ser Asp Gly Ser Phe Ala Leu Val Ser Lys Leu Thr
450 455 460

Val Asp Lys Ser Arg Trp Gln Gln Gly Asn Val Phe Ser Cys Ser Val
465 470 475 480

Met His Glu Ala Leu His Asn His Tyr Thr Gln Lys Ser Leu Ser Leu
485 490 495

Ser Pro Gly Lys
500

<210> 234
<211> 1500
<212> DNA
<213> Artificial Sequence

<220>
<223> nucleotide sequence of C3E-8028

<400> 234
atgcgcccta cctgggcctg gtggctgttc ctggtgctgc tgctggccct gtgggcccca 60

gcccggggcg gagaagtgca gctggtggaa tccggggggg gcctggtgca gcctgggggg 120

agcctgagac tgagttgtgc cgcctctggg gtgacattta actactatgg catgtcttgg 180

atccgccagg cacctggaaa gggcctggag tgggtggcca gcatcacttc ttccggcggg 240

cgaatctact atcccgacag cgtcaagggc aggttcacaa tttcccgcga gaacacacag 300

aaaactctgt acctgcagat gaatagcctg agagccgaag atacagctgt gtactattgc 360

actctggacg gcagggatgg gtgggtcgcc tattgggggc agggaaccct ggtgacagtc 420

agctccggag gaggaggatc tggcggagga ggcagtgggg gaggcgggtc aaactttatg 480

ctgacccagc cccacagtgt gtcagagagc cctggcaaga ctgtcaccat ctcttgtaaa 540

aggaacaccg gaaatattgg cagtaactac gtgaattggt atcagcagca tgaagggtct 600

agtccaacca caatcatcta ccggaacgat aagagacccg acggggtgtc cgatcgattc 660

tccggatcta tcgaccggtc aagcaagagt gcttcactga ccattagcaa tctgaaaaca 720

gaggacgaag cagattactt ttgccagtcc tattcctctg gcttcatctt tggaggcggg 780

actaaactga ccgtgctggc ggccgagcct aaaagtagcg ataaaaccca tacctgcccc 840

ccctgcccgg cgccagaggc tgcaggagga ccaagcgtgt tcctgtttcc acccaagcct 900

aaagacacac tgatgatttc ccgaaccccc gaagtcacat gcgtggtcgt gtctgtgagt 960

cacgaggacc ctgaagtcaa gttcaactgg tacgtggatg gcgtcgaggt gcataatgcc 1020

aagactaaac ctagggagga acagtacaac tcaacctatc gcgtcgtgag cgtcctgaca 1080

gtgctgcacc aggattggct gaacggcaaa gaatataagt gcaaagtgag caataaggcc 1140

ctgcccgctc ctatcgagaa aaccatttcc aaggctaaag ggcagcctcg cgaaccacag 1200

gtctacgtct accccccatc aagagatgaa ctgacaaaaa atcaggtctc tctgacatgc 1260

ctggtcaaag gattctaccc ttccgacatc gccgtggagt gggaaagtaa cggccagccc 1320

gagaacaatt acaagaccac acccccctgtc ctggactctg atgggagttt cgctctggtg 1380

tcaaagctga ccgtcgataa aagccggtgg cagcagggca atgtgtttag ctgctccgtc 1440

atgcacgaag ccctgcacaa tcactacaca cagaagtccc tgagcctgag ccctggcaag 1500

<210> 235
<211> 500
<212> PRT
<213> Artificial Sequence

<220>
<223> amino acid sequence of C3E-8028

<400> 235

Met Arg Pro Thr Trp Ala Trp Trp Leu Phe Leu Val Leu Leu Leu Ala
1 5 10 15

Leu Trp Ala Pro Ala Arg Gly Gly Glu Val Gln Leu Val Glu Ser Gly
20 25 30

Gly Gly Leu Val Gln Pro Gly Gly Ser Leu Arg Leu Ser Cys Ala Ala
35 40 45

Ser Gly Val Thr Phe Asn Tyr Tyr Gly Met Ser Trp Ile Arg Gln Ala
50 55 60

Pro Gly Lys Gly Leu Glu Trp Val Ala Ser Ile Thr Ser Ser Gly Gly
65 70 75 80

Arg Ile Tyr Tyr Pro Asp Ser Val Lys Gly Arg Phe Thr Ile Ser Arg
85 90 95

Glu Asn Thr Gln Lys Thr Leu Tyr Leu Gln Met Asn Ser Leu Arg Ala
100 105 110

Glu Asp Thr Ala Val Tyr Tyr Cys Thr Leu Asp Gly Arg Asp Gly Trp
115 120 125

Val Ala Tyr Trp Gly Gln Gly Thr Leu Val Thr Val Ser Ser Gly Gly
130 135 140

Gly Gly Ser Gly Gly Gly Gly Ser Gly Gly Gly Gly Ser Asn Phe Met
145 150 155 160

Leu Thr Gln Pro His Ser Val Ser Glu Ser Pro Gly Lys Thr Val Thr
165 170 175

Ile Ser Cys Lys Arg Asn Thr Gly Asn Ile Gly Ser Asn Tyr Val Asn
180 185 190

Trp Tyr Gln Gln His Glu Gly Ser Ser Pro Thr Thr Ile Ile Tyr Arg
195 200 205

Asn Asp Lys Arg Pro Asp Gly Val Ser Asp Arg Phe Ser Gly Ser Ile
210 215 220

Asp Arg Ser Ser Lys Ser Ala Ser Leu Thr Ile Ser Asn Leu Lys Thr
225 230 235 240

Glu Asp Glu Ala Asp Tyr Phe Cys Gln Ser Tyr Ser Ser Gly Phe Ile
245 250 255

Phe Gly Gly Gly Thr Lys Leu Thr Val Leu Ala Ala Glu Pro Lys Ser
260 265 270

Ser Asp Lys Thr His Thr Cys Pro Pro Cys Pro Ala Pro Glu Ala Ala
275 280 285

Gly Gly Pro Ser Val Phe Leu Phe Pro Pro Lys Pro Lys Asp Thr Leu
290 295 300

Met Ile Ser Arg Thr Pro Glu Val Thr Cys Val Val Val Ser Val Ser
305 310 315 320

His Glu Asp Pro Glu Val Lys Phe Asn Trp Tyr Val Asp Gly Val Glu
325 330 335

Val His Asn Ala Lys Thr Lys Pro Arg Glu Glu Gln Tyr Asn Ser Thr
340 345 350

Tyr Arg Val Val Ser Val Leu Thr Val Leu His Gln Asp Trp Leu Asn
355 360 365

Gly Lys Glu Tyr Lys Cys Lys Val Ser Asn Lys Ala Leu Pro Ala Pro
370 375 380

Ile Glu Lys Thr Ile Ser Lys Ala Lys Gly Gln Pro Arg Glu Pro Gln
385 390 395 400

Val Tyr Val Tyr Pro Pro Ser Arg Asp Glu Leu Thr Lys Asn Gln Val
405 410 415

Ser Leu Thr Cys Leu Val Lys Gly Phe Tyr Pro Ser Asp Ile Ala Val
420 425 430

Glu Trp Glu Ser Asn Gly Gln Pro Glu Asn Asn Tyr Lys Thr Thr Pro
435 440 445

Pro Val Leu Asp Ser Asp Gly Ser Phe Ala Leu Val Ser Lys Leu Thr
450 455 460

Val Asp Lys Ser Arg Trp Gln Gln Gly Asn Val Phe Ser Cys Ser Val
465 470 475 480

Met His Glu Ala Leu His Asn His Tyr Thr Gln Lys Ser Leu Ser Leu
485 490 495

Ser Pro Gly Lys
500

<210> 236
<211> 1428
<212> DNA
<213> Artificial Sequence

<220>
<223> nucleotide sequence of h2B1 Fab HC_4

<400> 236
atgaggccta cctgggcctg gtggctgttc ctggtgctgc tgctggccct gtgggccccc 60

gcccggggcc aggtgcagct ggtggagtcc ggcggcggcc tggtgaagcc cagccagacc 120

ctgtccctga cctgcacatt cagcggcttt tccctgaaca catacgatat gggagtggga 180

tggatcagac agccacctgg caagggcctg gagtggatcg gcaacatctg gtgggacgat 240

gacaagtact ataatccctc tctgaagagc agagtgacca tctctaagga tacaagcaac 300

aatcaggcct ccctgaagct gagctccgtg accgccgccg acacagccgt gtactattgc 360

gccaggatcg agacagtgcg ggtgagccgg aagggattcg cacactgggg acagggcacc 420

ctggtgacag tgtctagcgc tagcacaaag ggacctagcg tgttcccact ggccccttcc 480

tctaagtcca cctctggagg aacagccgcc ctgggctgtc tggtgaagga ctatttccct 540

gagccagtga ccgtgtcctg gaactctggg gccctgacca gcggagtgca cacatttcct 600

gccgtgctgc agagctccgg cctgtacagc ctgtctagcg tggtgaccgt gccatcctct 660

agcctgggca cccagacata tatctgcaac gtgaatcaca agccaagcaa tacaaaggtc 720

gacaagagag tggagcccaa gtcctgtgac aagacccaca catgcccacc ctgtccggcg 780

ccagaggctg caggaggacc aagcgtgttc ctgtttccac ccaagcctaa agacacactg 840

atgatttccc gaacccccga agtcacatgc gtggtcgtgt ctgtgagtca cgaggaccct 900

gaagtcaagt tcaactggta cgtggatggc gtcgaggtgc ataatgccaa gactaaacct 960

agggaggaac agtacaactc aacctatcgc gtcgtgagcg tcctgacagt gctgcaccag 1020

gattggctga acggcaaaga atataagtgc aaagtgagca ataaggccct gcccgctcct 1080

atcgagaaaa ccatttccaa ggctaaaggg cagcctcgcg aaccacaggt ctacgtgctg 1140

ccccctagcc gcgacgaact gactaaaaat caggtctctc tgctgtgtct ggtcaaagga 1200

ttctaccctt ccgacatcgc cgtggagtgg gaaagtaacg gccagcccga gaacaattac 1260

ctgacctggc cccctgtgct ggactctgat gggagtttct ttctgtattc aaagctgaca 1320

gtcgataaaa gccggtggca gcagggcaat gtgttcagct gctccgtcat gcacgaagca 1380

ctgcacaacc attacactca gaagtccctg tccctgtcac ctggcaag 1428

<210> 237
<211> 476
<212> PRT
<213> Artificial Sequence

<220>
<223> nucleotide_sequence_of_h2B1_Fab_HC_4

<400> 237

Met Arg Pro Thr Trp Ala Trp Trp Leu Phe Leu Val Leu Leu Leu Ala
1 5 10 15

Leu Trp Ala Pro Ala Arg Gly Gln Val Gln Leu Val Glu Ser Gly Gly
20 25 30

Gly Leu Val Lys Pro Ser Gln Thr Leu Ser Leu Thr Cys Thr Phe Ser
35 40 45

Gly Phe Ser Leu Asn Thr Tyr Asp Met Gly Val Gly Trp Ile Arg Gln
50 55 60

Pro Pro Gly Lys Gly Leu Glu Trp Ile Gly Asn Ile Trp Trp Asp Asp
65 70 75 80

Asp Lys Tyr Tyr Asn Pro Ser Leu Lys Ser Arg Val Thr Ile Ser Lys
85 90 95

Asp Thr Ser Asn Asn Gln Ala Ser Leu Lys Leu Ser Ser Val Thr Ala

100 105 110

Ala Asp Thr Ala Val Tyr Tyr Cys Ala Arg Ile Glu Thr Val Arg Val
115 120 125

Ser Arg Lys Gly Phe Ala His Trp Gly Gln Gly Thr Leu Val Thr Val
130 135 140

Ser Ser Ala Ser Thr Lys Gly Pro Ser Val Phe Pro Leu Ala Pro Ser
145 150 155 160

Ser Lys Ser Thr Ser Gly Gly Thr Ala Ala Leu Gly Cys Leu Val Lys
165 170 175

Asp Tyr Phe Pro Glu Pro Val Thr Val Ser Trp Asn Ser Gly Ala Leu
180 185 190

Thr Ser Gly Val His Thr Phe Pro Ala Val Leu Gln Ser Ser Gly Leu
195 200 205

Tyr Ser Leu Ser Ser Val Val Thr Val Pro Ser Ser Ser Leu Gly Thr
210 215 220

Gln Thr Tyr Ile Cys Asn Val Asn His Lys Pro Ser Asn Thr Lys Val
225 230 235 240

Asp Lys Arg Val Glu Pro Lys Ser Cys Asp Lys Thr His Thr Cys Pro
245 250 255

Pro Cys Pro Ala Pro Glu Ala Ala Gly Gly Pro Ser Val Phe Leu Phe
260 265 270

Pro Pro Lys Pro Lys Asp Thr Leu Met Ile Ser Arg Thr Pro Glu Val
275 280 285

Thr Cys Val Val Val Ser Val Ser His Glu Asp Pro Glu Val Lys Phe
290 295 300

Asn Trp Tyr Val Asp Gly Val Glu Val His Asn Ala Lys Thr Lys Pro
305 310 315 320

Arg Glu Glu Gln Tyr Asn Ser Thr Tyr Arg Val Val Ser Val Leu Thr
325 330 335

Val Leu His Gln Asp Trp Leu Asn Gly Lys Glu Tyr Lys Cys Lys Val
340 345 350

Ser Asn Lys Ala Leu Pro Ala Pro Ile Glu Lys Thr Ile Ser Lys Ala
        355                 360                 365

Lys Gly Gln Pro Arg Glu Pro Gln Val Tyr Val Leu Pro Pro Ser Arg
    370                 375                 380

Asp Glu Leu Thr Lys Asn Gln Val Ser Leu Leu Cys Leu Val Lys Gly
385                 390                 395                 400

Phe Tyr Pro Ser Asp Ile Ala Val Glu Trp Glu Ser Asn Gly Gln Pro
                405                 410                 415

Glu Asn Asn Tyr Leu Thr Trp Pro Pro Val Leu Asp Ser Asp Gly Ser
            420                 425                 430

Phe Phe Leu Tyr Ser Lys Leu Thr Val Asp Lys Ser Arg Trp Gln Gln
        435                 440                 445

Gly Asn Val Phe Ser Cys Ser Val Met His Glu Ala Leu His Asn His
    450                 455                 460

Tyr Thr Gln Lys Ser Leu Ser Leu Ser Pro Gly Lys
465                 470                 475

<210>  238
<211>  8
<212>  PRT
<213>  Artificial Sequence

<220>
<223>  amino_acid_sequence_of_modified_H_CDR2

<220>
<221>  MISC_FEATURE
<222>  (2)..(2)
<223>  The first Xaa is any naturally occurring amino acid

<220>
<221>  MISC_FEATURE
<222>  (3)..(3)
<223>  The second Xaa is any naturally occurring amino acid

<220>
<221>  misc_feature
<222>  (4)..(4)
<223>  Xaa can be any naturally occurring amino acid

<400>  238

Ile Thr Xaa Xaa Gly Gly Arg Ile
1               5

<210>  239

<211> 3
<212> PRT
<213> Artificial Sequence

<220>
<223> amino_acid_sequence_of_modified_L_CDR2

<220>
<221> MISC_FEATURE
<222> (2)..(2)
<223> Xaa is any naturally occurring amino acid

<220>
<221> misc_feature
<222> (5)..(5)
<223> Xaa can be any naturally occurring amino acid

<400> 239

Arg Xaa Asp
1

<210> 240
<211> 118
<212> PRT
<213> Artificial Sequence

<220>
<223> amino_acid_sequence_of_VH_of_variant_of_C3E-7034

<220>
<221> MISC_FEATURE
<222> (53)..(54)
<223> The first and second Xaa's are any naturally occurring amino acid

<400> 240

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Val Thr Phe Asn Tyr Tyr
            20                  25                  30

Gly Met Ser Trp Ile Arg Gln Ala Pro Gly Lys Gly Leu Glu Trp Val
        35                  40                  45

Ala Ser Ile Thr Xaa Xaa Gly Gly Arg Ile Tyr Tyr Pro Asp Ser Val
    50                  55                  60

Lys Gly Arg Phe Thr Ile Ser Arg Glu Asn Thr Gln Lys Thr Leu Tyr
65                  70                  75                  80

Leu Gln Met Asn Ser Leu Arg Ala Glu Asp Thr Ala Val Tyr Tyr Cys
                85                  90                  95
```

Thr Leu Asp Gly Arg Asp Gly Trp Val Ala Tyr Trp Gly Gln Gly Thr
100 105 110

Leu Val Thr Val Ser Ser
115

<210> 241
<211> 109
<212> PRT
<213> Artificial Sequence

<220>
<223> amino_acid_sequence_of_VL_of_variant_of_C3E-7034

<220>
<221> MISC_FEATURE
<222> (52)..(52)
<223> Xaa is any naturally occurring amino acid

<400> 241

Asn Phe Met Leu Thr Gln Pro His Ser Val Ser Glu Ser Pro Gly Lys
1 5 10 15

Thr Val Thr Ile Ser Cys Lys Arg Asn Thr Gly Asn Ile Gly Ser Asn
20 25 30

Tyr Val Asn Trp Tyr Gln Gln His Glu Gly Ser Ser Pro Thr Thr Ile
35 40 45

Ile Tyr Arg Xaa Asp Lys Arg Pro Asp Gly Val Ser Asp Arg Phe Ser
50 55 60

Gly Ser Ile Asp Arg Ser Ser Lys Ser Ala Ser Leu Thr Ile Ser Asn
65 70 75 80

Leu Lys Thr Glu Asp Glu Ala Asp Tyr Phe Cys Gln Ser Tyr Ser Ser
85 90 95

Gly Phe Ile Phe Gly Gly Gly Thr Lys Leu Thr Val Leu
100 105

<210> 242
<211> 107
<212> PRT
<213> Artificial Sequence

<220>
<223> amino_acid_sequence_of_VL_of_variant_of_C3E-7035

<220>

<221> MISC_FEATURE
<222> (52)..(52)
<223> Xaa is any naturally occurring amino acid

<400> 242

Gln Ala Val Leu Thr Gln Pro Ser Ser Val Ser Gly Val Pro Gly Gln
1 5 10 15

Arg Val Thr Ile Ser Cys Lys Arg Asn Thr Gly Asn Ile Gly Ser Asn
20 25 30

Tyr Val Asn Trp Tyr Gln Gln Leu Pro Gly Thr Ala Pro Lys Leu Leu
35 40 45

Ile Tyr Arg Xaa Asp Lys Arg Pro Ser Gly Val Pro Asp Arg Phe Ser
50 55 60

Gly Ser Lys Ser Gly Thr Ser Ala Ser Leu Ala Ile Thr Gly Phe Gln
65 70 75 80

Ala Glu Asp Glu Ala Asp Tyr Tyr Cys Gln Ser Tyr Ser Ser Gly Phe
85 90 95

Ile Phe Gly Gly Gly Thr Lys Leu Thr Val Leu
100 105

<210> 243
<211> 107
<212> PRT
<213> Artificial Sequence

<220>
<223> amino_acid_sequence_of_VL_of_variant_of_C3E-7036

<220>
<221> MISC_FEATURE
<222> (52)..(52)
<223> Xaa is any naturally occurring amino acid

<400> 243

Asn Phe Met Leu Thr Gln Pro Ser Ser Val Ser Gly Val Pro Gly Gln
1 5 10 15

Arg Val Thr Ile Ser Cys Thr Gly Asn Thr Gly Asn Ile Gly Ser Asn
20 25 30

Tyr Val Asn Trp Tyr Gln Gln Leu Pro Gly Thr Ala Pro Lys Leu Leu
35 40 45

Ile Tyr Arg Xaa Asp Lys Arg Pro Ser Gly Val Pro Asp Arg Phe Ser

50 55 60

Gly Ser Lys Ser Gly Thr Ser Ala Ser Leu Ala Ile Thr Gly Phe Gln
65 70 75 80

Ala Glu Asp Glu Ala Asp Tyr Tyr Cys Gln Ser Tyr Ser Ser Gly Phe
85 90 95

Ile Phe Gly Gly Gly Thr Lys Leu Thr Val Leu
100 105

<210> 244
<211> 269
<212> PRT
<213> Artificial Sequence

<220>
<223> amino_acid_sequence_of_C3E-7078

<400> 244

Gly Glu Val Gln Leu Val Glu Ser Gly Gly Gly Leu Val Gln Pro Gly
1 5 10 15

Gly Ser Leu Arg Leu Ser Cys Ala Ala Ser Gly Val Thr Phe Asn Tyr
20 25 30

Tyr Gly Met Ser Trp Ile Arg Gln Ala Pro Gly Lys Gly Leu Glu Trp
35 40 45

Val Ala Ser Ile Thr Arg Ser Gly Gly Arg Ile Tyr Tyr Pro Asp Ser
50 55 60

Val Lys Gly Arg Phe Thr Ile Ser Arg Glu Asn Thr Gln Lys Thr Leu
65 70 75 80

Tyr Leu Gln Met Asn Ser Leu Arg Ala Glu Asp Thr Ala Val Tyr Tyr
85 90 95

Cys Thr Leu Asp Gly Arg Asp Gly Trp Val Ala Tyr Trp Gly Gln Gly
100 105 110

Thr Leu Val Thr Val Ser Ser Gly Gly Gly Gly Ser Gly Gly Gly Gly
115 120 125

Ser Gly Gly Gly Gly Ser Asn Phe Met Leu Thr Gln Pro His Ser Val
130 135 140

Ser Glu Ser Pro Gly Lys Thr Val Thr Ile Ser Cys Lys Arg Asn Thr
145 150 155 160

Gly Asn Ile Gly Ser Asn Tyr Val Asn Trp Tyr Gln Gln His Glu Gly
165 170 175

Ser Ser Pro Thr Thr Ile Ile Tyr Arg Asp Asp Lys Arg Pro Asp Gly
180 185 190

Val Ser Asp Arg Phe Ser Gly Ser Ile Asp Arg Ser Ser Lys Ser Ala
195 200 205

Ser Leu Thr Ile Ser Asn Leu Lys Thr Glu Asp Glu Ala Asp Tyr Phe
210 215 220

Cys Gln Ser Tyr Ser Ser Gly Phe Ile Phe Gly Gly Gly Thr Lys Leu
225 230 235 240

Thr Val Leu Gly Ala Ala Ala Gly Ala Gly Gly Asp Tyr Lys Asp Asp
245 250 255

Asp Asp Lys Gly Ala Ala Ala His His His His His His
260 265

<210> 245
<211> 267
<212> PRT
<213> Artificial Sequence

<220>
<223> amino_acid_sequence_of_C3E-7085

<400> 245

Gly Glu Val Gln Leu Val Glu Ser Gly Gly Gly Leu Val Gln Pro Gly
1 5 10 15

Gly Ser Leu Arg Leu Ser Cys Ala Ala Ser Gly Val Thr Phe Asn Tyr
20 25 30

Tyr Gly Met Ser Trp Ile Arg Gln Ala Pro Gly Lys Gly Leu Glu Trp
35 40 45

Val Ala Ser Ile Thr Arg Ser Gly Gly Arg Ile Tyr Tyr Pro Asp Ser
50 55 60

Val Lys Gly Arg Phe Thr Ile Ser Arg Glu Asn Thr Gln Lys Thr Leu
65 70 75 80

Tyr Leu Gln Met Asn Ser Leu Arg Ala Glu Asp Thr Ala Val Tyr Tyr
85 90 95

```
Cys Thr Leu Asp Gly Arg Asp Gly Trp Val Ala Tyr Trp Gly Gln Gly
            100                 105                 110

Thr Leu Val Thr Val Ser Ser Gly Gly Gly Gly Ser Gly Gly Gly Gly
        115                 120                 125

Ser Gly Gly Gly Gly Ser Asn Phe Met Leu Thr Gln Pro Ser Ser Val
    130                 135                 140

Ser Gly Val Pro Gly Gln Arg Val Thr Ile Ser Cys Thr Gly Asn Thr
145                 150                 155                 160

Gly Asn Ile Gly Ser Asn Tyr Val Asn Trp Tyr Gln Gln Leu Pro Gly
                165                 170                 175

Thr Ala Pro Lys Leu Leu Ile Tyr Arg Asp Asp Lys Arg Pro Ser Gly
            180                 185                 190

Val Pro Asp Arg Phe Ser Gly Ser Lys Ser Gly Thr Ser Ala Ser Leu
        195                 200                 205

Ala Ile Thr Gly Phe Gln Ala Glu Asp Glu Ala Asp Tyr Tyr Cys Gln
    210                 215                 220

Ser Tyr Ser Ser Gly Phe Ile Phe Gly Gly Gly Thr Lys Leu Thr Val
225                 230                 235                 240

Leu Gly Ala Ala Ala Gly Ala Gly Gly Asp Tyr Lys Asp Asp Asp Asp
                245                 250                 255

Lys Gly Ala Ala Ala His His His His His His
            260                 265


<210>  246
<211>  269
<212>  PRT
<213>  Artificial Sequence

<220>
<223>  amino_acid_sequence_of_C3E-7086

<400>  246

Gly Glu Val Gln Leu Val Glu Ser Gly Gly Gly Leu Val Gln Pro Gly
1               5                   10                  15

Gly Ser Leu Arg Leu Ser Cys Ala Ala Ser Gly Val Thr Phe Asn Tyr
            20                  25                  30
```

```
Tyr Gly Met Ser Trp Ile Arg Gln Ala Pro Gly Lys Gly Leu Glu Trp
        35                  40                  45

Val Ala Ser Ile Thr Arg Ser Gly Gly Arg Ile Tyr Tyr Pro Asp Ser
    50                  55                  60

Val Lys Gly Arg Phe Thr Ile Ser Arg Glu Asn Thr Gln Lys Thr Leu
65                  70                  75                  80

Tyr Leu Gln Met Asn Ser Leu Arg Ala Glu Asp Thr Ala Val Tyr Tyr
                85                  90                  95

Cys Thr Leu Asp Gly Arg Asp Gly Trp Val Ala Tyr Trp Gly Gln Gly
            100                 105                 110

Thr Leu Val Thr Val Ser Ser Gly Gly Gly Gly Ser Gly Gly Gly Gly
        115                 120                 125

Ser Gly Gly Gly Gly Ser Asn Phe Met Leu Thr Gln Pro His Ser Val
    130                 135                 140

Ser Glu Ser Pro Gly Lys Thr Val Thr Ile Ser Cys Lys Arg Asn Thr
145                 150                 155                 160

Gly Asn Ile Gly Ser Asn Tyr Val Asn Trp Tyr Gln Gln His Glu Gly
                165                 170                 175

Ser Ser Pro Thr Thr Ile Ile Tyr Arg Gly Asp Lys Arg Pro Asp Gly
            180                 185                 190

Val Ser Asp Arg Phe Ser Gly Ser Ile Asp Arg Ser Ser Lys Ser Ala
        195                 200                 205

Ser Leu Thr Ile Ser Asn Leu Lys Thr Glu Asp Glu Ala Asp Tyr Phe
    210                 215                 220

Cys Gln Ser Tyr Ser Ser Gly Phe Ile Phe Gly Gly Gly Thr Lys Leu
225                 230                 235                 240

Thr Val Leu Gly Ala Ala Ala Gly Ala Gly Gly Asp Tyr Lys Asp Asp
                245                 250                 255

Asp Asp Lys Gly Ala Ala Ala His His His His His His
            260                 265
```

<210> 247
<211> 269
<212> PRT

<213> Artificial Sequence

<220>
<223> amino_acid_sequence_of_C3E-7087

<400> 247

```
Gly Glu Val Gln Leu Val Glu Ser Gly Gly Gly Leu Val Gln Pro Gly
1               5                   10                  15

Gly Ser Leu Arg Leu Ser Cys Ala Ala Ser Gly Val Thr Phe Asn Tyr
            20                  25                  30

Tyr Gly Met Ser Trp Ile Arg Gln Ala Pro Gly Lys Gly Leu Glu Trp
        35                  40                  45

Val Ala Ser Ile Thr Arg Ser Gly Gly Arg Ile Tyr Tyr Pro Asp Ser
    50                  55                  60

Val Lys Gly Arg Phe Thr Ile Ser Arg Glu Asn Thr Gln Lys Thr Leu
65                  70                  75                  80

Tyr Leu Gln Met Asn Ser Leu Arg Ala Glu Asp Thr Ala Val Tyr Tyr
                85                  90                  95

Cys Thr Leu Asp Gly Arg Asp Gly Trp Val Ala Tyr Trp Gly Gln Gly
            100                 105                 110

Thr Leu Val Thr Val Ser Ser Gly Gly Gly Gly Ser Gly Gly Gly Gly
        115                 120                 125

Ser Gly Gly Gly Gly Ser Asn Phe Met Leu Thr Gln Pro His Ser Val
    130                 135                 140

Ser Glu Ser Pro Gly Lys Thr Val Thr Ile Ser Cys Lys Arg Asn Thr
145                 150                 155                 160

Gly Asn Ile Gly Ser Asn Tyr Val Asn Trp Tyr Gln Gln His Glu Gly
                165                 170                 175

Ser Ser Pro Thr Thr Ile Ile Tyr Arg Gln Asp Lys Arg Pro Asp Gly
            180                 185                 190

Val Ser Asp Arg Phe Ser Gly Ser Ile Asp Arg Ser Ser Lys Ser Ala
        195                 200                 205

Ser Leu Thr Ile Ser Asn Leu Lys Thr Glu Asp Glu Ala Asp Tyr Phe
    210                 215                 220
```

Cys Gln Ser Tyr Ser Ser Gly Phe Ile Phe Gly Gly Gly Thr Lys Leu
225                 230                 235                 240

Thr Val Leu Gly Ala Ala Ala Gly Ala Gly Gly Asp Tyr Lys Asp Asp
                245                 250                 255

Asp Asp Lys Gly Ala Ala Ala His His His His His His
            260                 265

<210>  248
<211>  269
<212>  PRT
<213>  Artificial Sequence

<220>
<223>  amino_acid_sequence_of_C3E-7088

<400>  248

Gly Glu Val Gln Leu Val Glu Ser Gly Gly Gly Leu Val Gln Pro Gly
1               5                   10                  15

Gly Ser Leu Arg Leu Ser Cys Ala Ala Ser Gly Val Thr Phe Asn Tyr
            20                  25                  30

Tyr Gly Met Ser Trp Ile Arg Gln Ala Pro Gly Lys Gly Leu Glu Trp
        35                  40                  45

Val Ala Ser Ile Thr Arg Ser Gly Gly Arg Ile Tyr Tyr Pro Asp Ser
    50                  55                  60

Val Lys Gly Arg Phe Thr Ile Ser Arg Glu Asn Thr Gln Lys Thr Leu
65                  70                  75                  80

Tyr Leu Gln Met Asn Ser Leu Arg Ala Glu Asp Thr Ala Val Tyr Tyr
                85                  90                  95

Cys Thr Leu Asp Gly Arg Asp Gly Trp Val Ala Tyr Trp Gly Gln Gly
            100                 105                 110

Thr Leu Val Thr Val Ser Ser Gly Gly Gly Gly Ser Gly Gly Gly Gly
        115                 120                 125

Ser Gly Gly Gly Gly Ser Asn Phe Met Leu Thr Gln Pro His Ser Val
    130                 135                 140

Ser Glu Ser Pro Gly Lys Thr Val Thr Ile Ser Cys Lys Arg Asn Thr
145                 150                 155                 160

Gly Asn Ile Gly Ser Asn Tyr Val Asn Trp Tyr Gln Gln His Glu Gly

165 170 175

Ser Ser Pro Thr Thr Ile Ile Tyr Arg Asn Asp Lys Arg Pro Asp Gly
180 185 190

Val Ser Asp Arg Phe Ser Gly Ser Ile Asp Arg Ser Ser Lys Ser Ala
195 200 205

Ser Leu Thr Ile Ser Asn Leu Lys Thr Glu Asp Glu Ala Asp Tyr Phe
210 215 220

Cys Gln Ser Tyr Ser Ser Gly Phe Ile Phe Gly Gly Gly Thr Lys Leu
225 230 235 240

Thr Val Leu Gly Ala Ala Ala Gly Ala Gly Gly Asp Tyr Lys Asp Asp
245 250 255

Asp Asp Lys Gly Ala Ala Ala His His His His His His
260 265

<210> 249
<211> 269
<212> PRT
<213> Artificial Sequence

<220>
<223> amino_acid_sequence_of_C3E-7089

<400> 249

Gly Glu Val Gln Leu Val Glu Ser Gly Gly Gly Leu Val Gln Pro Gly
1 5 10 15

Gly Ser Leu Arg Leu Ser Cys Ala Ala Ser Gly Val Thr Phe Asn Tyr
20 25 30

Tyr Gly Met Ser Trp Ile Arg Gln Ala Pro Gly Lys Gly Leu Glu Trp
35 40 45

Val Ala Ser Ile Thr Arg Ser Gly Gly Arg Ile Tyr Tyr Pro Asp Ser
50 55 60

Val Lys Gly Arg Phe Thr Ile Ser Arg Glu Asn Thr Gln Lys Thr Leu
65 70 75 80

Tyr Leu Gln Met Asn Ser Leu Arg Ala Glu Asp Thr Ala Val Tyr Tyr
85 90 95

Cys Thr Leu Asp Gly Arg Asp Gly Trp Val Ala Tyr Trp Gly Gln Gly
100 105 110

```
Thr Leu Val Thr Val Ser Ser Gly Gly Gly Gly Ser Gly Gly Gly Gly
        115                 120                 125

Ser Gly Gly Gly Gly Ser Asn Phe Met Leu Thr Gln Pro His Ser Val
    130                 135                 140

Ser Glu Ser Pro Gly Lys Thr Val Thr Ile Ser Cys Lys Arg Asn Thr
145                 150                 155                 160

Gly Asn Ile Gly Ser Asn Tyr Val Asn Trp Tyr Gln Gln His Glu Gly
                165                 170                 175

Ser Ser Pro Thr Thr Ile Ile Tyr Arg Ser Asp Lys Arg Pro Asp Gly
            180                 185                 190

Val Ser Asp Arg Phe Ser Gly Ser Ile Asp Arg Ser Ser Lys Ser Ala
        195                 200                 205

Ser Leu Thr Ile Ser Asn Leu Lys Thr Glu Asp Glu Ala Asp Tyr Phe
    210                 215                 220

Cys Gln Ser Tyr Ser Ser Gly Phe Ile Phe Gly Gly Gly Thr Lys Leu
225                 230                 235                 240

Thr Val Leu Gly Ala Ala Ala Gly Ala Gly Gly Asp Tyr Lys Asp Asp
                245                 250                 255

Asp Asp Lys Gly Ala Ala Ala His His His His His His
            260                 265


<210>  250
<211>  269
<212>  PRT
<213>  Artificial Sequence

<220>
<223>  amino_acid_sequence_of_C3E-7090

<400>  250

Gly Glu Val Gln Leu Val Glu Ser Gly Gly Gly Leu Val Gln Pro Gly
1               5                   10                  15

Gly Ser Leu Arg Leu Ser Cys Ala Ala Ser Gly Val Thr Phe Asn Tyr
            20                  25                  30

Tyr Gly Met Ser Trp Ile Arg Gln Ala Pro Gly Lys Gly Leu Glu Trp
        35                  40                  45
```

Val Ala Ser Ile Thr Arg Ser Gly Gly Arg Ile Tyr Tyr Pro Asp Ser
    50                  55                  60

Val Lys Gly Arg Phe Thr Ile Ser Arg Glu Asn Thr Gln Lys Thr Leu
65                  70                  75                  80

Tyr Leu Gln Met Asn Ser Leu Arg Ala Glu Asp Thr Ala Val Tyr Tyr
                85                  90                  95

Cys Thr Leu Asp Gly Arg Asp Gly Trp Val Ala Tyr Trp Gly Gln Gly
            100                 105                 110

Thr Leu Val Thr Val Ser Ser Gly Gly Gly Gly Ser Gly Gly Gly Gly
        115                 120                 125

Ser Gly Gly Gly Gly Ser Asn Phe Met Leu Thr Gln Pro His Ser Val
    130                 135                 140

Ser Glu Ser Pro Gly Lys Thr Val Thr Ile Ser Cys Lys Arg Asn Thr
145                 150                 155                 160

Gly Asn Ile Gly Ser Asn Tyr Val Asn Trp Tyr Gln Gln His Glu Gly
                165                 170                 175

Ser Ser Pro Thr Thr Ile Ile Tyr Arg Ala Asp Lys Arg Pro Asp Gly
            180                 185                 190

Val Ser Asp Arg Phe Ser Gly Ser Ile Asp Arg Ser Ser Lys Ser Ala
        195                 200                 205

Ser Leu Thr Ile Ser Asn Leu Lys Thr Glu Asp Glu Ala Asp Tyr Phe
    210                 215                 220

Cys Gln Ser Tyr Ser Ser Gly Phe Ile Phe Gly Gly Gly Thr Lys Leu
225                 230                 235                 240

Thr Val Leu Gly Ala Ala Ala Gly Ala Gly Gly Asp Tyr Lys Asp Asp
                245                 250                 255

Asp Asp Lys Gly Ala Ala Ala His His His His His His
            260                 265

<210>  251
<211>  269
<212>  PRT
<213>  Artificial Sequence

<220>

<223> amino_acid_sequence_of_C3E-7091

<400> 251

Gly Glu Val Gln Leu Val Glu Ser Gly Gly Gly Leu Val Gln Pro Gly
1 5 10 15

Gly Ser Leu Arg Leu Ser Cys Ala Ala Ser Gly Val Thr Phe Asn Tyr
20 25 30

Tyr Gly Met Ser Trp Ile Arg Gln Ala Pro Gly Lys Gly Leu Glu Trp
35 40 45

Val Ala Ser Ile Thr Ser Ser Gly Gly Arg Ile Tyr Tyr Pro Asp Ser
50 55 60

Val Lys Gly Arg Phe Thr Ile Ser Arg Glu Asn Thr Gln Lys Thr Leu
65 70 75 80

Tyr Leu Gln Met Asn Ser Leu Arg Ala Glu Asp Thr Ala Val Tyr Tyr
85 90 95

Cys Thr Leu Asp Gly Arg Asp Gly Trp Val Ala Tyr Trp Gly Gln Gly
100 105 110

Thr Leu Val Thr Val Ser Ser Gly Gly Gly Gly Ser Gly Gly Gly Gly
115 120 125

Ser Gly Gly Gly Gly Ser Asn Phe Met Leu Thr Gln Pro His Ser Val
130 135 140

Ser Glu Ser Pro Gly Lys Thr Val Thr Ile Ser Cys Lys Arg Asn Thr
145 150 155 160

Gly Asn Ile Gly Ser Asn Tyr Val Asn Trp Tyr Gln Gln His Glu Gly
165 170 175

Ser Ser Pro Thr Thr Ile Ile Tyr Arg Gly Asp Lys Arg Pro Asp Gly
180 185 190

Val Ser Asp Arg Phe Ser Gly Ser Ile Asp Arg Ser Ser Lys Ser Ala
195 200 205

Ser Leu Thr Ile Ser Asn Leu Lys Thr Glu Asp Glu Ala Asp Tyr Phe
210 215 220

Cys Gln Ser Tyr Ser Ser Gly Phe Ile Phe Gly Gly Gly Thr Lys Leu
225 230 235 240

Thr Val Leu Gly Ala Ala Ala Gly Ala Gly Gly Asp Tyr Lys Asp Asp
245 250 255

Asp Asp Lys Gly Ala Ala Ala His His His His His His
260 265

<210> 252
<211> 269
<212> PRT
<213> Artificial Sequence

<220>
<223> amino_acid_sequence_of_C3E-7092

<400> 252

Gly Glu Val Gln Leu Val Glu Ser Gly Gly Gly Leu Val Gln Pro Gly
1 5 10 15

Gly Ser Leu Arg Leu Ser Cys Ala Ala Ser Gly Val Thr Phe Asn Tyr
20 25 30

Tyr Gly Met Ser Trp Ile Arg Gln Ala Pro Gly Lys Gly Leu Glu Trp
35 40 45

Val Ala Ser Ile Thr Ser Ser Gly Gly Arg Ile Tyr Tyr Pro Asp Ser
50 55 60

Val Lys Gly Arg Phe Thr Ile Ser Arg Glu Asn Thr Gln Lys Thr Leu
65 70 75 80

Tyr Leu Gln Met Asn Ser Leu Arg Ala Glu Asp Thr Ala Val Tyr Tyr
85 90 95

Cys Thr Leu Asp Gly Arg Asp Gly Trp Val Ala Tyr Trp Gly Gln Gly
100 105 110

Thr Leu Val Thr Val Ser Ser Gly Gly Gly Gly Ser Gly Gly Gly Gly
115 120 125

Ser Gly Gly Gly Gly Ser Asn Phe Met Leu Thr Gln Pro His Ser Val
130 135 140

Ser Glu Ser Pro Gly Lys Thr Val Thr Ile Ser Cys Lys Arg Asn Thr
145 150 155 160

Gly Asn Ile Gly Ser Asn Tyr Val Asn Trp Tyr Gln Gln His Glu Gly
165 170 175

Ser Ser Pro Thr Thr Ile Ile Tyr Arg Gln Asp Lys Arg Pro Asp Gly
180 185 190

Val Ser Asp Arg Phe Ser Gly Ser Ile Asp Arg Ser Ser Lys Ser Ala
195 200 205

Ser Leu Thr Ile Ser Asn Leu Lys Thr Glu Asp Glu Ala Asp Tyr Phe
210 215 220

Cys Gln Ser Tyr Ser Ser Gly Phe Ile Phe Gly Gly Gly Thr Lys Leu
225 230 235 240

Thr Val Leu Gly Ala Ala Ala Gly Ala Gly Gly Asp Tyr Lys Asp Asp
245 250 255

Asp Asp Lys Gly Ala Ala Ala His His His His His His
260 265

<210> 253
<211> 269
<212> PRT
<213> Artificial Sequence

<220>
<223> amino_acid_sequence_of_C3E-7093

<400> 253

Gly Glu Val Gln Leu Val Glu Ser Gly Gly Gly Leu Val Gln Pro Gly
1 5 10 15

Gly Ser Leu Arg Leu Ser Cys Ala Ala Ser Gly Val Thr Phe Asn Tyr
20 25 30

Tyr Gly Met Ser Trp Ile Arg Gln Ala Pro Gly Lys Gly Leu Glu Trp
35 40 45

Val Ala Ser Ile Thr Ser Ser Gly Gly Arg Ile Tyr Tyr Pro Asp Ser
50 55 60

Val Lys Gly Arg Phe Thr Ile Ser Arg Glu Asn Thr Gln Lys Thr Leu
65 70 75 80

Tyr Leu Gln Met Asn Ser Leu Arg Ala Glu Asp Thr Ala Val Tyr Tyr
85 90 95

Cys Thr Leu Asp Gly Arg Asp Gly Trp Val Ala Tyr Trp Gly Gln Gly
100 105 110

Thr Leu Val Thr Val Ser Ser Gly Gly Gly Gly Ser Gly Gly Gly Gly

115 120 125

Ser Gly Gly Gly Gly Ser Asn Phe Met Leu Thr Gln Pro His Ser Val
130 135 140

Ser Glu Ser Pro Gly Lys Thr Val Thr Ile Ser Cys Lys Arg Asn Thr
145 150 155 160

Gly Asn Ile Gly Ser Asn Tyr Val Asn Trp Tyr Gln Gln His Glu Gly
165 170 175

Ser Ser Pro Thr Thr Ile Ile Tyr Arg Asn Asp Lys Arg Pro Asp Gly
180 185 190

Val Ser Asp Arg Phe Ser Gly Ser Ile Asp Arg Ser Ser Lys Ser Ala
195 200 205

Ser Leu Thr Ile Ser Asn Leu Lys Thr Glu Asp Glu Ala Asp Tyr Phe
210 215 220

Cys Gln Ser Tyr Ser Ser Gly Phe Ile Phe Gly Gly Gly Thr Lys Leu
225 230 235 240

Thr Val Leu Gly Ala Ala Ala Gly Ala Gly Gly Asp Tyr Lys Asp Asp
245 250 255

Asp Asp Lys Gly Ala Ala Ala His His His His His His
260 265

<210> 254
<211> 269
<212> PRT
<213> Artificial Sequence

<220>
<223> amino_acid_sequence_of_C3E-7094

<400> 254

Gly Glu Val Gln Leu Val Glu Ser Gly Gly Gly Leu Val Gln Pro Gly
1 5 10 15

Gly Ser Leu Arg Leu Ser Cys Ala Ala Ser Gly Val Thr Phe Asn Tyr
20 25 30

Tyr Gly Met Ser Trp Ile Arg Gln Ala Pro Gly Lys Gly Leu Glu Trp
35 40 45

Val Ala Ser Ile Thr Ser Ser Gly Gly Arg Ile Tyr Tyr Pro Asp Ser
50 55 60

```
Val Lys Gly Arg Phe Thr Ile Ser Arg Glu Asn Thr Gln Lys Thr Leu
65                  70                  75                  80

Tyr Leu Gln Met Asn Ser Leu Arg Ala Glu Asp Thr Ala Val Tyr Tyr
                85                  90                  95

Cys Thr Leu Asp Gly Arg Asp Gly Trp Val Ala Tyr Trp Gly Gln Gly
            100                 105                 110

Thr Leu Val Thr Val Ser Ser Gly Gly Gly Gly Ser Gly Gly Gly Gly
        115                 120                 125

Ser Gly Gly Gly Gly Ser Asn Phe Met Leu Thr Gln Pro His Ser Val
    130                 135                 140

Ser Glu Ser Pro Gly Lys Thr Val Thr Ile Ser Cys Lys Arg Asn Thr
145                 150                 155                 160

Gly Asn Ile Gly Ser Asn Tyr Val Asn Trp Tyr Gln Gln His Glu Gly
                165                 170                 175

Ser Ser Pro Thr Thr Ile Ile Tyr Arg Ser Asp Lys Arg Pro Asp Gly
            180                 185                 190

Val Ser Asp Arg Phe Ser Gly Ser Ile Asp Arg Ser Ser Lys Ser Ala
        195                 200                 205

Ser Leu Thr Ile Ser Asn Leu Lys Thr Glu Asp Glu Ala Asp Tyr Phe
    210                 215                 220

Cys Gln Ser Tyr Ser Ser Gly Phe Ile Phe Gly Gly Gly Thr Lys Leu
225                 230                 235                 240

Thr Val Leu Gly Ala Ala Ala Gly Ala Gly Gly Asp Tyr Lys Asp Asp
                245                 250                 255

Asp Asp Lys Gly Ala Ala Ala His His His His His His
            260                 265


<210>  255
<211>  269
<212>  PRT
<213>  Artificial Sequence

<220>
<223>  amino_acid_sequence_of_C3E-7095

<400>  255
```

Gly Glu Val Gln Leu Val Glu Ser Gly Gly Gly Leu Val Gln Pro Gly
1               5                   10                  15

Gly Ser Leu Arg Leu Ser Cys Ala Ala Ser Gly Val Thr Phe Asn Tyr
            20                  25                  30

Tyr Gly Met Ser Trp Ile Arg Gln Ala Pro Gly Lys Gly Leu Glu Trp
        35                  40                  45

Val Ala Ser Ile Thr Ser Ser Gly Gly Arg Ile Tyr Tyr Pro Asp Ser
    50                  55                  60

Val Lys Gly Arg Phe Thr Ile Ser Arg Glu Asn Thr Gln Lys Thr Leu
65                  70                  75                  80

Tyr Leu Gln Met Asn Ser Leu Arg Ala Glu Asp Thr Ala Val Tyr Tyr
                85                  90                  95

Cys Thr Leu Asp Gly Arg Asp Gly Trp Val Ala Tyr Trp Gly Gln Gly
            100                 105                 110

Thr Leu Val Thr Val Ser Ser Gly Gly Gly Gly Ser Gly Gly Gly Gly
        115                 120                 125

Ser Gly Gly Gly Gly Ser Asn Phe Met Leu Thr Gln Pro His Ser Val
    130                 135                 140

Ser Glu Ser Pro Gly Lys Thr Val Thr Ile Ser Cys Lys Arg Asn Thr
145                 150                 155                 160

Gly Asn Ile Gly Ser Asn Tyr Val Asn Trp Tyr Gln Gln His Glu Gly
                165                 170                 175

Ser Ser Pro Thr Thr Ile Ile Tyr Arg Ala Asp Lys Arg Pro Asp Gly
            180                 185                 190

Val Ser Asp Arg Phe Ser Gly Ser Ile Asp Arg Ser Ser Lys Ser Ala
        195                 200                 205

Ser Leu Thr Ile Ser Asn Leu Lys Thr Glu Asp Glu Ala Asp Tyr Phe
    210                 215                 220

Cys Gln Ser Tyr Ser Ser Gly Phe Ile Phe Gly Gly Gly Thr Lys Leu
225                 230                 235                 240

Thr Val Leu Gly Ala Ala Ala Gly Ala Gly Gly Asp Tyr Lys Asp Asp
                245                 250                 255

```
Asp Asp Lys Gly Ala Ala Ala His His His His His His
            260                 265
```